# EUROPEAN PATENT SPECIFICATION

(11) **EP 1 660 492 B1**
(45) Date of publication and mention of the grant of the patent: **20.08.2008**
(21) Application number: 04779375.7
(22) Date of filing: 27.07.2004
(51) Int. Cl.: C07D 417/12, C07D 417/14, C07D 277/68, C07D 263/58, C07D 471/10, C07D 491/10, C07D 235/26, C07D 401/12, C07D 413/12, A61K 31/428, A61K 31/4184, A61K 31/4535, A61K 31/423, A61P 29/00

(54) **BENZIMIDAZOLE, BENZTHIAZOLE AND BENZOXAZOLE DERIVATIVES AND THEIR USE AS LTA4H MODULATORS**
BENZIMIDAZOL-, BENZOTHIAZOL- UND BENZOXAZOLDERIVATE UND DEREN VERWENDUNG ALS LTA4H-MODULATOREN
DERIVES DE BENZIMIDAZOLE, BENZTHIAZOLE AND BENZOXAZOLE ET LEUR UTILISATION EN TANT QUE MODULATEURS DE LTA4H

(30) Priority: 28.07.2003 US 490710 P
(43) Date of publication of application: 31.05.2006
(73) Proprietor: Janssen Pharmaceutica NV, 2340 Beerse (BE)
(72) Inventor: AXE, Frank, U., Escondido, CA 92027 (US); BEMBENEK, Scott, D., San Diego, CA 92130 (US); BUTLER, Christopher, R., Urbana, IL 61801 (US); EDWARDS, James, P., San Diego, CA 92129 (US); FOURIE, Anne, M., San Diego, CA 92130 (US); GRICE, Cheryl, A., Carlsbad, CA 92009 (US); SAVALL, Brad, M., San Diego, CA 92123 (US); TAYS, Kevin, L., Cardiff-By-The-Sea, CA 92007 (US); WEI, Jianmei, San Diego, CA 92129 (US)
(74) Representative: Fisher, Adrian John
(86) International application number: PCT/US2004/024309
(87) International publication number: WO 2005/012297

(56) References cited:
- EP-A- 1 221 441
- WO-A-98/40364
- US-A- 4 873 346
- PENNING THOMAS D ET AL: "Structure-Activity Relationship Studies on 1-[2-(4- Phenylphenoxy)ethyl]pyrrolidine (SC-22716), a Potent Inhibitor of Leukotriene A4 (LTA4) Hydrolase" JOURNAL OF MEDICINAL CHEMISTRY, AMERICAN CHEMICAL SOCIETY. WASHINGTON, US, vol. 43, 2000, pages 721-725, XP002197495 ISSN: 0022-2623
- PALMER P J ET AL: "ANTIMICROBIALS. 2. SUBSTITUTED BENZOTHIAZOLYLBENZYLAMINES AND RELATED COMPOUNDS" JOURNAL OF MEDICINAL CHEMISTRY, AMERICAN CHEMICAL SOCIETY. WASHINGTON, US, vol. 14, no. 12, 1971, pages 1226-1227, XP000877145 ISSN: 0022-2623
- DATABASE CA [Online] CHEMICAL ABSTRACTS SERVICE, COLUMBUS, OHIO, US; SHARPE, C. J. ET AL: "Basic ethers of 2-anilinobenzothiazoles and 2-anilinobenzoxazoles as potential antidepressants" XP002308822 retrieved from STN Database accession no. 1972:443068 & JOURNAL OF MEDICINAL CHEMISTRY , 15(5), 523-9 CODEN: JMCMAR; ISSN: 0022-2623, 1972,
- DATABASE CA [Online] CHEMICAL ABSTRACTS SERVICE, COLUMBUS, OHIO, US; HANKOVSZKY, H. O. ET AL: "Benzazoles. VI. O-Alkylation of 2-(hydroxyphenyl)- and 2-(hydroxybenzyl) benzazoles" XP002308823 retrieved from STN Database accession no. 1968:506619 & ACTA CHIMICA ACADEMIAE SCIENTIARUM HUNGARICAE , 57(2), 219-223 CODEN: ACASA2; ISSN: 0001-5407, 1968,
- DATABASE CA [Online] CHEMICAL ABSTRACTS SERVICE, COLUMBUS, OHIO, US; COSSEY, H. D. ET AL: "Some antimicrobial compounds in the heterocyclic series. IV. Nonphotosensitizing basic ethers in the benzothiazole series" XP002308824 retrieved from STN Database accession no. 1965:51592 & JOURNAL OF THE CHEMICAL SOCIETY, ABSTRACTS , (FEB.), 954-73 CODEN: JCSAAZ; ISSN: 0590-9791, 1965,

## Description

### Field of the Invention

This invention relates to leukotriene A4 hydrolase (LTA4H) inhibitors for the treatment of inflammation. More particularly, this invention relates to certain benzooxazol-2-yl, benzothiazol-2-yl and 1*H*-benzoimidazol-2-yl compounds useful as selective inhibitors of the LTA4H enzyme for the treatment of inflammatory conditions.

### Background of the Invention

Inflammation is normally an acute response by the immune system to invasion by microbial pathogens, chemicals or physical injury. In some cases, however, the inflammatory response can progress to a chronic state, and be the cause of inflammatory disease. Therapeutic control of this chronic inflammation in diverse diseases is a major medical need.

Leukotrienes (LT) are biologically active metabolites of arachidonic acid (B. Samuelsson, Science 1983, 220(4597):568-575) that have been implicated in inflammatory diseases, including asthma (D.A. Munafo et al., J. Clin. Invest. 1994, 93(3):1042-1050), inflammatory bowel disease (IBD) (P. Sharon and W.F. Stenson, Gastroenterology 1984, 86(3):453-460), chronic obstructive pulmonary disease (COPD) (P.J. Barnes, Respiration 2001, 68(5):441-448), arthritis (R.J. Griffiths et al., Proc. Natl. Acad. Sci. U.S.A. 1995, 92(2):517-521; F. Tsuji et al., Life Sci. 1998, 64(3):L51-L56), psoriasis (K. Ikai, J. Dermatol. Sci. 1999, 21(3):135-146; Y.I. Zhu and M.J. Stiller, Skin Pharmacol. Appl. Skin Physiol. 2000, 13(5):235-245) and atherosclerosis (Friedrich, E. B. et al. Arterioscler Thromb Vasc Biol 23, 1761-7 (2003); Subbarao, K. et al. Arterioscler Thromb Vasc Biol 24, 369-75 (2004); Helgadottir, A. et al. Nat Genet 36, 233-9 (2004); Jala, V.R. et al Trends in Immun. 25, 315-322 (2004)). The synthesis of leukotrienes is initiated by the conversion of arachidonic acid to an unstable epoxide intermediate, leukotriene A4 (LTA4), by 5-lipoxygenase (5-LO)(A.W. Ford-Hutchinson et al., Annu. Rev. Biochem. 1994, 63:383-347). This enzyme is expressed predominantly by cells of myeloid origin, particularly neutrophils, eosinophils, monocytes/macrophages and mast cells (G.K. Reid et al., J. Biol. Chem. 1990, 265(32):19818-19823). LTA4 can either be conjugated with glutathione by leukotriene C4 (LTC4) synthase to produce the cysteinyl leukotriene, LTC4, or hydrolyzed to the diol, leukotriene B4 (LTB4) (B. Samuelsson, Science 1983, 220(4597):568-575). LTC4 and its metabolites, LTD4 and LTE4, induce smooth muscle contraction, broncho-constriction and vascular permeability, while LTB4 is a potent chemo-attractant and activator of neutrophils.

The stereospecific hydrolysis of LTA4 to LTB4 is catalyzed by leukotriene A4 hydrolase (LTA4H), a zinc-containing, cytosolic enzyme. This enzyme is ubiquitously expressed, with high levels in small intestinal epithelial cells, lung, and aorta (B. Samuelsson and C.D. Funk, J. Biol. Chem. 1989, 264(33):19469-19472). Moderate expression of LTA4H is observed in leukocytes, particularly neutrophils (T. Yokomizo et al., J. Lipid Mediators Cell Signalling 1995, 12(2,3):321-332).

Leukotriene B4 is a key pro-inflammatory mediator, able to recruit inflammatory cells, such as neutrophils and eosinophils, as well as activate neutrophils (F.A. Fitzpatrick et al., Ann. N. Y. Acad. Sci. 1994, 714:64-74; S.W. Crooks and R.A. Stockley, Int. J. Biochem. Cell Biol. 1998, 30(2):173-178; A. Klein et al., J. Immunol. 2000, 164:4271-4276). LTB4 mediates its pro-inflammatory effects by binding to G protein-coupled receptors, leukotriene B4 receptor 1 (BLT1) and leukotriene B4 receptor 2 (BLT2) (T. Yokomizo et al., Arch. Biochem. Biophys. 2001, 385(2):231-241). The receptor first identified, BLT1, binds LTB₄ with high affinity, leading to intracellular signaling and chemotaxis. BLT1 is expressed mainly in peripheral leukocytes, particularly neutrophils, eosinophils, macrophages (Huang, W. W. et al. J Exp Med 188, 1063-74 (1998)) and monocytes (Yokomizo, T., Izumi, T. & Shimizu, T. Life Sci 68, 2207-12 (2001)). The murine receptor is also expressed on effector T cells and was recently shown to mediate LTB₄-dependent migration of effector CD8⁺ T cells (Goodarzi, K., Goodarzi, M., Tager, A. M., Luster, A. D. & von Andrian, U. H. Nat Immunol 4, 965-73 (2003).Ott, V. L., Cambier, J. C., Kappler, J., Marrack, P. & Swanson, B. J. Nat Immunol 4, 974-81 (2003)), early effector CD4⁺ T helper type 1 (T_{H}1) and T_{H}2 chemotaxis and adhesion to endothelial cells, as well as early effector CD4⁺ and CD8⁺ T cell recruitment in an asthma animal model (Tager, A. M. et al.. Nat Immunol 4, 982-90 (2003)).LTB4 receptor BLT2 (S. Wang et al., J. Biol. Chem. 2000, 275(52):40686-40694; T. Yokomizo et al., J. Exp. Med. 2000,192(3):421-431) shares 42% amino acid homology with BLT1, but is more broadly expressed, including in peripheral tissues such as the spleen, ovary and liver, as well as in leukocytes. BLT2 binds LTB4 with lower affinity than BLT1 does, mediates chemotaxis at higher concentrations of LTB4, and differs from BLT1 in its affinity for certain antagonists. While LTB4 receptor antagonists may differ in their affinity for BLT1 versus BLT2, blocking the production of LTB4 using LTA4H inhibitors would be expected to inhibit the downstream events mediated through both BLT1 and BLT2.

Studies have shown that introduction of exogenous LTB4 into normal tissues can induce inflammatory symptoms (R.D.R. Camp et al., Br. J. Pharmacol. 1983, 80(3):497-502; R. Camp et al., J. Invest. Dermatol. 1984, 82(2):202-204). Elevated levels of LTB4 have been observed in a number of inflammatory diseases including IBD, COPD, psoriasis, rheumatoid arthritis (RA), cystic fibrosis and asthma (S.W. Crooks and R.A. Stockley, Int. J. Biochem. Cell Biol. 1998, 30(2):173-178). Therefore, reduction of LTB4 production by an inhibitor of LTA4H activity would be predicted to have therapeutic potential in a wide range of diseases.

This idea is supported by a study of LTA4H-deficient mice that, while otherwise healthy, exhibited markedly decreased neutrophil influx in arachidonic acid-induced ear inflammation and zymosan-induced peritonitis models (R.S. Byrum et al., J. Immunol. 1999, 163(12): 6810-6819). LTA4H inhibitors have been shown to be effective anti-inflammatory agents in preclinical studies. For example, oral administration of LTA4H inhibitor SC57461 caused inhibition of ionophore-induced LTB4 production in mouse blood ex vivo, and in rat peritoneum in vivo (J.K. Kachur et al., J. Pharm. Exp. Ther. 2002, 300(2), 583-587). Eight weeks of treatment with the same inhibitor compound significantly improved colitis symptoms in cotton top tamarins (T.D. Penning, Curr. Pharm. Des. 2001, 7(3):163-179). The spontaneous colitis that develops in these animals is very similar to human IBD. The results therefore indicate that LTA4H inhibitors would have therapeutic utility in this and other human inflammatory diseases.

Events that elicit the inflammatory response include the formation of the pro-inflammatory mediator leukotriene B4. Hydrolase LTA4H catalyzes the formation of this mediator, and LTA4H inhibitors block the production of the pro-inflammatory mediator LTB4, thus providing the ability to prevent and/or treat leukotriene-mediated conditions, such as inflammation. The inflammatory response is characterized by pain, increased temperature, redness, swelling, or reduced function, or by a combination of two or more of these symptoms. Regarding the onset and evolution of inflammation, inflammatory diseases or inflammation-mediated diseases or conditions include, but are not limited to, acute inflammation, allergic inflammation, and chronic inflammation.

Examples of textbooks on the subject of inflammation include J. I. Gallin and R. Snyderman, Inflammation: Basic Principles and Clinical Correlates, 3rd Edition, (Lippincott Williams & Wilkins, Philadelphia, 1999); V. Stvrtinova, J. Jakubovsky and I. Hulin, "Inflammation and Fever", Pathophysiology Principles of Diseases (Textbook for Medical Students, Academic Press, 1995); Cecil et al., Textbook Of Medicine, 18th Edition (W.B. Saunders Company, 1988); and Steadmans Medical Dictionary.

Background and review material on inflammation and conditions related with inflammation can be found in articles such as the following: C. Nathan, Points of control in inflammation, Nature 2002, 420:846-852; K.J. Tracey, The inflammatory reflex, Nature 2002, 420:853-859; L.M. Coussens and Z. Werb, Inflammation and cancer, Nature 2002, 420:860-867; P. Libby, Inflammation in atherosclerosis, Nature 2002, 420:868-874; C. Benoist and D. Mathis, Mast cells in autoimmune disease, Nature 2002, 420:875-878; H.L. Weiner and D.J. Selkoe, Inflammation and therapeutic vaccination in CNS diseases, Nature 2002, 420:879-884; J. Cohen, The immunopathogenesis of sepsis, Nature 2002, 420:885-891; D. Steinberg, Atherogenesis in perspective: Hypercholesterolemia and inflammation as partners in crime, Nature Medicine 2002, 8(11):1211-1217. Cited references are incorporated herein by reference.

Inflammation is due to any one of a plurality of conditions, such as asthma, chronic obstructed pulmonary disease (COPD), atherosclerosis, rheumatoid arthritis, multiple sclerosis, inflammatory bowel diseases (including Crohn's disease and ulcerative colitis), or psoriasis, which are each characterized by excessive or prolonged inflammation at some stage of the disease.

O-Alkylation of 2-(hydroxyphenyl)- and 2-(hydroxybenzyl) benzazoles has been described by Hankovskzky, H.O. and Hideg, K. (Benzazoles. VI, Acta Chimica Academie Scientarium Hungaricae Tomus 57(2), pages 219-223, 1968). Nonphotosensitizing basic ethers in the benzothiazole series have also been described by Cossey, H.D. *et al*, (XP002308824 retrieved from STN Database accession no. 1965:51592; CHEMICAL ABSTRACTS SERVICE, COLUMBUS, OHIO, US). In addition basic ethers of 2-anilinobenzothiazoles and 2-anilinobenzoxazoles have been shown to be potential antidepressants (Sharpe, C..J. et. al.,: Journal of Med. Chemistry, 1972, 15:5 pages 523-529).

Applicants have discovered benzooxazol-2-yl, benzothiazol-2-yl and 1*H-*benzoimidazol-2-yl compounds and derivatives thereof; their use as inhibitors of enzymes, such as the LTA4H enzyme, in the formation of pro-inflammatory mediators, such as the LTB4 mediator; also their use for the treatment of inflammatory conditions; and the preparation of pharmaceutical compositions for the treatment of inflammation.

### Summary of the Invention

There are provided by the present invention LTA4H enzyme inhibitors, which have the following general formula (I): 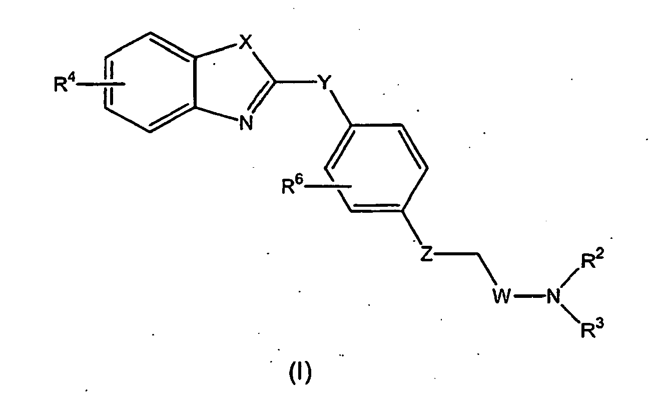 wherein
X is selected from the group consisting of NR⁵, O, and S, with R⁵ being one of H and CH₃;
Y is selected from the group consisting of CH₂, and O;
Z is selected from the group consisting of O and bond;
W is selected from the group consisting of CH₂ and CHR¹-CH₂, with R¹ being one of H and OH, wherein the R¹-attached carbon member in said CHR¹-CH₂ is not directly attached to the nitrogen member to which said W is attached;
R⁴ is selected from the group consisting of H, OCH₃, Cl, F, Br, I, OH, NH₂, CN, CF₃ and CH₃;
R⁶ is H or F; and
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said -C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two -N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=; and
L) one of 2-(4-ethyl-phenoxy)-benzothiazole, 2-(4-ethyl-phenoxy)-benzooxazole, and 2-(4-ethyl-phenoxy)-1*H*-benzoimidazole;
M) SO₂C₁₋₄alkyl;
alternatively R² and R³ are taken together with the nitrogen to which they are attached to form a heterocyclic ring that contains at least one heteroatom member that is said attachment nitrogen, said heterocyclic ring being selected from the group consisting of
i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄alkylOR^{Y}, -C₀₋₄alkylC(O)NR^{Y}R^{Z}, -C₀₋₄alkylNR^{Y}C(O)R^{Z}, - C(O)NR^{Z}OR^{Y}, -C₀₋₄alkylNR^{Y}C(O)CH₂OR^{Y}, - C₀₋₄alkylNR^{Y}C(O)CH₂C(O)R^{Y}, -C₀₋₄alkylNR^{Y}CO₂R^{Y}, - G₀₋₄alkylNR^{Y}C(O)NR^{Y}R^{Z}, -C₀₋₄alkylNR^{Y}C(S)NR^{Y}R^{Z}, -NR^{Y}C(O)CO₂R^{Y},-NR^{Y}R^{Z}, -C₀₋₄alkylNR^{W}SO₂R^{Y},1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H*-tetrazol-5-yl, pyrrolidine-2-thion-1-yl, piperidine-2-thion-1-yl, -C₀₋₄alkylC(O)N(R^{Y})(SO₂R^{Y}), -C₀₋₄alkylN(R^{Y})(SO₂)NR^{Y}R^{Y}, -C₀₋₄alkylN(R^{Y})(SO₂)NR^{Y}CO₂R^{Y}, 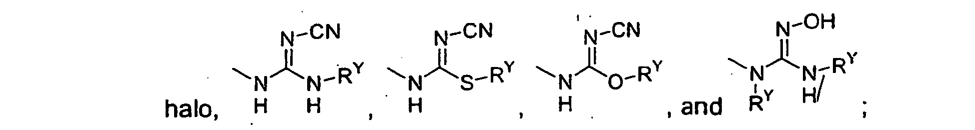
ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y}, -CO₂R^{Y} -C₃₋₄alkylCO₂R^{Y} and R^{Z};
iii) one of imidazolidin-1-yl, 2-imidazolin-1-yl, pyrazol-1-yl, imidazol-1-yl, 2*H*-tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1 H-tetrazol-1-yl is substituted at the carbon member with 0 or 1 of -C₀₋₄alkylR^{Z}, -C₀₋₄alkylSR^{Y}, -C₀₋₄alkylCO₂R^{Y}, and substituent HetR^{a}; and
iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR^{M} as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N}
R^{L} is selected from the group consisting of -CO₂R^{S} and -C(O)NR^{S}R^{S'};
R^{M} is selected from the group consisting of R^{Z}, indol-7-yl, -SO₂R^{Y}, -C₃₋₄alkylCO₂R^{Y}, -CO₂R^{Y}, -C(O)NR^{Z}OR^{Y}, -C(O)R^{Y}, -C(O)C₁₋₄alkylOR^{Y}, - C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H*-tetrazol-5-yl,
R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN, CF₃, CH₃, OC(O)CH₃, and NO₂;
R^{P} is selected from the group consisting of R^{Y}, -C₂₋₄alkylOR^{Y}, R^{Ar}, -C₁₋₂alkylCO₂R^{Y},-C₁₋₂alkylCONR^{S}R^{S'}, indol-7-yl, and -SO₂C₁₋₄alkyl;
R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y};
R^{S} and R^{S'} are independently selected from the group consisting of H, -C₁₋₄alkyl, and -C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L};
R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar};
R^{Y} is selected from the group consisting of H, -C₁₋₄alkyl, -C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y}, -C₁₋₂alkylCO₂R^{Y}, -C₁₋₂alkylC(O)NR^{S}R^{S'}, and -C₂₋₄alkylNR^{S}R^{S'};
when R^{Y} and R^{Z} are attached to a nitrogen member, R^{Y} and R^{Z} are selected as defined above, or R^{Y} and R^{Z} are taken together with the R^{Y}- and R^{Z}-attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring HetR^{d} having 0 or 1 valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1
unsaturated carbon-carbon bonds and having 0 or 1 carbonyl members;
or an enantiomer, diasteromer, racemic, tautomer, hydrate, solvate, or a pharmaceutically acceptable salt, ester, or amide thereof.

Embodiments of the present invention comprise new compounds that are LTA4H enzyme inhibitors and have the general formula (II): 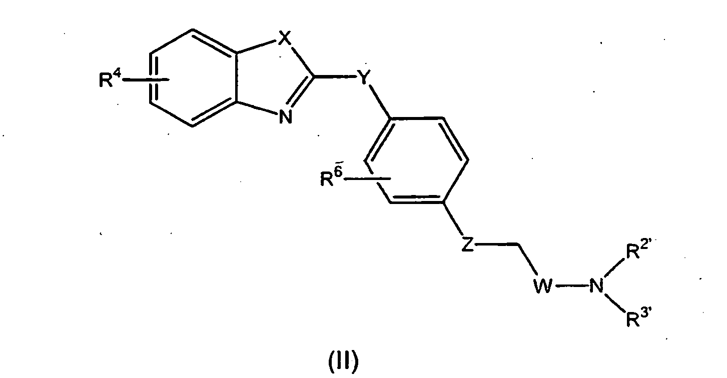 or an enantiomer, diasteromer, racemic, tautomer, hydrate, solvate, or a pharmaceutically acceptable salt, ester, or amide thereof,
wherein
R⁴, R⁶, X, Y, Z, and W are defined as in compound of formula (I), R^{2'} is defined as R² in compound of formula (I), and R^{3'} is defined as R³ in compound of formula (I), provided that
(a) at least one of said R^{2'} and R^{3'} is not ethyl when one selection in the group consisting of selections (s1), (s2), (s3), and (s4), is satisfied, and each of said selections is specified as
   (s1): R⁴ is H, Z is O, W is CH₂, Y is CH₂, and X is S;
   (s2): R⁴ is H, Z is O, W is CH₂, Y is CH₂, and X is NH;
   (s3): R⁴ is H, Z is O, W is CH₂, Y is O, and X is S;
   (s4): R⁴ is 5-chloro, Z is O, W is CH₂, Y is CH₂, and X is S;
(b) further provided that when Z is bond, Y is CH₂, W is CHR¹-CH₂, R¹ is H, and one of R^{2'} and R^{3'} is 1*H*-imidazol-2-yl, then the other of R^{2'} and R^{3'} is selected from A1), B)-L), wherein B)-L) are as defined above for compound of formula (I), and A1) consists of H, C₃₋₇alkenyl, wherein the carbon in said C₃₋₇alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl; and
(c) further provided that when X is S, Y is O, Z is bond, and W is CH₂, then one of R^{2'} and R^{3'} Is not XCG when the other is C₁₋₆alkyl, wherein XCG is the group 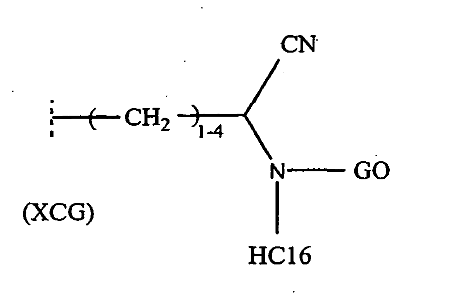
wherein HC16 is one of H, C₁₋₆alkyl, haloC₁₋₆alkyl, allyl, and C₁₋₆alkoxymethyl, and GO is a group attached by a carbon member that has a =O substituent forming an amido group (>N-C(O)-) with the nitrogen member to wich said GO group is attached.

Other embodiments of the present invention comprise new compounds that are LTA4H enzyme inhibitors and have the general formula (III): 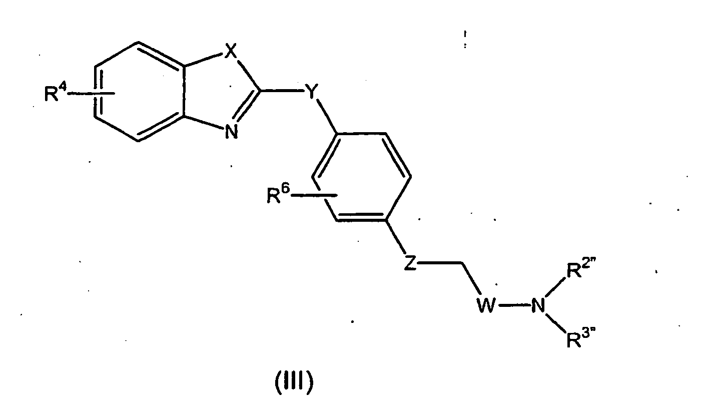 or an enantiomer, diasteromer, racemic, tautomer, hydrate, solvate, or a pharmaceutically acceptable salt, ester, or amide thereof,
wherein
R⁴, R⁶, X, Y, Z, and W are defined as in compound of formula (I), R^{2"} is defined as R² in compound of formula (I), and R^{3"} is defined as R³ in compound of formula (I), provided that
(a) said R^{2"} and R^{3"} further satisfy one of the following:
   (e1): at least one of said R^{2"} and R^{3"} is not C₁₋₅alkyl, when Z is O and X is S;
   (e2): none of R^{2"} and R^{3"} is C₁₋₄alkylC(O)R^{X}, with R^{X} being one of C₁₋₄alkyl, OH, -OC₁₋₄alkyl, -OC₀₋₄alkylR^{Ar}, or -NR^{Y}R^{Y}, when Y is O, Z is bond, and R^{2"} is different from R^{3"}; and
   (e3): none of R^{2"} and R^{3"} is -C₁₋₆alkylCN, when Y is O, Z is bond, and R^{2"} is different from R^{3"}; and
(b) further provided that when X is S, Y is O, Z is bond, and W is CH₂, then one of R^{2"} and R^{3"} is not XCG when the other is C₁₋₆alkyl, wherein XCG is the group 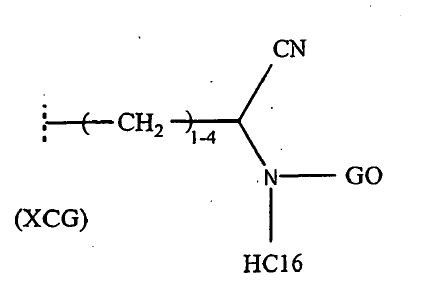
wherein HC16 is one of H, C₁₋₆alkyl, haloC₁₋₆alkyl, allyl, and C₁₋₆alkoxymethyl, and GO is a group attached by a carbon member that has a =O substituent forming an amido group (>N-C(O)-) with the nitrogen member to which said GO group is attached.

Isomeric forms of the compounds of the foregoing formulae, and of their pharmaceutically acceptable salts, amides and esters, are encompassed within the present invention, and reference herein to one of such isomeric forms is meant to refer to at least one of such isomeric forms. One of ordinary skill in the art will recognize that compounds according to this invention may exist, for example in a single isomeric form whereas other compounds may exist in the form of a regioisomeric mixture.

Whether stated explicitly or not in any part of the written description and claims, it is understood that each substituent and member assignment in the context of this invention is made independently of any other member and substituent assignment, unless stated otherwise. By way of a first example on substituent terminology, if substituent S¹ₑₓₐₘₚₗₑ is one of S₁ and S₂, and substituent S²ₑₓₐₘₚₗₑ is one of S₃ and S₄, then these assignments refer to embodiments of this invention given according to the choices S¹ₑₓₐₘₚₗₑ is S₁ and S²ₑₓₐₘₚₗₑ is S₃; S¹ₑₓₐₘₚₗₑ is S₁ and S²ₑₓₐₘₚₗₑ is S₄; S¹ₑₓₐₘₚₗₑ is S₂ and S²ₑₓₐₘₚₗₑ is S₃; S¹ₑₓₐₘₚₗₑ is S₂ and S²ₑₓₐₘₚₗₑ is S₄; and equivalents of each one of such choices. The shorter terminology "S¹ₑₓₐₘₚₗₑ is one of S₁ and S₂, and S²ₑₓₐₘₚₗₑ is one of S₃ and S₄" is accordingly used herein for the sake of brevity, but not by way of limitation. The foregoing first example on substituent terminology, which is stated in generic terms, is meant to illustrate the various substituent R assignments described herein. The foregoing convention given herein for substituents extends, when applicable, to members such as X, Y, Z, and W, and the index n.

Furthermore, when more than one assignment is given for any member or substituent, embodiments of this invention comprise the various groupings that can be made from the listed assignments, taken independently, and equivalents thereof. By way of a second example on substituent terminology, if it is herein described that substituent Sₑₓₐₘₚₗₑ is one of S₁, S₂, and S₃, this listing refers to embodiments of this invention for which Sₑₓₐₘₚₗₑ is S₁; Sₑₓₐₘₚₗₑ is S₂; Sₑₓₐₘₚₗₑ is S₃; Sₑₓₐₘₚₗₑ is one of S₁ and S₂; Sₑₓₐₘₚₗₑ is one of S₁ and S₃; Sₑₓₐₘₚₗₑ is one of S₂ and S₃; Sₑₓₐₘₚₗₑ is one of S₁, S₂ and S₃; and Sₑₓₐₘₚₗₑ is any equivalent of each one of these choices. The shorter terminology "Sₑₓₐₘₚₗₑ is one of S₁, S₂, and S₃" is accordingly used herein for the sake of brevity, but not by way of limitation. The foregoing second example on substituent terminology, which is stated in generic terms, is meant to illustrate the various substituent R assignments described herein. The foregoing convention given herein for substituents extends, when applicable, to members such as X, Y, Z, and W, and the index n.

The nomenclature "Cᵢ₋ⱼ" with j > i, when applied herein to a class of substituents, is meant to refer to embodiments of this invention for which each and every one of the number of carbon members, from i to j including i and j, is independently realized. By way of example, the term C₁₋₃ refers independently to embodiments that have one carbon member (C₁), embodiments that have two carbon members (C₂), and embodiments that have three carbon members (C₃).

The term Cₙ₋ₘalkyl refers to an aliphatic chain, whether straight or branched, with a total number N of carbon members in the chain that satisfies n ≤N ≤m, with m > n.

When any variable referring to a substituent, compound member or index, occurs more than once, the full range of assignments is meant to apply to each occurrence, independently of the specific assignment(s) to any other occurrence of such variable.

According to the foregoing interpretive considerations on assignments and nomenclature, it is understood that explicit reference herein to a set implies, where chemically meaningful and unless indicated otherwise, independent reference to embodiments of such set, and reference to each and every one of the possible embodiments of subsets of the set referred to explicitly.

The present invention also features the use of such compounds in the manufacture of a medicament for inhibiting LTA4H enzyme activity, and pharmaceutical compositions containing such compounds and use of such compositions in the manufacture of a medicament for treatment or prevention of conditions that are mediated by LTA4H enzyme activity.

Pharmaceutical compositions according to the present invention include at least one of the compounds of the present invention. If more than one of such compounds is included in a composition, the therapeutically effective amount may be a jointly effective amount. As such inhibitors of the LTA4H enzyme, compounds and compositions according to the present invention are useful in the manufacture of medicament for prevention, inhibition, or treatment of inflammation.

The invention also features the use of a pharmaceutical composition for the manufacture of a medicament treating or preventing an LTA4H-mediated condition in a subject, comprising a therapeutically effective amount of at least one LTA4H modulator selected from compounds of formulae (I), (II), and (II), enantiomers, diastereomers, racemates thereof, pharmaceutically acceptable salts, amides and esters thereof. In addition, the invention features the use of a pharmaceutical composition for the manufacture of a medicament inhibiting inflammatory response in a subject, comprising a therapeutically effective amount of at least LTA4H inhibitor selected from compounds of formulae (I), (II), and (III), enantiomers, diastereomers, racemates thereof, pharmaceutically acceptable salts, amides and esters thereof. The invention additionally features the use of an anti-inflammatory composition, comprising a therapeutically effective amount of at least one anti-inflammatory compound selected from compounds of formulae (I), (II), and (III), enantiomers, diastereomers, racemates thereof, pharmaceutically acceptable salts, amides and esters thereof.

The invention features the use of a therapeutically effective amount of at least one anti-inflammatory compound selected from compounds of formulae (I), (II), and (III), enantiomers, diastereomers, racemates thereof, pharmaceutically acceptable salts, amides and esters thereof in the manufacture of a medicament for treating or preventing inflammation in a subject. ] The invention also features the use of a therapeutically effective amount of at least one LTA4H modulator selected from compounds of formulae (I), (II), and (III), enantiomers, diastereomers, racemates thereof, pharmaceutically acceptable salts, amides and esters thereof in the manufacture of a medicament for treating or preventing an LTA4H-mediated condition in a subject. Furthermore, the invention features the use of a therapeutically effective amount of at least one LTA4H inhibitor selected from compounds of formulae (I), (II), and (III), enantiomers, diastereomers, racemates thereof, pharmaceutically acceptable salts, amides and esters thereof in the manufacture of a medicament for inhibiting inflammation in a subject.

This invention features the use of the compounds above in the manufacture of medicaments for the treatment, preventions and/or inhibition of conditions that are associated with and/or cause inflammation, such as any one or a plurality of the following conditions: Asthma, chronic obstructed pulmonary disease (COPD), atherosclerosis, rheumatoid arthritis, multiple sclerosis, inflammatory bowel diseases (including Crohn's disease and ulcerative colitis), or psoriasis, which are each characterized by excessive or prolonged inflammation at some stage of the disease.

Additional features and advantages of the invention will become apparent from the detailed description below, including examples, and the appended claims.

### Detailed Description of the Invention

The present invention is directed to compounds of formula (I), (II), or (III) as herein defined, enantiomers, diastereomers, racemates thereof, pharmaceutically acceptable salts, amides and esters thereof, pharmaceutical compositions that contain at least one of such compounds, use of such compound for in, manufacture of medicaments for treatment and/or prevention of conditions such as those that are mediated by LTA4H, and methods of making such pharmaceutical compositions.

The following terms are defined below, and by their usage throughout the disclosure.

"Alkyl" includes straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl does not include cycloalkyl.

"Alkenyl" includes straight chain and branched hydrocarbon radicals as above with at least one carbon-carbon double bond (sp²). Unless indicated otherwise by the prefix that indicates the number of carbon members, alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on.

"Alkynyl" includes straight chain and branched hydrocarbon radicals as above with at least one carbon-carbon triple bond (sp). Unless indicated otherwise by the prefix that indicates the number of carbon members, alkynyls include ethynyl, propynyls, butynyls, and pentynyls. Hydrocarbon radicals having a mixture of double bonds and triple bonds, such as 2-penten-4-ynyl, are grouped as alkynyls herein.

"Alkoxy" includes a straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing, the terminal oxygen atom of alkoxy with, respectively, NH (or NR), S, and SO₂.

Unless indicated otherwise by the prefix that indicates the number of carbon members, "cycloalkyl" includes cyclopropyl, cyclobutyl, cyclopentyl, cyclohexyl, cycloheptyl, cyclooctyl, and so on.

Unless indicated otherwise by the prefix that indicates the number of members in the cyclic structure, "heterocyclyl", "heterocyclic" or "heterocycle" is a 3- to 8-member aromatic, saturated, or partially saturated single or fused ring system that comprises carbon atoms wherein the heteroatoms are selected from N, O, and S. Examples of heterocyclyls include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imidazolidinyl, imidazolinyl, pyrazolidinyl, pyrazolinyl, piperidyl, piperazinyl, indolinyl, and morpholinyl. For example, preferred heterocyclyls or heterocyclic radicals include morpholinyl, piperazinyl, pyrrolidinyl, pyridyl, cyclohexylimino, cycloheptylimino, and more preferably, piperidyl.

Substitution positions are referred to in conventional terms. For example, piperidine and piperazine group substitution positions are numberd as follows:

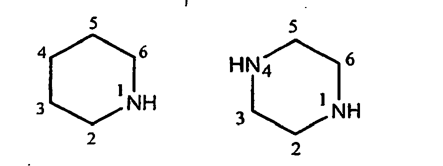

"Carbocycle" is a cycloalkyl or a partially saturated cycloalkyl that is not benzo

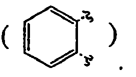

"Aryl" includes phenyl, naphthyl, biphenylyl, tetrahydronaphthyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on. Examples illustrating heteroaryl are thienyl, furanyl, pyrrolyl, imidazolyl, oxazolyl, thiazolyl, benzothienyl, benzofuranyl, benzimidazolyl, benzoxazolyl, benzothiazolyl.

"Halo" includes fluoro, chloro, bromo, and iodo, and is preferably fluoro or chloro.

The term "carbonyl" refers to a >C=O moiety, such that when this term is characterized as being part of a chain or cyclic structure, the carbon member in the carbonyl group is taken as being one of the carbon members of such chain or cyclic structure.

As in standard chemical nomenclature, the group phenyl is herein referred to as "phenyl" or as "Ph".

It is understood that substitutions and combinations of substitutions recited herein, whether stated explicitly or not, refer to substitutions that are consistent with the valency of the member being substituted. Terms such as "valence allowed site", "valence allowed member" and morphological variations thereof are used herein in this sense. For example, "valence allowed" when applied to a carbon member refers to the tetravalency of C; it refers to the trivalency of N when applied to a nitrogen member; and it refers to the four bonds of a nitrogen member that is conventionally characterized with a positive electric charge. Valence allowed options are part of the ordinary skill in the art.

"Patient" or "subject" includes mammals such as human beings and animals (e.g., dogs, cats, horses, rats, rabbits, mice, non-human primates) in need of observation, experiment, treatment or prevention in connection with the relevant disease or condition. Preferably, the patient is a human being.

"Composition" includes a product comprising the specified ingredients in the specified amounts, including in the effective amounts, as well as any product that results directly or indirectly from combinations of the specified ingredients in the specified amounts.

"Therapeutically effective amount" or "effective amount" and grammatically related terms mean that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

**Table of Acronyms**

| | |
|---|---|
| Term | Acronym |
| Tetrahydrofuran | THF |
| N,N-Dimethylformamide | DMF |
| N,N-Dimethylacetamide | DMA |
| Dimethyl sulfoxide | DMSO |
| tert-Butylcarbamoyl | BOC |
| Bovine serum albumin | BSA |
| High-pressure liquid chromatography | HPLC |
| Thin layer chromatography | TLC |

Compounds of formula (I), (II), or (III) comprise compounds that satisfy any one of the combinations of definitions given herein and equivalents thereof.

It is understood that some compounds refered to herein are chiral and/or have geometric isomeric centers, for example E- and Z- isomers. The present invention encompasses all such optical isomers, including diasteroisomers and racemic mixtures, and geometric isomers that possess the activity that characterizes the compounds of this invention. In addition, certain compounds referred to herein can exist in solvated as well as unsolvated forms. It is understood that this invention encompasses all such solvated and unsolvated forms that possess the activity that characterizes the compounds of this invention. Compounds according to the present invention that have been modified to be detectable by some analytic technique are also within the scope of this invention. An example of such compounds is an isotopically labeled compound, such as an ¹⁸F isotopically labeled compound that may be used as a probe in detection and/or imaging techniques, such as positron emission tomography (PET) and single-photon emission computed tomography (SPECT). Another example of such compounds is an isotopically labeled compound, such as a deuterium and/or tritium labeled compound that may be used in reaction kinetic studies.

It is understood that substitutions and combinations of substitutions recited herein, whether stated explicitly or not, refer to substitutions that are consistent with the valency of the member being substituted. For example, a substitution applied to a carbon member refers to the tetravalency of C; it refers to the trivalency of N when applied to a nitrogen member; and it refers to the four bonds of a nitrogen member that is conventionally characterized with a positive electric charge. Valence allowed options are part of the ordinary skill in the art.

The "pharmaceutically acceptable salts, amides or and esters thereof" refer to those salts, amides and ester forms of the compounds of the present invention that would be apparent to the pharmaceutical chemist, *i*.*e*., those that are non-toxic and that would favorably affect the pharmacological properties of said compounds of the present invention. Those compounds having favorable pharmacological properties would be apparent to the pharmaceutical chemist, i.e., those that are non-toxic and that possess such pharmacological properties to provide sufficient palatability, absorption, distribution, metabolism and excretion. Other factors, more practical in nature, that are also important in the selection are cost of raw materials, ease of crystallization, yield, stability, hygroscopicity, and flowability of the resulting bulk drug.

Representative acids and bases that may be used in the preparation of pharmaceutically acceptable salts include the following:
acids including acetic acid, 2,2-dichlorolactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, phosphoric acid, L-pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

See, for example, S.M. Berge, et al., "Pharmaceutical Salts", J. Pharm. Sci. 1977, 66:1-19, which is incorporated herein by reference. Examples of suitable esters include C₁₋₇alkyl, C₅₋₇cycloalkyl, phenyl, substituted phenyl, and phenylC₁₋₆alkyl- esters. Preferred esters include methyl esters.

The present invention includes within its scope prodrugs of the compounds of this invention. In general, such prodrugs will be functional derivatives of the compounds that are readily convertible *in vivo* into the required compound. Thus, in the methods of treatment of the present invention, the term "administering" shall encompass the treatment of the various disorders described with the compound specifically disclosed or with a compound that may not be specifically disclosed, but that converts to the specified compound *in vivo* after administration to the patient. Conventional procedures for the selection and preparation of suitable prodrug derivatives are described, for example, in "Design of Prodrugs", ed. H. Bundgaard, Elsevier, 1985.

Embodiments of this invention, where X is O, are made according to the synthetic methods outlined in Schemes A-D and F-L, have demonstrated LTA4H inhibitory activity, and are selected from the group consisting of:

| Example | Compound |
|---|---|
| 11 | 2-[4-(2-Piperidin-1-yl-ethoxy)-phenoxy]-benzooxazole; |
| 13 | (1-{2-[4-(Benzooxazol-2-yloxy)-phenoxy]ethyl}-piperidin-4-yl)-methanol; |
| 14 | 1-{2-[4-(Benzooxazol-2-yloxy)-phenoxy]-ethyl}-piperidin-4-ol; |
| 16 | {2-[4-(Benzooxazol-2-yloxy)-phenoxy]-ethyl}-dibutyl-amine; |
| 21 | (1-{2-[4-(Benzooxazol-2-yloxy)-phenoxy]-ethyl}-piperidin-2-yl)-methanol; |
| 27 | 1-{3-[4-(Benzooxazol-2-yloxy)-phenoxy]-propyl}-4-phenyl-piperidin-4-ol; |
| 28 | 1-{3-[4-(Benzooxazol-2-yloxy)-phenoxy]-propyl}-4-benzyl-piperidin-4-ol; |
| 29 | 2-[4-(2-Piperidin-1-yl-ethyl)-phenoxy]-benzooxazole; |
| 35 | {3-[4-(Benzooxazol-2-yloxy)-phenyl]-propyl}-cyclohexyl-ethyl-amine; |
| 36 | 1-{3-[4-(Benzooxazol-2-yloxy)-phenyl]-propyl}-piperidin-4-ol; |
| 40 | 1-{3-[4-(Benzooxazol-2-yloxy)-phenoxy]-2-hydroxy-propyl}-4-phenyl-piperidin-4-ol; |
| 41 | 1-[2-(4-Benzooxazol-2-ylmethyl-phenoxy)-ethyl]-piperidine-4-carboxylic acid ethyl ester; |
| 44 | 2-[4-(2-Pyrrolidin-1-yl-ethoxy)-phenoxy]-benzooxazole; |
| 45 | {3-[4-(Benzooxazol-2-yloxy)-phenoxy]-propyl}-dimethyl-amine; |
| 46 | {2-[4-(Benzooxazol-2-yloxy)-phenoxy]-ethyl}-dimethyl-amine; |
| 47 | 2-[4-(2-Azepan-1-yl-ethoxy)-phenoxy]-benzooxazole; |
| 53 | 1-{2-[4-(Benzooxazol-2-yloxy)-phenoxy]-ethyl}-4-phenyl-piperidin-4-ol; |
| 54 | {2-[4-(Benzooxazol-2-yloxy)-phenoxy]-ethyl}-cyclohexyl-ethyl-amine; |
| 55 | 2-{4-[2-(2-Ethyl-piperidin-1-yl)-ethoxy]-phenoxy}-benzooxazole; |
| 56 | 1-{2-[4-(Benzooxazol-2-yloxy)-phenoxy]-ethyl}-4-phenyl-piperidine-4-carbonitrile; |
| 57 | 1-(1-{2-[4-(Benzooxazol-2-yloxy)-phenoxy]-ethyl}-4-phenyl-piperidin-4-yl)-ethanone; |
| 58 | 2-{4-[2-(4-Methyl-piperidin-1-yl)-ethoxy]-phenoxy}-benzooxazole; |
| 59 | 1-{2-[4-(Benzooxazol-2-yloxy)-phenoxy]-ethyl}-4-(4-chloro-phenyl)-piperidin-4-ol; |
| 60 | 1-{2-[4-(Benzooxazol-2-yloxy)-phenoxy]-ethyl}-4-(4-bromo-phenyl)-piperidin-4-ol; |
| 61 | 1-{2-[4-(Benzooxazol-2-yloxy)-phenoxy]-ethyl}-4-(4-chloro-3-trifluoromethyl-phenyl)-piperidin-4-ol; |
| 62 | 1-{2-[4-(Benzooxazol-2-yloxy)-phenoxy]-ethyl}-4-benzyl-piperidin-4-ol; |
| 63 | {2-[4-(Benzooxazol-2-yloxy)-phenoxy]-ethyl}-cyclohexyl-methyl-amine; |
| 64 | {2-[4-(Benzooxazol-2-yloxy)-phenoxy]-ethyl}-cyclopropylmethyl-propyl-amine; |
| 65 | {2-[4-(Benzooxazol-2-yloxy)-phenoxy]-ethyl}-butyl-ethyl-amine; |
| 66 | 2-({2-[4-(Benzooxazol-2-yloxy)-phenoxy]-ethyl}-benzyl-amino)-ethanol; |
| 67 | 2-{4-[2-(4-Benzyl-piperidin-1-yl)-ethoxy]-phenoxy}-benzooxazole; |
| 68 | (1-{2-[4-(Benzooxazol-2-yloxy)-phenoxy]-ethyl}-piperidin-3-yl)-methanol; |
| 69 | 2-({2-[4-(Benzooxazol-2-yloxy)-phenoxy]-ethyl}-propyl-amino)-ethanol; |
| 77 | 2-[4-(2-Azetidin-1-yl-ethoxy)-phenoxy]-benzooxazole; |
| 78 | *N*-(1-{2-[4-(Benzooxazol-2-yloxy)-phenoxy]-ethyl}-piperidin-4-yl)-2-phenyl-acetamide; |
| 79 | 1-{2-[4-(Benzooxazol-2-yloxy)-phenoxy]-ethyl}-piperidine-3-carboxylic acid ethyl ester; |
| 86 | 2-{4-[3-(4-Phenyl-piperidin-1-yl)-propoxy]-phenoxy}-benzooxazole; |
| 88 | 1-{2-[4-(Benzooxazol-2-yloxy)-phenyl]-ethyl}-4-phenyl-piperidin-4-ol; |
| 89 | {2-[4-(Benzooxazol-2-yloxy)-phenyl]-ethyl}-cyclohexyl-ethyl-amine; |
| 90 | 2-[4-(2-Pyrrolidin-1-yl-ethyl)-phenoxy]-benzooxazole; |
| 91 | 2-[4-(2-Azepan-1-yl-ethyl)-phenoxy]-benzooxazole; |
| 92 | {2-[4-(Benzooxazol-2-yloxy)-phenyl]-ethyl}-cyclopropylmethyl-propyl-amine; |
| 93 | {2-[4-(Benzooxazol-2-yloxy)-phenyl]-ethyl}-dibutyl-amine; |
| 94 | 1-{2-[4-(Benzooxazol-2-yloxy)-phenyl]-ethyl}-piperidin-4-ol; |
| 96 | 1-{2-[4-(Benzooxazol-2-yloxy)-phenyl]-ethyl}-piperidine-4-carboxylic acid methyl ester; |
| 116 | 1-{3-[4-(Benzooxazol-2-yloxy)-phenyl]-propyl}-4-phenyl-piperidin-4-ol; |
| 120 | 2-[4-(3-Piperidin-1-yl-propyl)-phenoxy]-benzooxazole; |
| 121 | {3-[4-(Benzooxazol-2-yloxy)-phenyl]-propyl}-dibutyl-amine; |
| 122 | {3-[4-(Benzooxazol-2-yloxy)-phenyl]-propyl}-cyclopropylmethyl-propyl-amine; |
| 135 | 1-[4-(Benzooxazol-2-yloxy)-phenoxy]-3-pyrrolidin-1-yl-propan-2-ol; |
| 136 | 1-[2-(4-Benzooxazol-2-ylmethyl-phenoxy)-ethyl]-4-phenyl-piperidin-4-ol; |
| 137 | 1-[2-(4-Benzooxazol-2-ylmethyl-phenoxy)-ethyl]-piperidine-4-carboxylic acid amide; |
| 271 | 2-(4-Piperidin-1-ylmethyl-phenoxy)-benzooxazole; |
| 481 | 2-[4-(2-Morpholin-4-yl-ethoxy)-phenoxy]-benzooxazole; and |
| 484 | {2-[4-(Benzooxazol-2-yloxy)-phenoxy]-ethyl}-diethyl-amine. |

Other embodiments of this invention, where X is S, are made according to the synthetic methods outlined in Schemes A-G, and I-L, have demonstrated LTA4H inhibitory activity, and are selected from the group consisting of:

| Example | Compound |
|---|---|
| 12 | {2-[4-(6-Chloro-benzothiazol-2-yloxy)-phenoxy]-ethyl}-diethyl-amine; |
| 15 | 1-{2-[4-(Benzothiazol-2-yloxy)-phenoxy]-ethyl}-piperidin-4-ol; |
| 17 | 1-{2-[4-(Benzothiaiol-2-yloxy)-phenoxy]-ethyl}-piperidine-4-carboxylic acid ethyl ester; |
| 18 | 1-{2-[4-(Benzothiazol-2-yloxy)-phenoxy]-ethyl}-piperidine-4-carboxylic acid; |
| 19 | (1-{2-[4-(Benzothiazol-2-yloxy)-phenoxy]-ethyl}-piperidin-4-yl)-pyrrolidin-1-yl-methanone; |
| 20 | 3-[(1-{2-[4-(Benzothiazol-2-yloxy)-phenoxy]-ethyl}-piperidine-4-carbonyl)-amino]-propionic acid ethyl ester; |
| 22 | 1-{2-[4-(Benzothiazol-2-yloxy)-phenoxy]-ethyl}-piperidine-4-carboxylic acid amide; |
| 23 | 1-(1-{2-[4-(Benzothiazol-2-yloxy)-phenoxy]-ethyl}-piperidin-4-yl)-pyrrolidin-2-one; |
| 24 | 1'-{2-[4-(Benzothiazol-2-yloxy)-phenoxy]-ethyl}-[1,4']bipiperidinyl-2-one; |
| 25 | 8-{2-[4-(Benzothiazol-2-yloxy)-phenoxy]-ethyl}-2,8-diaza-spiro[4.5]decan-1-one; |
| 26 | 2-[4-(3-Pyrrolidin-1-yl-propoxy)-phenoxy]-benzothiazole; |
| 30 | {2-[4-(Benzothiazol-2-yloxy)-phenyl]-ethyl}-cyclohexyl-ethyl-amine; |
| 31 | 1-{2-[4-(Benzothiazol-2-yloxy)-phenyl]-ethyl}-piperidine-3-carboxylic acid amide; |
| 32 | 1-(1-{2-[4-(Benzothiazol-2-yloxy)-phenyl]-ethyl}-piperidin-4-yl)-3-methyl-1,3-dihydro-benzoimidazol-2-one; |
| 33 | 1-{2-[4-(Benzothiazol-2-yloxy)-phenyl]-ethyl}-piperidine-4-carboxylic acid methyl ester; |
| 34 | (1-{2-[4-(Benzothiazol-2-yloxy)-phenyl]-ethyl}-piperidin-4-yl)-(4-methyl-piperazin-1-yl)-methanone; |
| 42 | 1-[2-(4-Benzothiazol-2-ylmethyl-phenoxy)-ethyl]-piperidine-4-carboxylic acid methyl ester; |
| 43 | 3-({2-[4-(Benzothiazol-2-yloxy)-phenoxy]-ethyl}-cyclopropyl-amino)-propionic acid; |
| 48 | {2-[4-(Benzothiazol-2-yloxy)-phenoxy]-ethyl}-dimethyl-amine; |
| 49 | 2-[4-(2-Pyrrolidin-1-yl-ethoxy)-phenoxy]-benzothiazole; |
| 50 | {3-[4-(Benzothiazol-2-yloxy)-phenoxy]-propyl}-dimethyl-amine; |
| 51 | 2-[4-(2-Azepan-1-yl-ethoxy)-phenoxy]-benzothiazole; |
| 52 | 2-[4-(2-Azepan-1-yl-ethoxy)-phenoxy]-6-methoxy-benzothiazole; |
| 70 | 1-{2-[4-(Benzothiazol-2-yloxy)-phenoxy]-ethyl}-4-phenyl-piperidin-4-ol; |
| 71 | {2-[4-(Benzothiazol-2-yloxy)-phenoxy]-ethyl}-cyclohexyl-ethyl-amine; |
| 72 | 1-{2-[4-(Benzothiazol-2-yloxy)-phenoxy]-ethyl}-4-(4-chloro-phenyl)-piperidin-4-ol; |
| 73 | {2-[4-(Benzothiazol-2-yloxy)-phenoxy]-ethyl}-dibutyl-amine; |
| 74 | 1-{2-[4-(Benzothiazol-2-yloxy)-phenoxy]-ethyl}-4-(4-bromo-phenyl)-piperidin-4-ol; |
| 75 | 1-{2-[4-(Benzothiazol-2-yloxy)-phenoxy]-ethyl}-4-(4-chloro-3-trifluoromethyl-phenyl)-piperidin-4-ol; |
| 76 | 1-{2-[4-(Benzothiazol-2-yloxy)-phenoxy]-ethyl}-4-benzyl-piperidin-4-ol; |
| 80 | 1'-{2-[4-(Benzothiazol-2-yloxy)-phenoxy]-ethyl}-[1,4']bipiperidine; |
| 81 | (1-{2-[4-(Benzothiazol-2-yloxy)-phenoxy]-ethyl}-piperidin-4-yl)-methanol; |
| 82 | *N*-(1-{2-[4-(Benzothiazol-2-yloxy)-phenoxy]-ethyl}-piperidin-4-yl)-2-phenyl-acetamide; |
| 83 | 1'-{2-[4-(Benzothiazol-2-yloxy)-phenoxy]-ethyl}-[1,4']bipiperidinyl-2-one; |
| 84 | 2-(4-{2-[4-(2-Morpholin-4-yl-ethyl)-piperazin-1-yl]-ethoxy}-phenoxy)-benzothiazole; |
| 85 | 2-(4-{2-[4-(2-Morpholin-4-yl-ethyl)-piperazin-1-yl]-ethyl}-phenoxy)-benzothiazole; |
| 87 | 1-{3-[4-(Benzothiazol-2-yloxy)-phenoxy]-propyl}-4-phenyl-piperidin-4-ol; |
| 95 | 1-{2-[4-(Benzothiazol-2-yloxy)-phenyl]-ethyl}-4-phenyl-piperidin-4-ol; |
| 97 | 2-[4-(2-Pyrrolidin-1-yl-ethyl)-phenoxy]-benzothiazole; |
| 98 | 2-[4-(2-Azepan-1-yl-ethyl)-phenoxy]-benzothiazole; |
| 99 | {2-[4-(Benzothiazol-2-yloxy)-phenyl]-ethyl}-cyclopropylmethyl-propyl-amine; |
| 100 | {2-[4-(Benzothiazol-2-yloxy)-phenyl]-ethyl}-dibutyl-amine; |
| 101 | 2-[4-(2-Piperidin-1-yl-ethyl)-phenoxy]-benzothiazole; |
| 102 | 1-{2-[4-(Benzothiazol-2-yloxy)-phenyl]-ethyl}-piperidin-4-ol; |
| 103 | 1-{2-[4-(Benzothiazol-2-yloxy)-phenyl]-ethyl}-piperidine-4-carboxylic acid methyl ester; |
| 104 | 1-{2-[4-(Benzothiazol-2-yloxy)-phenyl]-ethyl}-piperidine-4-carboxylic acid amide; |
| 105 | 1-{2-[4-(Benzothiazol-2-yloxy)-phenyl]-ethyl}-piperidine-3-carboxylic acid ethyl ester; |
| 106 | 1-{2-[4-(Benzothiazol-2-yloxy)-phenyl]-ethyl}-4-phenyl-piperidine-4-carboxylic acid ethyl ester; |
| 107 | (1-{2-[4-(Benzothiazol-2-yloxy)-phenyl]-ethyl}-piperidin-4-yl)-acetic acid ethyl ester; |
| 108 | 1-(1-{2-[4-(Benzothiazol-2-yloxy)-phenyl]-ethyl}-piperidin-4-yl)-1,3-dihydro-indol-2-one; |
| 109 | 1-(1-{2-[4-(Benzothiazol-2-yloxy)-phenyl]-ethyl}-piperidin-4-yl)-pyrrolidin-2-one; |
| 110 | *N*-(1-{2-[4-(Benzothiazol-2-yloxy)-phenyl]-ethyl}-piperidin-4-yl)-2-phenyl-acetamide; |
| 111 | 8-{2-[4-(Benzothiazol-2-yloxy)-phenyl]-ethyl}-2,8-diaza-spiro[4.5]decan-1-one; |
| 112 | 1-{2-[4-(Benzothiazol-2-yloxy)-phenyl]-ethyl}-piperidin-3-ol; |
| 113 | 1-{2-[4-(Benzothiazol-2-yloxy)-phenyl]-ethyl}-piperidine-4-carboxylic acid ethyl ester; |
| 114 | 1'-{2-[4-(Benzothiazol-2-yloxy)-phenyl]-ethyl}-[1,4']bipiperidine; |
| 115 | 2-{4-[2-(4-Methyl-piperazin-1-yl)-ethyl]-phenoxy}-benzothiazole; |
| 143 | 2-(4-{2-[4-(1-Benzyl-1*H*-tetrazol-5-yl)-piperidin-1-yl]-ethoxy}-phenoxy)-benzothiazole; |
| 144 | 4-(1-{2-[4-(Benzothiazol-2-yloxy)-phenyl]-ethyl}-piperidine-4-carbonyl)-piperazine-1-carboxylic acid *tert*-butyl ester; |
| 146 | 2-(4-{2-[4-(2-Morpholin-4-yl-ethyl)-piperazin-1-yl]-ethyl}-phenoxy)-benzothiazole; |
| 147 | 1-[2-(4-Benzothiazol-2-ylmethyl-phenoxy)-ethyl]-piperidine-4-carboxylic acid amide; |
| 148 | 1-{1-[2-(4-Benzothiazol-2-ylmethyl-phenoxy)-ethyl]-piperidin-4-yl}-pyrrolidin-2-one; |
| 149 | 1-[4-(1-{2-[4-(Benzothiazol-2-yloxy)-phenyl]-ethyl}-piperidine-4-carbonyl)-piperazin-1-yl]-ethanone; |
| 150 | 1-{2-[4-(Benzothiazol-2-yloxy)-phenyl]-ethyl}-piperidine-4-carboxylic acid; |
| 151 | 1-(1-{2-[4-(Benzothiazol-2-yloxy)-phenoxy]-ethyl}-piperidin-4-yl)-pyrrolidine-2-thione; |
| 152 | 2-(4-{2-[4-(Benzothiazol-2-yloxy)-phenoxy]-ethyl}-piperazin-1-yl)-ethanol; |
| 153 | 2-(4-{2-[4-(Benzothiazol-2-yloxy)-phenoxy]-ethyl}-piperazin-1-yl)-1-pyrrolidin-1-yl-ethanone; |
| 154 | 2-(4-{2-[4-(Benzothiazol-2-yloxy)-phenoxy]-ethyl}-piperazin-1-yl)-1-morpholin-4-yl-ethanone; |
| 155 | 1-{2-[4-(Benzothiazol-2-yloxy)-phenyl]-ethyl}-piperidine-3-carboxylic acid; |
| 156 | 1-{2-[4-(Benzothiazol-2-yloxy)-phenyl]-ethyl}-piperidine-2-carboxylic acid; |
| 157 | (1-{2-[4-(Benzothiazol-2-yloxy)-phenyl]-ethyl}-piperidin-3-yl)-acetic acid; |
| 158 | (1-{2-[4-(Benzothiazol-2-yloxy)-phenoxy]-ethyl}-piperidin-4-yl)-acetic acid ethyl ester; |
| 159 | (1-{2-[4-(Benzothiazol-2-yloxy)-phenoxy]-ethyl}-piperidin-4-yl)-carbamic acid tert-butyl ester; |
| 160 | (1-{2-[4-(Benzothiazol-2-yloxy)-phenoxy]-ethyl}-piperidin-4-yl)-acetic acid; |
| 161 | (1-{2-[4-(Benzothiazol-2-yloxy)-phenyl]-ethyl}-piperidin-3-yl)-methanol; |
| 162 | ({2-[4-(Benzothiazol-2-yloxy)-phenyl]-ethyl}-cyclohexyl-amino)-acetic acid methyl ester; |
| 163 | (4-{2-[4-(Benzothiazol-2-yloxy)-phenoxy]-ethyl}-piperazin-1-yl)-acetic acid; |
| 164 | 1-(1-{2-[4-(Benzothiazol-2-yloxy)-phenoxy]-ethyl}-piperidin-4-yl)-5-oxo-pyrrolidine-2-carboxylic acid ethyl ester; |
| 166 | 1-(1-{2-[4-(Benzothiazol-2-yloxy)-phenoxy]-ethyl}-piperidin-4-yl)-5-oxo-pyrrolidine-2-carboxylic acid; |
| 167 | 4-(4-{2-[4-(Benzothiazol-2-yloxy)-phenoxy]-ethyl}-piperazin-1-yl)-phenol; |
| 168 | *N*-(1-{2-[4-(Benzothiazol-2-yloxy)-phenoxy]-ethyl}-piperidin-4-yl)-4-chloro-*N*-cyclopropyl-benzene sulfonamide; |
| 170 | 3-({2-[4-(Benzothiazol-2-yloxy)-phenoxy]-ethyl}-cyclopropylmethyl-amino)-propionic acid; |
| 171 | 3-({2-[4-(Benzothiazol-2-yloxy)-phenoxy]-ethyl}-isopropyl-amino)-propionic acid; |
| 172 | 1-{2-[4-(Benzothiazol-2-yloxy)-phenoxy]-ethyl}-piperidin-4-ylamine; |
| 173 | 3-[{2-[4-(Benzothiazol-2-yloxy)-phenoxy]-ethyl}-(1-methyl-piperidin-4-yl)-amino]-propionic acid; |
| 174 | 3-({2-[4-(Benzothiazol-2-yloxy)-phenoxy]-ethyl}-benzyl-amino)-propionic acid; |
| 175 | 3-((1-Acetyl-piperidin-4-yl)-{2-[4-(benzothiazol-2-yloxy)-phenoxy]-ethyl}-amino)-propionic acid; |
| 176 | 4-(1-{2-[4-(Benzothiazol-2-yloxy)-phenoxy]-ethyl}-1H-tetrazol-5-yl)-piperidine-1-carboxylic acid tert-butyl ester; |
| 177 | 3-({2-[4-(Benzothiazol-2-yloxy)-phenyl]-ethyl}-cyclopropyl-amino)-propionic acid; |
| 178 | 3-((1-Acetyl-piperidin-4-yl)-{2-[4-(benzothiazol-2-yloxy)-phenyl]-ethyl}-amino)-propionic acid; |
| 179 | 3-[{2-[4-(Benzothiazol-2-yloxy)-phenyl]-ethyl}-(1-methyl-piperidin-4-yl)-amino]-propionic acid; |
| 180 | 3-({2-[4-(Benzothiazol-2-yloxy)-phenyl]-ethyl}-cyclopropylmethyl-amino)-propionic acid; |
| 181 | 3-({2-[4-(Benzothiazol-2-yloxy)-phenyl]-ethyl}-isopropyl-amino)-propionic acid; |
| 182 | 2-(4-{2-[4-(Benzothiazol-2-yloxy)-phenyl]-ethyl}-piperazin-1-yl)-1-pyrrolidin-1-yl-ethanone; |
| 183 | (*R*)-1-{2-[4-(Benzothiazol-2-yloxy)-phenyl]-ethyl}-piperidine-3-carboxylic acid; |
| 184 | 1-(1-{2-[4-(Benzothiazol-2-yloxy)-phenyl]-ethyl}-piperidin-4-yl)-1,3-dihydro-benzoimidazol-2-one; |
| 185 | 2-(4-{2-[4-(6-Methyl-pyridin-2-yl)-piperazin-1-yl]-ethyl}-phenoxy)-benzothiazole; |
| 186 | 2-{4-[2-(4-Ethanesulfonyl-piperazin-1-yl)-ethyl]-phenoxy}-benzothiazole; |
| 187 | 2-(4-{2-[4-(Benzothiazol-2-yloxy)-phenyl]-ethyl}-piperazin-1-yl)-1-morpholin-4-yl-ethanone; |
| 188 | 3-({2-[4-(Benzothiazol-2-yloxy)-phenyl]-ethyl}-methyl-amino)-propionic acid; |
| 189 | 3-({2-[4-(Benzothiazol-2-yloxy)-phenyl]-ethyl}-cyclopentyl-amino)-propionic acid; |
| 190 | 3-({2-[4-(Benzothiazol-2-yloxy)-phenyl]-ethyl}-cyclobutyl-amino)-propionic acid; |
| 192 | 3-({2-[4-(Benzothiazol-2-yloxy)-phenyl]-ethyl}-benzyl-amino)-propionic acid; |
| 193 | (1-{2-[4-(Benzothiazol-2-yloxy)-phenoxy]-ethyl}-piperidin-4-yl)-(4- hydroxymethyl-piperidin-1-yl)-methanone; |
| 194 | {2-[4-(Benzothiazol-2-yloxy)-phenyl]-ethyl}-cyclopropyl-amine; |
| 195 | (1-{2-[4-(Benzothiazol-2-yloxy)-phenoxy]-ethyl}-piperidin-4-yl)-[4-(2-hydroxy-ethyl)-piperazin-1-yl]-methanone; |
| 196 | (1-{2-[4-(Benzothiazol-2-yloxy)-phenoxy]-ethyl}-piperidin-4-yl)-[4-(2-hydroxy-ethyl)-piperidin-1-yl]-methanone; |
| 197 | 2-({2-[4-(Benzothiazol-2-yloxy)-phenyl]-ethyl}-cyclopropyl-amino)-ethanol; |
| 198 | 3-({2-[4-(Benzothiazol-2-yloxy)-phenyl]-ethyl}-cyclopropyl-amino)-propan-1-ol; |
| 199 | 4-({2-[4-(Benzothiazol-2-yloxy)-phenyl]-ethyl}-cyclopropyl-amino)-butyric acid; |
| 200 | 3-[(1-{2-[4-(Benzothiazol-2-yloxy)-phenoxy]-ethyl}-piperidine-4-carbonyl)-amino]-propionic acid; |
| 201 | 4-({2-[4-(Benzothiazol-2-yloxy)-phenyl]-ethyl}-cyclopropyl-amino)-butyronitrile; |
| 202 | 3-(1-{2-[4-(Benzothiazol-2-yloxy)-phenyl]-ethyl}-piperidin-4-yl)-propionic acid; |
| 203 | [(1-{2-[4-(Benzothiazol-2-yloxy)-phenoxy]-ethyl}-piperidine-4-carbonyl)-methyl-amino]-acetic acid; |
| 204 | 3-(4-{2-[4-(Benzothiazol-2-yloxy)-phenoxy]-ethyl}-piperazin-1-yl)-phenol; |
| 205 | 2-(4-{2-[4-(4-Methoxy-phenyl)-piperazin-1-yl]-ethoxy}-phenoxy)-benzothiazole; |
| 206 | 2-{4-[2-(5-Piperidin-4-yl-tetrazol-1-yl)-ethoxy]-phenoxy}-benzothiazole; |
| 207 | (*S*)-1-(1-{2-[4-(Benzothiazol-2-yloxy)-phenoxy]-ethyl}-piperidin-4-yl)-4-hydroxy-pyrrolidin-2-one; |
| 208 | {2-[4-(Benzothiazol-2-yloxy)-phenyl]-ethyl}-cyclopropylmethyl-amine; |
| 209 | 2-[({2-[4-(Benzothiazol-2-yloxy)-phenyl]-ethyl}-cyclopropyl-amino)-methyl]-cyclopropanecarboxylic acid ethyl ester; |
| 210 | 4-(4-{2-[4-(Benzothiazol-2-yloxy)-phenoxy]-ethyl}-piperazine-1-carbonyl)-benzoic acid ethyl ester; |
| 211 | 2-[({2-[4-(Benzothiazol-2-yloxy)-phenyl]-ethyl}-cyclopropyl-amino)-methyl]-cyclopropanecarboxylic acid; |
| 212 | 1-({2-[4-(Benzothiazol-2-yloxy)-phenyl]-ethyl}-cyclopropyl-amino)-propan-2-ol; |
| 213 | 3-({2-[4-(Benzothiazol-2-yloxy)-phenyl]-ethyl}-cyclopropyl-amino)-1,1,1-trifluoro-propan-2-ol; |
| 214 | 3-({2-[4-(Benzothiazol-2-yloxy)-phenyl]-ethyl}-cyclopropyl-amino)-propionamide; |
| 215 | 3-({2-[4-(Benzothiazol-2-yloxy)-phenyl]-ethyl}-cyclopropyl-amino)-propane-1,2-diol; |
| 216 | 2-{4-[2-(5-Phenyl-tetrazol-2-yl)-ethoxy]-phenoxy}-benzothiazole; |
| 217 | 2-{4-[2-(5-Phenyl-tetrazol-1-yl)-ethoxy]-phenoxy}-benzothiazole; |
| 218 | *N*-{2-[4-(Benzothiazol-2-yloxy)-phenyl]-ethyl}-*N*-cyclopropyl-2-(2H-tetrazol-5-yl)-acetamide; |
| 219 | (*S*)-3-({2-[4-(Benzothiazol-2-yloxy)-phenyl]-ethyl}-cyclopropyl-amino)-2-methyl-propan-1-ol; |
| 220 | (*R*)-3-({2-[4-(Benzothiazol-2-yloxy)-phenyl]-ethyl}-cyclopropyl-amino)-2-methyl-propan-1-ol; |
| 221 | 2-{4-[2-(5-Methylsulfanyl-tetrazol-2-yl)-ethoxy]-phenoxy}-benzothiazole; |
| 222 | 2-{4-[2-(5-Methylsulfanyl-tetrazol-1-yl)-ethoxy]-phenoxy}-benzothiazole; |
| 223 | 2-[4-(2-Tetrazol-2-yl-ethoxy)-phenoxy]-benzothiazole; |
| 224 | 2-[4-(2-Tetrazol-1-yl-ethoxy)-phenoxy]-benzothiazole; |
| 225 | (1*R*,2*R*)-2-{2-[4-(Benzothiazol-2-yloxy)-phenyl]-ethylamino}-cyclohexanecarboxylic acid; |
| 226 | (1*S*,2*R*)-2-{2-[4-(Benzothiazol-2-yloxy)-phenyl]-ethylamino}-cyclohexanecarboxylic acid; |
| 227 | (1*R*,2*R*)-2-{2-[4-(Benzothiazol-2-yloxy)-phenyl]-ethylamino}-cyclohexanol; |
| 228 | (1*S*,2*R*)-2-{2-[4-(Benzothiazol-2-yloxy)-phenyl]-ethylamino}-cyclohexanol; |
| 229 | 4-(1-{2-[4-(Benzothiazol-2-yloxy)-phenyl]-ethyl}-piperidin-4-yl)-butyric acid; |
| 250 | 1-[4-(Benzothiazol-2-yloxy)-benzyl]-piperidine-4-carboxylic acid; |
| 251 | 1-{1-[4-(Benzothiazol-2-yloxy)-benzyl]-piperidin-4-yl}-pyrrolidin-2-one; |
| 252 | 2-(2-Fluoro-4-piperidin-1-ylmethyl-phenoxy)-benzothiazole; |
| 253 | N-{1-[4-(Benzothiazol-2-yloxy)-benzyl]-piperidin-4-yl}-2-hydroxy-acetamide; |
| 254 | 1-(2-{[4-(Benzothiazol-2-yloxy)-benzyl]-cyclopropyl-amino}-ethyl)-4-hydroxy-pyrrolidin-2-one; |
| 255 | N-{1-[4-(Benzothiazol-2-yloxy)-benzyl]-piperidin-4-yl}-N-methyl-methanesulfonamide; |
| 256 | 2-{4-[4-(1H-Tetrazol-5-yl)-piperidin-1-ylmethyl]-phenoxy}-benzothiazole; |
| 257 | 1-{4-[4-(Benzothiazol-2-yloxy)-benzyl]-piperazin-1-yl}-2-hydroxy-ethanone; |
| 258 | N-{1-[4-(Benzothiazol-2-yloxy)-benzyl]-piperidin-4-ylmethyl}-methanesulfonamide; |
| 259 | 3-{1-[4-(Benzothiazol-2-yloxy)-benzyl]-piperidin-4-yl}-oxazolidin-2-one; |
| 260 | 4-{1-[4-(Benzothiazol-2-yloxy)-benzyl]-piperidin-4-yl}-morpholin-3-one; |
| 261 | (*R*) 1-(1-{2-[4-(Benzothiazol-2-yloxy)-phenoxy]-ethyl}-piperidin-4 yl)-4-hydroxy-pyrrolidin-2-one; |
| 262 | 2-(4-{2-[4-(1H-Tetrazol-5-yl)-piperidin-1-yl]-ethyl}-phenoxy)-benzothiazole; |
| 263 | (1-{2-[4-(Benzothiazol-2-yloxy)-phenoxy]-ethyl}-piperidin-2-yl)-methanol; |
| 264 | (1-{2-[4-(Benzothiazol-2-yloxy)-phenoxy]-ethyl}-1H-tetrazol-5-yl)-acetic acid ethyl ester; |
| 265 | (1-{2-[4-(Benzothiazol-2-yloxy)-phenoxy]-ethyl}-1H-tetrazol-5-yl)-acetic acid ethyl ester; |
| 266 | 2-{4-[2-(5-Piperidin-4-yl-tetrazol-2-yl)-ethoxy]-phenoxy}-benzothiazole hydrochloride; |
| 267 | 7-{2-[4-(Benzothiazol-2-yloxy)-phenoxy]-ethyl}-4-spiro-[3-phthalide]-piperidine; |
| 268 | 1-{3-[4-(Benzothiazol-2-yloxy)-phenyl]-propyl}-piperidine-4-carboxylic acid ethyl ester; |
| 269 | 2-[4-(Benzothiazol-2-yloxy)-phenyl]-ethylamine hydrochloride; |
| 270 | 2-(4-{2-[4-(1H-Tetrazol-5-yl)-piperidin-1-yl]-ethoxy}-phenoxy)-benzothiazole; |
| 271 | 2-(4-Piperidin-1-ylmethyl-phenoxy)-benzooxazole; |
| 272 | [4-(Benzothiazol-2-yloxy)-benzyl]-cyclohexyl-ethyl-amine; |
| 273 | [4-(Benzothiazol-2-yloxy)-benzyl]-cyclopropylmethyl-propyl-amine; |
| 274 | 1-[4-(Benzothiazol-2-yloxy)-benzyl]-piperidine-4-carboxylic acid amide; |
| 275 | 1'-[4-(Benzothiazol-2-yloxy)-benzyl]-[1,4']bipiperidinyl-2-one; |
| 276 | {4-[4-(Benzothiazol-2-yloxy)-benzyl]-piperazin-1-yl}-pyridin-3-yl-methanone; |
| 277 | {1-[4-(Benzothiazol-2-yloxy)-benzyl]-piperidin-4-ylmethyl}-carbamic acid tert-butyl ester; |
| 278 | {1-[4-(Benzothiazol-2-yloxy)-benzyl]-piperidin-4-ylmethyl}-carbamic acid methyl ester; |
| 279 | N-{C-[[4-(benzothiazol-2-yloxy)-benzyl]-piperidin-4-yl]-methylamino sulfonyl}-carbamic acid tert-butyl ester, |
| 280 | N-{1-[4-(Benzothiazol-2-yloxy)-benzyl]-piperidin-4-ylmethyl}-sulfamide hydrochloride, |
| 281 | N-{1-[4-(Benzothiazol-2-yloxy)-benzyl]-piperidin-4-ylmethyl}-acetamide; |
| 282 | {1-[4-(Benzothiazol-2-yloxy)-benzyl]-piperidin-4-yl}-acetic acid; |
| 283 | Acetic acid ({1-[4-(benzothiazol-2-yloxy)-benzyl]-piperidin-4-ylmethyl}-carbamoyl)-methyl ester; |
| 284 | [2-({1-[4-(Benzothiazol-2-yloxy)-benzyl]-piperidin-4-ylmethyl}-carbamoyl)-cyclobutyl]-carbamic acid tert-butyl ester; |
| 285 | 2-Amino-cyclobutanecarboxylic acid {1-[4-(benzothiazol-2-yloxy)-benzyl]-piperidin-4-ylmethyl}-amide dihydrochloride; |
| 286 | 2-(4-Pyrrolidin-1-ylmethyl-phenoxy)-benzothiazole; |
| 287 | 2-{[4-(Benzothiazol-2-yloxy)-benzyl]-ethyl-amino}-ethanol; |
| 288 | 2-{1-[4-(Benzothiazol-2-yloxy)-benzyl]-piperidin-2-yl}-ethanol; |
| 289 | 1-{4-[4-(Benzothiazol-2-yloxy)-benzyl]-piperazin-1-yl}-ethanone; |
| 290 | 8-[4-(Benzothiazol-2-yloxy)-benzyl]-2,8-diaza-spiro[4.5]decan-1-one; |
| 291 | Spiro[isobenzofuran-1(3H), 4'-piperidin]-3-one, 1'-[4-(Benzothiazol-2-yloxy)-benzyl] |
| 292 | (*R*)-1-[4-(Benzothiazol-2-yloxy)-benzyl]-pyrrolidin-3-ol; |
| 293 | 2-[4-(2-Methyl-piperidin-1-ylmethyl)-phenoxy]-benzothiazole; |
| 294 | [4-(Benzothiazol-2-yloxy)-benzyl]-diethyl-amine; |
| 295 | [4-(Benzothiazol-2-yloxy)-benzyl]-butyl-methyl-amine; |
| 296 | 2-{1-[4-(Benzothiazol-2-yloxy)-benzyl]-piperidin-4-yl}-ethanol; |
| 297 | 1-[4-(Benzothiazol-2-yloxy)-benzyl]-piperidin-4-ol; |
| 298 | {1-[4-(Benzothiazol-2-yloxy)-benzyl]-piperidin-2-yl}-methanol; |
| 299 | (*R*)-{1-[4-(Benzothiazol-2-yloxy)-benzyl]-pyrrolidin-2-yl}-methanol; |
| 300 | 2-(4-Azetidin-1-ylmethyl-phenoxy)-benzothiazole; |
| 301 | 1-[4-(Benzothiazol-2-yloxy)-benzyl]-[1,4]diazepan-5-one; |
| 302 | {1-[4-(Benzothiazol-2-yloxy)-benzyl]-piperidin-3-yl}-methanol; |
| 303 | 1-[4-(Benzothiazol-2-yloxy)-benzyl]-piperidine-3-carboxylic acid amide; |
| 304 | 9-[4-(Benzothiazol-2-yloxy)-benzyl]-3,9-diaza-spiro[5.5]undecane-3-carboxylic acid tert-butyl ester; |
| 305 | 2-{1-[4-(Benzothiazol-2-yloxy)-benzyl]-piperidin-3-yl}-ethanol; |
| 306 | cis-4-{2-[4-(Benzothiazol-2-yloxy)-phenyl]-ethylamino}-cyclohexanecarboxylic acid trifluoromethanesulfonate salt; |
| 307 | (4-{2-[4-(Benzothiazol-2-yloxy)-phenyl]-ethyl}-piperazin-1-yl)-(tetrahydro-furan-2-yl)-methanone; |
| 308 | Propane-2-sulfonic acid (1-{2-[4-(benzothiazol-2-yloxy)-phenoxy]-ethyl}-piperidine-4-carbonyl)-amide; |
| 309 | (4-{2-[4-(Benzothiazol-2-yloxy)-phenyl]-ethyl}-piperazin-1-yl)-oxo-acetic acid methyl ester; |
| 310 | N-(1-{2-[4-(Benzothiazol-2-yloxy)-phenyl]-ethyl}-piperidine-4-carbonyl)-benzenesulfonamide trifluoromethanesulfonate salt; |
| 311 | N-(1-{2-[4-(Benzothiazol-2-yloxy)-phenyl]-ethyl}-piperidine-4-carbonyl)-methanesulfonamide trifluoromethanesulfonate salt; |
| 312 | (4-{2-[4-(Benzothiazol-2-yloxy)-phenyl]-ethyl}-piperazin-1-yl)-oxo-acetic acid trifluoromethanesulfonate salt; |
| 313 | (4-{2-[4-(Benzothiazol-2-yloxy)-phenyl]-ethyl}-piperazin-1-yl)-morpholin-4-yl-methanone; |
| 314 | 1-(4-{2-[4-(Benzothiazol-2-yloxy)-phenyl]-ethyl}-piperazin-1-yl)-2-thiophen-2-yl-ethanone; |
| 315 | (4-{2-[4-(Benzothiazol-2-yloxy)-phenyl]-ethyl}-piperazin-1-yl)-pyridin-3-yl-methanone; |
| 316 | (4-{2-[4-(Benzothiazol-2-yloxy)-phenyl]-ethyl}-piperazin-1-yl)-cyclopropyl-methanone; |
| 317 | 1-(4-{2-[4-(Benzothiazol-2-yloxy)-phenyl]-ethyl}-piperazin-1-yl)-2-methoxy-ethanone; |
| 318 | 1-(4-{2-[4-(Benzothiazol-2-yloxy)-phenyl]-ethyl}-piperazin-1-yl)-2,2,2-trifluoro-ethanone; |
| 319 | 4-(4-{2-[4-(Benzothiazol-2-yloxy)-phenyl]-ethyl}-piperazine-1-carbonyl)-benzoic acid; |
| 320 | (4-{2-[4-(Benzothiazol-2-yloxy)-phenyl]-ethyl}-piperazin-1-yl)-pyridin-4-yl-methanone; |
| 321 | (4-{2-[4-(Benzothiazol-2-yloxy)-phenyl]-ethyl}-piperazin-1-yl)-(5-methyl-pyrazin-2-yl)-methanone; |
| 322 | (*R*)-(4-{2-[4-(Benzothiazol-2-yloxy)-phenyl]-ethyl}-piperazin-1-yl)-(tetrahydro-furan-2-yl)-methanone; |
| 323 | (*S*)-(4-{2-[4-(Benzothiazol-2-yloxy)-phenyl]-ethyl}-piperazin-1-yl)-(tetrahydro-furan-2-yl)-methanone; |
| 324 | (4-{2-[4-(Benzothiazol-2-yloxy)-phenyl]-ethyl}-piperazin-1-yl)-(tetrahydro-furan-3-yl)-methanone; |
| 325 | 1-(4-{2-[4-(Benzothiazol-2-yloxy)-phenyl]-ethyl}-piperazin-1-yl)-2-hydroxy-ethanone; |
| 326 | 2-[2-(4-{2-[4-(Benzothiazol-2-yloxy)-phenyl]-ethyl}-piperazin-1-yl)-2-oxo-ethyl]-cyclopentanone; |
| 327 | 3-(4-{2-[4-(Benzothiazol-2-yloxy)-phenyl]-ethyl}-piperazin-1-yl)-propionic acid trifluoromethanesufonate salt; |
| 328 | 3-(1-{2-[4-(Benzothiazol-2-yloxy)-phenyl]-ethyl}-piperidin-4-yl)-oxazolidin-2-one; |
| 329 | 4-(1-{2-[4-(Benzothiazol-2-yloxy)-phenyl]-ethyl}-piperidin-4-yl)-morpholin-3-one; |
| 330 | 4-(1-{2-[4-(Benzothiazol-2-yloxy)-phenoxy]-ethyl}-piperidin-4-yl)-morpholin-3-one; |
| 331 | 3-(1-{2-[4-(Benzothiazol-2-yloxy)-phenoxy]-ethyl}-piperidin-4-yl)-oxazolidin-2-one; |
| 332 | 1-{2-[4-(Benzothiazol-2-yloxy)-phenyl]-ethyl}-piperidine-4-carboxylic acid benzyloxy-amide; |
| 333 | (1-{2-[4-(Benzothiazol-2-yloxy)-phenyl]-ethyl}-piperidin-4-yl)-acetic acid; |
| 334 | (*R*)-1-(1-{2-[4-(Benzothiazol-2-yloxy)-phenyl]-ethyl}-piperidin-4-yl)-4-hydroxy-pyrrolidin-2-one; |
| 335 | 1-{2-[4-(Benzothiazol-2-yloxy)-phenyl]-ethyl}-piperidine-4-carboxylic acid hydroxyamide; |
| 336 | (*S*)-1-(1-{2-[4-(Benzothiazol-2-yloxy)-phenyl]-ethyl}-piperidin-4-yl)-4-hydroxy-pyrrolidin-2-one; |
| 337 | (1-{2-[4-(Benzothiazol-2-yloxy)-phenyl]-ethyl}-piperidin-4-yl)-carbamic acid tert-butyl ester; |
| 338 | 2-{4-[2-(4-Fluoro-piperidin-1-yl)-ethyl]-phenoxy}-benzothiazole; |
| 339 | 2-{4-[2-(4,4-Difluoro-piperidin-1-yl)-ethyl]-phenoxy}-benzothiazole; |
| 340 | (*R*)-1-{2-[4-(Benzothiazol-2-yloxy)-phenyl]-ethyl}-pyrrolidin-3-ol; |
| 341 | N-(1-{2-[4-(Benzothiazol-2-yloxy)-phenyl]-ethyl}-piperidin-4-yl)-formamide; |
| 342 | (1-{2-[4-(Benzothiazol-2-yloxy)-phenyl]-ethyl}-piperidin-4-yl)-urea; |
| 343 | 1-(1-{2-[4-(Benzothiazol-2-yloxy)-phenyl]-ethyl}-piperidin-4-yl)-3-cyano-2-phenyl-isourea; |
| 344 | 1-(1-{2-[4-(Benzothiazol-2-yloxy)-phenyl]-ethyl}-piperidin-4-yl)-3-cyano-2-methyl-isothiourea; |
| 345 | N-(1-{2-[4-(Benzothiazol-2-yloxy)-phenyl]-ethyl}-piperidin-4-yl)-methanesulfonamide; |
| 346 | 1-(1-{2-[4-(Benzothiazol-2-yloxy)-phenyl]-ethyl}-piperidin-4-yl)-3-cyano-2-methyl-guanidine; |
| 347 | 8-{2-[4-(Benzothiazol-2-yloxy)-phenyl]-ethyl}-1-phenyl-1,3,8-triaza-spiro[4.5]decan-4-one; |
| 348 | 8-{2-[4-(Benzothiazol-2-yloxy)-phenyl]-ethyl}-1,3,8-triaza-spiro[4.5]decane-2,4-dione; |
| 349 | (1-{2-[4-(Benzothiazol-2-yloxy)-phenyl]-ethyl}-piperidin-4-yl)-methyl-carbamic acid tert-butyl ester; |
| 350 | N-(1-{2-[4-(Benzothiazol-2-yloxy)-phenyl]-ethyl}-piperidin-4-yl)-N-methyl-acetamide; |
| 351 | N-(1-{2-[4-(Benzothiazol-2-yloxy)-phenyl]-ethyl}-piperidin-4-yl)-N-methyl-methanesulfonamide; |
| 352 | Acetic acid [(1-{2-[4-(benzothiazol-2-yloxy)-phenyl]-ethyl}-piperidin-4-yl)-methyl-carbamoyl]-methyl ester; |
| 353 | N-(1-{2-[4-(Benzothiazol-2-yloxy)-phenyl]-ethyl}-piperidin-4-yl)-N-acetamide; |
| 354 | Acetic acid (1-{2-[4-(benzothiazol-2-yloxy)-phenyl]-ethyl}-piperidin-4-ylcarbamoyl)-methyl ester; |
| 355 | 2-({2-[4-(Benzothiazol-2-yloxy)-phenyl]-ethyl}-methyl-amino)-3-(1 H-imidazol-2-yl)-propionic acid; |
| 356 | 2-(4-{2-[4-(3-Nitro-pyridin-2-yl)-[1,4]diazepan-1-yl]-ethyl}-phenoxy)-benzothiazole; |
| 357 | 2-(4-Piperidin-1-ylmethyl-phenoxy)-benzothiazole; |
| 358 | 1-[4-(Benzothiazol-2-yloxy)-benzyl]-4-phenyl-piperidin-4-ol; |
| 359 | 1-[4-(Benzothiazol-2-yloxy)-benzyl]-piperidin-4-ol; |
| 360 | {1-[4-(Benzothiazol-2-yloxy)-benzyl]-piperidin-4-yl}-methanol; |
| 361 | N-{1-[4-(Benzothiazol-2-yloxy)-benzyl]-piperidin-4-yl}-methanesulfonamide; |
| 362 | N-{1-[4-(Benzothiazol-2-yloxy)-benzyl]-piperidin-4-ylmethyl}-2-hydroxy-acetamide; |
| 363 | {1-[4-(Benzothiazol-2-yloxy)-benzyl]-piperidin-4-yl}-carbamic acid methyl ester; |
| 364 | {1-[4-(Benzothiazol-2-yloxy)-benzyl]-piperidin-4-ylmethyl}-urea; |
| 365 | N-{1-[4-(Benzothiazol-2-yloxy)-benzyl]-piperidin-4-ylmethyl}-2,2,2-trifluoro-acetamide; |
| 366 | {4-[4-(Benzothiazol-2-yloxy)-benzyl]-piperazin-1-yl}-acetic acid; |
| 367 | 2-[4-(4-Methanesulfonyl-piperazin-1-ylmethyl)-phenoxy]-benzothiazole; |
| 368 | 1-{4-[4-(Benzothiazol-2-yloxy)-benzyl]-piperazin-1-yl}-2,2,2-trifluoro-ethanone; |
| 369 | 2-(4-Morpholin-4-ylmethyl-phenoxy)-benzothiazole; |
| 370 | {1-[4-(Benzothiazol-2-yloxy)-benzyl]-piperidin-4-yl}-carbamic acid phenyl ester; |
| 371 | N-{1-[4-(Benzothiazol-2-yloxy)-benzyl]-piperidin-4-yl}-benzenesulfonamide; |
| 372 | 3-[4-(Benzothiazol-2-yloxy)-benzylamino]-propionic acid ethyl ester; |
| 373 | 3-[4-(Benzothiazol-2-yloxy)-benzylamino]-propionic acid; |
| 374 | [(1-{2-[4-(Benzothiazol-2-yloxy)-phenoxy]-ethyl}-piperidine-4-carbonyl)-methyl-amino]-acetic acid ethyl ester; |
| 376 | 1'-{2-[4-(Benzothiazol-2-yloxy)-phenoxy]-ethyl}-[1,4']bipiperidinyl-4-carboxylic acid ethyl ester; |
| 377 | 1'-{2-[4-(Benzothiazol-2-yloxy)-phenoxy]-ethyl}-[1,4']bipiperidinyl-4-carboxylic acid; |
| 378 | {2-[4-(Benzothiazol-2-yloxy)-phenyl]-ethyl}-cyclopropyl-ethyl-amine; |
| 379 | 3-({2-[4-(Benzothiazol-2-yloxy)-phenyl]-ethyl}-cyclopropyl-amino)-2-methyl-propionic acid trifluoromethansulfonic acid salt; |
| 380 | 2-({2-[4-(Benzothiazol-2-yloxy)-phenyl]-ethyl}-cyclopropylmethyl-amino)-ethanol; |
| 381 | 2-[2-({2-[4-(Benzothiazol-2-yloxy)-phenyl]-ethyl}-cyclopropylmethyl-amino)-ethoxy]-ethanol; |
| 382 | 3-({2-[4-(Benzothiazol-2-yloxy)-phenyl]-ethyl}-cyclopropylmethyl-amino)-propan-1-ol; |
| 383 | {2-[4-(Benzothiazol-2-yloxy)-phenyl]-ethyl}-cyclopropylmethyl-(3-tetrazol-2-yl-propyl)-amine; |
| 384 | {2-[4-(Benzothiazol-2-yloxy)-phenyl]-ethyl}-cyclopropyl-(3-pyrrol-1-yl-propyl)-amine; |
| 385 | 4-({2-[4-(Benzothiazol-2-yloxy)-phenyl]-ethyl}-cyclopropylmethyl-amino)-butyronitrile; |
| 386 | 1-{2-[4-(Benzothiazol-2-yloxy)-phenyl]-ethyl}-piperidine-4-carboxylic acid (2-cyano-ethyl)-amide; |
| 387 | {2-[4-(Benzothiazol-2-yloxy)-phenyl]-ethyl}-cyclopropylmethyl-[3-(2H-tetrazol-5-yl)-propyl]-amine; |
| 388 | 3-[5-(1-{2-[4-(Benzothiazol-2-yloxy)-phenyl]-ethyl}-piperidin-4-yl)-tetrazol-1-yl]-propionitrile; |
| 389 | {2-[4-(Benzothiazol-2-yloxy)-phenyl]-ethyl}-cyclopropyl-[3-(2H-tetrazol-5-yl)-propyl]-amine; |
| 390 | 1-{2-[4-(Benzothiazol-2-yloxy)-phenyl]-ethyl}-piperidine-4-carboxylic acid (2-hydroxy-1,1-dimethyl-ethyl)-amide; |
| 391 | {2-[4-(Benzothiazol-2-yloxy)-phenyl]-ethyl}-cyclopropylmethyl-[3-(1 H-[1,2,4]triazol-3-yl)-propyl]-amine; |
| 392 | {2-[4-(Benzothiazol-2-yloxy)-phenyl]-ethyl}-cyclopropylmethyl-[3-(5-methyl-1 H-[1,2,4]triazol-3-yl)-propyl]-amine; |
| 393 | {2-[4-(Benzothiazol-2-yloxy)-phenyl]-ethyl}-cyclopropylmethyl-[3-(5-phenyl-1 H-[1,2,4]triazol-3-yl)-propyl]-amine; |
| 394 | 2-(4-{2-[4-(1-Methyl-1H-tetrazol-5-yl)-piperidin-1-yl]-ethyl}-phenoxy)-benzothiazole; |
| 395 | 2-(4-{2-[4-(2-Methyl-2H-tetrazol-5-yl)-piperidin-1-yl]-ethyl}-phenoxy)-benzothiazole; |
| 396 | 1-{2-[4-(Benzothiazol-2-yloxy)-phenyl]-ethyl}-piperidine-4-carbonitrile; |
| 397 | 2-(4-{2-[4-(1H-[1,2,3]Triazol-4-yl)-piperidin-1-yl]-ethyl}-phenoxy)-benzothiazole; |
| 398 | 4-{2-[4-(Benzothiazol-2-yloxy)-phenyl]-ethylamino}-butyric acid ethyl ester; |
| 399 | 4-({2-[4-(Benzothiazol-2-yloxy)-pheriyl]-ethyl}-cyclopropylmethyl-amino)-butyric acid ethyl, ester; |
| 400 | 2-[3-({2-[4-(Benzothiazol-2-yloxy)-phenyl]-ethyl}-cyclopropylmethyl-amino)-propyl]-isoindole-1,3-dione; |
| 401 | 4-({2-[4-(Benzothiazol-2-yloxy)-phenyl]-ethyl}-cyclopropylmethyl-amino)-butyric acid; |
| 402 | 1-(3-{2-[4-(Benzothiazol-2-yloxy)-phenyl]-ethylamino}-propyl)- pyrrolidin-2-one; |
| 403 | N-1-{2-[4-(Benzothiazol-2-yloxy)-phenyl]-ethyl}-N1-cyclopropylmethyl-propane-1,3-diamine; |
| 404 | 5-({2-[4-(Benzothiazol-2-yloxy)-phenyl]-ethyl}-cyclopropyl-amino)-pentanoic acid methyl ester; |
| 405 | N-[3-({2-[4-(Benzothiazol-2-yloxy)-phenyl]-ethyl}-cyclopropylmethyl-amino)-propyl]-acetamide; |
| 406 | Morpholine-4-carboxylic acid [3-({2-[4-(benzothiazol-2-yloxy)-phenyl]-ethyl}-cyclopropylmethyl-amino)-propyl]-amide; |
| 407 | N-[3-({2-[4-(Benzothiazol-2-yloxy)-phenyl]-ethyl}-cyclopropylmethyl-amino)-propyl]-methanesulfonamide |
| 408 | 5-({2-[4-(Benzothiazol-2-yloxy)-phenyl]-ethyl}-cyclopropyl-amino)-pentanoic acid; |
| 409 | 1-[3-({2-[4-(Benzothiazol-2-yloxy)-phenyl]-ethyl}-isopropyl-amino)-propyl]-pyrrolidin-2-one; |
| 410 | 1-[3-({2-[4-(Benzothiazol-2-yloxy)-phenyl]-ethyl}-cyclopropylmethyl-amino)-propyl]-pyrrolidin-2-one; |
| 411 | 1-[3-({2-[4-(Benzothiazol-2-yloxy)-phenyl]-ethyl}-cyclopropyl-amino)-propyl]-pyrrolidin-2-one; |
| 412 | 1-[3-({2-[4-(Benzothiazol-2-yloxy)-phenyl]-ethyl}-propyl-amino)-propyl]-pyrrolidin-2-one; |
| 413 | 4-((1-Acetyl-piperidin-4-yl)-{2-[4-(benzothiazol-2-yloxy)-phenyl]-ethyl}-amino)-butyric acid ethyl ester; |
| 414 | 4-((1-Acetyl-piperidin-4-yl)-{2-[4-(benzothiazol-2-yloxy)-phenyl]-ethyl}-amino)-butyric acid ethyl ester; |
| 415 | 4-({2-[4-(Benzothiazol-2-yloxy)-phenyl]-ethyl}-methanesulfonyl-amino)-butyric acid; |
| 416 | ({2-[4-(Benzothiazol-2-yloxy)-phenyl]-ethyl}-cyclopropyl-amino)-acetic acid; |
| 417 | 6-({2-[4-(Benzothiazol-2-yloxy)-phenyl]-ethyl}-cyclopropyl-amino)-hexanoic acid ethyl ester; |
| 418 | 7-({2-[4-(Benzothiazol-2-yloxy)-phenyl]-ethyl}-cyclopropyl-amino)-heptanoic acid ethyl ester; |
| 419 | 6-({2-[4-(Benzothiazol-2-yloxy)-phenyl]-ethyl}-cyclopropyl-amino)-hexanoic acid; |
| 420 | 7-({2-[4-(Benzothiazol-2-yloxy)-phenyl]-ethyl}-cyclopropyl-amino)-heptanoic acid; |
| 421 | N-1-{2-[4-(Benzothiazol-2-yloxy)-phenyl]-ethyl}-N1-cyclopropyl-propane-1,3-diamine; |
| 422 | N-[3-({2-[4-(Benzothiazol-2-yloxy)-phenyl]-ethyl}-cyclopropyl-amino)-propyl]-acetamide; |
| 423 | N-[3-({2-[4-(Benzothiazol-2-yloxy)-phenyl]-ethyl}-cyclopropyl-amino)-propyl]-isobutyramide; |
| 424 | N-[3-({2-[4-(Benzothiazol-2-yloxy)-phenyl]-ethyl}-cyclopropyl-amino)-propyl]-benzamide; |
| 425 | N-[3-({2-[4-(Benzothiazol-2-yloxy)-phenyl]-ethyl}-cyclopropyl-amino)-propyl]-4-chloro-benzamide; |
| 426 | N-[3-({2-[4-(Benzothiazol-2-yloxy)-phenyl]-ethyl}-cyclopropyl-amino)-propyl]-methanesulfonamide; |
| 427 | Propane-2-sulfonic acid [3-({2-[4-(benzothiazol-2-yloxy)-phenyl]-ethyl}-cyclopropyl-amino)-propyl]-amide trifluoromethansulfonic acid salt; |
| 428 | 8-({2-[4-(Benzothiazol-2-yloxy)-phenyl]-ethyl}-cyclopropyl-amino)-octanoic acid ethyl ester; |
| 429 | 1-[3-({2-[4-(Benzothiazol-2-yloxy)-phenyl]-ethyl}-cyclopropyl-amino)-propyl]-3-phenyl-urea; |
| 430 | 8-({2-[4-(Benzothiazol-2-yloxy)-phenyl]-ethyl}-cyclopropyl-amino)-octanoic acid; |
| 431 | Tetrahydro-furan-2-carboxylic acid [3-({2-[4-(benzothiazol-2-yloxy)-phenyl]-ethyl}-cyclopropyl-amino)-propyl]-amide; |
| 432 | N-[3-({2-[4-(Benzothiazol-2-yloxy)-phenyl]-ethyl}-cyclopropyl-amino)-propyl]-2-hydroxy-acetamide; |
| 433 | 4-({2-[4-(Benzothiazol-2-yloxy)-phenoxy]-ethyl}-cyclopropyl-amino)-butyric acid; |
| 434 | 1-{3-[4-(Benzothiazol-2-yloxy)-benzylamino]-propyl}-pyrrolidin-2-one; |
| 435 | 1-(3-{[4-(Benzothiazol-2-yloxy)-benzyl]-methyl-amino}-propyl)-pyrrolidin-2-one; |
| 436 | 1-(3-{[4-(Benzothiazol-2-yloxy)-benzyl]-isopropyl-amino}-propyl)-pyrrolidin-2-one; |
| 437 | 1-(3-{[4-(Benzothiazol-2-yloxy)-benzyl]-ethyl-amino}propyl)-pyrrolidin-2-one; |
| 438 | [4-(Benzothiazol-2-yloxy)-benzyl]-cyclopropyl-amine; |
| 439 | N-1-[4-(Benzothiazol-2-yloxy)-benzyl]-N1-cyclopropyl-propane-1,3-diamine; |
| 440 | N-(3-{[4-(Benzothiazol-2-yloxy)-benzyl]-cyclopropyl-amino}-propyl)-isobutyramide; |
| 441 | 1-(3-{[4-(Benzothiazol-2-yloxy)-benzyl]-cyclopropyl-amino}-propyl)-3-isopropyl-urea; |
| 442 | 1-{1-[4-(Benzothiazol-2-yloxy)-benzyl]-piperidin-4-yl}-3-isopropyl-urea; |
| 443 | N-{1-[4-(Benzothiazol-2-yloxy)-benzyl]-piperidin-4-yl}-oxalamic acid methyl ester; |
| 444 | N-{1-[4-(Benzothiazol-2-yloxy)-benzyl]-piperidin-4-yl}-isobutyramide; |
| 445 | Tetrahydro-furan-2-carboxylic acid {1-[4-(benzothiazol-2-yloxy)-benzyl]-piperidin-4-yl}-amide; |
| 446 | 1-{1-1-[4-(Benzothiazol-2-yloxy)-benzyl]-piperidin-4-yl}-4-hydroxy-pyrrolidin-2-one; |
| 447 | 1-{1-[4-(Benzothiazol-2-yloxy)-benzyl]-piperidin-4-yl}-4-hydroxy-pyrrolidin-2-one; |
| 448 | {1-[4-(Benzothiazol-2-yloxy)-benzyl]-piperidin-4-yl}-urea; |
| 449 | N-{1-[4-(Benzothiazol-2-yloxy)-benzyl]-piperidin-4-yl}-oxalamic acid; |
| 450 | N-{1-[4-(Benzothiazol-2-yloxy)-benzyl]-piperidin-4-yl}-2-hydroxy-acetamide; |
| 451 | N-{1-[4-(Benzothiazol-2-yloxy)-benzyl]-piperidin-4-yl}-2,2,2-trifluoro-acetamide; |
| 452 | 2-[4-(1,1-Dioxo-1l6-thiomorpholin-4-ylmethyl)-phenoxy]-benzothiazole; |
| 453 | N-{1-[4-(benzothiazol-2-yloxy)-benzyl]-piperidin-4-amino sulfonyl}-carbamic acid tert-butyl ester; |
| 454 | N-{1-[4-(Benzothiazol-2-yloxy)-benzyl]-piperidin-4-yl}-acetamide; |
| 455 | N-{1-[4-(Benzothiazol-2-yloxyybenzyl]-piperidin-4-yl}-N,N-dimethylsulfamide; |
| 456 | 1-{1-[4-(Benzothiazol-2-yloxy)-benzyl]-piperidin-4-yl}-3-ethyl-urea; |
| 457 | 1-{1-[4-(Benzothiazol-2-yloxy)-benzyl]-piperidin-4-yl}-3-ethyl-thiourea; |
| 458 | Propane-1-sulfonic acid {1-[4-(benzothiazol-2-yloxy)-benzyl]-piperidin-4-yl}-amide; |
| 459 | Propane-2-sulfonic acid {1-[4-(benzothiazol-2-yloxy)-benzyl]-piperidin-4-yl}-amide; |
| 460 | N-{1-[4-(Benzothiazol-2-yloxy)-benzyl]-piperidin-4-yl}-sulfamide; |
| 461 | N-{1-[4-(Benzothiazol-2-yloxy)-benzyl]-piperidin-4-yl}-formamide; |
| 462 | {1-[4-(Benzothiazol-2-yloxy)-benzyl]-piperidin-4-yl}-carbamic acid ethyl ester; |
| 463 | N-{1-[4-(Benzothiazol-2-yloxy)-benzyl]-piperidin-4-yl}-propionamide; |
| 464 | N-{1-[4-(Benzothiazol-2-yloxy)-benzyl]-piperidin-4-yl}-butyramide; |
| 465 | 1-{1-[4-(Benzothiazol-2-yloxy)-benzyl]-piperidin-4-yl}-3-propyl-urea; |
| 466 | {1-[4-(Benzothiazol-2-yloxy)-benzyl]-piperidin-4-yl}-carbamic acid propyl ester; |
| 467 | 1-{1-[4-(Benzothiazol-2-yloxy)-benzyl]-piperidin-4-yl}-3-methyl-urea; |
| 469 | 1-{1-[4-(Benzothiazol-2-yloxy)-benzyl]-piperidin-4-yl}-1,3-dimethyl-urea; |
| 470 | 1-{1-[4-(Benzothiazol-2-yloxy)-benzyl]-piperidin-4-yl}-1-methyl-urea; |
| 471 | N-{1-[4-(Benzothiazol-2-yloxy)-benzyl]-piperidin-4-yl}-N-methyl-acetamide; |
| 472 | {1-[4-(Benzothiazol-2-yloxy)-benzyl]-piperidin-4-yl}-methyl-carbamic acid methyl ester; |
| 473 | N-{1-[4-(Benzothiazol-2-yloxy)-benzyl]-piperidin-4-yl}-N-methyl-oxalamic acid methyl ester; |
| 474 | N-{1-[4-(Benzothiazol-2-yloxy)-benzyl]-piperidin-4-yl}-N-methyl-oxalamic acid; |
| 475 | Guanidine, N-{1-[4-(Benzothiazol-2-yloxy)-benzyl]-piperidin-4-yl}-N'-hydroxy; |
| 476 | {1-[4-(Benzothiazol-2-yloxy)-benzyl]-piperidin-4-yl}-carbamic acid isopropyl ester; |
| 477 | 3-{1-[4-(Benzothiazol-2-yloxyrbenzyl]-piperidin-4-yl}-1,1-dimethyl-urea; |
| 478 | Acetic acid {1-[4-(beniothiazol-2-yloxy)-benzyl]-piperidin-4-ylcarbamoyl}-methyl ester; |
| 479 | {1-[4-(Benzothiazol-2-yloxy)-benzyl]-piperidin-4-yl}-thiourea; |
| 482 | 2-[4-(2-Morpholin-4-yl-ethoxy)-phenoxy]-benzothiazole; and |
| 483 | 2-[4-(2-Piperidin-1-yl-ethoxy)-phenoxy]-benzothiazole. |

Additional embodiments of this invention, where X is NR⁵ with R⁵ being one of H and CH₃, are made according to the synthetic methods outlined in Schemes A-C, F, G and J-L, have demonstrated LTA4H inhibitory activity, and are selected from the group consisting of:

| Example | Compound |
|---|---|
| 37 | 1-{2-[4-(1*H*-Benzoimidazol-2-yloxy)-phenoxy]-ethyl}-4-phenyl-piperidin-4-ol; |
| 38 | {2-[4-(1*H*-Benzoimidazol-2-yloxy)-phenyl]-ethyl}-cyclopropylmethyl-propyl-amine; |
| 39 | Cyclohexyl-ethyl-{2-[4-(1-methyl-1*H*-benzoimidazol-2-yloxy)-phenyl]-ethyl}-amine; |
| 117 | 1-{2-[4-(1*H*-Benzoimidazol-2-yloxy)-phenoxy]-ethyl}-4-(4-bromo-phenyl)-piperidin-4-ol; |
| 118 | 1-{2-[4-(1*H*-Benzoimidazol-2-yloxy)-phenoxy]-ethyl)-4-(4-chloro-phenyl)-piperidin-4-ol; |
| 119 | 1-{2-[4-(1*H*-Benzoimidazol-2-yloxy)-phenoxy]-ethyl}-4-benzyl-piperidin-4-ol; |
| 123 | {2-[4-(1*H*-Benzoimidazol-2-yloxy)-phenyl]-ethyl}-cyclohexyl-ethyl-amine; |
| 124 | 2-[4-(2-Pyrrolidin-1-yl-ethyl)-phenoxyl-1*H*-benzoimidazole; |
| 125 | 2-[4-(2-Azepan-1-yl-ethyl)-phenoxy]-1*H*-benzoimidazole; |
| 126 | {2-[4-(1*H*-Benzoimidazol-2-yloxy)-phenyl]-ethyl}-dibutyl-amine; |
| 127 | 1-{2-[4-(1*H-*Benzoimidazol-2-yloxy)-phenyl]-ethyl}-piperidin-4-ol; |
| 128 | 1-{2-[4-(1*H*-Benzoimidazol-2-yloxy)-phenyl]-ethyl}-piperidine-4-carboxylic acid methyl ester; |
| 129 | {2-[4-(1*H*-Benzoimidazol-2-yloxy)-phenoxy]-ethyl}-cyclohexyl-ethyl-amine; |
| 130 | 2-{4-[2-(4-Methyl-piperidin-1-yl)-ethoxy]-phenoxy}-1*H*-benzoimidazole; |
| 131 | 2-{4-[2-(2-Ethyl-piperidin-1-yl)-ethoxy]-phenoxy}-1*H*-benzoimidazole; |
| 132 | 2-[4-(2-Piperidin-1-yl-ethoxy)-phenoxy]-1*H*-benzoimidazole; |
| 133 | (1-{2-[4-(1*H*-Benzoimidazol-2-yloxy)-phenoxy]-ethyl}-piperidin-4-yl)-methanol; |
| 134 | 1-{2-[4-(1*H*-Benzoimidazol-2-yloxy)-phenoxy]-ethyl}-piperidin-4-ol; |
| 138 | 2-[4-(2-Azepan-1-yl-ethoxy)-phenoxy]-1*H*-benzoimidazole amide; |
| 139 | {3-[4-(1*H*-Benzoimidazol-2-yloxy)-phenoxy]-propyl}-dimethyl-amine; |
| 140 | 2-[4-(2-Pyrrolidin-1-yl-ethoxy)-phenoxy]-1*H*-benzoimidazole; |
| 141 | {2-[4-(1*H*-Benzoimidazol-2-yloxy)-phenoxy]-ethyl}-diethyl-amine; |
| 142 | 2-[4-(2-Morpholin-4-yl-ethoxy)-phenoxy]-1*H*-benzoimidazole; |
| 230 | 2-[4-(2-Piperidin-1-yl-ethyl)-phenoxy]-1*H*-benzoimidazole; |
| 231 | 1-(1-{2-[4-(1*H*-Benzoimidazol-2-yloxy)-phenoxy]-ethyl}-piperidin-4-yl)-pyrrolidin-2-one; |
| 480 | (1-{2-[4-(1H-Benzoimidazol-2-yloxy)-phenoxy]-ethyl}-piperidin-4-yl)-methanol; and |
| 485 | 1-{2-[4-(1H-Benzoimidazol-2-yloxy)-phenoxy]-ethyl}-piperidine-4-carboxylic acid ethyl ester. |

Compounds of the present invention may be prepared according to the reaction schemes described below. The schemes represent two basic approaches to target compound synthesis, both in a linear fashion, commencing from either end of the molecule. Persons skilled in the art will recognize that certain compounds are more advantageously produced by one scheme over another.

To obtain the various compounds herein, starting materials may be employed which carry the ultimately desired substituents though the reaction scheme with or without protection as appropriate. Starting materials may be obtained from commercial sources or synthesized by methods known to one skilled in the art. Alternatively, it may be necessary to employ, in the place of the ultimately desired substituent, a suitable group, which may be carried through the reaction scheme and replaced as appropriate with the desired substituent. Any product containing a chiral center may be separated into its enantiomers by conventional techniques.

Embodiments of processes illustrated herein include, when chemically meaningful, one or more steps such as hydrolysis, halogenation, protection, and deprotection. These steps can be implemented in light of the teachings provided herein and the ordinary skill in the art.

During any of the processes for preparation of the compounds of the present invention, it may be necessary and/or desirable to protect sensitive or reactive groups on any of the molecules concerned. In addition, compounds of the invention may be modified by using protecting groups; such compounds, precursors, or prodrugs are also within the scope of the invention. This may be achieved by means of conventional protecting groups, such as those described in "Protective Groups in Organic Chemistry", ed. J.F.W. McOmie, Plenum Press, 1973; and T.W. Greene & P.G.M. Wuts, "Protective Groups in Organic Synthesis", 3rd ed., John Wiley & Sons, 1999. The protecting groups may be removed at a convenient subsequent stage using methods known from the art.

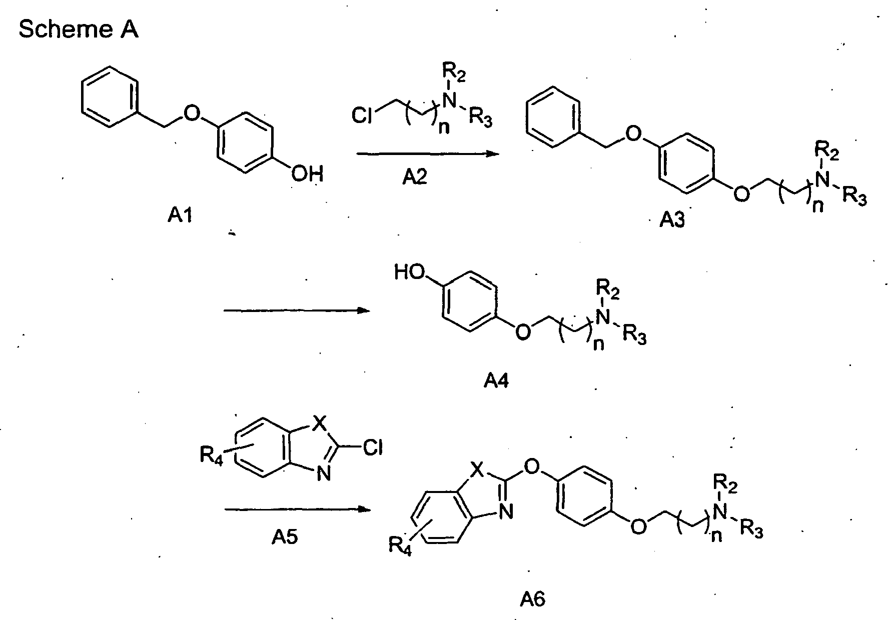

Referring to Scheme A, n is 1 or 2 commercially available 4-benzyloxyphenol, A1, is alkylated with amino alkyl halides, A2; several amino alkyl chlorides are commercially available. The reactions can be run under a wide range of temperatures, including room temperature and more elevated temperatures, in the presence of an inorganic base known to facilitate O-alkylation, such as, but not limited to, K₂CO₃, Cs₂CO₃ and mixtures thereof (J. Med. Chem, 1997, 40, 1407-1416). Suitable solvents include but are not limited to DMF. Removal of the benzyl group on A3 may be accomplished using catalytic hydrogenation conditions well known to those skilled in the art (Greene, T.W.; Wuts, P.G.M. Protective Groups in Organic Synthesis, 3rd ed.; John Wiley & Sons: New York, 1999.). Suitable catalysts include but are not limited to Pd on carbon (Pd/C), in solvents such as ethyl acetate, alcohols and mixtures thereof. Examples of alcohols include but are not limited to CH₃OH, ethanol, *i*-PrOH. These reactions are typically run at room temperature. Removal of the benzyl group on A3 may be accomplished in some embodiments by using dissolving metal reductions or transfer hydrogenation conditions at suitable temperatures. For example, dissolving metal reductions are typically performed at temperatures below room temperature (-33 °C). Reaction of A4 with the aromatic bicyclic ring system, A5, suitably protected if appropriate, may be accomplished within a wide range of temperatures including room temperature and more elevated temperatures in the presence of a suitable base including but not limited to amine or inorganic base as defined above. Suitable amine bases include but are not limited to triethylamine (TEA), *N,N*-diisopropylethylamine, 1,8-diazabicyclo[5.4.0]undec-7-ene (DBU), resin-bound amine bases and mixtures thereof. When X is oxygen or sulfur, protecting groups are not applicable. Suitable solvents include but are not limited to DMF, CH₃CN, acetone and mixtures thereof. When X is NR⁵, and R⁵ is a suitable silicon based protecting group, such as SEM (trimethylsilylethoxymethyl), removal of the silicon-based protecting group on NR⁵ can be accomplished using conditions well known to those skilled in the art (Greene et. al. as cited above). Typical reaction conditions include but are not limited to the use of tetrabutylammonium fluoride (TBAF), in suitable solvents such as THF at elevated temperatures.

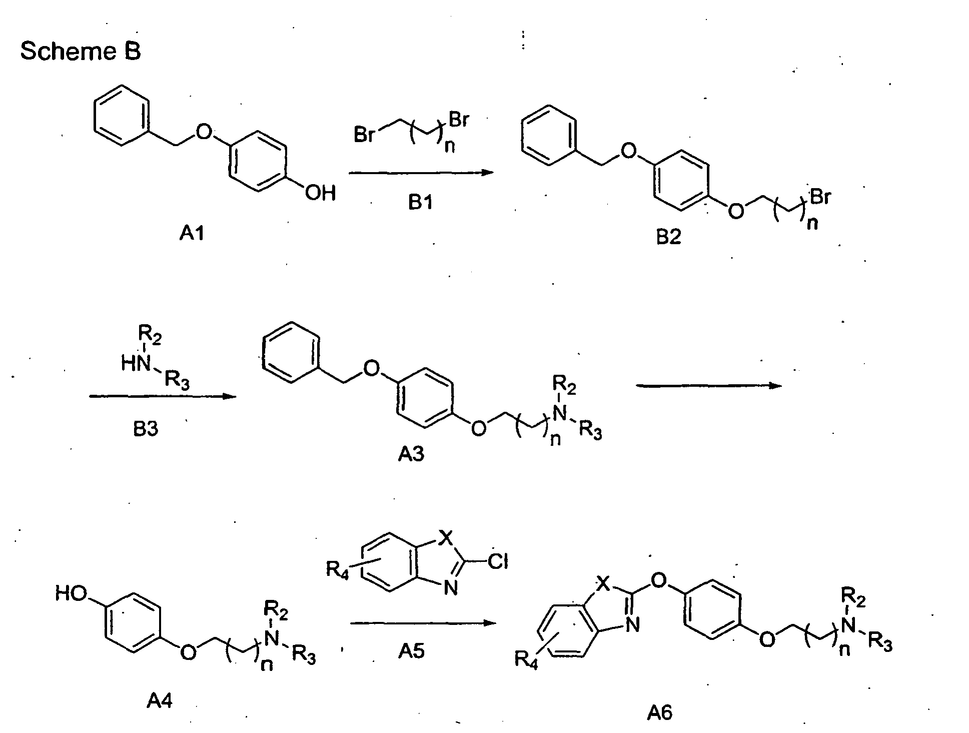

Referring to Scheme B, commercially available 4-benzyloxyphenol, A1, is alkylated with dihaloalkanes, preferably dibromoalkanes such as 1,2-dibromoethane and 1,3-dibromopropane, B1, both of which are commercially available, under a wide range of temperatures with elevated temperatures preferred (Zhou, Z. -L. et al., J. Med. Chem. 1999, 42:2993-3000). The reactions are conducted in the presence of an inorganic base known to facilitate O-alkylation such as, but not limited to, K₂CO₃, Cs₂CO₃ and mixtures thereof. Suitable solvents include but are not limited to CH₃CN and DMF. Compounds of structure B2 are treated with amines, B3, either in the presence or absence of a suitable amine base as described above under a wide range of temperatures with elevated temperatures preferred. Suitable solvents include CH₃CN, CH₂Cl₂ and DMF. Further conversion of the resulting products, of structure A3, to compounds of structure A6, is as detailed above for Scheme A.

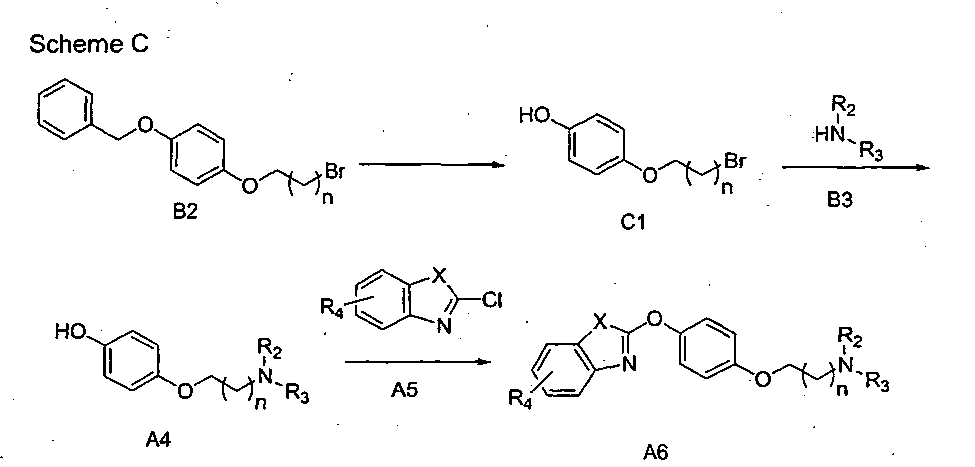

Referring to Scheme C, the benzyl group of compounds of structure B2 can be removed using catalytic hydrogenation conditions well known to those skilled in the art (Greene et. al. as cited above). Suitable catalysts include but are not limited to Pd/C, in solvents such as THF and THF/ethanol mixtures. These reactions are typically run at room temperature. Removal of the benzyl group on B2 can be accomplished in some embodiments using transfer-hydrogenation conditions using suitable solvents and temperatures. Compounds of general structure C1 are treated with amines of structure B3 either in the presence or absence of a suitable amine base as described above under a wide range of temperatures with elevated temperatures preferred. Suitable solvents include but are not limited to CH₃CN, CH₂Cl₂ and DMF. Further conversion of the resulting products, A4, to compounds A6, is as detailed above for Scheme A.

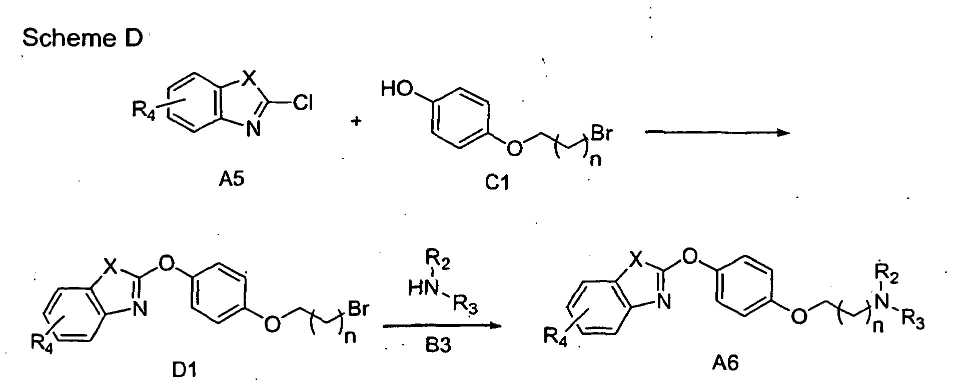

Referring to Scheme D, the compounds of structure A6 can also be prepared by treatment of compounds of structure C1 with aromatic bicyclic compounds, A5, where X = S and O, in the presence of a suitable inorganic base, as defined above, under a wide range of temperatures with elevated temperatures preferred. Suitable solvents include but are not limited to DMF, CH₃CN and mixtures thereof. Conversion of compounds of structure D1 to compounds of structure A6 can be accomplished by treatment with compounds of structure B3. These reactions can be performed either in the presence or absence of a suitable amine base as defined above or an inorganic base such as, but not limited to, K₂CO₃, Cs₂CO₃ and mixtures thereof as described above, under a wide range of temperatures with elevated temperatures preferred. Suitable solvents include but are not limited to CH₃CN and DMF.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as (Greene et. al. as cited above).

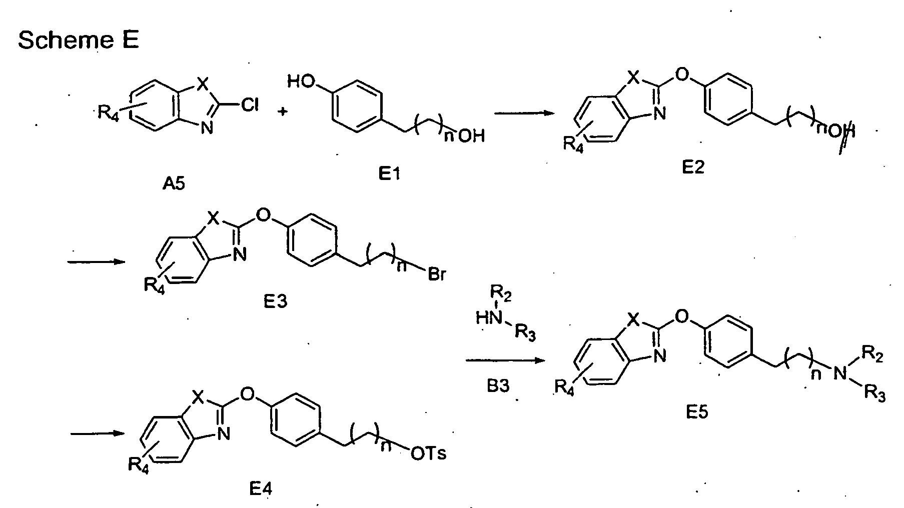

Referring to Scheme E, treatment of compounds of structure E1 with aromatic bicyclic compounds, A5, where X is Swhy not O also?, in the presence of a suitable inorganic base, as defined above, under a wide range of temperatures with elevated temperatures are preferred. Suitable solvents include but are not limited to DMF, CH₃CN and mixtures thereof. Compounds of structure E2 can be converted to compounds of structure E3 using typical brominating conditions including but not limited to the use of PBr₃ at elevated temperatures. Suitable solvents include but are not limited to benzene. Compounds of structure E2 can also be converted to compounds of structure E4 using standard conditions for sulphonylation well known to those skilled in the art. These include but are not limited to the use of TsCl to prepare tosylates, as denoted in the scheme, in the presence of an amine base at room temperature in CH₂Cl₂. Conversion of compounds of structure E3 to compounds of structure E5 can be accomplished by treatment with compounds of structure B3. These reactions can be performed either in the presence or absence of a suitable amine base as described above or an inorganic base such as, but not limited to, K₂CO₃, Cs₂CO₃ and mixtures thereof under a wide range of temperatures with elevated temperatures preferred. Suitable solvents include but are not limited to CH₃CN and DMF. Compounds of structure E4 can be converted to the compounds of the structure E5 by treatment with compounds of structure B3. These reactions can be performed either in the presence or absence of a suitable gamine base as described above under a wide range of temperatures. Suitable solvents include but are not limited to CH₃CN and DMF.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

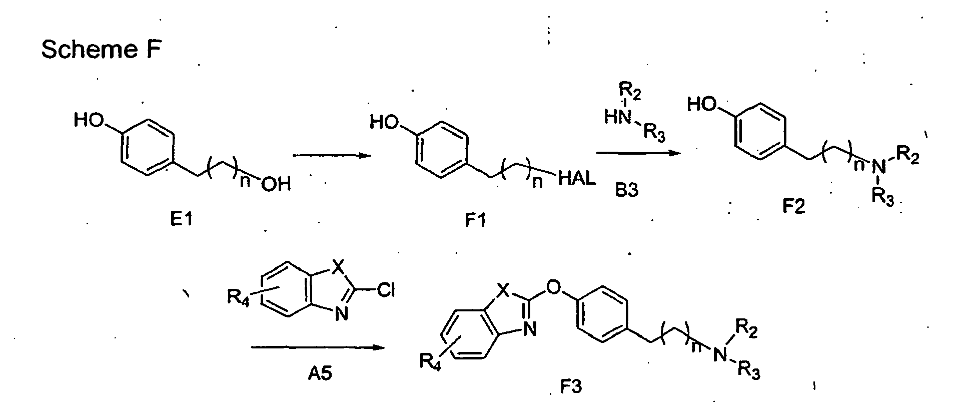

Referring to Scheme F, commercially available 4-(2-hydroxy-ethyl)-phenol and 4-(2-hydroxy-propyl)-phenol are converted to the corresponding alkyl halides F1, where HAL is chloride or bromide, using typical brominating or chlorinating conditions. These conditions include but are not limited to treatment with 48% HBr solutions at elevated temperatures. The resulting bromophenols of structure F1. can then be treated with amines of the structure of B3 either in the presence or absence of a suitable amine base as described above under a wide range of temperatures. Suitable solvents include but are not limited to CH₃CN and DMF. Reaction of F2 with the aromatic bicyclic ring system, A5, suitably protected if appropriate, may be accomplished within a wide range of temperatures including room temperature and more elevated temperatures, in the presence of a suitable amine or inorganic base as defined above. Suitable solvents include but are not limited to DMF, CH₃CN, acetone and mixtures thereof. When X is O or S, protecting groups are not applicable. When X is NR⁵, and R⁵ is a suitable silicon-based protecting group, such as SEM (trimethylsilylethoxymethyl), removal of the silicon-based protecting group on NR⁵ can be accomplished using conditions well known to those skilled in the art (Greene et. al. as cited above). Typical reaction conditions include but are not limited to the use of TBAF, in suitable solvents such as THF at elevated temperatures.

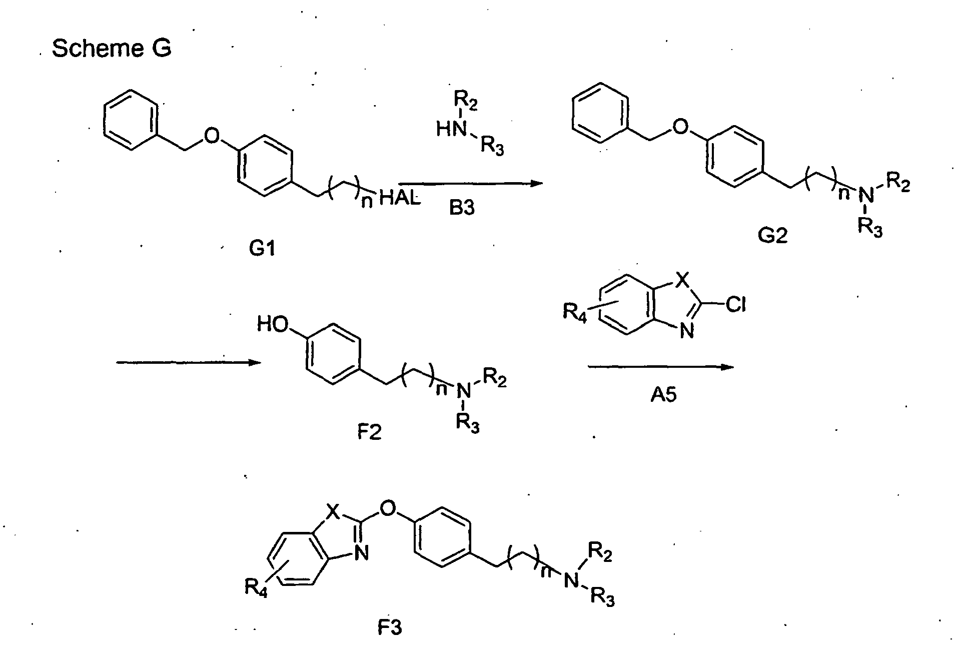

Referring to Scheme G, G1, where n is 0 or 2 and HAL is bromide or chloride, are commercially available materials or may be obtained from **, and G1, where n is 1, is envisaged to be available using standard alkylation conditions starting from 4-(2-hydroxy-ethyl)-phenol and benzyl bromide. The benzyl group in G1 serves as a protecting group. Other compatible protecting groups known to one skilled in the art may be employed in this sequence. Compounds with the general structure G2 can be obtained by treatment with amines of the general structure B3, either in the presence or absence of a suitable amine base as described above under a wide range of temperatures. Suitable solvents include but are not limited to CH₃CN and DMF. Removal of the benzyl may be accomplished using catalytic hydrogenation conditions well known to those skilled in the art (Greene et. al. as cited above). Suitable catalysts include but are not limited to Pd/C, in solvents such as ethyl acetate, alcohols and mixtures thereof. Examples of alcohols include but are not limited to CH₃OH, ethanol, *i*-PrOH. These reactions are typically run at room temperature. Removal of the benzyl group on G2 may be accomplished in some embodiments using transfer-hydrogenation conditions at suitable temperatures. Further conversion of the resulting products, F2, to the final target compounds F3 is as detailed above for Scheme F.

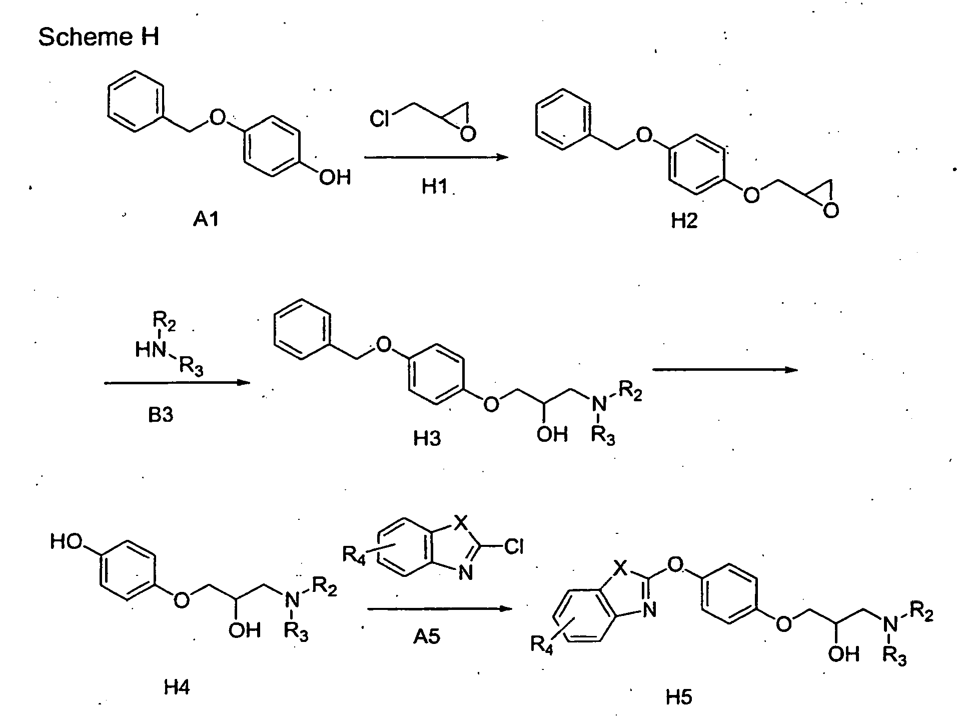

Referring to Scheme H, commercially available 4-benzyloxyphenol, A1, is treated with epichlorohydrin, H1, both of which are commercially available. The reaction can be run under a wide range of temperatures, with elevated temperatures preferred, in the presence of an inorganic base such as, but not limited to, K₂CO₃, Cs₂CO₃ and mixtures thereof. Suitable solvents include but are not limited to DMF. Conversion of compounds of structure H2 to compounds of structure H3 can be accomplished by treatment with amines of general structure B3 either in the presence or absence of a suitable amine base as described above or an inorganic base such as, but not limited to, K₂CO₃, Cs₂CO₃ and mixtures thereof under a wide range of temperatures with elevated temperatures preferred. Suitable solvents include but are not limited to CH₃CN and DMF. Removal of the benzyl group on H3 may be accomplished using catalytic-hydrogenation conditions well known to those skilled in the art (Greene et. al. as cited above). Suitable catalysts include but are not limited to Pd/C, in solvents such as ethyl acetate, alcohols and mixtures thereof. Examples of alcohols include but are not limited to ethanol, CH₃OH, *i-*PrOH. These reactions are typically run at room temperature. Removal of the benzyl group on B2 can be accomplished in some embodiments using transfer-hydrogenation conditions using suitable solvents and temperatures. Conversion of compounds of structure H4 to final target compounds H5 can be accomplished by treatment with the aromatic bicyclic ring system, A5, where X is O, in the presence of a suitable inorganic base, as defined above, under a wide range of temperatures with lower temperatures preferred. Suitable solvents include but are not limited to acetone.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

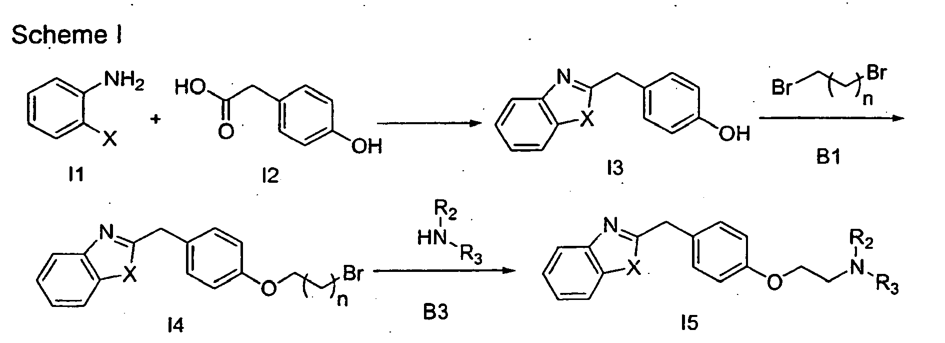

Referring to Scheme I, compounds of type I5 are prepared by heating commercially available 4-hydroxyphenyl acetic acid with, in the case of X is S, 2-aminothiophenol. In the case of X is O 2-aminophenol is used. The two starting materials are heated in the absence of solvent, and the resulting phenols, I3, are treated with dihaloalkanes, preferably dibromoalkanes such as 1,2-dibromoethane and 1,3-dibromopropane, B1, both of which are commercially available, under a wide range of temperatures with elevated temperatures preferred (*Zhou, Z. -L. et al.* as cited above). The reactions are conducted in the presence of an inorganic base known to facilitate O-alkylation such as, but not limited to, K₂CO₃, Cs₂CO₃ and mixtures thereof. Suitable solvents include but are not limited to CH₃CN and DMF. Compounds of structure I4 are treated with amines, B3, either in the presence or absence of a suitable amine base as described above under a wide range of temperatures with elevated temperatures preferred. Suitable solvents include CH₃CN, CH₂Cl₂ and DMF.

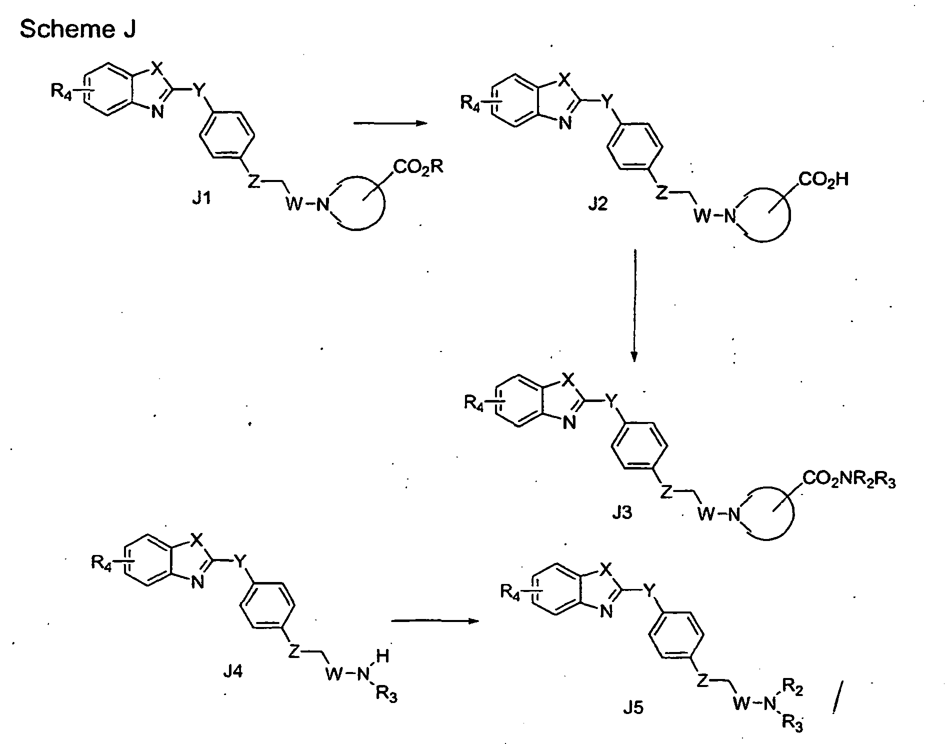

Referring to Scheme J, compounds of the structure J1 can be further elaborated within the limits of the claims to give more highly functionalized target compounds. For example, hydrolysis using methods well known to those skilled in the art such as but not limited to the use of aqueous solutions of LiOH, KOH or NaOH, or aqueous solutions of HCl or CH₃CO₂H, or the use of (CH₃)₃SiOK. Furthermore, persons skilled in the art will recognize that certain compounds are more advantageously produced by one method as compared to another and that salts of the desired compounds may initially result. Compounds of the structure J2 can be further modified to give amides using methods well known to those skilled in the art including but not limited to using (COCl₂)₂ to convert to the intermediary acid chloride followed by exposure to amines of the structure B3. Alternatively, standard amide bond-forming conditions may be utilized, including but not limited to the use of 1,(3-dimethylaminopropyly3-ethylcarbodiimide (EDCI), with or without additives such as HOBT, and amines of the structure B3. Compounds of the structure J4 can be further modified by reductive amination using standard conditions well known to those skilled in the art, including but not limited to the use of an amine of the structure B3 and NaBH(OAc)₃ in an appropriate solvent such as CH₂Cl₂, ClCH₂CH₂Cl or CF₃CH₂OH.

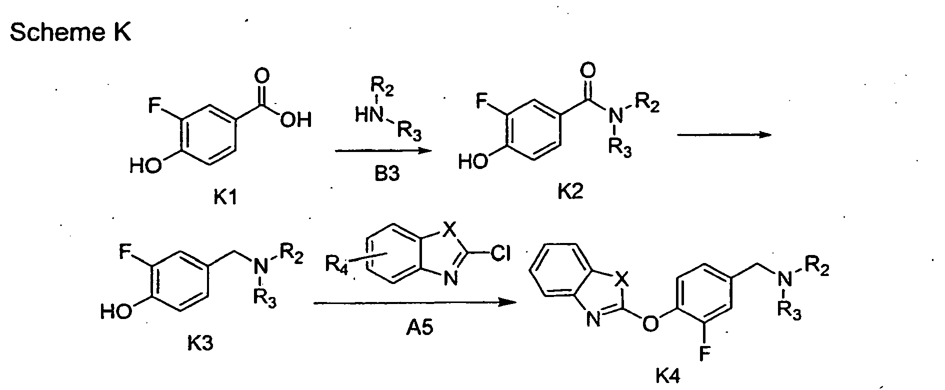

Referring to Scheme K, commercially available 3-fluoro-4-hydroxybenzoic acid, K11, is converted to amides K2, with amines of structure B3, using standard peptide coupling conditions well known to those skilled in the art such as, but not limited to, 1-(3-dimethylaminopropyl)-3-ethylcarbodiimide hydrochloride (EDCI), 1,3-dicyclohexylcarbodiimide (DCC), *O*-(7-azabenzotriazol-1-yl)-*N*,*N*,*N'*,*N'*-tetramethyluronium hexafluorophoshate (HATU), *O*-benzotriazol-1-*N*,*N*,*N*',*N*'-tetramethyluronium hexafluorophosphate (HBTU) and mixtures thereof. Suitable solvents include, but are not limited to, CH₂Cl₂ and THF. The resulting amides of structure K2 are reduced to amines of formula K3 under reducing conditions well known to those skilled in the art, including but not limited to, lithium aluminum hydride in an appropriate solvent such as, but not limited to, THF. Conversion of benzyl amines K3 to the final target compounds, K4, can be accomplished by treatment with the aromatic bicyclic ring system, A5, where X is S or O, in the presence of a suitable inorganic base under a wide range of temperatures with elevated temperatures preferred. Suitable inorganic bases include, but are not limited to, K₂CO₃, Cs₂CO₃ and mixtures thereof. Suitable solvents include, but are not limited to, acetone and CH₃CN.

It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above).

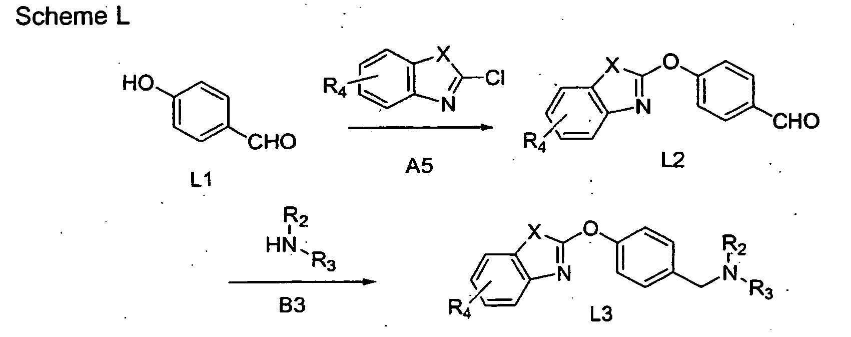

Referring to Scheme L, an alternate embodiment toward the preparation of compounds of formula (I) where n is 0 is described. The starting material L1, 4-hydroxybenzaldehyde, is converted to ethers of formula L2 by treatment with the aromatic bicyclic ring system, A5, where X is S or O, in the presence of a suitable inorganic base under a wide range of temperatures with elevated temperatures preferred. Suitable inorganic bases include, but are not limited to, K₂CO₃, Cs₂CO₃ and mixtures thereof. Suitable solvents include, but are not limited to, acetone and CH₃CN. It is envisaged that when X is NR⁵, and R⁵ is a suitable silicon-based protecting group that the synthesis would follow that described above. The removal of the silicon-based protecting group at the end of the synthetic sequence is further envisaged to occur using conditions as described by texts such as Greene et. al. (as cited above). Aldehydes of formula L2 are converted to amines of formula L3 under reductive amination conditions with amines of formula B3. Suitable reducing agents include Na(OAc)₃BH and NaCNBH₃, with or without the addition of activating agents such as acetic acid or ZnCl₂. Suitable solvents include THF and methanol, and reaction temperatures may range from 0 °C to 70 °C. Preferred reaction conditions are Na(OAc)₃BH in THF at room temperature.

Pharmaceutically acceptable salts, esters, and amides of compounds according to the present invention refer to those salt, ester, and amide forms of the compounds of the present invention which would be apparent to the pharmaceutical chemist, *i.e*., those which are non-toxic and which would favorably affect the pharmacokinetic properties of said compounds of the present invention. Those compounds having favorable pharmacokinetic properties would be apparent to the pharmaceutical chemist, *i*.*e*., those which are non-toxic and which possess such pharmacokinetic properties to provide sufficient palatability, absorption, distribution, metabolism and excretion. Other factors, more practical in nature, which are also important in the selection, are cost of raw materials, ease of crystallization, yield, stability, hygroscopicity and flowability of the resulting bulk drug.

In addition, acceptable salts of carboxylates include sodium, potassium, calcium and magnesium. Examples of suitable cationic salts include hydrobromic, hydroiodic, hydrochloric, perchloric, sulfuric, maleic, fumaric, malic, tartaric, citric, benzoic, mandelic, methanesulfonic, hydroethanesulfonic, benzenesulfonic, oxalic, palmitic, 2-naphthalenesulfonic, p-toluenesulfonic, cyclohexanesulfamic and saccharic.

More extensive sets of examples of acids and bases that may be used in the preparation of pharmaceutically acceptable salts include the following: Acids such as acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitric acid, pamoic acid, phosphoric acid, L-pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid and undecylenic acid; and bases such as ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amines, sodium hydroxide, triethanolamine, tromethamine and zinc hydroxide. *See, e*.*g*., S.M. Berge, et al., "Pharmacetuical Salts", J. Pharm. Sci., 1977, 66:1-19, which is incorporated herein by reference.

Examples of suitable esters include such esters where one or more carboxyl substituents is replaced with p-methoxybenzyloxycarbonyl, 2,4,6-trimethylbenzyloxycarbonyl, 9-anthryloxycarbonyl, CH₃SCH₂COO-, tetrahydrofur-2-yloxycarbonyl, tetrahydropyran-2-yloxycarbonyl, fur-2-yloxycarbonyl, benzoylmethoxycarbonyl, p-nitrobenzyloxycarbonyl, 4-pyridylmethoxycarbonyl, 2,2,2-trichloroethoxycarbonyl, 2,2,2-tribromoethoxycarbonyl, t-butyloxycarbonyl, t-amyloxycarbonyl, diphenylmethoxycarbonyl, triphenylmethoxycarbonyl, adamantyloxycarbonyl, 2-benzyloxyphenyloxycarbonyl, 4-methylthiophenyloxycarbonyl, or tetrahydropyran-2-yloxycarbonyl.

Compounds of the present invention may be used in pharmaceutical compositions to treat patients (humans and other mammals) with disorders involving the action of the LTA4H enzyme. In particular, compounds of the present invention may be used in pharmaceutical compositions to treat inflammation. More particularly, compounds of the present invention may be used in pharmaceutical compositions to treat inflammatory conditions such as inflammatory bowel disease (IBD) (such as Crohn's disease and ulcerative colitis), chronic obstructive pulmonary disease (COPD), arthritis, psoriasis, asthma, cystic fibrosis, atherosclerosis, rheumatoid arthritis, and multiple sclerosis.

The present invention features pharmaceutical compositions containing such compounds and the use of such compositions in the manufacture of medicaments the treatment or prevention of conditions that are mediated by LTA4H enzyme activity. Accordingly, the present invention also contemplates a pharmaceutical composition that comprises at least one compound according to this invention, preferably dispersed in a pharmaceutically acceptable carrier. The at least one compound according to this invention is present in such composition in an amount sufficient to inhibit LTA4H enzyme activity. More particularly, the at least one compound according to this invention is present in such composition in an anti-inflammatory amount.

Accordingly, a pharmaceutical composition that comprises an anti-inflammatory amount of at least one compound according to the present invention in a pharmaceutically acceptable carrier is also contemplated herein. The composition comprises a unit dosage of the at least one compound according to this invention. In preferred practice, the at least one compound according to the present invention that is comprised in the pharmaceutical composition is capable of inhibiting LTA4H enzyme activity in the amount at which that compound is present in the pharmaceutical composition, when that pharmaceutical composition is introduced as a unit dose into an appropriate patient or subject.

The terms "unit dose" and their grammatical equivalent forms are used herein to refer to physically discrete units suitable as unitary dosages for human patients and other animals, each unit containing a predetermined effective, pharmacologic amount of the active ingredient calculated to produce the desired pharmacological effect. The specifications for the novel unit dosage forms of this invention are determined by, and are directly dependent on, the characteristics of the active ingredient, and on the limitations inherent in the art of compounding such an active ingredient for therapeutic use in humans and other animals.

The pharmaceutical compositions can be prepared using conventional pharmaceutical excipients and compounding techniques. Examples of suitable unit dosage forms are tablets, capsules, pills, powder packets, granules, wafers, and the like, segregated multiples of any unit dosage form, as well as liquid solutions, and suspensions. Oral dosage forms may be elixirs, syrups, capsules tablets and the like. Examples of solid carriers include those materials usually employed in the manufacture of pills or tablets, such as lactose, starch, glucose, methylcellulose, magnesium stearate, dicalcium phosphate, mannitol and the like, thickeners such as tragacanth and methylcellulose USP, finely divided SiO₂, polyvinylpyrrolidone, magnesium stearate, and the like. Typical liquid oral excipients include ethanol, glycerol, water and the like. All excipients may be mixed as needed with inert diluents (for example, sodium and calcium carbonates, sodium and calcium phosphates, and lactose), disintegrants (for example, cornstarch and alginic acid), diluents, granulating agents, lubricants (for example, magnesium stearate, stearic acid, and talc), binders (for example, starch and gelatin), thickeners (for example, paraffin, waxes, and petrolatum), flavoring agents, coloring agents, preservatives, and the like by conventional techniques known to those of ordinary skill in the art of preparing dosage forms. Coatings can be present and include, for example, glyceryl monostearate and/or glyceryl distearate. Capsules for oral use include hard gelatin capsules in which the active ingredient is mixed with a solid diluent, and soft gelatin capsules, in which the active ingredient is mixed with water or oil, such as peanut oil, liquid paraffin, or olive oil.

Parenteral dosage forms may be prepared using water or another sterile carrier. For intramuscular, intraperitoneal, subcutaneous, and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions may include suspending agents such as cellulose derivatives, sodium alginate, polyvinyl-pyrrolidone, and gum tragacanth, and a wetting agent, such as lecithin. Suitable preservatives for aqueous suspensions include ethyl and n-propyl p-hydroxybenzoate.

Physiologically acceptable carriers are well known in the art. Examples of liquid carriers are solutions in which compounds according to the present invention form solutions, emulsions, and dispersions. Compatible antioxidants, such as methlyparaben and propylparaben, can be present in solid and liquid compositions, as can sweeteners.

Pharmacetuical compositions according to the present invention may include suitable emulsifiers typically used in emulsion compositions. Such emulsifiers are described in standard publications such as H.P. Fiedler, 1989, Lexikon der Hilfsstoffe für Pharmazie, Kosmetic und agrenzende Gebiete, Cantor ed., Aulendorf, Germany, and in Handbook of Pharmacetutical Excipients, 1986, American Pharmaceutical Association, Washington, DC, and the Pharmaceutical Society of Great Britain, London, UK, which are incorporated herein by reference. Gelling agents may also be added to compositions according to this invention. Polyacrylic acid derivatives, such as carbomers, are examples of gelling agents, and more particularly, various types of carbopol, which are typically used in amounts from about 0.2% to about 2%. Suspensions may be prepared as a cream, an ointment, including a water-free ointment, a water-in-oil emulsion, an oil-in-water emulsion, an emulsion gel, or a gel.

It is anticipated that the compounds of the invention can be administered by oral or parenteral routes, including intravenous, intramuscular, intraperitoneal, subcutaneous, rectal, and topical administration, and inhalation. For oral administration, the compounds of the invention will generally be provided in the form of tablets, capsules, or as a solution or suspension.

"Therapeutically effective amount" or "effective amount" and grammatically related terms mean that amount of active compound or pharmaceutical agent that elicits the biological or medicinal response in a subject that is being sought by a researcher, veterinarian, medical doctor, or other clinician, which includes alleviation of the symptoms of the disease or disorder being treated. "Subject" or "patient" includes mammals such as human beings and animals (e.g., dogs, cats, horses, rats, rabbits, mice, non-human primates) in need of observation, experiment, treatment or prevention in connection with the relevant disease or condition. Preferably, the patient or subject is a human being.

Effective doses of the compounds of the present invention may be ascertained by conventional methods. The specific dosage level required for any particular patient will depend on a number of factors, including severity of the condition, the route of administration, and the weight of the patient.

In general, it is anticipated that the daily dose (whether administered as a single dose or as divided doses) will be in the range from about 0.01 mg to about 1000 mg per day, more usually from about 1 mg to about 500 mg per day, and most usually form about 10 mg to about 200 mg per day. Expressed as dosage per unit body weight, a typical dose will be expected to be between about 0.0001 mg/kg and about 15 mg/kg, especially between about 0.01 mg/kg and about 7 mg/kg, and most especially between about 0.15 mg/kg and 2.5 mg/kg.

Anticipated oral dose ranges include from about 0.01 to 500 mg/kg, daily, more preferably from about 0.05 to about 100 mg/kg, taken in 1-4 separate doses. Some compounds of the invention may be orally dosed in the range of about 0.05 to about 50 mg/kg daily, while others may be dosed at 0.05 to about 20 mg/kg daily. Infusion doses can range from about 1.0 to about 1.0 × 10⁴ µg/(kg.min) of inhibitor, admixed with a pharmaceutical carrier over a period ranging from several minutes to several days. For topical administration, compounds of the present invention may be mixed with a pharmaceutical carrier at a concentration from about 0.1 to about 10% of drug to vehicle.

Use or the compounds of invention for the manufactured medicaments for treating inflammation in a patient exhibiting or susceptible to an inflammatory condition is also contemplated. Use or the compounds of invention for the manufactured medicaments [ for treating an LTA4H-mediated condition is also contemplated.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value. Whenever a yield is given as a percentage, such yield refers to a mass of the entity for which the yield is given with respect to the maximum amount of the same entity that could be obtained under the particular stoichiometric conditions. Concentrations that are given as percentages refer to mass ratios, unless indicated differently.

### EXAMPLES

### General Experimental Procedures:

NMR spectra were obtained on either a Bruker model DPX400 (400 MHz) or DPX500 (500 MHz) spectrometer. The format of the ¹H NMR data below is: chemical shift in ppm down field of the tetramethylsilane reference (multiplicity, coupling constant *J* in Hz, integration).

Mass spectra were obtained on an Agilent series 1100 MSD using electrospray ionization (ESI) in either positive or negative mode as indicated. The "mass calculated" for a molecular formula is the monoisotopic mass of the compound.

Reversed-Phase HPLC retention times are reported in minutes, using the methods and conditions reported below.

| | |
|---|---|
| Instrument: | Gilson 215 |
| Solvent: | CH₃CN (0.05% trifluoroacetic acid, TFA)/H₂O (0.05% TFA) |
| Flow rate: | 25 mL/min |
| Gradient: | 0 min at 10% CH₃CN; 20 min linear ramp to 99% CH₃CN; |
| Column: | YMC-Pack ODS-A AA 12505-1530WT SH-362-5 |
| | (S-5 um, 12 nM, 150x30 mm) |
| Temperature: | 25 °C |
| Wavelength: | Dual detection at 220 and 254 nM |

Flash column chromatography was accomplished using ISCO Foxy 200 or ISCO OPTIX 10X systems employing one of the following commercially available prepacked columns: Biotage 40S (SiO₂ 40 g), Biotage 40M (SiO₂ 90 g), Biotage 40L (SiO₂ 120 g), Biotage 65M (SiO₂ 300 g) or ISCO Redisep (SiO₂, 10 g, 12 g, 35 g, 40 g, or 120 g).

### EXAMPLE 1

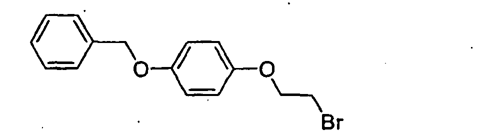

2-(4-Benzyloxy-phenoxy)-ethyl bromide.

To a stirring solution of 4-benzyloxyphenol (72 g, 359.6 mmol) in CH₃CN (600 mL) was added dibromoethane (155 mL, 1.80 mol) and K₂CO₃ (105 g, 759.9 mmol). This brown suspension was heated at reflux and allowed to stir for 96 h. The resulting suspension was cooled to room temperature, diluted with acetone (250 mL), and filtered through diatomaceous earth, which was then rinsed with additional acetone. The filtrate was concentrated under reduced pressure. The resulting oil was dissolved in CH₃OH (500 mL), and the solution was stirred for 2 h. The title compound was obtained by filtration and air-dried to give 70 g (228 mmol), 63% yield) as a tan solid. ¹H NMR (400 MHz, CDCl₃): 7.60-7.30 (m, 5H), 6.88 (d, *J* = 8.4, 2H), 6.80 (d, *J* = 8.4, 2H), 4.70 (s, 2H), 3.79 (t, *J* = 5.8, 2H), 3.07 (t, *J* = 5.8, 2H).

### EXAMPLE 2

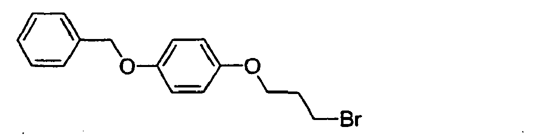

1-[3-(4-Benzyloxy-phenoxy)-propyl]-bromide.

To a stirring solution of 4-benzyloxyphenol (25 g, 124.9 mmol) in CH₃CN (125 mL) was added dibromopropane (63 mL, 624 mmol) and K₂CO₃ (34.5 g, 250 mmol). This brown suspension was heated at reflux and allowed to stir for 66 h. The suspension was then cooled to room temperature and filtered twice through diatomaceous earth pads. The pads were rinsed with CH₃CN, and the combined filtrates were concentrated under reduced pressure. The resultant oil was purified on SiO₂ (300 g; 33% CH₂Cl₂/hexanes) to give 35.4 g (110 mmol, 88% yield) of a brown solid. ¹H NMR (400 MHz, CDCl₃): 7.46-7.29 (m, 5H), 6.85 (q, *J* = 8.1, 2H), 6.82 (q, *J*= 7.2, 2H), 5.03 (s, 2H), 4.06 (t, *J* =5.8, 2H), 3.61 (t, *J* = 6.5, 2H), 2.39 (m, *J* = 6.2, 2H).

### EXAMPLE 3

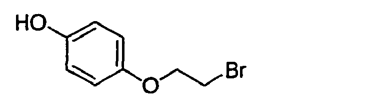

4-(2-Bromo-ethoxy)-phenol.

2-(4-Benzyloxy-phenoxy)-ethyl bromide (EXAMPLE 1; 70 g, 227 mmol) was dissolved in THF (500 mL). To this solution was added Pd on carbon (10 wt %, 7 g) as a suspension in ethanol (50 mL). The resulting suspension was placed on a Parr hydrogenator at 40 psi of H₂ and shaken overnight. The reaction mixture was filtered through a pad of diatomaceous earth, and the filtrate was concentrated under reduced pressure to give 48.5 g (224 mmol, 99% yield) of a tan solid. ¹H NMR (400 MHz, CDCl₃): 6.83 (d, *J* = 9.1, 2H), 6.77 (d, *J* = 9.1, 2H), 4.51 (s, 1 H), 4.24 (t, *J* = 6.3, 2H), 3.62 (t, *J* = 6.3, 2H).

### EXAMPLE 4

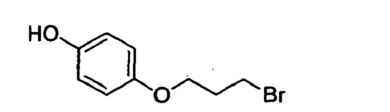

4-(3-Bromo-propoxy)-phenol.

[3-(4-Benzyloxy-phenoxy-propyl]-bromide (10 g, 31.1 mmol) was dissolved in THF (100 mL). To this solution was added 10% palladium on carbon (1 g) as a suspension in THF (20 mL). The resulting suspension was placed on a Parr hydrogenator at 40 psi of H₂, and shaken overnight. The reaction mixture was filtered through a pad of diatomaceous earth, and the filtrate was concentrated under reduced pressure to give 7 g (30.5 mmol, 98% yield) of a tan solid. ¹H NMR: (400 MHz, CDCl₃) 6.76 (d, *J* = 9.1, 2H), 6.69 (d, 9.1, 2H), 4.00 (t, *J* = 5.9, 2H), 3.60 (t, *J* = 6.6, 2H), 2.23 (m, *J* = 6.1, 2H).

### EXAMPLE 5

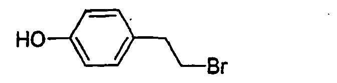

4-(2-Bromo-ethyl)-phenol.

4-(2-Hydroxy-ethyl)-phenol (50 g, 362 mmol) was dissolved in 48 wt % HBr (250 mL). This light yellow solution was heated to 80 °C and stirred for 16 h. The reaction mixture was allowed to cool to room temperature and was then extracted with CH₂Cl₂ (3 x 50 mL). The combined extracts were dried, filtered, and concentrated under reduced pressure to afford 72 g (100% crude yield) of a tan solid. ¹H NMR (400 MHz, CDCl₃): 9.25 (s, 1H), 7.04 (d, *J* = 8.4,2H), 6.67 (d, *J* = 8.4, 2H), 3.62 (t, *J* = 7.4, 2H), 2.97 (t, *J* = 7.4, 2H).

### EXAMPLE 6

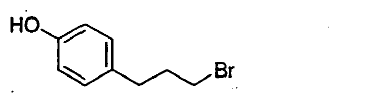

4-(3-bromo-propyl)-phenol.

A mixture of 4-(3-hydroxy-propyl)-phenol (52.7 g, 346.3 mmol) in 48 wt % HBr (265 mL) was stirred at 80 °C for 20 h and then cooled to room temperature. Water (400 mL) was added, and the product was extracted with CH₂Cl₂ (500 mL). The extract was dried (MgSO₄) and concentrated under reduced pressure to give the desired product as a beige solid (69 g, 92% yield). TLC (SiO₂, CH₂Cl₂): R_{f} = 0.37. ¹H NMR (400 MHz, DMSO-*d*₆): 9.18 (s, 1H), 6.99 (d, *J* = 8.3, 2H), 6.67 (d, *J* = 8.4, 2H), 3.47 (t, *J* = 6.6, 2H), 2.58 (t, *J* = 7.2, 2H), 2.05-1.95 (m, 2H).

### EXAMPLE 7

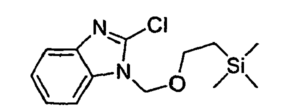

2-Chloro-1-(2-trimethylsilanyl-ethoxymethyl)-1*H*-benzoimidazole.

To a suspension of sodium hydride (6.2 g, 245 mmol) in DMF (275 mL) at 5 °C was added 2-chlorobenzimidazole (37 g, 243 mmol) via a solid-addition funnel over 30 min while maintaining the internal temperature of the mixture below 10 °C. An additional 25 mL of DMF was added, and the ice bath was removed. After 2 h, 2-(trimethylsilyl)ethoxymethyl chloride (SEM-CI) was added dropwise over 5 min. A white precipitate formed. The reaction mixture was stirred at room temperature for 18 h. To the mixture was added H₂O (500 mL) and ethyl acetate (750 mL). The organic layer was washed with additional H₂O (500 mL), dried (MgSO₄), and concentrated under reduced pressure, giving 65.8 g (96% yield) of the desired product as a clear golden oil, which solidified upon standing to give a beige solid. TLC (SiO₂, 5% acetone/CH₂Cl₂): R_{f} = 0.64. MS (ESI): mass calculated for C₁₃H₁₉ClN₂OSi, 282.10; m/z found, 283.1. ¹H NMR (400 MHz, CDCl₃): 7.70 (d, *J* = 7.3, 1 H), 7.46 (d, *J* = 7.6, 1H), 7.40-7.25 (m, 2H), 3.58 (t, *J* = 7.9, 2H), 0.92 (t, *J* = 8.3, 2H), 0.04 (s, 9H).

### EXAMPLE 8

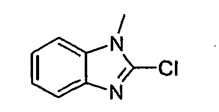

2-Chloro-1-methyl-1*H*-benzoimidazole.

Dimethyl sulfate (11.0 mL, 116 mmol) was added to a solution of 2-chlorobenzimidazole (10.6 g, 70 mmol) in 2.5 M NaOH (70 mL, 175 mmol), and the mixture was stirred at 23 °C for 2 h. The reaction mixture was filtered, and the solid product was washed with H₂O (6 x 50 mL) and dried in vacuo to afford a light brown solid (9.4 g, 81 % yield). MS (ESI): mass calculated for C₈H₇ClN₂, 166.03; m/z found, 167.0 [M+H]⁺. ¹H NMR (400 MHz, CDCl₃): 7.75-7.70 (m, 1H), 7.35-7.29 (m, 3H), 3.82 (s, 3H).

### EXAMPLE 9

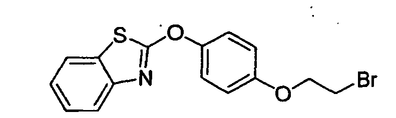

2-[4-(2-Bromo-ethoxy)-phenoxy]-benzothiazole.
A solution of 4-(2-bromo-ethoxy)-phenol (EXAMPLE 3; 8.7 g, 40.1 mmol) and 2-chlorobenzothiazole (12 mL, 92 mmol) in CH₃CN was treated with finely powdered Cs₂CO₃ (26 g, 80 mmol), and the resulting mixture was stirred at 23 °C for 30 h. The reaction mixture was filtered through diatomaceous earth, and the filtrate was concentrated under reduced pressure. The crude solid was purified on SiO₂ (100 g; 0-40% ethyl acetate/hexanes) to provide 6.7 g (47% yield) of a white solid. MS (ESI): mass calculated for C₁₅H₁₂BrNO₂S, 348.98; m/z found, 350.0 [M+H]⁺. ¹H NMR (400 MHz, CDCl₃): 7.78, (dd, *J* = 8.0, 0.4, 1H), 7.70 (dd, *J* = 8.0, 0.7, 1H), 7.42 (dt, *J* = 7.5, 1.3, 1H), 7.34 (dd, *J* = 9.1, 2H), 7.01 (dd, *J* = 9.1, 2H), 4.34 (t, *J* = 6.2, 2H), 3.70 (t, *J* = 6.2, 2H).

### EXAMPLE 10

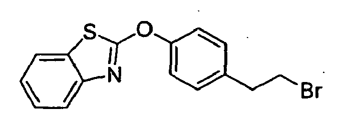

2-[4-(2-Bromo-ethyl)-phenoxy]-benzothiazole.
A. 2-[4-(Benzothiazol-2-yloxy)-phenyl]-ethanol. A solution of 4-hydroxyphenethyl alcohol (867 mg, 6.3 mmol) and 2-chlorobenzothiazole 0.82 mL, 6.3 mmol) in CH₃CN was treated with finely powdered Cs₂CO₃ (4.1 g, 12.5 mmol), and the resulting suspension was stirred for 40 h at 70 °C. The reaction mixture was filtered through diatomaceous earth, and the filtrate was concentrated to a crude oil, which was purified on SiO₂ (40 g; 0-50% ethyl acetate/hexanes) to provide 940 mg (55% yield) of a clear oil. MS (ESI): mass calculated for C₁₅H₁₃NO₂S, 271.07; m/z found, 272.2 [M+H]⁺. ¹H NMR (400 MHz, CDCl₃): 7.78 (dd, *J* = 7.9, 0.4, 1 H), 7.75 (dd, *J* = 7.9, 0.7, 1 H), 7.46 (dt, *J* = 7.4, 1.3, 1 H), 7.38-7.29 (m, 3H), 3.93 (q, *J* = 6.4, 2H), 2.94, (t, *J* = 6.5, 2H), 1.50 (m, 1H).
B. 2-[4-(2-Bromo-ethyl)-phenoxy]-benzothiazole. A solution of 2-[4-(benzothiazol-2-yloxy)-phenyl]-ethanol (174 mg, 0.64 mmol) in benzene (3 mL) was treated with PBr₃ (0.060 mL, 0.64 mmol), and the resulting suspension was heated to 70 °C for 90 min. The reaction mixture was cooled and diluted with ethyl acetate (30 mL). This solution was washed with H₂O (10mL) then brine (10 mL), dried, and concentrated under reduced pressure. The crude product was purified on SiO₂ (12 g; 0-50% ethyl acetate/hexanes) to provide 120 mg of a light yellow oil. MS (ESI): mass calculated for C₁₅H₁₂BrNOS, 332.98; m/z found, 335.2 [M+H)⁺. ¹H NMR (400 MHz, CDCl₃): 7.79 (dd, *J* = 8.0, 0.3, 1 H), 7.76 (dd, *J* = 8.0, 0.6, 1H), 7.42 (dt, *J* = 7.4, 1.2, 1 H), 7.38-7.29 (m, 3H), 3.62 (t, *J* = 7.5, 2H), 3.25 (t, *J* =7.5, 2H).

### EXAMPLE 11

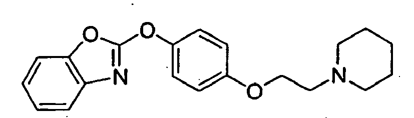

2-[4-(2-Piperidin-1-yl-ethoxy)-phenoxy]-benzooxazole.
A. 1-[2-(4-Benzyloxy-phenoxy)-ethyl]-piperidine. To a mixture of 4-(benzyloxy)phenol (24.6 g, 123 mmol) and 1-(2-chloroethyl)piperidine hydrochloride (20.6 g, 112 mmol) in DMF (175 mL) was added K₂CO₃ (25 g,181 mmol) and Cs₂CO₃ (40 g, 123 mmol). The reaction mixture was stirred for 3 days at room temperature. To the mixture was added H₂O (300 mL) and CH₂Cl₂. The organic layer was washed with 10% NaOH then brine, dried (MgSO₄), filtered, and concentrated under reduced pressure to give 33 g of a clear, dark purple liquid. The liquid was purified on SiO₂ (300 g; 0-50% ethyl acetate/hexanes) to give 23.4 g (67% yield) of a light yellow solid. TLC (SiO₂, 50% hexanes/ethyl acetate): R_{f} = 0.11. MS (ESI): mass calculated for C₂₀H₂₅NO₂, 311.19; m/z found, 312.2 [M+H]⁺. ¹H NMR (400 MHz, CDCl₃): 7.50-7.26 (m, 5H), 6.91 (d, *J* = 9.2, 2H,), 6.85 (d, *J* = 9.2, 2H), 5.02 (s, 2H), 4.06 (t, *J* = 6.1, 2H), 2.76 (t, *J* = 6.1, 2H), 2.51 (br s, 4H), 1.65-1.55 (m, 4H), 1.45 (br s, 2H).
B. 4-(2-Piperidin-1-yl-ethoxy)-phenol. To a solution of 1-[2-(4-benzyloxy-phenoxy)-ethyl]-piperidine (15.0 g, 48.2 mmol) in 1:1 ethanol/ethyl acetate (400 mL) was added Pd on carbon (10 wt %, 1.5 g). The mixture was placed on a Parr hydrogenator at 40 psi of H₂ for 20 h. The reaction mixture was filtered through diatomaceous earth, and the filtrate was concentrated under reduced pressure to give 9.4 g (88% yield) of the desired product as a light gray solid. TLC (SiO₂, 50% acetone/CH₂Cl₂): R_{f} = 0.16. MS (ESI): mass calculated for C₁₃H₁₉NO₂, 221.14; m/z found, 222.1 [M+H]⁺. ¹H NMR (400 MHz, DMSO-*d*₆): 8.88 (s, 1 H), 6.73 (d, *J* = 6.6, 2H), 6.65 (d, *J* = 6.6, 2H), 3.93 (t, *J* = 6.0, 2H), 2.58 (t, *J* = 6.0, 2H), 2.40 (s, 4H), 1.51-1.45 (m, 4H), 1.35 (br s, 2H).
C. 2-[4-(2-Piperidin-1-yl-ethoxy)-phenoxy] benzooxazole. To a stirred solution of 4-(2-piperidin-1-yl-ethoxy)-phenol (1.5 g, 6.8 mmol) in acetone (20 mL) at 5 °C was added K₂CO₃ (1.0 g, 7.2 mmol). To the mixture was added 2-chloro-benzooxazole (0.5 mL, 4.4 mmol) at 5 °C. The resulting mixture was warmed to room termperature overnight. After 20 h the mixture was filtered, and the filtrate was concentrated under reduced pressure to a brown solid, which was purified on SiO₂ (35 g; 50% acetone/CH₂Cl₂). The desired fractions were combined and concentrated under reduced pressure to give 1.2 g (80% yield) of the desired product as a white solid. TLC (SiO₂, 50% acetone/CH₂Cl₂): R_{f} = 0.18. MS (ESI): mass calculated for C₂₀H₂₂N₂O₃, 338.16; m/z found, 339.1 [M+H]⁺. ¹H NMR (400 MHz, CDCl₃): 7.52 (d, *J* =7.2, 1H), 7.41 (d, *J* = 7.2, 1 H), 7.35-7.20 (m, 4H), 6.97 (d, *J* = 9.1, 2H), 4.12 (t, *J* = 6.1, 2H), 2.79 (t, *J* = 6.0, 2H), 2.52 (s, 4H), 1.67-1.55 (m, 4H), 1.50-1.40 (m, 2H).

### EXAMPLE 12

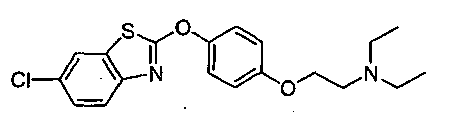

{2-[4-(6-Chloro-benzothiazol-2-yloxy)-phenoxy]-ethyl}-diethyl-amine.
A. [2-(4-Benzyloxy-phenoxy)-ethyl]-diethyl-amine. To a mixture of 4-(benzyloxy)phenol (51 g, 255 mmol) and (2-chloro-ethyl)-diethyl-amine hydrochloride (41.6 g, 242 mmol) in DMF (400 mL) was added K₂CO₃ (37 g, 268 mmol) and Cs₂CO₃ (87 g, 267 mmol). The reaction mixture was stirred at room temperature for 17 days. To the mixture was added H₂O (600 mL) and CH₂Cl₂. The organic layer was washed with H₂O, dried (MgSO₄), filtered, and concentrated under reduced pressure to give 33 g of a clear, dark purple liquid, which was purified on SiO₂ (300 g; ethyl acetate) to give a light yellow oil (34.5 g, 48% yield). TLC (SiO₂, 50% hexanes/ethyl acetate): R_{f} = 0.10. MS (ESI): mass calculated for C₁₉H₂₅NO₂, 299.19; m/z found, 300.2 [M+H]⁺. ¹H NMR (400 MHz, CDCl₃): 7.50-7.28 (m, 5H), 6.89 (d, *J* = 9.2, 2H), 6.85 (d, *J* = 9.2, 2H), 5.02 (s, 2H), 4.00 (t, *J* = 6.4, 2H), 2.86 (t, *J* = 6.4, 2H), 2.63 (q, *J* = 7.2, 4H), 1.08 (t, *J* = 7.1, 6H).
B. 4-(2-Diethylamino-ethoxy)-phenol. To a solution of [2-(4-benzyloxy-phenoxy)-ethyl]-diethyl-amine (21 g, 70 mmol) in 1:1 ethanol/ethyl acetate (500 mL) was added Pd on carbon (10 wt %, 1.5 g). The mixture was placed on a Parr hydrogenator at 40 psi of H₂ for 20 h. The reaction mixture was filtered through diatomaceous earth, and the filtrate was concentrated under reduced pressure to give 13.1 g (89% yield) of the desired product as a clear brown oil. MS (ESI): mass calculated for C₁₂H₁₉NO₂, 209.14; m/z found, 210.2 [M+H]⁺. ¹H NMR (400 MHz, CDCl₃): 6.68 (s, 4H,), 3.99 (t, *J* = 6.2, 2H), 2.88 (t, J = 6.2, 2H), 2.69 (q, J = 7.2, 4H), 1.09 (t, J = 7.1, 6H).
C. {2-[4-(6-Chloro-benzothiazol-2-yloxy)-phenoxy]-ethyl}-dimethyl-amine. To a solution of 4-(2-diethylamino-ethoxy)-phenol (500 mg, 2.39 mmol) in acetone (7 mL) containing Cs₂CO₃ (876 mg, 2.69 mmol) was added 2,6-dichlorobenzthiazole (365 mg, 1.79 mmol). The mixture was heated at reflux for 3 days. The reaction mixture was filtered, and the filtrate was concentrated under reduced pressure to give a brown oil, which was purified on SiO₂ (35 g; acetone) to give 624 mg (76% yield) of the desired product. TLC (SiO₂, acetone): R_{f} = 0.23. MS (ESI): mass found for C₁₉H₂₁CIN₂O₂S, 376.10; m/z found, 377.1 [M+H]⁺. ¹H NMR (400 MHz, CDCl₃): 7.64 (d, *J* = 8.5, 2H), 7.34 (d, *J* = 8.8, 1H); 7.25 (d, *J* = 6.8, 2H), 6.96 (d, *J* = 9.1, 2H).

### EXAMPLE 13

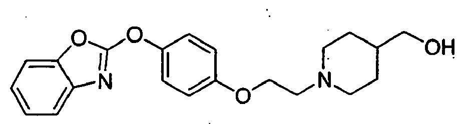

(1-{2-[4-(Benzooxazol-2-yloxy)-phenoxy]ethyl}-piperidin-4-yl)-methanol.
A. 4-[2-(4-Hydroxymethyl-piperidin-1-yl)-ethoxyl-phenol. A solution of 4-(2-bromo-ethoxy)-phenol (EXAMPLE 3; 7 g, 32.2 mmol), piperidinemethanol (5.2 g, 45.3 mmol), and *N,N*-diisopropylethylamine (7.9 mL, 45.3 mmol) in CH₃CN (100 mL) was stirred at 65 °C for 18 h. The reaction mixture was cooled to room temperature and stirred an additional 48 h. The solvent was removed under reduced pressure to yield a black semi-solid. This material was dissolved in CH₂Cl₂ (200 mL), and the solution was washed with H₂O (2 x 50 mL). The aqueous phase was back-extracted with 10% CH₃OH/CH₂Cl₂ (100 mL). The organic layers were combined, dried (Na₂SO₄), filtered, and concentrated under reduced pressure to a black oil, which was purified on SiO₂ (120 g; 0-100% acetone/CH₂Cl₂) to give the desired product as a brown oil. Diethyl ether was added to the oil to precipitate the product. Filtration yielded the title compound as,a tan solid (1.9 g, 23% yield). TLC (SiO₂, 5% 2 M NH₃ in CH₃OH/CH₂Cl₂): R_{f} = 0.09. MS (ESI): mass calculated for C₁₄H₂₁NO₃, 251.15; m/z found, 252.3 [M+H]⁺. ¹H NMR (400 MHz, CDCl₃): 6.69 (s, 4H), 4.03 (t, *J* = 5.9, 2H,), 3.51 (d, *J* = 6.5, 2H), 3.09 (d, *J* = 11.6, 2H), 2.79 (t, *J* = 5.9, 2H), 2.18-2.11 (m, 2H), 1.79 (d, *J* = 16.2, 2H), 1.49-1.62 (m, 1H), 1.36 (dq, *J* = 3.6, 12.3, 2H), 1.23 (br s, 2H).
B. (1-{2-[4-Benzooxazol-2-loxy)-phenoxy]ethyl}-piperidin-4-yl)-methanol. A mixture of 4-[2-(4-hydroxymethyl-piperidin-1-yl)-ethoxy]-phenol (0.5 g, 1.98 mmol), 2-chloro-benzooxazole (205 µL, 1.8 mmol) and Cs₂CO₃ (1.35 g, 4.15 mmol) in acetone (8.0 mL) was stirred at room temperature for 48 h. The resulting mixture was filtered through diatomaceous earth, and the pad was washed with CH₂Cl₂. The combined filtrates were concentrated under reduced pressure to a yellow oil. The oil was purified on SiO₂ (40 g; 0-100% acetone/CH₂Cl₂) to provide a solid, which was dissolved in CH₂Cl₂ (100 mL). This solution was washed with 1 N NaOH (3 x 5 mL) then H₂O (5 mL), dried (Na₂SO₄), filtered, and concentrated under reduced pressure to yield the title compound as a white solid (424 mg, 64% yield). TLC (SiO₂, 5% 2 M NH₃ in CH₃OH/CH₂Cl₂): R_{f} = 0.17. MS (ESI): mass calculated for C₂₁H₂₄N₂O₄, 368.17; m/z found, 369.3 [M+H]⁺. ¹H NMR (400 MHz, CDCl₃): 7.53-7.50 (m, 1H), 7.44-7.42 (m, 1H), 7.34-7.21 (m, 4H), 7.00-6.96 (m, 2H), 4.13 (t, *J* = 6.0, 2H,), 3.55-3.48 (m, 2H), 3.02-3.09 (m, 2H), 2.82 (t, *J* = 6.0, 2H), 2.13 (dt, *J* = 2.4, 11.8, 2H), 1.78-1.75 (m, 2H), 1.59-1.48 (m, 2H), 1.33 (dq, *J* = 3.7, 12.4, 2H).

### EXAMPLE 14

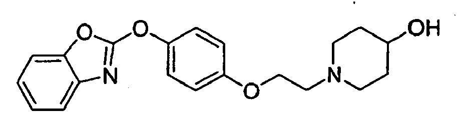

1-{2-[4-(Benzooxazol-2-yloxy)-phenoxy]-ethyl}-piperidin-4-ol.
A. 1-[2-(4-Hydroxy-phenoxy)-ethyl]-piperidin-4-ol. To a stirring solution of 4-(2-bromo-ethoxy)-phenol (EXAMPLE 3; 9 g, 42 mmol) in CH₃CN (150 mL) was added 4-hydroxypiperidine (5.3 g, 52.5 mmol), followed by *N,N-*diisopropylethylamine (6.7 g, 52.5 mmol). The resulting solution was stirred overnight at room temperature, yielding a suspension. The suspension was filtered, and the filtrate was concentrated under reduced pressure. Diethyl ether was added to the resultant oil, and the mixture was warmed to 45 °C for 2 min, forming a white precipitate. This suspension was stirred at room temperature for 2 h, then filtered, giving 7.9 g (33 mmol, 79% yield) of an off-white solid. MS (ESI): mass calculated for C₁₃H₁₉NO₃, 237.14; m/z found, 238.2 [M+H]⁺¹. ¹H NMR (400 MHz, CD₃OD): 7.10 and 7.02 (q, *J* = 32.3, 9.0, 2H), 4.35 (t, *J* = 5.7, 2H), 3.59 (m, 1H), 3.26-3.19 (m, 2H), 3:07 (t, *J* = 5.7, 2H), 2.60 (t, *J* = 9.9, 2H), 2.20-2.12 (m, 2H), 1.94-1.82 (m, 2H).
B. 1-{2-[4-(Benzooxazol-2-yloxy)-phenoxy]-ethyl}-piperidin-4-ol. To a stirring solution of 1-[2-(4-hydroxy-phenoxy)-ethyl]-piperidin-4-ol (500 mg, 2.1 mmol) in acetone (10 mL) was added Cs₂CO₃ (1.4 g, 4.41 mmol). This suspension was cooled to 0 °C, and 2-chloro-benzooxazole (388 mg, 2.5 mmol, 0.29 mL) was added dropwise. The reaction mixture was allowed to warm to room temperature overnight, and was then filtered, and concentrated under reduced pressure. The resultant oil was purified on SiO₂ (40 g; 0-100% acetone/CH₂Cl₂) to give 400 mg (1.1 mmol, 54% yield) of a white solid. MS (ESI): mass calculated for C₂₀H₂₂N₂O₄, 354.16; m/z found, 355.2 [M+H]⁺¹. ¹H NMR: (400 MHz, CDCl₃) 7.55 (dd, *J* = 7.2, 1.8, 1H), 7.46 (dd, *J* = 7.3, 2.0, 1H), 7.36 (d, *J* = 9.1, 2H), 7.32-7.25 (m, 2H), 7.01 (d, *J* = 9.1, 2H), 4.18 (t, *J* = 5.4, 2H), 3.80 (m, 1H), 3.01-2.86 (m, 4H), 2.40 (br s, 1H), 1.99 (m, 2H), 1.74-1.65 (m, 2H), 1.48 (d, *J* = 4.1, 1H).

### EXAMPLE 15

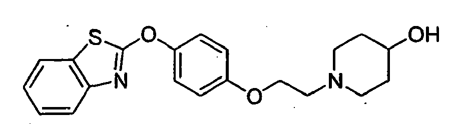

1-{2-[4-(Benzothiazol-2-yloxy)-phenoxy]-ethyl}-piperidin-4-ol.

To a stirring solution of 1-[2-(4-hydroxy-phenoxy)-ethyl]-piperidin-4-ol (EXAMPLE 14, step A; 500 mg, 1.25 mmol) in DMF (10 mL), was added Cs₂CO₃ (1.4 g, 4.41 mmol) and 2-chlorobenzothiazole (0.33 mL, 2.5 mmol). The suspension was heated to 80 °C and stirred overnight. The reaction mixture was allowed to cool to room temperature and then filtered through diatomaceous earth. The filtrate was concentrated under reduced pressure, and the residue was purified on SiO₂ (40 g; 0-100% acetone/CH₂Cl₂), giving 321 mg (0.86 mmol, 69% yield) of a tan solid. MS (ESI): mass calculated for C₂₀H₂₂N₂O₃S, 370.14; m/z found, 371.2 [M+H]⁺. ¹H NMR (400 MHz, CDCl₃): 7.64 (d, *J* = 8.0, 1H), 7.56 (d, *J* = 7.8, 1H), 7.30 (t, *J* = 7.2, 1H), 7.21-7.14 (m, 3H), 6.87 (d, *J* = 9.1, 2H), 4.05 (t, *J* = 5.8, 2H), 3.67 (br s, 1H), 2.82 (m, 2H), 2.76 (t, *J* = 5.8, 2H), 2.27 (t, *J* = 9.5, 2H), 2.01 (br s, 1H), 1.90-1.82 (m, 2H), 1.64-1.52 (m, 2H).

### EXAMPLE 16

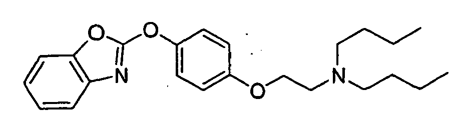

{2-[4-(Benzooxazol-2-yloxy)-phenoxy]-ethyl}-dibutyl-amine.
A. 4-(2-Dibutylamino-ethoxy)-phenol. To a stirring solution of 4-(2-bromo-ethoxy)-phenol (EXAMPLE 3; 8.2 g, 37 mmol) in CH₃CN (150 mL), was added dibutylamine (5.98 g, 46.3 mmol) and *N,N*-diisopropylethylamine (5.98 g, 46.3 mmol). The mixture was stirred overnight at 75 °C. The resulting suspension was filtered, and the filtrate was concentrated under reduced pressure. The resultant oil was purified on SiO₂ (110 g; 0-100% acetone/CH₂Cl₂) to give 7.7 g (29 mmol, 78% yield) of a brown solid. MS (ESI): mass calculated for C₁₆H₂₇NO₂, 265.2; m/z found, 266.2 [M+H]⁺. ¹H NMR (400 MHz, CDCl₃): 6.81 (d, *J* = 7.0, 2H), 6.63 (d, *J* = 6.0, 2H), 4.22 (br s, 2H), 3.25 (br s, 2H), 2.93 (br s,' 4H), 2.16 (br s, 2H), 1.88 (br s, 1H), 1.68 (br s, 4H), 1.33 (d, *J* = 5.7, 4H), 0.94 (br s, 6H).
B. {2-[4-(Benzooxazol-2-yloxy)-phenoxy]-ethyl}-dibutyl-amine. To a stirring solution of 4-(2-dibutylamino-ethoxy)-phenol (500 mg, 1.9 mmol) in acetone (9.4 mL) was added Cs₂CO₃ (1.3 g, 3.9 mmol). This suspension was cooled to 0 °C, and 2-chloro-benzooxazole (346 mg, 2.2 mmol, 0.26 mL) was added dropwise. The reaction mixture was allowed to warm to room temperature overnight, then was filtered. The filtrate was concentrated under reduced pressure. The resultant oil was dissolved in CH₂Cl₂ and purified on SiO₂ (40 g; 0-100% acetone/CH₂Cl₂) to give 180 mg (0.47 mmol, 25% yield) of a white solid. MS (ESI): mass calculated for C₂₃H₃₀N₂O₃, 382.23; m/z found, 383.3 [M+H]⁺. ¹H NMR (400 MHz, CDCl₃): 7.50 (d, *J* = 7.2, 2H), 7.41 (d, *J* = 7.3, 2H), 7.31 (d, *J* = 9.1, 2H), 7.28-7.19 (m, 2H), 6.96 (d, *J* = 9.1, 2H), 4.13 (m, 2H), 2.88 (m, 2H), 2.54 (m, 4H), 1.47 (m, 4H), 1.32 (m, 4H), 0.92 (t, *J* = 7.3, 6H).

### EXAMPLE 17

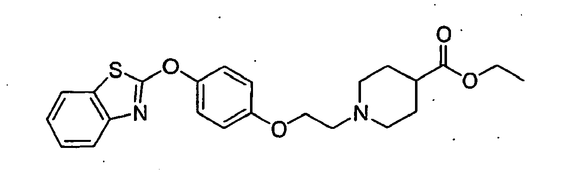

1-{2-[4-(Benzothiazol-2-yloxy)-phenoxy]-ethyl}-piperidine-4-carboxylic acid ethyl ester.
A. 1-[2-(4-Hydroxy-phenoxy)-ethyl]-piperidine-4-carboxylic acid ethyl ester. To a stirring solution of 4-(2-bromo-ethoxy)-phenol (EXAMPLE 3; 5 g, 23.1 mmol) in CH₃CN (200 mL) was added ethyl isonipecotate (5.3 mL, 34.7 mmol). The reaction mixture was heated to 84 °C and stirred for 16 h, then cooled to room temperature and concentrated under reduced pressure. The resultant oil was dissolved in CH₂Cl₂ and purified on SiO₂ (300 g; 0-25% acetone/CH₂Cl₂) to give a white solid (6.3 g, 93% yield). MS (ESI): mass calculated for C₁₆H₂₃NO₄, 293.16; m/z found, 294.3 [M+H]⁺. ¹H NMR (400 MHz, CDCl₃): 6.63 (m, 4H), 4.07 (q, *J* = 7.2, 2H), 3.96 (t, *J* = 5.7, 2H), 2.96 (m, 2H), 2.74 (t, *J* = 5.6, 2H), 2.26-2.23 (m, 3H), 1.88-1.77 (m, 5H), 1.17 (t, *J* = 7.2, 3H).
B. 1-{2-[4-(Benzothiazol-2-yloxy)-phenoxy]-ethyl}-piperidine-4-carboxylic acid ethyl ester. To a stirring solution of 1-[2-(4-hydroxy-phenoxy)-ethyl]-piperidine-4-carboxylic acid ethyl ester (2.5 g, 6.8 mmol) in CH₃CN (34 mL) was added Cs₂CO₃ (5 g, 14.3 mmol) and 2-chlorobenzothiazole (1.26 mL, 10.2 mmol). The reaction mixture was heated to 75 °C for 3 h, then cooled to room temperature and filtered. The filtrate was concentrated under reduced pressure. The residue was dissolved in CH₂Cl₂ and purified on SiO₂ (40 g; 0-25% acetone/CH₂Cl₂) to give a white solid (2.94 g, 100% yield). MS (ESI): mass calculated for C₂₃H₂₆N₂O₄S, 426.16; m/z found, 427.1 [M+H]⁺. ¹H NMR (400 MHz, CDCl₃): 7.76 (d, *J* = 8.0, 1H), 7.64 (t, *J* = 8.1, 1H), 7.41 (t, *J* = 8.4, 1H), 7.30 (d, *J* = 9.0, 3H), 7.05 (d, *J* = 6.8, 2H), 4.19 *(*t, J = 5.5, 2H), 4.12 (q, *J* = 7.1, 2H), 3.02 (m, 2H), 2.83 (t, *J* = 5.5, 2H), 2.37 (m, 1 H), 2.25 (t, *J* = 11.6, 2H), 1.93 (m, 2H), 1.79 (m, 2H), 1.24 (t, *J* = 7.2, 3H).

### EXAMPLE 18

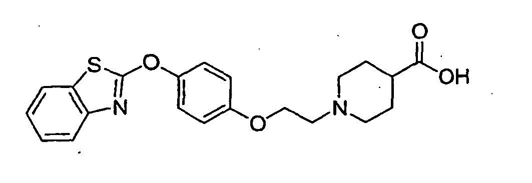

1-{2-[4-(Benzothiazol-2-yloxy)-phenoxy]-ethyl}-piperidine-4-carboxylic acid

To a stirring solution of 1-{2-[4-(benzothiazol-2-yloxy)-phenoxy]-ethyl}-piperidine-4-carboxylic acid ethyl ester (EXAMPLE 17; 235 mg, 0.5 mmol) in THF (2.5 mL) was added potassium trimethylsilanoate (282 mg, 2.2 mmol). The reaction mixture was stirred at room temperature for 2 h, then concentrated under reduced pressure. The resultant oil was dissolved in H₂O, and the solution was treated to pH 9 with 1 M HCl. The resulting solution was extracted with 1:3 isopropyl alcohol/chloroform (3 x 25 mL). The combined extracts were concentrated under reduced pressure to yield a tan solid that was triturated with diethyl ether. Filtration of the suspension afforded a tan solid (140 mg, 64% yield). MS (ESI): mass calculated for C₂₁H₂₂N₂O₄S.(free acid), 398.13; m/z found, 399.1 [M+H]⁺. ¹H NMR (400 MHz, DMSO-*d*₆): 8.01 (d, *J* = 7.3, 1H), 7.76 (d, *J* = 7.5, 1H), 7.51 (t, *J* = 8.4, 1 H), 7.45 (d, *J* = 9.0, 2H), 7.40 (t, *J* = 7.2, 1H), 7.14 (d, *J* = 9.0, 2H), 4.18 (t, *J* = 5.5, 2H), 2.95 (m; 2H), 2.74 (t, *J* = 5.8, 2H), 2.10 (t, *J* = 10.5, 2H), 1.81 (m, 2H), 1.59 (m, 2H).

### Example 19

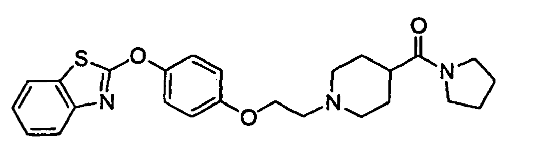

(1-{2-[4-(Benzothiazol-2-yloxy)-phenoxy]-ethyl}-piperidin-4-yl)-pyrrolidin-1-yl-methanone.

To a suspension of 1-{2-[4-(benzothiazol-2-yloxy)-phenoxy]-ethyl}-piperidine-4-carboxylic acid (EXAMPLE 18; 100 mg, 0.25 mmol) in CHCl₃ (2 mL) was added oxalyl chloride (132 µL, 1.5 mmol). The reaction mixture was stirred at room temperature for 2 h, and then concentrated under reduced pressure. The resultant solid was re-suspended in CHCl₃ (2 mL), and pyrrolidine was added (100 µL, 1.2 mmol). The solution that formed was stirred for 1 h and then was diluted with CH₂Cl₂ (10 mL) and washed with sat. aq. NaHCO₃ (10 mL). The organic layer was dried (Na₂SO₄), filtered, and concentrated under reduced pressure to a yellow oil. The crude oil was purified on SiO₂ (10 g; 0-100% acetone/CH₂Cl₂) to provide the desired product as a colorless oil (71 mg, 63% yield). MS (ESI): mass calculated for C₂₅H₂₉N₃O₃S, 451.19; m/z found, 452.1 [M+H]⁺. ¹H NMR (400 MHz, CDCl₃): 7.74 (d, *J* = 7.6, 1H), 7.66 (dd, *J* = 8.0, 0.8, 1H), 7.41-7.37 (m, 1H), 7.29-7.24 (m, 3H), 6.99-6.96 (m, 2H), 4.13 (t, *J* = 6.0, 2H), 3.50-3.45 (m, 4H), 3.10-3.04 (m, 2H), 2.83 (t, *J* = 6.0, 2H), 2.38-2.31 (m, 1H), 2.21-2.14 (m, 2H), 1.98-1.84 (m, 6H), 1.74-1.71 (m, 2H).

### Example 20

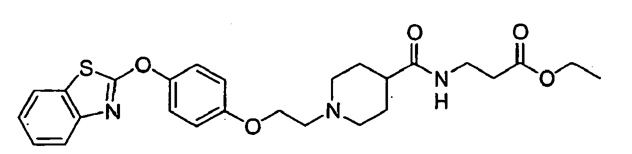

3-[(1-{2-[4-(Benzothiazol-2-yloxy)-phenoxy]-ethyl}-piperidine-4-carbonyl)-amino]-propionic acid ethyl ester.

1-Hydroxybenzotriazole hydrate (HOBT; 1.0 mL, 0.5 M in DMF, 0.5 mmol), 3-amino-propionic acid ethyl ester (120 mg, 0.785 mmol) and 1-(3-dimethylaminopropyl)-3-ethylcarbodiimide hydrochloride (EDCI; 150 m, 0.785 mmol) were added in succession at 5 min intervals to a stirred suspension of 1-{2-[4-(benzothiazol-2-yloxy)-phenoxy]-ethyl}-piperidine-4-carboxylic acid (EXAMPLE 18; 210 mg, 0.53 mmol) in CH₂Cl₂ (3 mL). The resulting mixture was stirred overnight at room temperature. The reaction mixture was diluted with CH₂Cl₂ (10 mL) and washed with sat. aq. NaHCO₃ (10 mL) then H₂O (10 mL). The organic layer was dried (Na₂SO₄), filtered, and concentrated under reduced pressure to yield an off-white semi-solid. The crude residue was purified on SiO₂ (35 g; 0-5% 2 M NH₃ in CH₃OH/CH₂Cl₂) to provide the desired product as a white solid (186 mg, 48% yield). MS (ESI): mass calculated for C₂₆H₃₁N₃O₅S, 497.20; m/z found, 498.4 [M+H]⁺. ¹H NMR (400 MHz, CDCl₃): 7.74 (dd, *J* = 8.1, 0.6, 1H), 7.66 (dd, *J* = 8.1, 0.8, 1H), 7.39 (dt, *J* = 7.4, 1.3, 1H), 7.29-7.24 (m, 3H), 7.01-6.94 (m, 2H), 6.18-6.15 (m, 1H), 4.19-4.11 (m, 4H), 3.53 (q, *J* = 6.0, 2H), 3.06-3.04 (m, 2H), 2.81 (t, *J* = 5.9, 2H), 2.53 (t, *J* = 6.0, 2H), 2.18-2.06 (m, 3H), 1.88-1.72 (m, 4H), 1.28 (t, *J* = 7.2, 3H).

### EXAMPLE 21

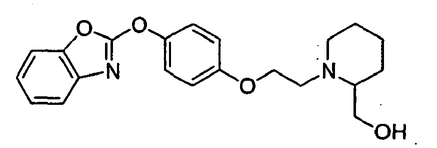

(1-{2-[4-(Benzooxazol-2-yloxy)-phenoxy]-ethyl}-piperidin-2-yl)-methanol.
A. {1-[2-(4-Benzyloxy-phenoxy)-ethyl]-piperidin-2-yl}-methanol. To a stirred solution of 2-(4-benzyloxy-phenoxy)-ethyl bromide (EXAMPLE 1; 7.0 g mg, 22.8 mmol) and piperidin-2-yl-methanol (3.3 g, 28.7 mmol) in CH₃CN (100 mL) was added K₂CO₃ (7.1 g, 51.4 mmol). The mixture was heated at reflux for 20 h and then filtered. The filtrate was concentrated under reduced pressure to a clear golden oil, which was purified on SiO₂ (120 g; 0-100% acetone/CH₂Cl₂) to give 6.0 g (77% yield) of a white solid. TLC (SiO₂, acetone): R_{f} = 0.15. MS (ESI): mass calculated for C₂₁H₂₇NO₃, 3.41.20; m/z found, 342.3 [M+H]⁺. ¹H NMR (400 MHz, DMSO-*d*₆): 7.48-7.25 (m, 5H), 6.92 (d, *J* = 9.1, 2H), 6.85 (d, *J* = 9.1, 2H), 5.02 (s, 2H), 4.38 (t, *J* = 5.3, 2H), 3.96 (t, *J* = 6.2, 2H), 3.55-3.48 (m, 1H), 3.43-3.32 (m, 1H), 3.15-3.00 (m, 1H), 2.88-2.85 (m, 1H), 2.75-2.62 (m, 1H), 2.30-2.22 (m, 2H), 1.61 (d, *J* = 9.4, 2H), 1.52-1.44 (m, 1H), 1.44-1.31 (m, 1H), 1.30-1.17 (m, 2H).
B. 4-[2-(2-Hydroxymethyl-piperidin-1-yl)-ethoxy]-phenol. To a solution of {1-[2-(4-benzyloxy-phenoxy)-ethyl]-piperidin-2-yl}-methanol (6.0 g, 17.6 mmol) in 1:1 ethanol/ethyl acetate (75 mL) was added Pd on carbon (10 wt %, 614 mg). The mixture was placed on a Parr hydrogenator at 40 psi of H₂ for 20 h. The reaction mixture was filtered through diatomaceous earth, and the filtrate was concentrated under reduced pressure to give 4.5 g (100% yield) of the desired product as a clear and colorless oil. TLC (SiO₂, acetone): R_{f} = 0.29. MS (ESI): mass calculated for C₁₄H₂₁NO₃, 251.15; m/z found, 252.3 [M+H]⁺. ¹H NMR (400 MHz, CD₃OD): 6.77 (d, *J* = 6.6, 2H), 6.68 (d, *J* = 6.6, 2H), 4.06-4.01 (m, 2H), 3.70-3.55 (m, 2H), 3.22-3.10 (m, 1H), 3.05-2.96 (m, 1H), 2.88-2.79 (m, 1H), 2.47-2.36 (m, 2H), 1.76-1.70 (m, 2H), 1.63-1.32 (m, 4H).
C. (1-{2-[4-(Benzooxazol-2-yloxy)-phenoxy]-ethyl}-piperidin-2-yl)-methanol. A mixture of 4-[2-(2-hydroxymethyl-piperidin-1-yl)-ethoxy]-phenol (197 mg, 0.78 mmol), 2-chloro-benzooxazole (116 µL, 1.02 mmol) and Cs₂CO₃ (700 mg, 2.15 mmol) in acetone (10 mL) was stirred at room temperature for 20 h. The resulting mixture was filtered through diatomaceous earth. The pad was washed with acetone, and the combined filtrates were concentrated under reduced pressure to a golden oil. The oil was purified on SiO₂ (10 g; 0-100% acetone/CH₂Cl₂) to give the desired product as a clear and colorless oil (202 mg, 70%). TLC (SiO₂, acetone): R_{f} = 0.17. MS (ESI): mass calculated for C₂₁H₂₄N₂O₃S, 384.15; m/z found, 369.3 [M+H]⁺. ¹H NMR (400 MHz, DMSO-*d*₆): 7.61 (d, *J* = 7.0, 2H), 7.50 (d, *J* = 7.0, 2H), 7.41 (d, *J* = 9.1, 4H), 7.35-7.30 (m, 4H), 7.03 (d, *J* = 9.1, 4H,), 4.41 (t, *J* = 5.3, 2H), 4.07 (t, *J* = 6.2, 4H), 3.60-3.52 (m, 2H), 3.44-3.35 (m, 2H), 3.18-3.08 (m, 2H), 2.90-2.85 (m, 2H), 2.78-2.72 (m, 2H), 2.35-2.28 (m, 4H), 1.62 (d, *J* = 9.2, 4H), 1.53-1.47 (m, 2H), 1.47-1.34 (m, 2H), 1.32-1.18 (m, 4H).

### EXAMPLE 22

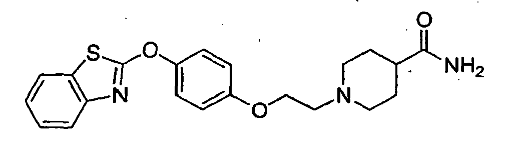

1-{2-[4-(Benzothiazol-2-yloxy)-phenoxy]-ethyl}-piperidine-4-carboxylic acid amide.

A suspension of 2-[4-(2-bromo-ethoxy)-phenoxy]-benzothiazole (EXAMPLE 9; 200 mg, 0.57 mmol), isonipecotamide (73 mg, 0.57 mmol) and Silicycle^{®} dimethylamine resin (800 mg, 1.14 mmol) in CH₃CN was heated to 70 °C for 18 h. The reaction mixture was filtered, and the collected resin was rinsed with CH₃CN. The combined filtrates were concentrated under reduced pressure yielding a crude solid, which was purified on SiO₂ (10 g; 0-100% 10% [2 M NH₃ in CH₃OH] in CH₂Cl₂/CH₂Cl₂) to provide 142 mg (63% yield) of a white solid. MS (ESI): mass calculated for C₂₁H₂₃N₃O₃S, 397.15; m/z found, 398.4 [M+H]⁺. ¹H NMR (400 MHz, CDCl₃): 7.85 (dd, *J*=8.0, 0.3, 1H), 7.75 (dd, *J* =8.0, 0.6, 1H), 7.42 (dd, *J* =7.4, 1.1, 1H), 7.32-7.22 (m, 3H), 7.02-6.91 (m, 2H), 5.67 (br d, *J* =47, 2H), 4.15 (t, *J* = 5.8, 2H), 3.09 (br d, *J* =8.8, 2H), 2.85 (t, *J* =5.7, 2H), 2.28-2.12 (m, 3H), 2.00-1.88 (m, 2H), 1.87-1.72 (m, 2H).

### EXAMPLE 23

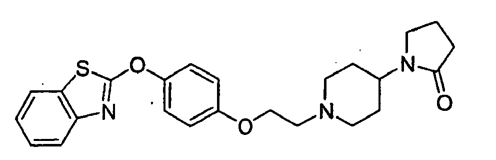

1-(1-{2-[4-(Benzothiazol-2-yloxy)-phenoxy]-ethyl}-piperidin-4-yl)-pyrrolidin-2-one

A suspension of 2-[4-(2-bromo-ethoxy)-phenoxy]-benzothiazole (EXAMPLE 9; 200 mg, 0.57 mmol), 1-piperidin-4-yl-pyrrolidin-2-one hydrochloride (117 mg, 0.57 mmol), and Silicycle^{®} dimethylamine resin (800 mg, 1.14 mmol) in CH₃CN was heated to 70 °C for 18 h. The reaction mixture was filtered, and the collected resin was rinsed with CH₃CN. The combined filtrates were concentrated under reduced pressure yielding a crude solid, which was purified on SiO₂ (10 g; 0-100% 10% [2 M NH₃ in CH₃OH] in CH₂Cl₂/CH₂Cl₂) to provide a tacky off-white solid (142 mg, 63% yield). MS (ESI): mass calculated for C₂₄H₂₇N₃O₃S, 337.18; m/z found, 348.5 [M+H]⁺. ¹H NMR (400 MHz, CDCl₃): 7.85 (dd, *J* = 8.0, 0.5, 1H), 7.75 (dd, *J* = 8.0, 0.8, 1H), 7.41 (dt, *J* = 7.3, 1.5, 1H), 7.34-7.22 (m, 3H), 7.02-6.92 (m, 2H), 4.15 (br d, *J* = 48.8, 2H), 3.80-3.65 (m, 1H), 3.40 (t, *J* = 7.0, 1H), 3.30-3.10 (br s, 1H), 3.15, (q, *J* = 7.2, 1H), 2.96 (br s, 1H), 2.42, (t, *J* = 7.9, 2H), 2.10-1.99 (m, 1H), 1.81-1.70 (m, 1 H) 1.68-1.52 (m, 4H), 1.50 (d, *J* = 6.5, 3H).

### EXAMPLE 24

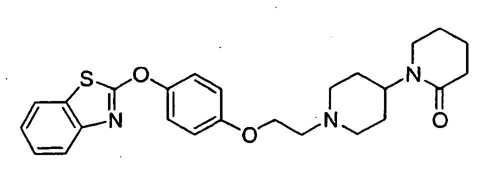

1'-{2-[4-(Benzothiazol-2-yloxy)-phenoxy]-ethyl}-[1,4']bipiperidinyl-2-one.

To a stirred solution of 2-[4-(2-bromo-ethoxy)-phenoxy]-benzothiazole (EXAMPLE 9; 542 mg, 1.55 mmol) and [1,4']bipiperidinyl-2-one hydrochloride (371 mg, 1.69 mmol) in CH₃CN (20 mL) was added K₂CO₃ (517 mg, 3.74 mmol). The mixture was heated at reflux for 20 h and then was filtered. The filtrate was concentrated under reduced pressure to a clear golden oil, which was purified on SiO₂ (10 g; 0-100% acetone/CH₂Cl₂) to give 294 mg (42% yield) of a white solid. TLC (SiO₂, acetone): R_{f} = 0.15. MS (ESI): mass calculated for C₂₅H₂₉N₃O₃S, 451.19; m/z found, 452.4 [M+H]⁺. ¹H NMR (400 MHz, DMSO-*d*₆): 7.92 (d, *J* = 1.1, 2H), 7.90 (d, *J* = 1.1, 2H), 7.42 (t, *J* = 7.3, 2H), 7.37 (d, *J* = 9.0, 2H), 7.31 (t, *J* = 7.3 2H), 7.06 (d, *J* = 9.0, 2H), 4.32-4.21 (m, 1H), 4.10 (t, *J* = 5.7, 2H), 3.15 (t., *J* = 5.3, 2H), 3.00 (d, *J* = 11.5, 2H), 2.71 (t, *J* = 5.7, 2H), 2.21 (t, *J* = 6.5, 2H), 2.10 (t, *J* = 11.4, 2H), 1.75-1.58 (m, 6H), 1.43 (d, *J* = 10.0, 2H).

### EXAMPLE 25

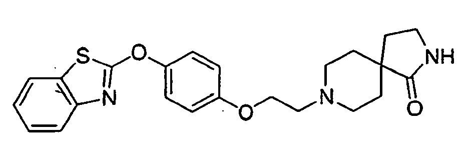

8-{2-[4-(Benzothiazol-2-yloxy)-phenoxy]-ethyl}-2,8-diaza-spiro[4.5]decan-1-one.

A suspension of 2-[4-(2-bromo-ethoxy)phenoxy]-benzothiazole (EXAMPLE 9; 257 mg, 0.73 mmol), 2,8-diaza-spiro[4.5]decan-1-one hydrochloride (153 mg, 0.80 mmol) and Silicycle^{®} dimethylamine resin (1.7 g, 2.4 mmol) in CH₃CN was heated to 80 °C for 18 h. The reaction mixture was filtered, and the collected resin was rinsed with CH₃CN. The combined filtrates were concentrated under reduced pressure to a crude solid, which was purified on SiO₂ (10 g; 0-100% 10% [2 M NH₃ in CH₃OH] in CH₂Cl₂/CH₂Cl₂) to provide an off-white solid (152 mg, 49% yield). MS (ESI): mass calculated for C₂₃H₂₅N₃O₃S, 423.16; m/z found, 424.2 [M+H]⁺. ¹H NMR (400 MHz, CDCl₃): 7.78 (dd, *J* = 8.1, 0.6, 1H), 7.69 (dd, *J* = 8.0, 0.8, 1H), 7.41 (dt, *J* = 7.5, 1.3, 1H), 7.32-7:27 (m, 3H), 7.00 (m, 2H), 6.36 (br s, 1H), 4.15 (t, *J* = 5.9, 2H), 3.36 (t, *J* = 7.0, 2H), 3.00, (dt, *J* = 11.9, 3.9, 2H), 2.87 (t, *J* = 5.8, 2H), 2.32 (dt, *J* = 11.5, 2.4, 2H), 2.10-1.98 (m, 2H), 2.07 (t, *J* = 7.0, 2H), 1.50 (br d, *J* = 13.3, 2H).

### EXAMPLE 26

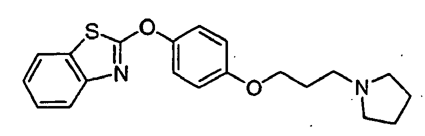

2-[4-(3-Pyrrolidin-1-yl-propoxy)-phenoxy]-benzothiazole.
A. 1-[3-(4-Benzyloxy-phenoxy)-propyl]-pyrrolidine. To a mixture of 1-[3-(4-benzyloxy-phenoxy)-propyl]-bromide (EXAMPLE 2; 1.50 g, 4.7 mmol) was added pyrrolidine (2.0 mL, 24.0 mmol) in CH₃CN. The mixture was stirred for 20 h then concentrated under reduced pressure to give a yellow oil, which was purified on SiO₂ (35 g; acetone) to give 1.2 g (80% yield) of the desired product as a white solid. TLC (SiO₂, acetone): R_{f} = 0.05. MS (ESI): mass calculated for C₂₆H₂₅NO₂, 3111.19; m/z found, 312.2 [M+H]⁺. ¹H NMR (400 MHz, CDCl₃): 7.50-7.30 (m, 5H), 6.90 (d, *J* = 9.1, 2H), 6.82 (d, *J* = 9.1, 2H), 5.02 (s, 2H), 4.00 (t, *J* = 6.1, 2H), 3.0-2.75 (m, 6H), 2.15 (d, *J* = 6.2, 2H), 1.94 (br s, 4H).
B. 4-(3-Pyrrolidin-1-yl-propoxy)-phenol. To a solution of 1-[3-(4-benzyloxy-phenoxy)-propyl]-pyrrolidine (1.2 g, 3.9 mmol) in 1:1 ethanol/ethyl acetate (65 mL) was added Pd on carbon (10 wt %, 206 mg). The mixture was placed on a Parr hydrogenator at 40 psi of H₂ for 20 h. The resultant mixture was filtered through diatomaceous earth, and the filtrate was concentrated under reduced pressure to give 875 mg (100% yield) of the desired product as a brown solid. MS (ESI): mass calculated for C₁₃H₁₉NO₂, 221.14; m/z found, 222.2 [M+H]⁺. ¹H NMR (400 MHz, DMSO-*d*₆): 8.90 (s, 1H), 6.74 (d, *J* = 9.0, 2H), 6.65 (d, *J* = 9.0, 2H), 3.90 (t, *J* = 6.3, 2H), 2.72 (br s, 6H), 1.90 (quint, *J* = 7.4, 2H), 1.76 (s, 4H).
C. 2-[4-(3-Pyrrolidin-1-yl-propoxy)-phenoxy]-benzothiazole. To a stirred solution of 4-(3-pyrrolidin-1-yl-propoxy)-phenol (100 mg, 0.45 mmol) in acetone (5 mL) containing Cs₂CO₃ (213 mg, 0.65 mmol) was added 2-chlorobenzthiazole (65 µL, 0.50 mmol). The mixture was heated at reflux for 24 h and then filtered. The filtrate was concentrated under reduced pressure to give a clear golden oil, which was purified on SiO₂ (10 g; acetone) to give 97 mg (61% yield) of the desired product. TLC (SiO₂, acetone): R_{f} = 0.02. MS (ESI): mass calculated for C₂₀H₂₂N₂O₂S, 354.14; m/z found, 355.1 [M+H]⁺. ¹H NMR (400 MHz, DMSO-*d*₆): 7.73 (d, 1H), 6.95 (d, 1H), 7.39 (m, 1H), 7.26 (m, 3H), 6.96 (d, *J* = 9.1, 2H), 4.06 (t, *J* = 6.4, 2H), 2.65 (t, *J* = 7.3, 2H), 2.55 (br s, 4H), 2.04 (quint, *J* = 6.5, 2H), 1.82 (br s, 4H).

### EXAMPLE 27

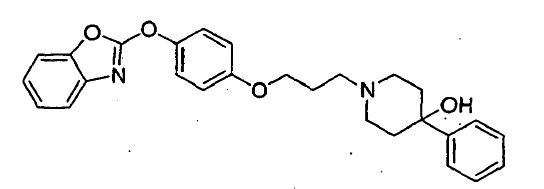

1-{3-[4-(Benzooxazol-2-yloxy)-phenoxy]-propyl}-4-phenyl-piperidin-4-ol.
A. 1-[3-(4-Hydroxy-phenoxy)-propyl]-4-phenyl-piperidin-4-ol hydrogen bromide. 4-(3-Bromo-propoxy)-phenol (EXAMPLE 4; 3 g, 13 mmol) was dissolved in CH₃CN (65 mL). To this solution was added 4-hydroxy-4-phenylpiperidine (6.8 g, 39 mmol), and the mixture was stirred at room temperature overnight, yielding a white precipitate. The suspension was filtered to give the title compound as a white solid (5 g, 11.9 mmol, 91 % yield). MS (ESI): mass calculated for C₂₀H₂₅NO₃ (free base), 327.18; m/z found, 328.2 [M+H]⁺. ¹H NMR (400 MHz, CD₃OD): 7.49 (d, *J* = 7.5, 2H), 7.32 (t, *J* = 7.5, 2H), 7.21 (t, *J* =7.2, 1H), 6.73 (q, *J* = 12.3, 4H), 3.96 (t, *J* = 6.1, 2H), 2.85 (d, *J* = 11.3, 2H), 2.64-2.54 (m, 4H), 2.13 (dt, *J* = 9.0, 3.9, 2H), 2.00 (m, 2H), 1.75 (d, *J* = 12.2, 2H).
B. 1-{3-[4-(Benzooxazol-2-yloxy)-phenoxy]-propyl}-4-phenyl-piperidin-4-ol. To a stirring solution of 1-[3-(4-hydroxy-phenoxy)-propyl]-4-phenyl-piperidin-4-ol hydrogen bromide (500 mg, 1.5 mmol) in acetone (7 mL) was added Cs₂CO₃ (1.03 g, 3.15 mmol). This suspension was cooled to 0 °C, and 2-chloro-benzooxazole (276 mg, 1.8 mmol, 0.2 mL) was added dropwise. The reaction mixture was allowed to warm to room temperature overnight, then filtered and concentrated under reduced pressure. The resultant oil was dissolved in CH₂Cl₂ and purified on SiO₂ (40 g; 0-100% acetone/CH₂Cl₂) to give 450 mg (1.05 mmol, 70% yield) of a white solid. MS (ESI): mass calculated for C₂₆H₂₆N₂O₄, 444.20; m/z found, 445.20 [M+H]⁺. ¹H NMR (400 MHz, CD₃OD): 7.47-7.43 (m, 3H), 7.36-7.11 (m, 8H), 6.90 (d, *J* = 9.1, 2H), 3.99 (t, *J* = 5.8, 2H), 2.80 (d, *J* = 9.5, 2H), 2.56 (t, *J* = 6.8 Hz, 2H), 2.44 (t, *J* = 10.9, 2H), 2.13 (t, *J* = 11.0, 2H), 1.98 (m, *J* = 6.8, 2H), 1.71 (d, *J* = 6.9, 2H).

### EXAMPLE 28

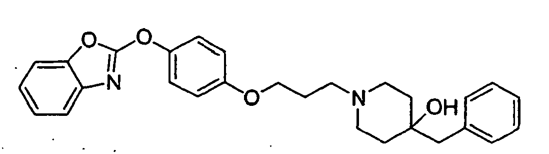

1-{3-[4-(Benzooxazol-2-yloxy)-phenoxy]-propyl}-4-benzyl-piperidin-4-ol.
A. 4-Benzyl-1-[3-(4-hydroxy-phenoxy)-propyl]-piperidin-4-ol. 4-Benzyl-4-hydroxy-piperidine (750 mg, 3.9 mmol) was added to a solution of 4-(3-bromo-propoxy)-phenol (EXAMPLE 4; 300 mg, 1.31 mmol) in CH₃CN (6 mL). The reaction mixture was stirred at room temperature overnight, yielding a white precipitate. The suspension was filtered, and the filtrate was concentrated under reduced pressure. The resultant oil was purified on SiO₂ (10 g; 0-100% acetone/CH₂Cl₂) to give 110 mg (0.32 mmol, 25% yield) of a white solid. MS (ESI): mass calculated for C₂₁H₂₂NO₃, 341.20; m/z found, 342.2 [M+H]⁺. ¹H NMR (400 MHz, CDCl₃): 7.36-7.24 (m, 3H), 7.22 (d, *J* = 6.9, 2H), 6.7 (m, 4H), 4.07 (t, *J* = 6.5, 2H), 3.41 (d, *J* = 11.7, 2H), 3.19 (t, *J* = 7.8, 2H), 3.11 (t, *J* = 11.8, 4H), 2.84 (br s, 2H), 2.34 (br s, 4H), 2.18 (br s, 1H), 1.72 (d, 14.5, 2H).
B. 1-{3-[4-(Benzooxazol-2-yloxy)-phenoxy]-propyl}-4-benzyl-piperidin-4-ol. To a stirring solution of 4-benzyl-1-[3-(4-hydroxy-phenoxy)-propyl]-piperidin-4-ol (2.5 g, 7.3 mmol) in acetone (37 mL) was added Cs₂CO₃ (4.99 g, 15.3 mmol). This suspension was cooled to 0 °C, and 2-chloro-benzooxazole (1.1 mL, 9.5 mmol) was added dropwise. The reaction mixture was allowed to warm to room temperature overnight, was filtered and then was concentrated under reduced pressure. The resultant oil was dis solved in CH₂Cl₂ and purified on SiO₂ (110 g; 0-100% acetone/CH₂Cl₂) to give 310 mg (0.67 mmol, 9% yield) of a white solid. MS (ESI): mass calculated for C₂₈H₃₀N₂O₄, 458.2; m/z found, 459.3 [M+H]⁺. ¹H NMR (400 MHz, CDCl₃): 7.49 (d, *J* = 7.2, 1H), 7.41 (d, *J* = 7.2, 1H), 7.35-7.18 (m, 9H), 6.94 (d, *J* = 9.1, 2H), 4.04 (t, *J* = 6.5, 2H), 2.93-2.45 (m, 7H), 2.16 (s, 4H), 1.60 (d, *J* = 13.0, 2H), 1.29 (br s, 1H).

### EXAMPLE 29

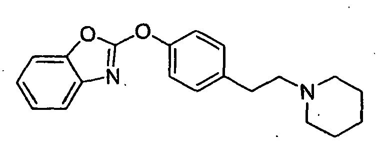

2-[4-(2-Piperidin-1-yl-ethyl)-phenoxy]-benzooxazole.
A. 4-(2-Piperidin-1-yl-ethyl)-phenol. A solution of 4-(2-bromo-ethyl)-phenol (EXAMPLE 5; 4.5 g, 22.4 mmol), piperidine (3.3 mL, 33.5 mmol), and *N,N-*diisopropylethylamine (5.8 mL, 33.5 mmol) in CH₃CN (100 mL) was stirred at 60 °C for 18 h. The resulting solution was cooled to room temperature and concentrated under reduced pressure to yield a pale orange solid. Diethyl ether (100 mL) was added, and the title compound was collected by filtration as an off-white solid (4.6 g, 100% crude yield). TLC (SiO₂, 5% 2 M NH₃ in CH₃OH/CH₂Cl₂): R_{f} = 0.19. MS (ESI): mass calculated for C₁₃H₁₉NO₂, 205.15; m/z found, 206.1 M+H]⁺. ¹H NMR (400 MHz, CD₃OD): 7.07-7.04 (m 2H), 6.74-6.71 (m, 2H), 3.32-3.30 (m, 2H), 3.14-3.11 (m, 3H), 2.87-2.80 (m, 1H), 1.82-1.67 (m, 6H), 1.65-1.55 (m, 2H).
B. 2-[4-(2-Piperidin-1-yl-ethyl)-phenoxy]-benzooxazole. A mixture of 4-(2-piperidin-1-yl-ethyl)-phenol (0.5 g, 2.43 mmol), 2-chloro-benzooxazole (304 µL, 2.67 mmol) and Cs₂CO₃ (1.8 g, 5.62 mmol) in acetone (10 mL) was stirred at room temperature for 48 h. The reaction mixture was filtered through diatomaceous earth, and the pad was washed with CH₂Cl₂. The combined filtrates were concentrated under reduced pressure to an orange oil, which was purified on SiO₂ (40 g; 0-100% acetone/CH₂Cl₂) to give the desired product as white solid (325 mg, 42% yield). TLC (SiO₂, 5% 2 M NH₃ in CH₃OH/CH₂Cl₂): R_{f} = 0.36. MS (ESI): mass calculated for C₂₀H₂₂N₂O₂, 322.17; m/z found, 323.1 [M+H]⁺. ¹H NMR (400 MHz, CDCl₃): 7.53-7.51 (m, 1H), 7.44-7.42 (m, 1H), 7.34-7.20 (m, 6H), 2.87-2.83 (m, 2H), 2.60-2.56 (m, 2H), 2.48 (br s, 2H), 1.66-1.59 (m, 6H), 1.50-1.43 (m, 2H).

### EXAMPLE 30

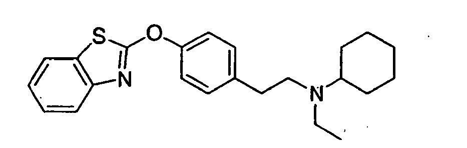

{2-[4-(Benzothiazol-2-yloxy)-phenyl]-ethyl}-cyclohexyl-ethyl-amine.
A. 4-[2-(Cyclohexyl-ethyl-amino)-ethyl]-phenol. To a stirring solution of 4-(2-bromo-ethyl)-phenol (EXAMPLE 5; 4.48 g, 22.3 mmol) in CH₃CN (100 mL) was added cyclohexyl-ethyl-amine (5.0 mL, 33.4 mmol), followed by *N,N-*diisopropylethylamine (7.76 mL, 44.6 mmol). The resulting solution was stirred at 60 °C for 16 h, yielding a suspension. The suspension was allowed to cool to room temperature and was filtered. The filtered solid was washed with ethyl acetate (2 x 20 mL) and dried to give a white solid (4.8 g, 87% yield). MS (ESI): mass calculated for C₁₆H₂₅NO, 247.19; m/z found, 248.2 [M+H]⁺. ¹H NMR: (400 MHz, CDCl₃): 7.01 (d, *J* = 8.6, 2H), 6.87 (d, *J* = 8.6, 2H), 3.32-3.17 (m, 2H), 3.15-2.98 (m, 4H), 2.30-2.21 (m, 2H), 1.77-1.59 (m, 2H), 1.57-1.46 (m, 5H), 1.40-1.10 (m, 3H).
B. {2-[4-(Benzothiazol-2-yloxy)-phenyl]-ethyl}cyclohexyl-ethyl-amine. To a stirring solution of 1-[2-(4-hydroxy-phenoxy)-ethyl]-piperidin-4-ol (495 mg, 2.0 mmol) in DMF (10 mL), was added Cs₂CO₃ (1.3 g, 4.0 mmol) and 2-chlorobenzothiazole (0.33 mL, 2.5 mmol). The suspension was stirred at 80 °C overnight. The reaction mixture was allowed to cool to room temperature and was then filtered through diatomaceous earth. The filtrate was concentrated under reduced pressure, and the residue was purified on SiO₂ (40 g; 0-100% acetone/CH₂Cl₂) to give 548 mg (72% yield) of a tan solid. MS (ESI): mass calculated for C₂₃H₂₈N₂OS, 380.19; m/z found, 381.4 [M+H]⁺. ¹H NMR (400 MHz, CDCl₃): 7.73 (d, *J* = 8.0, 1H), 7.63 (d, *J* = 8.0, 1H), 7.30 (t, *J* = 8.0, 1H), 7.29-7.20 (m, 5H), 2.78-2.68 (m, 4H), 2.62 (dd, *J* = 6.8, 7.4, 2H), 2.56-2.46 (m 1H), 1.83-1.74 (m, 4H), 1.66-1.57 (m, 1H), 1.21 (dd, *J* = 9.0, 8.6, 4H), 1.15-1.11 (m, 1H), 1.06 (t, *J* = 7.2, 3H).

### EXAMPLE 31

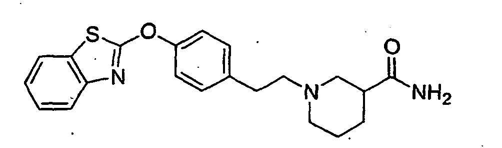

1-{2-[4-(Benzothiazol-2-yloxy)-phenyl]-ethyl}-piperidine-3-carboxylic acid amide.

A suspension of 2-[4-(2-bromo-ethyl)-phenoxy]-benzothiazole (EXAMPLE 10; 250 mg, 0.75 mmol), nipecotamide (96 mg, 0.75 mmol) and Silicycle^{®} dimethylamine resin (1.1 g, 1.50 mmol) in CH₃CN was heated to 70 °C for 18 h. The reaction mixture was filtered, and the collected resin was washed with CH₃CN. The combined filtrates were concentrated under reduced pressure to a crude solid, which was purified on SiO₂ (10 g; 0-100% 10% [2 M NH₃ in CH₃OH] in CH₂Cl₂/CH₂Cl₂) to provide 135 mg (47% yield) of a white solid. MS (ESI): mass calculated for C₂₁H₂₃N₃O₂S, 381.15; m/z found, 382.4 [M+H]⁺. ¹H NMR (400 MHz, CDCl₃): 8.10 (d, *J* = 8.1, 1H), 7.75 (dd, *J* = 8.0, 0.8, 1H), 7.42 (dt, *J* = 7.4, 1.3, 1H), 7.21 (br s, 1H) 6.42 (br s, 1H), 3.05 (br d, *J* = 10.3, 1H), 2.95-2.80 (m, 3H), 2.80-2.62 (m, 2H), 2.47-2.40 (m, 1H), 2.36 (d, *J* = 11.5, 1H), 2.08 (t, *J* = 10.5, 1H), 1.98 (d, *J* = 11.0, 1H), 1.76-1.63 (m, 1 H), 1.63-1.50 (m, 2H).

### EXAMPLE 32

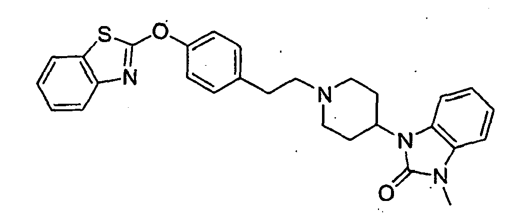

1-(1-{2-[4-(Benzothiazol-2-yloxy)-phenyl]-ethyl}-piperidin-4-yl)-3-methyl-1,3-dihydro-benzoimidazol-2-one.
A. Toluene-4-sulfonic acid 2-[4-(benzothiazol-2-yloxy)-phenyl]-ethyl ester. To a stirring solution of 2-[4-(benzothiazol-2-yloxy)-phenyl]-ethanol (EXAMPLE 10; 12.25 g, 45.2 mmol) in CH₂Cl₂ (225 mL) was added *p*-toluenesulfonyl chloride (17.23 g, 90 mmol) and TEA (31 mL, 225 mmol). The mixture was stirred at room temperature for 72 h. The reaction mixture was concentrated under reduced pressure, and the residue was dissolved in ethyl acetate (400 mL). The solution was washed with sat. aq. NaHCO₃ (3 x 200 mL), dried (Na₂SO₄), and concentrated under reduced pressure. The resultant oil was dissolved in CH₂Cl₂ and purified on SiO₂ (300 g; CH₂Cl₂) to give a tan solid (8.8 g, 45% yield). MS (ESI): mass calculated for C₂₂H₁₉N₂O₄S₂, 425.08; m/z found, 426.0 [M+H]⁺. ¹H NMR (400 MHz, CDCl₃): 7.70 (q, *J* = 9.7, 4H), 7.39 (t, *J* = 7.8, 1 H), 7.32-7.17 (m, 8H), 4.23 (t, *J* = 6.9, 2H), 2.99 (d, *J* = 6.9, 2H), 2.43 (s, 3H).
B. 1-(1-{2-[4-(Benzothiazol-2-yloxy)-phenyl]-ethyl}-piperidin-4-yl)-3-methyl-1,3-dihydro-benzoimidazol-2-one. To a stirring solution of toluene-4-sulfonic acid 2-[4-(benzothiazol-2-yloxy)-phenyl]-ethyl ester (400 mg, 0.94 mmol) in CH₃CN (5 mL), was added 1-methyl-3-piperidin-4-yl-1,3-dihydro-benzoimidazol-2-one (653 mg, 2.82 mmol). The reaction mixture was stirred at room temperature overnight, then concentrated under reduced pressure and purified on SiO₂ (12 g; 50% acetone/CH₂Cl₂) to give a clear oil (16 mg, 3.3% yield). MS (ESI): mass calculated for C₂₈H₂₈N₄O₂S, 484.19; m/z found, 485.5 [M+H]⁺. ¹H NMR (400 MHz, CD₃OD): 7.75 (d, *J* = 7.9, 1H), 7.65 (d, *J* = 8.1, 1H), 7.41-7.29 (m, 7H), 7.13 (m, 3H), 4.37 (m, 1H), 3.40 (s, 3H), 3.25 (d, *J* = 11.8, 2H), 2.94 (m, 2H), 2.75 (m, 2h), 2.57 (qd, *J* = 12.6, 3.7, 2H), 2.33 (t, *J* = 11.3, 2H), 1.81 (d, *J* = 12.2, 2H).

### EXAMPLE 33

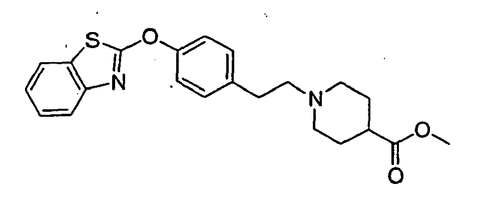

1-{2-[4-(Benzothiazol-2-yloxy)-phenyl]-ethyl}-piperidine-4-carboxylic acid methyl ester.
A. 1-[2-(4-Hydroxy-phenyl)-ethyl]-piperidine-4-carboxylic acid methyl ester. To a stirring solution of 4-(2-bromo-ethyl)-phenol (EXAMPLE 5; 5.05 g, 25 mmol) in CH₃CN (100 mL) was added piperidine-4-carboxylic acid methyl ester (5.07 mL, 37.5 mmol), followed by *N*,*N*-diisopropylethylamine (8.7 mL, 50 mmol). The reaction mixture was stirred at 60 °C for 16 h, and then allowed to cool to room temperature. CH₂Cl₂ (250 mL) was added, and the resulting solution was washed with H₂O (2 x 30 mL), dried, filtered, and then concentrated under reduced pressure to afford 5.2 g (79%) of a tan solid. MS (ESI): mass calculated for C₁₅H₂₁NO₃, 263.15; m/z found, 264.3 [M+H]⁺. ¹H NMR (400 MHz, CDCl₃): 6.97 (d, *J* = 8.2, 2H), 6.70 (d, *J* = 8.6, 2H), 3.67 (s, 3H), 2.99 (d, *J* = 11.5, 2H), 2.76-2.68 (m, 2H), 2.60-2.54 (m, 2H), 2.40-2.30 (m, 1H), 2.18 (t, *J* = 10.8, 2H), 1.99-1.90 (m, 2H), 1.90-1.78 (m, 2H).
B. 1-{2-[4-(Benzothiazol-2-yloxy)-phenyl]-ethyl}-piperidine-4-carboxylic acid methyl ester. To a stirring solution of 1-[2-(4-hydroxy-phenyl)-ethyl]-piperidine-4-carboxylic acid methyl ester (790 mg, 3.0 mmol) in DMF (15 mL), was added Cs₂CO₃ (1.95 g, 6.0 mmol) and 2-chlorobenzothiazole (0.47 mL, 3.9 mmol). The suspension was heated to 100 °C and stirred overnight. The reaction mixture was allowed to cool to room temperature and then filtered through diatomaceous earth. The filtrate was concentrated under reduced pressure, and the residue was purified on SiO₂ (40 g; 0-100% acetone/CH₂Cl₂) to give 1.04 g (87% yield) of a tan solid. MS (ESI): mass calculated for C₂₂H₂₄N₂O₃S, 396.15; m/z found, 397.4 [M+H]⁺. ¹H NMR (400 MHz, CDCl₃): 7.73 (d, *J* = 7.8, 1H), 7.65 (d, *J* = 7.8, 1H), 7.38 (t, *J* = 7.6, 1H), 7.28-7.25 (m, 5H), 3.69 (s, 3H), 3.00-2.93 (m, 2H), 2.83 (dd, *J* = 7.6, 3.0, 2H), 2.61 (dd, *J* = 7.6, 3.0, 2H), 2.38-2.28 (m 1H), 2.11 (t, *J* = 10.4, 2H), 1.98-1.89 (m, 2H), 1.87-1.74 (m, 2H).

### EXAMPLE 34

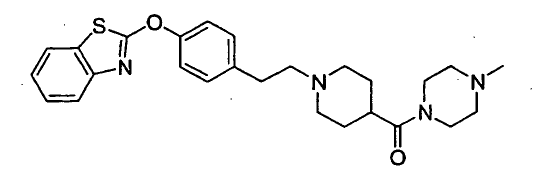

(1-{2-[4-(Benzothiazol-2-yloxy)-phenyl]-ethyl}-piperidin-4-yl)-(4-methyl-piperazin-1-yl)-methanone.
A. 1-{2-[4-(Benzothiazol-2-yloxy)-phenyl]-ethyl}-piperidine-4-carboxylic acid trifluoroacetic acid salt. To a solution of 1-{2-[4-(benzothiazol-2-yloxy)-phenyl]-ethyl}-piperidine-4-carboxylic acid methyl ester (EXAMPLE 33; 4.6 g, 11.7 mmol) in 3:1 THF/CH₃OH (100 mL), was added lithium hydroxide (1.1 g, 46.6 mmol) in H₂O (25 mL). This dark yellow solution was stirred at room temperature for 16 h and then concentrated under reduced pressure. The residue was dissolved in CH₃OH and purified by reversed-phase HPLC to give the title compound as a light yellow solid (3.9 g, 68% yield). MS (ESI): mass calculated for C₂₁H₂₂N₂O₃S, 382.14; m/z found, 383.4 [M+H]⁺.
B. (1-{2-[4-(Benzothiazol-2-yloxy)-phenyl]-ethyl}-piperidin-4-yl)-(4-methyl-piperazin-1-yl)-methanone. To a mixture of 1-{2-[4-(benzothiazol-2-yloxy)-phenyl]-ethyl}-piperidine-4-carboxylic acid TFA salt (300 mg, 0.6 mmol) in CH₂Cl₂ (15 mL) and a drop of DMF, was added oxalyl chloride (0.11 mL, 1.2 mmol). The mixture was stirred at room temperature for 1 h. The resulting mixture was concentrated under reduced pressure. The residue was dissolved in CH₂Cl₂ (15 mL), and *N*-methylpiperizine (0.2 mL, 1.8 mmol) was added. This reaction mixture was stirred at room temperature for 1 h, diluted with CH₂Cl₂ (100 mL), washed with H₂O, sat. aq. NaHCO₃ then brine, dried (Na₂SO₄), and filtered. The filtrate was concentrated under reduced pressure, and the residue was purified on SiO₂ (40 g; 0-10% 2 M NH₃ in CH₃OH/CH₂Cl₂) to give the title compound (240 mg, 86%). MS (ESI): mass calculated for C₂₆H₃₂N₂O₃S, 464.22; m/z found, 465.5 [M+H]⁺. ¹H NMR (400 MHz, DMSO-d₆): 7.72 (d, *J* = 7.6, 1H), 7.65 (d, *J* = 7.6, 1H), 7.37 (t, *J* = 7.6, 1H), 7.29-7.22 (m, 5H), 3.64 (br s, 2H), 3.51 (br s, 2H), 3.06 (d, *J* = 11.4, 2H), 2.83 (dd, *J* = 7.3, 3.5, 2H), 2.60 (dd, *J* = 7.3, 3.5, 2H), 2.51-2.43 (m 1H), 2.42-2.33 (m, 4H), 2.30 (s, 3H), 2.06 (t, *J* = 12.1, 2H), 1.99-1.84 (m, 2H), 1.72 (d, *J*= 13.1, 2H).

### EXAMPLE 35

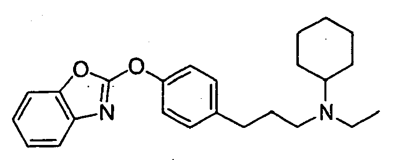

{3-[4-(Benzooxazol-2-yloxy)-phenyl]-propyl}-cyclohexyl-ethyl-amine.
A. [3-(4-Benzyloxy-phenyl)-propyl]-cyclohexyl-ethyl-amine. To a solution of 3-(4-benzyloxy-phenyl)-propyl-1-bromide (504 mg, 1.65 mmol) and *N*-ethylcyclohexylamine (497 µL, 3.30 mmol) in CH₃CN (15 mL) was added K₂CO₃ (510 mg, 3.69 mmol). The reaction mixture was heated at reflux for 20 h. The mixture was filtered and then concentrated under reduced pressure to a golden oil, which was purified on SiO₂ (10 g; 50% acetone/CH₂Cl₂) to give 484 mg (83% yield) of the desired product as a clear and colorless oil. TLC (SiO₂, acetone): R_{f} = 0.13. MS (ESI): mass calculated for C₂₄H₃₃NO, 351.26; m/z found, 352.4 [M+H]⁺. ¹H NMR (400 MHz, CDCl₃): 7.50-7.28 (m, 5H), 7.12 (d, *J* = 8.6, 2H), 6.90 (d, *J* = 8.6, 2H), 5.06 (s, 2H), 2.60-2.45 (m, 7H), 1.78 (br s, 6H), 1.20 (br s, 4H), 1.05 (t, *J* = 7.1, 4H).
B. 4-[3-(Cyclhexyl-ethyl-amino)-propyl]-phenol. To a solution of [3-(4-benzyloxy-phenyl)-propyl]-cyclohexyl-ethyl-amine (420 mg, 1.19 mmol) in 1:1 ethanol/ethyl acetate (16 mL) was added Pd on carbon (10 wt %, 46 mg). The mixture was placed on a Parr hydrogenator at 40 psi of H₂ for 20 h. The resultant mixture was filtered through diatomaceous earth, and the filtrate was concentrated under reduced pressure to give 308 mg (99% yield) of the desired product as a golden oil. TLC (SiO₂, acetone): R_{f} = 0.18. MS (ESI): mass calculated for C₁₇H₂₇NO, 261.21; m/z found, 262.3 [M+H]⁺. ¹H NMR (400 MHz, CDCl₃): 7.00 (d, *J* = 8.5, 2H), 6.75 (d, *J* = 8.5, 2H), 2.65 (br s, 5H), 2.54 (t, *J* = 7.6, 2H), 1.82 (br s, 6H), 1.30-1.05 (m, 8H).
C. {3-[4-(Benzooxazol-2-yloxy)-phenyl]-propyl}-cyclohexyl-ethyl-amine. To a mixture of 4-[3-(cyclohexyl-ethyl-amino)-propyl]-phenol (283 mg, 1.08 mmol) and Cs₂CO₃ (885 mg, 2.72 mmol) in acetone (15 mL) at 5 °C was added 2-chloro-benzooxazole (155 µL, 1.36 mmol). The reaction mixture was allowed to warm slowly to room temperature overnight, then was filtered. The filtrate was concentrated under reduced pressure to give a golden oil, which was purified on SiO₂ (10 g; acetone) to give 333 mg (81% yield) of the desired product as a golden oil. TLC (SiO₂, acetone): R_{f} = 0.12. MS (ESI): mass calculated for C₂₄H₃₀N₂O₂, 378.23; m/z found, 379.3 [M+H]⁺. ¹H NMR (400 MHz, CDCl₃): 7.61 (d, *J* = 7.3, 1H), 7.49 (d, *J* = 7.8, 1H), 7.39 (d, *J* = 8.6, 2H), 7.38-7.25 (m, 4H), 2.62 (t, *J* = 7.5, 2H), 2.65-2.35 (m, 8H), 1.67 (br s, 5H), 1.19-1.10 (m, 4H), 0.95 (t, *J* = 7.1, 3H).

### EXAMPLE 36

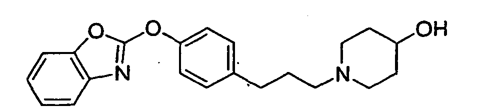

1-{3-[4-(Benzooxazol-2-yloxy)-phenyl]-propyl}-piperidin-4-ol.
A. 1-[3-(4-Hydroxy-phenyl)-propyl]-piperidin-4-ol. A solution of 4-(3-bromopropyl)-phenol (EXAMPLE 6; 1.42 g, 6.6 mmol), 4-hydroxypiperidine hydrochloride (908 mg, 6.6 mmol), and *N*,*N-*diisopropylethyamine (2.2 mL, 12.3 mmol) in CH₃CN (20 mL) was stirred at 60°C for 18 h. The reaction mixture was cooled to room temperature, and the solvent was removed under reduced pressure to yield an off-white solid. This material was dissolved in CH₂Cl₂ (200 mL), and the solution was washed with H₂O (2 x 50 mL), dried (Na₂SO₄), filtered and concentrated under reduce pressure to yield a white solid. Diethyl ether was added, and the title compound was collected by filtration (1.4 g, 90% yield). TLC (SiO₂, 5% 2 M NH₃ in CH₃OH/CH₂Cl₂): R_{f} = 0.05. MS (ESI): mass calculated for C₁₄H₂₁NO₂, 235.16; m/z found, 236.2 [M+H]⁺. ¹H NMR (400 MHz, CD₃OD): 6.96-6.94 (m, 2H), 6.64-6.61 (m, 2H), 3.89-3.82 (m, 1H), 3.29-3.20 (m, 4H), 3.02-2.95 (m, 4H), 2.52 (t, *J* = 7.4, 2H), 1.95-1.87 (m, 4H), 1.68-1.60 (m, 2H).
B. 1-{3-[4-(Benzooxazol-2-yloxy)-phenyl]-propyl}-piperidin-4-ol. A mixture of 1-[3-(4-hydroxy-phenyl)-propyl]-piperidin-4-ol (0.4 g, 1.69 mmol), 2-chloro-benzooxazole (176 µL, 1.54 mmol) and Cs₂CO₃ (1.45 g, 4.45 mmol) in acetone (8.0 mL) was stirred at room temperature for 48 h. The resulting mixture was filtered through diatomaceous earth, which was then washed with CH₂Cl₂. The combined filtrates were concentrated under reduced pressure to a yellow oil. The oil was purified on SiO₂ (40 g; 0-100% acetone/CH₂Cl₂) to give a white solid, which was dissolved in CH₂Cl₂. The solution was washed with 1 M NaOH (3 x 5 mL) then H₂O (5 mL), dried (Na₂SO₄), filtered, and concentrated under reduced pressure to yield the title compound as a white solid (98 mg, 16.4% yield). TLC (SiO₂, 5% 2 M NH₃ in CH₃OH/CH₂Cl₂): R_{f} = 0.14. MS (ESI): mass calculated for C₂₁H₂₄N₂O₃, 352.18; m/z found, 363.1 [M+H]⁺. ¹H NMR (400 MHz, CDCl₃): 7.66-7.51 (m, 1H), 7.44-7.42 (m, 1H), 7.34-7.21 (m, 6H), 3.72-3.69 (m, 1H), 2.79-2.76 (m, 2H), 2.67 (t, *J* = 7.8, 2H), 2.38 (t, *J* = 7.6, 2H), 2.15-2.11 (m, 2H), 1.98-1.80 (m, 3H), 1.65-1.55 (m, 3H), 1.42 (m, 1H).

### EXAMPLE 37

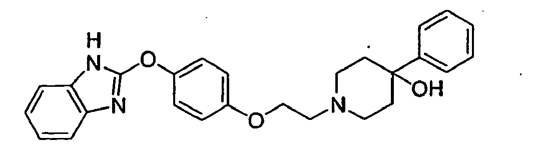

1-{2-[4-(1*H-*Benzoimidazol-2-yloxy)-phenoxy]-ethyl}-4-phenyl-piperidin-4-ol.
A. 1-[2-(4-Hydroxy-phenoxy)-ethyl]-4-phenyl-piperidin-4-ol. To a solution of 4-(2-bromo-ethoxy)-phenol (EXAMPLE 3; 8.0 g, 36.8 mmol) and 4-hydroxy-4-phenylpiperidine (8.2 g, 46.3 mmol) in CH₃CN (150 mL) was added DIEA (7.0 mL, 40.2 mmol). The mixture was stirred for 20 h at room temperature and for an additional 4 h at 65°C, then was concentrated under reduced pressure to give a brown solid. The solid was dissolved in ethyl acetate (250 mL), and the solution was washed with H₂O (250 mL, 100 mL), dried (MgSO₄), and concentrated under reduced pressure to give a brown solid. The solid was purified on SiO₂ (120 g; 0-100% acetone/CH₂Cl₂) e to give 8.9 g (77% yield) of the desired product as a tan solid. TLC (SiO₂, acetone): R_{f} = 0.42. MS (ESI): mass calculated for C₁₉H₂₃NO₃, 313.17; m/z found 314.2 [M+H]⁺. ¹H NMR (400 MHz, CDCl₃): 7.52 (d, *J* = 8.6, 2H), 7.37 (t, *J* = 7.3, 2H), 7.27 (m, 1H), 6.75 (s, 4H), 4.08 (t, *J* = 5.8, 2H), 3.05-2.90 (m, 2H), 2.88 (t, *J* = 5.8, 2H), 2.80-2.62 (m, 2H), 2.31-2.18 (m, 2H), 1.81 (d, *J* = 11.8, 2H).
B. 4-Phenyl-1-(2-{4-[1-(2-timethylsilanyl-ethoxymethyl)-1*H*-benzoimidazol-2-yloxy]-phenoxy}-ethyl)-piperidin-4-ol. To a mixture of 2-chloro-1-(2-trimethylsilanyl-ethoxymethyl)-1*H*-benzoimidazole (EXAMPLE 7; 630 mg, 2.2 mmol) and 1-[2-(4-hydroxy-phenoxy)-ethyl]-4-phenyl-piperidin-4-ol (690 mg, 2.2 mmol) in DMF (10 mL) was added Cs₂CO₃ (1.5 g, 4.6 mmol). The reaction mixture was stirred at 100°C for 18 h, and then partitioned in 1:1 ethyl acetate/H₂O (50 mL). The organic layer was collected, dried (MgSO₄), and concentrated under reduced pressure to give a clear brown oil, which was purified on SiO₂ (35 g; acetone) to give 867 mg (70% yield) of the desired product as a clear golden oil. TLC (SiO₂, acetone): R_{f} = 0.38. MS (ESI): mass calculated for C₃₂H₄₁N₃O₄Si, 559.29; m/z found, 560.3 [M+H]⁺. ¹H NMR (400 MHz, CDCl₃): 7.55 (m, 3H), 7.38 (m, 3H), 7.30-7.20 (m, 5H), 6.99 (d, *J* = 9.0, 2H), 5.55 (s, 2H), 4.17 (t, *J* = 5.9, 2H), 6.37 (m, 2H), 2.92 (m, 5H), 2.65 (t, *J* = 12.4, 2H), 2.21 (t, *J* = 15.9, 2H), 1.81 (d, *J* = 12.1, 2H), 0.96 (t, *J* = 8.1, 2H).
C. 1-{2-[4-(1*H*-Benzoimidazol-2-yloxy)-phenoxy]ethyl}-4-phenyl-piperidin-4-ol. To a solution of 4-phenyl-1-(2-{4-[1-(2-trimethylsilanyl-ethoxymethyl)-1*H-*benzoimidazol-2-yloxy]-phenoxy}-ethyl)-piperidin-4-ol (816 mg, 1.46 mmol) in THF (5 mL) containing *N*,*N*,*N*,*N*-tetramethylethylenediamine (TMEDA, 2.2 mL, 14.6 mmol), was added a 1 M THF solution of tetrabutylammonium fluoride (TBAF, 15 mL, 15 mmol). The mixture was stirred at 55°C for 5 h, then was concentrated under reduced pressure. The resulting oil was dissolved in diethyl ether(100 mL), and the solution was washed with H₂O (3 x 75 mL), dried (MgSO₄), filtered, and concentrated under reduced pressure to give a white solid. Diethyl ether was added, and filtration gave the desired product as a white solid (155 mg, 25% yield). TLC (SiO₂, acetone): R_{f} = 0.16. MS (ESI): mass calculated for C₂₆H₂₇N₃O₃, 429.21; m/z found, 430.2 [M+H]⁺. ¹H NMR (400 MHz, DMSO-*d*₆): 12.23 (br s, 1H), 7.48 (d, *J* = 7.3, 2H), 7.35-7.25 (m, 6H), 7.20 (t, *J* = 7.4, 1H), 7.10-7.04 (m, 2H), 7.00 (d, *J* = 9.0, 2H), 4.80 (s, 1H), 4.13 (t, *J* = 5.8, 2H), 2.76 (t, *J* = 5.8, 2H), 2.58-2.48 (m, 2H), 1.94 (dt, *J* = 12.7, 4.0, 2H), 1.58 (d, *J* = 12.1, 2H).

### EXAMPLE 38

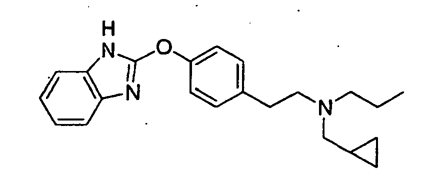

{2-[4-(1*H*-Benzoimidazol-2-yloxy)-phenyl]-ethyl}-cyclopropylmethyl-propyl-amine.
A. 4-[2-(Cyclopropylmethyl-propyl-amino)-ethyl]-phenol. To a solution of 4-(2-bromo-ethyl)-phenol (EXAMPLE 5; 5.0 g, 25.0 mmol) in CH₃CN (100 mL) was added cyclopropylmethyl-propyl-amine (5.4 mL, 37.5 mmol), followed by *N*,*N-*diisopropylethylamine (8.70 mL, 50.0 mmol). The resulting solution was stirred at 60°C for 16 h, yielding a suspension, which was cooled to room temperature and filtered. The filtered solid was washed with ethyl acetate (2 x 20 mL) and dried to give a white solid (5.7 g, 98% yield). MS (ESI): mass calculated for C₁₅H₂₃NO, 233.18; m/z found, 234.1 [M+H]⁺. ¹H NMR (400 MHz, DMSO-*d*₆): 7.01 (d, *J* = 8.6, 2H); 6.87 (d, *J* = 8.6, 2H), 3.34-3.30 (m, 2H), 3.19-3.04 (m, 2H), 2.94-2.75 (m, 4H), 1.75-1.56 (m, 2H), 1.16-0.99 (m, 1H), 0.95-0.87 (m, 3H), 0.68-0.53 (m, 2H), 0.44-0.32 (m, 2H).
B. {2-[4-(1*H*-Benzoimidazol-2-yloxy)-phenyl]-ethyl}-cyclopropylmethyl-propyl-amine. A mixture of 2-chloro-1-(2-trimethylsilanyl-ethoxymethyl)-1*H-*benzoimidazole (EXAMPLE 7; 707 mg, 2.5 mmol), 4-[2-(cyclopropylmethyl-propyl-amino)-ethyl]-phenol (467 mg, 2.0 mmol), and Cs₂CO₃ (1.3 g, 4.0 mmol) in DMF (10 mL) was stirred at 100 °C for 18 h. The reaction mixture was cooled to room temperature, and then partitioned in 1:1 ethyl acetate/H₂O (200 mL). The organic layer was collected, dried (MgSO₄), and concentrated under reduced pressure to give a clear brown oil, which was purified on SiO₂ (40 g; 0-100% acetone/CH₂Cl₂), giving 729 mg (76% yield) of a clear golden oil. MS (ESI): mass calculated for C₂₈H₄₁N₃O₂Si, 479.30; m/z found, 480.5 [M+H]⁺. ¹H NMR (400 MHz, CDCl₃): 7.63-7.58 (m, 1H), 7.49-7.39 (m, 1H), 7.31 (s, 4H), 7.26-7.22 (m, 2H), 5.58 (s, 2H), 3.69 (t, *J* = 8.2, 2H), 2.89-2.79 (m, 4H), 22.63-2.58 (m, 2H), 2.48 (d, *J* = 6.5, 2H), 1.61-1.50 (m, 2H), 1.01-0.91 (m, 6H), 0.59-0.53 (m, 2H), 0.20-0.15 (m, 2H), 0.1 (s, 9H).
C. {2-[4-(1*H*-Benzoimidazol-2-yloxy)-phenyl]-ethyl}-cyclopropylmethyl-propyl-amine. To a solution of cyclopropylmethyl-propyl-(2-{4-[1-(2-trimethylsilanyl-ethoxymethyl)-1*H*-benzoimidazol-2-yloxy]-phenyl}-ethyl)-amine (411 mg, 0.87 mmol) in THF (5 mL), TBAF (1 M in THF, 2.57 mL, 2.57 mmol) was added via syringe, and the mixture was stirred at reflux for 16 h. The reaction mixture was cooled to room temperature and concentrated under reduced pressure. The resulting oil was purified on SiO₂ (40 g; 0-20% CH₃OH/CH₂Cl₂), giving 284 mg (95% yield) of a clear oil. MS (ESI): mass calculated for C₂₂H₂₇N₃O, 349.22; m/z found, 350.4 [M+H]⁺. ¹H NMR (400 MHz, CDCl₃): 7.34 (br s, 2H), 7.18-7.05 (m, 6H), 2.82-2.70 (m, 4H), 2.69-2.62 (m, 2H), 2.52 (d, *J* = 6.8, 2H), 1.61-1.50 (m, 2H), 0.91 (t, *J* = 6.8, 4H), 0.57-0.51 (m, 2H), 0.16 (dd, *J* = 5.3, 5.1, 2H).

### EXAMPLE 39

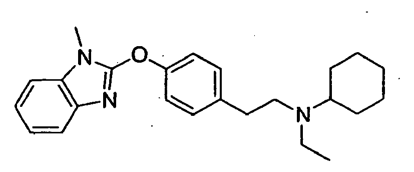

Cyclohexyl-ethyl-{2-[4-(1-methyl-1*H*-benzoimidazol-2-yloxy)-phenyl]-ethyl}-amine.

A mixture of 4-[2-(cyclohexyl-ethyl-amino)-ethyl]-phenol (247 mg, 1.0 mmol), *N-*methyl benzimidazole (EXAMPLE 8; 200 mg, 1.2 mmol), and Cs₂CO₃ (652 mg, 2.0 mmol) in DMF (3 mL) was stirred at 100 °C for 16 h. The reaction mixture was cooled and filtered through diatomaceous earth, which was then washed with ethyl acetate (30 mL). The combined filtrates were washed with H₂O (3 x 10 mL) then brine (10 mL), dried (Na₂SO₄), filtered, and concentrated under reduced pressure. The crude material was purified on SiO₂ (10 g; 0-100% 10% [2 M NH₃ in CH₃OH] in CH₂Cl₂/CH₂Cl₂) to provide 105 mg (28% yield) of a brown oil. MS (ESI): mass calculated for C₂₄H₃₁N₃O, 377.53; m/z found, 378.4 [M+H]⁺. ¹H NMR (400 MHz, CDCl₃): 7.52 (d, *J* =7.2, 1H), 7.63-7.58 (m, 1H), 7.33-7.18 (m, 7H), 3.75 (s, 3H), 2.81-2.70, m, 4H), 2.68 (q, *J* = 7.1, 2H), 2.60-2.50 (m, 1H), 1.90-1.80 (m, 4H), 1.67 (d, *J* = 12.3, 1H), 1.35-1.18 (m, 4H), 1.15-1.05 (m, 1H), 1.12 (t, *J* = 7.1, 3H).

### EXAMPLE 40

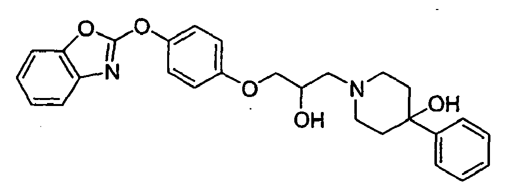

1-{3-[4-(Benzooxazol-2-yloxy)-phenoxy]-2-hydroxy-propyl}-4-phenyl-piperidin-4-ol.
A. 2-(4-Benzyloxy-phenoxymethyl)-oxirane. To a solution of 4-(benzyloxy)phenol (15.0 g, 74.9 mmol) and epichlorohydrin (30 mL, 384 mmol) in DMF (200 mL) was added Cs₂CO₃ (51.2 g, 157 mmol). The mixture was heated to 75 °C for 3 days. The reaction mixture was cooled to room temperature, and then partitioned in 1:1 ethyl acetate/H₂O (600 mL). The organic layer was collected, washed with H₂O (3 x 250 mL), dried (MgSO₄), filtered, and concentrated under reduced pressure to give 38 g of a clear, dark brown liquid. The liquid was purified on SiO₂ (300 g; 50-100% CH₂Cl₂/hexanes) to give the desired product as a white solid (13 g, 68% yield). TLC (SiO₂, CH₂Cl₂): R_{f} = 0.64. ¹H NMR (400 MHz, CDCl₃): 7.55-7.28 (m, 5H), 6.91 (d, *J* = 9.3, 2H), 6.86 (d, *J* = 9.3, 2H), 5.03 (s, 2H), 4.17 (dd, *J* = 11.0, 3.2, 1H), 3.95-3.88 (m, 1H), 3.36-3.33 (m, 1H), 2.91 (t, *J* = 4.8, 1H), 2.75 (dd, *J* = 4.9, 2.6, 1H).
B. 1-[3-(4-Benzyloxy-phenoxy)-2-hydroxy-propyl]-4-phenyl-piperidin-4-ol. To a solution of 2-(4-benzyloxy-phenoxymethyl)-oxirane (1.50 g, 5.85 mmol) and 4-hydroxy-4-phenylpiperidine (1.30 g, 7.33 mmol) in CH₃CN (50 mL) was added K₂CO₃ (1.0 g, 7.23 mmol). The mixture was heated at reflux for 20 h. The reaction mixture was filtered, and the filtrate was concentrated under reduced pressure to give a white solid, which was purified on SiO₂ (40 g; acetone) to give 1.4 g (56% yield) of the desired product as a white solid. TLC (SiO₂, acetone): R_{f} = 0.36. MS (ESI): mass calculated for C₂₇H₃₁NO₄, 433.23; m/z found, 434.3 [M+H]⁺. ¹H NMR (400 MHz, DMSO-*d*₆): 7.56-7.23 (m, 10 H), 6.93 (d, *J* = 9.2, 2H), 6.88 (d, *J* = 9.2, 2H), 5.03 (s, 2H), 4.77 (s, 2H), 3.93 (br d, *J* = 10.8, 2H), 3.81 (t, *J* = 7.2, 1H), 2.80-2.60 (m, 2H), 2.50-2.35 (m, 4H), 1.97-1.90 (m, 2H), 1.60-1.50 (d, *J* = 13.4, 2H).
C. 1-[2-Hydroxy-3-(4-hydroxy-phenoxy)-propyl]-4-phenyl-piperidin-4-ol. To a solution of 1-[3-(4-benzyloxy-phenoxy)-2-hydroxy-propyl]-4-phenyl-piperidin-4-ol (1.3 g, 3.0 mmol) in 1:1 ethanol/ethyl acetate (50 mL) was added Pd on carbon (10 wt %, 165 mg). The mixture was placed on a Parr hydrogenator at 40 psi of H₂ for 20 h. The reaction mixture was filtered through diatomaceous earth, and the filtrate was concentrated under reduced pressure to give 1.0 g (100% yield) of desired product as a white solid. TLC (SiO₂, acetone): R_{f} = 0.27. MS (ESI): mass calculated for C₂₀H₂₅NO₄, 343.18; m/z found, 344.3 [M+H]⁺. ¹H NMR (400 MHz, CD₃OD): 7.61 (d, *J* = 8.1, 2H), 7.42 (t, *J* = 7.4, 2H), 7.30 (t, *J* = 7.3, 1H), 6.90 (d, *J* = 9.0, 2H), 6.81 (d, *J* = 9.0, 2H), 4.25 (br s, 1H), 4.05-3.95 (m, 2H), 3.10-2.92 (m, 2H), 2.80-2.60 (m, 4H), 2.30-2.20 (m, 2H), 1.81 (d, *J* = 11.8, 2H).
D. 1-{3-[4-(Benzooxazol-2-yloxy)-phenoxy]-2-hydroxy-propyl}-4-phenyl-piperidin-4-ol. To a mixture of 1-[2-hydroxy-3-(4-hydroxy-phenoxy)-propyl]-4-phenyl-piperidin-4-ol (251 mg, 0.73 mmol) and Cs₂CO₃ (667 mg, 2.05 mmol) in acetone (10 mL) at 5 °C, was added 2-chloro-benzooxazole (108 µL, 0.95 mmol). The reaction mixture, left on ice, warmed to room temperature overnight and was then filtered. The filtrate was concentrated under reduced pressure to give a golden oil, which was purified on SiO₂ (10 g; acetone) to give 127 mg (38% yield) of the desired product as a white solid. TLC (SiO₂, acetone): R_{f} = 0.27. MS (ESI): mass calculated for C₂₇H₂₈N₂O₅, 460.20; m/z found, 461.3 [M+H]⁺. ¹H NMR (400 MHz, DMSO-*d*₆): 7.60 (d, *J* = 6.6, 1H), 7.55-7.35 (m, 5H), 7.32-7.25 (m, 4H), 7.20 (t, *J* = 7.3, 1H), 7.06 (d, *J* = 9.1, 2H), 4.78 (s, 1H), 4.10-3.85 (m, 3H), 2.80-2.65 (m, 2H), 2.55-2.35 (m, 4H), 1.95 (t, *J* = 13.1, 2H), 1.59 (d, *J* = 13.2, 2H).

### EXAMPLE 41

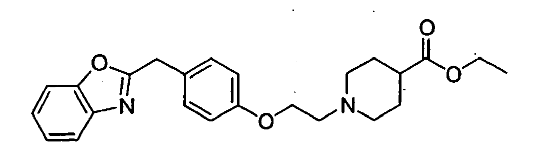

1-[2-(4-Benzooxazol-2-ylmethyl-phenoxy)-ethyl]-piperidine-4-carboxylic acid ethyl ester.
A. 4-Benzooxazol-2-ylmethyl-phenol. A mixture of 4-hydroxyphenylacetic acid (35 g, 230 mmol) and 2-aminophenol (43 g, 400 mmol) was heated at 180°C for 3 h and then cooled to room temperature. The resultant solid was ground and dissolved in THF (200 mL), and carbonyldiimidazole (27 g, 170 mmol) was added. The solution was stirred at 60 °C overnight. The reaction mixture was concentrated under reduced pressure, and partitioned between ethyl acetate (400 mL) and H₂O (300 mL). The organic layer was concentrated under reduced pressure. The resultant oil was dissolved in CH₂Cl₂ and purified on SiO₂ (200 g; 0-50% acetone/CH₂Cl₂) to give a brown solid (31 g, 40% yield): MS (ESI): mass calculated for C₁₄H₁₁NO₂, 225.08; m/z found, 226.1 [M+H]⁺. ¹H NMR (400 MHz, CD₃OD): 7.62 (m, 1H), 7.53 (m, 1H), 7.32 (m, 2H), 7.17 (d, *J* = 8.5, 2H), 6.75 (d, *J* = 8.5, 2H), 4.18 (s, 2H).
B. 2-[4-(2-Bromo-ethoxy)-benzyl]-benzooxazole. To a stirring solution of 4-benzooxazol-2-ylmethyl-phenol (5 g, 22.2 mmol) in CH₃CN (100 mL) was added Na₂CO₃ (6.4 g, 46.6 mmol) and dibromoethane (7.9 mL, 88.8 mmol). The resulting suspension was heated to 70°C for 72 h and filtered while hot. The filtrate was concentrated under reduced pressure. The resulting solid was suspended in diethyl ether and filtered, and the filtrate was concentrated under reduced pressure. The resultant oil was dissolved in CH₂Cl₂ and purified on SiO₂ (110 g; CH₂Cl₂) to give an off-white solid (1g, 13.7% yield). MS (ESI): mass calculated for C₁₆H₁₄BrNO₂, 331.02; m/z found, 332.0 [M+H]⁺. ¹H NMR (400 MHz, CD₃OD): 7.62 (m, 1H), 7.53 (m, 1H), 7.34 (m, 2H), 7.29 (d, *J* = 8.6, 2H), 6.92 (d, *J* = 8.7, 2H), 4.28 (t, *J* = 5.9, 2H), 4.23 (s, 2H), 3.67 (t, *J* = 5.9, 2H).
C. 1-[2-(4-Benzooxazol-2-ylmethyl-phenoxy)-ethyl]-piperidine-4-carboxylic acid ethyl ester. To a stirring solution of 2-[4-(2-bromo-ethoxy)-benzyl]-benzooxazole (0.5 g, 1.5 mmol) in CH₃CN (7.5 mL) was added ethyl isonepicotate (0.7 mL, 4.5 mmol). The mixture was stirred at room temperature for 48 h, then concentrated under reduced pressure. The resultant oil was dissolved in CH₂Cl₂ and purified on SiO₂ (40 g; 0-25% acetone/CH₂Cl₂) to give an off-white solid (275 mg, 45% yield). MS (ESI): mass calculated for C₂₄H₂₈N₂O₄, 408.20; m/z found, 409.2 [M+H]⁺. ¹H NMR (400 MHz, CDCl₃): 7.67 (m, 1H), 7.45 (m, 1H), 7.28 (d, *J* = 9.0, 4H), 6.88 (d, *J* = 8.7, 2H), 4.20 (s, 2H), 4.12 (q, *J* = 7.1, 2H), 4.08 (t, *J* = 5.9, 2H), 2.95 (d, *J* = 11.5, 2H), 2.77 (t, *J* = 5.9, 2H), 2.24 (m, 1H), 2.16 (m, 2H), 1.84 (m, 2H), 1.76 (m, 2H). 1.24 (t, *J* = 7.2, 3H).

### EXAMPLE 42

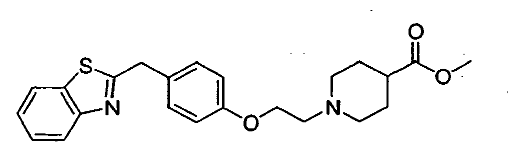

1-[2-(4-Benzothiazol-2-ylmethyl-phenoxy)-ethyl]-piperidine-4-carboxylic acid methyl ester.
A. 4-Benzothiazol-2-ylmethyl-phenol. A mixture of 4-hydroxyphenylacetic acid (15.2 g, 100 mmol) and 2-amino-benzenethiol (10.7 mL, 100 mmol) was heated at 150°C for 16 h and then cooled to room temperature. The resultant solid was ground and dissolved in CH₂Cl₂ (400 mL). The solution was washed with 1 N HCl (2 x 50 mL) then sat. aq. NaHCO₃ (2 x 50 mL), dried, filtered, and concentrated under reduced pressure. The residue was purified on SiO₂ (0-50% ethyl acetate/hexanes) to give a white solid (10.5 g, 44% yield). MS (ESI): mass calculated for C₁₄H₁₁NOS, 241.06; m/z found, 342.1 [M+H]⁺. ¹H NMR (400 MHz, CDCl₃): 7.89 (dd, *J* = 8.6, 4.8, 2H), 7.47 (t, *J* = 7.8, 1 H), 7.31 (t, *J* = 7.8, 1H), 7.18 (d, *J* = 8.3, 2H), 6.77 (d, *J* = 8.6, 2H), 4.89 (s, 1H), 4.33 (s, 2H).
B. 2-[4-(2-Bromo-ethoxy)-benzyl]-benzothiazole. To a stirring solution of 4-benzothiazol-2-ylmethyl-phenol (10.5 g, 43.5 mmol) in CH₃CN (100 mL) was added Cs₂CO₃ (28.3 g, 87 mmol) and dibromoethane (18.7 mL, 21.8 mmol). The resulting suspension was heated to 70 °C for 16 h, cooled to room temperature, and then dissolved in CH₂Cl₂ (400 mL). The solution was washed with H₂O (2 x 50 mL), dried, and concentrated. The resultant oil was dissolved in CH₂Cl2 and purified on SiO₂ (0-50% ethyl acetate/hexanes) to give a white solid (7.5 g, 49.5% yield). MS (ESI): mass calculated for C₁₆H₁₄BrNOS, 347.00; m/z found, 348.1 [M+H]⁺. ¹H NMR (400 MHz, CDCl₃): 7.99 (d, *J* = 8.1, 1H), 7.79 (d, *J* = 8.1, 1H), 7.45 (t, *J* = 7.6, 1H), 7.36-7.25 (m, 3H), 6.89 (d, *J* = 8.8, 2H), 4.38 (s, 2H), 4.28 (t, *J* = 6.3, 2H), 3.63 (t, *J* = 6.3, 2H).
C. 1-[2-(4-Benzothiazol-2-ylmethyl-phenoxy)-ethyl]-piperidine-4-carboylic acid methyl ester. To a stirring solution of 2-[4-(2-bromo-ethoxy)-benzyl]-benzothiazole (1.0 g, 2.9 mmol) in CH₃CN (20 mL) was added methyl isonepicotate (0.7 mL, 55.7 mmol) and *N*,*N*-diisopropylethylamine (1.5 mL, 8.6 mmol). The mixture was heated to 60 °C for 16 h, cooled to room temperature, and then dissolved in CH₂Cl₂ (100 mL). The solution was washed with H₂O (2 x 20 mL), dried, and concentrated. The resultant oil was dissolved in CH₂Cl₂ and purified on SiO₂ (0-15% CH₃OH/CH₂Cl₂) to give a white solid (1.1 g, 92% yield). MS (ESI): mass calculated for C₂₃H₂₆N₂O₃S, 410.17; m/z found, 411.1 [M+H]⁺. ¹H NMR (400 MHz, CDCl₃): 7.98 (d, *J* = 8.3, 1H), 7.76 (d, *J* = 8.3, 1H), 7.42 (t, *J* = 7.8, 1H), 7.33-7.24 (m, 3H), 6.88 (d, J = 8.8, 2H), 4.36 (s, 2H), 4.07 (t, *J* = 6.1, 2H), 3.66 (s, 3H), 2.97-2.90 (m, 2H), 2.76 (t, *J* = 6.1, 2H), 2.33-2.24 (m 1H), 2.15 (t, *J* = 11.4, 2H), 1.93-1.86 (m, 2H), 1.83-1.72 (m, 2H).

### Example 43

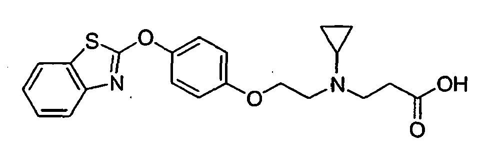

3-({2-[4-(Benzothiazol-2-yloxy)-phenoxy]-ethyl}-cyclopropyl-amino)-propionic acid trifluoroacetic acid salt.
A. 3-[2-(4-Hydroxy-phenoxy)-ethylamino]-propionic acid ethyl ester. To a stirring solution of 4-(2-bromo-ethoxy)-phenol (EXAMPLE 3; 2.4 g, 11 mmol) in CH₃CN (50 mL) was added 3-amino-propionic acid ethyl ester hydrochloride (3.4 g, 22 mmol) followed by *N*,*N*-diisopropylethylamine (7.7 mL, 44 mmol). The mixture was stirred at 60 °C for 16 h, allowed to cool to room temperature, and then dissolved in CH₂Cl₂ (100 mL). The resulting solution was washed with H₂O (2 x 15 mL), dried, filtered, and then concentrated under reduced pressure to afford a brown oil, which was used in the next step.
B. 3-{2-[4-(Benzothiazol-2-yloxy)-phenoxy]-ethylamino}-propionic acid ethyl ester. To a stirring solution of 3-[2-(4-hydroxy-phenoxy)-ethylamino]-propionic acid ethyl ester (11 mmol) in DMF (30 mL), was added Cs₂CO₃ (10.8 g, 33 mmol) and 2-chlorobenzothiazole (2.72 mL, 22 mmol). The suspension was stirred at 100 °C overnight. The reaction mixture was allowed to cool to room temperature and then filtered through diatomaceous earth. The filtrate was concentrated under reduced pressure, and purified on SiO₂ (100 g; 0-100% CH₃OH/CH₂Cl₂), giving 1.9 g (45% yield) of a light brown oil. MS (ESI): mass calculated for C₂₂H₂₂N₂O₄S, 386.13; m/z found, 387.1 [M+H]⁺. ¹H NMR (400 MHz, CDCl₃): 7.66 (d, *J* = 8.1, 1H), 7.58 (d, *J* = 8.3, 1H), 7.30 (d, *J* = 7.8, 1H), 7.22-7.15 (m, 3H), 6.86 (d, *J* = 9.1, 2H), 4.09 (dd, *J* = 7.1, 7.1, 2H), 4.01 (t, *J* = 5.3, 2H), 2.98 (t, *J* = 5.3, 2H), 2.92 (t, *J* = 6.6, 2H), 2.49 (t, *J* = 6.6, 2H), 2.20 (br s, 1H), 1.20 (t, *J* = 7.1, 3H).
C. 3-({2-[4-(Benzothiazol-2-yloxy)-phenoxy]-ethyl}-cyclopropyl-amino)-propionic acid ethyl ester. To a solution of 3-{2-[4-(benzothiazol-2-yloxy)-phenoxy]-ethylamino}-propionic acid ethyl ester (500 mg, 1.29 mmol) in CH₃OH (15 mL) was added acetic acid (0.73 mL, 12.9 mmol), 3 Å molecular sieves and [(1-ethoxycyclopropyl)oxy]trimethylsilane (1.55 mL, 7.7 mmol). Sodium cyanoborohydride (365 mg, 5.8 mmol) was added, and the mixture was heated at reflux overnight. The mixture was cooled, filtered and concentrated. The residue was dissolved in CH₂Cl₂, and the resulting solution was washed with sat. aq. NaHCO₃ then brine, dried, and concentrated. This residue was purified on SiO₂ (40 g; 10-50% ethyl acetate/hexanes), giving 374 mg (68% yield) of a colorless oil. MS (ESI): mass calculated for C₂₃H₂₆N₂O₄S, 426.16; m/z found, 427.1.1 [M+H]⁺. ¹H NMR (400 MHz, CDCl₃): 7.73 (d, *J* = 8.3, 1H), 7.64 (d, *J* = 8.1, 1H), 7.38 (d, *J* = 7.8, 1H), 7.29-7.22 (m, 3H), 6.94 (d, *J* = 9.1, 2H), 4.14 (dd, *J* = 7.1, 7.1, 2H), 4.10 (t, *J* = 6.1, 2H), 3.07 (t, *J* = 7.1, 2H), 3.05 (t, *J* = 6.1, 2H), 2.58 (t, *J* = 7.3, 2H), 1.92-1.86 (m, 1H), 1.26 (t, *J* = 7.1, 3H), 0.54-0.43 (m, 4H).
D. 3-({2-[4-(Benzothiazol-2-yloxy)-phenoxy]-ethyl}-cyclopropyl-amino)-propionic acid trifluoroacetic acid salt. To a solution of 3-({2-[4-(benzothiazol-2-yloxy)-phenoxy]-ethyl}-cyclopropyl-amino)-propionic acid ethyl ester (355 mg, 11.7 mmol) in THF (15 mL) and CH₃OH (5 mL), was added lithium hydroxide (80 mg, 3.3 mmol) in H₂O (10 mL). The mixture was stirred at room temperature for 16 h, and then concentrated under reduced pressure. The residue was dissolved in CH₃OH and was purified by reversed-phase HPLC to give the title compound (391 mg, 92% yield). MS (ESI): mass calculated for C₂₁H₂₂N₂O₄S, 398.13; m/z found, 399.1 [M+H]⁺. ¹H NMR (400 MHz, CD₃OD): 7.73 (d, *J* = 8.3, 1H), 7.64 (d, *J* = 8.1, 1H), 7.48 (d, *J* = 7.8, 1H), 7.35-7.25 (m, 3H), 7.11 (d, *J* = 9.1, 2H), 5.17 (br s, 1H), 4.48 (t, *J* = 4.6, 2H), 3.81 (t; *J* = 4.6, 2H), 3.76 (t, *J* = 7.1, 2H), 3.04-2.93 (m, 3H), 1.17-1.10 (m, 2H), 1.05-0.97 (m, 2H).

### EXAMPLE 44

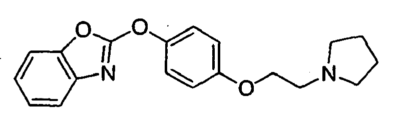

2-[4-(2-Pyrrolidin-1-yl-ethoxy)-phenoxy]-benzooxazole.

The title compound was prepared according to the procedure for EXAMPLE 11 using 1-(2-chloro-ethyl)-pyrrolidine. MS (ESI): mass calculated for C₁₉H₂₀N₂O₃, 324.15; m/z found, 325.1 [M+H]⁺. ¹H NMR (400 MHz, CDCl₃): 7.75 (d, *J* = 8.1, 1H), 7.66 (d, *J* = 8.0, 1H), 7.40 (dt, *J* = 7.4, 1.3, 1H), 7.31-7.23 (m, 3H), 6.98 (d, *J* = 6.8, 2H), 4.08 (t, *J* = 6.3, 2H), 2.91 (t, *J* = 6.3, 2H), 2.67 (q, *J* = 7.2, 4H), 1.10 (t, *J* = 7.2, 6H).

### EXAMPLE 45

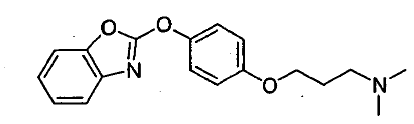

{3-[4-(Benzooxazol-2-yloxy)-phenoxy]-propyl}-dimethyl-amine.

The title compound was prepared according to the procedure for EXAMPLE 11 using (2-chloro-propyl)-dimethyl-amine hydrochloride. MS (ESI): mass calculated for C₁₈H₂₀N₂O₃, 312.15; m/z found, 313.1 [M+H]⁺. ¹H NMR (400 MHz, CDCl₃): 7.48-7.45 (m, 1H), 7.40-7.38 (m, 1H), 7.30 (d, *J* = 7.9, 2H), 7.23-7.18 (m, 2H), 6.91 (d, *J* = 8.3, 2H), 4.09 (br s, 2H), 3.23 (br s, 2H), 2.85 (br s, 6H), 2.42 (br s, 2H).

### EXAMPLE 46

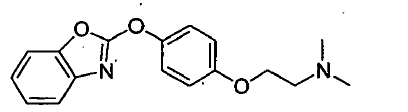

{2-[4-(Benzooxazol-2-yloxy)-phenoxy]-ethyl}-dimethyl-amine.

The title compound was prepared according to the procedure for EXAMPLE 11 using (2-chloro-ethyl)-dimethyl-amine hydrochloride. MS (ESI): mass calculated for C₁₇H₁₈N₂O₃, 298.13; m/z found, 299.1 [M+H]⁺. ¹H NMR (400 MHz, CDCl₃): 7.49-7.47 (m, 1H), 7.41-7.38 (m, 1H), 7.32-7.27 (m, 2H), 7.26-7.18 (m, 2H), 6.98-6.95 (m, 2H), 4.05 (t, *J* = 5.7, 2H), 2.72 (t, *J* = 5.7, 2H), 2.32 (s, 6H).

### EXAMPLE 47

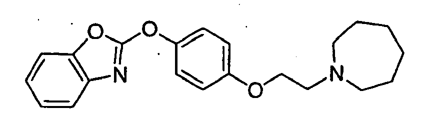

2-[4-(2-Azepan-1-yl-ethoxy)-phenoxy]-benzooxazole.

The title compound was prepared according to the procedure for EXAMPLE 11 using 1-(2-chloro-ethyl)-azepane hydrochloride. MS (ESI): mass calculated for C₂₁H₂₄N₂O₃, 352.18; m/z found, 353.2 [M+H]⁺. ¹H NMR (400 MHz, CDCl₃): 7.49-7.47 (m, 1H), 7.41-7.38 (m, 1H), 7.31-7.27 (m, 2H), 7.26-7.18 (m, 2H), 6.97-6.93 (m, 2H), 4.06 (t, *J* = 6.2, 2H), 2.94 (t, *J* = 6.2, 2H), 2.77-2.75 (m, 4H), 1.65-1.58 (m, 8H).

### EXAMPLE 48

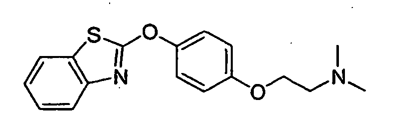

{2-[4-(Benzothiazol-2-yloxy)-phenoxy]-ethyl}-dimethyl-amine.

The title compound was prepared according to the procedure for EXAMPLE 12 using (2-chloro-ethyl)-dimethyl-amine hydrochloride and 2-chlorobenzothiazole. MS (ESI): mass calculated for C₁₇H₁₈N₂O₂S, 314.11; m/z found, 315.1 [M+H]⁺. ¹H NMR (400 MHz, CD₃OD): 7.79-7.77 (m, 1H), 7.65-7.63 (m, 1H), 7.45-7.29 (m, 4H), 7.18-7.14 (m, 2H), 4.41 (t, *J* =4.9, 2H), 3.63 (t, *J* = 4.9, 2H), 3.04 (s, 6H).

### EXAMPLE 49

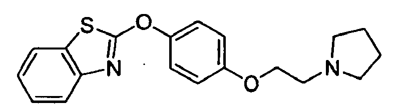

2-[4-(2-Pyrrolidin-1-yl-ethoxy)-phenoxy]-benzothiazole.

The title compound was prepared according to the procedure for EXAMPLE 12 using 1-(2-chloro-ethyl)-pyrrolidine and 2-chlorobenzothiazole. MS (ESI): mass calculated for C₁₉H₂₀N₂O₂S, 340.12; m/z found, 341.1. [M+H]⁺. ¹H NMR (400 MHz, CDCl₃): 7.77 (d, *J* = 7.6, 1H), 7.66 (d, *J* = 8.6, 1H), 7.41 (t, *J* = 7.4, 1H), 7.32-7.25 (m, 3H), 7.00 (d, *J* = 9.0, 2H), 4.16 (t, *J* = 6.0, 2H), 2.96 (t, *J* = 6.0, 2H), 2.67 (t, *J* = 6.6, 4H), 1.86 (quint, *J* = 3.5, 4H).

### EXAMPLE 50

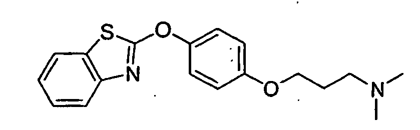

{3-[4-(Benzothiazol-2-yloxy)-phenoxy]-propyl}-dimethyl-amine.

The title compound was prepared according to the procedure for EXAMPLE 12 using (2-chloro-propyl)-dimethyl-amine hydrochloride and 2-chlorobenzothiazole. MS (ESI): mass calculated for C₁₈H₂₀N₂O₂S, 328.12; m/z found, 329.1 [M+H]⁺. ¹H NMR (400 MHz, CDCl₃): 7.72 (d, *J* = 8.1, 1H), 7.64 (d, *J* = 7.9, 1H), 7.37 (t, *J* = 7.5, 1H), 7.31-7.20 (m, 3H), 6.92 (d, *J* = 7.9, 2H), 4.11 (s, 2H), 3.25 (s, 2H), 2.85 (s, 6H), 2.43 (s, 2H).

### EXAMPLE 51

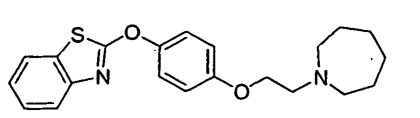

2-[4-(2-Azepan-1-yl-ethoxy)-phenoxy]-benzothiazole.

The title compound was prepared according to the procedure for EXAMPLE 12 using 1-(2-chloro-ethyl)-azepane hydrochloride and 2-chlorobenzothiazole. MS (ESI): mass calculated for C₂₁H₂₄N₂O₂S, 368.16; m/z found, 369.2 [M+H]⁺. ¹H NMR (400 MHz, CDCl₃): 7.70 (d, *J* = 7.5, 1H), 7.65 (d, *J* = 7.9, 1H), 7.38-7.34 (m, 1H), 7.29-7.23 (m, 3H), 6.97 (br d, 2H), 4.59 (br s, 2H), 3.64 (br s, 2H), 3.46 (br s, 2H), 3.13 (br s, 3H), 2.19 (br s, 2H), 1.87 (br s, 2H), 1.72-1.58 (m, 2H).

### EXAMPLE 52

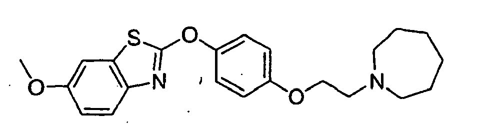

2-[4-(2-Azepan-1-yl-ethoxy)-phenoxy]-6-methoxy-benzothiazole.

The title compound was prepared according to the procedure for EXAMPLE 12 using 1-(2-chloro-ethyl)-azepane hydrochloride and 2-chloro-6-methoxy-benzothiazole. MS (ESI): mass calculated for C₂₂H₂₆N₂O₃S, 398.17; m/z found, 399.2 [M+H]⁺. ¹H NMR (400 MHz, CDCl₃): 7.75 (d, *J* = 8.9, 1H), 7.37-7.35 (m, 2H), 7.26 (br d, 2H), 7.09-7.05 (m, 3H), 4.19 (t, *J* = 6.1, 2H), 3.95 (s, 3H), 3.08 (t, *J* = 6.1, 2H), 2.91-2.88 (m, 4H), 1.76 (br d, 8H).

### EXAMPLE 53

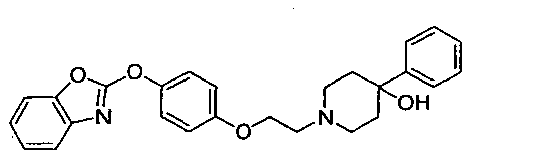

1-{2-[4-(Benzooxazol-2-yloxy)-phenoxy]-ethyl}-4-phenyl-piperidin-4-ol.

The title compound was prepared according to the procedure for EXAMPLE 13 using 4-phenyl-piperidin-4-ol. MS (ESI): mass calculated for C₂₆H₂₆N₂O₄, 430.19; m/z found, 431.2 [M+H]⁺. ¹H NMR (400 MHz, CDCl₃): 7.60-7.45 (m, 3H), 7.40-7.18 (m, 8H), 7.00 (d, *J* = 9.1, 2H), 4.18 (t, *J* = 5.9, 2H), 2.92 (t, *J* = 5.9, 2H), 2.66 (dt, *J* = 12.1, 2.4, 2H), 2.22 (dt, *J* = 13.4, 4.5, 2H), 1.80 (dd, *J* = 14.1, 2.4, 2H).

### EXAMPLE 54

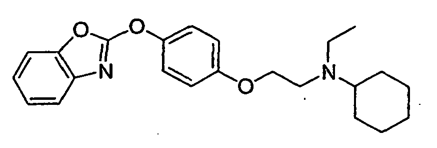

{2-[4-(Benzooxazol-2-yloxy)-phenoxy]-ethyl}-cyclohexyl-ethyl-amine.

The title compound was prepared according to the procedure for EXAMPLE 13 using cyclohexyl-ethyl-amine. MS (ESI): mass calculated for C₂₃H₂₈N₂O₃, 380.21; m/z found, 381.2 [M+H]⁺. ¹H NMR (400 MHz, CDCl₃): 7.51 (d, *J* = 8.4, 1H), 7.42 (d, *J* = 7.5, 1H), 7.35-7.18 (m, 4H), 6.97 (d, *J* = 9.1, 2H), 3.98 (t, *J* = 6.9, 2H), 2.88 (t, *J* = 6.9, 2H), 2.67 (q, *J* = 7.1, 2H), 2.55-2.50 (m, 1H), 1.82 (t, *J* = 7.4, 4H), 1.64 (d, *J* = 12.4, 1H), 1.21 (t, *J* = 7.9, 4H), 1.08 (t, *J* = 7.1, 4H).

### EXAMPLE 55

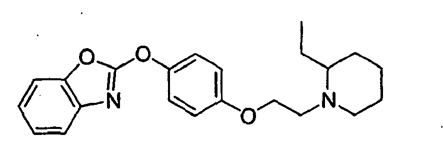

2-{4-[2-(2-Ethyl-piperidin-1-yl)-ethoxy]-phenoxy}-benzooxazole.

The title compound was prepared according to the procedure for EXAMPLE 13 using 2-ethyl-piperidine. MS (ESI): mass calculated for C₂₂H₂₆N₂O₃, 366.19; m/z found, 367.2 [M+H]⁺. ¹H NMR (400 MHz, CDCl₃): 7.51 (d, *J* = 7.4, 1 H), 7.42 (d, *J* = 7.4, 1 H), 7.31 (d, *J* = 9.1, 2H), 7.30-7.20 (m, 2H), 6.97 (d, *J* = 9.1, 2H), 4.09 (t, *J* = 6.4, 2H), 3.15-3.05 (m, 1H), 3.00-2.92 (m, 1H), 2.88-2.80 (m, 1H), 2.48-2.37 (m, 1H), 2.30-2.25 (m, 1H), 1.75-1.65 (m, 4H), 1.60-1.54 (m, 2H), 1.52-1.42 (m, 1H), 1.38-1.24 (m, 2H), 0.92 (t, *J* = 7.4, 3H).

### EXAMPLE 56

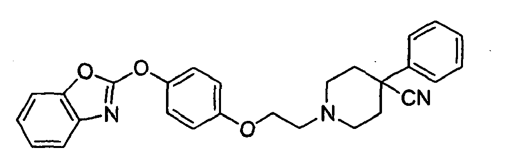

1-{2-[4-(Benzooxazol-2-yloxy)-phenoxy]-ethyl}-4-phenyl-piperidine-4-carbonitrile.

The title compound was prepared according to the procedure for EXAMPLE 13 using 4-phenyl-piperidine-4-carbonitrile. MS (ESI): mass calculated for C₂₇H₂₅N₃O₅, 439.19; m/z found, 440.2 [M+H]⁺. ¹H NMR (400 MHz, CDCl₃): 7.61-7.49 (m, 3H), 7.43-7.38 (m, 3H), 7.36-7.29 (m, 3H), 728-7.20 (m, 3H), 7.03-6.96 (m, 2H), 4.15 (t, *J* = 5.6, 2H), 3.18 (br d, 2H), 2.93 (t, *J* = 5.6, 2H), 2.71-2.61 (m, 2H), 2.22-2.10 (m, 8H).

### EXAMPLE 57

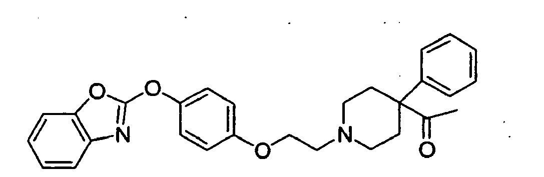

1-(1-{2-[4-(Benzooxazol-2-yloxy)-phenoxy]-ethyl}-4-phenyl-piperidin-4-yl)-ethanone.

The title compound was prepared according to the procedure for EXAMPLE 13 using 1-(4-phenyl-piperidin-4-yl)-ethanone. MS (ESI): mass calculated for C₂₈H₂₈N₂O₄, 456.20; m/z found, 457.2 [M+H]⁺. ¹H NMR (400 MHz, CDCl₃): 7.52-7.49 (m, 1H), 7.43-7.40 (m, 1H), 7.37-7.16 (m, 9H), 6.99-6.92 (m, 2H), 4.10 (t, *J* = 5.8, 2H), 2.87-2.82 (m, 2H), 2.78 (t, *J* = 5.8, 2H), 2.51-2.47 (m, 2H), 2.39 (br t, 2H), 2.17-2.05 (m, 2H), 1.92 (s, 3H).

### EXAMPLE 58

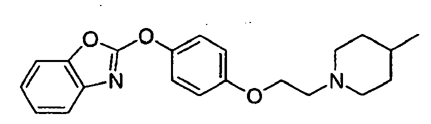

2-{4-[2-(4-Methyl-piperidin-1-yl)-ethoxy]-phenoxy}-benzooxazole.

The title compound was prepared according to the procedure for EXAMPLE 13 using 4-methyl-piperidine. MS (ESI): mass calculated for C₂₁H₂₄N₂O₃, 352.18; m/z found, 353.2 [M+H]⁺. ¹H NMR (400 MHz, CDCl₃): 7.50 (d, *J* = 6.8, 1H), 7.42 (d, *J* = 6.8, 1H), 7.31 (d, *J* = 9.0, 2H), 7.30-7.20 (m, 2H), 6.97 (d, *J* = 9.0, 2H), 4.13 (t, *J* = 6.0, 2H), 2.97 (d, *J* = 11.7, 2H), 2.80 (t, *J* = 6.0, 2H), 2.11 (dt, *J* = 11.7, *J* = 2.2, 2H), 1.67 (s, 2H), 1.34 (br s, 1H), 1.28 (dt, *J* = 12.2, *J* = 3.4, 2H), 0.94 (d, *J* = 6.2, 3H).

### EXAMPLE 59

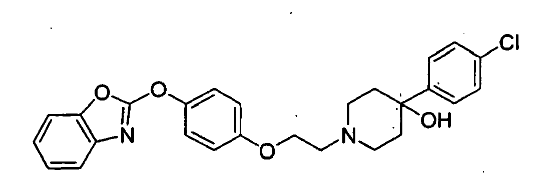

1-{2-[4-(Benzooxazol-2-yloxy)-phenoxy]-ethyl}-4-(4-chloro-phenyl)-piperidin-4-ol.

The title compound was prepared according to the procedure for EXAMPLE 13 using 4-(4-chloro-phenyl)-piperidin-4-ol. MS (ESI): mass calculated for C₂₆H₂₅ClN₂O₄, 464.15; m/z found, 465.1 [M+H]⁺. ¹H NMR (400 MHz, CDCl₃): 7.54-7.42 (m, 4H), 7.37-7.31 (m, 4H), 7.29-7.21 (m, 2H), 7.03-6.98 (m, 2H), 4.16 (t, *J* = 5.8, 2H), 2.93-2.89 (m, 4H), 2.62 (dt, *J* = 12.2, 2.4, 2H), 2.21-2.14 (m, 2H), 1.78-1.74 (m, 2H), 1.56 (br s, 1H).

### EXAMPLE 60

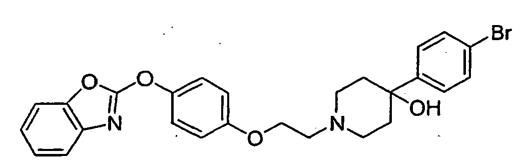

1-{2-[4-(Benzooxazol-2-yloxy)-phenoxy]-ethyl}-4-(4-bromo-phenyl)-piperidin-4-ol.

The title compound was prepared according to the procedure for EXAMPLE 13 using 4-(4-bromo-phenyl)-piperidin-4-ol. MS (ESI): mass calculated for C₂₆H₂₅BrN₂O₄, 508.10; m/z found, 509.1 [M+H]⁺. ¹H NMR (400 MHz, CDCl₃): 7.55-7.47 (m, 3H), 7.44-7.39 (m, 3H), 7.37-7.31 (m, 2H), 7.30-7.19 (m, 2H), 7.02-6.98 (m, 2H), 4.17 (t, *J* = 5.7, 2H), 2.94-2.89 (m, 4H), 2.62 (dt, *J* = 12.2, 2.4, 2H), 2.21-2.13 (m, 2H), 1.78-1.74 (m, 2H), 1.56 (br s, 1H).

### EXAMPLE 61

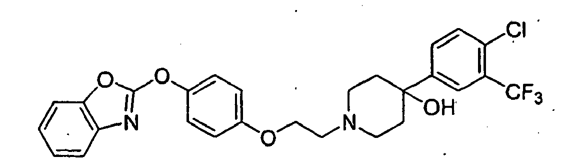

1-{2-[4-(Benzooxazol-2-yloxy)-phenoxy]-ethyl}-4-(4-chloro-3-trifluoromethylphenyl)-piperidin-4-ol.

The title compound was prepared according to the procedure for EXAMPLE 13 using 4-(4-chloro-3-trifluoromethyl-phenyl)-piperidin-4-ol. MS (ESI): mass calculated for C₂₇H₂₄ClF₃N₂O₄, 532.14; m/z found, 533.1 [M+H]⁺. ¹H NMR (400 MHz, CDCl₃): 7.89 (d, *J* = 2.1, 1H), 7.62 (dd, *J* = 8.4, 2.1, 1H), 7.51-7.41 (m, 3H), 7.33-7.21 (m, 4H), 6.99-6.95 (m, 2H), 4.14 (t, *J* = 5.7, 2H), 2.94-2.89 (m, 4H), 2.65-2.59 (m, 2H), 2.21-2.13 (m, 4H), 1.74 (br s, 1H).

### EXAMPLE 62

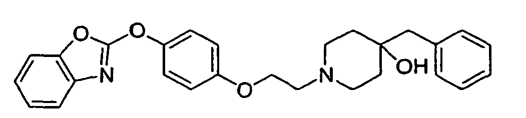

1-{2-[4-(Benzooxazol-2-yloxy)-phenoxy]-ethyl}-4-benzyl-piperidin-4-ol.

The title compound was prepared according to the procedure for EXAMPLE 13 using 4-benzyl-piperidin-4-ol. MS (ESI): mass calculated for C₂₇H₂₈N₂O₄, 444.20; m/z found, 445.1 [M+H]⁺. ¹H NMR: (400 MHz, CDCl₃) 7.53-7.50 (m, 1H), 7.43-7.40 (m, 1H), 7.53-7.21 (m, 10H), 7.01-6.95 (m, 2H), 4.12 (t, *J* = 5.9, 2H), 2.84 (t, *J* = 5.8, 2H), 2.78-2.76 (m, 4H), 2.47 (dt, *J* = 11.6, 2.4, 2H), 1.83-1.75 (m, 2H), 1.59-1.53 (m, 2H).

### EXAMPLE 63

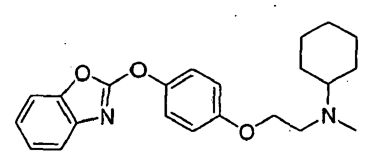

{2-[4-(Benzooxazol-2-yloxy)-phenoxy]-ethyl}-cyclohexyl-methyl-amine.

The title compound was prepared according to the procedure for EXAMPLE 13 using cyclohexyl-methyl-amine. MS (ESI): mass calculated for C₂₂H₂₆N₂O₃, 366.19; m/z found, 367:3 [M+H]⁺. ¹H NMR (400 MHz, DMSO-*d*₆): 7.62 (dd, *J* = 6.2, *J* = 2.0, 1H), 7.50 (dd, *J* = 6.1, 2.1, 1H), 7.42 (d, *J* = 9.1, 2H), 7.35-7.25 (m, 2H), 7.03 (d, *J* = 9.1, 2H), 4.04 (t, *J* = 6.1, 2H), 2.79 (t, *J* = 6.1, 2H), 2.45-2.32 (m, 1H), 1.73 (d, *J* = 7.9, 4H), 1.57 (d, *J* = 12.2, 2H), 1.30-1.12 (m, 4H), 1.10-1.00 (m, 1H).

### EXAMPLE 64

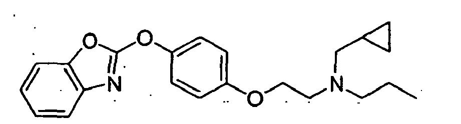

{2-[4-(Benzooxazol-2-yloxy)-phenoxy]-ethyl}-cyclopropylmethyl-propyl-amine.

The title compound was prepared according to the procedure for EXAMPLE 13 using cyclopropylmethyl-propyl-amine. MS (ESI): mass calculated for C₂₂H₂₆N₂O₃, 366.19; m/z found, 367.3 [M+H]⁺. ¹H NMR (400 MHz, CDCl₃): 7.52-7.50 (m, 1H), 7.44-7.41 (m, 1H), 7.33-7.21 (m, 4H), 6.99-6.95 (m, 2H), 4.09 (t, *J* = 6.2, 2H), 3.01 (t, *J* = 6.2, 2H), 2.64-2.59 (m, 2H), 2.49 (d, *J* = 6.3, 2H), 1.60-1.50 (m, 2H), 0.92 (t, *J* = 7.3, 4H), 0.55-0.52 (m, 2H), 0.16-0.13 (m, 2H).

### EXAMPLE 65

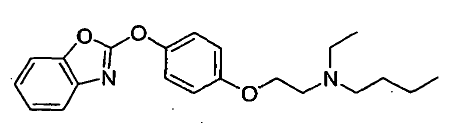

{2-[4-(Benzooxazol-2-yloxy)-phenoxy]-ethyl}-butyl-ethyl-amine.

The title compound was prepared according to the procedure for EXAMPLE 13 using butyl-ethyl-amine. MS (ESI): mass calculated for C₂₁H₂₆N₂O₃, 354.19; m/z found, 355.2 [M+H]⁺. ¹H NMR (400 MHz, CDCl₃): 7.41(d, *J* = 7.2, 1H), 7.32 (dd, *J* = 7.3, 1.6, 1H), 7.22 (d, *J* = 9.1, 1H), 7.18-7.10 (m, 2H), 6.87 (d, *J* = 9.1, 2H), 4.02 (br s, 2H), 2.85 (br s, 2H), 2.61 (br s, 2H), 2.49 (br s, 2H), 1.42 (br s, 2H), 1.31-1.19 (m, 2H), 1.05 (br s, 3H), 0.83 (t, *J* = 7.3, 3H).

### EXAMPLE 66

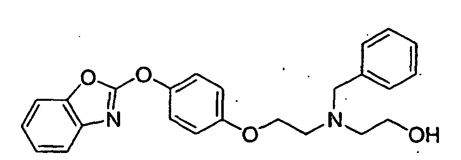

2-({2-[4-(Benzooxazol-2-yloxy)-phenoxy]-ethyl}-benzyl-amino)-ethanol.

The title compound was prepared according to the procedure for EXAMPLE 13 using benzylamino-ethanol. MS (ESI): mass calculated for C₂₄H₂₄N₂O₄, 404.17; m/z found, 405.2 [M+H]⁺. ¹H NMR (400 MHz, CD₃OD): 7.46-7.37(m, 3H), 7.33 (d, *J* = 16.9, 2H), 7.29-7.00 (m, 6H), 6.95 (d, *J* = 6.9, 2H), 4.06 (t, *J* = 5.8, 2H), 3.76 (s, 2H), 3.64 (t, *J* = 6.2, 2H), 2.95 (d, *J* = 5.8, 2H), 2.76 (t, *J* = 6.1, 2H).

### EXAMPLE 67

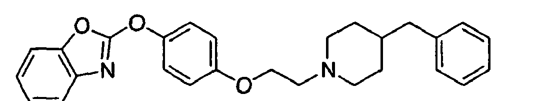

2-{4-[2-(4-Benzyl-piperidin-1-yl)-ethoxy]-phenoxy}-benzooxazole.

The title compound was prepared according to the procedure for EXAMPLE 13 using 4-benzyl-piperidine. MS (ESI): mass calculated for C₂₇H₂₈N₂O₃, 428.21; m/z found, 429.1 [M+H]⁺. ¹H NMR (400 MHz, CDCl₃): 7.53-7.50 (m, 1H), 7.44-7.41 (m, 1H), 7.33-7.15 (m, 9H), 6.98-6.95 (m, 2H), 4.11 (t, *J* = 6.0, 2H), 3.01-2.96 (m, 2H), 2.79 (t, *J* = 6.0, 2H), 2.55 (d, *J* = 7.0, 2H), 2.09-2.02 (m, 2H), 1.67-1.64 (m, 2H), 1.59-1.51 (m, 1H) 1.40-1.29 (m, 2H).

### EXAMPLE 68

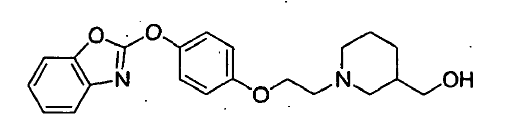

(1-{2-[4-(Benzooxazol-2-yloxy)-phenoxy]-ethyl}-piperidin-3-yl)-methanol.

The title compound was prepared according to the procedure for EXAMPLE 13 using piperidin-3-yl-methanol. MS (ESI): mass calculated for C₂₁H₂₄N₂O₄, 368.17; m/z found, 369.4 [M+H]⁺. ¹H NMR (400 MHz, CDCl₃): 7.55-7.52 (m, 1H), 7.48-7.44 (m, 1H), 7.38-7.23 (m, 4H), 7.05-6.99 (m, 2H), 4.23 (t, *J* = 5.6, 2H), 3.71 (dd, *J* = 10.7, 5.1, 1H), 3.59 (dd, *J* = 10.6, 6.4, 1H), 3.09 (br d, *J* = 9.7, 1H), 2.94 (t, *J* = 5.6, 2H), 2.42 (t, *J* = 9.1, 1H), 2.29 (t, *J* = 9.3, 1H), 2.24-1.98 (m, 2H), 2.00-1.90 (m; 1H), 1.90-1.80 (m, 1H), 1.80-1.71 (m, 2H), 1:20-1.10 (m, 1H).

### EXAMPLE 69

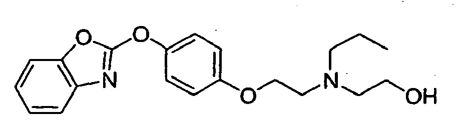

2-({2-[4-(Benzooxazol-2-yloxy)-phenoxy]-ethyl}-propyl-amino)-ethanol.

The title compound was prepared according to the procedure for EXAMPLE 13 using 2-propylamino-ethanol. MS (ESI): mass calculated for C₂₀H₂₄N₂O₄, 356.17; m/z found, 357.3 [M+H]⁺. ¹H NMR (400 MHz, DMSO-*d*₆): 7.62 (dd, *J* = 6.2, 2.0, 1H), 7.50 (dd, *J* = 6.1, 2.1, 1H), 7.42 (d, *J* = 9.1, 2H), 7.35-7.25 (m, 2H), 7.03 (d, *J* = 9.1, 2H), 4.31 (t, *J* = 5.4, 1H), 4.04 (t, *J* = 6.0, 2H), 3.46 (q, *J* = 6.4, 2H), 2.85 (t, *J* = 6.0, 2H), 2.59 (t, *J* = 6.5, 2H), 2.51-2.48 (m, 2H), 1.41 (q, *J* = 7.4, 2H), 0.84 (t, *J* = 7.3, 3H).

### EXAMPLE 70

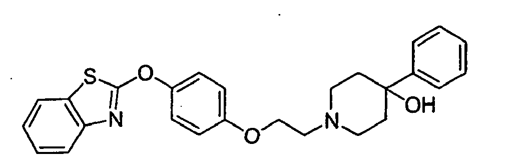

1-{2-[4-(Benzothiazol-2-yloxy)-phenoxy]-ethyl}-4-phenyl-piperidin-4-ol.

The title compound was prepared according to the procedure for EXAMPLE 17 using 4-phenyl-piperidin-4-ol. MS (ESI): mass calculated for C₂₆H₂₆N₂O₃S, 446.17; m/z found, 447.2 [M+H]⁺. ¹H NMR (400 MHz, CDCl₃): 7.74 (d, *J* = 8:1, 1H), 7.66 (d, *J* = 7.4, 1 H), 7.54 (d, *J* = 7.6, 2H), 7.42-7.35 (m, 3H), 7.33-7.22 (m, 4H), 6.99 (d, *J* = 9.0, 2H), 4.19 (t, *J* = 5.8, 2H), 2.98-2.86 (m, 4H), 2.65 (dt, *J* = 13.8, 2.1, 2H), 2.24 (dt, *J* = 13.4, 4.5, 2H), 1.80 (dd, *J* = 14.1, 2.2, 2H).

### EXAMPLE 71

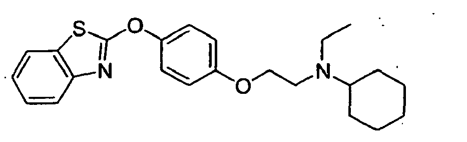

{2-[4-(Benzothiazol-2-yloxy)-phenoxy]-ethyl}-cyclohexyl-ethyl-amine.

The title compound was prepared according to the procedure for EXAMPLE 17 using cyclohexyl-ethyl-amine. MS (ESI): mass calculated for C₂₃H₂₈N₂O₂S, 396.19; m/z found, 397.2 [M+H]⁺. ¹H NMR (400 MHz, CDCl₃): 7.74 (d, *J* = 8.1, 1 H), 7.65 (d, *J* = 7.9, 1 H), 7.39 (t, *J* = 7.9, 4H), 7.30-7.20 (m, 4H), 6.96 (d, *J* = 8.9, 2H), 3.98 (t, *J* = 6.8, 2H), 2.89 (t, *J* = 6.8, 2H), 2.66 (q, *J* = 7.1, 2H), 2.55-2.50 (m, 1H), 1.82 (t, *J* = 7.7, 4H), 1.65 (d, *J* = 11.9, 1 H), 1.30-1.18 (m, 4H), 1.09 (t, *J* = 7.2, 4H).

### EXAMPLE 72

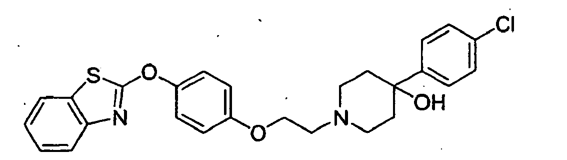

1-{2-[4-(Benzothiazol-2-yloxy)-phenoxy]-ethyl}-4-(4-chloro-phenyl)-piperidin-4-ol.

The title compound was prepared according to the procedure for EXAMPLE 17 using 4-(4-chloro-phenyl)-piperidin-4-ol. MS (ESI): mass calculated for C₂₆H₂₅ClN₃O₃S, 480.13; m/z found, 481.1 [M+H]⁺. ¹H NMR (400 MHz, CDCl₃): 7.74 (d, *J* = 7.6, 1 H), 7.66 (dd, *J* = 7.9, 0.8, 1H), 7.49-7.45 (m, 2H), 7.41-7.22 (m, 6H), 7.01-6.97 (m, 2H), 4.18 (t, *J* = 5.8, 2H), 2.96-2.89 (m, 4H), 2.63 (dt, *J* = 12.1, 2.4, 2H), 2.21-2.14 (m, 2H), 1.79-1.74 (m, 2H), 1.56 (br s, 1H).

### EXAMPLE 73

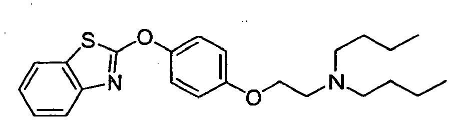

{2-[4-(Benzothiazol-2-yloxy)-phenoxy]-ethyl}-dibutyl-amine.

The title compound was prepared according to the procedure for EXAMPLE 17 using dibutyl-amine. MS (ESI): mass calculated for C₂₃H₃₀N₂O₂S, 398.20; m/z found, 399.2 [M+H]⁺. ¹H NMR (400 MHz, CDCl₃): 7.63 (d, *J* = 8.1, 1H), 7.55 (d, *J* = 7.9, 1H), 7.28 (tt, *J* = 7.7, 1.3, 1 H), 7.20-7.13 (m, 3H), 6.85 (d, *J* = 12.8, 2H), 3.96 (t, *J* = 5.6, 2H), 2.80 (m, 2H), 2.43 (m, 4H), 1.38 (m, 4H), 1.22 (m, 4H), 0.83 (t, *J* = 7.3 Hz, 6H).

### EXAMPLE 74

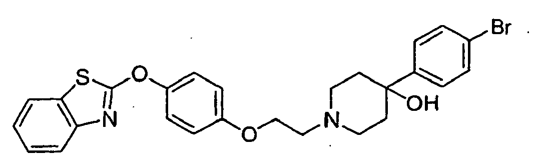

1-{2-[4-(Benzothiazol-2-yloxy)-phenoxy]-ethyl}-4-(4-bromo-phenyl)-piperidin-4-ol.

The title compound was prepared according to the procedure for EXAMPLE 17 using 4-(4-bromo-phenyl)-piperidin-4-ol. MS (ESI): mass calculated for C₂₆H₂₅BrN₂O₃S, 524.08; m/z found, 525.1 [M+H]⁺. ¹H NMR (400 MHz, CDCl₃): 7.74 (dd, *J* = 8.0, 0.6, 1H), 7.67 (dd, *J* = 8.0, 0.8, 1 H), 7.53-7.47 (m, 2H), 7.43-7.37 (m, 3H), 7.30-7.22 (m, 3H), 7.01-6.98 (m, 2H), 4.17 (t, *J* = 5.8, 2H), 2.94-2.89 (m, 4H), 2.63 (dt, *J* = 12.1, 2.5, 2H), 2.21-2.14 (m, 2H), 1.76 (dd, *J* = 14.22.6, 2H), 1.56 (br s, 1 H).

### EXAMPLE 75

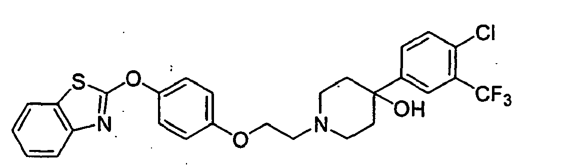

1-{2-[4-(Benzothiazol-2-yloxy)-phenoxy]-ethyl}-4-(4-chloro-3-trifluoromethyl-phenyl)-piperidin-4-ol.

The title compound was prepared according to the procedure for EXAMPLE 17 using 4-(4-chloro-3-trifluoromethyl-phenyl)-piperidin-4-ol. MS (ESI): mass calculated for C₂₇H₂₄ClF₃N₂O₃S, 548.11; m/z found, 549.1 [M+H]⁺. ¹H NMR (400 MHz, CDCl₃): 7.89 (d, *J* = 2.1, 1H), 7.62-7.48 (m, 4H), 7.41-7.37 (m, 1H), 7.30-7.24 (m, 3H), 7.01-6.97 (m, 2H), 4.17 (t, *J* = 5.8, 2H), 2.94-2.91 (m, 6H), 2.66-2.59 (m, 2H), 2.22-2.15 (m, 2H), 1.83 (br s, 1 H).

### EXAMPLE 76

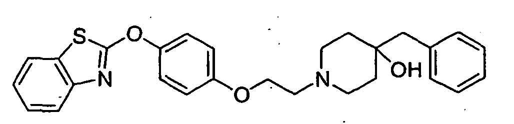

1-{2-[4-(Benzothiazol-2-yloxy)-phenoxy]-ethyl}-4-benzyl-piperidin-4-ol.

The title compound was prepared according to the procedure for EXAMPLE 17 using 4-benzyl-piperidin-4-ol. MS (ESI): mass calculated for C₂₇H₂₈N₂O₃S, 460.18; m/z found, 461.2 [M+H]⁺. ¹H NMR (400 MHz, CDCl₃): 7.74 (d; *J* = 8.0, 1 H), 7.66 (d, *J* = 7.7, 1 H), 7.41-7.23 (m, 9H), 6.99-6.94 (m, 2H), 4.13 (t, *J* = 5.9, 2H), 4.84 (t, *J* = 5.9, 2H), 2.78 (m, 4H), 2.50-2.45 (m, 2H), 1.83-1.76 (m, 2H), 1.59-1.52 (m, 2H), 1.23 (br s, 1 H).

### EXAMPLE 77

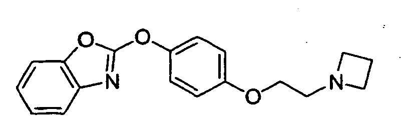

2-[4-(2-Azetidin-1-yl-ethoxy)-phenoxy]-benzooxazole.

The title compound was prepared according to the procedure for EXAMPLE 21 using azetidine. MS (ESI): mass calculated for C₁₈H₁₈N₂O₃, 310.13; m/z found, 311.2 [M+H]⁺. ¹H NMR (400 MHz, DMSO-*d*₆): 7.61 (d, *J* = 7.0, 2H), 7.48 (d, *J* = 7.0, 2H), 7.41 (d, *J* = 9.1, 2H), 7.32-7.24 (m, 2H), 7.00 (d, *J* = 6.9, 2H), 3.93 (t, *J* = 5.6, 2H), 3.18 (t, *J* = 6.9, 4H), 2.70 (t, J = 5.6, 2H), 1.97 (t, *J* = 6.9, 2H).

### EXAMPLE 78

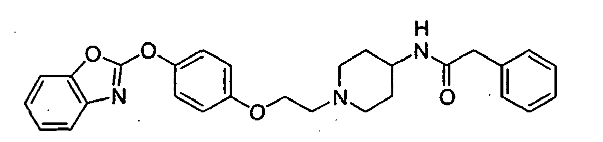

*N*-(1-{2-[4-(Benzooxazol-2-yloxy)-phenoxy]-ethyl}-piperidin-4-yl)-2-phenyl-acetamide.

The title compound was prepared according to the procedure for EXAMPLE 22 using 2-phenyl-N-piperidin-4-yl-acetamide and 2-chloro-benzooxazole. MS (ESI): mass calculated for C₂₈H₂₉N₃O₄, 471.22; m/z found, 472.5 [M+H]⁺. ¹H NMR (400 MHz, CDCl₃): 7.55-7.51 (m, 1 H), 7.47-7.43 (m, 1H), 7.41-7.23 (m, 9H), 7.00-6.95 (m, 2H), 5.28 (br d, *J* = 7.8, 1 H), 4.10 (t, *J* = 5.7, 2H), 3.90-3.80 (m, 1 H), 3.60 (s, 2H), 2.89 (br d, *J* = 11.5, 2H), 2.81 (t, *J* = 5.8, 2H), 2.28 (dt, *J* = 11.6, 2.2, 2H), 1.92 (br dd, *J* = 12.6, 2.3, 2H), 1.46-1.37 (m, 2H).

### EXAMPLE.79

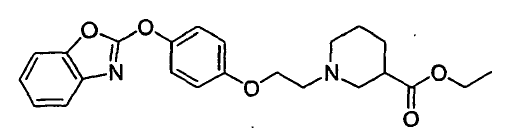

1-{2-[4-(Benzooxazol-2-yloxy)-phenoxy]-ethyl}-piperidine-3-carboxylic acid ethyl ester.

The title compound was prepared according to the procedure for EXAMPLE 22 using piperidine-3-carboxylic acid ethyl ester and 2-chloro-benzooxazole. MS (ESI): mass calculated for C₂₃H₂₆N₂O₅, 410.18; m/z found, 411.4 [M+H]⁺. ¹H NMR (400 MHz, CDCl₃): 7.56-7.52 (m, 1 H), 7.47-7.43 (m, 1H), 7.37-7.32 (m, 2H), 7.32-7.23 (m, 2H), 7.02-6.98 (m, 2H), 4.18 (q, *J* = 7.1, 2H), 4.15 (t, *J* = 5.9, 2H), 3.14 (br d, *J* = 8.2, 1 H), 2.96-2.82 (m, 3H), 2.65 (tt, *J* = 10.6, 3.7, 1 H), 2.37 (t, *J* = 10.7, 1 H), 2.20 (dt, *J* = 10.9, 2.7, 1 H), 2.03-1.97 (m, 1 H), 1.82-1.75 (m, 1 H), 1.73-1.60 (m, 1 H), 1.54-1.45 (m, 1 H) 1.30 (t, *J* = 7.2, 3H).

### EXAMPLE 80

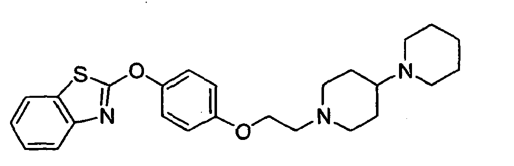

1'-{2-[4-(Benzothiazol-2-yloxy)-phenoxy]-ethyl}-[1,4']bipiperidine.

The title compound was prepared according to the procedure for EXAMPLE 22 using 4-piperidinyl-piperidine. MS (ESI): mass calculated for C₂₅H₃₁N₃O₂S, 437.21; m/z found, 438.4 [M+H]⁺. ¹H NMR (400 MHz, CDCl₃): 7.77 (d, *J* = 8.0, 1 H), 7.69 (d, *J* = 7.0, 1 H), 7.42 (dt, *J* = 7.1, 1.1, 1 H), 7.35-7.26 (m, 3H), 7.04-6.97 (m, 2H), 4.15 (t, *J* = 5.9, 2H), 3.11 (br d, J = 11.7, 2H), 2.84 (t, *J* = 6.0, 2H), 2.57 (br s, 2H), 2.40-2.30 (m, 1 H), 2.16 (dt, *J* = 11.7, 1.6, 1 H), 1.85 (br d, *J* = 12.1, 2H), 1.75-1.59 (m, 8H), 1.52-1.40 (m, 2H).

### EXAMPLE 81

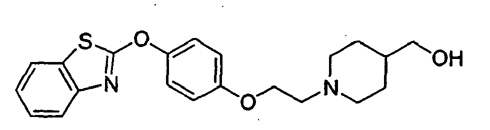

(1-{2-[4-(Benzothiazol-2-yloxy)-phenoxy]-ethyl}-piperidin-4-yl)-methanol.

The title compound was prepared according to the procedure for EXAMPLE 17 using piperidin-4-yl-methanol. MS (ESI): mass calculated for C₂₁H₂₄N₂O₃S, 384.15; m/z found, 385.1 [M+H]⁺. ¹H NMR (500 MHz, CDCl₃): 7.75-7.73 (m, 1 H), 7.67-7.65 (m, 1 H), 7.41-7.37 (m, 1H), 7.31-7.36 (m, 3H), 6.98-6.95 (m, 2H), 4.13 (t, *J* = 5.9, 2H), 3.52 (t, *J* = 5.4, 2H), 3.10-3.03 (m, 2H), 2.83 (t, *J* = 5.9, 2H), 2.14 (dt, *J* = 11.8, 2.4, 2H), 1.78-1.75 (m, 2H), 1.58-1.52 (m, 2H), 1.37-1.30 (m, 2H).

### EXAMPLE 82

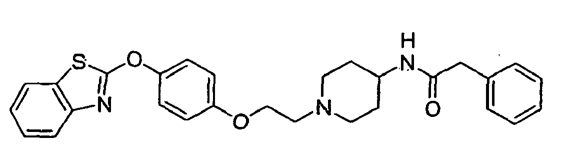

*N*-(1-{2-[4-(Benzothiazol-2-yloxy)-phenoxy]-ethyl}-piperidin-4-yl)-2-phenyl-acetamide.

The title compound was prepared according to the procedure for EXAMPLE 22 using 2-phenyl-*N*-piperidin-4-yl-acetamide. MS (ESI): mass calculated for C₂₈H₂₉N₃O₃S, 487.19; m/z found, 488.2 [M+H]⁺. ¹H NMR (400 MHz, CDCl₃): 7.78 (dd, *J* = 8.0, 0.6, 1 H), 7.69 (dd, *J* = 7.9, 0.7, 1 H), 7.45-7.24 (m, 9H), 7.00-6.88 (m, 2H), 5.33 (d, *J* = 7.9, 1 H), 4.10 (t, *J* = 5.8, 2H), 3.90-3.79 (m, 1 H), 3.60 (s, 2H), 2.88 (d, *J* = 11.7, 2H), 2.80 (t, *J* = 5.7, 2H), 2.27 (dt, *J* = 11.6, 2.2, 2H), 1.92 (dd, *J* = 12.7, 3.6, 2H), 1.39 (dq, *J* = 11.1, 3.8, 2H).

### EXAMPLE 83

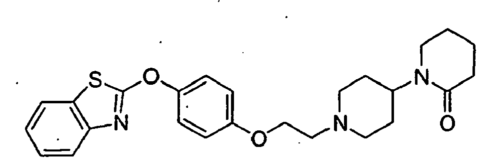

1'-{2-[4-(Benzothiazol-2-yloxy)-phenoxy]-ethyl}-[1,4']bipiperidinyl-2-one.

The title compound was prepared according to the procedure for EXAMPLE 22 using [1,4']bipiperidinyl-2-one. MS (ESI): mass calculated for C₂₅H₂₉N₃O₃S, 451.59; m/z found, 452.4 [M+H]⁺. ¹H NMR (400 MHz, DMSO-*d*₆): 7.91 (d, *J* = 7.4, 1 H), 7.67 (d, *J* = 7.4, 1 H), 7.45-7.32 (m, 4H), 7.06 (d, *J* = 9.0, 2H), 4.35-4.15 (m, 1H), 4.10 (t, *J* = 5.7, 2H), 3.15 (t, *J* = 5.3, 2H), 3.00 (d, *J* = 11.5, 2H), 2.71 (t, *J* = 5.7, 2H), 2.21 (t, *J* = 6.5, 2H), 2.10 (t, *J* = 11.5, 2H), 1.75-1.60 (m, 6H), 1.43 (d, *J* = 10.0, 2H).

### EXAMPLE 84

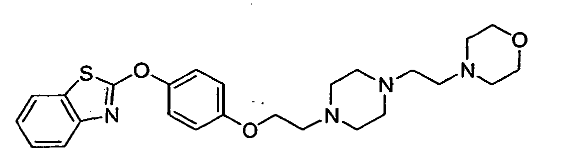

2-(4-{2-[4-(2-Morpholin-4-yl-ethyl)-piperazin-1-yl]-ethoxy}-phenoxy)-benzothiazole.

The title compound was prepared according to the procedure for EXAMPLE 22 using 4-(2-piperazin-1-yl-ethyl)-morpholine. MS (ESI): mass calculated for C₂₅H₃₂N₄O₃S, 468.22; m/z found, 469.2 [M+H]⁺. ¹H NMR (400 MHz, DMSO-*d*₆): 7.91 (d, *J* = 7.9, 1 H), 7.67 (d, *J* = 7.9, 1 H), 7.45-7.32 (m, 4H), 7.06 (d, *J* = 9.1, 2H), 4.09 (t, *J* = 5.8, 2H), 3.54 (t, *J* = 4.6, 2H), 2.68 (t, *J* = 5.7, 2H), 2.50-2.20 (br s, 16H).

### EXAMPLE 85

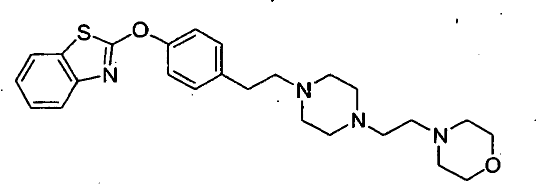

2-(4-{2-[4-(2-Morpholin-4-yl-ethyl)-piperazin-1-yl]-ethyl}-phenoxy)-benzothiazole.

The title compound was prepared according to the procedure for EXAMPLE 32 using 4-(2-piperazin-1-yl-ethyl)-morpholine. MS (ESI): mass calculated for C₂₅H₃₂N₄O₂S, 452.22; m/z found, 453.2 [M+H]⁺. ¹H NMR (400 MHz, DMSO-*d*₆): 7.92 (d, *J* = 8.0, 1 H), 7.68 (d, *J* = 8.0, 1H), 7.45-7.30 (m, 6H), 3.54 (t, *J* = 4.5, 4H), 2.75 (t, *J* = 8.3, 4H), 2.50-2.20 (br s, 16H).

### EXAMPLE 86

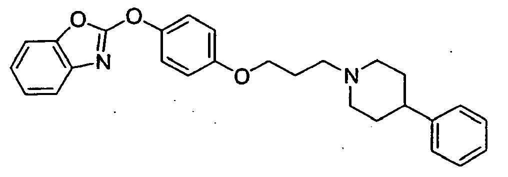

2-{4-[3-(4-Phenyl-piperidin-1-yl)-propoxy]-phenoxy}-benzooxazole.

The title compound was prepared according to the procedure for EXAMPLE 27 using 4-phenyl-piperidine. MS (ESI): mass calculated for C₂₇H₂₈N₂O₃, 428.21; m/z found, 429.2 [M+H]⁺. ¹H NMR (400 MHz, CD₃OD): 7.40-7.38 (m, 1 H), 7.34-7.32 (m, 1H), 7.24 (d, *J* = 9.1, 2H), 7.22-7.12 (m, 4H), 7.07 (t, *J* = 7.0, 1 H), 6.94 (d, *J* = 9.1, 2H), 3.99 (t, *J* = 6.1, 2H), 3.06 (d, *J* = 11.7, 2H), 2.57 (t, *J* = 7.6, 2H), 2.49 (m, 1 H), 2.13 (t, *J* = 11.6, 2H), 1.97 (m, 2H), 1.73 (m, 4H).

### EXAMPLE 87

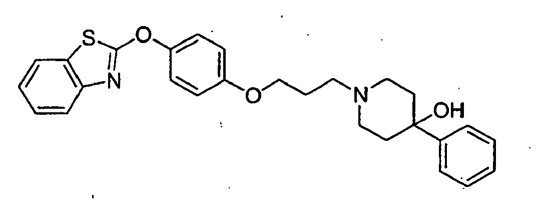

1-{3-[4-(Benzothiazol-2-yloxy)-phenoxy]-propyl}-4-phenyl-piperidin-4-ol.

The title compound was prepared according to the procedure for EXAMPLE 27 using 2-chlorobenzothiazole. MS (ESI): mass calculated for C₂₇H₂₈N₂O₃S, 460.18; m/z found, 461.2 [M+H]⁺. ¹H NMR (400 MHz, CDCl₃): 7.73 (d, *J* = 8.1, 1 H), 7.65 (d, *J* = 7.8, 1 H), 7.53 (d, *J* = 8.2, 2H), 7.39-7.35 (m, 3H), 7.29-7.23 (m, 5H), 6.96 (d, *J* = 9.1, 2H), 4.08 (t, *J* = 6.2, 2H), 2.92 (m, 2H), 2.72-2.49 (m, 4H), 2.34-2.02 (m, 4H), 1.81 (d, *J* = 12.3, 2H).

### EXAMPLE 88

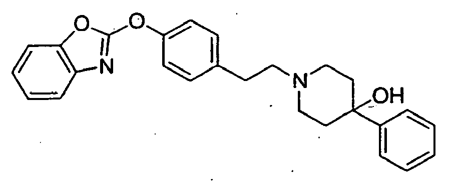

1-{2-[4-(Benzooxazol-2-yloxy)-phenyl]-ethyl}-4-phenyl-piperidin-4-ol.

The title compound was prepared according to the procedure for EXAMPLE 29 using 4-phenyl-piperidin-4-ol. MS (ESI): mass calculated for C₂₆H₂₆N₂O₃, 414.51; m/z found, 415.1 [M+H]⁺. ¹H NMR (400 MHz, CDCl₃): 7.56-7.51 (m, 3H), 7.45-7.32 (m, 6H), 7.30-7.22 (m, 4H), 2.96-2.89 (m, 2H), 2.74-2.69 (m, 2H), 2.61-2.54 (m, 2H), 2.26-2.18 (m, 2H), 1.84-1.79 (m, 2H), 1.62 (br s, 1H).

### EXAMPLE 89

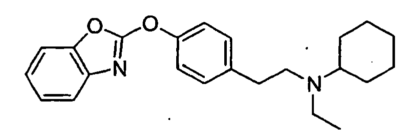

{2-[4-(Benzooxazol-2-yloxy)-phenyl]-ethyl}-cyclohexyl-ethyl-amine.

The title compound was prepared according to the procedure for EXAMPLE 29 using cyclohexyl-ethyl-amine. MS (ESI): mass calculated for C₂₃H₂₈N₂O₂, 364.22; m/z found, 365.1 [M+H]⁺. ¹H NMR (400 MHz, CDCl₃): 7.51 (d, *J* = 7.8, 1 H), 7.41 (d, *J* = 7.6, 1 H), 7.33-7.18 (m, 6H), 2.80-2.68 (m, 4H), 2.64 (dd, *J* = 6.8, 7.04, 2H), 2.58-2.50 (m 1H), 1.80 (t, *J* = 8.8, 4H), 1.63 (d, *J* = 12.3, 1 H), 1.22 (dd, *J* = 9.8, 8.4, 4H), 1.08 (t, *J* = 7.0, 4H).

### EXAMPLE 90

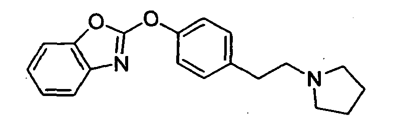

2-[4-(2-Pyrrolidin-1-yl-ethyl)-phenoxy]-benzooxazole.

The title compound was prepared according to the procedure for EXAMPLE 29 using pyrrolidine. MS (ESI): mass calculated for C₁₉H₂₀N₂O₂, 308.15; m/z found, 309.1 [M+H]⁺. ¹H NMR (400 MHz, CDCl₃): 7.51 (d, *J* = 7.6, 1 H), 7.42 (d, *J* = 7.2, 1 H), 7.35-7.20 (m, 6H), 2.90-2.84 (m, 2H), 2.75-2.68 (m 2H), 2.62-2.55 (m, 4H), 1.85-1.79 (m, 4H).

### EXAMPLE 91

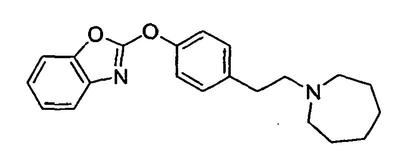

2-[4-(2-Azepan-1-yl-ethyl)-phenoxy]-benzooxazole.

The title compound was prepared according to the procedure for EXAMPLE 29 using azepane. MS (ESI): mass calculated for C₂₁H₂₄N₂O₂, 336.18; m/z found, 337.1 [M+H]⁺. ¹H NMR (400 MHz, CDCl₃): 7.50 (d, *J* = 7.6, 1 H), 7.39 (d, *J* = 7.8, 1H), 7.33-7.17 (m, 6H), 2.84-2.73 (m, 4H), 2.71 (t, *J* = 5.3, 4H), 1.71-1.57 (m, 8H).

### EXAMPLE 92

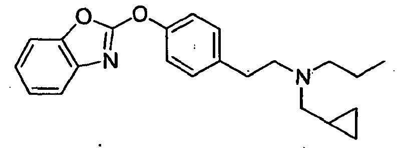

{2-[4-(Benzooxazol-2-yloxy)-phenyl]-ethyl}-cyclopropylmethyl-propyl-amine.

The title compound was prepared according to the procedure for EXAMPLE 29 using cyclopropylmethyl-propyl-amine. MS (ESI): mass calculated for C₂₂H₂₆N₂O₂, 350.20; m/z found, 351.1 [M+H]⁺. ¹H NMR (400 MHz, CDCl₃): 7.50 (d, *J* = 7.6, 1H), 7.39 (d, *J* = 7.8, 1H), 7.33-7.17 (m, 6H), 2.85-2.74 (m, 4H), 2.56 (t, *J* = 7.6, 2H), 2.43 (d, *J* = 6.5, 2H), 1.56-1.44 (m, 2H), 0.90 (t, *J* = 7.2, 4H), 0.51 (dd, *J* = 7.8, 5.5, 2H), 0.13 (dd, *J* = 7.8, 5.5, 2H).

### EXAMPLE 93

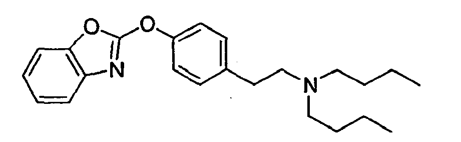

{2-[4-(Benzooxazol-2-yloxy)-phenyl]-ethyl}-dibutyl-amine.

The title compound was prepared according to the procedure for EXAMPLE 29 using dibutyl-amine. MS (ESI): mass calculated for C₂₃H₃₀N₂O₂, 366.23; m/z found, 367.2 [M+H]⁺. ¹H NMR (400 MHz, CDCl₃): 7.51 (d, *J* = 7.4, 1 H), 7.39 (d, *J* = 7.6, 1 H), 7.33-7.17 (m, 6H), 2.80-2.66 (m, 4H), 2.49 (t, *J* = 7.3, 4H), 1.50-1.40 (m, 4H), 1.37-1.26 (m, 4H), 0.93 (t, *J* = 7.2, 6H).

### EXAMPLE 94

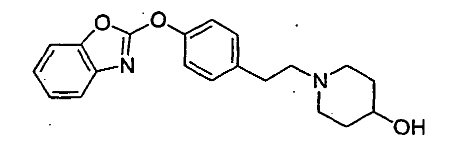

1-{2-[4-(Benzooxazol-2-yloxy)-phenyl]-ethyl}-piperidin-4-ol.

The title compound was prepared according to the procedure for EXAMPLE 29 using 4-hydroxypiperidine. MS (ESI): mass calculated for C₂₀H₂₂N₂O₃, 338.16; m/z found, 339.4 [M+H]⁴. ¹H NMR (400 MHz, CDCl₃): 7.50 (d, *J* = 7.6, 1H), 7.39 (d, *J* = 7.6, 1 H), 7.34-7.16 (m, 6H), 3.72-3.63 (m, 1 H), 3.29 (br s, 1 H), 2.91-2.78 (m, 4H), 2.63-2.56 (m, 2H), 2.21 (t, *J* = 10.0, 2H), 1.96-1.86 (m, 2H), 1.70-1.57 (m, 2H).

### EXAMPLE 95

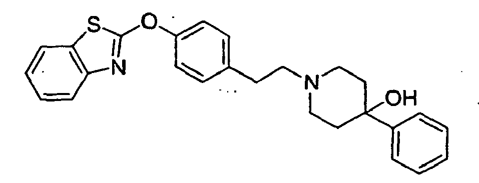

1-{2-[4-(Benzothiazol-2-yloxy)-phenyl]-ethyl}-4-phenyl-piperidin-4-ol.

The title compound was prepared according to the procedure for EXAMPLE 30 using 4-phenyl-piperidin-4-ol. MS (ESI): mass calculated for C₂₆H₂₆N2O₃S, 430.57; m/z found, 431.4 [M+H]⁺. ¹H NMR (400 MHz, CDCl₃): 7.76-7.74 (m, 1 H), 7.67 (dd, *J* = 8.0, 0.7, 1 H), 7.56-7.53 (m, 2H), 7.42-7.36 (m, 3H), 7.33-7.26 (m, 6H), 2.96-2.86 (m, 4H), 2.75-7.71 (m, 2H), 2.64-2.56 (m, 2H) 2.27-2.18 (m, 2H), 1.86-1.80 (m, 2H), 1.67-1.66 (br m, 2H).

### EXAMPLE 96

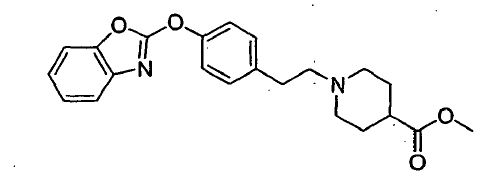

1-{2-[4-(Benzooxazol-2-yloxy)-phenyl]-ethyl}-piperidine-4-carboxylic acid methyl ester.

The title compound was prepared according to the procedure for EXAMPLE 29 using piperidine-4-carboxylic acid methyl ester. MS (ESI): mass calculated for C₂₂H₂₄N₂O₄, 380.17; m/z found, 381.4 [M+H]⁺. ¹H NMR (400 MHz, CDCl₃): 7.50 (d, *J* = 8.1, 1H), 7.39 (d, *J* = 8.1, 1H), 7.34-7.16 (m, 6H), 3.67 (s, 3H), 2.97-2.90 (m, 2H), 2.84-2.78 (m, 2H), 2.60-2.55 (m, 2H), 2.35-2.25 (m, 1H), 2.10 (t, *J* = 11.4, 2H), 1.96-1.88 (m, 2H), 1.84-1.73 (m, 2H).

### EXAMPLE 97

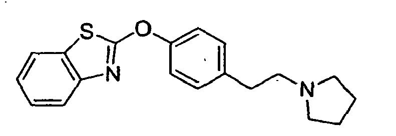

2-[4-(2-Pyrrolidin-1-yl-ethyl)-phenoxy]-benzothiazole.

The title compound was prepared according to the procedure for EXAMPLE 30 using pyrrolidine. MS (ESI): mass calculated for C₁₉H₂₀N₂OS, 324.13; m/z found, 325.3 [M+H]⁺. ¹H NMR (400 MHz, CDCl₃): 7.73 (d, *J* = 8.0, 1 H), 7.65 (d, *J* = 8.0, 1H), 7.38 (t, *J* = 7.2, 1 H), 7.31-7.22 (m, 5H), 2.91-2.83 (m, 2H), 2.77-2.69 (m 2H), 2.64-2.55 (m, 4H), 1.87-1.77 (m, 4H).

### EXAMPLE 98

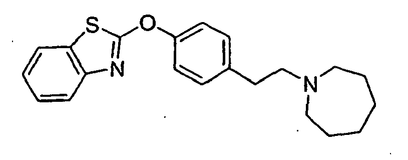

2-[4-(2-Azepan-1-yl-ethyl)-phenoxy]-benzothiazole.

The title compound was prepared according to the procedure for EXAMPLE 30 using azepane. MS (ESI): mass calculated for C₂₁H₂₄N₂OS, 352.16; m/z found, 353.4 [M+H]⁺. ¹H NMR (400 MHz, CDCl₃): 7.73 (d, *J* = 8.0, 1H), 7.65 (d, *J* = 8.0, 1 H), 7.37 (t, *J* = 7.4, 1 H), 7.30-7.22 (m, 5H), 2.87-2.70 (m, 8H), 1.73-1.57 (m, 8H).

### EXAMPLE 99

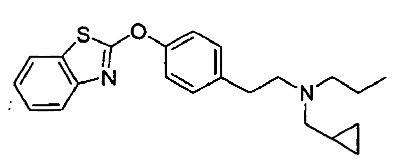

{2-[4-(Benzothiazol-2-yloxy)-phenyl]-ethyl}-cyclopropylmethyl-propyl-amine.

The title compound was prepared according to the procedure for EXAMPLE 30 using cyclopropylmethyl-propyl-amine. MS (ESI): mass calculated for C₂₂H₂₆N₂OS, 366.18; m/z found, 367.4 [M+H]⁺. ¹H NMR (400 MHz, CDCl₃): 7.73 (d, *J* = 8.6, 1 H), 7.65 (d, *J* = 8.2, 1H), 7.38 (t, *J* = 8.6, 1 H), 7.30-7.22 (m, 5H), 2.87-2.76 (m, 4H), 2.57 (t, *J* = 7.6, 2H), 2.44 (d, *J* = 6.5, 2H), 1.56-1.45 (m, 2H), 0.90 (t, *J* = 7.4, 4H), 0.52 (dd, *J* = 7.8, 5.5, 2H), 0.14 (dd, *J* = 5.7, 5.1, 2H).

### EXAMPLE 100

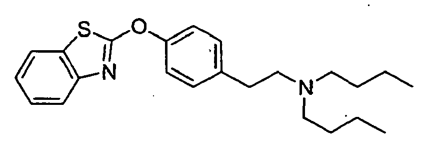

{2-[4-(Benzothiazol-2-yloxy)-phenyl]-ethyl}-dibutyl-amine.

The title compound was prepared according to the procedure for EXAMPLE 30 using dibutyl-amine. MS (ESI): mass calculated for C₂₃H₃₀N₂OS, 382.21; m/z found, 383.4 [M+H]⁺. ¹H NMR (400 MHz, CDCl₃): 7.73 (d, *J* = 8.2, 1 H), 7.65 (d, *J* = 8.2, 1 H), 7.38 (t, *J* = 8.2, 1 H), 7.30-7.22 (m, 5H), 2.82-2.67 (m, 4H), 2.51 (t, *J* = 7.2, 4H), 1.50-1.40 (m, 4H), 1.37-1.25 (m, 4H), 0.93 (t, *J* = 7.2, 6H).

### EXAMPLE 101

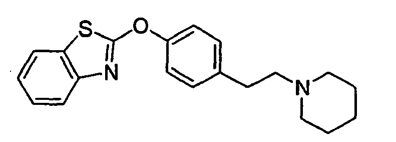

2-[4-(2-Piperidin-1-yl-ethyl)-phenoxy]-benzothiazole.

The title compound was prepared according to the procedure for EXAMPLE 30 using piperidine. MS (ESI): mass calculated for C₂₀H₂₂N₂OS, 338.48; m/z found, 339.3 [M+H]⁺. ¹H NMR (400 MHz, CD₃OD): 7.76-7.73 (m, 1H), 7.64-7.61 (m, 1 H), 7.42-7.25 (m, 6H), 2.88-2.83 (m, 2H), 2.60-2.52 (m, 6H), 1.66-1.61 (m, 4H), 1.53-1.45 (m, 2H).

### EXAMPLE 102

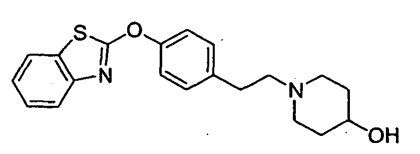

1-{2-[4-(Benzothiazol-2-yloxy)-phenyl]-ethyl}-piperidin-4-ol.

The title compound was prepared according to the procedure for EXAMPLE 30 using 4-hydroxypiperidine. MS (ESI): mass calculated for C₂₀H₂₂N₂O₂S, 354.14; m/z found, 355.3 [M+H]⁺. ¹H NMR (400 MHz, CDCl₃): 7.73 (d, *J* = 8.2, 1 H), 7.64 (d, *J* = 8.0, 1 H), 7.37 (t, *J* = 8.2, 1 H), 7.29-7.20 (m, 5H), 3.75-3.65 (m, 1 H), 2.92-2.79 (m, 4H), 2.76 (br s, 1H), 2.65-2.55 (m, 2H), 2.21 (t, *J* = 10.0, 2H), 1.98-1.87 (m, 2H), 1.70-1.57 (m, 2H).

### EXAMPLE 103

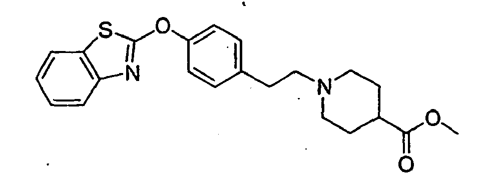

1-{2-[4-(Benzothiazol-2-yloxy)-phenyl]-ethyl}-piperidine-4-carboxylic acid methyl ester.

The title compound was prepared according to the procedure for EXAMPLE 30 using piperidine-4-carboxylic acid methyl ester. MS (ESI): mass calculated for C₂₂H₂₄N₂O₃S, 396.15; m/z found, 397.4 [M+H]⁺. ¹H NMR (400 MHz, CDCl₃): 7.73 (d, *J* = 8.3, 1 H), 7.66 (d, *J* = 8.3, 1H), 7.38 (t, *J* = 8.3, 1 H), 7.29-7.23 (m, 5H), 3.69 (s, 3H), 3.00-2.93 (m, 2H), 2.87-2.80 (m, 2H), 2.63-2.56 (m, 2H), 2.38-2.28 (m, 1H), 2.11 (t, *J* = 11.1, 2H), 1.98-1.91 (m, 2H), 1.86-1.74 (m, 2H).

### EXAMPLE 104

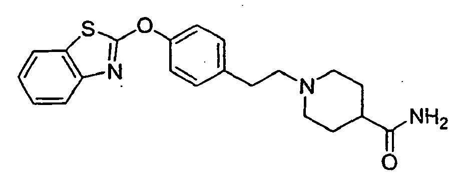

1-{2-[4-(Benzothiazol-2-yloxy)-phenyl]-ethyl}-piperidine-4-carboxylic acid amide.

The title compound was prepared according to the procedure for EXAMPLE 31 using piperidine-4-carboxylic acid amide. MS (ESI): mass calculated for C₂₁H₂₃N₃O2S, 381.15; m/z found, 382.3 [M+H]⁺. ¹H NMR (400 MHz, CDCl₃): 7.78 (dd, *J* = 8.1, 0.4, 1 H), 7.71 (dd, *J* = 7.9, 0.7, 1 H), 7.43 (dt, *J* = 7.5, 2.3, 1H), 7.35-7.25 (m, 5H), 5.51 (br d, *J* = 26.0, 1 H), 3.09 (br d, *J* = 11.7, 2H), 2.87, (dd, *J* = 8.3, 7.6, 2H), 2.65 (dd, *J* = 8.5, 5.4, 2H), 2.29-2.18 (m, 1 H), 2.13 (t, *J* = 11.4, 2H), 1.98 (br d, *J* = 11.2, 2H), 1.87-1.77 (m, 2H).

### EXAMPLE 105

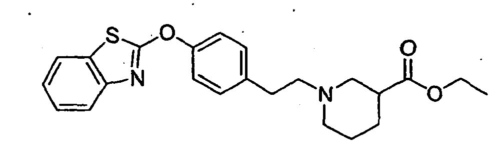

1-{2-[4-(Benzothiazol-2-yloxy)-phenyl]-ethyl}-piperidine-3-carboxylic acid ethyl ester.

The title compound was prepared according to the procedure for EXAMPLE 31 using piperidine-3-carboxylic acid ethyl ester. MS (ESI): mass calculated for C₂₃H₂₆N₂O₃S, 410.17; m/z found, 411.4 [M+H]⁺. ¹H NMR (400 MHz, CDCl₃): 7.77 (d, *J* = 8.0, 1 H), 7.70 (dd, *J* = 7.9, 0.5, 1 H), 7.42 (dt, *J* = 7.5, 1.3, 1 H), 7.34-7.27 (m, 5H), 4.18 (q, *J* = 7.1, 2H), 3.13 (br d, *J* = 8.8, 1 H), 2.92-2.85 (m, 2H), 2.70-2.59 (m, 2H), 2.30 (t, *J* = 10.7, 1 H), 2.13 (dt, *J* = 10.9, 2.7, 1 H), 2.04-1:97 (m, 1H), 1.84-1.76 (m, 1 H), 1.71-1.59 (m, 1 H), 1.57-1.45 (m, 1H) 1.30 (t, *J* = 7.2, 3H).

### EXAMPLE 106

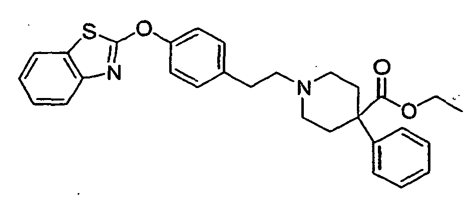

1-{2-[4-(Benzothiazol-2-yloxy)-phenyl]-ethyl}-4-phenyl-piperidine-4-carboxylic acid ethyl ester.

The title compound was prepared according to the procedure for EXAMPLE 31 using 4-phenyl-piperidine-4-carboxylic acid ethyl ester. MS (ESI): mass calculated for C₂₉H₃₀N₂O₃S, 486.20; m/z found, 487.5 [M+H]⁺. ¹H NMR (400 MHz, CDCl₃): 7.78 (dd, *J* = 8.1, 0.5, 1 H), 7.70 (dd, *J* = 7.9, 0.7, 1 H), 7.46-7.24 (m, 11H), 4.19 (q, *J* = 7.1, 2H), 3.06 (br d, *J* = 10.1, 2H), 2.96-2.90 (m, 2H), 2.75-2.64 (m, 4H), 2.35 (t, *J* = 11.0, 2H), 2.14, (t, *J* = 11.3, 2H), 1.24, (t, *J* = 7.3, 3H).

### EXAMPLE 107

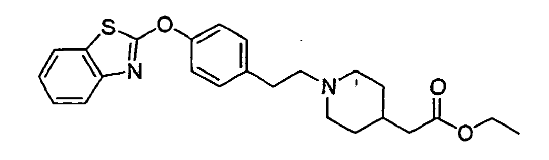

(1-{2-[4-(Benzothiazol-2-yloxy)-phenyl]-ethyl}-piperidin-4-yl)-acetic acid ethyl ester.

The title compound was prepared according to the procedure for EXAMPLE 31 using piperidin-4-yl-acetic acid ethyl ester. MS (ESI): mass calculated for C₂₄H₂₈N₂O₃S, 424.18; m/z found, 425.4 [M+H]⁺. ¹H NMR (400 MHz, CDCl₃): 7.76 (d, *J* = 7.8, 1 H), 7.70 (dd, *J* = 7.9, 0.6, 1 H), 7.41 (dt, *J* = 7.2, 1.2, 1 H), 7.36-7.27 (m, 5H), 4.18 (q, *J* = 5.3, 2H), 3.21 (d, *J* = 11.3, 2H), 3.03-2.99, (m, 2H), 2.83-2.78 (m, 2H), 2.33-2.25 (m, 4H), 2.00-1.87 (m, 1 H), 1.86 (d, *J* = 14.3, 2H), 1.67-1.55 (m, 2H), 1.29 (t, *J* = 7.1, 3H).

### EXAMPLE 108

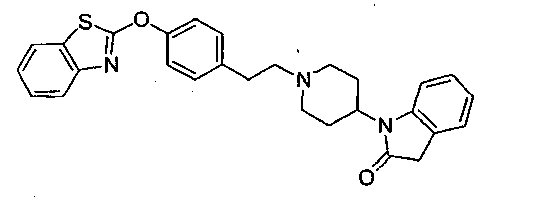

1-(1-{2-[4-(Benzothiazol-2-yloxy)-phenyl]-ethyl}-piperidin-4-yl)-1,3-dihydro-indol-2-one.

The title compound was prepared according to the procedure for EXAMPLE 31 using 1-piperidin-4-yl-1,3-dihydro-indol-2-one. MS (ESI): mass calculated for C₂₈H₂₇N₃O₂S, 469.18; m/z found, 470.4 [M+H]⁺. ¹H NMR (400 MHz, CDCl₃): 7.75 (dd, *J* = 8.1, 0.4, 1H), 7.68 (dd, *J* = 7.9, 0.6, 1H), 7.40 (dt, *J* = 7.5, 1.2, 1 H), 7.36-7.23 (m, 8H), 7.04 (dt, *J* = 7.3, 1.7, 1H), 4.57-4.45 (m, 1 H), 3.54 (s, 2H), 3.36 (br d, *J* = 10.9, 2H), 3.00 (dd, *J* = 11.4, 6.3, 1 H), 2.88 (dd, *J* = 8.8, 4.5, 2H), 2.75-2.62 (m, 2H), 2.45 (t, *J* = 11.5, 2H), 1.80 (dd, *J* = 12.3, 2.1, 2H).

### EXAMPLE 109

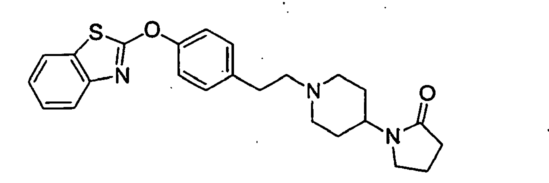

1-(1-{2-[4-(Benzothiazol-2-yloxy)-phenyl]-ethyl}-piperidin-4-yl)-pyrrolidin-2-one.

The title compound was prepared according to the procedure for EXAMPLE 31 using 1-piperidin-4-yl-pyrrolidin-2-one. MS (ESI): mass calculated for C₂₄H₂₇N₃O₂S, 421.18; m/z found, 422.4 [M+H]⁺. ¹H NMR (400 MHz, CDCl₃): 7.76 (d, *J* = 8.0, 1H), 7.69 (dd, *J* = 8.0, 0.9, 1H), 7.41 (dt, *J* = 7.5, 1.2, 1 H), 7.32-7.27 (m, 5H), 4.05 (dt, *J* = 11.9, 4.5, 1 H), 3.40 (t, *J* = 7.0, 2H), 3.10 (br d, *J* = 11.7, 2H), 2.86 (dd, *J* = 11.0, 7.7, 2H), 2.66 (dd, *J* = 8.8, 5.3, 2H), 2.43 (t, *J* = 8.1, 2H), 2.19 (dt, *J* = 11.6, 2.8, 2H), 2.09-2.00 (m, 2H), 1.85-1.69 (m, 4H).

### EXAMPLE 110

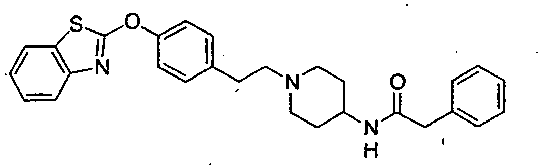

*N*-(11-{2-[4-(Benzothiazol-2-yloxy)-phenyl]-ethyl}-piperidin-4-yl)-2-phenyl-acetamide.

The title compound was prepared according to the procedure for EXAMPLE 31 using 2-phenyl-N-piperidin-4-yl-acetamide. MS (ESI): mass calculated for C₂₈H₂₉N₃O₂S, 471.20; m/z found, 472.5 [M+H]⁺. ¹H NMR (400 MHz, CDCl₃): 7.77 (dd *J* = 8.1, 0.5, 1H), 7.70 (dd, *J* = 7.8, 0.7, 1H), 7.45-7.37 (m, 3H), 7.36-7.26 (m, 8H), 5.39 (br d, *J* = 7.9, 1 H), 3.93-3.82 (m, 1 H), 3.60 (s, 2H), 2.93 (br d, *J* = 11.1, 2H), 2.86 (dd, *J* = 10.9, 7.6, 2H), 2.65 (dd, *J* = 8.5, 5.2, 2H), 2.25 (t, *J* = 11.2, 2H), 1.95 (dd, *J* = 12.8, 3.3, 2H), 1.47 (m, 2H).

### EXAMPLE 111

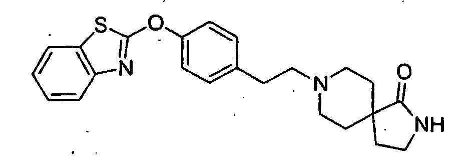

8-{2-[4-(Benzothiazol-2-yloxy)-phenyl]-ethyl}-2,8-diaza-spiro[4.5]decan-1-one.

The title compound was prepared according to the procedure for EXAMPLE 31 using 2,8-diaza-spiro[4.5]decan-1-one. MS (ESI): mass calculated for C₂₃H₂₅N₃O₂S, 407.17; m/z found, 408.4 [M+H]⁺. ¹H NMR (400 MHz, CDCl₃): 7.78 (d, *J* = 8.1, 1 H), 7.70 (dd, *J* = 7.8, 0.6, 1 H), 7.42 (dt, *J* = 7.6, 1.3, 1 H), 7.35-7.25 (m, 5H), 6.40 (br s, 1 H), 3.40 (t, *J* = 6.9, 2H), 3.00 (dt, *J* = 11.6, 3.6, 2H), 2.88 (dd, *J* = 10.3, 7.4, 2H), 2.67 (dd, *J* = 8.5, 5.7, 2H), 2.23 (t, *J* = 10.7, 2H), 2.10-2.00 (m, 4H), 1.52 (br d, *J* = 13.1, 2H).

### EXAMPLE 112

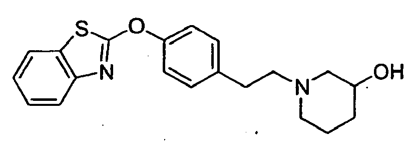

1-{2-[4-(Benzothiazol-2-yloxy)-phenyl]-ethyl}-piperidin-3-ol.

The title compound was prepared according to the procedure for EXAMPLE 31 using 3-hydroxypiperidine. MS (ESI): mass calculated for C₂₀H₂₂N₂O₂S, 354.14; m/z found, 355.3 [M+H]⁺. ¹H NMR (400 MHz, CDCl₃): 7.77 (d, *J* = 7.6, 1 H), 7.70 (dd, *J* = 8.0, 0.8, 1H), 7.42 (dt, *J* = 7.5, 1.2, 1 H), 7.35-7.28 (m, 5H), 3.89 (m, 1 H), 2.89 (dd, *J* = 10.1, 7.3, 2H), 2.71-2.65 (m, 2H), 2.64-2.54 (m, 2H), 2.53-2.36 (m, 3H), 1.92-1.80 (m, 1 H), 1.74-1.55 (m, 3H).

### EXAMPLE 113

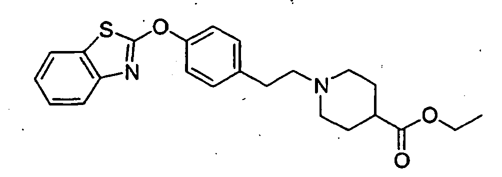

1-{2-[4-(Benzothiazol-2-yloxy)-phenyl]-ethyl}-piperidine-4-carboxylic acid ethyl ester.

The title compound was prepared according to the procedure for EXAMPLE 31 using piperidine-3-carboxylic acid ethyl ester. MS (ESI): mass calculated for C₂₃H₂₆N₂O₃S, 410.17; m/z found, 411.4 [M+H]⁺. ¹H NMR (400 MHz, CDCl₃): 7.77 (d, *J* = 8.0, 1 H), 7.69 (dd, *J* = 7.9, 0.7, 1 H), 7.41 (dt, *J* = 7.5, 1.2, 1 H), 7.32-7.27 (m, 5H), 4.18 (q, *J* = 7.1, 2H), 3.00 (br d, *J* = 11.5, 2H), 2.88 (dd, *J* = 10.9, 7.7, 2H), 2.64 (dd, *J* = 8.5, 5.3, 2H), 2.35 (tt, *J* = 10.9, 4.3, 1 H), 2.14, (t, *J* = 10.9, 2H), 2.02-1.94 (m, 2H), 1.90-1.78 (m, 2H), 1.30 (t, *J* = 7.3, 3H).

### EXAMPLE 114

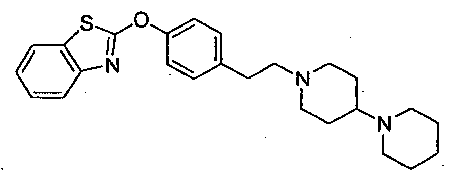

1'-{2-[4-(Benzothiazol-2-yloxy)-phenyl]-ethyl}-[1,4']bipiperidine.

The title compound was prepared according to the procedure for EXAMPLE 31 using 4-piperidinyl-piperidine. MS (ESI): mass calculated for C₂₅H₃₁N₃OS, 421.22; m/z found, 422.4 [M+H]⁺. ¹H NMR (400 MHz, CDCl₃): 7.75 (d, *J* = 7.8, 1 H), 7.69 (dd, *J* = 7.8, 0.6, 1 H), 7.41 (dt, *J* = 7.6, 1.2, 1 H), 7.33-7.25 (m, 5H), 3.15 (br d, *J* = 11.6, 2H), 2.90-2.70 (m, 7H) 2.65 (dd, *J* = 8.5, 5.3, 2H), 2.18-2.06 (m, 4H), 1.99-1.87 (m, 4H), 1.85-1.75 (m, 2H), 1.64-1.56 (m, 2H).

### EXAMPLE 115

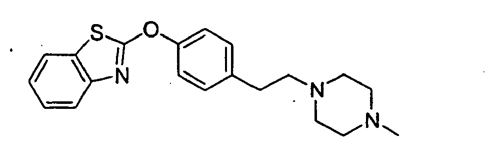

2-{4-[2-(4-Methyl-piperazin-1-yl)-ethyl]-phenoxy}-benzothiazole.

The title compound was prepared according to the procedure for EXAMPLE 32 using 1-methyl-piperazine. MS (ESI): mass calculated for C₂₀H₂₃N₃OS, 353.16; m/z found, 354.1 [M+H]⁺. ¹H NMR (400 MHz, CD₃OD): 7.77 (d, *J* = 8.01, 1 H), 7.64 (d, *J* = 8.1, 1 H), 7.41 (t, *J* = 8.2, 1 H), 7.37 (d, *J* = 8.6, 2H), 7.32-7.28 (m, 3H), 2.81-2.35 (m, 8H), 2.87 (m, 2H), 2.65 (m, 2H), 2.30 (s, 3H).

### EXAMPLE 116

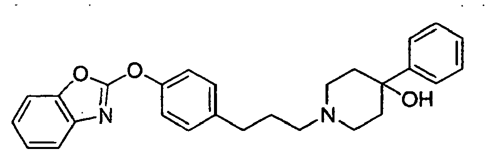

1-{3-[4-(Benzooxazol-2-yloxy)-phenyl]-propyl}-4-phenyl-piperidin-4-ol.

The title compound was prepared according to the procedure for EXAMPLE 35 using 4-phenyl-piperidin-4-ol. MS (ESI): mass calculated for C₂₇H₂₈N₂O₃, 428.21; m/z found, 429.3 [M+H]⁺. ¹H NMR (400 MHz, DMSO-*d*₆): 7.48 (d, *J* = 8.4, 1 H), 7.45 (m, 11 H), 7.19 (t, *J* = 8.3, 1 H), 4.76 (s, 1H), 2.66 (t, *J* = 7.6, 4H), 2.45 (m, 4H), 1.92 (t, *J* = 7.1, 2H), 1.78 (t, *J* = 7.1, 2H), 1.58 (d, *J* = 12.3, 2H).

### EXAMPLE 117

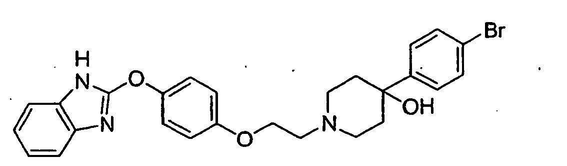

1-{2-[4-(1*H*-Benzoimidazol-2-yloxy)-phenoxy]-ethyl}-4-(4-bromo-phenyl)-piperidin-4-ol.

The title compound was prepared according to the procedure for EXAMPLE 37 using 4-(4-bromo-phenyl)-piperidin-4-ol. MS (ESI): mass calculated:for C₂₆H₂₆BrN₃O₃, 507.12; m/z found, 508.2 [M+H]⁺. ¹H NMR (400 MHz, CDCl₃): 7.43-7.07 (m, 10H), 6.94-6.89 (m, 2H), 4.11 (t, *J* = 5.8, 2H), 2.87-2.83 (m, 4H), 2.65-2.58 (m, 2H), 2.13-2.05 (m, 2H), 1.75-1.68 (m, 2H).

### EXAMPLE 118

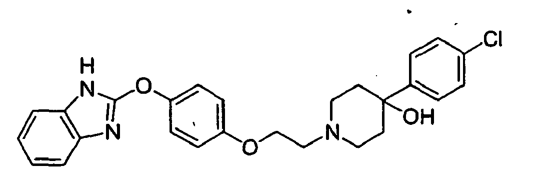

1-{2-[4-(1*H*-Benzoimidazol-2-yloxy)-phenoxy]-ethyl}-4-(4-chloro-phenyl)-piperidin-4-ol.

The title compound was prepared according to the procedure for EXAMPLE 37 using 4-(4-chloro-phenyl)-piperidin-4-ol. MS (ESI): mass calculated for C₂₆H₂₆ClN₃O₃, 463.17; m/z found, 464.2 [M+H]⁺. ¹H NMR (400 MHz, CDCl₃): 7.38-7.22 (m, 6H), 7.17-7.12 (m, 2H), 7.09-7.04 (m, 2H), 6.91-6.81 (m, 2H), 4.08 (t, *J* = 5.6, 2H), 2.84-2.80 (m, 4H), 2.62 (t, *J* = 11.8, 2H), 2.09-2.02 (m, 2H), 1.70-1.65 (m, 2H).

### EXAMPLE 119

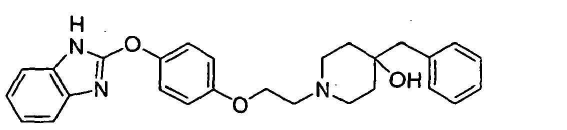

1-{2-[4-(1*H*-Benzoimidazol-2-yloxy)-phenoxy]-ethyl}-4-benzyl-piperidin-4-ol.

The title compound was prepared according to the procedure for EXAMPLE 37 using 4-benzyl-piperidin-4-ol. MS (ESI): mass calculated for C₂₇H₂₉N₃O₃, 443.22; m/z found, 444.3 [M+H]⁺. ¹H NMR (400 MHz, CDCl₃): 7.53 (br s, 1H), 7.34-7.24 (m, 4H), 7.26-7.16 (m, 7H), 6.91-6.85 (m, 2H), 4.08 (t, *J* = 5.8, 2H), 2.85-2.77 (m, 6H), 2.48 (dt, *J* = 2.5, 11.7, 2H), 1.84-1.76 (m, 2H), 1.56 (m, 2H).

### EXAMPLE 120

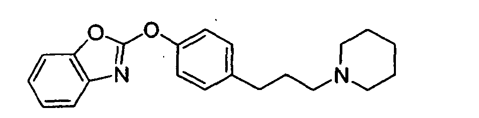

2-[4-(3-Piperidin-1-yl-propyl)-phenoxy]-benzooxazole.

The title compound was prepared according to the procedure for EXAMPLE 35 using piperidine. MS (ESI): mass calculated for C₂₁H₂₄N₂O₂, 336.18; m/z found, 337.3 [M+H]⁺. ¹H NMR (400 MHz, DMSO-*d*₆): 7.62 (dd, *J* = 5.7, 2.1, 1 H), 7.50 (dd, *J* = 6.1, 2.1, 1 H), 7.45-7.35 (m, 2H), 7.35-7.25 (m, 4H), 2.62 (t, *J* = 7.6, 2H), 2.30 (s, 4H), 2.25 (t, *J* = 7.4, 2H), 1.80-1.70 (m, 2H), 1.55-1.45 (m, 4H), 1.42-1.35 (m, 2H).

### EXAMPLE 121

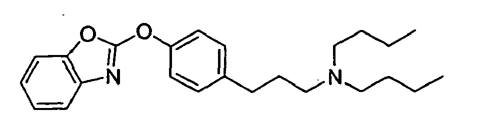

{3-[4-(Benzooxazol-2-yloxy)-phenyl]-propyl}-dibutyl-amine.

The title compound was prepared according to the procedure for EXAMPLE 35 using dibutyl-amine. MS (ESI): mass calculated for C₂₄H₃₂N₂O₂, 380.25; m/z found, 381.3 [M+H]⁺. ¹H NMR (400 MHz, DMSO-*d*₆): 7.63 (dd, *J* = 6.1, 2.1, 1H), 7.50 (dd, *J* = 6.1, 2.1, 1 H), 7.40 (d, *J* = 8.6, 2H), 2.63 (t, *J* = 7.7, 2H), 2.50-2.30 (m, 6H), 1.70 (quint, *J* = 7.2, 2H), 1.40-1.20 (m, 8H), 0.88 (t, *J* = 7.1, 6H).

### EXAMPLE 122

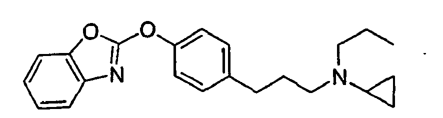

{3-[4-(Benzooxazol-2-yloxy)-phenyl]-propyl}-cyclopropylmethyl-propyl-amine.

The title compound was prepared according to the procedure,for EXAMPLE 35 using cyclopropylmethyl-propyl-amine. MS (ESI): mass calculated for C₂₃H₂₈N₂O₂, 364.22; m/z found, 365.3 [M+H]⁺. ¹H NMR (400 MHz, DMSO-*d*₆): 7.63 (d, *J* = 8.6, 1H), 7.40 (d, *J* = 8.6, 1H), 7.32 (d, *J* = 8.5, 2H), 7.30-7.25 (m, 4H), 2.63 (t, *J* = 7.6, 2H), 2.41 (t, *J* = 7.2, 2H), 2.28 (d, *J* = 6.3, 2H), 1.71 (quint, *J* = 7.2, 2H), 1.38 (q, *J* = 7.2, 2H), 0.85 (t, *J* = 7.3, 2H), 0.88-0.77 (m, 2H), 0.42 (d, *J* = 4.2, 2H), 0.05 (d, *J* = 4.8, 2H).

### EXAMPLE 123

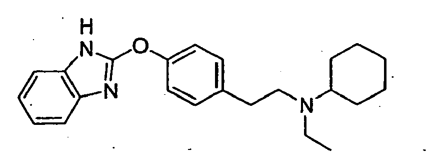

{2-[4-(1*H*-Benzoimidazol-2-yloxy)-phenyl]-ethyl}-cyclohexyl-ethyl-amine.

The title compound was prepared according to the procedure for EXAMPLE 38 using cyclohexyl-ethyl-amine. MS (ESI): mass calculated for C₂₃H₂₉N₃O, 363.23; m/z found, 364.4 [M+H]⁺. ¹H NMR (400 MHz, CDCl₃): 11.44 (br s, 1H), 7.43-7.33 (m, 2H), 7.13-7.05 (m, 6H), 3.17 (t, *J* = 11.7, 1H), 3.10 (dd, *J* = 7.0, 7.0, 2H), 2.99-2.83 (m, 4H), 2.06 (d, *J* =9.8, 2H), 1.89 (d, *J* = 12.5, 2H), 1.69 (d, *J* = 12.5, 1 H), 1.49-1.22 (m, 7H), 1.19-1.05 (m, 1 H).

### EXAMPLE 124

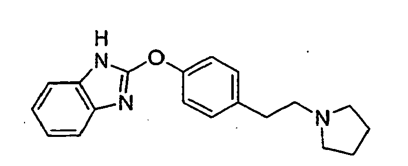

2-[4-(2-Pyrrolidin-1-yl-ethyl)-phenoxy]-1*H*-benzoimidazole.

The title compound was prepared according to the procedure for EXAMPLE 38 using pyrrolidine. MS (ESI): mass calculated for C₁₉H₂₁N₃O, 307.17; m/z found, 308.3 [M+H]⁺. ¹H NMR (400 MHz, CDCl₃): 7.35-7.22 (m, 2H), 7.14-7.04 (m, 6H), 2.79-2.72 (m, 2H), 2.67-2.57 (m, 6H), 1.87-1.77 (m, 4H).

### EXAMPLE 125

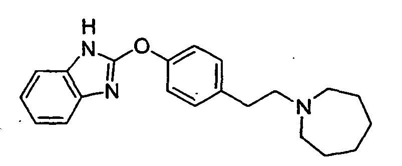

2-[4-(2-Azepan-1-yl-ethyl)-phenoxy]-1*H*-benzoimidazole.

The title compound was prepared according to the procedure for EXAMPLE 38 using azepane. MS (ESI): mass calculated for C₂₁H₂₅N₃O, 335.20; m/z found, 336.4 [M+H]⁺. ¹H NMR (400 MHz, CDCl₃): 7.38 (dd, *J* = 3.1, 2.7, 2H), 7.11 (dd, *J* = 3.1, 2.9, 2H), 7.10-7.00 (m, 4H), 3.25-3.15 (m, 4H), 3.06 (dd, *J* = 6.3, 5.3, 2H), 2.93 (dd, *J* = 6.1, 4.3, 2H), 2.00-1.91 (m, 4H), 1.75-1.67 (m, 4H).

### EXAMPLE 126

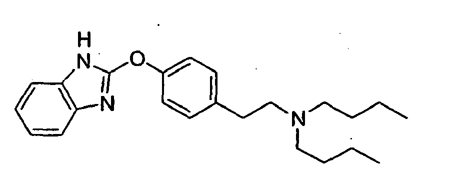

{2-[4-(1*H*-Benzoimidazol-2-yloxy)-phenyl]-ethyl}-dibutyl-amine.

The title compound was prepared according to the procedure for EXAMPLE 38 using dibutyl-amine. MS (ESI): mass calculated for C₂₃H₃₁N₃O, 365.25; m/z found, 366.5 [M+H]⁺. ¹H NMR (400 MHz, CDCl₃): 7.40-7.29 (m, 2H), 7.17-7.06 (m, 6H), 2.83-2.60 (m, 8H), 1.59-1.47 (m, 4H), 1.38-1.26 (m, 4H), 0.93 (t, *J* = 7.2, 6H).

### EXAMPLE 127

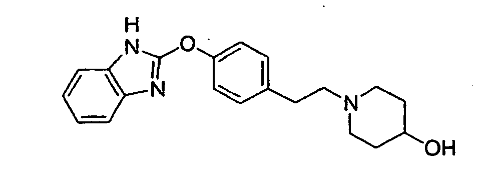

1-{2-[4-(1*H*-Benzoimidazol-2-yloxy)-phenyl]-ethyl}-piperidin-4-ol.

The title compound was prepared according to the procedure for EXAMPLE 38 using 4-hydroxypiperidine. MS (ESI): mass calculated for C₂₀H₂₃N₃O₂, 337.18; m/z found, 338.4 [M+H]⁺. ¹H NMR (400 MHz, CDCl₃): 11.70 (br s, 1H), 7.50-7.44 (m, 1H), 7.33-7.27 (m, 1H), 7.24 (br s, 4H), 7.14-7.07 (m, 2H), 3.93 (br s, 1H), 3.68-3.58 (m, 1H), 2.97-2.75 (m, 4H), 2.63-2.52 (m, 2H), 2.19 (t, *J* = 9.8, 2H), 1.94-1.84 (m, 2H), 1.65-1.54 (m, 2H).

### EXAMPLE 128

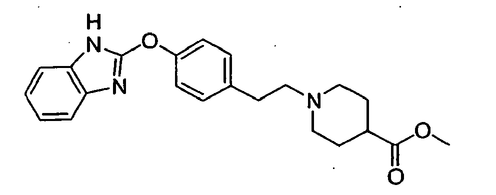

1-{2-[4-(1*H*-Benzoimidazol-2-yloxy)-phenyl]-ethyl}-piperidine-4-carboxylic acid methyl ester.

The title compound was prepared according to the procedure for EXAMPLE 38 using piperidine-4-carboxylic acid methyl ester. MS (ESI): mass calculated for C₂₂H₂₅N₃O₃, 379.19; m/z found, 380.4 [M+H]⁺. ¹H NMR (400 MHz, CDCl₃): 11.76 (br s, 1H), 7.50-7.44 (m, 1 H), 7.24-7.06 (m, 7H), 3.68 (s, 3H), 3.00-2.92 (m, 2H), 2.77-2.70 (m, 2H), 2.53-2.46 (m, 2H), 2.38-2.28 (m, 1 H), 2.10 (t, *J* = 11.1, 2H), 1.98-1.90 (m, 2H), 1.86-1.74 (m, 2H).

### EXAMPLE 129

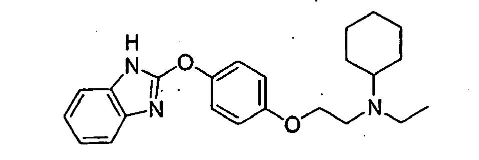

{2-[4-(1*H*-Benzoimidazol-2-yloxy)-phenoxy]-ethyl}-cyclohexyl-ethyl-amine.

The title compound was prepared according to the procedure for EXAMPLE 37 using cyclohexyl-ethyl-amine. MS (ESI): mass calculated for C₂₃H₂₉N₃O₂, 379.23; m/z found, 380.3 [M+H]⁺. ¹H NMR (400 MHz, DMSO-*d*₆): 12.23 (s, 1H), 7.34-7.25 (m, 4H), 7.07 (d, *J* = 3.5, 2H), 6.98 (d, *J* = 9.0, 2H), 3.94 (t, *J* = 6.4, 2H), 2.80 (t, *J* = 6.4, 2H), 2.57 (d, *J* = 7.1, 2H), 2.50 (s, 2H), 1.72 (d, *J* = 7.8, 2H), 1.55 (s, 1 H), 1.20 (t, *J* = 9.0, 4H), 0.99 (t *J* = 7.1, 3H).

### EXAMPLE 130

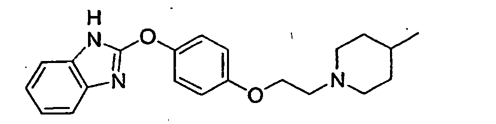

2-{4-[2-(4-Methyl-piperidin-1-yl)-ethoxy]-phenoxy}-1*H*-benzoimidazole.

The title compound was prepared according to the procedure for EXAMPLE 35 using 4-methyl-piperidine. MS (ESI): mass calculated for C₂₁H₂₅N₃O₂, 351.19; m/z found, 352.3 [M+H]⁺. ¹H NMR (400 MHz, DMSO-*d*₆): 12.30 (s, 1 H), 7.34-7.25 (m, 4H), 7.08 (d, *J* = 3.8, 2H), 6.99 (d, *J* = 9.0, 2H), 4.08 (t, *J* = 5.8, 2H), 2.89 (d, *J* = 11.4, 2H), 2.67 (t, *J* = 5.8, 2H), 2.00 (t, *J* = 11.4, 2H), 1.56 (d, *J* = 11.5, 2H), 1.36-1.25 (m, 1 H), 1.21- 1.08 (m, 2H), 0.88 (d, *J* = 6.4, 3H).

### EXAMPLE 131

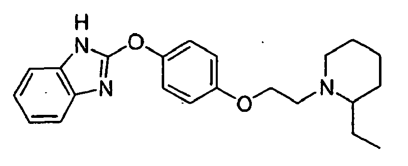

2-{4-[2-(2-Ethyl-piperidin-1-yl)-ethoxy]-phenoxy}-1*H*-benzoimidazole.

The title compound was prepared according to the procedure for EXAMPLE 37 using 2-ethyl-piperidine. MS (ESI): mass calculated for C₂₂H₂₇N₃O₂, 365.21; m/z found, 366.3 [M+H]⁺. ¹H NMR (400 MHz, DMSO-*d*₆): 12.28 (s, 1H), 7.32-7.25 (m, 4H), 7.08 (d, *J* = 3.8, 2H), 6.99 (d, *J* = 8.4, 2H), 4.05 (t, *J* = 6.1, 2H), 3.00-2.92 (m, 1H), 2.91-2.85 (m, 1H), 2.72-2.64 (m, 1 H), 2.38-2.20 (m, 2H), 1.67-1.36 (m, 6H), 1.35-1.19 (m, 2H), 0.84 (t, *J* = 7.5, 3H).

### EXAMPLE 132

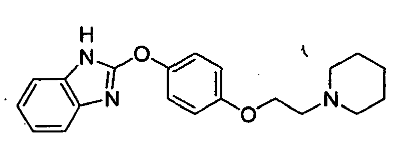

2-[4-(2-Piperidin-1-yl-ethoxy)-phenoxy]-1*H*-benzoimidazole.

The title compound was prepared according to the procedure for EXAMPLE 37 using piperidine. MS (ESI): mass calculated for C₂₀H₂₃N₃O₂, 337.18; m/z found, 338.3 [M+H]⁺. ¹H NMR (400 MHz, DMSO-*d*₆): 12.25 (br s, 1H), 7.35-7.25 (m, 4H), 7.10-7.00 (m, 2H), 6.99 (d, *J* = 9.0, 2H), 4.07 (t, *J* = 5.9, 2H), 2.79 (t, *J* = 5.9, 2H), 2.43 (s, 4H), 1.55-1.45 (m, 4H), 1.38 (s, 2H).

### EXAMPLE 133 .

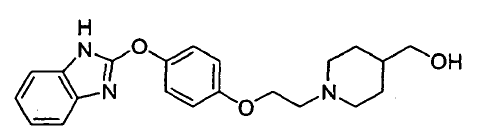

(1-{2-[4-(1*H*-Benzoimidazol-2-yloxy)-phenoxy]-ethyl}-piperidin-4-yl)methanol.

The title compound was prepared according to the procedure for EXAMPLE 35 using piperidin-4-yl-methanol. MS (ESI): mass calculated for C₂₁H₂₅N₃O₃, 367.19; m/z found, 368.4 [M+H]⁺. ¹H NMR (400 MHz, DMSO-*d*₆): 12.24 (s, 1 H), 7.35-7.25 (m, 4H), 7.01 (s, 2H), 6.99 (d, *J* = 7.1, 2H), 4.40 (s, 1H), 4.10 (t, *J* = 5.7, 2H), 3.25 (t, *J* = 5.4, 2H), 2.93 (d, *J* = 11.1, 2H), 2.67 (t, *J* = 5.6, 2H), 1.98 (t, *J* = 11.6, 2H), 1.63 (d, *J* = 11.6, 2H), 1.28 (s, 1H), 1.12 (d, *J* = 9.1, 2H).

### EXAMPLE 134

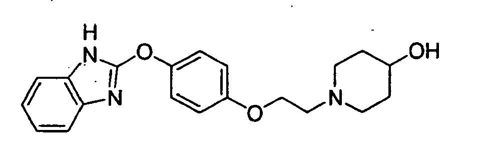

1-{2-[4-(1*H*-Benzoimidazol-2-yloxy)-phenoxy]-ethyl}-piperidin-4-ol.

The title compound was prepared according to the procedure for EXAMPLE 37 using 4-hydroxypiperidine. MS (ESI): mass calculated for C₂₀H₂₃N₃O₃, 353.17; m/z found, 354.4 [M+H]⁺. ¹H NMR (400 MHz, DMSO-*d*₆): 12.24 (s, 1H), 7.35-7.25 (m, 4H), 7.07 (d, *J* = 9.0, 2H), 6.99 (d, *J* = 9.0, 2H), 4.40 (s, 1 H), 4.08 (t, *J* = 5.8, 2H), 2.53 (s, 1H), 2.78 (d, *J* = 11.2, 2H), 2.66 (t, *J* = 5.8, 2H), 2.12 (t, *J* = 10.3, 2H), 1.70 (d, *J* = 9.1, 2H), 1.38 (d, *J* = 9.9, 2H).

### EXAMPLE 135

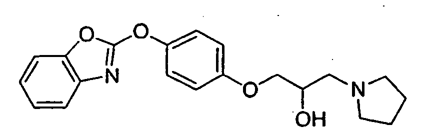

1-[4-(Benzooxazol-2-yloxy)-phenoxy]-3-pyrrolidin-1-yl-propan-2-ol.

The title compound was prepared according to the procedure for EXAMPLE 40 using pyrrolidine. MS (ESI): mass calculated for C₂₀H₂₂N₂O₄, 354.16; m/z found, 355.2 [M+H]⁺. ¹H NMR (400 MHz, DMSO-*d*₆): 7.62 (dd, *J* = 6.2, 2.0, 1 H), 7.50 (dd, *J* = 6.1, 2.1, 1 H), 7.42 (d, *J* = 9.1, 2H), 7.35-7.25 (m, 2H), 7.03 (d, *J* = 9.1, 2H), 4.91 (d, *J* = 4.5, 1H), 4.05-4.00 (m, 1 H), 4.00-3.85 (m, 2H), 2.70 (m, 1 H), 2.50-2.40 (m, 5H), 1.68 (s, 4H).

### EXAMPLE 136

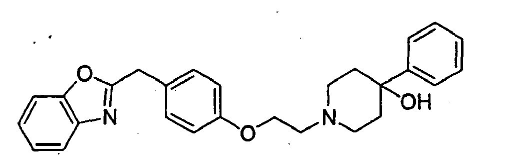

1-[2-(4-Benzooxazol-2-ylmethyl-phenoxy)-ethyl]-4-phenyl-piperidin-4-ol.

The title compound was prepared according to the procedure for EXAMPLE 41 using 4-phenyl-piperidin-4-ol. MS (ESI): mass calculated for C₂₇H₂₈N₂O₃, 428.21; m/z found, 429.2 [M+H]⁺. ¹H NMR (400 MHz, CD₃OD): 7.62-7.58 (m, 1 H), 7.54-7.45 (m, 3H), 7.33-7.25 (m, 6H), 7.20-7.15 (m, 1H), 6.91 (d, *J* = 8.6, 2H), 4.20 (s, 2H), 4.13 (t, *J* = 5.5, 2H), 2.87 (m, 4H), 2.66 (t, *J* = 11.4, 2H), 2.13 (dt, *J* = 12.8, 3.6, 2H), 1.72 (d, *J* = 12.6, 2H).

### EXAMPLE 137

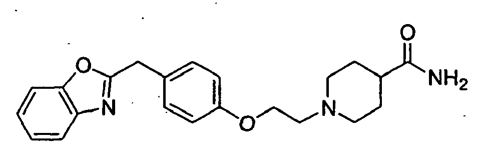

1-[2-(4-Benzooxazol-2-ylmethyl-phenoxy)-ethyl]-piperidine-4-carboxylic acid amide.

The title compound was prepared according to the procedure for EXAMPLE 41 using piperidine-4-carboxylic acid amide. MS (ESI): mass calculated for C₂₂H₂₅N₃O₃, 379.19; m/z found, 380.4 [M+H]⁺. ¹H NMR (400 MHz, CD₃OD): 7.64-7.60 (m, 1 H), 7.56-7.52 (m, 1 H), 7.36-7.31 (m, 2H), 7.28 (d, *J* = 8.7, 2H), 6.92 (d, *J* = 8.7, 2H), 4.22 (s, 2H), 4.11 (t, *J* = 5.6, 2H), 3.07 (d, *J* = 12.0, 2H), 2.79 (t, *J* = 5.6, 2H), 2.18-2.15 (m, 3H), 1.80-1.70 (m, 4H).

### EXAMPLE 138

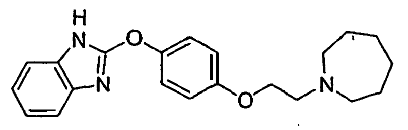

2-[4-(2-Azepan-1-yl-ethoxy)-phenoxy]-1*H*-benzoimidazole amide.

The title compound was prepared according to the procedure for EXAMPLE 11 steps A and B and EXAMPLE 37 steps B and C using 1-(2-chloro-ethyl)-azepane hydrochloride. MS (ESI): mass calculated for C₂₁H₂₅N₃O₂, 351.19; m/z found, 352.3 [M+H]⁺. ¹H NMR (400 MHz, DMSO-*d*₆): 12.28 (s, 1H), 7.35-7.25 (m, 4H), 7.07 (d, *J* = 9.1, 2H), 7.00 (d, *J* = 9.1, 2H), 4.05 (t, *J* = 6.0, 2H), 2.86 (t, *J* = 6.0, 2H), 2.70 (t, *J* = 5.1, 4H), 1.65-1.50 (m, 8H).

### EXAMPLE 139

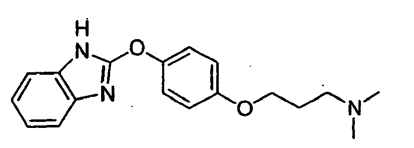

{3-[4-(1*H*-Benzoimidazol-2-yloxy)-phenoxy]-propyl}-dimethyl-amine.

The title compound was prepared according to the procedure for EXAMPLE 11 steps A and B and EXAMPLE 37 steps B and C using (2-chloro-propyl)-dimethyl-amine hydrochloride. MS (ESI): mass calculated for C₁₈H₂₁N₃O₂, 311.16; m/z found, 312.2 [M+H]⁺. ¹H NMR (400 MHz, CDCl₃): 7.32-7.23 (m, 2H), 7.21-7.14 (m, 4H), 6.89-6.83 (m, 2H), 3.99-3.91 (m, 2H), 2.51 (t, *J* = 7.2, 2H), 2.31 (s, 6H) 2.02-1.95 (m, 2H).

### EXAMPLE 140

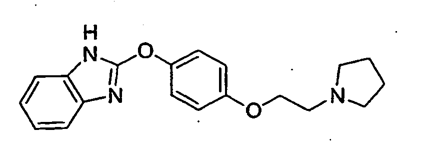

2-[4-(2-Pyrrolidin-1-yl-ethoxy)-phenoxy]-1*H*-benzoimidazole.

The title compound was prepared according to the procedure for EXAMPLE 21 steps A and B and EXAMPLE 35 steps B and C using pyrrolidine. MS (ESI): mass calculated for C₁₉H₂₁N₃O₂, 323.16; m/z found, 324.2 [M+H]⁺. ¹H NMR (400 MHz, CDCl₃): 7.27 (br s, 2H), 7.15 (d, *J* = 9.1, 4H), 6.81 (d, *J* = 6.8, 2H), 4.16 (t, *J* = 5.5, 2H), 3.05 (t, *J* = 5.5, 2H), 2.86 (br s, 4H), 1.92 (br s, 4H).

### EXAMPLE 141

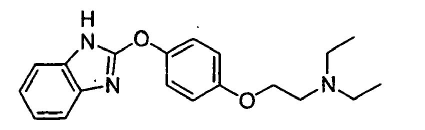

{2-[4-(1*H*-Benzoimidazol-2-yloxy)-phenoxy]-ethyl}-diethyl-amine.

The title compound was prepared according to the procedure for EXAMPLE 21 steps A and B and EXAMPLE 35 steps B and C using diethylamine. MS (ESI): mass calculated for C₁₉H₂₃N₃O₂, 325.18; m/z found, 326.3 [M+H]⁺. ¹H NMR (400 MHz, CDCl₃): 7.29 (br s, 2H), 7.18 (d, *J* = 9.0, 2H), 7.10-7.05 (m, 2H), 6.98 (d, *J* = 9.0, 2H), 4.03 (t, *J* = 6.2, 2H), 2.78 (t, *J* = 6.1, 2H), 2.55 (q, *J* = 7.1, 4H), 0.98 (t, *J* = 7.1, 6H).

### EXAMPLE 142

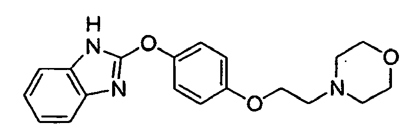

2-[4-(2-Morpholin-4-yl-ethoxy)-phenoxy]-1*H*-benzoimidazole.

The title compound was prepared according to the procedure for EXAMPLE 21 steps A and B and EXAMPLE 37 steps B and C using morpholine. MS (ESI): mass calculated for C₁₉H₂₁N₃O₃, 339.16; m/z found, 340.2 [M+H]⁺. ¹H NMR (400 MHz, OMSO-*d₆*): 7.40-7.25 (m, 4H), 7.12-7.08 (m, 2H), 7.00 (d, *J* = 9.0, 2H), 4.10 (t, *J* = 5.7, 2H), 3.58 (t, *J* = 4.5, 4H), 2.70 (t, *J* = 5.7, 2H), 2.52-2.46 (m, 4H).

### EXAMPLES 143-202 and 204-229

Compounds of this invention that are referred to herein for which no explicit preparation description is provided can be prepared according to procedures analogous to those described herein in light of the knowledge of one of ordinary skill in the art and the teachings provided herein. For example, benzothiazole derivatives listed herein with reference numerals 143-202 and 204-229 can be prepared according to procedures analogous to those described herein for related compounds.

### EXAMPLE 203

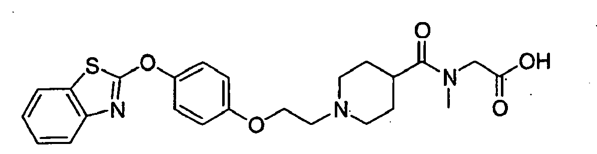

[(1-{2-[4-(Benzothiazol-2-yloxy)-phenoxy]-ethyl}-piperidine-4-carbonyl)-methylamino]-acetic acid

The title compound was prepared according to the procedures for EXAMPLE 20 and EXAMPLE 18 using sarcosine ethyl ester hydrochloride. MS (ESI): mass calculated for C₂₄H₂₇N₃O₅S, 469.56; m/z found, 470.3 [M+H]⁺. ¹H NMR (400 MHz, CDCl₃): 7.71 (d, *J* = 8.02, 1 H), 7.65 (d, *J* = 8.02, 1 H), 7.34-7.40 (m, 1 H), 7.23-7.30 (m, 3H), 6.92-6.98 (m, 2H), 4.34-4.44 (m, 2H), 3.92 (br s, 2H), 3.45-3.54 (m, 3H), 3.35 (br s, 1H), 3.25 (br s, 1 H), 3.12 (s, 2H), 2.96 (s, 1 H), 2.76 (br s, 2H), 1.92-2.08 (m, 4H).

### EXAMPLES 230-231 and 485

As indicated in the context of this written description, compounds of this invention that are referred to herein for which no explicit preparation description is provided can be prepared according to procedures analogous to those described herein in light of the knowledge of one of ordinary skill in the art and the teachings provided herein. For example, benzoimidazole derivatives listed herein with reference numerals 230-231 and 485 can be prepared according to procedures analogous to those described herein for related compounds.

### EXAMPLE 250

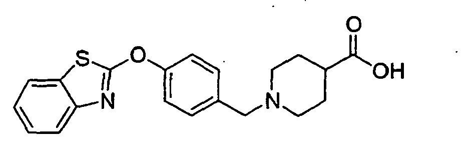

1-[4-(Benzothiazol-2-yloxy)-benzyl]-piperidine-4-carboxylic acid.
A. 1-(4-Benzyloxy-benzyl)-piperidine-4-carboxylic acid ethyl ester. A mixture of 4-benzyloxybenzyl chloride (15.2 g, 65.3 mmol), isonipecotic acid ethyl ester (15 mL, 97 mmol), and K₂CO₃ (13.5 g, 97.6 mmol) in CH₃CN (300 mL) was stirred at reflux for 20 h. The reaction mixture was cooled to room temperature and filtered. The solvent was removed under reduced pressure to yield a clear golden oil. This material was diluted with *i*PrOH (100 mL), and the mixture was filtered. The solid was air dried to yield a white solid (19.7 g, 85% yield). TLC (SiO₂, 15% acetone/CH₂Cl₂): R_{f} = 0.32. MS (ESI): mass calculated for C₂₂H₂₇NO₃, 353.2; m/z found, 354.3 [M+H]⁺. ¹H NMR (400 MHz, DMSO-*d*₆): 7.44 (d, *J* = 7.1, 2H), 7.39 (t, *J* = 7.1, 2H), 7.33 (d, *J* = 7.2, 1 H), 7.18 (d, *J* = 8.2, 2H), 6.94 (2H, *J* = 8.6, 2H), 5.08 (s, 2H), 4.04 (q, *J* = 7.09, 2H), 2.72 (d, *J* = 11.5, 2H), 2.32-2.18 (m, 1H), 1.94(t, *J* = 11.6, 2H), 1.76 (d, *J* = 10.2, 2H), 1.59-1.48 (m, 2H), 1.17 (t, *J* = 7.1, 3H).
B. 1-(4-Hydroxy-benzyl)-piperidine-4-carboxylic acid ethyl ester. 1-(4-Benzyloxy-benzyl)-piperidine-4-carboxylic acid ethyl ester (10.0 g, 28.3 mmol) was dissolved in 1:1 ethanol/ethyl acetate (150 mL). To this solution was added Pd on carbon (10 wt %, 503 mg) as a suspension in ethanol (5.0 mL). The resulting suspension was placed on a Parr hydrogenator at 40 psi of H₂ and shaken overnight. The reaction mixture was filtered through a pad of diatomaceous earth, and the filtrate was concentrated under reduced pressure to give a clear golden oil. The oil was purified on SiO₂ (90 g; 50% acetone/CH₂Cl₂) to give a white solid (2.0 g, 27% yield). TLC (SiO₂, 50% acetone/CH₂Cl₂): R_{f} = 0.32. MS (ESI): mass calculated for C₁₅H₂₁NO₃, 263.2; m/z found, 264.2 [M+H]⁺. ¹H NMR (400 MHz, DMSO-*d₆*): 9.25 (s, 1H), 7.05 (d, *J* = 8.4, 2H), 6.68 (d, *J* = 8.4, 2H), 4.04 (q, *J* = 7.1, 2H), 3.34 (s, 2H), 2.71 (d, *J* = 11.5, 2H), 2.32-2.18 (m, 1H), 1.92 (t, *J* = 11.6, 2H), 1.76 (d, *J* = 10.2, 2H), 1.59- 1.48 (m, 2H), 1.17 (t, *J* = 7.1, 3H).
C. 1-[4-(Benzothiazol-2-yloxy)-benzyl]-piperidine-4-carboxylic acid ethyl ester. To a stirring solution of 1-(4-hydroxy-benzyl)-piperidine-4-carboxylic acid ethyl ester (508 mg, 1.93 mmol) in CH₃CN (15 mL), was added K₂CO₃ (564 mg, 4.1 mmol) and 2-chlorobenzothiazole (0.50 mL, 4.0 mmol). The suspension was heated to 80 °C and stirred overnight. The reaction mixture was allowed to cool to room temperature and then filtered through diatomaceous earth. The filtrate was concentrated under reduced pressure, and the residue was purified on SiO₂ (12 g; 0-15% acetone/CH₂Cl₂) to give a clear and colorless tacky oil (717 mg, 94% yield). TLC (SiO₂, 15% acetone/CH₂Cl₂): R_{f} = 0.5. MS (ESI): mass calculated for C₂₂H₂₄N₂O₃S, 396.2; m/z found, 397.3 [M+H]⁺. ¹H NMR (400 MHz, DMSO-*d*₆): 7.92 (d, *J* = 8.0, 1 H), 7.68 (d, *J* = 8.0, 1 H), 7.48-7.33 (m, 5H), 7.33 (t, *J* = 7.1, 1 H), 4.06 (q, *J* = 7.1, 2H), 3.49 (s, 2H), 2.76 (d, *J* = 11.5, 2H), 2.34-2.22 (m, 1 H), 2.02 (t, *J* = 11.6, 2H), 1.80 (d, *J* = 10.2, 2H), 1.64- 1.54 (m, 2H), 1.18 (t, *J* = 7.1, 3H).
D. 1-[4-(Benzothiazol-2-yloxy)-benzyl]-piperidine-4-carboxylic acid. To a stirring solution of 1-[4-(benzothiazol-2-yloxy)-benzyl]-piperidine-4-carboxylic acid ethyl ester (663 mg, 1.7 mmol) in 25% *i*PrOH/H₂O (20 mL) was added potassium hydroxide (206 mg, 3.1 mmol). The reaction mixture was stirred at room temperature for 20 h, and the solution was treated to pH 5.5 with 1 M HCl. The resulting solution was extracted with 10% *i*PrOH/CHCl₃ (3 x 50 mL). The combined extracts were dried (MgSO₄), filtered, and concentrated under reduced pressure to yield a white solid (561 mg, 91% yield). MS (ESI): mass calculated for C₂₀H₂₀N₂O₃S, 368.1; m/z found, 369.3 [M+H]⁺. ¹H NMR (400 MHz, DMSO-*d₆*): 7.92 (d, *J* = 7.6, 1 H), 7.69 (d, *J* = 7.6, 1H), 7.48-7.34 (m, 5H), 7.33 (t, *J* = 7.1, 1 H), 3.49 (s, 2H), 2.76 (d, *J* = 11.4, 2H), 2.22-2.11 (m, 1 H), 2.02 (t, *J* = 11.2, 2H), 1.80 (d, *J* = 13.2, 2H), 1.62- 1.48 (m, 2H), 1.18 (t, *J* = 7.1, 3H).

### EXAMPLE 251

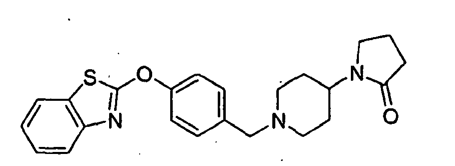

1-{1-[4-(Benzothiazol-2-yloxy)-benzyl]-piperidin-4-yl}-pyrrolidin-2-one.
A. 4-(Bemzothiazol-2-yloxy)-benzaldehyde. To a mixture of 4-hydroxybenzaldehyde (1 g, 8.2 mmol) and 2-chlorobenzothiazole (2.03 mL, 16.4 mmol) in CH₃CN (100 mL) was added Cs₂CO₃ (5.5 g, 17.2 mmol). The reaction mixture was stirred at 60 °C for 24 h. The resulting mixture was cooled to room temperature, filtered through diatomaceous earth and concentrated under reduced pressure to yield the crude product as an orange oil. The oil was triturated with hexanes/CH₂Cl₂ (100 mL) and the solvent layer decanted and concentrated under reduced pressure to yield an orange oil which was further purified on SiO₂ (120 g; 0-50% ethyl acetate/hexanes) to give a white solid (853 mg, 41% yield). ¹H NMR (400 MHz, CDCl₃): 10.1 (s, 1H), 7.97-7.78 (m, 2H), 7.78-7.70 (m, 2H), 7.60-7.50 (m, 2H), 7.48-7.38 (m, 1H), 7.36-7.30 (m, 1 H).
B. 1-{1-[4-(Benzothiazol-2-yloxy)-benzyl]-piperidin-4-yl}-pyrrolidin-2-one. A mixture of 4-(benzothiazol-2-yloxy)-benzaldehyde (500 mg, 1.9 mmol), 1-piperidin-4-yl-pyrrolidin-2-one hydrochloride (440 mg, 2.2 mmol), Et₃N (300 µL, 2.2 mmol) and molecular sieves (500 mg, crushed, 4Å) in ClCH₂CH₂Cl (10 mL) was stirred at room temperature for 1 h. To the resulting mixture was added NaBH(OAc)₃ (830 mg, 3.92 mmol). The mixture was stirred at room temperature for 24 h, filtered through diatomaceous earth, rinsed with CH₂Cl₂ (50 mL) and concentrated under reduced pressure to yield the crude product as a yellow oil. The crude product was purified on SiO₂ (40 g; 0-100% acetone/CH₂Cl₂) to give a clear oil which crystallized on standing (287 mg, 36% yield). MS (ESI): mass calculated for C₂₃H₂₅N₃O₂S, 407.5; m/z found, 408.3 [M+H]⁺. ¹H NMR (400 MHz, CDCl₃): 7.76 (d, *J* = 8.0, 1 H), 7.69 (d, *J* = 8.0, 1H), 7.41-7.30 (m, 3H), 7.35-7.27 (m, 3H), 4.46-3.98 (m, 1H), 3.71 (s, 2H), 3.36 (t, *J* = 6.9, 2H), 2.97 (d, *J* = 11.7, 2H), 2.40 (t, *J* = 8.1, 2H), 2.17-1.97 (m, 4H), 1.81-1.62 (m, 4H).

### EXAMPLE 252

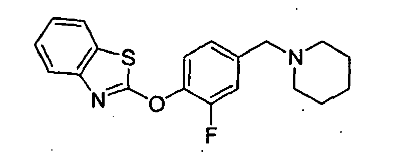

2-(2-Fluoro-4-piperidin-1-ylmethyl-phenoxy)-benzothiazole.
A. (3-Fluoro-4-hydroxy-phenyl)-piperidin-1-yl-methanone. A solution of 3-fluoro-4-hydroxybenzoic acid (5.0 g, 32 mmol), piperidine (5 mL, 51 mmol), and EDCl (9.3 g, 49 mmol) in CH₂Cl₂ (100 mL) was stirred at room temperature for 20 h. The reaction mixture was added to CH₂Cl₂ (200 mL) and was washed with 1 M HCl (2 x 100 mL). The organic layers were combined, dried (MgSO₄), and concentrated under reduced pressure to yield a clear golden oil. The oil was purified on SiO₂ (120 g; 0-10% acetone/CH₂Cl₂) to give a white solid (2.4 g, 34% yield). TLC (SiO₂, 15% acetone/CH₂Cl₂): R_{f} = 0.35. MS (ESI): mass calculated for C₁₂H₁₄FNO₂, 223.1; m/z found, 224.3 [M+H]⁺. ¹H NMR (400 MHz, DMSO-*d₆*): 10.26 (s, 1 H), 7.18 (d, *J* = 11.6, 1 H), 7.00 (d, *J* = 8.3, 1 H), 6.98 (t, *J* = 8.5, 1 H), 3.42 (br s, 4H), 1.65-1.45 (m, 6H).
B. 2-Fluoro-4-piperidin-1-ylmethyl-phenol. A solution of lithium aluminum hydride (1.9 g, 50 mmol) in THF (40 mL) was stirred at 5 °C. To the mixture was added (3-fluoro-4-hydroxy-phenyl)-piperidin-1-yl-methanone (2.3 g, 10.4 mmol) in THF (10 mL) over 15 min and then the mixture was heated to 60 °C. After 20 h the mixture was cooled to 5 °C and saturated NH₄Cl (200 mL) was added followed by CH₂Cl₂ (200 mL). The organic layer was separated, dried (MgSO₄), and concentrated under reduced pressure to yield a white solid (812 mg, 37% yield). TLC (SiO₂, acetone): R_{f} = 0.22. MS (ESI): mass calculated for C₁₂H₁₆FNO, 209.1; m/z found, 210.3 [M+H]⁺. ¹H NMR (400 MHz, DMSO-*d*₆): 6.58 (d, *J* = 13.4, 1 H), 6.48 (d, *J* = 8.2, 1 H), 6.40 (t, *J* = 9.9, 1 H), 3.14 (s, 2H), 2.24 (br s, 4H), 1.54-1.30 (m, 6H).
C. 2-(2-Fluoro-4-piperidin-1-ylmethyl-phenoxy)-benzothiazole. To a stirring solution of 2-fluoro-4-piperidin-1-ylmethyl-phenol (152 mg, 0.73 mmol) in CH₃CN (10 mL), was added K₂CO₃ (201 mg, 1.5 mmol) and 2-chlorobenzothiazole (0.14 mL, 1.1 mmol). The suspension was heated to 80 °C and stirred overnight. The reaction mixture was allowed to cool to room temperature and then filtered through diatomaceous earth. The filtrate was concentrated under reduced pressure, and the residue was purified on SiO₂ (12 g; 0-50% acetone/CH₂Cl₂) to give a clear golden oil (142 mg, 57% yield). TLC (SiO₂, 50% acetone/CH₂Cl₂): R_{f} = 0.44. MS (ESI): mass calculated for C₁₉H₁₉FN₂OS, 342.1; m/z found, 343.3 [M+H]⁺. ¹H NMR (400 MHz, DMSO-*d₆*): 7.95 (d, *J* = 7.9, 1H), 7.69 (d, *J* = 7.5, 1 H), 7.56 (t, *J* = 8.2, 1 H), 7.47-7.32 (m, 3H), 7.44 (d, *J* = 15.4, 1 H), 3.48 (s, 2H), 2.36 (s, 4H), 1.63-1.37 (m, 6H).

### EXAMPLE 253

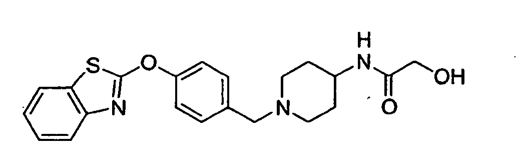

N-{1-[4-(Benzothiazol-2-yloxy)-benzyl]-piperidin-4-yl}-2-hydroxy-acetamide.
A. {1-[4-(Benzothiazol-2-yloxy)-benzyl]-piperidin-4-yl}-carbamic acid tert-butyl ester. A mixture of 4-(benzothiazol-2-yloxy)-benzaldehyde (EXAMPLE 251, step A, 500 mg, 1.9 mmol), piperidin-4-yl-carbamic acid tert-butyl ester (785 mg, 3.9 mmol) and molecular sieves (500 mg, crushed, 4Å) in ClCH₂CH₂Cl (10 mL) was stirred at room temperature for 40 min. To the resulting reaction mixture was added NaBH(OAc)₃ portion wise over 1.5 h (4 x 207 mg, 3.9 mmol). The resulting mixture was stirred at room temperature for 24 h, filtered through diatomaceous earth and rinsed with CH₂Cl₂ (50 mL). The filtrate was washed with sat. aq. NaHCO₃ (1 x 25 mL), dried (Na₂SO₄) and concentrated under reduced pressure to yield the crude product as a light yellow oil. The crude product was purified on SiO₂ (40 g; 0-5% 2 M NH₃ in CH₃OH/CH₂Cl₂) to give a white foam (504 mg, 59% yield). MS (ESI): mass calculated for C₂₄H₂₉N₃O₃S, 439.6; m/z found, 440.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 4.44 (br s, 1 H), 3.50 (s, 2H), 2.82-2.76 (m, 2H), 2.16-2.06 (m, 2H), 1.96-1.88 (m, 2H), 1.45 (s, 9H), 1.48-1.38 (m, 2H).
B. 1-[4-(Benzothiazol-2-yloxy)-benzyl]-piperidin-4-ylamine. To a solution of {1-[4-(benzothiazol-2-yloxy)-benzyl]-piperidin-4-yl}-carbamic acid tert-butyl ester (200 mg, 0.45 mmol) in CH₂Cl₂ (2 mL) at 0 °C was added 4 N HCl in dioxane (1.8 mL, 7.2 mmol) dropwise. The resulting reaction mixture was stirred at room temperature for 2 h. The desired product was isolated by filtration and was rinsed with Et₂O (50 mL) to yield a white powder (187 mg, 100% yield). MS (ESI): mass calculated for C₁₉H₂₁N₃OS, 339.5; m/z found, 340.4 [M+H]⁺. ¹H NMR (400 MHz, CDCl₃): 7.68-7.64 (m, 1H), 7.58-7.52 (m, 2H), 7.48-7.44 (m, 1H), 7.40-7.35 (m, 2H), 7.30-7.24 (m, 1H), 7.20-7.14 (m, 1H), 4.25 (s, 2H), 3.52-3.46 (m, 2H), 3.36-3.28 (m, 1H), 3.08-2.99 (m, 2H), 2.16-2.08 (m, 2H), 1.92-1.80 (m, 2H).
C. Acetic acid {1-[4-(benzothiazol-2-yloxy)-benzyl]-piperidin-4-ylcarbamoyl}-methyl ester. To a solution of {1-[4-(benzothiazol-2-yloxy)-benzyl]-piperidin-4-ylamine dihydrochloride (413 mg, 1.0 mmol) in CH₂Cl₂ (20 mL) at room temperature was added TEA (0.70 mL, 5.0 mmol), followed by acetoxyacetyl chloride (0.16 mL, 1.5 mmol). The resulting mixture was stirred at room temperature overnight. The reaction mixture was dissolved in CH₂Cl₂ (100 mL), washed with sat. aq. NaHCO₃ (1 x 25 mL), dried (Na₂SO₄) and concentrated under reduced pressure to yield the crude product as an off-white solid. The crude product was purified on SiO₂ (40 g; 0-10% CH₃OH/CH₂Cl₂) to give a white solid (410 mg, 93% yield). MS (ESI): mass calculated for C₂₃H₂₅N₃O₄S, 439.2; m/z found, 440.4 [M+H]⁺. ¹H NMR (400 MHz, CDCl₃): 7.72 (d, *J* = 7.8, 1 H), 7.65 (d, *J* = 8.1, 1 H), 7.40-7.35 (m, 3H), 7.32-7.23 (m, 3H), 6.11 (d, *J* = 8.3, 1 H), 4.53, (s, 2H), 3.93-3.82 (m, 1 H), 3.50(s, 2H), 2.83 (d, *J* = 11.9, 2H), 2.15 (s, 3H), 2.14 (t, *J* = 11.9, 2H), 1.93 (d, *J* = 12.1, 2H), 1.56-1.45 (m, 2H).
D. N-{1-[4-(Benzothiazol-2-yloxy)-benzyl]-piperidin-4-yl}-2-hydroxy-acetamide. To a solution of acetic acid {1-[4-(benzothiazol-2-yloxy)-benzyl]-piperidin-4-ylcarbamoyl}-methyl ester (368 mg, 0.84 mmol) in THF (30 mL), CH₃OH (10 mL) and H₂O (10 mL) was added lithium hydroxide (80.2 mg, 3.34 mmol). The resulting mixture was stirred at room temperature overnight. The mixture was extracted with CH₂Cl₂ (30 mL x 3). The combined organic phases were concentrated under reduced pressure to yield the crude product as an off-white solid. The crude product was purified on SiO₂ (40 g; 0-10% CH₃₀H/CH₂Cl₂) to give a white solid (297 mg, 82% yield). MS (ESI): mass calculated for C₂₁H₂₃N₃O₃S, 397.2; m/z found, 398.3 [M+H]⁺. ¹H NMR (400 MHz, CDCl₃): 7.72 (d, *J* = 7.8, 1 H), 7.65 (d, *J* = 8.1,1H), 7.40-7.35 (m, 3H), 7.31-7.23 (m, 3H), 6.83 (d, *J* = 8.1, 1 H), 5.33 (br s, 1 H), 3.99 (s, 2H), 3.87-3.75 (m, 1 H), 3.50 (s, 2H), 2.85 (d, *J* = 11.4, 2H), 2.14 (t, *J* = 10.9, 2H), 1.92 (d, *J* = 12.6, 2H), 1.56-1.43 (m, 2H).

### EXAMPLE 254

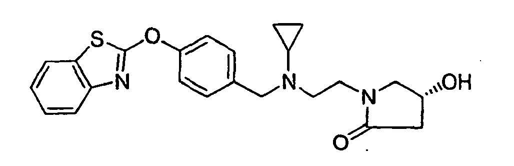

1-(2-{[4-(Benzothiazol-2-yloxy)-benzyl]-cyclopropyl-amino}-ethyl)-4-hydroxy-pyrrolidin-2-one.
A. [4-(Benzothiazol-2-yloxy)-benzyl]-cyclopropyl-amine. A mixture of 4-(benzothiazol-2-yloxy)-benzaldehyde (5.0 g, 19.6 mmol), cyclopropylamine (3.35 g, 58.7 mmol) in ClCH₂CH₂Cl (80 mL) was stirred at room temperature for 40 min. To the resulting reaction mixture was added NaBH(OAC)₃ portion wise over 1.5 h (4 x 2.1 g, 39.2 mmol). The resulting mixture was stirred at room temperature for 24 h, filtered through diatomaceous earth and rinsed with CH₂Cl₂ (500 mL). The filtrate was washed with sat. aq. NaHCO₃ (1 x 250 mL), dried (Na₂SO₄) and concentrated under reduced pressure to yield the crude product as a pale yellow oil. The crude product was purified on SiO₂ (330 g; 0-5% CH₃OH/CH₂Cl₂) to give a white solid (3.95 g, 68% yield). MS (ESI): mass calculated for C₁₇H₁₆N₂OS, 296.1; m/z found, 297.3 [M+H]⁺. ¹H NMR (400 MHz, CDCl₃): 7.72 (d, *J* = 8.1, 1H), 7.63 (d, *J* = 8.1, 1 H), 7.40-7.33 (m, 3H), 7.32-7.27 (m, 2H), 7.24 (t, *J* = 8.1, 1 H), 3.85 (s, 2H), 2.19-2.12 (m, 1 H), 1.86 (br s, 1 H), 0.48-0.35 (m, 4H).
B. (2-{[4-(Benzothiazol-2-yloxx)-benzyl-cyclopropyl-amino}-ethyl)-carbamic acid tert-butyl ester. To a solution of [4-(benzothiazol-2-yloxy)-benzyl]-cyclopropyl-amine (2.96 g, 10 mmol) in CH₃CN (40 mL) at room temperature was added *N,N*-diisopropylethylamine (3.48 mL, 20 mmol), followed by (2-bromo-ethyl)-carbamic acid tert-butyl ester (3.36 g, 15 mmol). The resulting mixture was heated at 60 °C overnight. The mixture was cooled and dissolved in CH₂Cl₂ (200 mL), washed with sat. aq. NaHCO₃ (1 x 25 mL) and H₂O (2 x 25 mL), dried (Na₂SO₄) and concentrated under reduced pressure to yield the crude product as a pale yellow solid. The crude product was purified on SiO₂ (120 g; 0-50% ethyl acetate/hexanes) to give a white solid (3.44 g, 78% yield). MS (ESI): mass calculated for C₂₄H₂₉N₃O₃S, 439.2; m/z found, 440.4 [M+H]⁺. ¹H NMR (400 MHz, CDCl₃): 7.73 (d, *J* = 8.1, 1 H), 7.64 (d, *J* = 8.1, 1 H), 7.39-7.22 (m, 6H), 4.75 (br s, 1 H), 3.74 (s, 2H), 3.25 (dd, *J* = 5.8, 6.1, 2H), 2.65 (t, *J* = 6.3, 2H), 1.82-1.75 (m, 1 H), 1.43 (s, 9H), 0.54-0.48 (m, 2H), 0.43-0.37 (m, 2H).
C. N1-[4-(Benzothiazol-2-yloxy)-benzyl]-N1-cyclopropyl-ethane-1,2-diamine. To a solution of (2-{[4-(benzothiazol-2-yloxy)-benzyl]-cyclopropyl-amino}-ethyl)-carbamic acid tert-butyl ester in CH₂Cl₂ (16 mL) at 0 °C was added trifluoroacetic acid in dioxane (4 mL) dropwise. The resulting reaction mixture was stirred at room temperature for 2 h and concentrated under reduced pressure to yield the crude product as a pale yellow oil. The oil was dissolved in CH₂Cl₂ (100 mL), washed with sat. aq. NaHCO₃ (1 x 25 mL), dried (Na₂SO₄) and concentrated under reduced pressure to yield the product as a clear oil. MS (ESI): mass calculated for C₁₉H₂₁N₃OS, 339.1; m/z found, 340.4 [M+H]⁺. ¹H NMR (400 MHz, CDCl₃): 7.71 (d, *J* = 8.1, 1 H), 7.61 (d, *J* = 8.1, 1 H), 7.37-7.18 (m, 6H), 3.75 (s, 2H), 2.76 (t, *J* = 6.3, 2H), 2.60 (t, *J* = 6.3, 2H), 1.98 (s, 2H), 1.79-1.73 (m, 1H), 0.51-0.45 (m, 2H), 0.42-0.37 (m, 2H).
D. 1-(2-{[4-(Benzothiazol-2-yloxy)-benzyl]-cyclopropyl-amino}-ethyl)-4-hydroxy-pyrrolidin-2-one. To a solution of N1-[4-(benzothiazol-2-yloxy)-benzyl]-N1-cyclopropyl-ethane-1,2-diamine (1.5 g, 4.4 mmol) in CH₃CN (18 mL) at room temperature was added *N,N*-diisopropylethylamine (1.15 mL, 6.63 mmol), followed by 4-(R)-bromo-3-hydroxy-butyric acid ethyl ester (1.11 g, 5.3 mmol). The resulting mixture was heated at 60 °C overnight. The mixture was cooled and dissolved in CH₂Cl₂ (100 mL), washed with sat. aq. NaHCO₃ (1 x 10 mL) and H₂O (2 x 10 mL), dried (Na₂SO₄) and concentrated under reduced pressure to yield the crude product as a light brown oil. The crude product was purified on reverse phase HPLC (0-99%, 0.05 % TFA in H₂O/CH₃CN) to give a clear oil (435 mg, 18.3% yield). MS (ESI): mass calculated for C₂₃H₂₅N₃O₃S, 423.2; m/z found, 424.3 [M+H]⁺. ¹H NMR (400 MHz, CD₃OD): 7.64(d, *J* = 8.1, 1 H), 7.58-7.54 (m, 2H), 7.49 (d, *J* = 8.1, 1 H), 7.37-7.33 (m, 2H), 7.26 (t, *J* = 7.6, 1 H), 7.16 (t, *J* = 7.6, 1 H), 4.48 (dd, *J* = 8.8, 12.9, 2H), 4.32 (t, *J* = 5.8, 1 H), 3.84-3.75 (m, 1 H), 3.64-3.56 (m, 2H), 3.38 (t, *J* = 6.3, 2H), 3.24(t, *J* = 10.9, 1 H), 2.73-2.66 (m, 1 H), 2.59 (dd, *J* = 6.3, 11.1, 1H), 2.15 (d, *J* = 17.2, 1H), 0.79 (d, *J* = 6.6, 4H).

### EXAMPLE 255

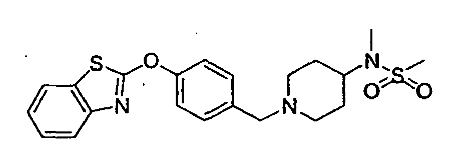

N-{1-[4-(Benzothiazol-2-yloxy)-benzyl]-piperidin-4-yl}-N-methyl-methanesulfonamide.
A. {1-[4-(Benzothiazol-2-yloxy)-benzyl]-piperidin-4-yl}-methyl-carbamic acid tert-butyl ester. A mixture of 4-(benzothiazol-2-yloxy)-benzaldehyde (4.4 g, 17.2 mmol), methyl-piperidin-4-yl-carbamic acid tert-butyl ester (4.06 g, 18.9 mmol) in ClCH₂CH₂Cl (172 mL) was stirred at room temperature for 40 min. To the resulting reaction mixture was added NaBH(OAc)₃ portion wise over 1.5 h (4 x 1.82 g, 34.4 mmol). The resulting mixture was stirred at room temperature for 24 h, filtered through diatomaceous earth and rinsed with CH₂Cl₂ (300 mL). The filtrate was washed with sat. aq. NaHCO₃ (1 x 50 mL), dried (Na₂SO₄) and concentrated under reduced pressure to yield the crude product as a pale yellow oil. The crude product was purified on SiO₂ (330 g; 0-100% ethyl acetate/hexanes) to give a light yellow foam (3.75 g, 48% yield). MS (ESI): mass calculated for C₂₅H₃₁N₃O₃S, 453.2; m/z found, 454.5 [M+H]⁺. H NMR (400 MHz, CDCl₃): 7.73 (d, *J* = 8.1, 1H), 7.66 (d, *J* = 8.1, 1H), 7.41-7.35 (m, 3H), 7.33-7.23 (m, 3H), 4.13-3.94 (m, 1 H), 3.53 (s, 2H), 2.93 (d, *J* = 11.6, 2H), 2.74 (s, 3H), 2.08 (t, *J* = 11.6, 2H), 1.81-1.69 (m, 2H), 1.65-1.57 (m, 2H), 1.46 (s, 9H).
B. {1-[4-(Benzothiazol-2-yloxy)-benzyl]-piperidin-4-yl}-methyl-amine. To a solution of {1-[4-(benzothiazol-2-yloxy)-benzyl]-piperidin-4-yl}-methyl-carbamic acid tert-butyl ester (3.7 g, 8.2 mmol) in CH₂Cl₂ (41 mL) at 0 °C was added 4 N HCl in dioxane (8.2 mL, 32.6 mmol) dropwise. The resulting mixture was stirred at room temperature for 2 h. The desired product was isolated by filtration and was washed with Et₂O (150 mL) to yield a white powder (3.38 g, 97% yield). MS (ESI): mass calculated for C₂₀H₂₃N₃OS, 353.2; m/z found, 354.4 [M+H]⁺. ¹H NMR (400 MHz, CDCl₃): 8.92 (br s, 1 H), 7.72 (d, *J* = 8.1, 1H), 7.63 (d, *J* = 8.1, 1H), 7.39-7.33 (m, 3H), 7.30-7.21 (m, 3H), 3.49 (s, 2H), 2.98-2.86 (m, 3H), 2.63 (s, 3H), 2.08-1.98 (m, 4H), 1.84-1.71 (m, 2H).
C. N-{1-[4-(Benzothiazol-2-yloxy)-benzyl]-piperidin-4-yl}-N-methyl-methanesulfonamide. To a solution of {1-[4-(benzothiazol-2-yloxy)-benzyl]-piperidin-4-yl}-methyl-amine dihydrochloride (354 mg, 1.0 mmol) in CH₂Cl₂ (20 mL) at room temperature was added TEA (0.70 mL, 5.0 mmol), followed by methanesulfonyl chloride (0.12 mL, 1.5 mmol). The resulting mixture was stirred at room temperature overnight. The mixture was dissolved in CH₂Cl₂ (100 mL), washed with sat. aq. NaHCO₃ (1 x 25 mL), dried (Na₂SO₄) and concentrated under reduced pressure to yield the crude product as a pale yellow solid. The crude product was purified on SiO₂ (40 g; 0-10% CH₃OH/CH₂Cl₂) to give a white solid (378 mg, 88% yield). MS (ESI): mass calculated for C₂₁H₂₅N₃O₃S₂, 431.1; m/z found, 432.4 [M+H]⁺. ¹H NMR (400 MHz, CDCl₃): 7.73 (d, *J* = 8.1, 1H), 7.66 (d, *J* = 8.1, 1 H), 7.41-7.35 (m, 3H), 7.33-7.23 (m, 3H), 3.80-3.70 (m, 1 H), 3.50 (s, 2H), 2.96 (d, *J* = 11.6, 2H), 2.82 (s, 3H), 2.80 (s, 3H), 2.08 (t, *J* = 11.6, 2H), 1.89-1.77 (m, 2H), 1.70-1.60 (m, 2H).

### EXAMPLE 256

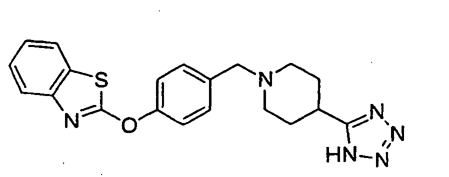

2-{4-[4-(1H-Tetrazol-5-yl)-piperidin-1-ylmethyl]-phenoxy}-benzothiazole.

A solution of 4-(benzothiazol-2-yloxy)-benzaldehyde (EXAMPLE 251 step A, 620 mg, 2.4 mmol), 4-(1*H*-tetrazol-5-yl)-piperidine hydrochloride (565 mg, 3.0 mmol), and Et₃N (0.43 mL, 3.1 mmol) in 30% THF/CH₂Cl₂ (35 mL) was stirred at room temperature for 30 min. To the stirring mixture was added Na(AcO)₃BH (787 mg, 3.7 mmol). After 20 h the reaction mixture was added to 10% *i*PrOH/H₂O (50 mL) and the organic layer was separated and dried (MgSO₄). The solvent was removed under reduced pressure to yield a clear yellow tacky oil. This material was diluted with ethanol (10 mL), heated to 80 °C, and filtered while hot. To the filtrate was added Et₂O (10 mL) and the flask was cooled on ice. A solid formed and was filtered to yield a white solid (48 mg, 5% yield). MS (ESI): mass calculated for C₂₀H₂₀N₆OS, 392.1; m/z found, 393.4 [M+H]⁺. ¹H NMR (400 MHz, DMSO-*d₆*): 7.95 (d, *J* = 7.9, 1H), 7.70 (d, *J* = 7.9, 1 H), 7.53-7.35 (m, 5H), 7.33 (t, *J* = 7.9, 1 H), 3.59 (s, 2H), 3.09-3.00 (m, 1H), 2.91 (d, *J* = 10.8, 2H), 2.22 (t, *J* = 9.8, 2H), 1.85-1.72 (m, 2H).

### EXAMPLE 257

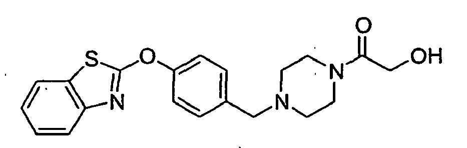

1-{4-[4-(Benzothiazol-2-yloxy)-benzyl]-piperazin-1-yl}-2-hydroxy-ethanone.
A. 4-[4-(Benzothiazol-2-yloxy)-benzyl]piperazine-1-carboxylic acid tert-butyl ester. A mixture of 4-(benzothiazol-2-yloxy)-benzaldehyde (2.5 g, 9.8 mmol), piperazine-1-carboxylic acid tert-butyl ester-2-one (3.7 g, 19.6 mmol) and molecular sieves (2.5 g, crushed, 4A) in ClCH₂CH₂Cl (25 mL) was stirred at room temperature for 40 min. To the resulting reaction mixture was added NaBH(OAc)₃ portion wise over 1.5 h (4 x 504 mg, 19.6 mmol). The resulting mixture was stirred at room temperature for 24 h, filtered through diatomaceous earth and rinsed with CH₂Cl₂ (200 mL). The filtrate was washed with sat. aq. NaHCO₃ (1 x 50 mL), dried (Na₂SO₄) and concentrated under reduced pressure to yield the crude product as a yellow semi-solid. The crude product was purified on SiO₂ (120 g; 0-100% acetone/CH₂Cl₂) to give an off-white solid (1.72 g, 42% yield). MS (ESI): mass calculated for C₂₃H₂₇N₃O₃S, 425.5; m/z found, 426.4 [M+H]⁺. ¹H NMR (400 MHz, CDCl₃): 7.74 (d, *J* = 8.2, 1H), 7.67 (d, *J* = 8.2, 1 H), 7.42-7.37 (m, 3H), 7.4-7.25 (m, 3H), 3.53 (s, 2H), 3.45 (br t, *J* = 4.9, 4H), 2.41 (br t, *J* = 4.5, 4H), 1.46 (s, 9H).
B. 2-(4-Piperazin-1-ylmethyl-phenoxy)-benzothiazole. To a solution of 4-[4-(benzothiazol-2-yloxy)-benzyl]-piperazine-1-carboxylic acid tert-butyl ester (1.7 g, 4.0 mmol) in CH₂Cl₂ (20 mL) at 0 °C was added 4 N HCl in dioxane (5 mL, 20 mmol) dropwise. The resulting mixture was stirred at room temperature for 2 h and concentrated under reduced pressure to yield the crude product as a white solid. The crude product was triturated with Et₂O (50 mL) and isolated by filtration to yield the desired product as a white powder (1.37 g, 87% yield). MS (ESI): mass calculated for C₁₈H₁₉N₃OS, 325.4; m/z found, 326.3 [M+H]⁺. ¹H NMR (400 MHz, CD₃OD): 7.81 (d, *J* = 8.0, 1 H), 7.77-7.72 (m, 2H), 7.62 (d, *J* = 8.2, 1 H), 7.57-7.53 (m, 2H), 7.45-7.40 (m, 1H), 7.35-7.31 (m, 1H), 4.45 (s, 2H), 3.62 (br s, 8H).
C. 1-{4-[4-(Benzothiazol-2-ylox)-benzyl]-piperazin-1-yl}-2-hydroxy-ethanone. To a mixture of glycolic acid (47 mg, 0.62 mmol) and HOBT (1.25 mL, 0.62 mmol, 0.5 M in DMF) in CH₂Cl₂ (25 mL) was added 2-(4-piperazin-1-ylmethyl-phenoxy)-benzothiazole (150 mg, 0.42 mmol) followed by EDCI (150 mg, 0.79 mmol). The resulting mixture was stirred at room temperature for 24 h, diluted with CH₂Cl₂ (50 mL) and washed with sat. aq. NaHCO₃ (1 x 20 mL). The organic layer was dried (Na₂SO₄) and concentrated under reduced pressure to yield the crude product as apale yellow oil. The crude product was purified on SiO₂ (40 g; 0-3% 2 M NH₃ in CH₃OH/CH₂Cl₂) to give a white foam (93 mg, 59% yield). MS (ESI): mass calculated for C₂₀H₂₁N₃O₃S, 383.5; m/z found, 384.4 [M+H]⁺. ¹H NMR (400 MHz, CDCl₃): 7.74 (d, *J* = 8.0, 1 H), 7.68 (d, *J* = 8.0, 1H), 7.45-7.25 (m, 6H), 4.16 (d, *J* = 4.1, 2H), 3.71-3.68 (m, 2H), 3.64-3.61 (m, 1 H), 3.55 (s, 2H), 3.32-3.25 (m, 2H), 2.53-2.44 (m, 4H).

### EXAMPLE 258

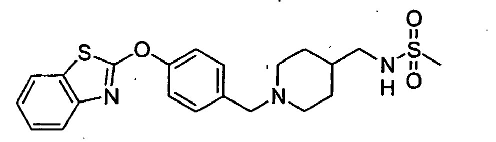

N-{1-[4-(Benzothiazol-2-yloxy)-benzyl]-piperidin-4-ylmethyl}-methanesulfonamide.
A. {1-[4-(Benzothiazol-2-yloxy)-benzyl]-piperidin-4-ylmethyl}-carbamic acid tert-butyl ester. A mixture of 4-(benzothiazol-2-yloxy)-benzaldehyde (1.0 g, 3.9 mmol), piperidin-4-ylmethyl-carbamic acid tert-butyl ester (1.3 g, 5.9 mmol) and molecular sieves (1.0 g, crushed, 4A) in ClCH₂CH₂Cl (15 mL) was stirred at room temperature for 40 min. To the resulting mixture was added NaBH(OAc)₃ portion wise over 1.5 h (4 x 412 mg, 7.8 mmol). The mixture was stirred at room temperature for 24 h, filtered through diatomaceous earth and rinsed with CH₂Cl₂ (100 mL). The filtrate was washed with sat. aq. NaHCO₃ (1 x 50 mL), dried (Na₂SO₄) and concentrated under reduced pressure to yield the crude product as a yellow semi-solid. The crude product was purified on SiO₂ (40 g; 0-100% acetone/CH₂Cl₂) to give a white foam (890 mg, 50% yield). MS (ESI): mass calculated for C₂₅H₃₁N₃O₃S, 453.6; m/z found, 454.4 [M+H]⁺. ¹H NMR (400 MHz, CDCl₃): 7.74 (d, *J* = 8.0, 1 H), 7.67 (d, *J* = 8.0, 1 H), 7.41-7.36 (m, 3H), 7.32-7.24 (m, 3H), 4.59 (br s, 1 H), 3.50 (s, 2H), 3.06-3.00 (m, 2H), 2.94-2.88 (m, 2H), 1.97 (t, *J* = 11.4, 2H), 1.68 (d, *J* = 11.4, 2H), 1.44 (s, 9H), 1.22-1.33 (m, 2H).
B. C-{1-[4-(Benzothiazol-2-yloxy)-benzyl]-piperidin-4-yl}-methylamine. To a solution of {1-[4-(benzothiazol-2-yloxy)-benzyl]-piperidin-4-ylmethyl}-carbamic acid tert-butyl ester (866 mg, 1.9 mmol) in CH₂Cl₂ (5 mL) at 0 °C was added 4 N HCl in dioxane (2.4 mL, 9.5 mmol) dropwise. The resulting mixture was stirred at room temperature for 24 h and concentrated under reduced pressure to yield the crude product as a white solid. The crude product was triturated with Et₂O (50 mL) and isolated by filtration to yield the desired product as a white powder (813 mg, 100% yield). MS (ESI): mass calculated for C₂₀H₂₃N₃OS, 353.5; m/z found, 354.4 [M+H]⁺. ¹H NMR (400 MHz, CDCl₃): 7.80 (d, *J* = 8.0, 1 H), 7.69 (d, *J* = 8.6, 2H), 7.60 (d, *J* = 8.0, 1 H), 7.51 (d, *J* = 8.6, 1 H), 7.42-7.38 (m, 1 H), 7.33-7.28 (m, 1 H), 4.37 (s, 2H), 3.78 (br d, *J* = 12.7, 1 H), 2H), 3.13-3.04 (m, 2H), 2.89 (d, *J* = 6.6, 2H), 2.08-1.92 (m, 3H), 1.68-1.56 (m, 2H).
C. N-{1-[4-(Benzothiazol-2-yloxy)-benzyl]-piperidin-4-ylmethyl}-methanesulfonamide. To a mixture of C-{1-[4-(benzothiazol-2-yloxy)-benzyl]-piperidin-4-yl}-methylamine (150 mg, 0.39 mmol) in CH₂Cl₂ (5 mL) was added Et₃N (400 µL, 2.86 mmol). The resulting mixture was cooled to 0 °C and CH₃SO₂Cl was added via syringe (41 µL, 0.52 mmol). The mixture was stirred at room temperature for 24 h, diluted with CH₂Cl₂ (10 mL) and washed with sat. aq. NaHCO₃ (1 x 5 mL). The organic layer was dried (Na₂SO₄) and concentrated under reduced pressure to yield the crude product as a white solid. The crude product was purified on SiO₂ (10 g; 0-3% 2 M NH₃ in CH₃OH/CH₂Cl₂) to give a white solid (112 mg, 67% yield). MS (ESI): mass calculated for C₂₁H₂₅N₃O₃S₂, 431.6; m/z found, 432.4 [M+H]⁺. ¹H NMR (400 MHz, CDCl₃): 7.74 (d, *J* = 8.0, 1 H), 7.67 (d, *J* = 8.0, 1 H), 7.42-7.36 (m, 3H), 7.33-7.25 (m, 3H), 4.25 (br t, *J* = 6.6, 1 H), 3.51 (s, 2H), 3.04 (d, *J* = 6.6, 2H), 2.96 (s, 3H), 2.91-2.84 (m, 2H), 1.98 (t, *J* = 11.7, 2H), 1.74 (br d, *J* = 12.7, 2H), 1.60-1.48 (m, 2H), 1.36-1.24 (m, 2H).

### EXAMPLE 259

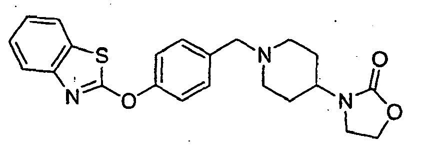

3-{1-[4-(Benzothiazol-2-yloxy)-benzyl]-piperidin-4-yl}-oxazolidin-2-one.
A. 3-Piperidin-4-yl-oxazolidin-2-one hydrochloride salt. To a solution of 1-benzyl-4-piperidinone (10.3 g, 54 mmol) and ethanolamine (13.2 mL, 218 mmol) in CH₃OH (20 mL) was added sodium cyanoborohydride (10.2 g, 163 mmol) and trifluoromethanesulfonic acid (5 mL) and the reaction was stirred at 23 °C for 3 days. The mixture was cooled to 0 °C and 12 N HCl was slowly added until gas evolution ceased and the resulting mixture was stirred for a further 3 h. The mixture was filtered and the filtrate concentrated under reduced pressure. The crude oil was redissolved in H₂O (50 mL), the solution was made basic by the addition of 10 N NaOH. The mixture was extracted with CH₂Cl₂ (8 x 70 mL). The combined CH₂Cl₂ extracts were dried and concentrated under reduced pressure to yield the crude product (12.0 g, 95%). A solution of 2-(1-benzyl-piperidin-4-ylamino)-ethanol (3.6 g, 15.3 mmol) in ClCH₂CH₂Cl (5 mL) was treated with carbonyl diimidazole (CDl) (2.6 g, 16 mmol) and the mixture stirred at 23 °C for 30 min. The mixture was diluted with CH₂Cl₂ (100 mL), washed with H₂O (1 x 50 mL) and sat. aq. NaHCO₃ (1 x 50 mL), dried, and concentrated under reduced pressure to yield 2.85 g (65%) of 3-(1-benzyl-piperidin-4-yl)-oxazolidin-2-one. To a solution of 3-(1-benzyl-piperidin-4-yl)-oxazolidin-2-one (2.3 g, 8.8 mmol) in ClCH₂CH₂Cl (40 mL) was added α-chloroethyl acetylchloride (1.5 g, 10.6 mmol) and the mixture was heated to 100 °C for 90 min. The mixture was cooled to 23 °C and concentrated under reduced pressure. The crude residue was dissolved in CH₃OH and heated to reflux for 1 h. The mixture was cooled to 0 °C and concentrated under reduced pressure to yield the title compound (1.89 g, 99%). MS (ESI): exact mass calculated for C₈H₁₄N₂O₂, 170.1; m/z found, 171.2 [M+H]⁺. ¹H NMR (400 MHz, DMSO-*d₆*): 8.97 (br s, 2H), 4.27 (dd, *J* = 9.1, 7.8, 2H), 3.80 (tt, *J* = 11.8, 4.2, 1 H), 3.49 (dd, *J* = 8.0, 6.6, 1 H), 3.30 (br d, *J* = 12.7, 2H), 2.97 (dt, *J* = 12.6, 2.3, 2H), 1.90 (ddd, *J* = 16.6, 13.0, 4.1, 2H), 1.86-1.75 (m, 2H).
B. [4-(Benzothiazol-2-yloxy)-phenyl]-methanol. To a mixture of 4-hydroxybenzyl alcohol (12 g, 97 mmol) in CH₃CN (200 mL) containing K₂CO₃ (22 g, 159 mmol) was added 2-chlorobenzothiazole (22 g, 130 mmol) and the mixture was heated to reflux for 72 h. The mixture was cooled to room temperature, filtered, and concentrated under reduced pressure to give the crude product as a golden oil. The crude oil was purified on SiO₂ (300 g; 5% acetone/CH₂Cl₂) to give a clear and colorless oil. (15 g, 60% yield). MS (ESI): exact mass calculated for C₁₄H₁₁NO₂S, 257.1; m/z found, 258.3 [M+H]⁺. ¹H NMR (400 MHz, DMSO-*d₆*): 7.92 (d, *J* = 7.4, 1 H), 7.69 (d, *J* = 8.0, 1 H), 7.50-7.31 (m, 5H), 7.32 (t, *J* = 7.5, 1H), 5.32 (t, *J* = 5.7, 1 H), 4.55 (d, *J* = 5.7, 2H).
C. 2-(4-Chloromethyl-phenoxy)-benzothiazole. To a mixture of [4-(benzothiazol-2-yloxy)-phenyl]-methanol (11 g, 43 mmol) in CH₂Cl₂ (100 mL) containing triethytlamine (9 mL, 65 mmol) at 5°C was added dropwise over 15 minutes thionyl chloride (4 mL g, 55 mmol). The ice bath was removed and the mixture warmed to room temperature and stirred for 24 hr. The mixture was washed once with saturated K₂ CO₃ (100 mL), dried (MgSO₄), and concentrated under reduced pressure to give a black oil. The crude oil was purified on SiO₂ (300 g; 100% CH₂Cl₂) to give a clear orange oil. (10 g, 84% yield). MS (ESI): exact mass calculated for C₁₄H₁₀ClNOS, 275.0; m/z found, 276.2 [M+H]⁺. ¹H NMR (400 MHz, DMSO-*d₆*): 7.95 (d, *J* = 7.3, 1 H), 7.70 (d, *J* = 7.6, 1 H), 7.59 (d, *J* = 8.6, 2H), 7.47 (d, *J* = 8.6, 2H), 7.37 (t, *J* = 7.4, 1 H), 7.33 (t, *J* = 7.5, 1H), 4.84 (s, 2H).
D. 3-{1-[4-(Benzothiazol-2-yloxy)-benzyl]-piperidin-4-yl}-oxazolidin-2-one. To a mixture of 2-(4-chloromethyl-phenoxy)-benzothiazole (670 mg, 2.4 mmol) in CH₃CN (15 mL) containing K₂CO₃ (544 mg, 3.9 mmol) was added 3-piperidin-4-yl-oxazolidin-2-one hydrochloride salt (355 mg, 1.7 mmol) and the mixture was heated to 60 °C for 24 h. The mixture was cooled to room temperature, filtered, and concentrated under reduced pressure to give the crude product as a golden oil. The crude oil was purified on SiO₂ (12 g; acetone) to give a white solid (591 mg, 84% yield). MS (ESI): mass calculated for C₂₂H₂₃N₃O₃S, 409.2; m/z found, 410.3 [M+H]⁺. ¹H NMR (400 MHz, DMSO-*d*₆): 7.94 (d, *J* = 8.7, 1H), 7.70 (d, *J* = 7.5, 1 H), 7.48-7.37 (m, 5H), 7.33 (d, *J* = 8.0, 1 H), 4.25 (t, *J* = 7.7, 2H), 3.60-3.42 (m, 5H), 2.85 (d, *J* = 11.5, 2H), 2.04 (t, *J* =11.4, 2H), 1.78-1.58 (m, 4H).

### EXAMPLE 260

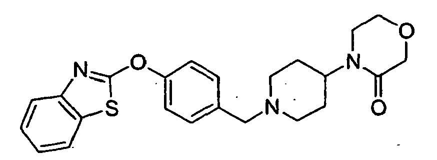

4-{1-[4-(Benzothiazol-2-yloxy)-benzyl]-piperidin-4-yl}-morpholin-3-one.
A. 4-Piperidin-4-yl-morpholin-3-one. To a solution of 1-benzyl-4-piperidinone (10.3 g, 54 mmol) and ethanolamine (13.2 mL, 218 mmol) in CH₃OH (20 mL) was added sodium cyanoborohydride (10.2 g, 163 mmol) and trifluoromethanesulfonic acid (5 mL) and the reaction mixture was stirred at 23 °C for 3 days. The mixture was cooled to 0 °C and 12 N HCl was slowly added until gas evolution ceased and the resulting mixture was stirred for a further 3 h. The mixture was filtered and the filtrate concentrated. The crude oil was redissolved in H₂O (50 mL), and the solution was made basic by the addition of 10 N NaOH. The mixture was extracted with CH₂Cl₂ (8 x 70 mL). The combined CH₂Cl₂ extracts were dried and concentrated under reduced pressure to yield 12.0 g (95% yield) crude product. A 0 °C solution of 2-(1-benzyl-piperidin-4-ylamino)-ethanol (2.13 g, 9.1 mmol) in ethanol (11 mL) and H₂O (5 mL) was treated simultaneously with chloroacetyl chloride (1.8 mL, 22.7 mmol) and 35% aq. NaOH solution and the mixture was stirred below 20 °C for 3 h. The reaction was diluted with H₂O (20 mL) and extracted with ethyl acetate (2 x 50 mL). The combined organic layers were washed with brine, dried, and concentrated to yield 1.83 g (66% yield) of 4-(1-benzyl-piperidin-4-yl)-morpholin-3-one. To a solution of 4-(1-benzyl-piperidin-4-yl)-morpholin-3-one (1.1 g, 3.9 mmol) in ClCH₂CH₂Cl (20 mL) was added α-chloroethyl acetylchloride (660 mg, 4.6 mmol) and the reaction mixture was heated to 100 °C for 16 h. The mixture was cooled to 23 °C and concentrated under reduced pressure. The resulting crude material was purified on SiO₂ (12 g, 0-10% 2 M ammonia in CH₃OH/CH₂Cl₂) to yield 282 mg (53% yield) of 4-piperidin-4-yl-morpholin-3-one. MS (ESI): exact mass calculated for C₈H₁₄N₂O₂, 184.1; m/z found, 185.4 [M+H]⁺. ¹H NMR (400 MHz, CDCl₃): 3.72 (s, 2H), 2.94 (t, *J* = 6.6, 2H), 2.55 (t, *J* = 3.6, 2H), 2.46 (tt, *J* = 10.3, 3.7, 1 H), 1.92 (dd, *J* = 12.3, 2.3, 2H), 1.82-1.70 (m, 2H), 1.70-1.60 (m, 1 H), 1.40-1.02 (m, 4H).
B. 4-{1-[4-(Benzothiazol-2-yloxy)-benzyl]-piperidin-4-yl}-morpholin-3-one. The title compound was prepared according to EXAMPLE 259, step D. MS (ESI): exact mass calculated for C₂₄H₂₇N₃O₃S₁, 423.16; m/z found, 424.3 [M+H]⁺. ¹H NMR (400 MHz, CDCl₃): 7.77 (dd, *J* = 8.1, 0.6, 1H), 7.70 (dd, *J* = 7.9, 0.7, 1H), 7.45-7.40 (m, 2H), 7.36-7.28 (m, 4H), 4.57 (tt, *J* = 12.1, 4.2, 1 H), 4.22 (s, 2H), 3.90 (t, *J* = 4.9, 2H), 3.56 (s, 2H), 3.34 (t, J = 5.1, 2H), 3.01 (br d, *J* = 11.6, 2H), 2.18 (ddd, *J* = 11.7, 11.7, 2.2, 2H), 1.79 (dddd, *J* = 12.1, 12.1, 12.0, 3.8, 2H), 1.72-1.65 (m, 2H).

### EXAMPLE 261

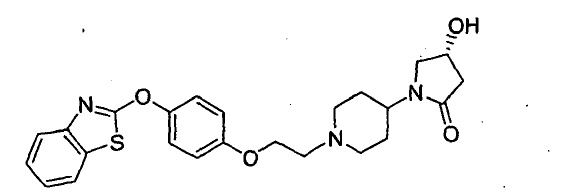

(*R*) 1-(1-{2-[4-(Benzothiazol-2-yloxy)-phenoxy]-ethyl}-piperidin-4-yl)-4-hydroxy-pyrrolidin-2-one.
A. (R)-4-Hydroxy-1-piperidin-4-yl-pyrrolidin-2-one acetic acid salt. A solution of 4-amino-1-benzylpiperidine (1.0 g, 5.2 mmol) and *N,N*-diisopropylethylamine (2.3 mL, 13.1 mmol) in CH₃CN (12 mL) was treated with (*R*)-4-bromo-3-hydroxybutyrate (1.4 g, 6.8 mmol) and the mixture was heated at 65 °C for 48 h. The mixture was cooled and diluted with ethyl acetate (70 mL) and washed with H₂O (20 mL). The organic layer was dried and concentrated under reduced pressure and the crude residue purified on SiO₂ (12 g, 0-5% 2 M ammonia in CH₃OH/CH₂Cl₂). The product was dissolved in ethanol (20 mL) and heated to 80 °C for 48 h. The mixture was concentrated under reduced pressure and the crude residue purified on SiO₂ (12 g, 0-5% 2 M ammonia in CH₃OH/CH₂Cl₂) to give 346 mg (25% yield) of 1-(1-benzyl-piperidin-4-yl)-4-hydroxy-pyrrolidin-2-one. A solution of 1-(1-benzyl-piperidin-4-yl)-4-hydroxy-pyrrolidin-2-one (346 mg, 1.3 mmol) in ethanol (7 mL) was treated with Pd(OH)₂ (60 mg) and the mixture charged with hydrogen gas on a Parr apparatus to 50 psi and shaken for 5 days. The mixture was filtered through diatomaceous earth and concentrated to give 280 mg (91 % yield) of the title compound, which was used without purification in subsequent reactions. MS (ESI): exact mass calculated for C₉H₁₆N₂O₂, 184.1; m/z found, 185.2 [M+H]⁺.
B. (*R*) 1-1-{2-[4-(Benzothiazol-2-yloxy)-phenoxy]-ethyl}-piperidin-4-yl)-4-hydroxy-pyrrolidin-2-one. The title compound was prepared according to EXAMPLE 24 using 4-hydroxy-1-piperidin-4-yl-pyrrolidin-2-one acetic acid salt. MS (ESI): exact mass calculated for C₂₄H₂₇N₃O₄S₁, 453.2; m/z found, 454.5 [M+H]⁺. ¹H NMR (400 MHz, CDCl₃): 7.77 (dd, *J* = 8.1, 0.5, 1H), 7.69 (dd, *J* = 8.0, 0.7, 1H), 7.42 (dt, *J* = 8.5, 1.3, 1H), 7.34-7.27 (m, 5H), 7.02-6.97 (m, 2H), 4.58-4.53 (m, 1H), 4.18-4.04 (m, 3H), 3.62 (dd, *J* = 10.7, 5.6, 1H), 3.34 (dd, *J* = 10.7, 2.2, 1 H), 3.15-3.08 (m, 1 H), 2.87 (t, *J* = 5.7, 2H), 2.75 (dd, *J* = 17.2, 6.6, 1 H), 2.44 (dd, *J* = 17.2, 2.6, 1 H), 2.34-2.24 (m, 2H), 1.90-1.68 (m, 3H).

### EXAMPLE 262

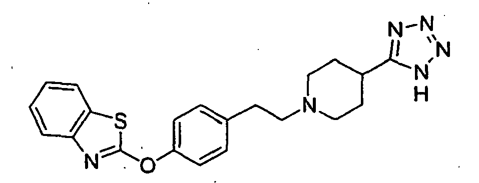

2-(4-{2-[4-(1H-Tetrazol-5-yl)-piperidin-1-yl]-ethyl}-phenoxy)-benzothiazole.
A. [4-(Benzothiazol-2-yloxy)-phenyl]-acetaldehyde. A solution of (4-hydroxyphenyl)-acetic acid methyl ester (11.2 g, 62 mmol) and 2-chlorobenzothiazole (9.5 g mL, 56 mmol) in CH₃CN was treated with finely powdered Cs₂CO₃ (27 g, 84 mmol), and the resulting mixture was stirred at 40 °C for 17 h and 60 °C for 2 h. The reaction mixture was filtered through diatomaceous earth, and the filtrate was concentrated under reduced pressure. The crude solid was purified by dissolving in ethyl acetate (350 mL) and washing with 10% NaOH (3 x 30 mL), 0.5 M citric acid (1 x 30 mL), sat. aq. NaHCO₃ (1 x 30 mL), brine (1 x 30 mL), then dried over Na₂SO₄, filtered and concentrated under reduced pressure to provide 16 g (95% yield) of [4-(benzothiazol-2-yloxy)-phenyl]-acetic acid methyl ester as a white solid. A solution of [4-(benzothiazol-2-yloxy)-phenyl]-acetic acid methyl ester (5.4 g, 18 mmol) in 80 mL of toluene at -90 °C was treated by dropwise addition of a 1.0 M solution of diisobutyl aluminum hydride in hexanes (27 mL, 27 mmol). The reaction was slowly warmed to -68 °C over 30 min and then quenched by the addition of methanol (2.0 mL). The reaction mixture was warmed to -20 °C and diluted with diethyl ether (100 mL) and 2.0 M HCl (60 mL) and stirred vigorously for 30 min. The organic layer was separated, washed with sat. aq. NaHCO₃, dried over Na₂SO₄, filtered and concentrated to yield the title compound (4.84 g, 99% yield).
B. 2-(4-{2-[4-(1H-Tetrazol-5-yl)-piperidin-1-yl]-ethyl}-phenoxy)-benzothiazole. A solution of [4-(benzothiazol-2-yloxy)-phenyl]-acetaldehyde (628 mg, 2.3 mmol) and 4-(1 H-tetrazol-5-yl)-piperidine (443 mg, 2.34 mmol) in CH₂Cl₂ (10 mL) containing triethylamine (360 µL, 2.6 mmol) was treated with sodium triacetoxyborohydride (599 mg, 2.7 mmol) and the mixture was stirred at room temperature for 24 h. The mixture was washed with sat. aq. NaHCO₃ (15 mL), dried (MgSO₄), and concentrated under reduced pressure to give a white solid. The solid was washed with diethyl ether and air dried to give the product as a white solid (214 mg, 23% yield). MS (ESI): mass calculated for C₂₁H₂₂N₆OS, 406.2; m/z found, 407.3 [M+H]⁺. ¹H NMR (400 MHz, DMSO-*d*₆): 7.93 (d, *J* = 7.9, 1 H), 7.69 (d, *J* = 7.7, 1H), 7.48-7.30 (m, 6H), 3.10 (d, *J* = 11.6, 2H), 3.08-2.97 (m, 1 H), 2.87 (t, *J* = 6.7, 2H), 2.72 (t, *J* = 8.6, 2H), 2.34 (t, *J* = 11.0, 2H), 2.01 (d, *J* = 11.2, 2H), 1.79 (q, *J* = 9.9, 2H).

### EXAMPLE 263

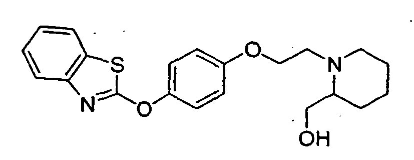

(1-{2-[4-(Benzothiazol-2-yloxy)-phenoxy]-ethyl}-piperidin-2-yl)-methanol.

The title compound was prepared according to the procedure for EXAMPLE 24 using piperidin-2-yl-methanol. MS (ESI): mass calculated for C₂₁H₂₄N₂O₃S, 384.2; m/z found, 385.3 [M+H]⁺. ¹H NMR (400 MHz, DMSO-*d*₆): 7.91 (d, *J* = 7.9, 1 H), 7.68 (d, *J* = 8.0, 1 H), 7.42 (t, *J* = 7.3, 1 H), 7.37 (d, *J* = 9.0, 2H), 7.31 (t, *J* = 8.1, 1 H), 7.05 (d, *J* = 9.1, 2H), 4.43 (t, *J* = 5.3, 1 H), 4.08 (t, *J* = 6.2, 2H), 3.60-3.51 (m, 1H), 3.48-3.39 (m, 1H), 3.17-3.09 (m, 1 H), 2.94-2.84 (m, 1H), 2.80-2.70 (m, 1 H), 2.33 (t, *J* = 10.4, 2H), 1.62 (d, *J* = 9.3, 2H), 1.58-1.47 (m, 1H), 1.47-1.35 (m, 1 H), 1.35-1.20 (m, 2H).

### EXAMPLE 264

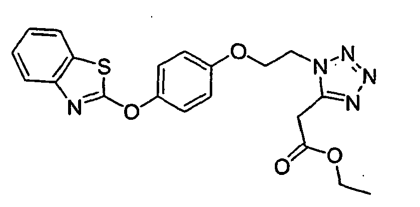

(1-{2-[4-(Benzothiazol-2-yloxy)-phenoxy]-ethyl}-1H-tetrazol-5-yl)-acetic acid ethyl ester.

The title compound was prepared according to the procedure for EXAMPLE 24 using (1 H-tetrazol-5-yl)-acetic acid ethyl ester. MS (ESI): mass calculated for C₂₀H₁₉N₅O₄S, 425.1; m/z found, 426.3 [M+H]⁺. ¹H NMR (400 MHz, DMSO-*d*₆): 7.91 (d, *J* = 8.0, 1 H), 7.67 (d, *J* = 7.6, 1 H), 7.46-7.36 (m, 3H), 7.32 (t, *J* = 8.4, 1H), 7.03 (d, *J* = 9.1, 2H), 4.88 (t, *J* = 5.0, 2H), 4.43 (t, *J* = 5.0, 2H), 4.35 (s, 2H), 4.14 (q, *J* = 7.1, 2H), 1.20 (t, *J* = 7.1, 3H).

### EXAMPLE 265

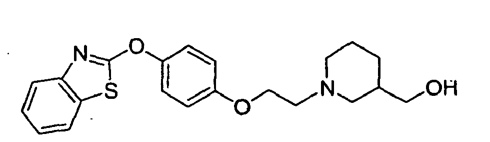

(1-{2-[4-(Benzothiazol-2-yloxy)-phenoxy]-ethyl}-piperidin-3-yl)-methanol.

The title compound was prepared according to the procedure for EXAMPLE 24 using piperidin-3-yl-methanol. MS (ESI): exact mass calculated for C₂₁H₂₄N₂O₃S₁, 384.2; m/z found, 385.1 [M+H]⁺. ¹H NMR (400 MHz, CDCl₃): 7.77 (d, *J* = 8.1, 1H), 7.68 (d, *J* = 7.9, 1H), 7.42 (dt, *J* = 8.4, 1.2, 1H), 7.34-7.26 (m, 3H), 7.02-6.97 (m, 2H), 4.15 (t, *J* = 5.9, 2H), 3.68 (dd, *J* = 10.6, 5.2, 1H), 3.56 (dd, *J* = 10.4, 6.3, 1H), 3.00 (d, *J* = 5.3, 1H), 2.84 (t, *J* = 5.9, 2H), 2.83-2.79 (m, 1H), 2.75-2.60 (m, 1H), 2.30 (t, *J* = 9.5, 1H), 2.16 (t, *J* = 9.5, 1H), 1.92-1.78 (m, 2H), 1.777-1.68 (m, 1H), 1.68-1.60 (m, 1H), 1.24-1.12 (m, 1H).

### EXAMPLE 266

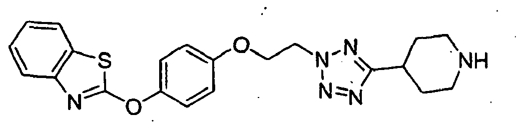

2-{4-[2-(5-Piperidin-4-yl-tetrazol-2-yl)-ethoxy]-phenoxy}-benzothiazole hydrochloride.
A. 4-(2-{2-[4-(Benzothiazol-2-yloxy)-phenoxy]-ethyl}-2H-tetrazol-5-yl)-piperidine-1-carboxylic acid tert-butyl ester. To a stirred solution of 2-[4-(2-bromo-ethoxy)-phenoxy]-benzothiazole (EXAMPLE 9; 500 mg, 1.4 mmol) and 4-(2H-tetrazol-5-yl)-piperidine-1-carboxylic acid tert-butyl ester (404 mg, 1.6 mmol) in CH₃CN (10 mL) was added Cs₂CO₃ (537 mg, 1.7 mmol). The mixture was heated at 60 °C for 20 h and then filtered. The filtrate was concentrated under reduced pressure to a clear golden oil, which was purified on SiO₂ (40 g; 0-15% acetone/CH₂Cl₂) to give a white solid (483 mg, 65% yield). TLC (SiO₂, 15% acetone/CH₂Cl₂): R_{f} = 0.84. MS (ESI): mass calculated for C₂₆H₃₀N₆O₄S, 522.2; m/z found, 523.2 [M+H]⁺. ¹H NMR (400 MHz, DMSO-*d*₆): 7.90 (d, *J* = 7.7, 1 H), 7.67 (d, *J* = 7.9, 1 H), 7.42 (t, *J* = 7.1, 1 H), 7.36 (d, *J* = 6.8, 2H), 7.31 (t, *J* = 8.1, 1 H), 7.01 (d, *J* = 6.9, 2H), 5.05 (t, *J* = 4.7, 2H), 4.57 (t, *J* = 4.8, 2H), 3.92 (d, *J* = 12.4, 2H), 3.20-3.12 (m, 1 H), 2.96 (br.s, 2H), 1.97 (dd, *J* = 13.3, 3, 2H), 1.65-1.52 (m, 2H), 1.40 (s, 9H).
B. 2-{4-[2-(5-Piperidin-4-yl-tetrazol-2-yl)-ethoxy]-phenoxy}-benzothiazole hydrochloride. To a stirred solution of 4-(2-{2-[4-(benzothiazol-2-yloxy)-phenoxy]-ethyl}-2H-tetrazol-5-yl)-piperidine-1-carboxylic acid tert-butyl ester (440 mg, 0.84 mmol) in 88% formic acid (7.5 mL) was added concentrated HCl (75 µL, 0.009 mmol). The mixture was stirred at room temperature for 20 h and concentrated under reduced pressure to give a clear, colorless oil. The oil was dried under high vacuum for 2 h and a white solid was obtained (337 mg, 99% yield). MS (ESI): mass calculated for C₂₁H₂₂N₆O₂S, 422.2; m/z found, 423.3 [M+H]⁺. ¹H NMR (400 MHz, DMSO-*d₆*): 7.92 (d, *J* = 7.9, 1H), 7.67 (d, *J* = 8.0, 1H), 7.42 (t, *J* = 7.8, 1H), 7.39 (d, *J* = 9.4, 2H), 7.32 (t, *J* = 7.4, 1H), 5.08 (t, *J* = 4.7, 2H), 4.58 (t, *J* = 4.8, 2H), 3.40-3.22 (m, 4H), 3.03 (q, *J* = 15.1, 2H), 2.16 (d, *J* = 14.1, 2H), 1.92 (q, *J* = 14.3, 2H).

### EXAMPLE 267

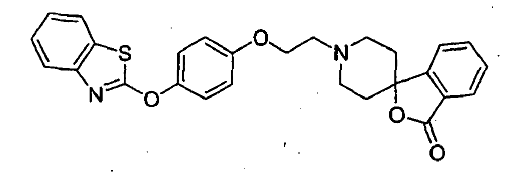

7-{2-[4-(Benzothiazol-2-yloxy)-phenoxy]-ethyl}-4-spiro-[3-phthalide]-piperidine.

The title compound was prepared according to the procedure for EXAMPLE 24 using 4-spiro-[3-phthalide]-piperidine. MS (ESI): mass calculated for C₂₇H₂₄N₂O₄S, 472.2; m/z found, 473.3 [M+H]⁺. ¹H NMR (400 MHz, DMSO-*d*₆): 7.92 (d, *J* = 7.9, 1 H), 7.83 (d, *J* = 7.7, 1H), 7.82-7.75 (m, 2H), 7.70 (d, *J* = 10.2, 1H), 7.61 (t; *J* = 11.6, 1 H), 7.48-7.38 (m, 3H), 7.32 (t, *J*= 8.2, 1H), 7.10 (d, *J* = 9.1, 2H), 4.19 (t, *J* = 5.7, 2H), 3.05 (d, *J* = 13.0, 2H), 2.86 (t, *J* = 5.6, 2H), 2.50-2.44 (m, 2H), 2.28 (t, *J* = 13.5, 2H), 1.65 (d, *J* = 12.5, 2H).

### EXAMPLE 268

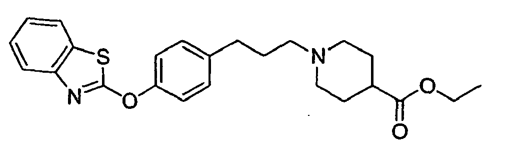

1-{3-[4-(Benzothiazol-2-yloxyrphenyl]-propyl}-piperidine-4-carboxylic acid ethyl ester.

The title compound was prepared according to the procedure for EXAMPLE 35 using piperidine-4-carboxylic acid ethyl ester for step A and 2-chlorobenzothiazole for step C. MS (ESI): mass calculated for C₂₄H₂₈N₂O₃S, 424.2; m/z found, 425.3 [M+H]⁺. ¹H NMR (400 MHz, DMSO-*d*₆): 7.92 (d, *J* = 7.9, 1H), 7.68 (d, *J* = 8.0, 1H), 7.43 (t, *J* = 8.1, 1H), 7.38-7.28 (m, 5H), 4.05 (q, *J* = 7.1, 2H), 2.78 (d, *J* = 11.3, 2H), 2.63 (t, *J* = 7.5, 2H), 2.25 (t, *J* = 7.0, 3H), 1.93 (t, *J* = 13.2, 2H), 1.84-1.68 (m, 4H), 1.54 (q, *J* = 14.9, 2H), 1.18 (t, *J* = 7.1, 3H).

### EXAMPLE 269

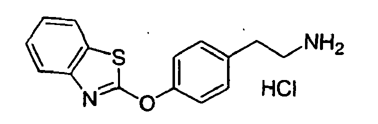

2-[4-(Benzothiazol-2-yloxy)-phenyl]-ethylamine hydrochloride.
A. [2-(4-Hydroxy-phenyl)-ethyl]-carbamic acid tert-butyl ester. To a stirring solution of di-tert-butyl dicarbonate (34.2 g, 157 mmol) in THF (200 mL) was added tyramine (21.3 g, 155 mmol) in THF (100 mL) over 1 h. The mixture was stirred for 2.5 h and concentrated under reduced pressure to give a clear golden oil which was purified on SiO₂ (300 g; 0-25% acetone/CH₂Cl₂). The desired fractions were combined and concentrated under reduced pressure to give the product as a clear pink oil (37 g, 100% yield). TLC (SiO₂, 5% acetone/CH₂Cl₂): R_{f} = 0.31. MS (ESI): mass calculated for C₁₃H₁₉NO₃, 237.1; m/z found, 260.2 [M+Na]⁺. ¹H NMR (400 MHz, DMSO-*d*₆): 9.16 (s, 1H), 6.96 (d, *J* = 8.4, 2H), 6.82 (s, 1 H), 6.66 (d, *J* = 8.4, 2H), 3.05 (q, *J* = 8.3, 2H), 2.56 (t, *J* = 8.1, 2H), 1.37 (s, 9H).
B. {2-[4-(Benzothiazol-2-yloxy)-phenyl]-ethyl}-carbamic acid tert-butyl ester. The title compound was prepared following the procedure from EXAMPLE 30, step B using [2-(4-hydroxy-phenyl)-ethyl]-carbamic acid tert-butyl ester to give a white solid (9 g, 56% yield). TLC (SiO₂, CH₂Cl₂): R_{f} = 0.19. MS (ESI): mass calculated for C₂₀H₂₂N2O₃S, 370.1; m/z found, 371.3 [M+H]⁺. ¹H NMR (400 MHz, DMSO-*d*₆): 7.92 (d, *J* = 7.9, 1 H), 7.68 (d, *J* = 8.0, 1 H), 7.48-7.30 (m, 6H), 7.34 (t, *J* = 7.8, 1 H), 3.09 (d, *J* = 7.1, 2H), 2.75 (t, *J* = 7.4, 2H), 1.38 (s, 9H).
C. 2-[4-(Benzothiazol-2-yloxy)-phenyl]-ethylamine hydrochloride. The title compound was prepared according to the procedure for EXAMPLE 266, step B to give a white solid (6.4 g, 100% yield). MS (ESI): mass calculated for C₁₅H₁₄N₂OS, 270.1; m/z found, 271.3 [M+H]⁺. ¹H NMR (400 MHz, DMSO-*d₆*): 8.05 (br s, 2H), 7.94 (d, *J* = 7.8, 1 H), 7.68 (d, *J* = 7.8, 1 H), 7.53-7.39 (m, 5H), 7.34 (t, *J* = 7.9, 1 H), 3.09 (t, *J* = 6.9, 2H), 2.95 (t, *J* = 8.6, 2H).

### EXAMPLE 270

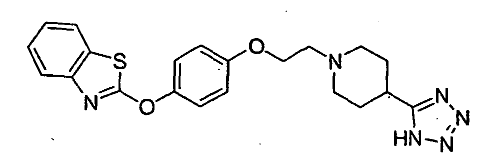

2-(4-{2-[4-(1H-Tetrazol-5-yl)-piperidin-1-yl]-ethoxy}-phenoxy)-benzothiazole.
A. [4-(Benzothiazol-2-yloxy)-phenoxy]-acetic acid methyl ester. To a stirring mixture of sodium hydride (7.5 g, 187.5 mmol) in DMSO (100 mL) was added hydroquinone (10.0 g, 91.2 mmol) over 30 minutes. The mixture was then heated to 80 °C for 2 h and cooled to room temperature. To the stirring mixture was added 2-chlorobenzothiazole (11.3 mL, 91.3 mmol) over 30 minutes and the mixture was stirred at room temperature for 24 h. To the mixture was added methyl-2-bromoacetate (8.6 mL, 90.8 mmol) over 30 minutes and the mixture stirred for 24 h. To the mixture was added H₂O (1 L) and the product extracted with Et₂O (2 x 500 mL), dried (MgSO₄), and concentrated under reduced pressure to give a beige solid. The solid was stirred in CH₂Cl₂ (200 mL) and filtered to give the product as a white solid (24.2 g, 84% yield). MS (ESI): mass calculated for C₁₆H₁₃NO₄S, 315.1; m/z found, 316.2 [M+H]⁺. ¹H NMR (400 MHz, DMSO-*d*₆): 7.92 (d, *J* = 8.0, 1 H), 7.66 (d, *J* = 8.1, 1 H), 7.47-7:40 (m, 3H), 7.32 (t, *J* = 7.5, 1H), 7.07 (d, *J* = 8.6, 2H), 4.87 (s, 2H), 3.73 (s, 3H).
B. [4-(Benzothiazol-2-yloxy)-phenoxyl-acetaldehyde. To a stirring solution of [4-(benzothiazol-2-yloxy)-phenoxy]-acetic acid methyl ester (1.0 g, 3.17 mmol) in THF (15 mL) at -78 °C was added DIBAL-H (5 mL, 5 mmol) while maintaining the temperature below -75 °C. The mixture was stirred at -72 °C for 4 h and quenched with H2O (10 mL), extracted with CH₂Cl₂ (2 x 10 mL), dried (MgS04), and concentrated under reduced pressure to give a clear golden oil (708 mg, 78% yield). MS (ESI): mass calculated for C₁₅H₁₁NO₃S, 285.1; m/z found, 286.3 [M+H]⁺.
C. 2-(4-{2-[4-(1H-Tetrazol-5-yl)-piperidin-1-yl]-ethoxy}-phenoxy)-benzothiazole. The title compound was prepared according to the procedure for EXAMPLE 262, step B using 4-(1 H-tetrazol-5-yl)-piperidine. MS (ESI): mass calculated for C₂₁H₂₂N₆O₂S, 422.2; m/z found, 422.3 [M+H]⁺. ¹H NMR (400 MHz, DMSO-*d*₆): 7.93 (d, *J* = 7.8, 1H), 7.68 (d, *J* = 7.9, 1H), 7.49-7.40 (m, 3H), 7.33 (t, *J* = 7.3, 1 H), 7.14 (d, *J* = 9.0, 2H), 4.43 (t, *J* = 4.3, 2H), 3.62-3.43 (m, 4H), 3.25-3.10 (m, 3H), 2.25 (d, *J* = 12.0, 2H), 2.18-2.02 (m, 2H).

### EXAMPLE 271

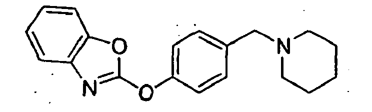

2-(4-Piperidin-1-ylmethyl-phenoxy)-benzooxazole.
A. 4-Piperidin-1-ylmethyl-phenol. A mixture of 4-hydroxybenzaldehyde (10 g, 82 mmol), piperidine (16 mL, 164 mmol), and molecular sieves (10 g, crushed, 4A) in ClCH₂CH₂Cl (150 mL) was stirred at room temperature for 40 min. To the resulting mixture was added NaBH(OAc)₃ portion wise over 1.5 h (7 x 5 g, 164 mmol). The mixture was stirred at room temperature for 24 h. The resulting mixture was diluted with CH₂Cl₂, (300 mL) filtered through diatomaceous earth and rinsed with additional CH₂Cl₂ (100 mL). The filtrate was washed with sat. aq. NaHCO₃ (3 x 150 mL), and extracted with 25% isopropanol/CHCl₃, dried (Na₂SO₄) and concentrated under reduced pressure to yield the crude product as a orange semi-solid. The crude product was purified on SiO₂ (120 g; 0-100% acetonelCH₂Cl2) to give a yellow solid (3.08 g, 48% yield). MS (ESI): mass calculated for C₁₂H₁₇NO, 191.1; m/z found, 192.4 [M+H]⁺. ¹H NMR (400 MHz, CDCl₃): 7.18 (d, *J* = 8.4, 2H), 6.75 (d, *J* = 8.4, 2H), 3.83 (s, 2H), 2.81 (br s, 4H), 1.77 (quint, *J* = 5.7, 4H), 1.54 (br s, 2H).
B. 2-(4-Piperidin-1-ylmethyl-phenoxy)-benzooxazole. The title compound was prepared according to the procedure for EXAMPLE 13, step B using 4-piperidin-1-ylmethyl-phenol. MS (ESI): mass calculated for C₁₉H₂₀N₂O₂S, 308.2; m/z found, 309.4 [M+H]⁺. ¹H NMR (400 MHz, DMSO-*d*₆): 7.64 (d, *J* = 3.1 Hz, 1 H), 7.52 (d, *J* = 8.9 Hz, 1H), 7.3 (q, *J* = 8.3 Hz, 4H), 7.34-7.26 (m, 2H), 3.45 (s, 2H), 2.33 (br.s, 4H), 1.57-1.46 (m, 4H), 1.44-1.32 (m, 2H).

### EXAMPLE 272

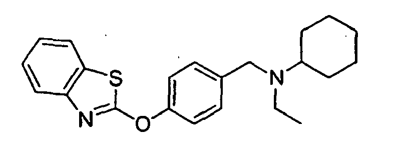

[4-(Benzothiazol-2-yloxy)-benzyl]-cyclohexyl-ethyl-amine.

The title compound was prepared according to the procedure for EXAMPLE 259, step D using cyclohexyl-ethyl-amine. MS (ESI): mass calculated for C₂₂H₂₆N₂OS, 366.2; m/z found, 367.4 [M+H]⁺. ¹H NMR (400 MHz, DMSO-*d*₆): 7.93 (d, *J* = 8.5, 1H), 7.70 (d, *J* = 8.0, 1H), 7.45 (t, *J* = 8.5, 3H), 7.37 (d, *J* = 8.6, 2H), 7.33 (t, *J* = 8.3, 1H), 3.63 (s, 2H), 2.58-2.41 (m, 3H), 1.76 (t, *J* = 11.9, 4H), 1.58 (d, *J* = 12.1, 1 H); 1.32-1.01 (m, 5H), 0.95 (t, *J* = 7.1, 3H).

### EXAMPLE 273

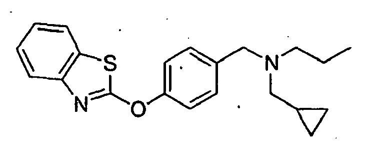

[4-(Benzothiazol-2-yloxy)-benzyl]-cyclopropylmethyl-propyl-amine.

The title compound was prepared according to the procedure for EXAMPLE 259, step D using (2-cyclopropyl-methyl)-ethyl-amine. MS (ESI): mass calculated for C₂₁H₂₄N₂OS, 352.2; m/z found, 353.3 [M+H]⁺. ¹H NMR (400 MHz, DMSO-*d*₆): 7.94 (d, *J* = 8.0, 1 H), 7.70 (d, *J* = 8.1, 1 H), 7.45 (t, *J* = 8.5, 3H), 7.39 (d, *J* = 8.6, 2H), 7.33 (t, *J* = 8.0, 1 H), 3.66 (s, 2H), 2.53-2.44 (m, 3H), 2.32 (d, *J* = = 6.5, 2H), 1.54-1.42 (m, 2H), 1.32-1.01 (m, 5H), 0.85 (d, *J* = 7.3, 4H), 0.45 (d, *J* = 9.7, 2H), 0.06 (t, *J* = 6.2, 2H).

### EXAMPLE 274

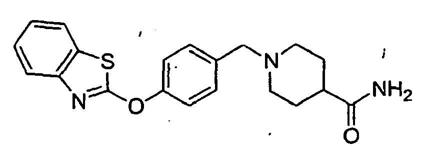

1-[4-(Benzothiazol-2-yloxy)-benzyl]-piperidine-4-carboxylic acid amide.

The title compound was prepared according to the procedure for EXAMPLE 259, step D using piperidine-4-carboxylic acid amide. MS (ESI): mass calculated for C₂₀H₂₁N₃O₂S, 367.1; m/z found, 368.3 [M+H]⁺. ¹H NMR (400 MHz, DMSO-*d₆*): 7.94 (d, *J* = 7.4, 1 H), 7.70 (d, *J* = 7.9, 1 H), 7.48-7.38 (m, 5H), 7.33 (d, *J* = 7.1, 1 H), 7.22 (s, 1 H), 6.73 (s, 1 H), 3.49 (s, 2H), 2.83 (d, *J* = 11.4, 2H), 2.14-2.02 (m, 1 H), 2.00-1.93 (m, 2H), 1.73-1.62 (m, 2H), 1.58 (t, *J* = 15.4, 2H).

### EXAMPLE 275

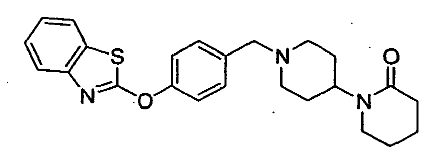

1'-[4-(Benzothiazol-2-yloxy)benzyl]-[1,4']bipiperidinyl-2-one.

The title compound was prepared according to the procedure for EXAMPLE 259, step D using [1,4']bipiperidinyl-2-one. MS (ESI): mass calculated for C₂₄H₂₇N₃O₂S, 421.2; m/z found, 422.5 [M+H]⁺. ¹H NMR (400 MHz, DMSO-*d*₆): 7.94 (d, *J* = 8.8, 1 H), 7.70 (d, *J* = 8.6, 1 H), 7.48-7.38 (m, 5H), 7.33 (d, *J* = 9.0, 1 H), 4.37-4.25 (m, 1 H), 3.52 (s, 2H), 3.16 (t, *J* = 5.2, 2H), 2.89 (d, *J* =11.2, 2H), 2.22 (t, *J* = 6.6, 2H), 2.03 (t, *J* = 11.1, 2H), 1.78-1.58 (m, 6H), 1.45 (d, *J* = 10.8, 2H).

### EXAMPLE 276

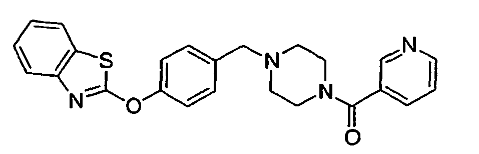

{4-[4-(Benzothiazol-2-yloxy)-benzyl]-piperazin-1-yl}-pyridin-3-yl-methanone.

The title compound was prepared according to the procedure for EXAMPLE 259, step D using piperazin-1-yl-pyridin-3-yl-methanone. MS (ESI): mass calculated for C₂₄H₂₂N₄O₂S, 430.2; m/z found, 431.4 [M+H]⁺. ¹H NMR (400 MHz, DMSO-*d₆*): 8.65 (d, *J* = 4.8, 1H), 8.61 (d, *J* = 1.9, 1H), 7.94 (d, *J* = 7.9, 1 H), 7.84 (d, *J* = 7.8, 1 H), 7.69 (d, *J* = 8.0, 1 H), 7.60-7.40 (m, 6H), 7.33 (d, *J* = 8.3, 1 H), 3.64 (br s, 2H), 3.58 (s, 2H), 3.37 (br s, 2H), 2.41 (br s, 4H).

### EXAMPLE 277

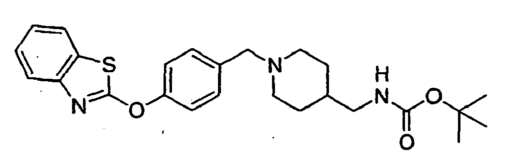

{1-[4-(Benzothiazol-2-yloxy)-benzyl]-piperidin-4-ylmethyl}-carbamic acid tert-butyl ester.

The title compound was prepared according to the procedure for EXAMPLE 259, step D using piperidin-4-ylmethyl-carbamic acid tert-butyl ester. MS (ESI): mass calculated for C₂₅H₃₁N₃O₃S, 453.2; m/z found, 454.4 [M+H]⁺. ¹H NMR (400 MHz, DMSO-*d*₆): 7.94 (d, *J* = 7.3, 1 H), 7.70 (d, *J* = 7.9, 1 H), 7.48-7.37 (m, 5H), 7.33 (d, *J* = 8.2, 1 H), 6.85 (t, *J* = 5.9, 1 H), 3.48 (s, 2H), 2.86-2.76 (m, 4H), 1.90 (t, *J* =11.1, 2H), 1.56 (d, *J* = 11.8, 2H), 1.37 (s, 10H), 1.11 (t, *J* = 9.9, 2H).

### EXAMPLE 278

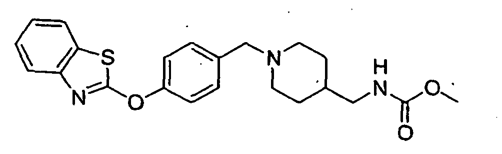

{1-[4-(Benzothiazol-2-yloxy)-benzyl]-iperidin-4-ylmethyl}-carbamic acid methyl ester.

The title compound was prepared according to the procedure for EXAMPLE 258, step C using methyl chloroformate. MS (ESI): mass calculated for C₂₂H₂₅N₃O₃S, 411.2; m/z found, 412.4 [M+H]⁺. ¹H NMR (400 MHz, DMSO-*d*₆): 7.94 (d, *J* = 7.9, 1 H), 7.70 (d, *J* = 8.0, 1 H), 7.48-7.36 (m, 5H), 7.33 (d, *J* = 8.0, 1 H), 7.17 (t, *J* = 5.6, 1H), 3.51 (s, 3H), 3.48 (s, 2H), 2.87 (t, *J* = 6.2, 2H), 2.80 (d, *J* =11.1, 2H), 1.90 (t, *J* = 10.5, 2H), 1.60 (t, *J* = 12.8, 2H), 1.38 (br s, 1 H), 1.20-1.06 (m, 2H).

### EXAMPLE 279

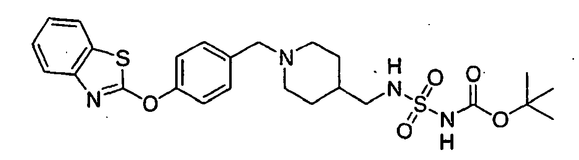

N-{C-[[4-(benzothiazol-2-yloxy)-benzyl]-piperidin-4-yl]-methylamino sulfonyl}-carbamic acid tert-butyl ester

To a stirring solution of C-{1-[4-(benzothiazol-2-yloxy)-benzyl]-piperidin-4-yl}-methylamine (EXAMPLE 258, step B, 657 mg, 1.5 mmol) in CH₂Cl₂ (15 mL) containing triethylamine (1 mL, 7.2 mmol) was added carbamic acid, N-(sulfonyl chloride) tert butyl ester (440 mg, 2.1 mmol). The mixture was stirred for 48 h and brought up in CH₂Cl₂ (50 mL) and washed once with H₂O (75 mL), dried (MgSO₄) and concentrated under reduced pressure to give a clear and colorless oil. The oil was purified on SiO₂ (12 g, 0-50% acetone/CH₂Cl₂) and the desired fractions combined and concentrated under reduced pressure to give a white solid (112 mg, 14% yield). TLC (SiO₂, 50% acetone/CH₂Cl₂): R_{f} = 0.40. MS (ESI): mass calculated for C₂₅H₃₂N₄O₅S₂, 532.2; m/z found, 533.4 [M+H]⁺. ¹H NMR (400 MHz, DMSO-*d*₆): 10.74 (br s, 1 H), 7.90 (d, *J* = 7.9, 1 H), 7.66 (d, *J* = 8.0, 1H), 7.54 (br s, 1 H), 7.44-7.34 (m, 5H), 7.30 (d, *J* = 8.3, 1 H), 3.48 (s, 2H), 2.84-2.70 (m, 4H), 1.90 (t, *J* = 10.4, 2H), 1.64 (d, *J* = 11.4, 2H), 1.38 (s, 10H), 1.16-1.02 (m, 2H).

### EXAMPLE 280

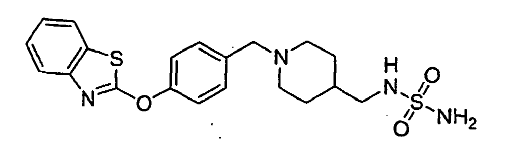

N-{1-4-(Benzothiazol-2-yloxy)-benzyl]-piperidin-4-ylmethyl}-sulfamide hydrochloride

The title compound was prepared from EXAMPLE 279 following the procedure for EXAMPLE 266, step B. MS (ESI): mass calculated for C₂₀H₂₄N₄O₃S₂, 432.1; m/z found, 433.4 [M+H]⁺. ¹H NMR (400 MHz, DMSO-*d₆*): 10.08 (br s, 1 H), 7.80 (d, *J* = 8.2, 1 H), 7.76-7.68 (m, 3H), 7.58 (d, *J* = 8.6, 2H), 7.45 (t, *J* = 8.4, 2H), 7.36 (t, *J* = 8.8, 2H), 6.70-6.50 (br s, 2H), 4.33 (d, *J* = 5.4, 2H), 3.00-2.86 (br s, 1 H), 2.77 (t, *J* = 6.2, 2H), 1.91 (d, *J* = 13.4, 2H), 1.71 (br s, 1H), 1.50-1.36 (m, 2H), 1.34-1.26 (m, 1H).

### EXAMPLE 281

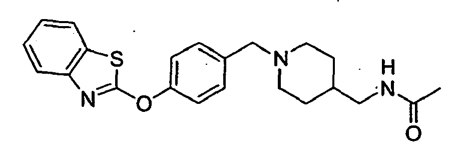

N-{1-[4-(Benzothiazol-2-yloxy)-benzyl]-piperidin-4-ylmethyl}-acetamide.

The title compound was prepared according to the procedure for EXAMPLE 258, step C using acetyl chloride. MS (ESI): mass calculated for C₂₂H₂₅N₃O₂S, 395.2; m/z found, 396.4 [M+H]⁺. ¹H NMR (400 MHz, DMSO-*d*₆): 7.94 (d, *J* = 7.8, 1 H), 7.83 (t, *J* = 5.5, 1 H), 7.70 (d, *J* = 8.0, 1 H), 7.48-7.36 (m, 5H), 7.33 (d, *J* = 8.2, 1 H), 3.48 (s, 2H), 2.93 (t, *J* = 6.2, 2H), 2.81 (d, *J* = 11.4, 2H), 1.91 (t, *J* = 11.3, 2H), 1.80 (s, 3H), 1.61 (d, *J* = 11.0, 2H), 1.38 (br s, 1H), 1.20-1.06 (m, 2H).

### EXAMPLE 282

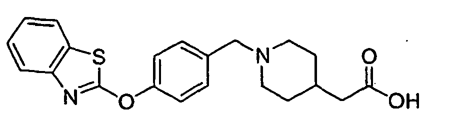

{1-[4-(Benzothiazol-2-yloxy)-benzyl]-piperidin-4-yl}-acetic acid.

The title compound was prepared according to the procedure for EXAMPLE 259, step D using piperidin-4-yl-acetic acid ethyl ester and EXAMPLE 250, step D. MS (ESI): mass calculated for C₂₁H₂₂N₂O₃S, 382.1; m/z found, 383.4 [M+H]⁺. ¹H NMR (400 MHz, DMSO-*d*₆): 10.1 (br s, 1H), 7.97 (d, *J* = 7.9, 1H), 7.70 (d, *J* = 7.6, 3H), 7.58 (br s, 2H), 7.45 (t, *J* = 7.2, 1H), 7.36 (t, *J* = 7.3, 1H), 4.31 (br s, 1H), 2.98 (br s, 3H), 2.20 (d, *J* = 5.6, 3H), 1.87 (br s, 4H), 1.51 (br s, 2H).

### EXAMPLE 283

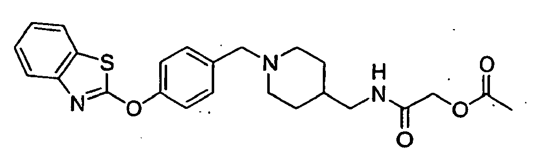

Acetic acid ({1-[4-(benzothiazol-2-yloxy)benzyl]-piperidin-4-ylmethyl} carbamoyl)-methyl ester.

The title compound was prepared according to the procedure for EXAMPLE 258, step D using acetic acid [(piperidin-4-ylmethyl)-carbamoyl]-methyl ester and EXAMPLE 253, step D. MS (ESI): mass calculated for C₂₄H₂₇N₃O₄S, 453.2; m/z found, 454.4 [M+H]⁺. ¹H NMR (400 MHz, DMSO-*d*₆): 8.01 (t, *J* = 5.8, 1H), 7.94 (d, *J* = 7.5, 1H), 7.70 (d, *J* = 8.0, 1H), 7.80-7.36 (m, 5H), 7.33 (t, *J* = 7.2, 1H), 4.43 (s, 2H), 3.48 (s, 2H), 2.98 (t, *J* = 6.3, 2H), 2.81 (d, *J* = 11.4, 2H), 2.08 (s, 3H), 1.91 (t, *J* = 11.2, 2H), 1.60 (d, *J* = 11.2, 2H), 1.42 (br s, 1H), 1.20-1.08 (m, 2H).

### EXAMPLE 284

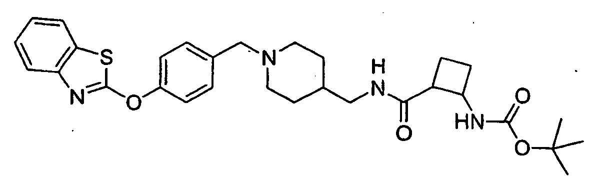

[2-({1-[4-(Benzothiazol-2-yloxy)-benzyl]-piperidin-4-ylmethyl}-carbamoyl)-cyclobutyl]-carbamic acid tert-butyl ester.

The title compound was prepared from EXAMPLE 258, step B following EXAMPLE 257, step C using 2-tert-butoxycarbonylamino-cyclobutanecarboxylic acid. MS (ESI): mass calculated for C₃₀H₃₅N₄O₄S, 550.3; m/z found, 551.5 [M+H]⁺. ¹H NMR (400 MHz, DMSO-*d*₆): 7.94 (d, *J* = 7.9, 1H), 7.70 (d, *J* = 8.0, 1H), 7.47-7.35 (m, 7H), 7.33 (t, *J* = 7.3, 1H), 3.47 (s, 2H), 2.95 (t, *J* = 6.1, 2H), 2.78 (t, *J* = 9.6, 2H), 2.45-2.35 (m, 2H), 2.13 (s, 1H), 2.05-1.95 (m, 2H), 1.93-1.70 (m, 4H), 1.59 (d, *J* = 10.9, 2H), 1.38 (s, 9H), 1.20-1.05 (m, 2H).

### EXAMPLE 285

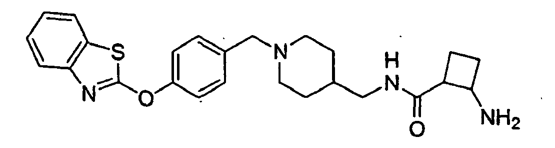

2-Amino-cyclobutanecarboxylic acid {1-[4-(benzothiazol-2-yloxy)-benzyl]-piperidin-4-ylmethyl}-amide dihydrochloride.

The title compound was prepared according to the procedure for EXAMPLE 266, step B. MS (ESI): mass calculated for C₂₅H₃₀N₄O₂S, 450.2; m/z found, 451.4 [M+H]⁺. ¹H NMR (400 MHz, DMSO-*d*₆): 8.78-8.55 (m, 4H), 7.97 (d, *J* = 8.1, 1H), 7.78 (d, *J* = 8.6, 3H), 7.71 (d, *J* = 7.8, 1H), 7.45 (t, *J* = 8.5, 1 H), 7.36 (t, *J* = 7.1, 1H), 4.32 (s, 2H), 3.20-3.05 (m, 2H), 3.00-2.85 (m, 2H), 2.472 (d, *J* = 4.7, 2H), 2.62-2.50 (m, 2H), 2.65-2.42 (m, 2H), 2.38-2.05 (m, 1H), 2.02-1.70 (m, 6H), 1.68-1.50 (s, 2H).

### EXAMPLE 286

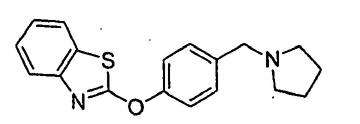

2-(4-Pyrrolidin-1-ylmethyl-phenoxy)-benzothiazole.

The title compound was prepared according to the procedure for EXAMPLE 259, step D using pyrrolidine. MS (ESI): mass calculated for C₁₈H₁₈N₂OS, 310.1; m/z found, 311.3 [M+H]⁺. ¹H NMR (400 MHz, DMSO-*d*₆): 7.94 (d, *J* = 9.0, 1H), 7.70 (d, *J* = 8.0 1H), 7.48-7.37 (m, 5H), 7.33 (t, *J* = 7.7, 1H), 3.62 (s, 2H), 2.49-2.40 (m, 4H), 1.78-1.65 (m, 4H).

### EXAMPLE 287

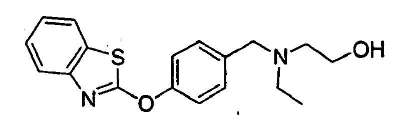

2-{[4-(Benzothiazol-2-yloxy)-benzyl]-ethyl-amino}-ethanol.

The title compound was prepared according to the procedure for EXAMPLE 259, step D using 2-ethylamino-ethanol. MS (ESI): mass calculated for C₁₈H₂₀N₂O₂S, 328.1; m/z found, 329.3 [M+H]⁺. ¹H NMR (400 MHz, DMSO-*d*₆): 7.94 (d, *J* = 8.0, 1H), 7.70 (d, *J* = 7.7, 1H), 7.50-7.36 (m, 5H), 7.33 (t, *J* = 7.7, 1H), 4.39 (t, *J* = 5.4, 1H), 3.63 (s, 2H), 3.49 (q, *J* = 6.4, 2H), 2.57-2.45 (m, 4H), 1.00 (t, *J* = 7.1, 3H).

### EXAMPLE 288

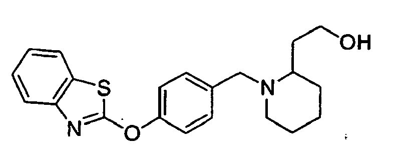

2-{1-[4-(Benzothiazol-2-yloxy)-benzyl]-piperidin-2-yl}-ethanol.

The title compound was prepared according to the procedure for EXAMPLE 259, step D using 2-piperidin-2-yl-ethanol. MS (ESI): mass calculated for C₂₁H₂₄N₂O₂S, 368.2; m/z found, 369.4 [M+H]⁺. ¹H NMR (400 MHz, DMSO-*d*₆): 7.93 (d, *J* = 8.0, 1H), 7.70 (d, *J* = 7.7, 1H), 7.48-7.36 (m, 5H), 7.33 (t, *J* = 6.9, 1 H), 4.42 (s, 1H), 3.92 (d, *J* = 14.0, 1H), 3.50 (br s, 2H), 3.31 (d, *J* = 6.4, 1H), 2.70-2.60 (m, 1H), 2.14-2.03 (m, 1H), 1.87-1.75 (m, 1 H), 1.70-1.55 (m, 3H), 1.53-1.27 (m, 5H).

### EXAMPLE 289

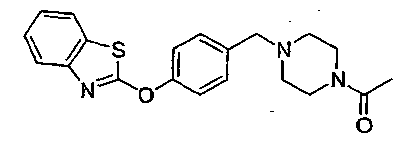

1-{4-[4-(Benzothiazol-2-yloxy)-benzyl]-piperazin-1-yl}-ethanone.

The title compound was prepared according to the procedure for EXAMPLE 259, step D using 1-piperazin-1-yl-ethanone. MS (ESI): mass calculated for C₂₀H₂₁N₃O₂S, 367.1; m/z found, 368.3 [M+H]⁺. ¹H NMR (400 MHz, DMSO-*d*₆): 7.94 (d, *J* = 8.0, 1 H), 7.70 (d, *J* = 7.5, 1 H), 7.48-7.39 (m, 5H), 7.33 (t, *J* = 7.7, 1H), 3.55 (s, 2H), 3.50-3.40 (m, 4H), 2.40 (t, *J* = 4.9, 2H), 2.33 (t, *J* = 5.0, 2H), 1.99 (s, 3H).

### EXAMPLE 290

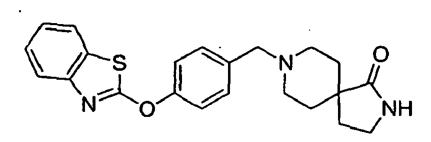

8-[4-(Benzothiazol-2-yloxy)-benzyl]-2,8-diaza-spiro[4.5]decan-1-one.

The title compound was prepared according to the procedure for EXAMPLE 259, step D using 2,8-diaza-spiro[4.5]decan-1-one. MS (ESI): mass calculated for C₂₂H₂₃N₃O₂S, 393.2; m/z found, 394.3 [M+H]⁺. ¹H NMR (400 MHz, DMSO-*d*₆): 7.94 (d, *J* = 7.4, 1 H), 7.70 (d, *J* = 8.0, 1 H), 7.54 (s, 1H), 7.48-7.36 (m, 5H), 7.33 (t, *J* = 8.2, 1 H), 3.51 (s, 2H), 3.15 (t, *J* = 6.8, 2H), 2.75(d, *J* = 11.6, 2H), 2.05 (t, *J* = 10.0, 2H), 1.91 (t, *J* = 6.8, 2H), 1.70 (t, *J* = 12.6, 2H), 1.33 (d, *J* = 12.8, 2H).

### EXAMPLE 291

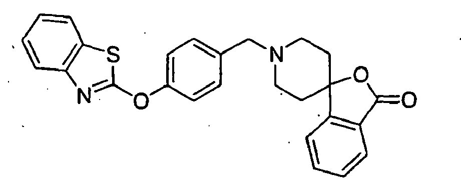

Spiro[isobenzofuran-1(3H), 4'-piperidin]-3-one, 1'-[4-(Benzothiazol-2-yloxy)-benzyl]

The title compound was prepared according to the procedure for EXAMPLE 259, step D using spiro[isobenzofuran-1(3H), 4'-piperidin]-3-one. MS (ESI): mass calculated for C₂₆H₂₂N₂O₃S, 442.1; m/z found, 443.3 [M+H]⁺. ¹H NMR (400 MHz, DMSO-*d*₆): 7.95 (d, *J* = 7.9, 1 H), 7.83 (d, *J* = 7.6, 1H), 7.82-7.76 (m, 2H), 7.70 (d, *J* = 8.0, 1 H), 7.61 (t, *J* = 7.8, 1 H), 7.56-7.48 (m, 2H), 7.47-7.40 (m, 3H), 7.33 (t, *J* = 7.4, 1 H), 3.66 (s, 2H), 2.90 (d, *J* = 11.1, 2H), 2.41 (t, *J* = 11.0, 2H), 2.28 (t, *J* = 13.3, 2H), 1.66 (d, *J* = 12.6, 2H).

### EXAMPLE 292

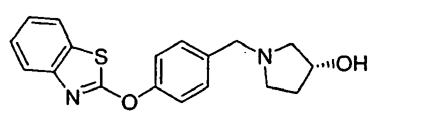

(*R*)-1-[4-(Benzothiazol-2-yloxy)-benzyl]-pyrrolidin-3-ol.

The title compound was prepared according to the procedure for EXAMPLE 259, step D using (R)-pyrrolidin-3-ol. MS (ESI): mass calculated for C₁₈H₁₈N₂O₂S, 326.1; m/z found, 327.2 [M+H]⁺. ¹H NMR (400 MHz, DMSO-*d*₆): 7.94 (d, *J* = 8.0, 1 H), 7.70 (d, *J* = 8.1, 1H), 7.48-7.36 (m, 5H), 7.33 (t, *J* = 7.2, 1H), 4.72 (d, *J* = 4.5, 1H), 4.27-4.16 (m, 1H), 3.60 (q, *J* = 13.2, 2H), 2.73-2.65 (m, 1 H), 2.60 (q, *J* = 8.1, 1H), 2.47-2.38 (m, 1H), 2.36-2.30 (m, 1 H), 2.07-1.95 (m, 1 H), 1.62-1.50 (m, 1 H).

### EXAMPLE 293

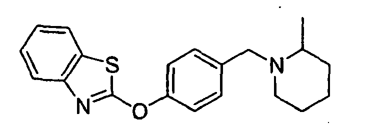

2-[4-(2-Methyl-piperidin-1-ylmethyl)-phenoxy]-benzothiazole.

The title compound was prepared according to the procedure for EXAMPLE 259, step D using 2-methyl-piperidine. MS (ESI): mass calculated for C₂₀H₂₂N₂OS, 338.2; m/z found, 339.3 [M+H]⁺. ¹H NMR (400 MHz, DMSO-*d*₆): 7.94 (d, *J* = 8.0, 1 H), 7.70 (d, *J* = 8.1, 1H), 7.30-7.33 (m, 5H), 7.33 (t, *J* = 7.9, 1 H), 3.96 (d, *J* = 13.9, 1 H), 3.19 (d, *J* = 13.9, 1 H), 2.70-2.60 (m, 1 H), 2.40-2.30 (m, 1H), 1.98 (t, *J* = 13.0, 1H), 1.68-1.56 (m, 2H), 1.54-1.34 (m, 2H), 1.32-1.24 (m, 2H), 1.11 (d, *J* = 6.2, 3H).

### EXAMPLE 294

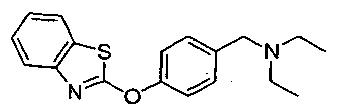

[4-(Benzothiazol-2-yloxy)-benzyl]-diethyl-amine.

The title compound was prepared according to the procedure for EXAMPLE 259; step D using diethylamine. MS (ESI): mass calculated for C₁₈H₂₀N₂OS, 312.1; m/z found, 313.3 [M+H]⁺. ¹H NMR (400 MHz, DMSO-*d*₆): 7.94 (d, *J* = 7.9, 1 H), 7.70 (d, *J* = 8.0, 1 H), 7.48-7.36 (m, 5H), 7.33 (t, *J* = 7.2, 1 H), 3.57 (s, 2H), 2.48 (q, *J* = 7.1, 4H), 1.00 (t, *J* = 7.1, 6H).

### EXAMPLE 295

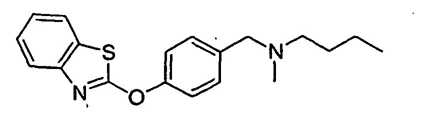

[4-(Benzothiazol-2-yloxy)-benzyl]-butyl-methyl-amine.

The title compound was prepared according to the procedure for EXAMPLE 259, step D using butyl-methyl-amine. MS (ESI): mass calculated for C₁₉H₂₂N₂OS, 326.2; m/z found, 327.3 [M+H]⁺. ¹H NMR (400 MHz, DMSO-*d*₆): 7.94 (d, *J* = 7.9, 1 H), 7.70 (d, *J* = 7.5, 1 H), 7.48-7.36 (m, 5H), 7.33 (t, *J* = 8.0, 1H), 3.49 (s, 2H), 2.34 (t, *J* = 7.1, 2H), 2.13 (s, 3H), 1.50-1.40 (m, 2H), 1.38-1.24 (m, 2H), 0.88 (t, *J* = 7.3, 3H).

### EXAMPLE 296

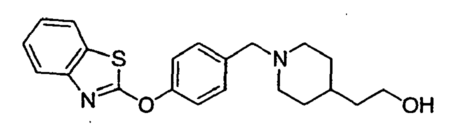

2-{1-[4-(Benzothiazol-2-yloxy)-benzyl]-piperidin-4-yl}-ethanol.

The title compound was prepared according to the procedure for EXAMPLE 259, step D using 2-piperidin-4-yl-ethanol. MS (ESI): mass calculated for C₂₁H₂₄N₂O₂S, 368.2; m/z found, 369.4 [M+H]⁺. ¹H NMR (400 MHz, DMSO-*d*₆): 7.74 (d, *J* = 7.9, 1 H), 7.49 (d, *J* = 8.0, 1 H), 7.30-7.16 (m, 5H), 7.13 (t, *J* = 7.9, 1 H), 4.13 (t, *J* = 5.1, 1 H), 3.27 (s, 2H), 3.23 (q, *J* = 6.2, 2H), 2.59 (d, *J* = 11.1, 2H), 1.72 (t, *J* = 10.3, 2H), 1.42 (d, *J* = 12.1, 2H), 1.16 (t, *J* = 3.8, 3H), 1.00-0.85 (m, 2H).

### EXAMPLE 297

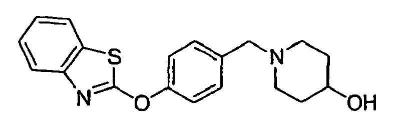

1-[4-(Benzothiazol-2-yloxy)-benzyl]-piperidin-4-ol.

The title compound was prepared according to the procedure for EXAMPLE 259, step D using piperidin-4-ol. MS (ESI): mass calculated for C₁₉H₂₀N₂O₂S, 340.1; m/z found, 341.3 [M+H]⁺. ¹H NMR (4.00 MHz, DMSO-*d*₆): 7.94 (d, *J* = 7.9, 1 H), 7.68 (d, *J* = 8.0, 1 H), 7.46-7.36 (m, 5H), 7.32 (t, *J* = 8.1, 1H), 4.60 (d, *J* = 4.9, 1 H), 3.58-3.40 (m, 3H), 2.80 (d, *J* = 9.9, 1 H), 2.65 (d, *J* = 10.7, 1 H), 1.87 (t, *J* = 9.2, 1 H), 1.80 (d, *J* = 8.6, 1 H), 1.71 (t, *J* = 9.8, 1 H), 1.62 (d, *J* = 13.3, 1H), 1.48-1.38 (m, 1 H), 1.12-1.00 (m, 1 H).

### EXAMPLE 298

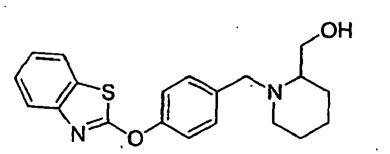

{1-[4-(Benzothiazol-2-yloxy)-benzyl]-piperidin-2-yl}-methanol.

The title compound was prepared according to the procedure for EXAMPLE 259, step D using piperidin-2-yl-methanol. MS (ESI): mass calculated for C₂₀H₂₂N₂O₂S, 354.1; m/z found, 355.3 [M+H]⁺. ¹H NMR (400 MHz, DMSO-*d*₆): 7.98 (d, *J* = 7.2, 1 H), 7.74 (d, *J* = 8.0, 1 H), 7.53-7.42 (m, 5H), 7.37 (t, *J* = 8.3, 1 H), 4.60 (t, *J* = 5.2, 1 H), 4.18 (d, *J* = 14,1, 1 H), 3.75-3.65 (m, 1 H), 3.56-3.46 (m, 1H), 3.37 (d, *J* = 3.8, 1 H), 2.72 (d, *J* = 11.9, 1 H), 2.36 (d, *J* = 4.8, 1 H), 2.07 (t, *J* = 9.7, 1 H), 1.72 (t, *J* = 13.2, 2H), 1.58-1.26 (m, 4H).

### EXAMPLE 299

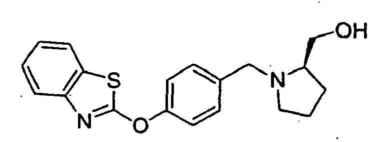

(*R*)-{1-[4-(Benzothiazol-2-yloxy)-benzyl]-pyrrolidin-2-yl}-methanol.

The title compound was prepared according to the procedure for EXAMPLE 259, step D using (*R*)-pyrrolidin-2-yl-methanol. MS (ESI): mass calculated for C₁₉H₂₀N₂O₂S, 340.1; m/z found, 340.1 [M+H]⁺. ¹H NMR (400 MHz, DMSO-*d*₆): 7.97 (d, *J* = 7.2, 1 H), 7.73 (d, *J* = 7.8, 1H), 7.50-7.38 (m, 5H), 7.36 (t, *J* = 7.9, 1 H), 4.90 (t, *J* = 5.4, 1 H), 4.14 (d, *J* = 13.4, 1 H), 3.55-3.48 (m, 1H), 3.42 (d, *J* = 13.4, 2H), 2.84 (t, *J* = 6.6, 1 H), 2.68-2.58 (m, 1 H), 2.20 (q, *J* = 8.6, 1 H), 1.95-1.83 (m, 1 H), 1.72-1.55 (m, 3H).

### EXAMPLE 300

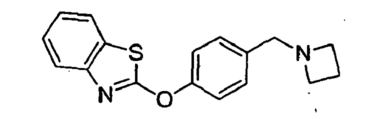

2-(4-Azetidin-1-ylmethyl-phenoxy)-benzothiazole.

The title compound was prepared according to the procedure for EXAMPLE 259, step D using azetidine. MS (ESI): mass calculated for C₁₇H₁₆N₂OS, 296.1; m/z found, 297.3 [M+H]⁺. ¹H NMR (400 MHz, DMSO-*d*₆): 7.80 (d, *J* = 8.5, 1 H), 7.56 (d, *J* = 8.0, 1 H), 7.35-7.24 (m, 5H), 7.19 (t, *J* = 7.3, 1H), 3.43 (s, 2H), 3.02 (t, *J* = 7.0, 4H), 1.92-1.80 (m, 2H).

### EXAMPLE 301

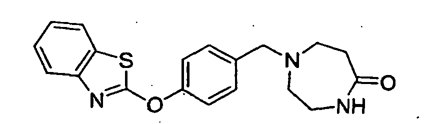

1-[4-(Benzothiazol-2-yloxy)-benzyl]-[1,4]diazepan-5-one.

The title compound was prepared according to the procedure for EXAMPLE 259, step D using [1,4]diazepan-5-one. MS (ESI): mass calculated for C₁₉H₁₉N₃O₂S, 353.1; m/z found, 354.3 [M+H]⁺. ¹H NMR (400 MHz, DMSO-*d*₆): 7.94 (d, *J* = 7.9, 1 H), 7.69 (d, *J* = 7.9, 1 H), 7.57 (t, *J* = 5.4, 1 H), 7.44-7.33 (m, 5H), 7.33 (t, *J* = 8.2, 1 H), 3.62 (s, 2H), 3.18-3.10 (m, 2H), 2.58-2.40 (m, 6H).

### EXAMPLE 302

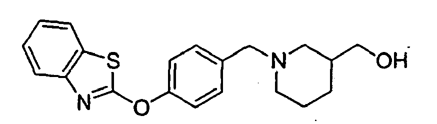

{1-[4-(Benzothiazol-2-yloxy)-benzyl]-piperidin-3-yl}-methanol.

The title compound was prepared according to the procedure for EXAMPLE 259, step D using piperidin-3-yl-methanol. MS (ESI): mass calculated for C₂₀H₂₂N₂O₂S, 354.1; m/z found, 355.3 [M+H]⁺. ¹H NMR (400 MHz, DMSO-*d*₆): 7.98 (d, *J* = 7.2, 1 H), 7.74 (d, *J* = 7.9, 1 H), 7.50-7.42 (m, 5H), 7.38 (t, *J* = 8.2, 1 H), 4.46 (t, *J* = 5.3, 1H), 3.52 (d, *J* = 3.4, 2H), 3.36-3.29 (m, 1 H), 3.27-3.18 (m, 1 H), 2.92 (d, *J* = 8.1, 1 H), 2.77 (d, *J* = 10.8, 1 H), 1.96 (d, *J* = 12.8, 1H), 1.75-1.60 (m, 4H), 1.58-1.45 (m, 1 H), 1.00-0.85 (m, 1 H).

### EXAMPLE 303

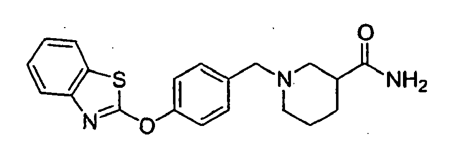

1-[4-(Benzothiazol-2-yloxy)-benzyl]-piperidine-3-carboxylic acid amide.

The title compound was prepared according to the procedure for EXAMPLE 259, step D using piperidine-3-carboxylic acid amide. MS (ESI): mass calculated for C₂₀H₂₁N₃O₂S, 367.1; m/z found, 368.4 [M+H]⁺. ¹H NMR (400 MHz, DMSO-*d*₆): 7.70 (d, *J* = 7.8, 1H), 7.46 (d, *J* = 8.0, 1 H), 7.25-7.00 (m, 7H), 6.53 (s, 1H), 3.26 (d, *J* = 2.9, 2H), 2.57 (d, *J* = 10.3, 1 H), 2.49 (d, *J* = 11.3, 1 H), 2.15-2.00 (m, 1 H), 1.78 (t, *J* = 10.7, 1 H), 1.69 (t, *J* = 9.4, 1 H), 1.51 (d, *J* = 10.0, 1 H), 1.40 (d, *J* = 12.9, 1 H), 1.22 (q, *J* = 12.6, 1 H), 1.09 (q, *J* = 12.2, 1H).

### EXAMPLE 304

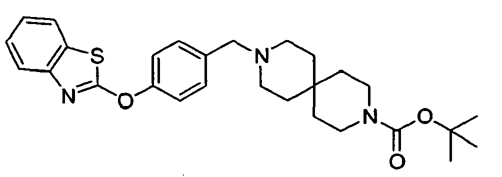

9-[4-(Benzothiazol-2-yloxy)-benzyl]-3,9-diaza-spiro[5.5]undecane-3-carboxylic acid tert-butyl ester.

The title compound was prepared according to the procedure for EXAMPLE 259, step D using 3,9-diaza-spiro[5.5]undecane-3-carboxylic acid tert-butyl ester. MS (ESI): mass calculated for C₂₈H₃₅N₃O₃S, 493.2; m/z found, 494.5 [M+H]⁺. ¹H NMR (400 MHz, DMSO-*d*₆): 8.13 (d, *J* = 7.9, 1 H), 7.89 (d, *J* = 8.0, 1H), 7.68-7.56 (m, 5H), 7.53 (t, *J* = 7.1, 1H), 3.70 (s, 2H), 3.51-3.45 (m, 4H), 2.60-2.50 (m, 4H), 1.66 (t, *J* = 5.3, 4H), 1.58 (s, 9H), 1.54 (t, *J* = 5.4, 4H).

### EXAMPLE 305

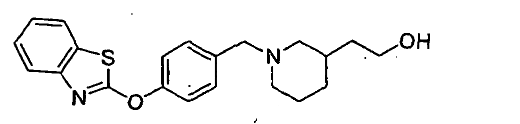

2-{1-[4-(Benzothiazol-2-yloxy)-benzyl]-piperidin-3-yl}-ethanol.

The title compound was prepared according to the procedure for EXAMPLE 259, step D using 2-piperidin-3-yl-ethanol. MS (ESI): mass calculated for C₂₁H₂₄N₂O₂S, 368.2; m/z found, 369.4 [M+H]⁺. ¹H NMR (400 MHz, DMSO-*d*₆): 7.71 (d, *J* = 8.4, 1 H), 7.47 (d, *J* = 7.9, 1 H), 7.25-7.15 (m, 5H), 7.10 (t, *J* = 8.1, 1H), 4.12 (t, *J* = 5.1, 1 H), 3.33-3.15 (m, 4H), 2.58-2.42 (m, 2H), 1.67 (t, *J* = 9.6, 1 H), 1.53-1.33 (m, 4H), 1.30-1.00 (m, 3H), 0.70-0.58 (m, 1 H).

### EXAMPLE 306

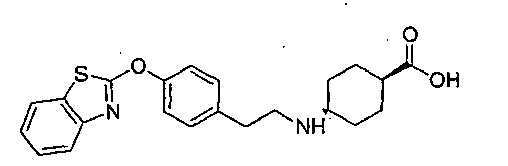

cis-4-{2-[4-(Benzothiazol-2-yloxy)-phenyl]-ethylamino}-cyclohexanecarboxylic acid trifluoromethanesulfonate salt.

The title compound was prepared according to the procedure for EXAMPLE 262, step B using cis-4-amino-cyclohexanecarboxylic acid. MS (ESI): exact mass calculated for C₂₂H₂₄N₂O₃S, 396.2; m/z found, 397.3 [M+H]⁺. ¹H NMR (400 MHz, CDCl₃): 9.15, (s, 1 H), 7.75 (d, *J* = 7.6, 1H), 7.72 (dd, *J* = 8.0, 0.7, 1 H), 7.43 (dt, *J* = 7.2, 1.2, 1H), 7.37-7.29 (m, 5H), 5.10 (br s, 1 H), 3.29 (br s, 2H), 3.10 (t, *J* = 7.4, 3H), 2.73 (br s, 1H), 2.24 (br d, *J* = 10.6, 2H), 2.09 (br d, *J* = 9.3, 2H), 1.72-1.52 (m, 3H).

### EXAMPLE 307

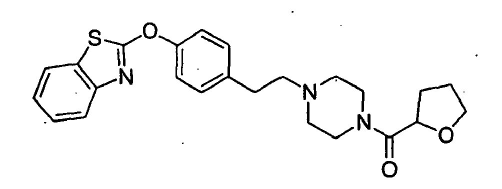

(4-{2-[4-(Benzothiazol-2-yloxy)-phenyl]-ethyl}-piperazin-1-yl)-(tetrahydro-furan-2-yl)-methanone.
A. 2-[4-(2-Piperazin-1-yl-ethyl)-phenoxy]-benzothiazole bis trifluoromethane sulfonate. 4-{2-[4-(Benzothiazol-2-yloxy)-phenyl]-ethyl}-piperazine-1-carboxylic acid tert-butyl ester was prepared according to Example 262, step B, using piperazine-1-carboxylic acid tert-butyl ester. A solution of 4-{2-[4-(benzothiazol-2-yloxy)-phenyl]-ethyl}-piperazine-1-carboxylic acid tert-butyl ester (3.9 g, 8.9 mmol) in CH₂Cl₂ (5 mL) with a solution of 50% trifluoromethansulfonic acid in CH₂Cl₂ (35 ml) and stirring at 23 °C for 4h. The reaction was concentrated and the residue suspended in diethyl ether, filtered and dried to give the title compound as a white solid (5.4 g, 94% yield). MS (ESI): exact mass calculated for C₁₉H₂₁N₃O₁S₁, 339.1; m/z found, 340.4 [M+H]⁺. ¹H NMR (400 MHz, D₆-DMSO): 9.50-9.30 (br. s, 1H), 7.94 (dd, *J* = 7.9, 0.6, 1 H), 7.68 (d, *J* = 7.6 Hz, 1 H), 7.48-7.41 (m, 5H), 7.39-7.30 (m, 1 H), 4.15-3.50 (br. s, 1H), 3.45-3.32 (m, 8H), 3.32-3.20 (m, 2H), 3.01 (dd, *J*= 8.8, 5.2 Hz, 2H).
B. (4-{2-[4-(Benzothiazol-2-yloxy)-phenyl]-ethyl}-piperazin-1-yl)-(tetrahydrofuran-2-yl)-methanone. The title compound was prepared according to the procedure for Example 257 step C, using tetrahydro-furan-2-carboxylic acid and triethylamine. MS (ESI): exact mass calculated for C₂₄H₂₇N₃O₃S, 437.2; m/z found, 438.5 [M+H]⁺. ¹H NMR (400 MHz, CDCl₃): 7.77 (d, *J* = 7.3, 1 H), 7.71 (dd, *J* = 7.8, 0.8, 1 H), 7.42 (dt, *J* = 7.3, 1.2, 1 H), 7.33-7.26 (m, 5H), 4.65 (dd, *J* = 7.0, 5.3, 1 H), 3.99 (ddd, *J* = 7.7, 7.1, 6.7, 1H), 3.92-3.86 (m, 1 H), 3.82-3.72 (m, 2H), 3.76 (s, 1H), 3.70-3.58 (m, 2H), 2.90 (dd, *J* = 10.3, 7.3, 2H), 2.70 (dd, *J* = 8.6, 5.6, 2H), 2.64-2.54 (m, 3H), 2.38-2.28 (m, 1 H), 2.14-1.90 (m, 4H).

### EXAMPLE 308

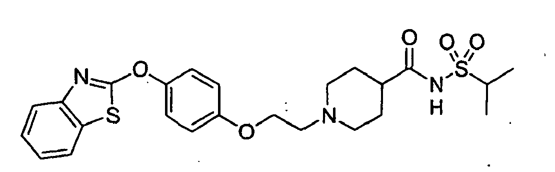

Propane-2-sulfonic acid (1-{2-[4-(benzothiazol-2-yloxy)-phenoxy]-ethyl}-piperidine-4-carbonyl)-amide.

A solution of 1-{2-[4-(benzothiazol-2-yloxy)-phenoxy]-ethyl}-piperidine-4-carboxylic acid amide (Example 22, 197 mg, 0.5 mmol) in THF (5 mL) was treated with NaH (14 mg, 0.6 mmol) and isopropylsulfonyl chloride (86 mg, 0.6 mmol) and the reaction was stirred at 60 °C for 16 h. The reaction was cooled and quenched by the addition of CH₃OH (5 mL) follwed by H₂O (5 mL)and the pH was adjusted to pH 7. The solution was extracted with ethyl acetate (2 x 25 mL) and the ethyl acetate solution was washed with 1 M NaOH (2 x 30 mL). The combined base washes were neutralized by the addition of 2 M HCl and extracted with chloroform (2 x 30 mL). The chloroform solutions were dried over Na₂SO₄, filtered and concentrated under reduced pressure. The crude product was purified on SiO₂ (4 g, 0-10% 2 M NH₃/CH₃OH:CH₂Cl₂) to give the title compound as a solid (45 mg, 18% yield). MS (ESI): exact mass calculated for C₂₄H₂₉N₃O₅S₂, 503.2; m/z found, 504.5 [M+H]⁺. ¹H NMR (400 MHz, C₆D₆): 7.77 (d, *J*= 7.8, 1 H), 7.20 (dd, *J* = 8.0, 0.8, 1H), 7.16-7.10 (m, 2H), 7.07 (dt, *J* = 8.3, 1.0, 1 H), 6.90 (dt, *J* = 8.0, 1.0, 1 H), 6.76 (d, *J* = 8.8, 1 H), 6.13 (s, 1 H), 3.98 (br s, 2H), 3.51 (sept., *J* = 6.8, 2H), 3.20 (br s, 2H), 2.82 (br s, 1 H), 2.37-2.00 (m, 4H), 1.96-1.84 (m, 2H), 1.33 (d, *J* = 6.6, 6H) 1.38-1.28 (m, 2H).

### EXAMPLE 309

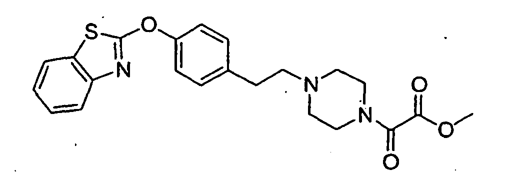

(4-{2-[4-(Benzothiazol-2-yloxy)-phenyl]-ethyl}-piperazin-1-yl)-oxo-acetic acid methyl ester.

A solution of 2-[4-(2-piperazin-1-yl-ethyl)-phenoxy]-benzothiazole bis-trifluoromethane sulfonate (296 mg, 0.52 mmol) in CH₂Cl₂ (3 mL) was treated with triethylamine (0.25 mL, 1.8 mmol), followed by addition of methyl chlorooxoacetate (0.07 mL, 0.8 mmol) and the reaction was stirred at 23°C for 20 min. The reaction was diluted with CH₂Cl₂ (10 mL) and washed with sat. aq. NaHCO₃(10 mL). The organic layer was dried over Na₂SO₄, filtered and concentrated under reduced pressure to give the title compound (234 mg, 99% yield). MS (ESI): exact mass calculated for C₂₂H₂₃N₃O₄S, 425.1; m/z found, 426.3 [M+H]⁺. ¹H NMR (400 MHz, CDCl₃): 7.76 (d, *J*= 7.6, 0.5,1H), 7.70 (d, *J* = 7.8, 1H), 7.41 (dt, *J* = 73, 1.0, 1H), 7.34-7.26 (m, 5H), 3.91 (s, 3H), 3.72 (t, *J* = 4.8, 2H), 3.51 (*t*, *J* = 4.8, 2H), 2.86, (dd, *J* = 10, 6.8, 2H), 2.70, (dd, *J* = 8.6, 5.6, 2H), 2.62-2.57 (m, 4H).

### EXAMPLE 310

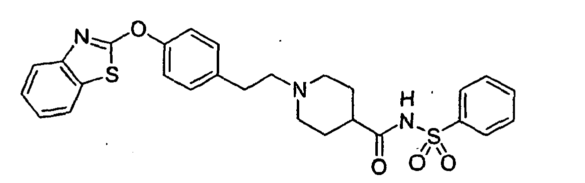

N-(1-{2-[4-(Benzothiazol-2-yloxy)-phenyl]-ethyl}-piperidine-4-carbonyl)-benzenesulfonamide trifluoromethanesulfonate salt.

The title compound was prepared according to the procedure for Example 34, step B using benzene sulfonamide. MS (ESI): exact mass calculated for C₂₇H₂₇N₃O₄S₂, 521.1; m/z found, 522.3 [M+H]⁺. ¹H NMR (400 MHz, CD₃OD): 8.60-8.00 (m, 2H), 7.82-7.76 (m, 1H), 7.73-7.57 (m, 4H), 7.48-7.28 (m, 5H), 3.70 (br s, 1H), 3.41-3.34 (m, 2H), 3.14-2.98 (m, 3H), 2.60 (br s, 1H), 2.08 (br d, *J* = 13.6, 2H), 1.86 (br s, 2H) 1.30 (br s, 2H), 0.94-0.88 (m, 2H).

### EXAMPLE 311

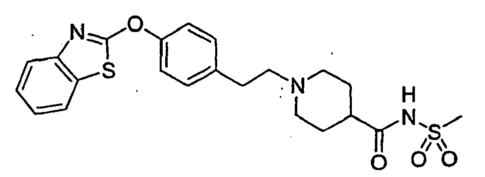

N-(1-{2-[4-(Benzothiazol-2-yloxy)-phenyl]-ethyl}-piperidine-4-carbonyl)-methanesulfonamide trifluoromethanesulfonate salt.

The title compound was prepared according to the procedure for EXAMPLE 34, step B using methane sulfonamide. MS (ESI): exact mass calculated for C₂₂H₂₅N₃O₄S₂, 459.1; m/z found, 460.3 [M+H]⁺. ¹H NMR (400 MHz, CD₃OD): 7.81 (d, *J* = 7.8, 1H), 7.62 (d, *J* = 8.1, 1H) 7.49-7.43 (m, 3H), 7.43-7.38 (m, 2H), 7.34 (t, *J* = 7.8, 1H), 3.80 (br d, *J* = 10.4, 1H), 3.48-3.40 (m, 2H), 3.33 (s, 3H), 3.28 (s, 2H), 3.20-3.04 (m, 3H), 2.70-2.60 (m, 1H), 2.21 (br d, *J* = 12.4, 2H), 2.06-1.93 (m, 2H).

### EXAMPLE 312

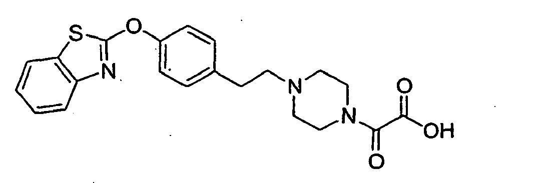

(4-{2-[4-(Benzothiazol-2-yloxy)-phenyl]-ethyl}-piperazin-1-yl)-oxo-acetic acid trifluoromethanesulfonate salt.

The title compound was prepared according to the procedure for Example 253, step D. MS (ESI): exact mass calculated for C₂₁H₂₁N₃O₄S, 411.1; m/z found, 412.3 [M+H]⁺. ¹H NMR (400 MHz, CDCl₃): 7.75 (t, *J* = 8.3, 2H), 7.70 (dt, *J* = 8.0, 0.8, 1H), 7.28-7.22 (m, 5H), 4.28-3.88 (br m, 4H), 3.58-3.20 (br m, 6H), 3.16-3.04 (br m, 2H).

### EXAMPLE 313

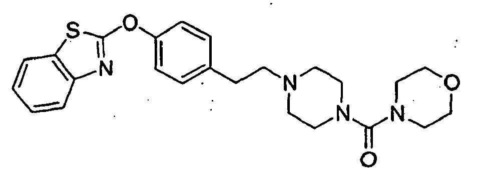

(4-{2-[4-(Benzothiazol-2-yloxy)-phenyl]-ethyl}-piperazin-1-yl)-morpholin-4-yl-methanone.

A solution of 2-[4-(2-piperazin-1-yl-ethyl)-phenoxy]-benzothiazole bis trifluoromethane sulfonate Example 307, step A (268 mg, 0.47 mmol) in CH₂Cl₂ (6 mL) was treated with PS-dimethylamine resin (1.3 g, 0.90 mmol) followed by 4-morpholine carbonyl chloride (0.07 mL, 0.6 mmol) and the reaction shaken for 1 h. The reaction was treated with PS-isocyanate resin to scavenge excess isocyanate and after 15 min, the reaction was filtered and concentrated to give the title compound (225 mg, 99% yield). MS (ESI): exact mass calculated for C₂₄H₂₈N₄O₃S₁, 452.2; m/z found, 453.3 [M+H]⁺. ¹H NMR (400 MHz, CDCl₃): 7.77 (dd, *J* = 8.0, 0.5, 1H), 7.71 (dd, *J* = 7.8, 0.5, 1H), 7.42 (dt, *J* = 7.3, 1.2, 1H), 7.34-7.28 (m, 5H), 3.78-3.70 (m, 4H), 3.39 (br t, *J* = 4.3, 4H), 3.31 (t, *J* = 4.8, 4H), 2.90 (dd, *J* = 10.4, 7.3, 2H), 2.70 (dd, *J* = 8.3, 7.3, 2H), 2.59 (br s, 4H).

### EXAMPLE 314

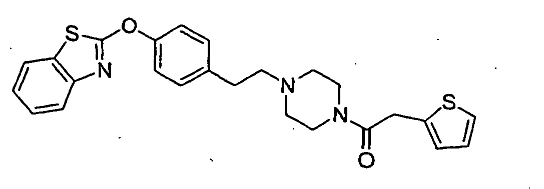

1-(4-{2-[4-(Benzothiazol-2-yloxy)-phenyl]-ethyl}-piperazin-1-yl)-2-thiophen-2-yl-ethanone

The title compound was prepared according to the procedure for EXAMPLE 313 using 2-thiopheneacetyl chloride. MS (ESI): exact mass calculated for C₂₅H₂₅N₃O₃S₂, 463.1; m/z found, 464.3 [M+H]⁺. ¹H NMR (400 MHz, CDCl₃): 7.77 (dd, *J* = 8.0, 0.5, 1H), 7.71 (dd, *J* = 7.8, 0.8, 1H), 7.43 (dt, *J* = 7.3, 1.2, 1H), 7.34-7.28 (m, 5H), 7.25 (dd, *J* = 5.3, 1.2, 1H), 7.00 (dd, *J* = 5.0, 3.2, 1H), 6.96-6.93 (m, 1H), 3.96 (d, *J* = 0.8, 2H), 3.74 (t, *J* = 4.8, 2H), 3.59 (t, *J* = 4.8, 2H), 2.86 (dd, *J* = 10.4, 7.6, 2H), 2.66 (dd, *J* = 8.3, 5.6, 2H), 2.55 (t, *J* = 5.3, 2H), 2.47 (t, *J* = 5.0, 2H).

### EXAMPLE 315

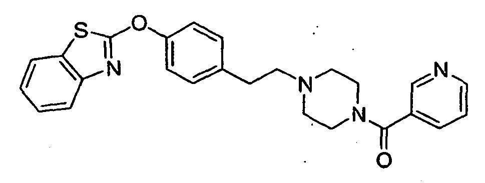

(4-{2-[4-(Benzothiazol-2-yloxy)-phenyl]-ethyl}-piperazin-1-yl)-pyridin-3-yl-methanone.

The title compound was prepared according to the procedure for EXAMPLE 313 using 2-nicotonyl chloride-hydrochloride. MS (ESI): exact mass calculated for C₂₅H₂₄N₄O₂S, 444.2; m/z found, 445.4 [M+H]⁺. ¹H NMR (400 MHz, CDCl₃): 8.73-8.70 (m, 2H), 7.81 (dt, *J* = 7.9, 1.9, 1H), 7.77 (dd, *J* = 8.1, 0.5, 1H), 7.71 (ddd, *J* = 7.9, 0.7, 0.4, 1H), 7.45-7.39 (m, 2H), 7.34-7.28 (m, 5H), 3.90 (br s, 2H), 3.52 (br s, 2H), 2.92-2.85 (m, 2H), 2.71 (dd, *J* = 8.9, 5.6, 2H), 2.74-2.60 (m, 2H), 2.54 (br s, 2H).

### EXAMPLE 316

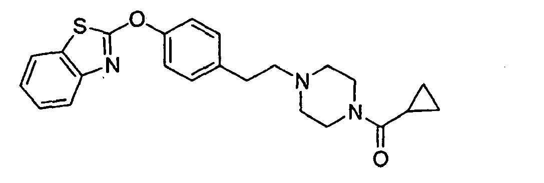

(4-{2-[4-(Benzothiazol-2-yloxy)-phenyl]-ethyl}-piperazin-1-yl)-cyclopropyl-methanone.

The title compound was prepared according to the procedure for EXAMPLE 313 using cyclopropane carbonylchloride. MS (ESI): exact mass calculated for C₂₃H₂₅N₃O₂S, 407.2; m/z found, 408.3 [M+H]⁺. ¹H NMR (400 MHz, CDCl₃): 7.77 (dd, *J* = 8.1, 0.5, 1H), 7.71 (dd, *J* = 7.9, 0.8, 1H), 7.43 (dt, *J* = 7.4, 1.3, 1H), 7.34-7.28 (m, 5H), 3.74 (d, *J* = 16.0, 4H), 2.89 (dd, *J* = 9.4, 6.5, 2H), 2.70 (dd, *J* = 9.4, 6.5, 2H), 2.58 (br d, *J* = 20.8, 4H), 1.78 (tt, *J* = 8.0, 4.7, 1H), 1.06-1.01 (m, 2H), 0.83-0.78 (m, 2H).

### EXAMPLE 317

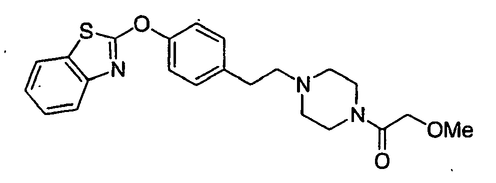

1-(4-{2-[4-(Benzothiazol-2-yloxy)-phenyl]-ethyl}-piperazin-1-yl)-2-methoxy-ethanone.

A solution of 2-[4-(2-piperazin-1-yl-ethyl)-phenoxy]-benzothiazole bis trifluoromethane sulfonate Example 307, step A (200 mg, 0.35 mmol) in CHCl₃ (14 mL) was treated with PS-dimethylamine resin (740 mg, 1.0 mmol) and the reaction shaken for 1h. The resin was filtered, and the resulting solution of free base was treated with methoxy acetic acid (0.04 ml, 0.5 mmol) and PS-carbonyl diimidazole resin (500 mg, 0.6 mmol) and the reaction shaken for 1 h. The reaction was filtered and concentrated and the crude product was purified on SiO₂ (12 g, 0-10% 2M NH₃/MeOH : CH₂Cl₂) to give the title compound (143 mg, 99% yield). MS (ESI): exact mass calculated for C₂₂H₂₅N₃O₂S, 411.2; m/z found, 412.4 [M+H]⁺. ¹H NMR (400 MHz, CDCl₃): 7.77 (dd, *J* = 8.1, 0.6, 1H), 7.71 (dd, *J* = 7.9, 0.8, 1H), 7.42 (dt, *J* = 7.5, 1.3, 1H), 7.35-7.28 (m, 5H), 4.14 (s, 2H), 3.72 (t, *J* = 4.8, 2H), 3.58 (t, *J* = 4.8, 2H), 3.47 (s, 3H), 2.89 (dd, *J* = 10.6, 7.3, 2H), 2.70 (dd, *J* = 8.5, 5.6, 2H), 2.58 (t, *J* = 5.1, 4H).

### EXAMPLE 318

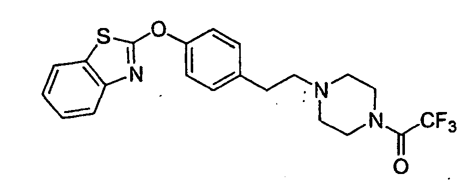

1-(4-{2-[4-(Benzothiazol-2-yloxy)-phenyl]-ethyl}-piperazin-1-yl)-2,2,2-trifluoroethanone.

The title compound was prepared according to the procedure for EXAMPLE 317 using trifluromethanesulfonic acid. MS (ESI): exact mass calculated for C₂₁H₂₀F₃N₃O₂S, 435.1; m/z found, 436.3 [M+H]⁺. ¹H NMR (400 MHz, CDCl₃): 7.77 (dd, *J* = 8.2, 0.5, 1H), 7.71 (dd, *J* = 7.9, 0.7, 1H), 7.43 (dt, *J* = 8.5, 1.2, 1H), 7.35-7.28 (m, 5H), 4.14 (s, 2H), 3.76 (t, *J* = 4.8, 2H), 3.68 (t, *J* = 4.7, 2H), 2.88 (dd, *J* = 10.0, 7.0, 2H), 2.70 (dd, *J* = 8.7, 5.6, 2H), 2.62 (t, *J* = 4.6, 4H).

### EXAMPLE 319

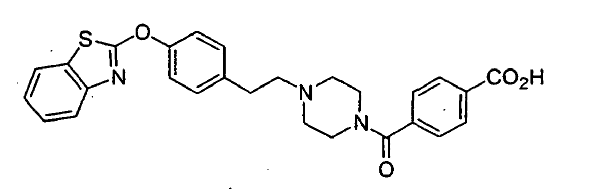

4-(4-{2-[4-(Benzothiazol-2-yloxy)-phenyl]-ethyl}-piperazine-1-carbonyl)-benzoic acid.

The title compound was prepared according to the procedure for EXAMPLE 262, step A using terephthalic acid monomethyl ester and EXAMPLE 250, step D. MS (ESI): exact mass calculated for C₂₇H₂₅N₃O₄S, 487.2; m/z found, 488.4 [M+H]⁺. ¹H NMR (400 MHz, DMSO-*d₆*): 8.00 (d, *J* = 8.3, 2H), 7.93 (dd, *J* = 8.0, 0.8, 1H), 7.69 (dd, *J* = 8.0, 0.4, 1H), 7.50 (d, *J* = 8.3, 2H), 7.43 (dt, *J* = 7.5, 1.3, 1H), 7.40-7.29 (m, 5H), 3.66 (br s, 2H), 3.30 (br s, 2H), 2.88 (dd, *J* = 7.0, 7.0, 2H), 2.60 (dd, *J* = 8.3, 8.3, 2H), 2.55 (br s, 2H), 2.44 (br s, 2H).

### EXAMPLE 320

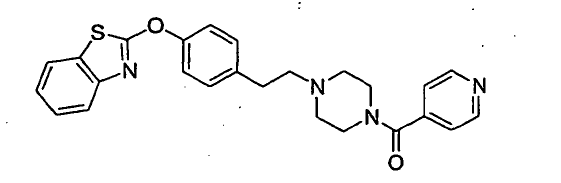

(4-{2-[4-(Benzothiazol-2-yloxy)-phenyl]-ethyl}-piperazin-1-yl)-pyridin-4-yl-methanone.

The title compound was prepared according to the procedure for EXAMPLE 317 using isonicotinic acid. MS (ESI): exact mass calculated for C₂₅H₂₄N₄O₂S, 444.2; m/z found, 445.4 [M+H]⁺. ¹H NMR (400 MHz, CDCl₃): 8.74 (dd, *J* = 4.4, 1.6, 1H), 7.77 (dd, *J* = 8.1, 0.5, 1H), 7.71 (dd, *J* = 7.9, 0.8, 1H), 7.43 (dt, *J* = 7.4, 1.3, 1H), 7.45-7.39 (m, 2H), 7.35-7.28 (m, 7H), 3.87 (br s, 2H), 3.44 (br t, *J* = 3.9, 2H), 2.88 (dd, *J* = 10.1, 6.9, 2H), 2.71 (dd, *J* = 8.8, 5.5, 2H), 2.70-2.64 (m, 2H), 2.54-2.49 (m, 2H).

### EXAMPLE 321

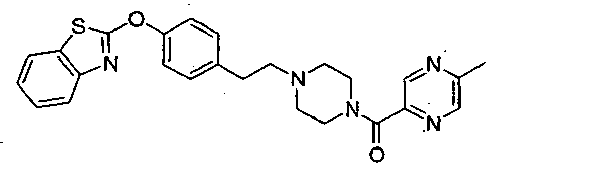

(4-{2-[4-(Benzothiazol-2-yloxy)-phenyl]-ethyl}-piperazin-1-yl)-(5-methyl-pyrazin-2-yl)-methanone.

The title compound was prepared according to the procedure for EXAMPLE 317 using 5-methyl-pyrazine-2-carboxylic acid. MS (ESI): exact mass calculated for C₂₅H₂₅N₅O₂S, 444.2; m/z found, 445.4 [M+H]⁺. ¹H NMR (400 MHz, CDCl₃): 8.89 (d, *J* =1.4, 1H), 8.45 (d, *J* =1.0, 1 H), 7.76 (dd, *J* = 8.1, 0.5, 1H), 7.70 (dd, *J* = 8.0, 0.8, 1H), 7.43 (dt, *J* = 7.4, 1.3, 1H), 7.33-7.28 (m, 5H), 3.90 (br t, *J* = 4.7, 2H), 3.77 (br t, *J* = 4.8, 2H), 2.92 (dd, *J* = 10.5, 6.5, 2H), 2.80-2.72 (m, 2H), 2.69-2.64 (m, 2H), 2.66 (s, 3H).

### EXAMPLE 322

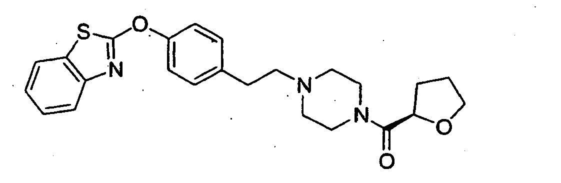

(*R*)-(4-{2-[4-(Benzothiazol-2-yloxy)-phenyl]-ethyl}-piperazin-1-yl)-(tetrahydrofuran-2-yl)-methanone.

The title compound was prepared according to the procedure for EXAMPLE 317 using 5-(R)-tetrahydro-furan-2-carboxylic acid. MS (ESI): exact mass calculated for C₂₄H₂₇N₃O₃S, 437.2; m/z found, 438.5 [M+H]⁺. ¹H NMR (400 MHz, CDCl₃): 7.77 (d, *J* = 7.3, 1H), 7.71 (dd, *J* = 7.8, 0.8, 1 H), 7.42 (dt, *J* = 7.3, 1.2, 1H), 7.33-7.26 (m, 5H), 4.65 (dd, *J* = 7.0, 5.3, 1 H), 3.99 (ddd, *J* = 7.1, 6.7, 6.7, 1H), 3.92-3.86 (m, 1H), 3.82-3.72 (m, 2H), 3.76 (s, 1 H), 3.70-3.58 (m, 2H), 2.90 (dd, *J* = 10.3, 7.3, 2H), 2.70 (dd, *J* = 8.6, 5.6, 2H), 2.64-2.54 (m, 3H), 2.38-2.28 (m, 1H), 2.14-1.90 (m, 4H).

### EXAMPLE 323

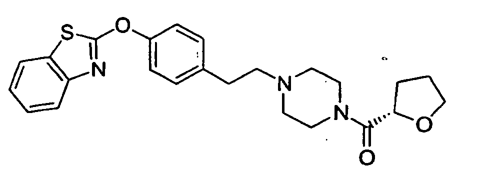

(*S*)-(4-{2-[4-(Benzothiazol-2-yloxy)-phenyl]-ethyl}-piperazin-1-yl)-(tetrahydrofuran-2-yl)-methanone.

The title compound was prepared according to the procedure for EXAMPLE 317 using 5-(*S*)-tetrahydro-furan-2-carboxylic acid. MS (ESI): exact mass calculated for C₂₄H₂₇N₃O₃S, 437.2; m/z found, 438.5 [M+H]⁺. ¹H NMR (400 MHz, CDCl₃): 7.77 (d, *J* = 7.3, 1 H), 7.71 (dd, *J* = 7.8, 0.8, 1H), 7.42 (dt, *J* = 7.3, 1.2, 1H), 7.33-7.26 (m, 5H), 4.65 (dd, *J* = 7.0, 5.3, 1H), 3.99 (ddd, *J* = 7.1, 6.7, 6.7, 1H), 3.89 (ddd, *J* = 7.7, 7.5, 5.8, 1H), 3.82-3.72 (m, 2H), 3.76 (s, 1H), 3.70-3.58 (m, 2H), 2.90 (dd, *J* = 10.3, 7.3, 2H), 2.70 (dd, *J* = 8.6, 5.6, 2H), 2.64-2.54 (m, 3H), 2.38-2.28 (m, 1H), 2.14-1.90 (m, 4H).

### EXAMPLE 324

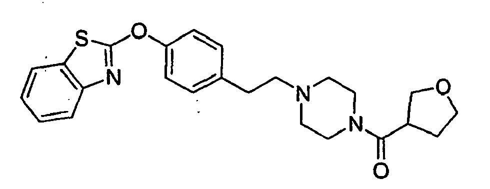

(4-{2-[4-(Benzothiazol-2-yloxy)-phenyl]-ethyl}-piperazin-1-yl)-(tetrahydro-furan-3-yl)methanone.

The title compound was prepared according to the procedure for EXAMPLE 317 using 5-tetrahydro-furan-3-carboxylic acid. MS (ESI): exact mass calculated for C₂₄H₂₇N₃O₃S, 437.2; m/z found, 438.5 [M+H]⁺. ¹H NMR (400 MHz, CDCl₃): 7.77 (dd, *J* = 8.1, 0.5, 1H), 7.71 (dd, *J* = 7.9, 0.8, 1H), 7.42 (dt, *J* = 7.5, 1.3, 1H), 7.33-7.26 (m, 5H), 4.06 (t, *J* = 8.2, 1H), 3.97-3.87 (m, 3H), 3.77-3.67 (m, 2H), 3.59 (t, *J* = 5.1, 1H), 3.35-3.24 (m, 1H), 2.88 (dd, *J* = 10.2, 7.3, 2H), 2.79 (dd, *J* = 8.5, 5.7, 2H), 2.60-2.50 (m, 4H), 2.29 (dddd, *J* = 12.6, 7.6, 6.4, 6.4, 1H), 2.17-2.08 (m, 1H).

### EXAMPLE 325

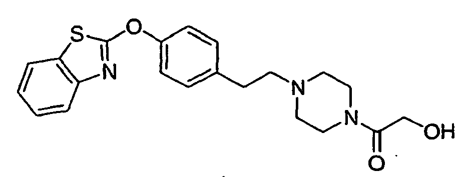

1-(4-{2-[4-(Benzothiazol-2-yloxy)-phonyl]-ethyl}-piperazin-1-yl)-2-hydroxy-ethanone.

The title compound was prepared according to the procedure for EXAMPLE 317 using gylcolic acid. MS (ESI): exact mass calculated for C₂₁H₂₃N₃O₃S, 397.2; m/z found, 398.3 [M+H]⁺. ¹H NMR (400 MHz, CDCl₃): 7.77 (dd, *J* = 8.1, 0.5, 1H), 7.71 (dd, *J* = 8.0, 0.7, 1 H), 7.43 (dt, *J* = 7.5, 1.3, 1H), 7.35-7.28 (m, 5H), 4.21 (s, 2H), 3.75 (t, *J* = 5.0, 2H), 3.68 (br s, 1H), 3.35 (t, *J* = 5.0, 2H), 2.88 (dd, *J* = 10.1, 7.0, 2H), 2.70 (dd, *J* = 8.6, 5.6, 2H), 2.58 (ddd, *J* = 8.4, 8.3, 5.2, 1H).

### EXAMPLE 326

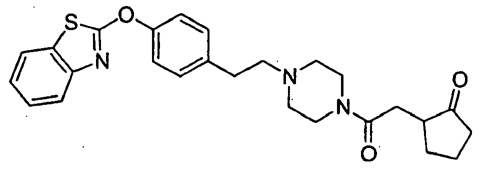

2-[2-(4-{2-[4-(Benzothiazol-2-yloxy)phenyl]-ethyl}-piperazin-1-yl)-2-oxo-ethyl]-cyclopentanone.

The title compound was prepared according to the procedure for EXAMPLE 317 using 5- (2-oxo-cyclopentyl)-acetic acid. MS (ESI): exact mass calculated for C₂₆H₂₉N₃O₃S, 463.2; m/z found, 464.4 [M+H]⁺. ¹H NMR (400 MHz, CDCl₃): 7.77 (d, *J* = 7.7, 1H), 7.71 (dd, *J* = 7.9, 0.7, 1H), 7.42 (dt, *J* = 7.5, 1.2, 1H), 7.33-7.28 (m, 5H), 3.80-3.64 (m, 2H), 3.54 (dd, *J* = 9.2, 3.9, 1H), 2.92-2.84 (m, 3H), 2.88 (dd, *J* = 10.1, 7.0, 2H), 2.68 (dd, *J* = 8.5, 5.6, 2H), 2.60-2.25 (m, 2H), 2.15-2.06 (m, 1H), 1.93-1.80 (m, 1H), 1.78-1.64 (m, 2H).

### EXAMPLE 327

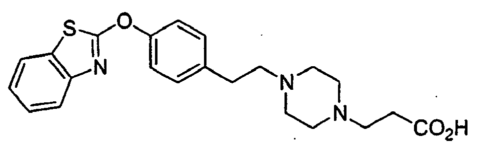

3-(4-{2-[4-(Benzothiazol-2-yloxy)-phenyl]-ethyl}-piperazin-1-yl)-propionic acid trifluoromethansufonate salt.

The title compound was prepared according to the procedure for EXAMPLE 262, step A using 3-piperazin-1-yl-propionic acid. MS (ESI): exact mass calculated for C₂₁H₂₃N₃O₃S₁, 411.2; m/z found, 412.4 [M+H]⁺. ¹H NMR (400 MHz, CDCl₃): 11.25 (br s, 1H), 7.76 (dd, *J* = 8.0, 0.5, 1H), 7.69 (dd, *J* = 7.8, 0.6, 1H), 7.42 (dt, *J* = 7.4, 1.2, 1H), 7.35-7.28 (m, 5H), 3.76 (s, 1H), 3.04 (t, *J* = 6.4, 4H), 2.91 (dd, *J* = 10.3, 5.4, 4H), 2.86-2.79 (m, 2H), 2.63 (t, *J* = 6.4, 1H), 2.08 (s, 4H).

### EXAMPLE 328

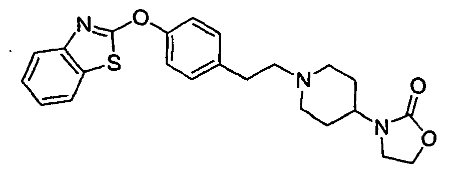

3-(1-{2-[4-(Benzothiazol-2-yloxy)-phenyl]-ethyl}-piperidin-4-yl)-oxazolidin-2-one.

The title compound was prepared according to the procedure for EXAMPLE 262, step A using 3-piperidin-4-yl-oxazolidin-2-one hydrochloride salt and EXAMPLE 259, step A. MS (ESI): exact mass calculated for C₂₃H₂₅N₃O₃S₁, 423.2; m/z found, 424.4 [M+H]⁺. ¹H NMR (400 MHz, CDCl₃): 7.77 (dd, *J* = 8.1, 0.5, 1H), 7.69 (dd, *J* = 7.9, 1.1, 1H), 7.41 (dt, *J* = 8.5, 1.2, 1H), 7.35-7.28 (m, 5H), 4.36, (t, *J* = 7.8, 2H), 3.72 (t, *J* = 4.8, 2H), 3.86-3.76 (m, 1H), 3.57 (t, *J* = 8.1, 2H), 3.11 (br d, *J* = 11.8, 2H), 2.86 (dd, *J* = 11.0, 7.6, 2H), 2.66 (dd, *J* = 8.6, 5.2, 2H), 2.18 (dt, *J* = 11.7, 2.7, 2H), 1.89-1.72 (m, 4H).

### EXAMPLE 329

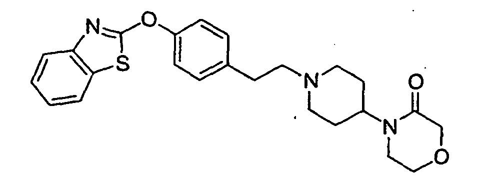

4-(1-{2-[4-(Benzothiazol-2-yloxy)-phenyl]-ethyl}-piperidin-4-yl)-morpholin-3-one.

The title compound was prepared according to the procedure for EXAMPLE 262, step A using piperidin-4-yl-morpholin-3-one and EXAMPLE 260, step A. MS (ESI): exact mass calculated for C₂₄H₂₇N₃O₃S₁, 437.2; m/z found, 438.4 [M+H]⁺. ¹H NMR (400 MHz, CDCl₃): 7.76 (d, *J* = 8.0, 1H), 7.70 (dd, *J* = 7.9, 0.7, 1H), 7.42 (dt, *J* = 7.4, 1.2, 1H), 7.33-7.28 (m, 5H), 4.57 (tt, *J* = 11.9, 4.3, 1H), 4.23 (s, 2H), 3.91, (t, *J* = 4.9, 2H), 3.34 (t, *J* = 5.1, 2H), 3.12 (br d, *J* = 11.6, 2H), 2.87 (dd, *J* = 10.9, 7.6, 2H), 2.67 (dd, *J* = 8.7, 5.2, 2H), 2.23 (ddd, *J* = 11.7, 11.6, 2.5, 2H), 1.82 (ddd, *J* = 24.1, 12.1, 3.8, 2H), 1.76-1.70 (m, 2H).

### EXAMPLE 330

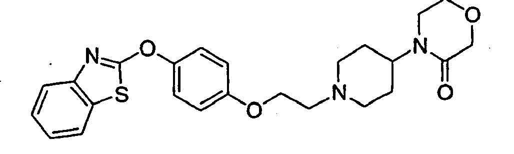

4-(1-{2-[4-(Benzothiazol-2-yloxy)-phenoxy]-ethyl}-piperidin-4-yl)-morpholin-3-one.

The title compound was prepared according to the procedure for EXAMPLE 17, step A using piperidin-4-yl-morpholin-3-one and EXAMPLE 260, step A. MS (ESI): exact mass calculated for C₂₄H₂₇N₃O₄S₁, 453.2; m/z found, 454.4 [M+H]⁺. ¹H NMR (400 MHz, CDCl₃): 7.76 (dd, *J* = 8.1, 0.5, 1H), 7.68 (dd, *J* = 7.9, 0.7, 1H), 7.40 (dt, *J* = 7.4, 1.3, 1H), 7.31-7.28 (m, 3H), 7.02-6.96 (m, 2H), 4.57 (tt, *J* = 12.1, 4.2, 1H), 4.22 (s, 2H), 4.14 (t, *J* = 5.6, 2H), 3.90 (t, *J* = 5.0, 2H), 3.33 (t, *J* = 5.1,2H), 3.12 (br d, *J* = 11.7, 2H), 2.87 (t, *J* = 5.7, 2H), 2.32 (ddd, *J* = 11.8, 11.8, 2.4, 2H), 1.82 (dddd, *J* = 12.2, 12.1, 12.1, 3.8, 2H), 1.75-1.68 (m, 2H).

### EXAMPLE 331

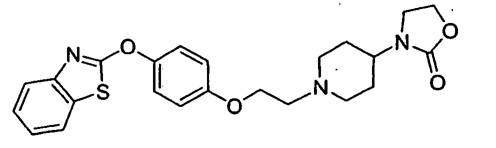

3-(1-{2-[4-(Benzothiazol-2-yloxy)-phenoxy]-ethyl}-piperidin-4-yl)-oxazolidin-2-one.

The title compound was prepared according to the procedure for EXAMPLE 17, step A using 3-piperidin-4-yl-oxazolidin-2-one hydrochloride salt and EXAMPLE 259, step A. MS (ESI): exact mass calculated for C₂₃H₂₅N₃O₄S₁, 439.2; m/z found, 440.5 [M+H]⁺. ¹H NMR (400 MHz, CDCl₃): 7.76 (dd, *J* = 8.1, 0.7, 1H), 7.69 (dd, *J* = 8.0, 0.7, 1H), 7.42 (dt, *J* = 8.6, 1.3, 1H), 7.35-7.26 (m, 3H), 7.02-6.97 (m, 2H), 4.36 (dd, *J* = 8.7, 7.3, 2H), 4.14 (t, *J* = 5.7, 2H), 3.80 (ddd, *J* = 16.4, 11.0, 5.6, 1H), 3.60-3.53 (m, 2H), 3.12 (br d, *J* = 12.0, 2H), 2.87 (d, *J* = 5.7, 2H), 2.28 (ddd, *J* = 11.7, 11.6, 3.8, 2H), 1.90-1.74(m, 4H).

### EXAMPLE 332

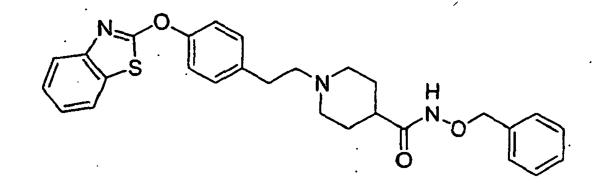

1-{2-[4-(Benzothiazol-2-yloxy)-phenyl]-ethyl}-piperidine-4-carboxylic acid benzyloxy-amide.

A solution of 1-{2-[4-(benzothiazol-2-yloxy)-phenyl]-ethyl}-piperidine-4-carboxylic acid EXAMPLE 34, step A (383 mg, 1.0 mmol) in CH₂Cl₂ (10 mL) was treated with N-methylmorpholine (0.24 ml, 2.2 mmol) and cyanuric chloride (61 mg, 0.3 mmol) and the resulting solution stirred for 1 h, at which time the reaction was treated with O-benzyl hydroxylamine and stirred for 16h. The reaction was filtered through diatomaceous earth concentrated and purified on SiO₂ (12 g, 0-10% 2M NH₃/MeOH/CH₂Cl₂) to give the title compound (81 mg, 17% yield). MS (ESI): exact mass calculated for C₂₈H₂₉N₃O₃S₁, 487.2; m/z found, 488.5 [M+H]⁺. ¹H NMR (400 MHz, CDCl₃): 8.34 (br s, 1H), 7.76 (dd, *J* = 8.1, 0.5, 1H), 7.70 (dd, *J* = 7.9, 0.7, 1H), 7.45-7.39 (m, 6H), 7.34-7.28 (m, 4H), 4.96 (br s, 2H), 3.05 (br d, *J* = 11.6, 2H), 2.85 (dd, *J* = 10.6, 7.6, 2H), 2.63 (dd, *J* = 8.5, 5.5, 2H), 2.04 (ddd, *J* = 11.0, 11.0, 2.8, 2H), 1.91-1.76 (m, 2H).

### EXAMPLE 333

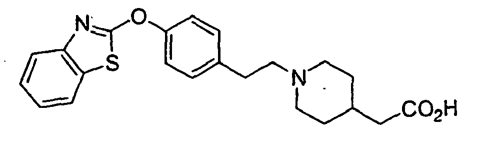

(1-{2-[4-(Benzothiazol-2-yloxy)-phenyl]-ethyl}-piperidin-4-yl)-acetic acid.

The title compound was prepared according to the procedure for EXAMPLE 262, step A using piperidin-4-yl-acetic acid ethyl ester and EXAMPLE 250, step D. MS (ESI): exact mass calculated for C₂₂H₂₄N₂O₃S₁, 396.2; m/z found, 397.4 [M+H]⁺. ¹H NMR (400 MHz, CD₃OD): 8.34 (br s, 1H), 7.82 (dd, *J* = 7.9, 0.6, 1H), 7.67 (dd, *J* = 8.1, 0.5, 1H), 7.53-7.33 (m, 6H), 3.78-3.66 (m, 2H), 3.46-3.38 (m, 2H), 3.22-3.14 (m, 2H), 3.14-3.07 (m, 2H), 2.39 (br d, *J* = 6.1, 1H), 2.20-2.08 (br s , 1H), 2.11 (br d, *J* = 13.5, 1H), 1.72-1.54 (m, 2H).

### EXAMPLE 334

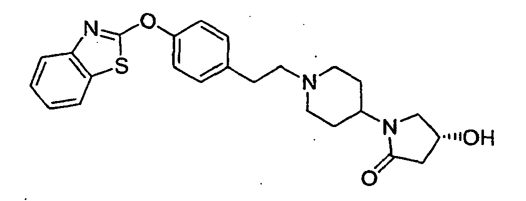

(*R*)-1-(1-{2-[4-(Benzothiazol-2-yloxy)-phenyl]-ethyl}-piperidin-4-yl)-4-hydroxy-pyrrolidin-2-one

The title compound was prepared according to the procedure for EXAMPLE 262, step A using (R)-4-hydroxy-1-piperidin-4-yl-pyrrolidin-2-one acetic acid salt and EXAMPLE 261, step A. MS (ESI): exact mass calculated for C₂₄H₂₇N₃O₃S₁, 437.2; m/z found, 438.4 [M+H]⁺. ¹H NMR (400 MHz, CDCl₃): 7.77 (dd, *J* = 8.1, 0.5, 1H), 7.66 (dd, *J* = 7.9, 0.7, 1H), 7.40 (dt, *J* = 7.7, 1.3, 1H), 7.28-7.24 (m, 5H), 4.52 (ddd, *J* = 8.5, 5.9, 2.4, 1H), 4.11-4.00 (m, 1H), 3.60 (dd, *J* = 10.7, 5.6, 1H), 3.31 (dd, *J* = 10.7, 2.2, 1H), 3.12-3.04 (m, 2H), 2.83 (dd, *J* = 9.7, 6.5, 2H), 2.72 (dd, *J* = 17.2, 6.6, 1H), 2.63 (dd, *J* = 9.8, 6.4, 2H), 2.72 (dd, *J* = 17.2, 2.6, 1H), 2.21-2.12 (m, 2H), 1.85-1.67 (m, 4H).

### EXAMPLE 335

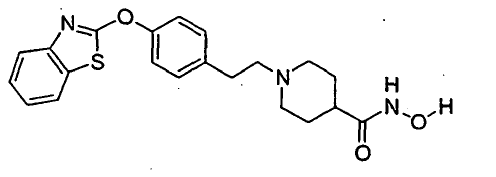

1-{2-[4-(Benzothiazol-2-yloxy)-phenyl]-ethyl}-piperidine-4-carboxylic acid hydroxyamide

The title compound was prepared according to the procedure for EXAMPLE 262, step A using piperidine-4-carboxylic acid hydroxyamide. MS (ESI): exact mass calculated for C₂₁H₂₃N₃O₃S₁, 397.2; m/z found, 398.4 [M+H]⁺. ¹H NMR (400 MHz, CDCl₃): 7.73-7.60 (m, 2H), 7.40-7.30 (m, 1H), 7.30-7.16 (m, 5H), 3.48-3.36 (br s, 2H), 3.10-2.95 (m, 4H), 2.80-2.50 (br m, 3H), 2.23-1.96 (br m, 5H).

### EXAMPLE 336

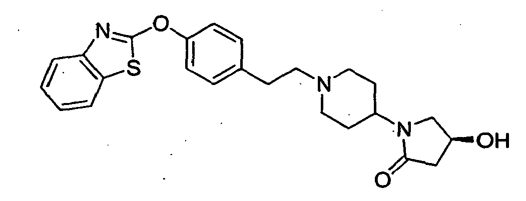

(*S*)-1-(1-{2-[4-(Benzothiazol-2-yloxy)-phenyl]-ethyl}-piperidin-4-yl)-4-hydroxypyrrolidin-2-one.

The title compound was prepared according to the procedure for EXAMPLE 262, step A using (*S*)-4-hydroxy-1-piperidin-4-yl-pyrrolidin-2-one acetic acid salt, EXAMPLE 261, step A using (*S*)-4-bromo-3-hydroxybutyrate MS (ESI): exact mass calculated for C₂₄H₂₇N₃O₃S₁, 437.2; m/z found, 438.4 [M+H]⁺. ¹H NMR (400 MHz, CDCl₃): 7.77 (dd, *J* = 8.1, 0.5, 1H), 7.66 (dd, *J* = 7.9, 0.7, 1H), 7.40 (dt, *J* = 7.7, 1.3, 1H), 7.28-7.24 (m, 5H), 4.52 (ddd, *J* = 8.5, 5.9, 2.4, 1H), 4.11-4.00 (m, 1H), 3.60 (dd, *J* = 10.7, 5.6, 1 H), 3.31 (dd, *J* = 10.7, 2.2, 1H), 3.12-3.04 (m, 2H), 2.83 (dd, *J* = 9.7, 6.5, 2H), 2.72 (dd, *J* = 17.2, 6.6, 1H), 2.63 (dd, *J* = 9.8, 6.4, 2H), 2.72 (dd, *J* = 17.2, 2.6, 1H), 2.21-2.12 (m, 2H), 1.85-1.67 (m, 4H).

### EXAMPLE 337

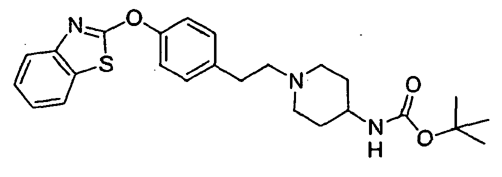

(1-{2-[4-(Benzothiazol-2-yloxy)-phenyl]-ethyl}-piperidin-4-yl)-carbamic acid tert-butyl ester.

The title compound was prepared according to the procedure for EXAMPLE 262, step A using piperidin-4-yl-carbamic acid tert-butyl ester. MS (ESI): exact mass calculated for C₂₅H₃₁N₃O₃S₁, 453.2; m/z found, 454.4 [M+H]⁺. ¹H NMR (400 MHz, CDCl₃): 7.73 (dd, *J* = 8.2, 0.6, 1H), 7.65 (dd, *J* = 7.9, 0.7, 1H), 7.39 (dt, *J* = 7.4, 1.3, 1H), 7.29-7.23 (m, 5H), 4.47 (br d, *J* = 6.6, 1 H), 3.55-3.45 (m, 1H), 2.93 (br d, *J* = 11.3, 2H), 2.82 (dd, *J* = 11.0, 7.7, 2H), 2.60 (dd, *J* = 8.6, 5.3, 2H), 2.16 (t, *J* = 11.3, 2H), 1.97 (br d, *J* = 11.1, 2H), 1.45 (s, 9H).

### EXAMPLE 338

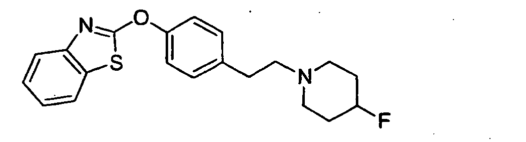

2-{4-[2-(4-Fluoro-piperidin-1-yl)-ethyl]-phenoxy}-benzothiazole.

The title compound was prepared according to the procedure for EXAMPLE 262, step A using 4-fluoro piperidine. MS (ESI): exact mass calculated for C₂₀H₂₁F₁N₃O₁S₁, 356.1; m/z found, 357.3 [M+H]⁺. ¹H NMR (400 MHz, CDCl₃); 7.73 (dd, *J* = 8.1, 0.5, 1H), 7.65 (dd, *J* = 8.0, 0.7, 1H), 7.41-7.36 (m, 1H), 7.28-7.24 (m, 5H), 4.70 (dm, *J* = 48.6, 1 H), 2.85 (dd, *J* = 10.9, 7.7, 2H), 2.71-2.63 (m, 2H), 2.62 (dd, *J* = 8.3, 5.4, 2H), 2.52-2.44 (m, 2H), 2.03-1.86 (m, 4H).

### EXAMPLE 339

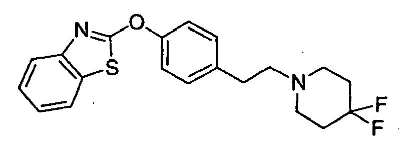

2-{4-[2-(4,4-Difluoro-piperidin-1-yl)-ethyl]-phenoxy}-benzothiazole.

The title compound was prepared according to the procedure for EXAMPLE 262, step A using 4,4-difluoro piperidine. MS (ESI): exact mass calculated for C₂₀H₂₀F₂N₃O₁S₁, 374.1; m/z found, 375.3 [M+H]⁺. ¹H NMR (400 MHz, CDCl₃): 7.73 (dd, *J* = 8.1, 0.6, 1H), 7.65 (dd, *J* = 8.0, 0.7, 1H), 7.41-7.36 (m, 1H), 7.28-7.24 (m, 5H), 2.82 (dd, *J* = 10.2, 6.4, 2H), 2.71-2.60 (m, 6H), 2.09-1.97 (m, 4H).

### EXAMPLE 340

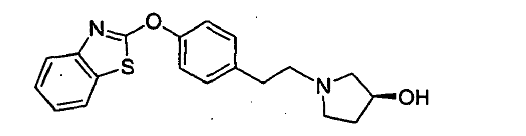

(*R*)-1-{2-[4-(Benzothiazol-2-yloxy)-phenyl]-ethyl}-pyrrolidin-3-ol,

The title compound was prepared according to the procedure for EXAMPLE 262, step A using (R)-3-hydroxypyrrolidine. MS (ESI): exact mass calculated for C₁₉H₂₀N₂O₂S₁, 340.1; m/z found, 341.1 [M+H]⁺. ¹H NMR (400 MHz, CDCl₃): 7.73 (dd, *J* = 8.0, 0.4, 1H), 7.66 (dd, *J* = 7.9, 0.8, 1H), 7.39 (dt, *J* = 7.4, 1.2, 1H), 7.31-7.24 (m, 5H), 4.36 (dddd, *J* = 7.3, 4.8, 2.2, 2.2, 1H), 2.96 (ddd, *J* = 8.5, 8.5, 5.1, 1H), 2.90-2.83 (m, 2H), 2.79-2.70 (m, 3H), 2.58 (dd, *J* = 10.0, 5.2, 2H), 2.36 (ddd, *J* = 8.8, 8.8, 6.5, 1H), 2.21 (dddd, *J* = 13.7, 8.6, 7.2, 5.1, 1H), 1.82-1.73 (m, 1H).

### EXAMPLE 341

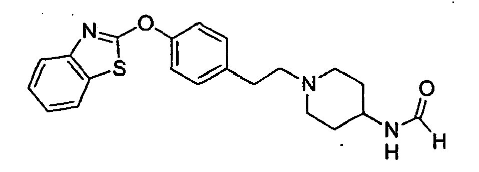

N-(1-{2-[4-(Benzothiazol-2-yloxy)-phenyl]-ethyl}-piperidin-4-yl)-formamide.

The title compound was prepared from EXAMPLE 337 following the procedure for EXAMPLE 266, step B and EXAMPLE 317. MS (ESI): exact mass calculated for C₂₁H₂₃N₃O₂S₁, 381.2; m/z found, 382.4 [M+H]⁺. ¹H NMR (400 MHz, CDCl₃): 8.16 (s, 1H), 7.72 (d, *J* = 8.6, 1H), 7.65 (dd, *J* = 7.9, 0.7, 1H), 7.42-7.36 (m, 1H), 7.31-7.23 (m, 5H), 4.03-3.93 (m, 1H), 3.13-3.03 (m, 2H), 2.90 (dd, *J* = 11.1, 7.2, 2H), 2.75-2.69 (m, 2H), 2.34 (t, *J* = 9.2, 2H), 2.08-2.00 (m, 2H), 1.72-1.60 (m, 2H).

### EXAMPLE 342

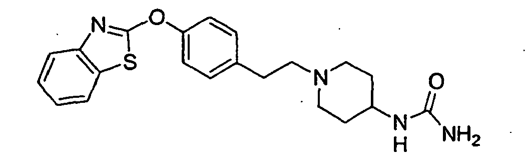

(1-{2-[4-(Benzothiazol-2-yloxy)-phenyl]-ethyl}-piperidin-4-yl)-urea

1-{2-[4-(Benzothiazol-2-yloxy)-phenyl]-ethyl}-piperidin-4-ylamine hydrochloride was prepared from (1-{2-[4-(benzothiazol-2-yloxy)-phenyl]-ethyl}-piperidin-4-yl)-carbamic acid tert-butyl ester EXAMPLE 337 following the procedure for EXAMPLE 266, step B. The resulting amine hydrochloride (227 mg, 0.58 mmol) in CH₂Cl₂ (5 mL) was treated with pyridine (0.14 mL, 1.7 mmol), trimethylisocyanate (0.09 ml, 0.64 mmol), and triethylamine (0.08 mL, 0.58 mmol) and the reaction stirred for 16h. The reaction was diluted with CH₂Cl₂ (25 mL) and washed with NaHCO₃ (30 mL). The organic layer was dried over Na₂SO₄, filtered and concentrated under reduced pressure. The crude material was purified on SiO₂ (12 g, 0-10% [2 M NH₃] CH₃OH/CH₂Cl₂) to give the title compound (134 mg, 58% yield). MS (ESI): exact mass calculated for C₂₁H₂₄N₄O₂S₁, 396.2; m/z found, 397.4 [M+H]⁺. ¹H NMR (400 MHz, CDCl₃): 7.72 (d, *J* = 8.7, 1H), 7.65 (dd, *J* = 7.9, 0.7, 1H), 7.42-7.36 (m, 1H), 7.31-7.23 (m, 5H), 4.93 (d, *J* = 8.0, 1H), 4.78 (s, 2H), 3.93 (br d, *J* = 11.7, 1H), 2.82 (dd, *J* = 10.7, 7.6, 2H), 2.60 (dd, *J* = 8.5, 5.4, 2H), 2.16 (t, *J* = 11.3, 2H), 1.97 (dd, *J* = 12.7, 3.1, 2H), 1.47 (dddd, *J* = 12.8, 12.6, 12.6, 3.7, 2H).

### EXAMPLE 343

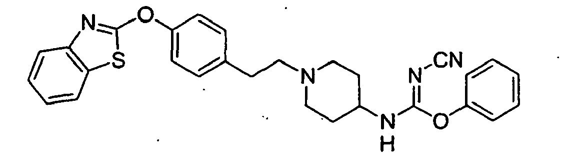

1-(1-{2-[4-(Benzothiazol-2-yloxy)-phenyl]-ethyl}-piperidin-4-yl)-3-cyano-2-phenyl-isourea

The 1-{2-[4-(benzothiazol-2-yloxy)-phenyl]-ethyl}-piperidin-4-ylamine hydrochloride was prepared from (1-{2-[4-(benzothiazol-2-yloxy)-phenyl]-ethyl}-piperidin-4-yl)-carbamic acid tert-butyl ester EXAMPLE 337 by removal of the Boc group according to EXAMPLE 266, step B. The free base was prepared by suspending 1-{2-[4-(benzothiazol-2-yloxy)-phenyl]-ethyl)-piperidin-4-ylamine hydrochloride (1.3 g, 3.3 mmol) in CH₂Cl₂ (20 mL) and washing with a solution of saturated NaHCO₃ (20 mL). The organic layer was dried over Na₂SO₄, filtered and concentrated under reduced pressure to give 1.0 g (83% yield) of 1-{2-[4-(benzothiazol-2-yloxy)-phenyl]-ethyl}-piperidin-4-ylamine free base. A solution of 1-{2-[4-(benzothiazol-2-yloxy)-phenyl]-ethyl}-piperidin-4-yl amine (145 mg, 0.4 mmol) in ethanol (5 mL) was treated with diphenyl cyanocarbodiimidate (319 mg. 1.34 mmol) and heated to 80 °C for 16h. The reaction was concentrated under reduced pressure and purified on SiO₂ (12 g, 0-10% [2 M NH₃ CH₃OH]/CH₂Cl₂) to give of the title compound (173 mg, 85% yield). MS (ESI): exact mass calculated for C₂₈H₂₇N₅O₂S₁, 496.2; m/z found, 498.4 [M+H]⁺. ¹H NMR (400 MHz, CDCl₃): 7.73 (d, *J* = 8.7, 1H), 7.66 (dd, *J* = 7.9, 0.7, 1H), 7.45-7.36 (m, 4H), 7.31-7.24 (m, 5H), 7.08 (d, *J* = 7.8, 2H), 6.36 (br d, *J* = 6.5, 1H), 3.87-3.76 (m, 1H), 3.02 (br d, *J* = 11.2, 2H), 2.83 (dd, *J* = 8.4, 7.3, 2H), 2.64 (dd, *J* = 8.6, 5.4, 2H), 2.21 (t, *J* = 11.1, 2H), 2.07 (d, *J* = 11.0, 2H), 1.76 (dd, *J* = 20.7, 10.4, 2H).

### EXAMPLE 344

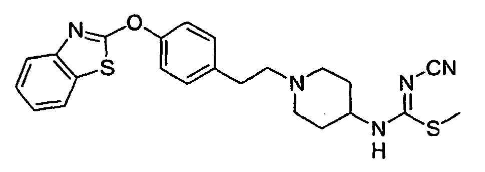

1-(1-{2-[4-(Benzothiazol-2-yloxy)-phenyl]-ethyl}-piperidin-4-yl)-3-cyano-2-methyl-isothiourea.

The 1-{2-[4-(benzothiazol-2-yloxy)-phenyl]-ethyl}-piperidin-4-ylamine hydrochloride was prepared from (1-{2-[4-(benzothiazol-2-yloxy)-phenyl]-ethyl}-piperidin-4-yl)-carbamic acid tert-butyl ester EXAMPLE 337 by removal of the Boc group according to EXAMPLE 266, step B. The free base was prepared according to EXAMPLE 343. A solution of 1-{2-[4-(benzothiazol-2-yloxy)-phenyl]-ethyl}-piperidin-4-yl amine (145 mg, 0.4 mmol) in ethanol (5 mL) was treated with dimethyl-N-cyanodithioiminocarbonate (180 mg. 1.34 mmol) and heated to 80 °C for 16h. The reaction was concentrated under reduced pressure and purified on SiO₂ (12 g, 0-10% 2M NH₃/CH₃OH/CH₂Cl₂) to give of the title compound (143 mg, 69% yield). MS (ESI): exact mass calculated for C₂₃H₂₅N₅O₁S₂, 451.2; m/z found, 452.3 [M+H]⁺. ¹H NMR (400 MHz, CDCl₃): 7.73 (dd, *J* = 8.1, 0.5, 1H), 7.66 (dd, *J* = 7.9, 0.7, 1H), 7.41-7.36 (m, 2H), 7.29-7.24 (m, 5H), 6.13 (br d, *J* = 196, 2H), 2.97 (br d, *J* = 12.0, 2H), 2.82 (dd, *J* = 10.3, 7.3, 2H), 2.62 (dd, *J* = 8.5, 5.5, 2H), 2.60-2.44 (br s, 3H), 2.18 (t, *J* = 11.5, 2H), 2.03 (d, *J* = 13.5, 2H), 1.7-1.55 (m, 2H).

### EXAMPLE 345

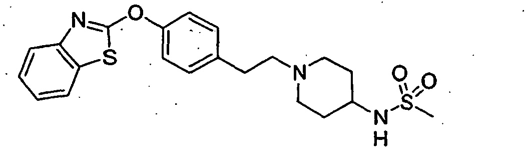

N-(1-{2-[4-(Benzothiazol-2-yloxy)-phenyl]-ethyl}-piperidin-4-yl)-methanesulfonamide.

The 1-{2-[4-(benzothiazol-2-yloxy)-phenyl]-ethyl}-piperidin-4-ylamine hydrochloride was prepared from (1-{2-[4-(benzothiazol-2-yloxy)-phenyl]-ethyl}-piperidin-4-yl)-carbamic acid tert-butyl ester EXAMPLE 337 by removal of the Boc group according to EXAMPLE 266, step B. The free base was prepared according to EXAMPLE 343. The free base (240 mg, 0.68 mmol) in CH₂Cl₂ (7 mL) was treated with triethylamine (142 µL, 1.0 mmol) followed by methanesulfonyl chloride (77 µL, 1.0 mmol) and the solution stirred for 16 h. The reaction was diluted with CH₂Cl₂ (20 mL) and washed NaHCO₃ solution (30 mL). The organic layer was dried and concentrated under reduced pressure then purified on SiO₂ (12 g, 0-10% 2M NH₃/MeOH : CH₂Cl₂) to give 168 mg (57% yield) of the title compound. MS (ESI): exact mass calculated for C₂₁H₂₅N₃O₃S₂, 431.1; m/z found, 432.3 [M+H]⁺. ¹H NMR (400 MHz, CDCl₃): 7.73 (d, *J* = 8.0, 1H), 7.65 (dd, *J* = 7.9, 0.7, 1H), 7.42-7.36 (m, 1H), 7.29-7.24 (m, 5H), 4.45 (d, *J* = 7.6, 1H), 3.42-3.31 (m, 1H), 2.99 (s, 3H), 2.93 (br d, *J* = 12.0, 2H), 2.81 (dd, *J* = 10.6, 7.5, 2H), 2.60 (dd, *J* = 8.5, 5.4, 2H), 2.18 (t, *J* = 11.0, 2H), 2.02 (d, *J* = 12.5, 2H), 1.47 (dddd, *J* = 11.2, 11.1, 11.1, 3.7, 2H).

### EXAMPLE 346

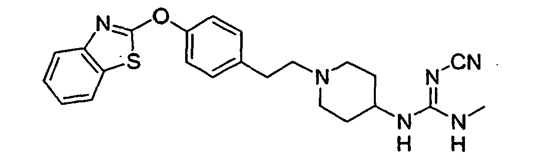

1-(1-{2-[4-(Benzothiazol-2-yloxy)-phenyl]-ethyl}-piperidin-4-yl)-3-cyano-2-methyl-guanidine.

A solution of 1-(1-{2-[4-(benzothiazol-2-yloxy)-phenyl]-ethyl}-piperidin-4-yl)-3-cyano-2-phenyl-isourea, EXAMPLE 343, (76 mg, 0.15 mmol) in ethanol (3 mL) was treated with a solution of 40% methylamine in water (0.2 ml) and the solution stirred at 80 °C for 1 h. The reaction was cooled to 23 °C and concentrated under reduced pressure to give a crude solid that was purified on SiO₂ (4 g, 0-10% 2M NH₃/MeOH : CH₂Cl₂) to give 50 mg (76% yield) of the title compound. MS (ESI): exact mass calculated for C₂₃H₂₆N₆O₁S₁, 434.2; m/z found, 435.4 [M+H]⁺. ¹H NMR (400 MHz, CDCl₃): 7.72 (d, *J*= 8.1, 1H), 7.67 (dd, *J* = 7.9, 0.7, 1H), 7.42-7.36 (m, 2H), 7.29-7.24 (m, 5H), 5.67 (br s, 1H), 4.75 (br s, 1H), 3.63 (br s, 1H), 2.94 (br d, *J* = 11.9, 2H), 2.86 (d, *J* = 4.9, 2H), 2.82 (dd, *J* = 10.4, 7.4, 2H), 2.61 (dd, *J* = 8.5, 5.5, 2H), 2.18 (t, *J* = 11.4, 2H), 2.05-1.96 (m, 2H), 2.61 (dddd, *J* = 12.3, 12.3, 12.3, 3.6, 2H).

### EXAMPLE 347

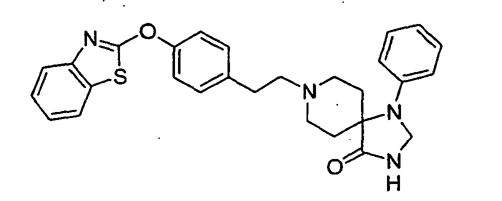

8-{2-[4-(Benzothiazol-2-yloxy)-phenyl]-ethyl}-1-phenyl-1,3,8-triaza-spiro[4.5]decan-4-one.

The title compound was prepared according to the procedure for EXAMPLE 262, step A using 1-phenyl-1,3,8-triaza-spiro[4.5]decan-4-one. MS (ESI): exact mass calculated for C₂₈H₂₈N₄O₂S₁, 484.2; m/z found, 485.4 [M+H]⁺. ¹H NMR (400 MHz, CDCl₃): 7.74 (dd, *J* = 8.0, 0.5, 1H), 7.66 (dd, *J* = 7.9, 0.7, 1H), 7.52 (s, 1H), 7.42-7.36 (m, 1H), 7.34-7.24 (m, 6H), 6.91 (d, *J* = 8.0, 1H), 6.87 (t, *J* = 7.3, 1H), 4.75 (s, 2H), 2.95-2.85 (m, 4H), 2.76-2.66 (m, 4H), 1.76 (d, *J* = 13.9, 2H).

### EXAMPLE 348

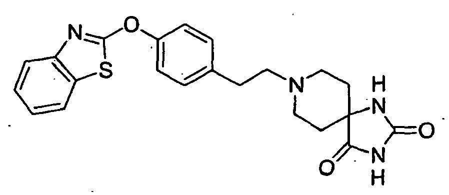

8-{2-[4-(Benzothiazol-2-yloxy)-phenyl]-ethyl}-1,3,8-triaza-spiro[4.5]decane-2,4-dione.

The title compound was prepared according to the procedure for EXAMPLE 262, step A using 1,3,8-triaza-spiro[4.5]decane-2,4-dione. MS (ESI): exact mass calculated for C₂₂H₂₂N₄O₃S₁, 422.1; m/z found, 423.4 [M+H]⁺. ¹H NMR (400 MHz, CDCl₃): 7.73 (dd, *J* = 8.1, 0.5, 1H), 7.67 (dd, *J* = 7.9, 1.1, 1H), 7.42-7.36 (m, 1H), 7.34-7.24 (m, 5H), 3.0 (ddd, *J* = 9.2, 4.3, 4.3, 2H), 2.86 (dd, *J* = 10.6, 6.9, 2H), 2.69 (dd, *J* = 9.0, 5.3, 2H), 2.72 (t, *J* = 10.2, 2H), 2.19-2.10 (m, 2H), 1.75 (d, *J* = 13.6, 2H).

### EXAMPLE 349

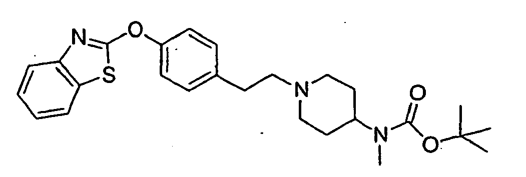

(1-{2-[4-(Benzothiazol-2-yloxy)-phenyl]-ethyl}-piperidin-4-yl)-methyl-carbamic acid tert-butyl ester.

The title compound was prepared according to the procedure for EXAMPLE 262, step A using methyl-piperidin-4-yl-carbamic acid tert-butyl ester. MS (ESI): exact mass calculated for C₂₆H₂₃N₃O₃S₁, 467.2; m/z found, 468.4 [M+H]⁺. ¹H NMR (400 MHz, CDCl₃): 7.73 (dd, *J* = 8.1, 0.5, 1H), 7.66 (dd, *J* = 7.9, 0.7, 1H), 7.41-7.36 (m, 1H), 7.29-7.25 (m, 5H), 4.10-3.95 (br m, 1H), 3.07 (d, *J* = 11.6, 2H), 2.83 (dd, *J* = 11.1, 7.8, 2H), 2.75 (s, 3H), 2.61 (d, *J* = 8.6, 5.2, 2H), 2.11 (t, *J* = 10.9, 2H), 2.19-2.10 (m, 2H), 1.77 (dddd, *J* = 12.1, 12.0, 12.0, 3.3, 2H), 1.70-1.64 (m, 2H), 1.47 (s, 9H).

### EXAMPLE 350

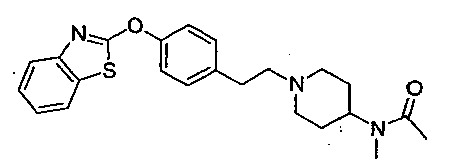

N-(1-{2-[4-(Benzothiazol-2-yloxy)-phenyl]-ethyl}-piperidin-4-yl)-N-methyl-acetamide.

The title compound was prepared from EXAMPLE 349 following EXAMPLE 266, step B, EXAMPLE 343 and EXAMPLE 345 using acetyl chloride. MS (ESI): exact mass calculated for C₂₃H₂₇N₃O₂S₁, 409.2; m/z found, 410.4 [M+H]⁺. ¹H NMR (400 MHz, CDCl₃): 7.73 (dd, *J* = 8.1, 0.6, 1H), 7.66 (dd, *J* = 8.0, 1.1, 1H), 7.41-7.36 (m, 1H), 7.31-7.25 (m, 5H), 4.53 (tt, *J* = 12.0, 4.2, 0.5H), 3.54 (tt, *J* = 11.7, 4.0, 0.5H), 3.15-3.04 (m, 2H), 2.90-2.78 (m, 5H), 2.70-2.58 (m, 2H), 2.24-2.06 (m, 5H), 2.00-1.96 (m, 1H), 1.82-1.60 (m, 3H).

### EXAMPLE 351

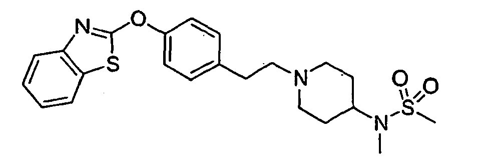

N-(1-{2-[4-(Benzothiazol-2-yloxy)-phenyl]-ethyl}-piperidin-4-yl)N-methyl-methanesulfonamide.

The title compound was prepared from EXAMPLE 349 following EXAMPLE 266, step B, EXAMPLE 343 and EXAMPLE 345 using methanesulfonyl chloride. MS (ESI): exact mass calculated for C₂₅H₂₉N₃O₄S, 467.2; m/z found, 468.4 [M+H]⁺. ¹H NMR (400 MHz, CDCl₃): 7.73 (d, *J* = 8.1, 1H), 7.66 (dd, *J* = 7.9, 0.7, 1H), 7.42-7.36 (m, 1H), 7.29-7.24 (m, 5H), 3.78 (tt, *J* = 11.9, 4.2, 1H), 3.08 (dd, *J* = 9.6, 2.1, 1H), 2.84 (s, 3H), 2.82 (s, 3H), 2.83-2.79 (m, 2H), 2.61 (dd, *J* = 8.5, 5.3, 2H), 2.18 (ddd, *J* = 11.8, 11.8, 2.2, 2H), 1.86 (dddd, *J* = 12.2, 12.0, 12.0, 3.4, 2H), 1.76-1.69 (m, 2H).

### EXAMPLE 352

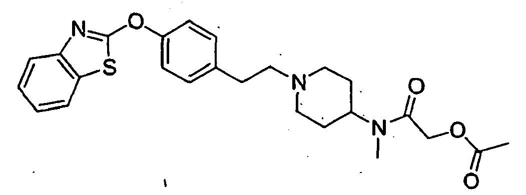

Acetic acid [(1-{2-[4-(benzothiazol-2-yloxy)phenyl]-ethyl}-piperidin-4-yl)-methylcarbamoyl]-methyl ester.

The title compound was prepared from EXAMPLE 349 following EXAMPLE 266, step B, EXAMPLE 343 and EXAMPLE 345 using acetic acid chlorocarbonylmethyl ester. MS (ESI): exact mass calculated for C₂₃H₂₇N₃O₂S₁, 467.2; m/z found, 468.4 [M+H]⁺. ¹H NMR (400 MHz, CDCl₃): 7.73 (dd, *J* = 8.1, 0.5, 1H), 7.66 (d, *J* = 7.9, 1H), 7.42-7.36 (m, 1H), 7.29-7.24 (m, 5H), 4.76 (s, 0.6H), 4.71 (s, 1.4H), 4.48 (tt, *J* = 12.0, 4.3, 0.7 H), 3.36 (tt, *J* = 11.6, 4.4, 0.3 H), 3.16-3.04 (m, 2H), 2.90-2.78 (m, 5H), 2.68-2.58 (m, 2H), 2.19 (s, 3H), 2.19-2.05 (m, 2H), 2.01-1.84 (m, 1H), 1.82-1.60 (m, 3H).

### EXAMPLE 353

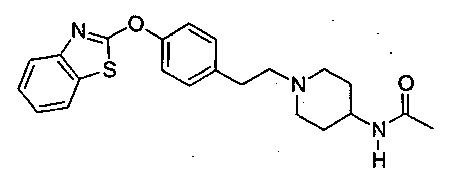

N-(1-{2-[4-(Benzothiazol-2-yloxy)-phenyl]-ethyl}-piperidin-4-yl)-N-acetamide.

The title compound was prepared from EXAMPLE 337 following EXAMPLE 266, step B, EXAMPLE 343 and EXAMPLE 345 using acetyl chloride. MS (ESI): exact mass calculated for C₂₅H₂₉N₃O₄S, 395.2; m/z found, 396.3 [M+H]⁺. ¹H NMR (400 MHz, CDCl₃): 7.73 (d, *J* = 8.0, 1 H), 7.66 (dd, *J* = 7.9, 0.6, 1H), 7.42-7.36 (m, 1H), 7.29-7.24 (m, 5H), 5.48 (d, *J* = 7.8, 1H), 3.87-3.77 (m 1H), 2.95 (dd, *J* = 11.6, 2H), 2.83 (dd, *J* = 10.8, 7.6, 2H), 2.62 (dd, *J* = 8.5, 5.3, 2H), 2.18 (d, *J* = 11.4, 2H), 2.00-1.94 (m, 2H), 1.98 (m, 2H), 1.49 (dddd, *J* = 12.3, 12.2, 12.2, 3.6, 2H).

### EXAMPLE 354

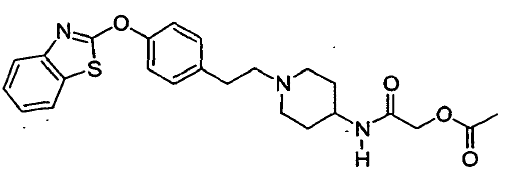

Acetic acid (1-{2-[4-(benzothiazol-2-yloxy)-phenyl]-ethyl}-piperidin-4-ylcarbamoyl)-methyl ester.

The title compound was prepared from EXAMPLE 337 according to EXAMPLE 266, step B, EXAMPLE 343 and EXAMPLE 354 using acetic acid chlorocarbonylmethyl ester. MS (ESI): exact mass calculated for C₂₄H₂₇N₃O₄S, 453.2; m/z found, 454.3 [M+H]⁺. ¹H NMR (400 MHz, CDCl₃): 7.73 (dd, *J* = 8.2, 0.6, 1H), 7.67 (dd, *J* = 7.9, 0.7, 1H), 7.42-7.36 (m, 1H), 7.29-7.24 (m, 5H), 6.05 (d, *J* = 8.0, 1H), 4.54 (s, 2H), 3.96-3.85 (m 1H), 3.00 (d, *J* = 8.2, 0.6, 2H), 2.85 (dd, *J* = 10.6, 7.4, 2H), 2.85 (dd, *J* = 10.6, 7.4, 2H), 2.66 (dd, *J* = 8.6, 5.3, 2H), 2.23 (t, *J* = 11.4, 2H), 2.18 (s, 3H), 1.98 (d, *J* = 9.7, 2H), 1.57 (dddd, *J* = 12.4, 12.4, 12.3, 3.6, 2H).

### EXAMPLE 355

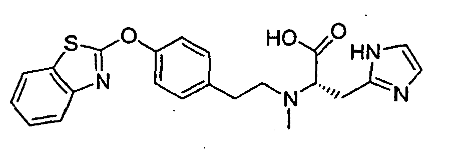

(*S*)-2-({2-[4-(Benzothiazol-2-yloxy)-phenyl]-ethyl}-methyl-amino)-3-(1H-imidazol-2-yl)-propionic acid

The title compound was prepared according to the procedure for EXAMPLE 262, step A using (*S*)-3-(1H-imidazol-2-yl)-2-methylamino-propionic acid. MS (ESI): exact mass calculated for C₂₂H₂₂N₄O₃S₁, 422.1; m/z found, 423.3 [M+H]⁺. ¹H NMR (400 MHz, CD₃OD): 8.85, (s, 1H), 7.81 (dd, *J* = 7.9, 0.6, 1H), 7.72 (dd, *J* = 8.1, 0.5, 1H), 7.58-7.42 (m, 4H), 7.39-7.34 (m, 3H), 4.34, (dd, *J* = 9.3, 4.9, 3H), 3.71-3.42, (m, 4H), 3.35-3.32 (m, 2H), 3.20 (d, *J* = 8.6, 2H), 3.08 (s, 3H).

### EXAMPLE 356

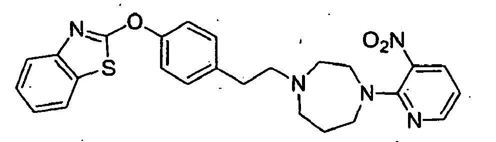

2-(4-{2-[4-(3-Nitro-pyridin-2-yl)-[1,4]diazepan-1-yl]-ethyl}-phenoxy)-benzothiazole.

The title compound was prepared according to the procedure for EXAMPLE 262, step A using 1-(3-nitro-pyridin-2-yl)-[1,4]diazepane. MS (ESI): exact mass calculated for C₂₅H₂₅N₅O₃S₁, 475.2; m/z found, 476.1 [M+H]⁺. ¹H NMR (400 MHz, CDCl₃): 8.36 (dd, *J* = 4.5, 1.7, 1H), 8.14 (dd, *J* = 8.1, 1.7, 1H), 7.77 (dd, *J* = 8.1, 0.5, 1H), 7.70 (dd, *J* = 8.0, 0.7, 1H), 7.45-7.40 (m, 1H), 7.29-7.24 (m, 5H), 6.73 (dd, *J* = 8.0, 4.4, 1H), 3.80-3.75 (br s, 2H), 3.47-3.40 (br s, 2H), 3.07-3.01 (br s, 2H), 2.95-2.88 (m, 2H), 2.86-2.79 (m, 4H), 2.20-2.10 (br s, 2H).

### EXAMPLE 357

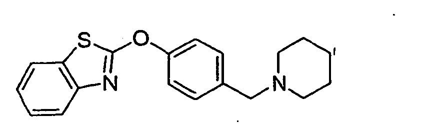

2-(4-Piperidin-1-ylmethyl-phenoxy)-benzothiazole.

The title compound was prepared according to the procedure for EXAMPLE 251, step B using piperidine. MS (ESI): mass calculated for C₁₉H₂₀N₂OS, 324.4; m/z found, 325.3 [M+H]⁺. ¹H NMR (400 MHz, CDCl₃): 7.75 (d, *J* = 8.2, 1H), 7.73 (d, *J* = 8.2, 1H), 7.36-7.42 (m, 3H), 7.24-7.32 (m, 3H), 3.49 (s, 2H), 2.39 (br s, 3H), 1.56-1.64 (m, 5H), 1.40-1.48 (m, 2H).

### EXAMPLE 358

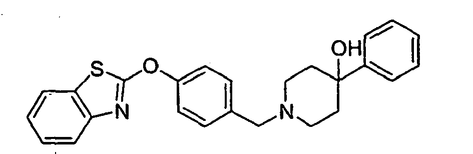

1-[4-(Benzothiazol-2-yloxy)-benzyl]-4-phenyl-piperidin-4-ol.

The title compound was prepared according to the procedure for EXAMPLE 251, step B using 4-phenyl-piperidin-4-ol. MS (ESI): mass calculated for C₂₅H₂₄N₂O₂S, 416.5; m/z found, 417.4 [M+H]⁺. ¹H NMR (400 MHz, CDCl₃): 7.75 (d, *J* = 8.0, 1 H), 7.66 (d, *J* = 8.0, 1H), 7.55-7.51 (m, 2H), 7.47-7.25 (m, 9H), 3.63 (s, 2H), 2.85-2.77 (m, 2H), 2.50 (dt, *J* = 11.8, 2.5, 2H), 2.18 (dt, *J* = 13.0, 4.4, 2H), 1.80-1.74 (m, 2H), 1.55 (br s, 1H).

### EXAMPLE 359

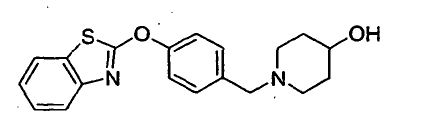

1-[4-(Benzothiazol-2-yloxy)-benzyl]-piperidin-4-ol.

The title compound was prepared according to the procedure for EXAMPLE 251, step B using 4-piperidinol. MS (ESI): mass calculated for C₁₉H₂₀N₂O₂S, 340.4; m/z found, 341.3 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 3.68-3.64 (m, 1H), 3.46 (s, 2H), 2.81-2.25 (m, 2H), 2.20-2.15 (m, 2H), 1.95-1.90 (m, 2H), 1.67-1.58 (m, 3H).

### EXAMPLE 360

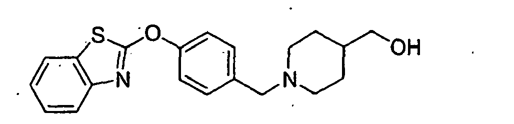

{1-[4-(Benzothiazol-2-yloxy)-benzyl]-piperidin-4-yl}-methanol.

The title compound was prepared according to the procedure for EXAMPLE 251, step B using piperidin-4-yl-methanol. MS (ESI): mass calculated for C₂₀H₂₂N₂O₂S, 354.5; m/z found, 355.3 [M+H]⁺. ¹H NMR (400 MHz, CDCl₃): 7.74 (d, *J* = 8.0, 1H), 7.67 (d, *J* = 8.0, 1H), 7.42-7.37 (m, 3H), 7.32-7.25 (m, 3H), 3.54 (s, 2H), 3.51 (d, *J* = 6.5, 2H), 2.97-2.90 (m, 2H), 2.01 (dt, *J* = 11.6, 2.4, 3H), 1.73 (d, *J* = 12.1, 2H), 1.58-1.47 (m, 1H), 1.37-1.26 (m, 2H).

### EXAMPLE 361

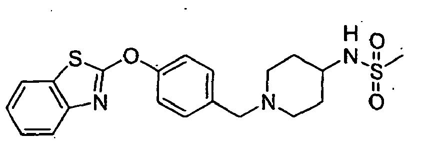

N-{1-[4-(Benzothiazol-2-yloxy)-benzyl]-piperidin-4-yl}-methanesulfonamide.

The title compound was prepared according to the procedure for EXAMPLE 253, step A and B followed by EXAMPLE 258 step C. MS (ESI): mass calculated for C₂₀H₂₃N₃O₃S₂, 417.6; m/z found, 418.3 [M+H]⁺. ¹H NMR (400 MHz, CDCl₃): 7.74 (d, *J* = 8.0, 1H), 7.68 (d, *J* = 8.0, 1H), 7.42-7.36 (m, 3H), 7.35-7.25 (m, 3H), 4.32-4.5 (m, 1H), 3.58 (s, 2H), 3.43-3.32 (m, 1H), 2.99 (s, 3H), 2.87-2.80 (m, 2H), 2.18-2.10 (m, 2H), 2.04-1.96 (m, 2H), 1.64-1.54 (m, 2H).

### EXAMPLE 362

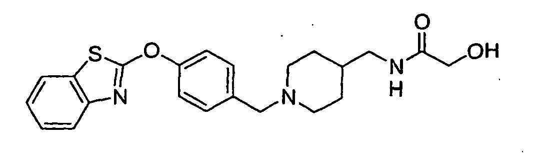

N-{1-[4-(Benzothiazol-2-yloxy)-benzyl]-piperidin-4-ylmethyl}-2-hydroxy-acetamide.

The title compound was prepared according to the procedure for EXAMPLE 258, step A and B followed by EXAMPLE 253 step C using glycolic acid. MS (ESI): mass calculated for C₂₂H₂₅N₃O₃S, 411.5; m/z found, 412.4 [M+H]⁺. ¹H NMR (400 MHz, CDCl₃): 7.74 (d, *J* = 8.2, 1H), 7.67 (d, *J* = 8.2, 1H), 7.42-7.37 (m, 3H), 7.32-7.25 (m, 3H), 4.11 (s, 2H), 3.51 (s, 2H), 3.24 (t, *J* = 6.3, 2H), 2.88-2.94 (m, 2H), 2.20 (d, *J* = 11.5, 1H), 2.02-1.92 (m, 2H), 1.72-1.65 (m, 2H), 1.62-1.50 (m, 2H), 1.36-1.26 (m, 2H).

### EXAMPLE 363

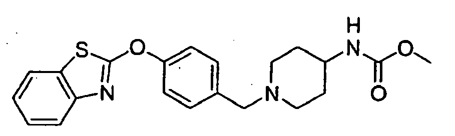

{1-[4-(Benzothiazol-2-yloxy)-benzyl]-piperidin-4-yl}-carbamic acid methyl ester.

The title compound was prepared according to the procedure for EXAMPLE 253, step A and B followed by EXAMPLE 258 step C using methyl isocyanate. MS (ESI): mass calculated for C₂₁H₂₃N₃O₃S, 397.5; m/z found, 398.3 [M+H]⁺. ¹H NMR (400 MHz, CDCl₃): 7.74 (d, *J* = 8.0, 1H), 7.67 (d, J = 8.0, 1H), 7.42-7.36 (m, 3H), 7.32-7.24 (m, 3H), 4.57 (br s, 1H), 3.66 (s, 3H), 3.51 (s, 2H), 2.85-2.79 (m, 2H), 2.18-2.09 (m, 2H), 1.98-1.90 (m, 2H), 1.51-1.40 (m, 2H).

### EXAMPLE 364

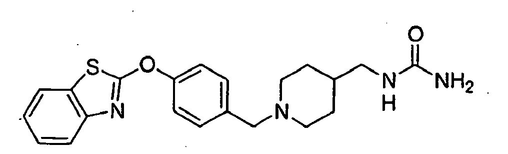

{1-[4-(Benzothiazol-2-yloxy)-benzyl]-piperidin-4-ylmethyl}-urea.

The title compound was prepared according to the procedure for EXAMPLE 258, step A, B and C using trimethylsilyl isocyanate. MS (ESI): mass calculated for C₂₁H₂₄N₄O₂S, 396.5; m/z found, 397.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 4.64 (br s, 1H), 4.35 (s, 2H), 3.50 (s, 2H), 3.07 (br t, *J* = 6.3, 2H), 2.94-2.88 (m, 2H), 1.86-2.01 (m, 2H), 1.72-1.66 (m, 2H), 1.56-1.44 (s, 1H), 1.24-1.23 (m, 2H).

### EXAMPLE 365

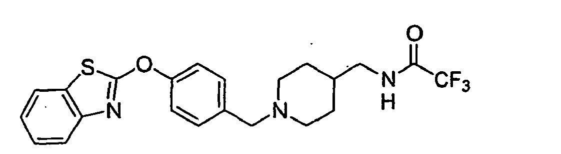

N-{1-[4-(Benzothiazol-2-yloxy)-benzyl]-piperidin-4-ylmethyl}-2,2,2-trifluoroacetamide

The title compound was prepared according to the procedure for EXAMPLE 258, step A and B and EXAMPLE 257, step C using trifluoroacetic acid. MS (ESI): mass calculated for C₂₂H₂₂F₃N₃O₂S, 449.5; m/z found, 450.4 [M+H]⁺. ¹H NMR (400 MHz, CDCl₃): 7.74 (d, *J* = 8.0, 1H), 7.67 (d, *J* = 8.0, 1H), 7.42-7.36 (m, 3H), 7.32-7.24 (m, 3H), 6.41 (br s, 1H), 3.51 (s, 2H), 3.28 (t, *J* = 6.46, 2H), 2.92 (d, *J* = 11.5, 2H), 2.02-1.94 (m, 2H), 1.74-1.54 (m, 3H), 1.38-1.27 (m, 2H).

### EXAMPLE 366

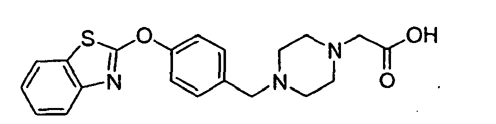

{4-[4-(Benzothiazol-2-yloxy)-benzyl]-piperazin-1-yl}-acetic acid.

The title compound was prepared according to the procedure for EXAMPLE 259, step C and D and EXAMPLE 250, step D using piperazin-1-yl-acetic acid. MS (ESI): mass calculated for C₂₀H₂₁N₃O₃S, 383.5; m/z found, 384.4 [M+H]⁺. ¹H NMR (400 MHz, CD₃OD): 7.77 (d, *J* = 8.0, 1H), 7.62 (d, *J* = 8.0, 1H), 7.50-7.46 (m, 2H), 7.42-7.26 (m, 4H), 6.67 (s, 2H), 3.60 (s, 2H), 3.34 (br s, 4H), 2.77 (br s, 4H).

### EXAMPLE 367

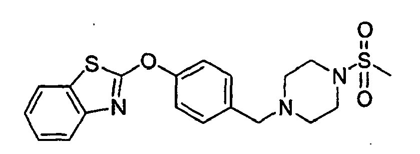

2-[4-(4-Methanesulfonyl-piperazin-1-ylmethyl)phenoxy]-benzothiazole.

The title compound was prepared according to the procedure for EXAMPLE 257, step A and B and EXAMPLE 258 step C. MS (ESI): mass calculated for C₁₉H₂₁N₃O₃S₂, 403.53; m/z found, 404.3 [M+H]⁺. ¹H NMR (400 MHz, CDCl₃): 7.74 (d, *J* = 8.0, 1 H), 7.68 (d, *J* = 8.0, 1H), 7.42-7.36 (m, 3H), 7.35-726 (m, 3H), 3.57 (s, 2H), 3.26 (br t, *J* = 4.7, 4H), 2.79 (s, 3H), 2.58 (br t, *J* = 4.7, 4H).

### EXAMPLE 368

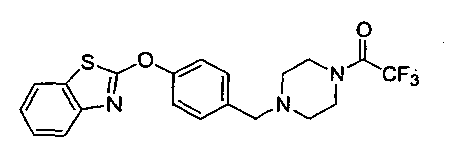

1-{4-[4-(Benzothiazol-2-yloxy)-benzyl]-piperazin-1-yl}-2,2,2-trifluoro-ethanone.

The title compound was prepared according to the procedure for EXAMPLE 257, step A, B and C using trifluoroacetic acid. MS (ESI): mass calculated for C₂₀H₁₈F₃N₃O₂S, 421.44; m/z found, 422.4 [M+H]⁺. ¹H NMR (400 MHz, CDCl₃): 7.74 (d, *J* = 8.0, 1H), 7.68 (d, *J* = 8.0, 1H), 7.42-7.27 (m, 6H), 3.74-3.70 (m, 2H), 3.65-3.60 (m, 2H), 3.57 (s, 2H), 2.56-2.50 (m, 4H).

### EXAMPLE 369

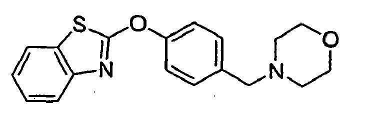

2-(4-Morpholin-4-ylmethyl-phenoxy)-benzothiazole.

The title compound was prepared according to the procedure for EXAMPLE 259, step C and D using morpholine. MS (ESI): mass calculated for C₁₈H₁₈N₂O₂S, 326.42; m/z found, 327.4 [M+H]⁺. ¹H NMR (500 MHz, CDCl₃): 7.74 (d, *J* = 7.7, 1H), 7.67 (d, *J* = 7.7, 1H), 7.42-7.37 (m, 3H), 7.33-7.25 (m, 3H), 3.72 (t, *J* = 3.7, 4H), 3.52 (s, 2H), 2.46 (br s, 4H).

### EXAMPLE 370

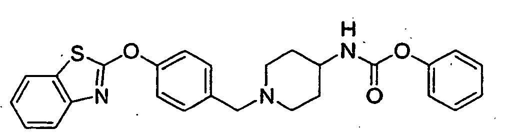

{1-[4-(Benzothiazo)-2-yloxy)-benzyl]-piperidin-4-yl}-carbamic acid phenyl ester.

The title compound was prepared according to the procedure for EXAMPLE 253, step A and B followed by EXAMPLE 258 step C using phenyl isocyanate. MS (ESI): mass calculated for C₂₆H₂₅N₃O₃S, 459.57; m/z found, 460.4 [M+H]⁺. ¹H NMR (400 MHz, CDCl₃): 7.74 (d, *J* = 8.0, 1H), 7.67 (d, *J* = 8.0, 1H), 7.42-7.10 (m, 11H), 4.95 (br d, *J* = 7.8, 1H), 3.67-3.58 (m, 1H), 3.53 (s, 2H), 2.88-2.82 (m, 2H), 2.21-2.06 (m, 2H), 2.03-1.99 (m, 2H), 1.61-1.44 (m, 2H).

### EXAMPLE 371

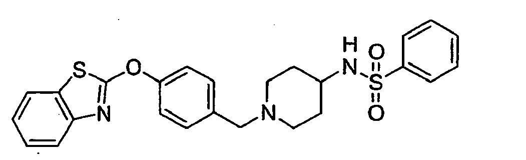

N-{1-[4-(Benzothiazol-2-yloxy)-benzyl]-piperidin-4-yl}benzenesulfonamide.

The title compound was prepared according to the procedure for EXAMPLE 253, step A and B followed by EXAMPLE 258, step C using benzenesulfonyl chloride. MS (ESI): mass calculated for C₂₅H₂₅N₃O₃S₂, 479.62; m/z found, 480.4 [M+H]⁺. ¹H NMR (400 MHz, CDCl₃): 7.86-7.84 (m, 2H), 7.75 (d, *J* = 8.0, 1H), 7.67 (d, *J* = 8.0, 1H), 7.60-7.48 (m, 3H), 7.41-7.24 (m, 6H), 4.56 (br d, *J* = 7.8, 1H), 3.48 (s, 2H), 3.26-3.16 (m, 1H), 2.76-2.68 (m, 2H), 2.06-1.97 (m, 2H), 1.78-1.70 (m, 1H), 1.52-1.42 (m, 2H).

### EXAMPLE 372

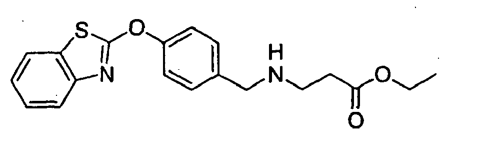

3-[4-(Benzothiazol-2-yloxy)-benzylamino]-propionic acid ethyl ester.

The title compound was prepared according to the procedure for EXAMPLE 251, step A and B using 3-amino-propionic acid ethyl ester. MS (ESI): mass calculated for C₁₉H₂₀N₂O₃S, 356.45; m/z found, 357.3 [M+H]⁺. ¹H NMR (400 MHz, CDCl₃): 7.74 (d, *J* = 8.0, 1H), 7.67 (d, *J* = 8.0, 1H), 7.44-7.25 (m, 6H), 4.21 (br s, 1H), 4.15 (q, *J* = 7.2, 2H), 3.87 (s, 2H), 2.95 (t, *J* = 6.3, 2H), 2.58 (t, *J* = 6.3, 2H), 1.26 (t, *J* = 7.2, 3H).

### EXAMPLE 373

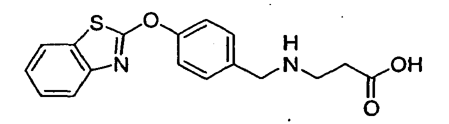

3-[4-(Benzothiazol-2-yloxy)-benzylamino]-propionic acid.

The title compound was prepared according to the procedure for EXAMPLE 372, and EXAMPLE 250, step D. MS (ESI): mass calculated for C₁₇H₁₆N₂O₃S, 328.39; m/z found, 329.3 [M+H]⁺. ¹H NMR (400 MHz, DMSO): 7.87 (d, *J* = 8.0, 1H), 7.62 (d, *J* = 8.0, 1H), 7.44-7.23 (m, 6H), 3.77 (s, 2H), 2.72 (t, *J* = 6.6, 2H), 2.29 (t, *J* = 6.6, 2H).

### EXAMPLE 374

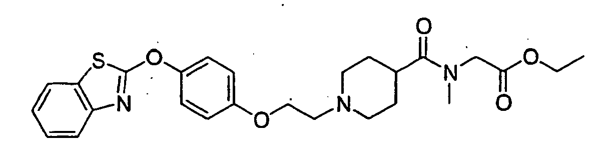

[(1-{2-[4-(Benzothiazol-2-yloxy)-phenoxy]-ethyl}-piperidine-4-carbonyl)-methylamino]-acetic acid ethyl ester.

The title compound was prepared according to the procedure for EXAMPLE 18, step A and EXAMPLE 20 using 3-methylamino-propionic acid ethyl ester. MS (ESI): mass calculated for C₂₆H₃₁N₃O₅S, 497.62; m/z found, 498.4 [M+H]⁺. ¹H NMR (400 MHz, CDCl₃): 7.73 (d, *J* = 8.0, 1H), 7.65 (d, *J* = 8.0, 1H), 7.41-7.35 (m, 1H), 7.28-7.23 (m, 3H), 6.99-6.94 (m, 2H), 4.28-4.08 (m, 5H), 3.12 (s, 3H), 3.10-3.04 (m, 1H), 2.98-2.81 (m, 4H), 2.62-2.53 (m, 1H), 2.24-2.12 (m, 2H), 1.97-1.83 (m, 2H), 1.81-1.64 (m, 2H), 1.32-1.24 (m, 3H).

### EXAMPLE 376

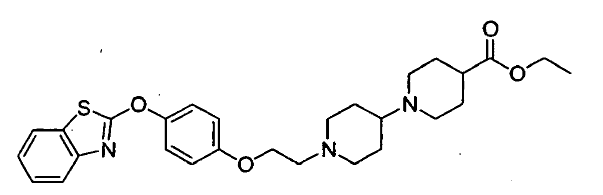

1'-{2-[4-(Benzothiazol-2-yloxy)-phenoxy]-ethyl}-[1,4']bipiperidinyl-4-carboxylic acid ethyl ester.

The title compound was prepared according to the procedure for EXAMPLE 9 and EXAMPLE 24 using [1,4']bipiperidinyl-4-carboxylic acid ethyl ester. MS (ESI): mass calculated for C₂₈H₃₅N₃O₄S, 509.67; m/z found, 510.4 [M+H]⁺. ¹H NMR (400 MHz, CDCl₃): 7.73 (d, *J* = 8.0, 1H), 7.65 (d, *J* = 8.0, 1H), 7.41-7.35 (m, 1H), 7.29-7.23 (m, 3H), 6.99-6.92 (m, 2H), 4.16-4.08 (m, 4H), 3.10-3.04 (m, 2H), 2.92-2.86 (m, 2H), 2.80 (t, *J* = 5.9, 1H), 2.36-2.20 (m, 4H), 2.16-2.07 (m, 2H), 1.95-1.87 (m, 2H), 1.82-1.57 (m, 7H), 1.24 (t, *J* = 7.0, 3H).

### EXAMPLE 377

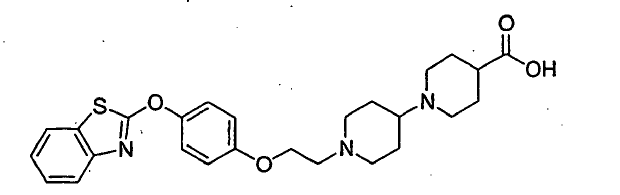

1'-{2-[4-(Benzothiazol-2-yloxy)-phenoxy]-ethyl}-[1,4']bipiperidinyl-4-carboxylic acid.

The title compound was prepared according to the procedure for EXAMPLE 376 and EXAMPLE 250, step D. MS (ESI): mass calculated for C₂₆H₃₁N₃O₄S, 481.62; m/z found, 482.3 [M+H]⁺. ¹H NMR (400 MHz, CDCl₃): 7.76 (d, *J* = 8.0, 1H), 7.64 (d, *J* = 8.0, 1H), 7.44-7.38 (m, 1H), 7.32-7.27 (m, 3H), 7.08-7.03 (m, 2H), 4.17 (t, *J* = 5.4, 2H), 3.44-3.36 (m, 2H), 3.24-3.19 (m, 2H), 3.05-2.84 (m, 5H), 2.40-2.20 (m, 3H), 2.14-2.04 (m, 4H), 1.86-1.72 (m, 4H).

### EXAMPLE 378

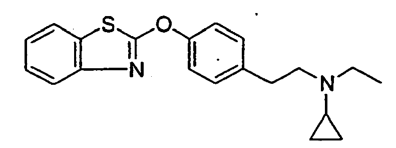

{2-[4-(Benzothiazol-2-yloxy)-phenyl]-ethyl}-cyclopropyl-ethyl-amine.
A. {2-[4-(Benzothiazol-2-yloxy)-phenyl]-ethyl}-cyclopropyl-amine. A mixture of [4-(benzothiazol-2-yloxy)-phenyl]-acetaldehyde (Example 262 step A, 4.2 g, 15.6 mmol), cyclopropylamine (5.4 mL, 78.0 mmol) and acetic acid (4.5 mL, 78 mmol) in CH₂Cl₂ (156 mL) was stirred at room temperature for 1 h. To the resulting mixture was added NaBH(OAc)₃ (6.61 g, 31.2 mmol). The mixture was stirred at room temperature for 24 h, filtered through diatomaceous earth, rinsed with CH₂Cl₂ (200 mL), washed with 1N NaOH (3 x 50 mL), and concentrated under reduced pressure to yield the crude product as a yellow oil. The crude product was purified on SiO₂ (330 g; 0-10% CH₃OH/CH₂Cl₂) to give a clear oil which crystallized on standing (3.4 g, 71% yield). MS (ESI): mass calculated for C₁₈H₁₈N₂OS, 310.11; m/z found, 311.2 [M+H]⁺. ¹H NMR (400 MHz, CDCl₃): 7.76 (d, *J* = 8.1, 1H), 7.62 (d, *J* = 8.1, 1H), 7.46-7.27 (m, 6H), 3.41 (t, *J* = 7.6, 2H), 3.08 (t, *J* = 7.6, 2H), 2.38-2.76 (m, 1H), 0.95-0.89 (m, 4H).
B. {2-[4-(Benzothiazol-2-yloxy)-phenyl]-ethyl}-cyclopropyl-ethyl-amine. A mixture of acetaldehyde (180 µL, 3.2 mmol), {2-[4-(benzothiazol-2-yloxy)-phenyl]-ethyl}-cyclopropyl-amine (500 mg, 1.6 mmol) in CH₂Cl₂ (32 mL) was stirred at room temperature for 1 h. To the resulting mixture was added NaBH(OAc)₃ (1.02 g, 4.8 mmol). The mixture was stirred at room temperature for 24 h, filtered through diatomaceous earth, rinsed with CH₂Cl₂ (100 mL), and concentrated under reduced pressure to yield the crude product as a yellow oil. The crude product was purified on SiO₂ (40 g; 0-10% CH₃OH/CH₂Cl₂) to give a clear oil (465 mg, 86% yield). MS (ESI): mass calculated for C₂₀H₂₂N₂OS, 338.15; m/z found, 339.3 [M+H]⁺. ¹H NMR (400 MHz, CDCl₃): 7.66 (d, *J* = 8.1, 1H), 7.58 (d, *J* = 8.1, 1H), 7.31 (t, *J* = 8.1, 1H), 7.23-7.15 (m, 5H), 2.80 (s, 4H), 2.72 (dd, *J* = 7.3, 7.1, 2H), 1.78-1.70 (m, 1H), 1.05 (t, *J* = 7.1, 3H), 0.50-0.36 (m, 4H).

### EXAMPLE 379

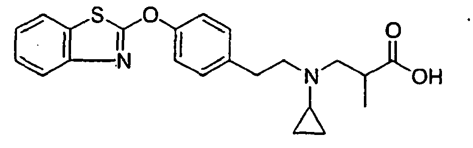

3-({2-[4-(Benzothiazol-2-yloxy)-phenyl]-ethyl}-cyclopropyl-amino)-2-methyl-propionic acid trifluoromethansulfonic acid salt.
A. 3-({2-[4-(Benzothiazol-2-yloxy)-phenyl]-ethyl}-cyclopropyl-amino)-2-methyl-propionic acid methyl ester. To a solution of {2-[4-(benzothiazol-2-yloxy)-phenyl]-ethyl}-cyclopropyl-amine (500 mg, 1.6 mmol) in CH₃CN (10 mL) at room temperature was added *N,N*-diisopropylethylamine (349 µL, 2 mmol), followed by 3-bromo-2-methyl-propionic acid methyl ester (362 mg, 2 mmol). The resulting mixture was heated at 60 °C overnight. The mixture was cooled and dissolved in CH₂Cl₂ (100 mL), washed with sat. aq. NaHCO₃ (1 x 25 mL) and H₂O (2 x 25 mL), dried (Na₂SO₄) and concentrated under reduced pressure to yield the crude product as a off-white solid. The crude product was purified on SiO₂ (40 g; 0-10% CH₃OH/CH₂Cl₂) to give a pale off-white solid (158 mg, 39% yield). MS (ESI): mass calculated for C₂₃H₂₆N₂O₃S, 410.17; m/z found, 411.3 [M+H]⁺. ¹H NMR (400 MHz, CD₃OD): 7.73 (d, *J* = 8.3, 1H), 7.65 (d, *J* = 8.3, 1H), 7.38 (t, *J* = 8.1, 1H), 7.28-7.23 (m, 5H), 3.66 (s, 3H), 3.01 (dd, *J* = 8.8, 3.8, 1H), 2.89-2.76 (m, 5H), 3.60 (dd, *J* = 6.6, 6.1, 1 H), 1.84-1.78 (m, 1H), 1.12 (d, *J* = 7.1, 3H), 0.51-0.30 (m, 4H).
B. 3-({2-[4-(Benzothiazol-2-yloxy)-phenyl]-ethyl}-cyclopropyl-amino)-2-methyl-propionic acid trifluoromethansulfonic acid salt. To a solution of 3-({2-[4-(benzothiazol-2-yloxy)-phenyl]-ethyl}-cyclopropyl-amino)-2-methyl-propionic acid methyl ester (158 mg, 0.38 mmol) in 3:1:1 THF/CH₃OH (13 mL), was added a solution of lithium hydroxide (37 mg, 1.54 mmol) in H₂O (3 mL). The reaction solution was stirred at room temperature for 16 h and then concentrated under reduced pressure. The residue was dissolved in CH₃OH and purified by reversed-phase HPLC. The desired fractions were collected and concentrated under reduced pressure, giving the the title compound as a clear oil (179 mg, 92% yield). MS (ESI): mass calculated for C₂₂H₂₄N₂O₃S, 396.15; m/z found, 397.3 [M+H]⁺. ¹H NMR (400 MHz, CD₃OD): 7.78 (d, *J* = 8.3, 1H), 7.64 (d, *J* = 8.1, 1H), 7.51-7.27 (m, 6H), 3.83 (t, *J* = 12.4, 1H), 3.55 (t, *J* = 8.1, 2H), 3.37 (d, *J* = 13.1, 1H), 3.25 (t, *J* = 8.8, 2H), 3.21-3.11 (m, 1H), 2.99-2.90 (m, 1H), 1.33 (d, *J* = 7.3, 3H), 1.17-0.97 (m, 4H).

### EXAMPLE 380

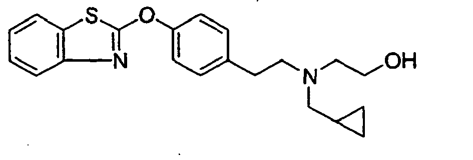

2-({2-[4-(Benzothiazol-2-yloxy)-phenyl]-ethyl}-cyclopropylmethyl-amino)-ethanol.

The title compound was prepared according to the procedure for EXAMPLE 378, step A using cyclopropyl-methylamine and EXAMPLE 379, step A using 2-bromoethanol. MS (ESI): mass calculated for C₂₁H₂₄N₂O₂S, 368.16; m/z found, 369.3 [M+H]⁺. ¹H NMR (400 MHz, CDCl₃): 7.70 (d, *J* = 8.1, 1H), 7.62 (d, *J* = 8.1, 1H), 7.34 (t, *J* = 7.3, 1H), 7.27-7.18 (m, 5H), 3.56 (t, *J* = 5.3, 2H), 2.92-2.86 (m, 2H), 2.84-2.73 (m, 4H), 2.49 (d, *J* = 6.6, 2H), 0.92-0.81 (m, 1H), 0.55-0.49 (m, 2H), 0.16-0.09 (m, 2H).

### EXAMPLE 381

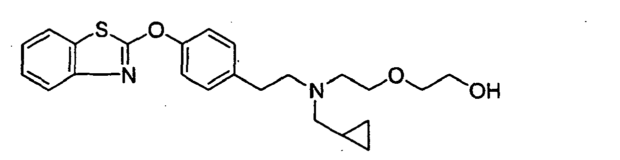

2-[2-({2-[4-(Benzothiazol-2-yloxy)-phenyl]-ethyl}-cyclopropylmethyl-amino)-ethoxy]-ethanol.

The title compound was prepared according to the procedure for EXAMPLE 380. MS (ESI): mass calculated for C₂₃H₂₈N₂O₃S, 412.18; m/z found, 413.3 [M+H]⁺. ¹H NMR (400 MHz, CDCl₃): 7.64 (d, *J* = 8.3, 2H), 7.41 (d, *J* = 8.3, 2H), 7.31 (t, *J* = 8.3, 1H), 7.25-7.17 (m, 3H), 4.08 (br s, 1H), 3.84-3.68 (m, 6H), 3.59-3.49 (m, 2H), 3.26-3.16 (m, 2H), 1.11-0.99 (m, 1H), 0.79-0.70 (m, 2H), 0.55-0.48 (m, 2H).

### EXAMPLE 382

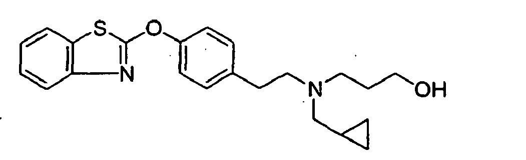

3-({2-[4-(Benzothiazol-2-yloxy)-phenyl]-ethyl}-cyclopropylmethyl-amino)-propan-1-ol.

The title compound was prepared according to the procedure for EXAMPLE 378, steps A using cyclopropyl-methylamine and EXAMPLE 379, step A using 3-bromo-propan-1-ol. MS (ESI): mass calculated for C₂₂H₂₆N₂O₂S, 382.17; m/z found, 383.3 [M+H]⁺. ¹H NMR (400 MHz, CDCl₃): 7.70 (d, *J* = 8.1, 1H), 7.62 (d, *J* = 8.1, 1H), 7.34 (t, *J* = 8.1, 1H), 7.27-7.18 (m, 5H), 3.77 (t, *J* = 5.3, 2H), 2.84-2.76 (m, 6H), 2.43 (d, *J* = 6.6, 2H), 1,72-1,65 (m, 2H), 0.94-0.83 (m, 1H), 0.57-0.49 (m, 2H), 0.18-0.11 (m, 2H).

### EXAMPLE 383

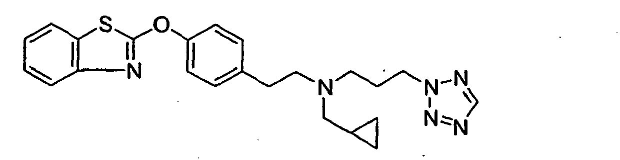

{2-[4-(Benzothiazol-2-yloxy)-phenyl]-ethyl}-cyclopropylmethyl-(3-tetrazol-2-yl-propyl)-amine trifluoromethansulfonic acid salt.

To a solution of 3-({2-[4-(benzothiazol-2-yloxy)-phenyl]-ethyl}-cyclopropylmethyl-amino)-propan-1-ol (Example 382, 220 mg, 0.58 mmol) and 2H-tetrazole (61 mg, 0.87 mmol) in CH₂Cl₂ (12 mL) was added polymer-supported triphenylphosphine (290 mg, 0.87 mmol) and di-tert-butyl azodicarboxylate (200 mg, 0.87 mmol). The reaction mixture was stirred at room temperature for 1 h, filtered and the collected solids were washed with CH₂Cl₂ (10 mL). The filtrate was concentrated under reduced pressure to yield the crude product as a light yellow oil. The crude product was dissolved in CH₃OH and purified by reversed-phase HPLC. The desired fractions were collected and concentrated under reduced pressure, giving the title compound as a clear oil (100 mg, 40% yield). MS (ESI): mass calculated for C₂₃H₂₆N₆OS, 434.19; m/z found, 435.3 [M+H]⁺. ¹H NMR (400 MHz, CDCl₃): 8.75 (s, 1H), 7.75 (d, *J* = 8.1, 1H), 7.60 (d, *J* = 8.1, 1H), 7.45-7.32 (m, 5H), 7.28 (t, *J* = 8.1, 1H), 3.54-3.40 (m, 4H), 3.29-3.25 (m, 2H), 3.19 (d, *J* = 7.3, 2H), 3.09 (t, *J* = 8.6, 2H), 2.56-2.47 (m, 2H), 1.16-1.05 (m, 1H), 0.78-0.71 (m, 2H), 0.46-0.40 (m, 2H).

### EXAMPLE 384

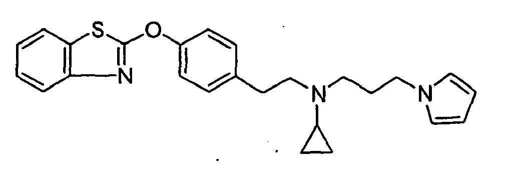

{2-[4-(Benzothiazol-2-yloxy)-phenyl]-ethyl}-cyclopropyl-(3-pyrrol-1-yl-propyl)-amine.

The title compound was prepared according to the procedure for EXAMPLE 379, step A using 1-(3-bromo-propyl)-1H-pyrrole. MS (ESI): mass calculated for C₂₅H₂₇N₃OS, 417.19; m/z found, 418.3 [M+H]⁺. ¹H NMR (400 MHz, CDCl₃): 7.65 (d, *J* = 7.6, 1H), 7.57 (d, *J* = 8.1, 1H), 7.30 (t, *J* = 8.1, 1H), 7.21-7.13 (m, 5H), 6.57 (t, *J* = 2.0, 2H), 6.07 (t, *J* = 2.0, 2H), 3.80 (t, *J* = 7.1, 2H), 2.80-2.69 (m, 4H), 2.57 (t, *J* = 7.1, 2H), 1.98-1.88 (m, 2H), 1.74-1.67 (m, 1H), 0.47-0.40 (m, 2H), 0.37-0.31 (m, 2H).

### EXAMPLE 385

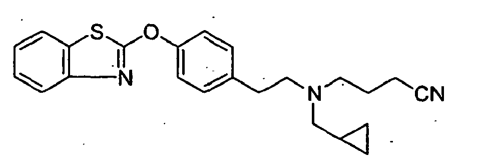

4-({2-[4-(Benzothiazol-2-yloxy)-phenyl]-ethyl}-cyclopropylmethyl-amino)-butyronitrile.

The title compound was prepared according to the procedure for EXAMPLE 378, step A using cyclopropyl-methylamine and EXAMPLE 319, step A using 4-bromo-butyronitrile. MS (ESI): mass calculated for C₂₃H₂₅N₃OS, 391.17; m/z found, 392.3 [M+H]⁺. ¹H NMR (400 MHz, CDCl₃): 7.70 (d, *J* = 8.1, 1H), 7.63 (d, *J* = 8.1, 1H), 7.35 (t, *J* = 8.1, 1H), 7.27-7.20 (m, 5H), 2.77-2.73 (m, 4H), 2.64 (t, *J* = 6.6, 2H), 2.39 (d, *J* = 6.3, 2H), 2.29 (t, *J* = 6.8, 2H), 1.74-1.66 (m, 2H), 0.89-0.78 (m, 1H), 0.55-0.48 (m, 2H), 0.13-0.08 (m, 2H).

### EXAMPLE 386

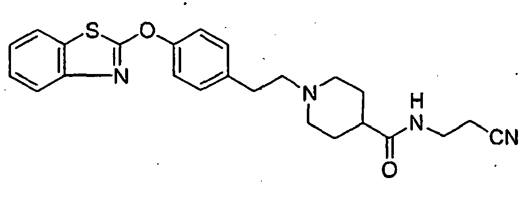

1-{2-[4-(Benzothiazol-2-yloxy)-phenyl]-ethyl}-piperidine-4-carboxylic acid (2-cyano-ethyl)-amide.

The title compound was prepared according to the procedure for EXAMPLE 33, step B, using 3-amino-propionitrile. MS (ESI): mass calculated for C₂₄H₂₆N₄O₂S, 434.18; m/z found, 435.3 [M+H]⁺. ¹H NMR (400 MHz, CDCl₃): 7.74 (d, *J* = 8.3, 1H), 7.67 (d, *J* = 8.1, 1H), 7.40 (t, *J* = 8.1, 1H), 7.32-7.24 (m, 5H), 6.39 (t, *J* = 6.6, 1H), 3.51 (dd, *J* = 6.3, 6.1, 2H), 3.11-3.00 (m, 2H), 2.89-2.82 (m, 2H), 2.69-2.58 (m, 4H), 2.23-2.13 (m, 1H), 2.08 (t, *J* = 12.1, 2H), 1.94-1.76 (m, 4H).

### EXAMPLE 387

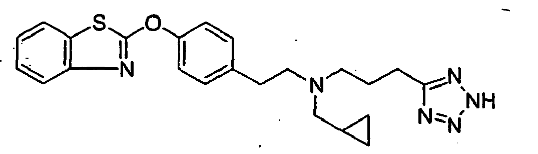

{2-[4-(Benzothiazol-2-yloxy)-phenyl]-ethyl}-cyclopropylmethyl-[3-(2H-tetrazol-5-yl)-propyl]-amine.

To a solution of 4-({2-[4-(benzothiazol-2-yloxy)-phenyl]-ethyl}-cyclopropylmethyl-amino)-butyronitrile (Example 385, 250 mg, 0.6 mmol) in toluene (32 mL) at room temperature was added trimethylaluminum (2.0 M solution in toluene, 1.53 mL, 3.08 mmol), followed by azidotrimethylsilane (404 µL, 3.08 mmol). The resulting mixture was heated at 80 °C for 18 h. The mixture was cooled to room temperature and diluted with CH₂Cl₂. (200 mL), washed with sat. aq. NaHCO₃ (1 × 25 ml) and H₂O (2 x 25 mL), dried (Na₂SO₄) and concentrated under reduced pressure to yield the crude product as an off-white solid. The crude product was purified on SiO₂ (40 g; 0-20% CH₃OH/CH₂Cl₂) to give an off-white solid (246 mg, 88% yield). MS (ESI): mass calculated for C₂₃H₂₆N₆OS, 434.19; m/z found, 435.5 [M+H]⁺. ¹H NMR (400 MHz, CDCl₃): 11.25 (brs, 1H), 7.54 (dd, *J* = 7.8, 5.0, 2H), 7.23 (dd, *J* = 8.1, 7.3, 1H), 7.19-7.09 (m, 5H), 3.23-3.11 (m, 4H), 3.01-2.90 (m, 4H), 2.83 (d, *J* = 7.3, 2H), 2.18-2.07 (m, 2H), 1.01-0.91 (m, 1H), 0.57-0.50 (m, 2H), 0.25-0.18 (m, 2H).

### EXAMPLE 388

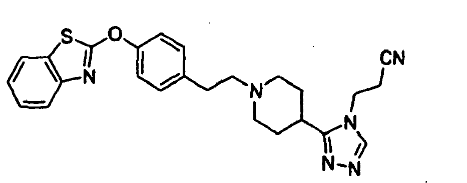

3-[5-(1-{2-[4-(Benzothiazol-2-yloxy)-phenyl]-ethyl}-piperidin-4-yl)-tetrazol-1-yl]-propionitrile.

To a stirred suspension of 1-{2-[4-(benzothiazol-2-yloxy)-phenyl]-ethyl}-piperidine-4-carboxylic acid (2-cyano-ethyl)-amide (Example 386, 500 mg, 1.15 mmol) and triphenylphosphine (755 mg, 2.88 mmol) in anhydrous CH₃CN (10 mL) at 0 °C were added diisopropyl azodicarboxylate (567 µL, 2.88 mmol) and 2 min later, azidotrimethylsilane (399 µL, 3.04 mmol) over 20 min. The reaction mixture was allowed to warm to room temperature over 30 min then was stirred for an additional 14 h. The reaction mixture was added to ice H₂O (20 mL) and extracted with CH₂Cl₂ (2 x 25 mL). The combined organic layers were dried (Na₂SO₄) and concentrated. The residue was purified on SiO₂ (40 g; 0-15% CH₃OH/CH₂Cl₂) to give an off-white solid (439 mg, 83% yield). MS (ESI): mass calculated for C₂₄H₂₅N₇OS, 459.18; m/z found, 460.5 [M+H]⁺. ¹H NMR (400 MHz, CDCl₃): 7.71 (d, *J* = 8.1, 1H), 7.66 (d, *J* = 8.1, 1H), 7.38 (t, *J* = 7.8, 1H), 7.31-7.23 (m, 5H), 4.62 (t, *J* = 6.8, 2H), 3.20-3.10 (m, 4H), 3.04-2.94 (m, 1H), 2.91-2.82 (m, 2H), 2.73-2.64 (m, 2H), 2.25 (t, *J* = 10.9, 2H), 2.18-2.05 (m, 2H), 2.04-1.95 (m, 2H).

### EXAMPLE 389

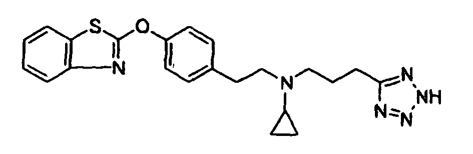

{2-[4-(Benzothiazol-2-yloxy)-phenyl]-ethyl}-cyclopropyl-[3-(2H-tetrazol-5-yl)-propyl]-amine.

The title compound was prepared according to the procedure for EXAMPLE 387. MS (ESI): mass calculated for C₂₀H₂₄N₆OS, 420.17; m/z found, 421.5 [M+H]⁺. ¹H NMR (400 MHz, CDCl₃): 13.02 (br s, 1H), 7.50 (dd, *J* = 8.1, 3.5, 2H), 7.20 (t, *J* = 8.1, 1H), 7.12-7.04 (m, 5H), 2.95-2.77 (m, 8H), 2.06-1.93 (m, 3H), 0.60-0.48 (m, 4H).

### EXAMPLE 390

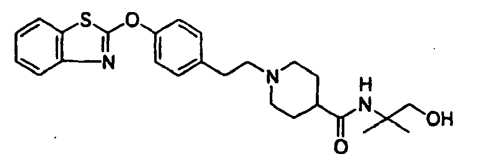

1-{2-[4-(Benzothiazol-2-yloxy)-phenyl]-ethyl}-piperidine-4-carboxylic acid (2-hydroxy-1,1-dimethyl-ethyl)-amide.

The title compound was prepared according to the procedure for EXAMPLE 33, steps B and C using 2-amino-2-methyl-propan-1-ol. MS (ESI): mass calculated for C₂₅H₃₁N₃O₃S, 453.21; m/z found, 454.4 [M+H]⁺. ¹H NMR (400 MHz, CDCl₃): 7.75 (d, *J* = 8.1, 1H), 7.68 (d, *J* = 8.1, 1H), 7.40 (t, *J* = 8.1, 1H), 7.32-7.25 (m, 5H), 5.83 (s, 1H), 5.06 (br s, 1H), 3.59 (s, 2H), 3.14-3.06 (m, 2H), 2.91-2.84 (m, 2H), 2.70-2.63 (m, 2H), 2.20-2.10 (m, 3H), 1.95-1.74 (m, 4H), 1.32 (s, 6H).

### EXAMPLE 391

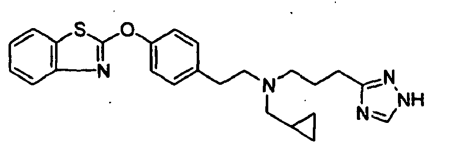

{2-[4-(Benzothiazol-2-yloxy)-phenyl]-ethyl}-cyclopropylmethyl-[3-(1H-[1,2,4]triazol-3-yl)-propyl]-amine trifluoromethansulfonic acid salt.
A. 4-({2-[4-(Benzothiazol-2-yloxy)-phenyl]-ethyl}-cyclopropylmethyl-amino)-butyrimidic acid ethyl ester hydrochloride salt. A solution of 4-({2-[4-(benzothiazol-2-yloxy)-phenyl]-ethyl}-cyclopropylmethyl-amino)-butyronitrile (Example 385, 932 mg, 2.38 mmol) in absolute ethanol (5 mL) and diethyl ether (10 mL) was treated with bubbled hydrogen chloride gas for 1 h. The resulting suspention was filtered and washed with ether to give the title compound (1.12 g, 92% yield). Due to instability, the crude material was used immediately without purification.
B. {2-[4-(Benzothiazol-2-yloxy)-phenyl]-ethyl}-cyclopropylmethyl-[3-(1H-[1,2,4]triazol-3-yl)-propyl]-amine trifluoromethansulfonic acid salt. To a solution of 4-({2-[4-(benzothiazol-2-yloxy)-phenyl]-ethyl}-cyclopropylmethyl-amino)-butyrimidic acid ethyl ester hydrochloride salt (511 mg, 1 mmol) in ethanol (4.5 mL) was added triethylamine (375 µL, 2.7 mmol), followed by a solution of formic hydrazide (60 mg, 1 mmol) in ethanol (4.5 mL). The resulting mixture was stirred at room temperature for 2 h and then at reflux for 1 h. The mixture was cooled and diluted with CH₂Cl₂ (1500 mL), washed with sat. aq. NaHCO₃ (1 x 25 mL) and H₂O (2 x 25 mL), dried (Na₂SO₄) and concentrated under reduced pressure to yield the crude product as a off-white solid. The crude product was purified on SiO₂ (40 g; 0-15% CH₃OH/CH₂Cl₂) to give a off-white solid (317 mg, 73% yield). MS (ESI): mass calculated for C₂₄H₂₇N₅OS, 433.19; m/z found, 434.4 [M+H]⁺. ¹H NMR (400 MHz, CDCl₃): 7.91 (s, 1H), 7.68 (d, *J* = 7.8, 1H), 7.63 (d, *J* = 8.1, 1H), 7.34 (t, *J* = 8.1, 1H), 7.25-7.19 (m, 5H), 2.90-2.76 (m, 6H), 2.72 (t, *J* = 6.3, 2H), 2.46 (d, *J* = 6.6, 2H), 2.00-1.90 (m, 2H), 0.93-0.82 (m, 1H), 0.56-0.49 (m, 2H), 0.16-0.10 (m, 2H).

### EXAMPLE 392

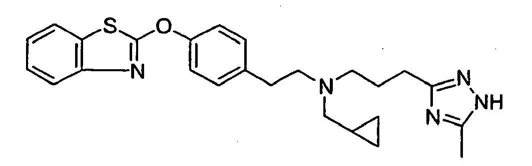

{2-[4-(Benzothiazol-2-yloxy)-phenyl]-ethyl}-cyclopropylmethyl-[3-(5-methyl-1H-[1,2,4]triazol-3-yl)-propyl]-amine.

The title compound was prepared according to the procedure for EXAMPLE 391, step B using acetic hydrazide. MS (ESI): mass calculated for C₂₅H₂₉N₅OS, 447.21; m/z found, 448.4 [M+H]⁺. ¹H NMR (400 MHz, CDCl₃): 7.65 (dd, *J* = 8.1, 8.1, 2H), 7.35 (t, *J* = 8.1, 1H), 7.27-7.21 (m, 5H), 3.01-2.94 (m, 2H), 2.93-2.84 (m, 4H), 2.81 (t, *J* = 6.6, 2H), 2.61 (d, *J* = 6.8, 2H), 2.37 (s, 3H), 2.07-1.97 (m, 2H), 1.01-0.89 (m, 1H), 0.62-0.53 (m, 2H), 0.25-0.17 (m, 2H).

### EXAMPLE 393

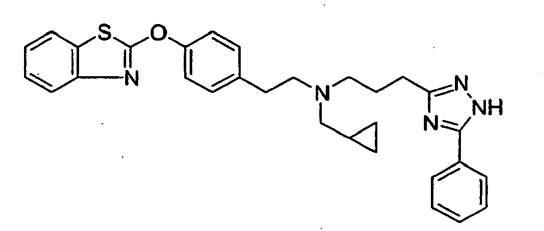

{2-[4-(Benzothiazol-2-yloxy)-phenyl]-ethyl}-cyclopropylmethyl-[3-(5-phenyl-1H-[1,2,4]triazol-3-yl)-propyl]-amine.

The title compound was prepared according to the procedure for EXAMPLE 391, step B using benzoic acid hydrazide. MS (ESI): mass calculated for C₃₀H₃₁N₅OS, 509:22; m/z found, 510.4 [M+H]⁺. ¹H NMR (400 MHz, CDCl₃): 8.06 (dd, *J* = 16.8, 0.5, 2H), 7.70 (d, *J* = 8.1, 1H), 7.63 (d, *J* = 8.1, 1H), 7.42-7.30 (m, 4H), 7.28-7.20 (m, 5H), 2.99-2.87 (m, 6H), 2.85 (t, *J* = 6.6, 2H), 2.58 (d, *J* = 6.8, 2H), 2.09-2.00 (m, 2H), 1.00-0.89 (m, 1H), 0.61-0.53 (m, 2H), 0.23-0.16 (m, 2H).

### EXAMPLE 394

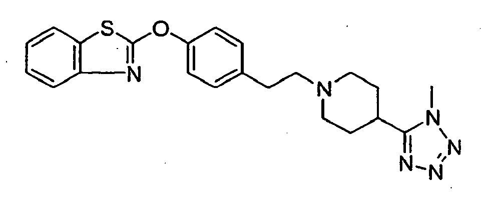

2-(4-{2-[4-(1-Methyl-1H-tetrazol-5-yl)-piperidin-1-yl]-ethyl}-phenoxy)-benzothiazole trifluoroacetic acid salt.

To a solution of 2-(4-{2-[4-(1H-tetrazol-5-yl)-piperidin-1-yl]-ethyl}-phenoxy)-benzothiazole (Example 262, 80 mg, 1.97 mmol) in DMF (20 mL) was added K₂CO₃ (256 mg, 1.85 mmol) and dimethyl carbonate (360 µL, 4.27 mmol). The reaction mixture was stirred at room temperature for 18 h, filtered and concentrated under reduced pressure. The crude product was dissolved in CH₃OH and purified by reversed-phase HPLC. The desired fractions were collected and concentrated under reduced pressure, giving the title compound as a colorless oil (289 mg, 27% yield). MS (ESI): mass calculated for C₂₂H₂₄N₆OS, 420.17; m/z found, 421.3 [M+H]⁺. ¹H NMR (400 MHz, CD₃OD): 7.72 (d, *J* = 7.8, 1H), 7.58 (d, *J* = 7.8, 1H), 7.44-7.28 (m, 5H), 7.25 (t, *J* = 8.1, 1H), 4.06 (s, 3H), 3.80 (d, *J* = 12.1, 1H), 3.66-3.35 (m, 4H), 3.28-3.17 (m, 2H), 3.16-3.08 (m, 2H), 2.33-2.23 (m, 2H), 2.22-2.07 (m, 2H).

### EXAMPLE 395

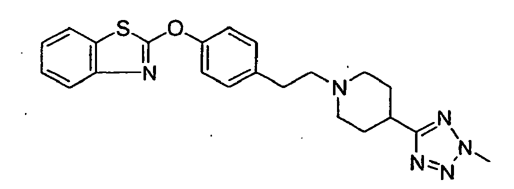

2-(4-{2-[4-(2-Methyl-2H-tetrazol-5-yl)-piperidin-1-yl]-ethyl}-phenoxy)-benzothiazole.

The title compound was prepared according to the procedure for EXAMPLE 394. MS (ESI): mass calculated for C₂₂H₂₄N₆OS, 420.17; m/z found, 421.3 [M+H]⁺. ¹H NMR (400 MHz, CD₃OD): 7.75 (d, *J* = 7.8, 1H), 7.60 (d, *J* = 7.8, 1H), 7.46-7.33 (m, 5H), 7.28 (t, *J* = 7.8, 1H), 4.31 (s, 3H), 3.79 (d, *J* = 13.4, 1H), 3.62-3.06 (m, 8H), 2.48-2.30 (m, 2H), 2.15-2.02 (m, 2H).

### EXAMPLE 396

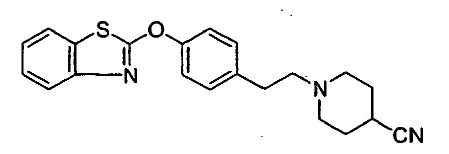

1-{2-[4-(Benzothiazol-2-yloxy)-phenyl]-ethyl}-piperidine-4-carbonitrile.

The title compound was prepared according to the procedure for EXAMPLE 262, using piperidine-4-carbonitrile. MS (ESI): mass calculated for C₂₁H₂₁N₃OS, 363.14; m/z found, 364.3 [M+H]⁺. ¹H NMR (400 MHz, CDCl₃): 7.63 (d, *J* = 7.6, 1H), 7.55 (d, *J* = 7.6, 1H), 7.28 (t, *J* = 8.1, 1H), 7.20-7.12 (m, 5H), 2.71 (dd, *J* = 7.1, 3.3, 2H), 2.68-2.55 (m, 3H), 2.52 (dd, *J* = 7.1, 3.3, 2H), 2.37-2.25 (m, 2H), 1.91-1.73 (m, 2H).

### EXAMPLE 397

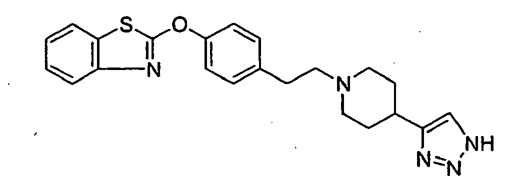

2-(4-{2-[4-(1H-[1,2,3]Triazol-4-yl)-piperidin-1-yl]-ethyl}-phenoxy)-benzothiazole.

To a solution of trimethylsilyldiazomethane (1.8 mL, 3.6 mmol) in diethyl ether (30 mL) at 0 °C under nitrogen was added dropwise n-butyllithium (2.5 M in hexane, 1.44 mL, 3.6 mmol) and the mixture was stirred at 0 °C for 20 min. To the resulting solution was added dropwise a solution of 1-{2-[4-(benzothiazol-2-yloxy)-phenyl]-ethyl}-piperidine-4-carbonitrile (Example 396, 1.09 g, 3.0 mmol) in THF (10 mL) at 0 °C. The resulting mixture was stirred at 0 °C for 3 h. The mixture was treated with sat. aq. NH₄Cl and extracted with CH₂Cl₂ (2 x 100 mL). The extracts were washed with H₂O (2 x 25 mL), dried (Na₂SO₄) and concentrated under reduced pressure to yield the crude product as an off-white solid. The crude product was purified on SiO₂ (40 g; 0-10% CH₃OH/CH₂Cl₂) to give an off-white solid (768 mg, 54% yield): MS (ESI): mass calculated for C₂₂H₂₃N₅OS, 405.16; m/z found, 406.3 [M+H]⁺. ¹H NMR (400 MHz, CDCl₃): 7.63 (d, *J* = 8.1, 1H), 7.56 (d, *J* = 8.1, 1H), 7.42 (s, 1H), 7.29 (t, *J* = 7.8, 1H), 7.23-7.14 (m, 5H), 4.67 (br s, 1 H), 3.04 (d, *J* = 11.9, 2H), 2.84-2.70 (m, 3H), 2.63-2.56 (m, 2H), 2.16 (t, *J* = 11.1, 2H), 1.97 (d, *J* = 12.6, 2H), 1.82-1.70 (m, 2H).

### EXAMPLE 398

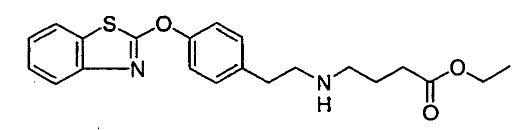

4-{2-[4-(Benzothiazol-2-yloxy)-phenyl]-ethylamino}-butyric acid ethyl ester.

The title compound was prepared according to the procedure for EXAMPLE 378; step A using 4-amino-butyric acid ethyl ester. MS (ESI): mass calculated for C₂₁H₂₄N₂O₃S, 384.15; m/z found, 385.3 [M+H]⁺. ¹H NMR (400 MHz, CDCl₃): 7.73 (d, *J* = 8.1, 1H), 7.66 (d, *J* = 8.1, 1H), 7.38 (t, *J* = 8.1, 1H), 7.31-7.22 (m, 5H), 6.67 (br s, 1H), 4.12 (dd, *J* = 7.3, 7.1, 2H), 2.95-2.80 (m, 2H), 2.69 (t, *J* = 6.8, 2H), 2.35 (t, *J* = 7.3, 2H), 2.29 (t, *J* = 7.3, 2H), 1.86-1.77 (m, 2H), 1.25 (t, *J* = 7.1, 3H).

### EXAMPLE 399

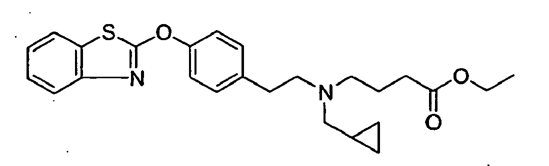

4-({2-[4-(Benzothiazol-2-yloxy)-phenyl]-ethyl}-cyclopropylmethyl-amino)-butyric acid ethyl ester.

The title compound was prepared according to the procedure for EXAMPLE 378, step A using cyclopropyl-methylamine and EXAMPLE 379, step A using 4-bromo-butyric acid ethyl ester. MS (ESI): mass calculated for C₂₅H₃₀N₂O₃S, 438.20; m/z found, 439.4 [M+H]⁺. ¹H NMR (400 MHz, CDCl₃): 7.71 (d, *J* = 8.1, 1H), 7.63 (d, *J* = 8.1, 1H), 7.35 (t, *J* = 8.1, 1H), 7.27-7.20 (m, 5H), 6.67 (br s, 1H), 4.10 (dd, *J* = 7.1, 7_{.}1, 2H), 2.83-2.71 (m, 4H), 2.60 (t, *J* = 7.1, 2H), 2.40 (t, *J* = 6.6, 2H), 2.31 (t, *J* = 7.3, 2H), 1.81-1.72 (m, 2H), 1.23 (t, *J* = 7.1, 3H), 0.88-0.79 (m, 1H), 0.52-0.45 (m, 2H), 0.13-0.07 (m, 2H).

### EXAMPLE 400

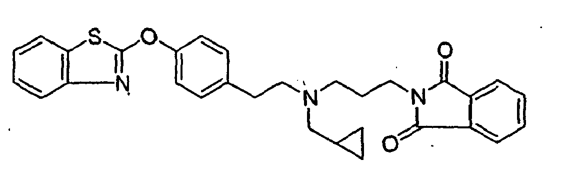

2-[3-({2-[4-(Benzothiazol-2-yloxy)-phenyl]-ethyl}-cyclopropylmethyl-amino)-propyl]-isoindole-1,3-dione.

The title compound was prepared according to the procedure for EXAMPLE 378, steps A using cyclopropyl-methylamine and EXAMPLE 379, step A using 2-(3-bromo-propyl)-isoindole-1,3-dione. MS (ESI): mass calculated for C₃₀H₂₉N₃O₃S, 511.19; m/z found, 512.3 [M+H]⁺. ¹H NMR (400 MHz, CDCl₃): 7.84-7.77 (m, 2H), 7.72-7.63 (m, 3H), 7.60 (d, *J* = 7.8, 1H), 7.34 (t, *J* = 8_{.}1, 1H), 7.27-7.18 (m, 5H), 3.71 (t, *J* = 7.1, 2H), 2.82-2.70 (m, 4H), 2.65 (t, *J* = 7.6, 2H), 2.39 (d, *J* = 6.6, 2H), 1.88-1.79 (m, 2H), 0.87-0.76 (m, 1H), 0.50-0.42 (m, 2H), 0.12-0.06 (m, 2H).

### EXAMPLE 401

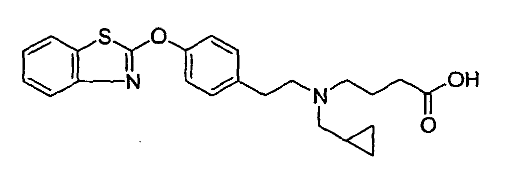

4-({2-[4-(Benzothiazol-2-yloxy)-phenyl]-ethyl}-cyclopropylmethyl-amino)-butyric acid.

The title compound was prepared from EXAMPLE 399 followed by the procedure for EXAMPLE 379, step B. MS (ESI): mass calculated for C₂₃H₂₆N₂O₃S, 410.17; m/z found, 411.4 [M+H]⁺. ¹H NMR (400 MHz, CD₃OD): 7.42 (d, *J* = 7.8, 1H), 7.31 (d, *J* = 7.8, 1 H), 7.16-7.10 (m, 2H), 7.07 (t, *J* = 7.8, 1H), 7.04-7.00 (m, 2H), 6.96 (t, *J* = 7.8, 1H), 3.23-3.11 (m, 2H), 3.07 (t, *J* = 7.8, 2H), 2.91-2.77 (m, 4H), 2.19 (t, *J* = 7.1, 2H), 1.78-1.68 (m, 2H), 0.91-0.82 (m, 1H), 0.49-0.41 (m, 2H), 0.20-0.13 (m, 2H).

### EXAMPLE 402

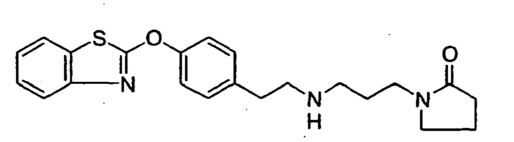

1-(3-{2-[4-(Benzothiazol-2-yloxy)-phenyl]-ethylamino}-propyl)-pyrrolidin-2-one.

The title compound was prepared according to the procedure for EXAMPLE 378, step A using 1-(3-aminio-propyl)-pyrrolidin-2-one. MS (ESI): mass calculated for C₂₂H₂₅N₃O₂S, 395.17; m/z found, 396.4 [M+H]⁺. ¹H NMR (400 MHz, CDCl₃): 7.67 (d, *J* = 8.1, 1H), 7.60 (d, *J* = 8.1, 1H), 7.32 (t, *J* = 7.8, 1H), 7.28-7.15 (m, 5H), 5.61 (br s, 1H), 3.34-3.25 (m, 4H), 2.94-2.81 (m, 4H), 2.65 (t, *J* = 7.3, 2H), 2.32 (t, *J* = 7.8, 2H), 1.94 (t, *J* = 7.3, 2H), 1.79-1.69 (m, 2H).

### EXAMPLE 403

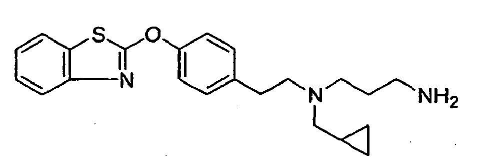

N-1-{2-[4-(Benzothiazol-2-yloxy)-phenyl]-ethyl}-N1-cyclopropylmethyl-propane-1,3-diamine.

To a solution of 2-[3-({2-[4-(benzothiazol-2-yloxy)-phenyl]-ethyl}-cyclopropylmethyl-amino)-propyl]-isoindole-1,3-dione (Example 400, 3.0 g, 5.86 mmol) in EtOH (12 mL) was added hydrazine (220 µL, 7.03 mmol). The reaction mixture was stirred at room temperature for 24 h. The mixture was dissolved in CH₂Cl₂ (200 mL), washed with saturated NaHCO₃ (2 x 20 ml), H₂O (1 x 20 mL), dried (Na₂SO₄) and concentrated under reduced pressure to yield the crude product as a off-white solid. The crude product was purified on SiO₂ (330 g; 0-10% 2 M NH₃ in CH₃OH/CH₂Cl₂) to give a clear oil (2.13 g, 96% yield). MS (ESI): mass calculated for C₂₂H₂₇N₃OS, 381.2; m/z found, 382.4 [M+H]⁺. ¹H NMR (400 MHz, CDCl₃): 7.68 (d, *J* = 8.1, 1 H), 7.58 (d, *J* = 8.1, 1H), 7.31 (t, *J* = 8.1, 1H), 7.24-7.16 (m, 5H), 2.79-2.71 (m, 4H), 2.67 (t, *J* = 6.8, 2H), 2.59 (t, *J* = 6.8, 2H), 2.37 (d, *J* = 6.6, 2H), 2.07 (s, 2H), 1.62-1.52 (m, 2H), 0.87-0.77 (m, 1H), 0.50-0.44 (m, 2H), 0.12-0.06 (m, 2H).

### EXAMPLE 404

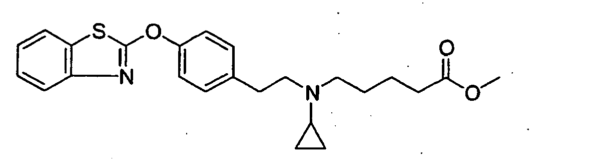

5-({2-[4-(Benzothiazol-2-yloxy)-phenyl]-ethyl}-cyclopropyl-amino)-pentanoic acid methyl ester.

The title compound was prepared according to the procedure for EXAMPLE 379, step A using 5-bromo-pentanoic acid methyl ester. MS (ESI): mass calculated for C₂₄H₂₈N₂O₃S, 424.18; m/z found, 425.3 [M+H]⁺. ¹H NMR (400 MHz, CDCl₃): 7.72 (d, *J* = 8.1, 1H), 7.63 (d, *J* = 8.1, 1H), 7.36 (t, *J* = 8.1, 1H), 7.27-7.20 (m, 5H), 3.65 (s, 3H), 2.85-2.80 (m, 4H), 2.66 (t, *J* = 7.6, 2H), 2.33 (t, *J* = 7.6, 2H), 1.81-1.75 (m, 1H), 1.67-1.50 (m, 4H), 0.52-0.40 (m, 2H), 0.43-0.37 (m, 2H).

### EXAMPLE 405

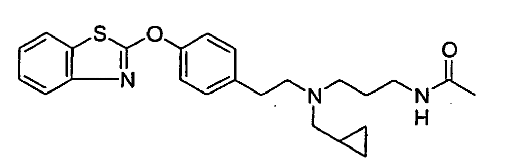

N-[3-({2-[4-(Benzothiazol-2-yloxy)-phenyl]-ethyl}-cyclopropylmethyl-amino)-propyl]-acetamide.

To a solution of N-1-{2-[4-(Benzothiazol-2-yloxy)-phenyl]-ethyl}-N1-cyclopropylmethyl-propane-1,3-diamine (Example 403, 180 mg, 0.47 mmol) in CH₂Cl₂ (10 mL) was added triethylamine (131 µL, 0.94 mmol), followed by acetic anhydride (76 µL, 0.71 mmol). The reaction mixture was stirred at room temperature for 24 h. The mixture was dissolved in CH₂Cl₂ (100 mL), washed with sat. NaHCO₃ (2 x 15 ml), H₂O (1 x 15 mL), dried (Na₂SO₄) and concentrated under reduced pressure to yield the crude product as a off-white solid. The crude product was purified on SiO₂ (12 g; 0-10% CH₃OH/CH₂Cl₂) to give a white solid (154 mg, 77% yield). MS (ESI): mass calculated for C₂₄H₂₉N₃OS, 423.2; m/z found, 424.4 [M+H]⁺. ¹H NMR (400 MHz, CDCl₃): 9.69 (br s, 1H), 7.67 (d, *J* = 8.1, 1H), 7.62 (d, *J* = 8.1, 1H), 7.33 (t, *J* = 8.1, 1H), 7.27-7.17 (m, 5H), 3.27 (dd, *J* = 6.6, 5.6, 2H), 2.96-2.89 (m, 2H), 2.87-2.81 (m, 2H), 2.78 (t, *J* = 6.8, 2H), 2.53 (d, *J* = 6.8, 2H), 1.88 (s, 3H), 1.76-1.67 (m, 2H), 0.94-0.83 (m, 1H), 0.60-0.53 (m, 2H), 0.21-0.15 (m, 2H).

### EXAMPLE 406

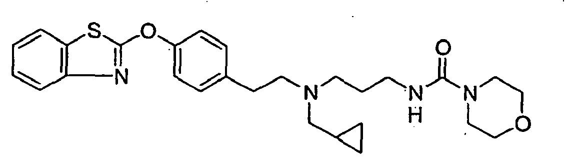

Morpholine-4-carboxylic acid [3-({2-[4-(benzothiazol-2-yloxy)-phenyl]-ethyl}-cyclopropylmethyl-amino)-propyl]-amide.

The title compound was prepared according to the procedure for EXAMPLE 405, using morpholine-4-carbonyl chloride. MS (ESI): mass calculated for C₂₈H₃₄N₄O₃S, 494.24; m/z found, 495.4 [M+H]⁺. ¹H NMR (400 MHz, CDCl₃): 7.69 (t, *J* = 8.5, 2H), 7.41-7.23 (m, 6H), 6.85 (br s, 1H), 3.68-3.60 (m, 4H), 3.51-3.40 (m, 6H), 3.39-3,27 (m, 4H), 3.26-3.17 (m, 2H), 3.09-3.01 (m, 2H), 2.19-2.06 (m, 2H), 1.25-1.14 (m, 1H), 0.85-0.76 (m, 2H), 0.53-0.45 (m, 2H).

### EXAMPLE 407

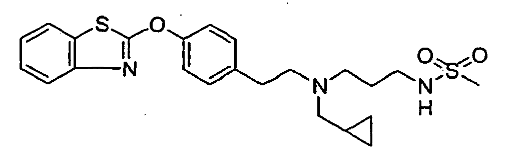

N-[3-({2-[4-(Benzothiazol-2-yloxy)-phenyl]-ethyl}-cyclopropylmethyl-amino)-propyl]-methanesulfonamide.

The title compound was prepared according to the procedure for EXAMPLE 405, using methanesulfonyl chloride. MS (ESI): mass calculated for C₂₃H₂₉N₃O₃S₂, 459.17; m/z found, 460.3 [M+H]⁺. ¹H NMR (400 MHz, CDCl₃): 7.67 (t, *J* = 8.6, 2H), 7.39-7.17 (m, 6H), 7.06 (br s, 1H), 3.43-3.09 (m, 8H), 3.07-2.98 (m, 2H), 2.96 (s, 3H), 2.20-2.07 (m, 2H), 1.23-1.11 (m, 1H), 0.79-0.67 (m, 2H), 0.49-0.41 (m, 2H).

### EXAMPLE 408

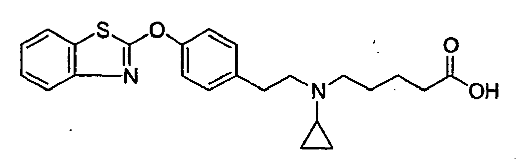

5-({2-[4-(Benzothiazol-2-yloxy)-phenyl]-ethyl}-cyclopropyl-amino)-pentanoic acid.

The title compound was prepared from EXAMPLE 379 and the procedure for EXAMPLE 404, step B. MS (ESI): mass calculated for C₂₃H₂₆N₂O₃S, 410.17; m/z found, 411.3 [M+H]⁺. ¹H NMR (400 MHz, CDCl₃): 12.39 (br s, 1H), 7.71 (d, *J* = 7.8, 1H), 7.62 (d, *J* = 7.8, 1H), 7.35 (t, *J* = 7.8, 1H), 7.31-7.20 (m, 5H), 3.04-2.90 (m, 4H), 2.82 (t, *J* = 7.8, 2H), 2.30 (t, *J* = 6.8, 2H), 1.98-1.91 (m, 1H), 1.72-1.54 (m, 4H), 0.83-0.76 (m, 2H), 0.63-0.55 (m, 2H).

### EXAMPLE 409

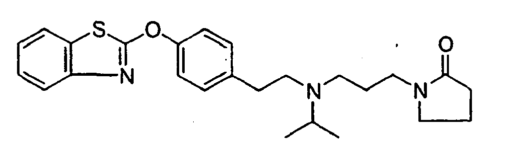

1-[3-({2-[4-(Benzothiazol-2-yloxy)-phenyl]-ethyl}-isopropyl-amino)-propyl]-pyrrolidin-2-one.

A mixture of 1-(3-{2-[4-(benzothiazol-2-yloxy)-phenyl]-ethylamino}-propyl)-pyrrolidin-2-one (Example 402, 500 mg, 1.26 mmol), acetone (185 µL, 2.52 mmol) in CH₂Cl₂ (25 mL) was stirred at room temperature for 1 h. To the resulting mixture was added NaBH(OAc)₃ (536 mg, 2.52 mmol). The mixture was stirred at room temperature for 16 h, filtered through diatomaceous earth, washed with CH₂Cl₂ (100 mL) and concentrated under reduced pressure to yield the crude product as a yellow oil. The crude product was purified on SiO₂ (40 g; 0-10% CH₃OH/CH₂Cl₂). The desired fractions were combined and concentrated under reduced pressure to give a clear oil which crystallized on standing (290 mg, 53% yield). MS (ESI): mass calculated for C₂₅H₃₁N₃O₂S, 437.21; m/z found, 438.4 [M+H]⁺. ¹H NMR (400 MHz, CDCl₃): 7.73 (d, *J* = 8.3, 1H), 7.66 (d, *J* = 8.3, 1H), 7.38 (t, *J* = 8.3, 1H), 7.28-7.23 (m, 5H), 3.35 (t, *J* = 6.8, 2H), 3.27 (t, *J* = 7.3, 2H), 3.05-2.95 (m, 1H), 2.78-2.70 (m, 2H), 2.69-2.61 (m, 2H), 2.47 (t, *J* = 7.3, 2H), 2.37 (t, *J* = 8.1, 2H), 2.04-1.94 (m, 2H), 1.69-1.58 (m, 2H), 0.99 (d, *J* = 6.8, 6H).

### EXAMPLE 410

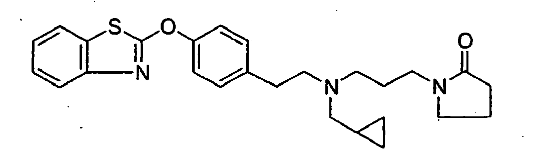

1-[3-({2-[4-(Benzothiazol-2-yloxy)-phenyl]-ethyl}-cyclopropylmethyl-amino)-propyl]-pyrrolidin-2-one.

The title compound was prepared according to the procedure for EXAMPLE 409, using cyclopropanecarbaldehyde. MS (ESI): mass calculated for C₂₆H₃₁N₃O₂S, 449.21; m/z found, 450.4 [M+H]⁺. ¹H NMR (400 MHz, CDCl₃): 7.70 (d, *J* = 7.8, 1H), 7.64 (d, *J* = 8.1, 1H), 7.36 (t, *J* = 7.8, 1H), 7.32-7.21 (m, 5H), 3.38 (t, *J*= 7.1, 2H), 3.31 (t, *J* = 7.1, 2H), 3.09-3.02 (m, 2H), 2.95-2.83 (m, 4H), 2.72 (d, *J* = 7.1, 2H), 2.37 (t, *J* = 7.8, 2H), 2.05-1.95 (m, 2H), 1.92-1.82 (m, 2H), 1.02-0.91 (m, 1H), 0.66-0.59 (m, 2H), 0.29-0.23 (m, 2H).

### EXAMPLE 411

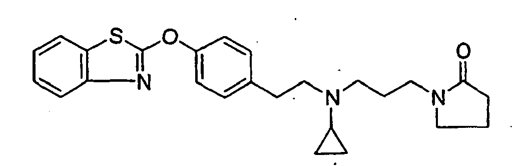

1-[3-({2-[4-(Benzothiazol-2-yloxy)-phenyl]-ethyl}-cyclopropyl-amino)-propyl]-pyrrolidin-2-one.

To a solution of 1-(3-{2-[4-(benzothiazol-2-yloxy)-phenyl]-ethylamino}-propyl)-pyrrolidin-2-one (Example 402, 500 mg, 1.26 mmol) in EtOH (20 mL) was added acetic acid (716 µL, 1.26 mmol), molecular sieves (500 mg, 3A), (1-ethoxy-cyclopropoxy)-trimethyl-silane (1.51 mL, 7.58 mmol) and sodium cyanoboronhydride (357 mg, 5.69 mmol). The reaction mixture was heated at reflux for 16 h, cooled and filtered through diatomaceous earth, washed with CH₂Cl₂ (100 mL) and concentrated under reduced pressure to yield the crude product as a clear oil. The crude product was purified on SiO₂ (40 g; 0-10% CH₃OH/CH₂Cl₂). The desired fractions were combined and concentrated under reduced pressure to give a white solid (434 mg, 66% yield). MS (ESI): mass calculated for C₂₅H₂₉N₃O₂S, 435.2; m/z found, 436.4 [M+H]⁺. ¹H NMR (400 MHz, CDCl₃): 7.72 (d, *J* = 7.8, 1H), 7.64 (d, *J* = 7.8, 1H), 7.36 (t, *J* = 7.8, 1H), 7.28-7.19 (m, 5H), 3.36 (t, *J* = 7.1, 2H), 3.28 (t, *J* = 7.1, 2H), 2.87-2.80 (m, 4H), 2.67 (t, *J* = 7.3, 2H), 2.37 (t, *J* = 7.8, 2H), 2.03-1.94 (m, 2H), 1.82-1.70 (m, 3H), 0.53-0.47 (m, 2H), 0.43-0.38 (m, 2H).

### EXAMPLE 412

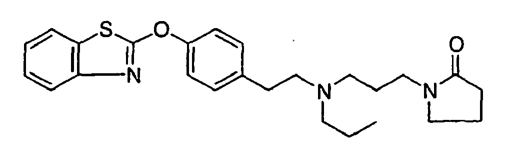

1-[3-({2-[4-(Benzothiazol-2-yloxy)-phenyl]-ethyl}-propyl-amino)-propyl]-pyrrolidin-2-one.

The title compound was prepared according to the procedure for EXAMPLE 409, using propionaldehyde. MS (ESI): mass calculated for C₂₅H₃₁N₃O₂S, 437.21; m/z found, 438.4 [M+H]⁺. ¹H NMR (400 MHz, CDCl₃): 7.72 (d, *J* = 8.1, 1H), 7.66 (d, *J* = 8.1, 1H), 7.38 (t, *J* = 8.1, 1H), 7.29-7.24 (m, 5H), 3.39 (t, *J* = 7.1, 2H), 3.30 (t, *J* = 7.1, 2H), 2.86-2.80 (m, 4H), 2.64 (t, *J* = 7.6, 2H), 2.58 (t, *J* = 7.6, 2H), 2.42-2.35 (m, 2H), 2.07-1.97 (m, 2H), 1.80-1.71 (m, 2H), 1.59-1.47 (m, 2H), 0.92 (d, *J* = 7.3, 3H).

### EXAMPLE 413

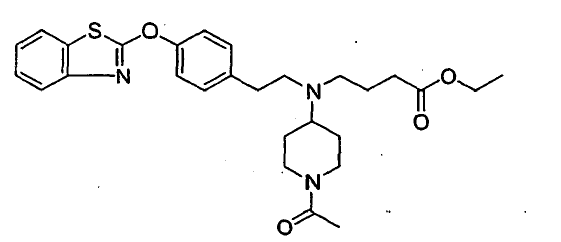

4-((1-Acetyl-piperidin-4-yl)-{2-[4-(benzothiazol-2-yloxy)-phenyl]-ethyl}-amino)-butyric acid ethyl ester.

The title compound was prepared according to the procedure for EXAMPLE 409, using 1-acetyl-piperidin-4-one. MS (ESI): mass calculated for C₂₈H₃₅N₃O₄S, 509.23; m/z found, 510.4 [M+H]⁺. ¹H NMR (400 MHz, CDCl₃): 7.72 (d, *J* = 8.1, 1H), 7.65 (d, *J* = 8.1, 1H), 7.38 (t, *J* = 8.1, 1H), 7.29-7.23 (m, 5H), 4.69 (d, *J* = 13.1, 1H), 4.13 (dd, *J* = 7.8, 6.8, 2H), 3.85 (d, *J* = 13.4, 1H), 3.09-2.98 (m, 2H), 2.79-2.74 (m, 4H), 2.60 (t, *J* = 6.8, 2H), 2.52-2.44 (m, 3H), 2.04 (s, 3H), 1.85-1.73 (m, 4H), 1.48-1.35 (m, 2H), 1.26 (t, *J* = 7.1, 3H).

### EXAMPLE 414

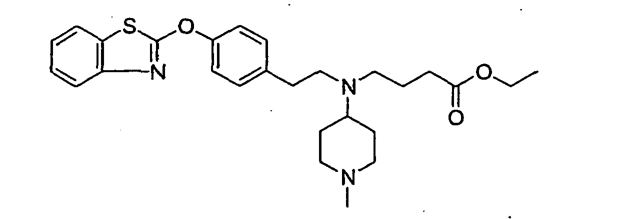

4-((1-Acetyl-piperidin-4-yl)-{2-[4-(benzothiazol-2-yloxy)-phenyl]-ethyl}-amino)-butyric acid ethyl ester.

The title compound was prepared according to the procedure for EXAMPLE 409, using 1-methyl-piperidin-4-one. MS (ESI): mass calculated for C₂₇H₃₅N₃O₃S, 481.2; m/z found, 482.4 [M+H]⁺. ¹H NMR (400 MHz, CDCl₃): 7.72 (d, *J* = 8.1, 1H), 7.65 (d, *J* = 8.1, 1H), 7.37 (t, *J* = 8.1, 1H), 7.28-7.19 (m, 5H), 4.12 (dd, *J* = 7.3, 7.1, 2H), 3.11 (d, *J* = 12.1, 2H), 2.75-2.69 (m, 4H), 2.58-2.50 (m, 3H), 2.38 (s, 3H), 2.29 (t, *J* = 7.1, 2H), 2.24-2.15 (m, 2H), 2.03-1.97 (m, 2H), 1.73 (t, *J* = 6.3, 4H), 1.25 (t, *J* = 7.1, 3H).

### EXAMPLE 415

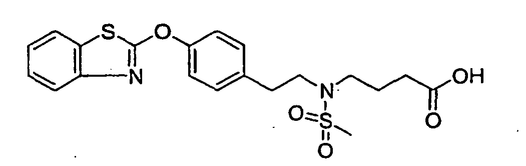

4-({2-[4-(Benzothiazol-2-yloxy)-phenyl]-ethyl}-methanesulfonyl-amino)-butyric acid.

The title compound was prepared from EXAMPLE 398 following the procedure for EXAMPLE 407 and EXAMPLE 379, step B. MS (ESI): mass calculated for C₂₀H₂₂N₂O₅S₂, 434.1; m/z found, 435.3 [M+H]⁺. ¹H NMR (400 MHz, CDCl₃): 7.72 (d, *J* = 8.1, 1H), 7.66 (d, *J* = 8.1, 1H), 7.38 (t, *J* = 8.1, 1H), 7.33-7.24 (m, 5H), 3.48 (t, *J* = 7.6, 2H), 3.20 (t, *J* = 7.6, 2H), 2.96 (t, *J* = 7.3, 2H), 2.77 (s, 3H), 2.37 (t, *J* = 6.6, 2H), 1.91-1.81 (m, 2H).

### EXAMPLE 416

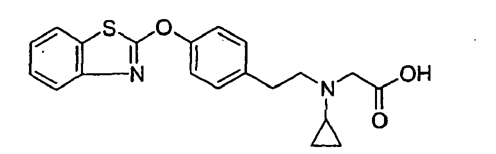

({2-[4-(Benzothiazol-2-yloxy)-phenyl]-ethyl}-cyclopropyl-amino)-acetic acid.

The title compound was prepared according to the procedure for EXAMPLE 379, step A and B using bromo-acetic acid methyl ester. MS (ESI): mass calculated for C₂₀H₂₀N₂O₃S, 368.12; m/z found, 369.3 [M+H]⁺. ¹H NMR (400 MHz, CDCl₃): 7.70 (d, *J* = 8.1, 1H), 7.64 (d, *J* = 8.1, 1H), 7.37 (t, *J* = 8.1, 1H), 7.31-7.22 (m, 5H), 3.64 (s, 2H), 3.26 (dd, *J* = 7.6, 3.3, 2H), 2.98 (dd, *J* = 7.6, 3.3, 2H), 2.50-2.43 (m, 1H), 0.84-0.79 (m, 2H), 0.71-0.65 (m, 2H).

### EXAMPLE 417

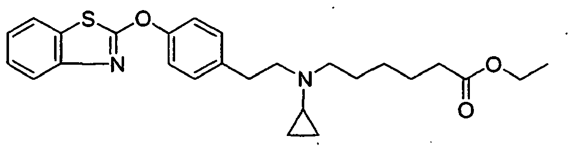

6-({2-[4-(Benzothiazol-2-yloxy)-phenyl]-ethyl}-cyclopropyl-amino)-hexanoic acid ethyl ester.

The title compound was prepared according to the procedure for EXAMPLE 379, step A using 6-bromo-hexanoic acid ethyl ester. MS (ESI): mass calculated for C₂₆H₃₂N₂O₃S, 452.21; m/z found, 453.4 [M+H]⁺. ¹H NMR (400 MHz, CDCl₃): 7.72 (d, *J* = 8.1, 1H), 7.64 (d, *J* = 8.1, 1H), 7.36 (t, *J* = 8.1, 1H), 7.28-7.20 (m, 5H), 4.12 (dd, *J* = 7.3, 7.1, 2H), 2.87-2.81 (m, 4H), 2.65 (t, *J* = 7.8, 2H), 2.30 (t, *J* = 7.6, 2H), 1.81-1.74 (m, 1H), 1.69-1.60 (m, 2H), 1.59-1.50 (m, 2H), 1.37-1.27 (m, 2H), 1.24 (t, *J* = 7.1, 3H), 0.52-0.46 (m, 2H), 0.43-0.37 (m, 2H).

### EXAMPLE 418

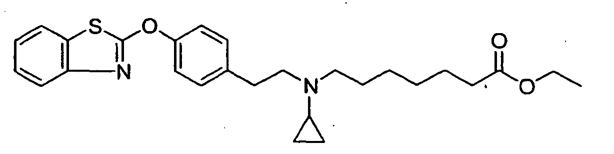

7-({2-[4-(Benzothiazol-2-yloxy)-phenyl]-ethyl}-cyclopropyl-amino)-heptanoic acid ethyl ester.

The title compound was prepared according to the procedure for EXAMPLE 379, step A using 7-bromo-heptanoic acid ethyl ester. MS (ESI): mass calculated for C₂₇H₃₄N₂O₃S, 466.23; m/z found, 467.4 [M+H]⁺. ¹H NMR (400 MHz, CDCl₃): 7.71 (d, *J* = 8.1, 1H), 7.60 (d, *J* = 8.1, 1H), 7.34 (t, *J* = 8.1, 1H), 7.27-7.18 (m, 5H), 4.10 (dd, *J* = 7.1, 7.1, 2H), 2.86-2.79 (m, 4H), 2.63 (t, *J* = 7.6, 2H), 2.28 (t, *J* = 7.6, 2H), 1.80-1.74 (m, 1H), 1.67-1.58 (m, 2H), 1.57-1.47 (m, 2H), 1.38-1.25 (m, 4H), 1.23 (t, *J* = 7.1, 3H), 0.50-0.44 (m, 2H), 0.43-0.37 (m, 2H).

### EXAMPLE 419

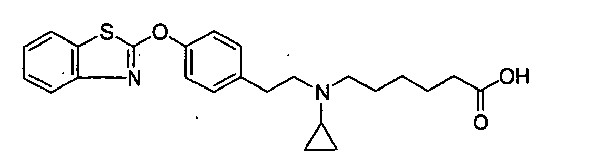

6-({2-[4-(Benzothiazol-2-yloxy)-phenyl]-ethyl}-cyclopropyl-amino)-hexanoic acid.

The title compound was prepared from EXAMPLE 417 following the procedure for EXAMPLE 379, step B. MS (ESI): mass calculated for C₂₄H₂₈N₂O₃S, 424.18; m/z found, 425.4 [M+H]⁺. ¹H NMR (400 MHz, CDCl₃): 11.17 (br s, 1H), 7.69 (d, *J* = 8.1, 1H), 7.65 (d, *J* = 8.1, 1H), 7.39-7.21 (m, 6H), 3.35-3.28 (m, 2H), 3.24-3.10 (m, 4H), 2.53-2.45 (m, 1H), 2.31 (t, *J* = 7.1, 2H), 1.92-1.81 (m, 2H), 1.70-1.61 (m, 2H), 1.44-1.34 (m, 4H), 0.91-0.83 (m, 2H).

### EXAMPLE 420

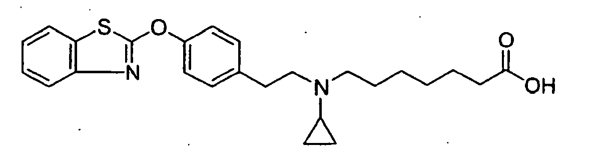

7-({2-[4-(Benzothiazo)-2-yloxy)-phenyl]-ethyl}-cyclopropyl-amino)-heptanoic acid.

The title compound was prepared from EXAMPLE 418 following the procedure for EXAMPLE 379, step B. MS (ESI): mass calculated for C₂₅H₃₀N₂O₃S, 438.2; m/z found, 439.4 [M+H]⁺. ¹H NMR (400 MHz, CDCl₃): 12.57 (br s, 1H), 7.72 (d, *J* = 8.1, 1H), 7.64 (d, *J* = 8.1, 1H), 7.36 (t, *J* = 8.1, 1H), 7.31-7.20 (m, 5H), 3.02-2.90 (m, 4H), 2.78 (t, *J* = 7.8, 2H), 2.26 (t, *J* = 7.6, 2H), 1.97-1.90 (m, 1H), 1.67-1.56 (m, 4H), 1.41-1.26 (m, 4H), 0.80-0.73 (m, 2H), 0.62-0.55 (m, 2H).

### EXAMPLE 421

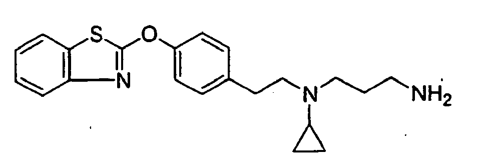

N-1-{2-[4-(Benzothiazol-2-yloxy)-phenyl]-ethyl}-N1-cyclopropyl-propane-1,3-diamine.

The title compound was prepared according to the procedures for EXAMPLE 378, step A using cyclopropylamine, EXAMPLE 379, step A using 2-(3-bromopropyl)isoindole-1,3-dione and EXAMPLE 403. MS (ESI): mass calculated for C₂₁H₂₅N₃OS, 367.17; m/z found, 468.4 [M+H]⁺. ¹H NMR (400 MHz, CDCl₃): 7.73 (d, *J* = 7.6, 1H), 7.65 (d, *J* = 7.8, 1H), 7.38 (t, *J* = 7.6, 1H), 7.29-7.23 (m, 5H), 2.89-2.82 (m, 4H), 2.71 (t, *J* = 7.1, 4H), 1.83-1.77 (m, 1H), 1.72-1.64 (m, 2H), 1.55 (br s, 2H), 0.54-0.48 (m, 2H), 0.44-0.39 (m, 2H).

### EXAMPLE 422

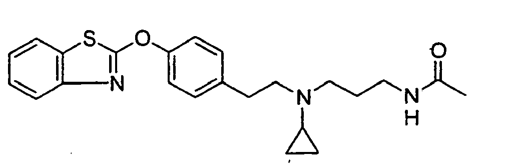

N-[3-({2-[4-(Benzothiazol-2-yloxy)-phenyl]-ethyl}-cyclopropyl-amino)-propyl]-acetamide.

The title compound was prepared from EXAMPLE 421 following the procedure for EXAMPLE 405. MS (ESI): mass calculated for C₂₃H₂₇N₃O₂S, 409.18; m/z found, 410.4 [M+H]⁺. ¹H NMR (400 MHz, CDCl₃): 7.71 (d, *J*= 8.1, 1H), 7.64 (d, *J* = 8.1, 1H), 7.36 (t, *J* = 8.0118, 1H), 7.28-7.21 (m, 5H), 6.77 (br s, 1H), 3.26 (dd, *J* = 6.1, 5.8, 2H), 2.89-2.83 (m, 4H), 2.75 (t, *J* = 6.8, 2H), 1.89 (s, 3H), 1.84-1.78 (m, 1H), 1.75-1.67 (m, 2H), 0.57-0.51 (m, 2H), 0.47-0.42 (m, 2H).

### EXAMPLE 423

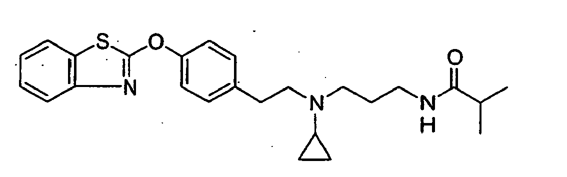

N-[3-({2-[4-(Benzothiazol-2-yloxy)-phenyl]-ethyl}-cyclopropyl-amino)-propyl]-isobutyramide.

The title compound was prepared according to the procedure for EXAMPLE 421, using isobutyryl chloride. MS (ESI): mass calculated for C₂₅H₃₁N₃O₂S, 437.21; m/z found, 438.4 [M+H]⁺. ¹H NMR (400 MHz, CDCl₃): 7.72 (d, *J* = 8.1, 1H), 7.66 (d, *J* = 8.1, 1H), 7.37 (t, *J* = 8.1, 1H), 7.30-7.23 (m, 5H), 6.54 (br s, 1H), 3.30 (dd, *J* = 6.1, 5.8, 2H), 2.90-2.83 (m, 4H), 2.77 (t, *J* = 6.3, 2H), 2.31-2.19 (m, 1H), 1.85-1.78 (m, 1H), 1.75-1.67 (m, 2H), 1.12 (d, *J* = 6.8, 6H), 0.58-0.52 (m, 2H), 0.45-0.40 (m, 2H).

### EXAMPLE 424

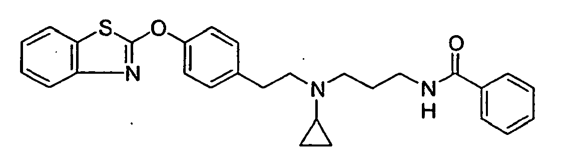

N-[3-({2-[4-(Benzothiazol-2-yloxy)-phenyl]-ethyl}-cyclopropyl-amino)-propyl]-benzamide.

The title compound was prepared according to the procedure for EXAMPLE 422, using benzoyl chloride. MS (ESI): mass calculated for C₂₈H₂₉N₃O₂S, 471.20; m/z found, 472.4 [M+H]⁺. ¹H NMR (400 MHz, CDCl₃): 7.78-7.67 (m, 4H), 7.61 (d, *J* = 7.8, 1H), 7.44-7.39 (m, 1H), 7.38-7.31 (m, 3H), 7.25-7.13 (m, 5H), 3.50 (dd, *J* = 6.1, 5.6, 2H), 2.89-2.77 (m, 6H), 1.85-1.75 (m, 3H), 0.56-0.49 (m, 2H), 0.45-0.38 (m, 2H).

### EXAMPLE 425

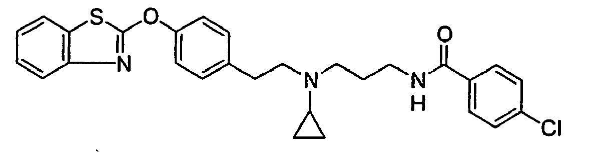

N-[3-({2-[4-(Benzothiazol-2-yloxyl)-phenyl]-ethyl}-cyclopropyl-amino)-propyl]-4-chloro-benzamide.

The title compound was prepared according to the procedure for EXAMPLE 422, using 4-chloro-benzoyl chloride. MS (ESI): mass calculated for C₂₈H₂₈ClN₃O₂S, 505.16; m/z found, 506.3 [M+H]⁺. ¹H NMR (400 MHz, CDCl₃): 7.80(t, *J* = 5.0, 1H), 7.68 (d, *J* = 7.6, 1H), 7.65-7.59 (m, 3H), 7.37-7.29 (m, 3H), 7.25-7.15 (m, 5H), 3.47 (dd, *J* = 6.1, 5.31, 2H), 2.89-2.77 (m, 6H), 1.84-1.75 (m, 3H), 0.57-0.51 (m, 2H), 0.42-0.36 (m, 2H).

### EXAMPLE 426

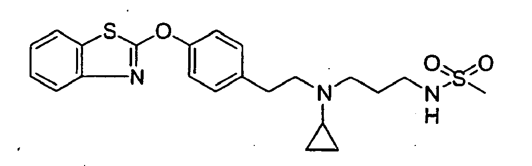

N-[3-({2-[4-(Benzothiazo)-2-yloxy)-phenyl]-ethyl}-cyclopropyl-amino)-propyl]-methanesulfonamide.

The title compound was prepared according to the procedure for EXAMPLE 422, using methanesulfonyl chloride. MS (ESI): mass calculated for C₂₂H₂₇N₃O₃S₂, 445.15; m/z found, 446.3 [M+H]⁺. ¹H NMR (400 MHz, CDCl₃): 7.72 (d, J = 7.8, 1 H), 7.64 (d, J = 7.8, 1 H), 7.36 (t, J = 7.8, 1 H), 7.28-7.21 (m, 5H), 6.03 (br s, 1H), 3.15 (t, *J* = 6.1, 2H), 2.88 (s; 3H), 2.87-2.82 (m, 4H), 2.78 (t, *J* = 6.1, 2H), 1.82-1.71 (m, 3H), 0.58-0.52 (m, 2H), 0.49-0.43 (m, 2H).

### EXAMPLE 427

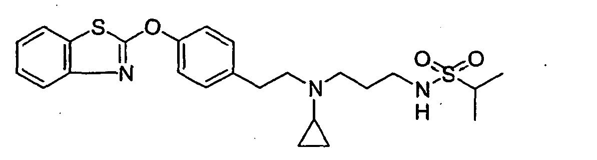

Propane-2-sulfonic acid [3-({2-[4-(benzothiazol-2-yloxy)-phenyl]-ethyl}-cyclopropyl-amino)-propyl]-amide trifluoromethansulfonic acid salt.

The title compound was prepared according to the procedure for EXAMPLE 422, using propane-2-sulfonyl chloride. MS (ESI): mass calculated for C₂₄H₃₁N₃O₃S₂, 473.18; m/z found, 474.4 [M+H]⁺. ¹H NMR (400 MHz, CD₃OD): 7.65 (d, *J* = 7.8, 1H), 7.53 (d, *J* = 8.1, 1H), 7.39-7.16 (m, 6H), 3.44 (t, *J* = 8.3, 2H), 3.37 (t, *J* = 8.3, 2H), 3.24-3.19 (m, 2H), 3.16-3.06 (m, 4H), 2.87-2.78 (m, 1H), 2.07-1.94 (m, 2H), 1.67 (d, *J* = 4.04, 3H), 1.23 (d, *J* = 6.1, 3H), 1.07-1.00 (m, 2H), 0.98-0.91 (m, 2H).

### EXAMPLE 428

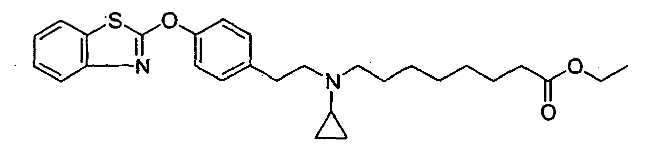

8-({2-[4-(Benzothiazol-2-yloxy)-phenyl]-ethyl}-cyclopropyl-amino)-octanoic acid ethyl ester.

The title compound was prepared according to the procedure for EXAMPLE 379, step A, using 8-bromo-octanoic acid ethyl ester. MS (ESI): mass calculated for C₂₈H₃₆N₂O₃S, 480.24; m/z found, 481.4 [M+H]⁺. ¹H NMR (400 MHz, CDCl₃): 7.72 (d, J = 8.1, 1H), 7.63 (d, J = 8.1, 1H), 7.36 (t, J = 8.1, 1H), 7.27-7.20 (m, 5H), 4.11 (dd, J = 7.3, 7.1, 2H), 2.87-2.81 (m, 4H), 2.64 (t, J = 7.3, 2H), 2.28 (t, J = 7.1, 2H), 1.82-1.75 (m, 1H), 1.66-1.58 (m, 2H), 1.56-1.47 (m, 2H), 1.36-1.27 (m, 6H), 1.24 (t, *J* = 7.1, 3H), 0.51-0.45 (m, 2H), 0.43-0.38 (m, 2H).

### EXAMPLE 429

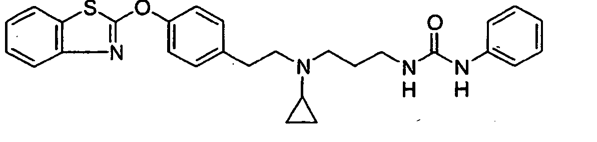

1-[3-({2-[4-(Benzothiazol-2-yloxy)-phenyl]-ethyl}-cyclopropyl-amino)-propyl]-3-phenyl-urea.

The title compound was prepared according to the procedure for EXAMPLE 422, using phenyl isocyanate. MS (ESI): mass calculated for C₂₈H₃₀N₄O₂S, 486.21; m/z found, 487.4 [M+H]⁺. ¹H NMR (400 MHz, CDCl₃): 7.61 (d, *J* = 8.1, 1H), 7.57 (d, *J* = 8.1, 1H), 7.28 (t, *J* = 8.1, 1H), 7.20-7.09 (m, 10H), 6.89 (t, *J* = 7.3, 1H), 5.64 (br s, 1H), 3.16-3.09 (m, 2H), 2.70-2.64 (m, 4H), 2.57 (t, *J* = 7.1, 2H), 1.67-1.60 (m, 1H), 1.60-1.52 (m, 2H), 0.39-0.33 (m, 2H), 0.25-0.20 (m, 2H).

### EXAMPLE 430

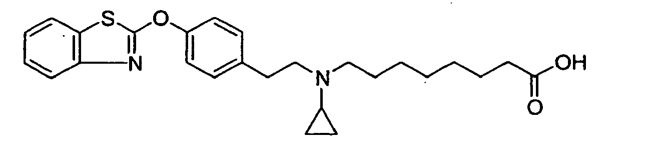

8-({2-[4-(Benzothiazol-2-yloxy)-phenyl]-ethyl}-cyclopropyl-amino)-octanoic acid.

The title compound was prepared from EXAMPLE 428 following the procedure for EXAMPLE 379, step B. MS (ESI): mass calculated for C₂₆H₃₂N₂O₃S, 452.21; m/z found, 453.4 [M+H]⁺. ¹H NMR (400 MHz, CDCl₃): 12.76 (br s, 1H), 7.72 (d, J = 7.6, 1H), 7.63 (d, J = 7.8, 1 H), 7.36 (t, J = 7.6, 1 H), 7.30-7.21 (m, 5H), 3.01-2.88 (m, 4H), 2.77 (t, J = 8.1, 2H), 2.27 (t, J = 7.8, 2H), 1.96-1.88 (m, 1H), 1.66-1.54 (m, 4H), 1.38-1.23 (m, 6H), 0.77-0.70 (m, 2H), 0.61-0.54 (m, 2H).

### EXAMPLE 431

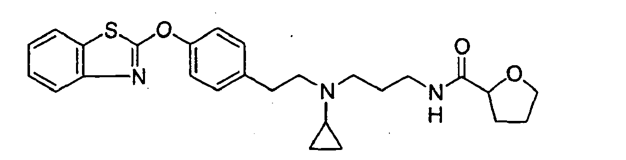

Tetrahydro-furan-2-carboxylic acid [3-({2-[4-(benzothiazol-2-yloxy)-phenyl]-ethyl}-cyclopropyl-amino)-propyl]-amide.

To a solution of N-1-{2-[4-(Benzothiazol-2-yloxy)-phenyl]-ethyl}-N1-cyclopropyl-propane-1,3-diamine (Example 421, 330 mg, 0.9 mmol) in CH₂Cl₂ (18 mL) was added 2-tetrahydrofuroic acid (129 µL, 1.35 mmol) and Si-carbodiimide (1.05 mmol/g, 1.71 g, 1.8 mmol). The reaction mixture was stirred at room temperature overnight, filtered and concentrated. The crude product was purified on SiO₂ (40 g; 0-10% CH₃OH/CH₂Cl₂) to give a white solid (267 mg, 64% yield). MS (ESI): mass calculated for C₂₆H₃₁N₃O₃S, 465.21; m/z found, 466.4 [M+H]⁺. ¹H NMR (400 MHz, CDCl₃): 7.72 (d, *J* = 8.1, 1H), 7.64 (d, *J* = 8.1, 1H), 7.36 (t, *J* = 8.1, 1H), 7.32 (br s, 1H), 7.29-7.21 (m, 5H), 4.32 (dd, *J* = 6.1, 2.3, 1H), 3.93-3.81 (m, 2H), 3.41-3.31 (m, 1H), 3.30-3.21 (m, 1H), 2.90-2.80 (m, 4H), 2.75 (t, *J* = 6.8, 2H), 2.32-2.21 (m, 1H), 2.08-1.97 (m, 1H), 1.92-1.77 (m, 3H), 1.76-1.67 (m, 2H), 0.56-0.49 (m, 2H), 0.48-0.41 (m, 2H).

### EXAMPLE 432

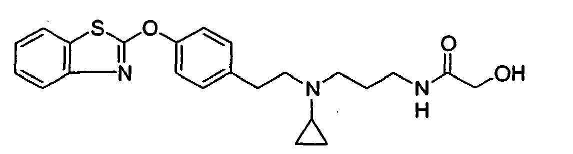

N-[3-({2-[4-(Benzothiazol-2-yloxy)-phenyl]-ethyl}-cyclopropyl-amino)-propyl]-2-hydroxy-acetamide.

The title compound was prepared according to the procedure for EXAMPLE 431 using gycolic acid. MS (ESI): mass calculated for C₂₃H₂₇N₃O₃S, 425.18; m/z found, 426.3 [M+H]⁺. ¹H NMR (400 MHz, CDCl₃): 7.73-7.63 (m, 2H), 7.44 (br s, 1H), 7.40-7.33 (m, 1H), 7.29-7.18 (m, 5H), 4.21 (s, 1H), 3.91 (s, 2H), 3.37-3.28 (m, 2H), 2.90-2.82 (m, 4H), 2.75 (t, *J* = 6.1, 2H), 1.85-1.78 (m, 1H), 1.77-1.70 (m, 2H), 0.58-0.38 (m, 4H).

### EXAMPLE 433

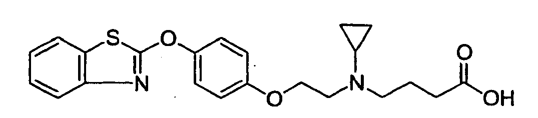

4-({2-[4-(Benzothiazol-2-yloxy)-phenoxy]-ethyl}-cyclopropyl-amino)-butyric acid.
A. {2-[4-(Benzothiazol-2-yloxy)-phenoxy]-ethyl}-cyclopropyl-amine. To a stirring solution of 2-[4-(2-bromo-ethoxy)-phenoxy]-benzothiazole (EXAMPLE 9, 1.1 g, 3.14 mmol) in CH₃CN (31 mL) was added cyclopropylamine (1.09 mL, 15.7 mmol) and *N*,*N*-diisopropylethylamine (1.1 mL, 6.28 mmol). The mixture was heated to 60 °C for 16 h, cooled to room temperature, and then dissolved in CH₂Cl₂ (100 mL). The solution was washed with H₂O (2 x 20 mL), dried, and concentrated. The resultant oil purified on SiO₂ (40 g, 0-15% CH₃OH/CH₂Cl₂) to give a clear oil (999 mg, 98% yield). MS (ESI): mass calculated for C₁₈H₁₈N₂O₂S, 326.11; m/z found, 327.2 [M+H]⁺. ¹H NMR (400 MHz, CDCl₃): 7.71 (d, *J* = 8.1, 1H), 7.59 (d, *J* = 8.1, 1H), 7.33 (t, *J* = 8.1, 1H), 7.27-7.17 (m, 3H), 6.95-6.89 (m, 2H), 4.03 (t, *J* = 5.3, 2H), 3.06 (t, *J* = 5.3, 2H), 2.20-2.14 (m, 1H), 2.02 (bs, 1H), 0.47-0.41 (m, 2H), 0.39-0.34 (m, 2H).
B. 4-({2-[4-(Benzothiazol-2-yloxy)-phenoxy]-ethyl}-cyclopropyl-amino)-butyric acid ethyl ester. To a stirring solution of {2-[4-(benzothiazol-2-yloxy)-phenoxy]-ethyl}-cyclopropyl-amine (950 mg, 2.91 mmol) in CH₃CN (29 mL) was added 4-bromo-butyric acid ethyl ester (625 µL, 4.37 mmol) and *N*,*N-*diisopropylethylamine (1.0 mL, 5.82 mmol). The mixture was heated to 60 °C for 16 h, cooled to room temperature, and then dissolved in CH₂Cl₂ (100 mL). The solution was washed with H₂O (2 x 20 mL), dried, and concentrated. The resultant oil was purified on SiO₂ (40 g, 0-50% EtOAc/Hexanes) to give a colorless oil (1.0 g, 78% yield). MS (ESI): mass calculated for C₂₄H₂₈N₂O₄S, 440.18; m/z found, 441.3 [M+H]⁺. ¹H NMR (400 MHz, CDCl₃): 7.71 (d, *J* = 8.1, 1H), 7.61 (d, *J* = 8.1, 1H), 7.34 (t, *J* = 8.1, 1H), 7.28-7.18 (m, 3H), 6.95-6.90 (m, 2H), 4.14-4.04 (m, 4H), 3.00 (t, *J* = 6.3, 2H); 2.71 (t, *J* = 7.1, 2H), 2.31 (t, *J* = 7.1, 2H), 1.90-1.80 (m, 3H), 1.23 (t, *J* = 7.1, 3H), 0.51-0.44 (m, 2H), 0.43-0.38 (m, 2H).
C. 4-({2-[4-(Benzothiazol-2-yloxy)-phenoxy]-ethyl}-cyclopropyl-amino)-butyric acid. To a solution of 4-({2-[4-(Benzothiazol-2-yloxy)-phenoxy]-ethyl}-cyclopropyl-amino)-butyric acid ethyl ester (970 mg, 2.2 mmol) in 3:1 THF/CH₃OH (80 mL), was added lithium hydroxide (211 mg, 8.8 mmol) in water (20 mL). This solution was stirred at room temperature for 16 h and then adjusted the pH to 7 using 1 N aqueous HCl. Extracted the mixture with CH₂Cl₂ (2 x 100 mL). The combined organic phase was concentrated under reduced pressure. The resultant oil was purified on SiO₂ (40 g, 0-10% CH₃OH/CH₂Cl₂) to give a white solid (877 mg, 97% yield). MS (ESI): mass calculated for C₂₂H₂₄N₂O₄S, 412.15; m/z found, 413.3 [M+H]⁺. ¹H NMR (400 MHz, CDCl₃): 11.44 (br s, 1H), 7.68 (d, *J* = 8.1, 1H), 7.62 (d, *J* = 8.1, 1H), 7.35 (t, *J* = 8.1, 1H), 7.27-7.20 (m, 3H), 7.01-6.95 (m, 2H), 4.44 (t, *J* = 5.0, 2H), 3.56 (t, *J* = 5.005, 2H), 3.32 (t, *J* = 8.1, 2H), 2.67-2.60 (m, 1H), 2.46 (t, *J* = 6.8, 2H), 2.18-2.09 (m, 2H), 1.32-1.25 (m, 2H), 0.88-0.81 (m, 2H).

### EXAMPLE 434

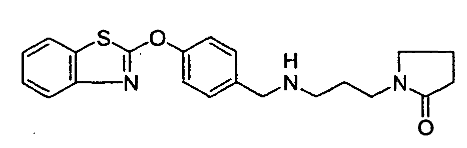

1-{3-[4-(Benzothiazol-2-yloxy)-benzylamino]-propyl}-pyrrolidin-2-one.

The title compound was prepared according to the procedure for EXAMPLE 251, step B using 1-(3-amino-propyl)-pyrrolidine-2-one. MS (ESI): mass calculated for C₂₁H₂₃N₃O₂S, 381.15; m/z found, 382.3 [M+H]⁺. ¹H NMR (400 MHz, CDCl₃): 7.71 (d, *J* = 8.1, 1H), 7.64 (d, *J* = 8.1, 1H), 7.45-7.41 (m, 2H), 7.36 (t, *J* = 8.1, 1H), 7.32-7.28 (m, 2H), 7.24 (t, *J* = 8.1, 1H), 3.81 (s, 2H), 3.38-3.27 (m, 5H), 3.63 (t, *J* = 6.8, 2H), 3.35 (t, *J* = 7.6, 2H), 2.02-1.93 (m, 2H), 1.82-1.73 (m, 2H).

### EXAMPLE 435

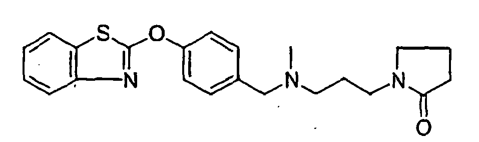

1-(3-{[4-(Benzothiazol-2-yloxy)-benzyl]-methyl-amino}-propyl)-pyrrolidin-2-one.

The title compound was prepared according to the procedure for EXAMPLE 409 using formaldehyde. MS (ESI): mass calculated for C₂₂H₂₅N₃O₂S, 395.17; m/z found, 396.4 [M+H]⁺. ¹H NMR (400 MHz, CDCl₃): 7.71 (d, *J* = 8.1, 1H), 7.64 (d, *J* = 8.1, 1H), 7.43-7.27 (m, 5H), 7.24 (t, *J* = 8.1, 1H), 3.54 (s, 2H), 3.32 (dd, *J* = 7.1, 7.1, 4H), 2.43 (t, *J* = 7.1, 2H), 2.34 (t, *J* = 7.1, 2H), 2.23 (s, 3H), 2.00-1.91 (m, 2H), 1.80-1.70 (m, 2H).

### EXAMPLE 436

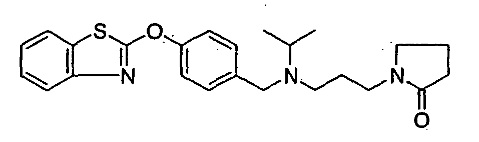

1-(3-{[4-(Benzothiazol-2-yloxy)-benzyl]-isopropyl-amino}-propyl)-pyrrolidin-2-one.

The title compound was prepared according to the procedure for EXAMPLE 409 using acetone. MS (ESI): mass calculated for C₂₄H₂₉N₃O₂S, 423.20; m/z found, 424.4 [M+H]⁺. ¹H NMR (400 MHz, CDCl₃): 7.72 (d, *J* = 8.1, 1H), 7.65 (d, *J* = 8.1, 1H), 7.45-7.40 (m, 2H), 7.37 (t, *J* = 7.8, 1H), 7.30-7.21 (m, 3H), 3.55 (s, 2H), 3.29-3.20 (m, 4H), 3.01-2.90 (m, 1H), 2.42 (t, *J* = 7.1, 2H), 2.32 (t, *J* = 7.8, 2H), 1.97-1.83 (m, 2H), 1.63-1.53 (m, 2H), 1.02 (d, *J* = 6.6, 6H).

### EXAMPLE 437

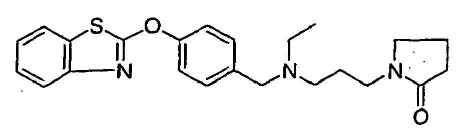

1-(3-{[4-(Benzothiazol-2-yloxy)-benzyl]-ethyl-amino}-propyl)-pyrrolidin-2-one.

The title compound was prepared according to the procedure for EXAMPLE 409 using acetaldehyde. MS (ESI): mass calculated for C₂₃H₂₇N₃O₂S, 409.18; m/z found, 410.4 [M+H]⁺. ¹H NMR (400 MHz, CDCl₃): 7.72 (d, *J* = 8.1, 1H), 7.66 (d, *J* = 8.1, 1H), 7.43-7.34 (m, 3H), 7.32-7.23 (m, 3H), 3.56 (s, 2H), 3.30 (dd, *J* = 7.1, 7.1, 4H), 2.53 (dd, *J* = 7.1, 7.1, 2H), 2.45 (t, *J* = 7.1, 2H), 2.34 (t, *J* = 7.8, 2H), 2.00-1.91 (m, 2H), 1.73-1.64 (m, 2H), 1.04 (t, *J* = 7.1, 3H).

### EXAMPLE 438

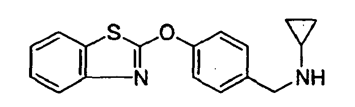

[4-(Benzothiazol-2-yloxy)-benzyl]-cyclopropyl-amine.

The title compound was prepared according to the procedure for EXAMPLE 251, step B using cyclopropylamine. MS (ESI): mass calculated for C₁₇H₁₆N₂OS, 296.10; m/z found, 297.3 [M+H]⁺. ¹H NMR (400 MHz, CDCl₃): 7.72 (d, *J* = 8.1, 1H), 7.63 (d, *J* = 8.1, 1H), 7.40-7.33 (m, 3H), 7.32-7.27 (m, 2H), 7.24 (t, *J* = 8.1, 1H), 3.85 (s, 2H), 2.19-2.12 (m, 1H), 1.86 (br s, 1H), 0.48-0.35 (m, 4H).

### EXAMPLE 439

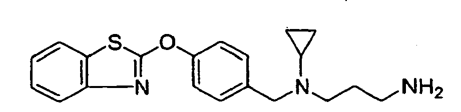

N-1-[4-(Benzothiazol-2-yloxy)-benzyl]-N1-cyclopropyl-propane-1,3-diamine.

The title compound was prepared according to the procedure for EXAMPLE 421 using compound from EXAMPLE 438. MS (ESI): mass calculated for C₂₀H₂₃N₃OS, 353.16; m/z found, 354.3 [M+H]⁺. ¹H NMR (400 MHz, CDCl₃): 7.73 (d, *J* = 8.1, 1H), 7.65 (d, *J* = 8.1, 1H), 7.41-7.32 (m, 3H), 7.30-7.23 (m, 3H), 3.75 (s, 2H), 2.67 (t, *J* = 7.1, 2H), 2.58 (t, *J* = 7.1, 2H), 1.80-1.73 (m, 1H), 1.72-1.64 (m, 2H), 1.19 (br s, 2H), 0.52-0.46 (m, 2H), 0.42-0.36 (m, 2H).

### EXAMPLE 440

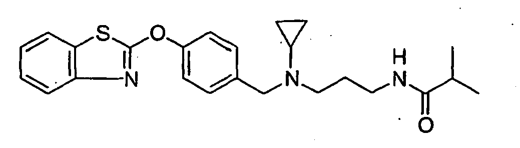

N-(3-{[4-(Benzothiazol-2-yloxy)-benzyl]-cyclopropyl-amino}-propyl)-isobutyramide.

The title compound was prepared according to the procedure for EXAMPLE 422 using isobutyryl chloride. MS (ESI): mass calculated for C₂₄H₂₉N₃O₂S, 423.20; m/z found, 424.4 [M+H]⁺. ¹H NMR (400 MHz, CDCl₃): 7.72 (d, *J* = 8.1, 1H), 7.65 (d, *J* = 8.1, 1H), 7.40-7.22 (m, 6H), 6.26 (br s, 1H), 3.73 (s, 2H), 3.23 (dd, *J* = 6.3, 5.8, 2H), 2.61 (t, *J* = 6.6, 2H), 2.29-2.18 (m, 1H), 1.79-1.68 (m, 3H), 1.09 (d, *J* = 6.8, 6H), 0.54-0.47 (m, 2H), 0.43-0.37 (m, 2H).

### EXAMPLE 441

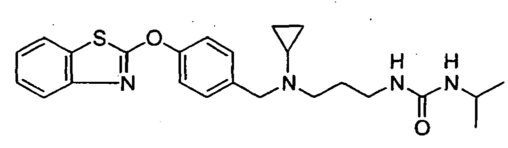

1-(3-{[4-(Benzothiazo)-2-yloxy)-benzyl]-cyclopropyl-amino}-propyl)-3-isopropyl-urea.

The title compound was prepared according to the procedure for EXAMPLE 422 using isopropyl isocyanate. MS (ESI): mass calculated for C₂₄H₃₀N₄O₂S, 438.21; m/z found, 439.4 [M+H]⁺. ¹H NMR (400 MHz, CDCl₃): 7.69 (d, *J* = 8.1, 1H), 7.63 (d, *J* = 8.1, 1H), 7.38-7.29 (m, 3H), 7.27-7.20 (m, 3H), 5.44 (br s, 1H), 5.15 (d, *J* = 7.8, 1H), 3.87-3.76 (m, 1H), 3.70 (s, 2H), 3.12 (dd, *J* = 6.3, 6.1, 2H), 2.56 (t, *J* = 7.1, 2H), 1.76-1.67 (m, 3H), 1.07 (d, *J* = 6.3, 6H), 0.49-0.42 (m, 2H), 0.40-0.34 (m, 2H).

### EXAMPLE 442

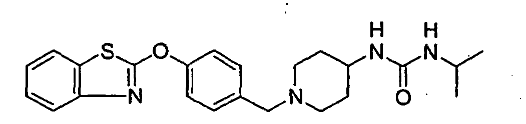

1 -{1-[4-(Benzothiazol-2-yloxy)-benzyl]-piperidin-4-yl}-3-isopropyl-urea.

The title compound was prepared according to the procedure for EXAMPLE 253, step C using isopropyl isocyanate. MS (ESI): mass calculated for C₂₃H₂₈N₄O₂S, 424.19; m/z found, 425.3 [M+H]⁺. ¹H NMR (400 MHz, CDCl₃): 7.73 (d, *J* = 8.1, 1H), 7.67 (d, *J* = 8.1, 1H), 7.41-7.35 (m, 3H), 7.32-7.24 (m, 3H), 4.77 (dd, *J* = 8.1, 7.8, 1H), 3.88-3.78 (m, 1H), 3.66-3.55 (m, 1H), 3.51 (s, 2H), 2.82 (d, *J* = 11.6, 2H), 2.13 (t, *J* = 10.9, 2H), 1.94 (d, *J* = 11.9, 2H), 1.69 (br s, 1H), 1.48-1.36 (m, 2H), 1.14 (d, *J* = 6.6, 6H).

### EXAMPLE 443

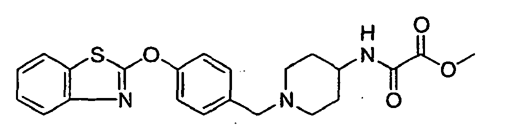

N-{1-[4-(Benzothiazol-2-yloxy)-benzyl]-piperidin-4-yl}-oxalamic acid methyl ester.

The title compound was prepared according to the procedure for EXAMPLE 253, step C using chloro-oxo-acetic acid methyl ester. MS (ESI): mass calculated for C₂₂H₂₃N₃O₄S, 425.14; m/z found, 426.2 [M+H]⁺. ¹H NMR (400 MHz, CDCl₃): 7.78 (d, *J* = 8.1, 1H), 7.64 (d, *J* = 8.1, 1H), 7.55-7.50 (m, 2H), 7.44-7.36 (m, 3H), 7.31 (t, *J* = 8.1, 1H), 3.84 (s, 2H), 3.81-3.72 (m, 1H), 3.41 (s, 3H), 3.11 (d, *J* = 10.9, 2H), 2.59-2.47 (m, 2H), 2.00-1.91 (m, 2H), 1.79-1.66 (m, 2H).

### EXAMPLE 444

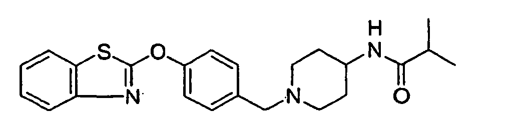

N-{1-[4-(Benzothiazol-2-yloxy)-benzyl]-piperidin-4-yl}-isobutyramide.

The title compound was prepared according to the procedure for EXAMPLE 253 using isobutyryl chloride. MS (ESI): mass calculated for C₂₃H₂₇N₃O₂S, 409.18; m/z found, 410.3 [M+H]⁺. ¹H NMR (400 MHz, CDCl₃): 7.84 (d, *J* = 8.6, 2H), 7.69 (t, *J* = 7.8, 2H), 7.44 (d, *J* = 8.6, 2H), 7.41-7.33 (m, 2H), 7.27 (t, *J* = 8.1, 1H), 4.33 (s, 2H), 4.16-4.00 (m, 1H), 3.57-3.42 (m, 2H), 3.31-3.16 (m, 2H), 2.58-2.45 (m, 1H), 2.31-2.07 (m, 4H), 1.10 (d, *J* = 6.8, 6H).

### EXAMPLE 445

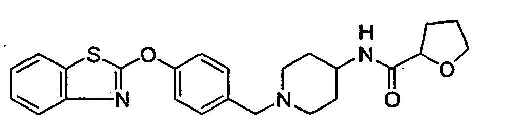

Tetrahydro-furan-2-carboxylic acid {1-[4-(benzothiazol-2-yloxy)-benzyl]-piperidin-4-yl}-amide.

The title compound was prepared from EXAMPLE 253, step D following the procedure for EXAMPLE 431. MS (ESI): mass calculated for C₂₄H₂₇N₃O₃S, 437.18; m/z found, 438.3 [M+H]⁺. ¹H NMR (400 MHz, CDCl₃): 11.03 (br s, 1H), 7.73 (d, *J* = 7.8, 1H), 7.67 (d, *J* = 7.8, 1H), 7.46-7.42 (m, 2H), 7.41-7.32 (m, 3H), 7.27 (d, *J* = 7.8, 1H), 4.31 (t, *J* = 8.34, 1H), 3.98-3.89 (m, 1H), 3.84 (s, 2H), 3.19 (d, *J* = 11.6, 2H), 2.43 (t, *J* = 10.4, 2H), 2.34-2.21 (m, 2H), 2.08-1.69 (m, 8H).

### EXAMPLE 446

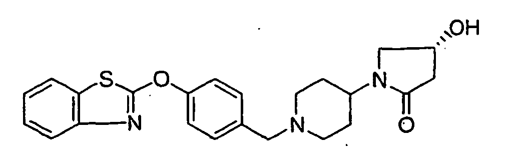

1-{1-[4-(Benzothiazol-2-yloxy)-benzyl]-piperidin-4-yl}-4-hydroxy-pyrrolidin-2-one.

The title compound was prepared from EXAMPLE 253, step D following the procedure for EXAMPLE 254, step D. MS (ESI): mass calculated for C₂₃H₂₅N₃O₃S, 423.16; m/z found, 424.3 [M+H]⁺. ¹H NMR (400 MHz, CD₃OD): 7.80 (d, *J* = 8.1, 1H), 7.68-7.62 (m, 3H), 7.53-7.49 (m, 2H), 7.42 (t, *J* = 8.1, 1H), 7.32 (t, *J* = 8.3, 1H), 4.44 (t, *J* = 6.3, 1H), 4.38 (s, 2H), 4.25-4.15 (m, 1H), 3.68-3.57 (m, 3H), 3.33-3.13 (m, 3H), 2.71 (dd, *J* = 10.9, 6.3, 1H), 2.28 (t, *J* = 17.7, 1H), 2.13-2.02 (m, 2H), 2.01-1.92 (m, 2H).

### EXAMPLE 447

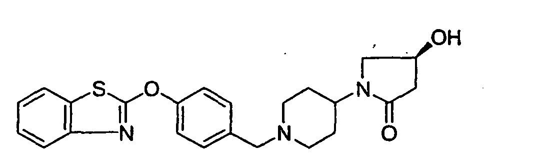

1-{1-[4-(Benzothiazol-2-yloxy)-benzyl]-pipendin-4-yl}-4-hydroxy-pyrrolidin-2-one.

The title compound was prepared from EXAMPLE 253, step D following the procedure for EXAMPLE 254, step D. MS (ESI): mass calculated for C₂₃H₂₅N₃O₃S, 423.16; m/z found, 424.3 [M+H]⁺. ¹H NMR (400 MHz, CD₃OD): 7.80 (d, *J* = 8.1, 1H), 7.68-7.62 (m, 3H), 7.53-7.49 (m, 2H), 7.42 (t, *J* = 8.1, 1H), 7.32 (t, *J* = 8.3, 1H), 4.44 (t, *J* = 6.3, 1H), 4.38 (s, 2H), 4.25-4.15 (m, 1H), 3.68-3.57 (m, 3H), 3.33-3.13 (m, 3H), 2.71 (dd, *J* = 10.9, 6.3, 1H), 2.28 (t, *J* = 17.7, 1H), 2.13-2.02 (m, 2H), 2.01-1.92 (m, 2H).

### EXAMPLE 448

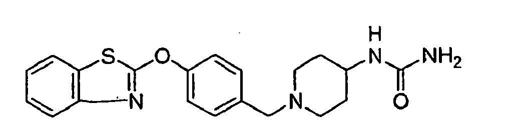

{1-[4-(Benzothiazol-2-yloxy)-benzyl]-piperidin-4-yl}-urea.

The title compound was prepared according to the procedure for EXAMPLE 253, step C using trimethylsilyl isocyanate. MS (ESI): mass calculated for C₂₀H₂₂N₄O₂S, 382.15; m/z found, 383.3 [M+H]⁺. ¹H NMR (400 MHz, CDCl₃): 7.71 (d, *J* = 7.8, 1H), 7.63 (d, *J* = 7.8, 1H), 7.40-7.33 (m, 3H), 7.30-7.21 (m, 3H), 5.82 (d, *J* = 7.8, 1H), 5.10 (d, *J* = 4.6, 1H), 3.84 (s, 1H), 3.56 (br s, 1H), 3.49 (s, 2H), 2.81 (d, *J* = 11.6, 2H), 2.12 (t, *J* = 11.1, 2H), 1.90 (d, *J* = 1.19, 2H), 1.51-1.39 (m, 2H).

### EXAMPLE 449

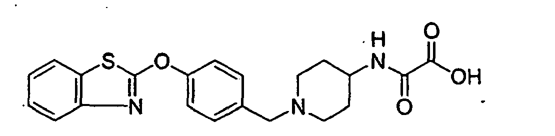

N-{1-[4-(Benzothiazol-2-yloxy)-benzyl]-piperidin-4-yl}-oxalamic acid.

The title compound was prepared from EXAMPLE 443 following the procedure for EXAMPLE 433, step C. MS (ESI): mass calculated for C₂₁H₂₁N₃O₄S, 411.13; m/z found, 412.3 [M+H]⁺. ¹H NMR (400 MHz, CD₃OD): 7.78 (d, *J* = 8.1, 1H), 7.64 (d, *J* = 8.1, 1H), 7.55-7.50 (m, 2H), 7.44-7.36 (m, 3H), 7.31 (t, *J* = 8.1, 1H), 3.84 (s, 2H), 3.81-3.72 (m, 1H), 3.11 (d, *J* = 10.9, 2H), 2.59-2.47 (m, 2H), 2.00-1.91 (m, 2H), 1.79-1.66 (m, 2H).

### EXAMPLE 450

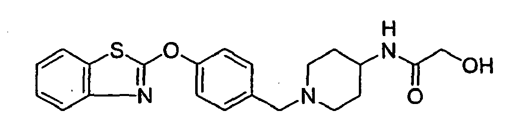

N-{1-[4-(Benzothiazol-2-yloxy)-benzyl]-piperidin-4-yl}-2-hydroxy-acetamide.

The title compound was prepared according to the procedure for EXAMPLE 431 using glycolic acid. MS (ESI): mass calculated for C₂₁H₂₃N₃O₃S, 397.15; m/z found, 398.3 [M+H]⁺. ¹H NMR (400 MHz, CDCl₃): 7.71 (d, J = 7.8, 1H), 7.65 (d, J = 8.1, 1 H), 7.40-7.35 (m, 3H), 7.31-7.23 (m, 3H), 6.83 (d, J = 8.1, 1H), 5.33, (br s, 1H), 3.99 (s, 2H), 3.87-3.75 (m, 1H), 3.50 (s, 2H), 2.85 (d, *J* = 11.4, 2H), 2.14 (t, *J* = 10.9, 2H), 1.92 (d, *J* = 12.6, 2H), 1.56-1.43 (m, 2H).

### EXAMPLE 451

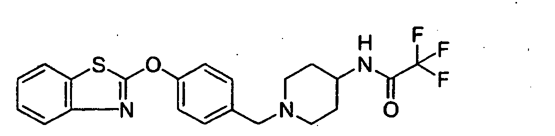

N-{1-[4-(Bezothiazol-2-yloxy)-benzyl]-piperidin-4-yl}-2,2,2-trifluoro-acetamide.

The title compound was prepared according to the procedure for EXAMPLE 431 using trifluoroacetic acid. MS (ESI): mass calculated for C₂₁H₂₀F₃N₃O₂S, 435.12; m/z found, 436.3 [M+H]⁺. ¹H NMR (400 MHz, CDCl₃): 7.71 (d, *J* = 8.3, 2H), 7.54 (d, *J* = 8.6, 2H), 7.47 (d, *J* = 8.8, 2H), 7.41 (t, *J* = 8.3, 1H), 7.34-7.28 (m, 1H), 4.24 (s, 2H), 4.15-4.02 (m, 1H), 3.59 (d, *J* = 12.1, 2H), 3.40, (br s, 1H), 2.87 (t, *J* = 11.9, 2H), 2.22-2.02 (m, 4H).

### EXAMPLE 452

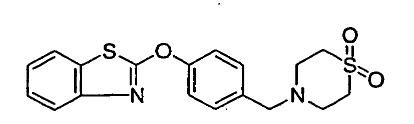

2-[4-(1,1-Dioxo-1l6-thiomorpholin-4-ylmethyl)-phenoxy]-benzothiazole.

The title compound was prepared according to the procedure for EXAMPLE 251 using thiomorpholine -1,1-dioxide. MS (ESI): mass calculated for C₁₈H₁₈N₂O₃S₂, 374.08; m/z found, 375.2 [M+H]⁺. ¹H NMR (400 MHz, CDCl₃): 7.72 (d, *J* = 8.1, 1H), 7.67 (d, *J* = 8.1, 1H), 7.42-7.31 (m, 5H), 7.27 (t, *J* = 7.8, 1H), 3.66 (s, 2H), 3.03 (d, *J* = 26.3, 8H).

### EXAMPLE 453

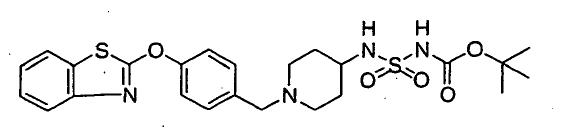

N-{1-[4-(benzothiazol-2-yloxy)-benzyl]-piperidin-4-amino sulfonyl}-carbamic acid tert-butyl ester

The title compound was prepared according to the procedure for EXAMPLE 253 using carbamic acid, N-(sulfonyl chloride) tert butyl ester. MS (ESI): mass calculated for C₂₄H₃₀N₄O₅S₂, 518.17; m/z found, 519.5 [M+H]⁺. ¹H NMR (400 MHz, CDCl₃): 7.73 (d, *J* = 8.2, 1H), 7.67 (d, *J* = 8.2, 1H), 7.42-7.35 (m, 3H), 7.34-7.24 (m, 3H), 5.18 (br s, 1H), 3.55 (s, 2H), 3.39-3.29 (m, 1H), 2.83 (d, *J* = 11.7, 2H), 2.31-2.18 (m, 2H), 1.98 (d, *J* = 11.7, 2H), 1.72-1.59 (m, 2H), 1.47 (s, 9H).

### EXAMPLE 454

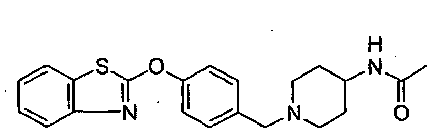

N-{1-[4-(Benzothiazol-2-yloxy)-benzyl]-piperidin-4-yl}-acetamide.

The title compound was prepared according to the procedure for EXAMPLE 253, step C using acetyl chloride. MS (ESI): mass calculated for C₂₁H₂₃N₃O₂S, 381.15; m/z found, 382.4 [M+H]⁺. ¹H NMR (400 MHz, CDCl₃): 7.73 (d, *J* = 7.8, 1H), 7.67 (d, *J* = 8.1, 1H), 7.43-7.36 (m, 3H), 7.33-7.24 (m, 3H), 5.65 (br s, 1H), 3.87-3.76 (m, 1H), 3.54 (s, 2H), 2.86 (d, *J* = 12.1, 2H), 2.17 (t, *J* = 11.4, 2H), 1.97 (s, 3H), 1.93 (d, *J* = 11.9, 2H), 1.56-1.44 (m, 2H).

### EXAMPLE 455

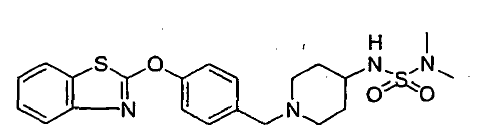

N-{1-[4-(Benzothiazol-2-yloxy)-benzyl]-piperidin-4-yl}-N,N-dimethylsulfamide

The title compound was prepared according to the procedure for EXAMPLE 253, step C using N,N-dimethyl-sulfamoyl chloride. MS (ESI): mass calculated for C₂₁H₂₆N₄O₃S₂, 446.14; m/z found, 447.4 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.32-7.23 (m, 3H), 4.37 (d, *J* = 7.8, 1H), 3.49 (s, 2H), 3.28-3.17 (m, 1H), 2.81 (d, *J* = 10.9, 2H), 2.78 (s, 6H), 2.11 (t, *J* = 11.1, 2H), 1.98 (d, *J* = 10.9, 2H), 1.61-1.50 (m, 2H).

### EXAMPLE 456

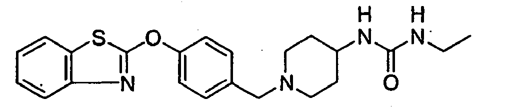

1-{1-[4-(Benzothiazol-2-yloxy)-benzyl]-piperidin-4-yl}-3-ethyl-urea.

The title compound was prepared according to the procedure for EXAMPLE 253, step C using ethyl isocyanate. MS (ESI): mass calculated for C₂₂H₂₆N₄O₂S, 410.18; m/z found, 411.5 [M+H]⁺. ¹H NMR (400 MHz, CDCl₃): 7.72 (d, *J* = 8.1, 1H), 7.66 (d, *J* = 8.1, 1H), 7.42-7.34 (m, 3H), 7.32-7.23 (m, 3H), 4.54 (br s, 1H), 4.45 (d, *J* = 7.6, 1H), 3.67-3.55 (m, 1H), 3.50 (s, 2H), 3.23-3.14 (m, 2H), 2.80 (d, *J* = 11.9, 2H), 2.13 (t, *J* = 11.4, 2H), 1.93 (d, *J* = 12.6, 2H), 1.48-1.36 (m, 2H), 1.12 (t, *J* = 7.3, 3H).

### EXAMPLE 457

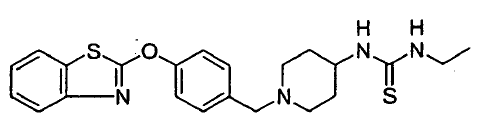

1-{1-[4-(Benzothiazol-2-yloxy)-benzyl]-piperidin-4-yl}-3-ethyl-thiourea.

The title compound was prepared according to the procedure for EXAMPLE 253, step C using ethyl isothiocyanate. MS (ESI): mass calculated for C₂₂H₂₆N₄OS₂, 426.15; m/z found, 427.4 [M+H]⁺. ¹H NMR (400 MHz, CDCl₃): 7.71 (d, *J* = 7.8, 1H), 7.67 (d, *J* = 8.1, 1H), 7.41-7.34 (m, 3H), 7.32-7.24 (m, 3H), 6.03 (br s, 1H), 5.74 (br s, 1H), 4.20-4.00 (m, 1H), 3.51 (s, 2H), 3.47-3.35 (m, 2H), 2.82 (d, *J* = 11.6, 2H), 2.05 (t, *J* = 11.1, 2H), 2.05 (d, *J* = 11.9, 2H), 1.57-1.45 (m, 2H), 1.21 (t, *J* = 7.3, 3H).

### EXAMPLE 458

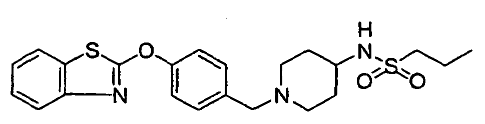

Propane-1-sulfonic acid {1-[4-(benzothiazol-2-yloxy)-benzyl]-piperidin-4-yl}-amide.

The title compound was prepared according to the procedure for EXAMPLE 253, step C using 1-propanesulfonyl chloride. MS (ESI): mass calculated for C₂₂H₂₇N₃O₃S₂, 445.15; m/z found, 446.4 [M+H]⁺. ¹H NMR (400 MHz, CDCl₃): 7.73 (d, *J* = 8.1, 1H), 7.65 (d, *J* = 8.1, 1H), 7.40-7.34 (m, 3H), 7.32-7.22 (m, 3H), 4.85 (d, *J* = 8.1, 1H), 3.48 (s, 2H), 3.36-3.24 (m, 1H), 3.02-2.94 (m, 2H), 2.81 (d, *J* = 11.9, 2H), 2.11 (t, *J* = 10.6, 2H), 1.95 (d, *J* = 12.6, 2H), 1.89-1.78 (m, 2H), 1.65-1.54 (m, 2H), 1.04 (t, *J* = 7.3, 3H).

### EXAMPLE 459

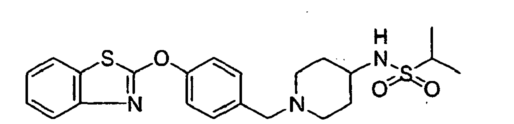

Propane-2-sulfonic acid {1-[4-(benzothiazol-2-yloxy)-benzyl]-piperidin-4-yl}-amide.

The title compound was prepared according to the procedure for EXAMPLE 253, step C using 2-propanesulfonyl chloride. MS (ESI): mass calculated for C₂₂H₂₇N₃O₃S₂, 445.15; m/z found, 446.4 [M+H]⁺. ¹H NMR (400 MHz, CDCl₃): 7.73 (d, *J* = 8.1, 1H), 7.66 (d, *J* = 8.1, 1H), 7.41-7.35 (m, 3H), 7.32-7.24 (m, 3H), 3.79 (d, *J* = 5.6, 1H), 3.51 (s, 2H), 3.38-3.27 (m, 1H), 2.89-2.78 (m, 2H), 2.19-1.94 (m, 5H), 1.76 (d, *J* = 30.8, 6H), 1.69-1.55 (m, 2H).

### EXAMPLE 460

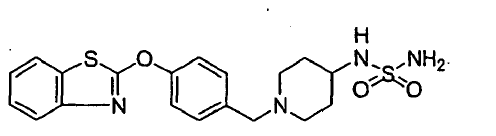

N-{1-[4-(Benzothiazol-2-yloxy)-benzyl]-piperidin-4-yl}-sulfamide

The title compound was prepared from EXAMPLE 453 following the procedure for EXAMPLE 253, step B. MS (ESI): mass calculated for C₁₉H₂₂N₄O₃S₂, 418.11; m/z found, 419.4 [M+H]⁺. ¹H NMR (400 MHz, CDCl₃): 10.68 (br s, 1H), 7.60 (d, *J* = 8.3, 1H), 7.56 (d, *J* = 8.3, 1H), 7.48-7.38 (m, 2H), 7.32-7.24 (m, 3H), 7.18 (t, *J* = 7_{.}8, 1H), 6.24 (br s, 2H), 4.10 (s, 2H), 3.80-3.66 (m, 1H), 3.57-3.10 (m, 2H), 3.04-2.61 (m, 2H), 2.41-1.61 (m, 4H).

### EXAMPLE 461

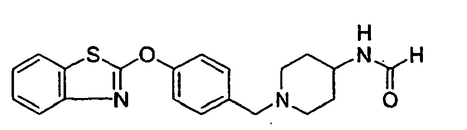

N-{1-[4-(Benzothiazol-2-yloxy)-benzyl]-piperidin-4-yl}-formamide.

The title compound was prepared according to the procedure for EXAMPLE 431, formic acid. MS (ESI): mass calculated for C₂₀H₂₁N₃O₂S, 367.14; m/z found, 368.4 [M+H]⁺. ¹H NMR (400 MHz, CDCl₃): 12.28 (br s, 1H), 7.73 (d, *J* = 7.8, 1H), 7.68 (d, *J* = 7.8, 1H), 7.47-7.24 (m, 6H), 6.33 (d, *J* = 7.3, 1H), 4.05-3.94 (m, 1H), 3.80 (s, 2H), 3.14 (d, *J* = 11.6, 2H), 2.41 (t, *J* = 11.9, 2H), 1.99 (d, *J* = 13.1, 2H), 1.83-1.70 (m, 2H).

### EXAMPLE 462

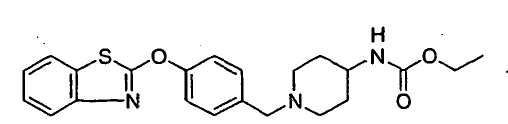

{1-[4-(Benzothiazol-2-yloxy)-benzyl]-piperidin-4-yl}-carbamic acid ethyl ester.

The title compound was prepared according to the procedure for EXAMPLE 253, step C using ethyl chloroformate. MS (ESI): mass calculated for C₂₂H₂₅N₃O₃S, 411.16; m/z found, 412.4 [M+H]⁺. ¹H NMR (400 MHz, CDCl₃): 7.73 (d, *J* = 8.1, 1H), 7.66 (d, *J* = 8.1, 1H), 7.41-7.34 (m, 3H), 7.32-7.22 (m, 3H), 4.62 (br s, 1H), 4.10 (dd, *J* = 7.3, 6.8, 2H), 3.59-3.51 (m, 1H), 3.49 (s, 2H), 2.80 (d, *J* = 11.6, 2H), 2.12 (t, *J* = 11.6, 2H), 1.93 (d, *J* = 12.1, 2H), 1.51-1.39 (m, 2H), 1.23 (t, *J* = 7.1, 3H).

### EXAMPLE 463

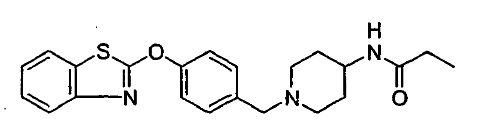

N-{1-[4-(Benzothiazol-2-yloxy)-benzyl]-piperidin-4-yl}-propionamide.

The title compound was prepared according to the procedure for EXAMPLE 253, step C using propionyl chloride. MS (ESI): mass calculated for C₂₂H₂₅N₃O₂S, 395.17; m/z found, 396.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.32-7.22 (m, 3H), 5.61 (d, *J* = 8.1, 1H), 3.87-3.76 (m, 1H), 3.49 (s, 2H), 2.82 (d, *J* = 11.9, 2H), 2.22-2.07 (m, 4H), 1.90 (d, *J* = 13.1, 2H), 1.51-1.39 (m, 2H), 1.14 (t, *J* = 7.6, 3H).

### EXAMPLE 464

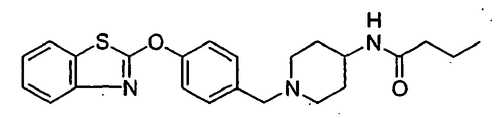

N-{1-[4-(Benzothiazol-2-yloxy)-benzyl]-piperidin-4-yl}-butyramide.

The title compound was prepared according to the procedure for EXAMPLE 253, step C using butyryl chloride. MS (ESI): mass calculated for C₂₃H₂₇N₃O₂S, 409.18; m/z found, 410.4 [M+H]⁺. ¹H NMR (400 MHz, CDCl₃): 7.72 (d, *J* = 8.1, 1H), 7.65 (d, *J* = 8.1, 1H), 7.40-7.35 (m, 3H), 7.31-7.23 (m, 3H), 5.64 (d, *J* = 8.1, 1H), 3.87-3.77 (m, 1H), 3.49 (s, 2H), 2.81 (d, *J* = 11.6, 2H), 2.16-2.07 (m, 4H), 1.91 (d, *J*= 12.9, 2H), 1.71-1.60 (m, 2H), 1.51-1.39 (m, 2H), 0.93 (t, *J* = 7.3, 3H).

### EXAMPLE 465

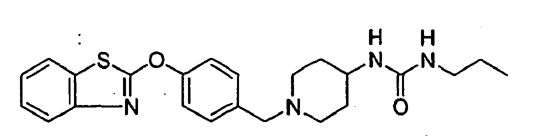

1-{1-[4-(Benzothiazol-2-yloxy)-benzyl]-piperidin-4-yl}-3-propyl-urea.

The title compound was prepared according to the procedure for EXAMPLE 253, step C using propyl isocyanate. MS (ESI): mass calculated for C₂₃H₂₈N₄O₂S, 424.19; m/z found, 425.5 [M+H]⁺. ¹H NMR (400 MHz, CDCl₃): 7.70 (d, *J* = 8.1, 1H), 7.63 (d, *J* = 8.1, 1H), 7.39-7.33 (m, 3H), 7.28-7.21 (m, 3H), 5.54 (br s, 1H), 5.37 (d, *J* = 8.1, 1H), 3.66-3.56 (m, 1H), 3.48 (s, 2H), 3.11 (dd, *J* = 7.1, 6.8, 2H), 2.80 (d, *J* = 11.1, 2H), 2.11 (t, *J* = 11.1, 2H), 1.91 (d, *J* = 11.1, 2H), 1.54-1.38 (m, 4H), 0.91 (t, *J* = 7.3, 3H).

### EXAMPLE 466

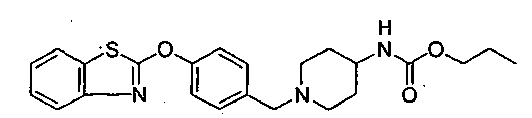

{1-[4-(Benzothiazol-2-yloxy)-benzyl]-piperidin-4-yl}-carbamic acid propyl ester.

The title compound was prepared according to the procedure for EXAMPLE 253, step C using propyl chloroformate. MS (ESI): mass calculated for C₂₃H₂₇N₃O₃S, 425.18; m/z found, 426.4 [M+H]⁺. ¹H NMR (400 MHz, CDCl₃): 7.72 (d, *J* = 8.1, 1H), 7.63 (d, J = 8.1, 1 H), 7.39-7.33 (m, 3H), 7.31-7.21 (m, 3H), 4.83 (d, *J* = 7.6, 1H), 4.00 (t, *J* = 6.6, 2H), 3.58-3.49 (m, 1H), 3.47 (s, 2H), 2.79 (d, *J* = 11.6, 2H), 2.10 (t, *J* = 10.9, 2H), 1.91 (d, *J* = 11.1, 2H), 1.67-1.56 (m, 2H), 1.52-1.40 (m, 2H), 0.92 (t, *J* = 7.6, 3H).

### EXAMPLE 467

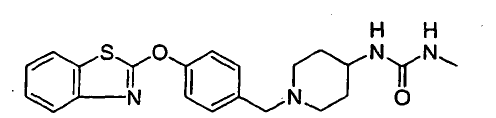

1-{1-[4-(Benzothiazol-2-yloxy)-benzyl]-piperidin-4-yl}-3-methyl-urea.

The title compound was prepared according to the procedure for EXAMPLE 253, step C using methyl isocyanate. MS (ESI): mass calculated for C₂₁H₂₄N₄O₂S, 396.16; m/z found, 397.4 [M+H]⁺. ¹H NMR (400 MHz, CDCl₃): 7.71 (d, *J* = 8.1, 1H), 7.65 (d, *J* = 8.1, 1H), 7.45-7.34 (m, 3H), 7.33-7.22 (m, 3H), 5.71 (br s, 2H), 3.69-3.59 (m, 1H), 3.56 (s, 2H), 2.87 (d, *J* = 11.9, 2H), 2.73 (d, *J* = 4.8, 3H), 2.21 (t, *J* = 10.1, 2H), 1.93 (d, *J* = 10.4, 2H), 1.58-1.45 (m, 2H).

### EXAMPLE 469

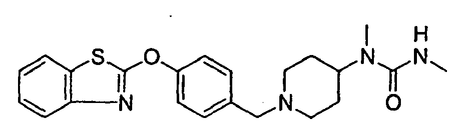

1-{1-[4-(Benzothiazol-2-yloxy)-benzyl]-piperidin-4-yl}-1,3-dimethyl-urea.

The title compound was prepared according to the procedure for EXAMPLE 255, step C using methyl isocyanate. MS (ESI): mass calculated for C₂₂H₂₆N₄O₂S, 410.18; m/z found, 411.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.31-7.22 (m, 3H), 4.63 (dd, *J* = 4.6, 4.6, 1H), 4.21-4.11 (m, 1H), 3.49 (s, 2H), 2.93 (d, *J* = 11.9, 2H), 2.79 (d, *J* = 4.8, 3H), 2.71 (s, 3H), 2.08 (t, *J* = 11.9, 2H), 1.76-1.64 (m, 2H), 1.63-1.54 (m, 2H).

### EXAMPLE 470

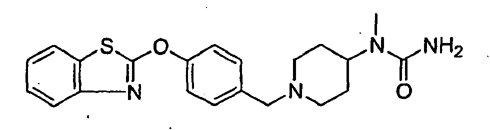

1-{1-[4-(Benzothiazol-2-yloxy)-benzyl]-piperidin-4-yl}-1-methyl-urea.

The title compound was prepared according to the procedure for EXAMPLE 255, step C using trimethylsilyl isocyanate. MS (ESI): mass calculated for C₂₁H₂₄N₄O₂S, 396.16; m/z found, 397.4 [M+H]⁺. ¹H NMR (400 MHz, CDCl₃): 7.72 (d, *J* = 8.1, 1H), 7.65 (d, *J* = 8.1, 1H), 7.42-7.34 (m, 3H), 7.32-7.21 (m, 3H), 4.93 (s, 2H), 4.16-4.03 (m, 1H), 3.50 (s, 2H), 2.94 (d, *J* = 11.4, 2H), 2.76 (s, 3H), 2.09 (t, *J* = 11.4, 2H), 1.79-1.66 (m, 2H), 1.65-1.56 (m, 2H).

### EXAMPLE 471

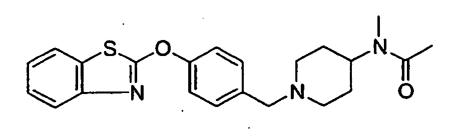

N-{1-[4-(Benzothiazol-2-yloxy)-benzyl]-piperidin-4-yl}-N-methyl-acetamide.

The title compound was prepared according to the procedure for EXAMPLE 255, step C using acetyl chloride. MS (ESI): mass calculated for C₂₂H₂₅N₃O₂S, 395.17; m/z found, 396.4 [M+H]⁺. ¹H NMR (400 MHz, CDCl₃): 7.72 (d, *J* = 8.1, 1H), 7.65 (d, *J* = 8.1, 1H), 7.41-7.34 (m, 3H), 7.31-7.22 (m, 3H), 4.55-4.44 (m, 1H), 3.49 (s, 2H), 3.01-2.90 (m, 2H), 2.84 (s, 3H), 2.15-2.09 (m, 2H), 2.07 (s, 3H), 1.77-1.65 (m, 2H), 1.64-1.53 (m, 2H).

### EXAMPLE 472

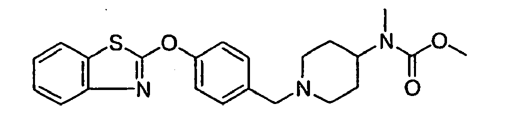

{1-[4-(Benzothiazol-2-yloxy)-benzyl]-piperidin-4-yl}-methyl-carbamic acid methyl ester.

The title compound was prepared according to the procedure for EXAMPLE 255, step C using methyl chloroformate. MS (ESI): mass calculated for C₂₁H₂₅N₃O₃S, 411.16; m/z found, 412.3 [M+H]⁺. ¹H NMR (400 MHz, CDCl₃): 7.73 (d, *J* = 8.1, 1H), 7.66 (d, *J* = 8.1, 1 H), 7.42-7.35 (m, 3H), 7.33-7.24 (m, 3H), 4.16-3.96 (m, 1H), 3.69 (s, 3H), 3.51 (s, 2H), 2.95 (d, *J* = 11.4, 2H), 2.78 (s, 3H), 2.08 (t, *J* = 10.9, 2H), 1.82-1.69 (m, 2H), 1.65-1.57 (m, 2H).

### EXAMPLE 473

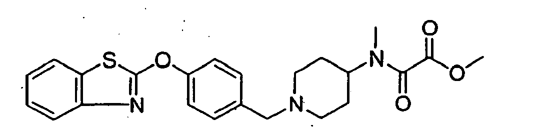

N-{1-[4-(Benzothiazol-2-yloxy)-benzyl]-piperidin-4-yl}-N-methyl-oxalamic acid methyl ester.

The title compound was prepared according to the procedure for EXAMPLE 255, step C using chloro-oxo-acetic acid methyl ester. MS (ESI): mass calculated for C₂₃H₂₅N₃O₄S, 439.16; m/z found, 440.4 [M+H]⁺. ¹H NMR (400 MHz, CDCl₃): 7.73 (d, *J* = 8.1, 1H), 7.66 (d, *J* = 8.1, 1H), 7.41-7.34 (m, 3H), 7.33-7.23 (m, 3H), 4.41-4.32 (m, 1H), 3.87 (s, 3H), 3.51 (s, 2H), 2.97 (d, *J* = 11.9, 2H), 2.88 (s, 3H), 2.13 (t, *J* = 11.9, 2H), 1.95-1.83 (m, 2H), 1.72-1.62 (m, 2H).

### EXAMPLE 474

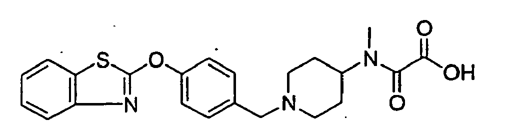

N-{1-[4-(Benzothiazol-2-yloxy)-benzyl]-piperidin-4-yl}-N-methyl-oxalamic acid.

The title compound was prepared from EXAMPLE 473 following the procedure for EXAMPLE 433, step C. MS (ESI): mass calculated for C₂₂H₂₃N₃O₄S, 425.14; m/z found, 426.3 [M+H]⁺. ¹H NMR (400 MHz, CDCl₃): 7.68 (d, *J* = 8.1, 1H), 7.59 (d, *J* = 8.1, 1H), 7.37-7.28 (m, 3H), 7.27-7.16 (m, 3H), 4.27-4.16 (m, 1H), 3.43 (s, 2H), 2.93 (d, *J* = 11.897, 2H), 2.75 (s, 3H), 2.01 (t, *J* = 11.9, 2H), 1.82-1.65 (m, 2H), 1.58-1.47 (m, 2H).

### EXAMPLE 475

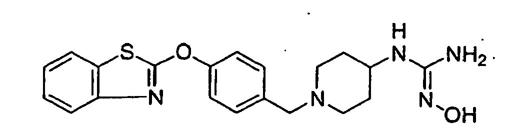

Guanidine, N-{1-[4-(Benzothiazo)-2-yloxy)-benzyl]-piperidin-4-yl}-N'-hydroxy
A. 1-[4-(Benzothiazol-2-yloxy)-benzyl]-peperidin-4-yl-cyanamide. To a suspension of 1-[4-(benzothiazol-2-yloxy)-benzyl]-piperidin-4-ylamine (339 mg, 1.0 mmol) in CH₃OH (2.0 mL) was added sodium acetate (180 mg, 2.2 mmol) at room temperature. The suspension became a clear solution. Cooled the solution to 0 °C. Cyanogen bromide (159 mg, 1.5 mmol) in CH₃OH (0.7 mL) was added dropwise. The reaction mixture was stirred at 5 °C for 2 h and then at ambient temperature overnight. The mixture was filtered through a frit fnnel and the filtrate was absorbed onto silica gel (40 g) and purified (0-10% CH₃OH/CH₂Cl₂) to give an unstable oil (123 mg, 34% yield). MS (ESI): mass calculated for C₂₀H₂₀N₄OS, 364.14; m/z found, 365.3 [M+H]⁺.
B. Guanidine, N-{1-[4-(Benzothiazol-2-yloxy)-benzyl]-piperidin-4-yl}-N'-hydroxy. B. Guanidine, N-{1-[4-(Benzothiazol-2-yloxy)-benzyl]-piperidin-4-yl}-N'-hydroxy. To a solution of 1-[4-(benzothiazol-2-yloxy)-benzyl]-piperidin-4-yl-cyanamide (123 mg, 0.34 mmol) in DMF (2 mL) was added hydroxylamine hydrochloride (41.5 mg, 0.60 mmol), followed by sodium carbonate (119 mg, 1.12 mmol) in small portions. The reaction mixture was stirred at room temerature for 2 h and partitioned between EtOAc (20 mL) and H₂O (20 mL). The organic phase was washed with brine, dried and concentrated under reduced pressure to yield the crude product as a pale solid. The crude product was purified on SiO₂ (12 g; 0-10% CH₃OH/CH₂Cl₂) to give a white soid (117 mg, 86% yield). MS (ESI): mass calculated for C₂₀H₂₃N₅O₄S, 397.16; m/z found, 398.4 [M+H]⁺. ¹H NMR (400 MHz, CDCl₃): 7.71 (d, *J* = 8.1, 1H), 7.63 (d, *J* = 8.1, 1H), 7.38-7.32 (m, 3H), 7.28-7.21 (m, 3H), 4.69 (br s, 2H), 3.46 (s, 2H), 3.27-3.16 (m, 1H), 2.78 (d, *J* = 10.9, 2H), 2.04 (t, *J* = 11.1, 2H), 1.92 (d, *J* = 11.1, 2H), 1.51-1.38 (m, 2H).

### EXAMPLE 476

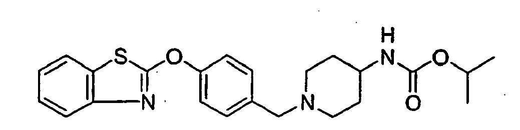

{1-[4-(Benzothiazol-2-yloxy)-benzyl]-piperidin-4-yl}-carbamic acid isopropyl ester.

The title compound was prepared according to the procedure for EXAMPLE 253, step C using isopropyl isocyanate. MS (ESI): mass calculated for C₂₃H₂₇N₃O₃S, 425.18; m/z found, 426.4 [M+H]⁺. ¹H NMR (400 MHz, CDCl₃): 7.72 (d, *J* = 8.1, 1H), 7.63 (d, *J* = 8.1, 1H), 7.38-7.33 (m, 3H), 7.31-7.21 (m, 3H), 4.95-4.85 (m, 1H), 4.75 (d, *J* = 7.1, 1H), 3.58-3.49 (m, 1H), 3.47 (s, 2H), 2.79 (d, *J* = 11.6, 2H), 2.09 (t, *J* = 11.4, 2H), 1.91 (d, *J* = 11.9, 2H), 1.50-1.39. (m, 2H), 1.21 (d, *J* = 6.5, 6H).

### EXAMPLE 477

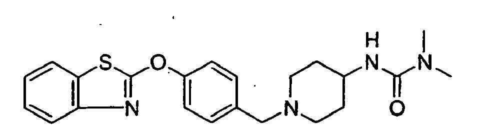

3-{1-[4-(Benzothiazol-2-yloxy)-benzyl]-piperidin-4-yl}-1,1-dimethyl-urea.

The title compound was prepared according to the procedure for EXAMPLE 253, step C using N,N-dimethylcarbamoyl chloride. MS (ESI): mass calculated for C₂₂H₂₆N₄O₂S, 410.18; m/z found, 411.4 [M+H]⁺. ¹H NMR (400 MHz, CDCl₃): 7.71 (d, *J* = 8.1, 1H), 7.64 (d, *J* = 8.1, 1H), 7.39-7.33 (m, 3H), 7.30-7.21 (m, 3H), 4.41 (d, *J* = 7.8, 1H), 3.73-3.62 (m, 1H), 3.48 (s, 2H), 2.87 (s, 6H), 2.81 (d, *J* = 11.6, 2H), 2.11 (t, *J* = 11.9, 2H), 1.93 (d, *J* = 11.6, 2H), 1.50-1.38 (m, 2H).

### EXAMPLE 478

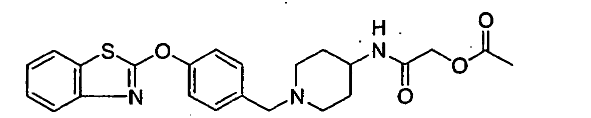

Acetic acid {1-[4-(benzothiazol-2-yloxy)-benzyl]-piperidin-4-ylcarbamoyl}-methyl ester.

The title compound was prepared according to the procedure for EXAMPLE 253, step C using acetic acid chlorocarbonyl methyl ester. MS (ESI): mass calculated for C₂₃H₂₅N₃O₄S, 439.16; m/z found, 440.4 [M+H]⁺. ¹H NMR (400 MHz, CDCl₃): 7.72 (d, *J* = 7.8, 1H), 7.65 (d, *J* = 8.1, 1H), 7.40-7.35 (m, 3H), 7.32-7.23 (m, 3H), 6.11 (d, J = 8.3, 1H), 4.53, (s, 2H), 3.93-3.82 (m, 1H), 3.50 (s, 2H), 2.83 (d, *J* = 11.9, 2H), 2.15 (s, 3H), 2.14 (t, *J* = 11.9, 2H), 1.93 (d, *J* = 12.1, 2H), 1.56-1.45 (m, 2H).

### EXAMPLE 479

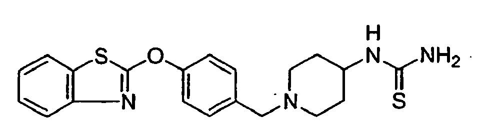

{1-[4-(Benzothiazol-2-yloxy)-benzyl]-piperidin-4-yl}-thiourea.
A. 1-{1-[4-(Benzothiazol-2-yloxy)-benzyl]-piperidin-4-yl}-3-benzoyl-thiourea. The title compound was prepared according to the procedure for EXAMPLE 253, step C using benzoyl isocyanate. MS (ESI): mass calculated for C₂₇H₂₆N₄O2S₂, 502.15; m/z found, 503.3 [M+H]⁺. ¹H NMR (400 MHz, CDCl₃): 10.80 (d, *J* = 8.1, 1H), 9.06 (s, 1H), 7.81 (d, *J* = 8.3, 2H), 7.72 (d, *J* = 8.3, 1H), 7.65 (d, *J* = 7.8, 1H), 7.59 (t, *J* = 7.6, 1H), 7.50-7.45 (m, 2H), 7.42-7.34 (m, 3H), 7.33-7.22 (m, 3H), 4.40-4.29 (m, 1H), 3.53 (s, 2H), 2.80 (d, *J* = 11.1, 2H), 2.26 (t, *J* = 10.1, 2H), 2.12 (d, *J* = 11.9, 2H), 1.76-1.64 (m, 2H).
B. {1-[4-(Benzothiazol-2-yloxy)-benzyl]-piperidin-4-yl}-thiourea. To 1-{1-[4-(benzothiazol-2-yloxy)-benzyl]-piperidin-4-yl}-3-benzoyl-thiourea (300 mg, 0.60 mmol) was added 10% aqueous sodium hydroxide (10 mL). The reaction mixture was heated at reflux for 1 h. The resulting white suspension was cooled to room temperature and extracted with 10% CH₃OH/CH₂Cl₂ (3 x 50 mL). The organic layer was dried (Na₂SO₄) and concentrated under reduced pressure to yield the crude product as an off-white solid. The crude product was purified on SiO₂ (40 g; 0-10% CH₃OH/CH₂Cl₂) to give the title compound as a white solid (219 mg, 92% yield). MS (ESI): mass calculated for C₂₀H₂₂N₄OS₂, 398.12; m/z found, 399.3 [M+H]⁺. ¹H NMR (400 MHz, CDCl₃): 7.72 (d, *J* = 8.1, 1H), 7.68 (d, *J* = 8.1, 1H), 7.42-7.36 (m, 3H), 7.34-7.26 (m, 3H), 6.09 (m, 1H), 4.29-4.02 (m, 1H), 3.57 (s, 2H), 2.88 (d, *J* = 11.1, 2H), 2.29 (s, 2H), 2.20 (t, *J* = 11.1, 2H), 2.04 (d, *J* = 10.1, 2H), 1.60-1.45 (m, 2H).

### EXAMPLE 480

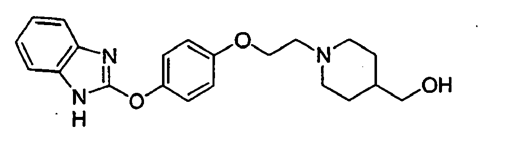

(1-{2-[4-(1H-Benzoimidazol-2-yloxy)-phenoxy]-ethyl}-piperidin-4-yl)-methanol.

The title compound was prepared according to the procedure for EXAMPLE 37, using piperidin-4-yl-methanol. MS (ESI): mass calculated for C₂₁H₂₅N₃O₃, 367.19; m/z found, 368.4 [M+H]⁺. ¹H NMR (400 MHz, DMSO-*d*₆): 12.24 (s, 1H), 7.32 (s, 2H), 7.27 (d, *J* = 8.9, 2H), 7.08 (s, 2H), 7.00 (d, *J* = 8.9, 2H), 4.41 (s, 1H), 4.09 (s, 2H), 3.24 (s, 2H), 2.92 (d, *J* = 11.1, 2H), 2.68 (s, 2H), 1.98 (t, *J* = 11.6, 2H), 1.62 (d, *J* = 11.6, 2H), 1.24 (br s, 1H), 1.11 (d, *J* = 9.2, 2H).

### EXAMPLE 481

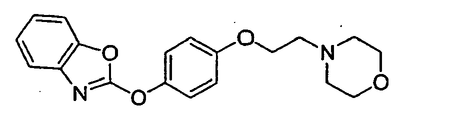

2-[4-(2-Morpholin-4-yl-ethoxy)-phenoxy]-benzooxazole.

The title compound was prepared according to the procedure for EXAMPLE 11, using 4-(2-chloro-ethyl)-morpholine. MS (ESI): mass calculated for C₁₉H₂₀N₂O₄, 340.14; m/z found, 341.1 [M+H]⁺. ¹H NMR (400 MHz, CDCl₃): 7.51 (d, *J* = 7.1, 1H), 7.42 (d, *J* = 7.2, 1H), 7.36-7.30 (m, 2H), 7.29-7.20 (m, 2H), 7.03-6.95 (m, 2H), 4.14 (t, *J* = 5.7, 2H), 3.80-3.72 (m, 4H), 2.83 (t, *J* = 5.7, 2H), 2.60 (t, *J* = 4.6, 4H).

### EXAMPLE 482

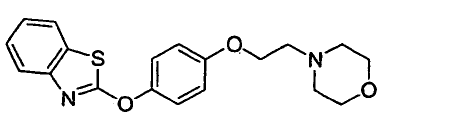

2-[4-(2-Morpholin-4-yl-ethoxy)-phenoxy]-benzothiazole.

The title compound was prepared according to the procedure for EXAMPLE 12, using 4-(2-chloro-ethyl)-morpholine and 2-chloro-benzothiazole. MS (ESI): mass calculated for C₁₉H₂₀N₂O₃S, 35; m/z found, 341.1 [M+H]⁺. ¹H 6.12 NMR (400 MHz, CDCl₃): 7.74 (d, *J* = 7.6, 1H), 7.66 (d, *J* = 7.3, 1H), 7.39 (t, *J* = 7.3, 1H), 7.31-7.20 (m, 3H), 7.00-6.93 (m, 2H), 4.14 (t, *J* = 5.7, 2H), 3.80-3.72 (m, 4H), 2.83 (t, *J* = 5.7, 2H), 2.60 (t, *J* = 4.6, 4H).

### EXAMPLE 483

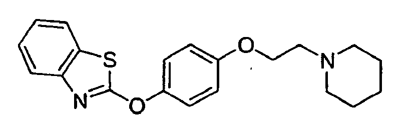

2-[4-(2-Piperidin-1-yl-ethoxy)-phenoxy]-benzothiazole.

The title compound was prepared according to the procedure for EXAMPLE 12, using 4-(2-chloro-ethyl)-piperidine and 2-chloro-benzothiazole. MS (ESI): mass calculated for C₂₀H₂₂N₂O₂S, 354.14; m/z found, 355.1 [M+H]⁺. ¹H NMR (400 MHz, CDCl₃): 7.74 (d, *J* = 8.1, 1H), 7.65 (d, *J* = 8.0, 1H), 7.39 (t, *J* = 7.3, 1H), 7.30-7.22 (m, 3H), 7.00-6.92 (m, 2H), 4.13 (t, *J* = 6.0, 2H), 2.81 (t, *J* = 6.0, 2H), 2.58-2.48 (br s, 4H), 1.68-1.58 (m, 4H), 1.52-1.42 (m, 2H).

### EXAMPLE 484

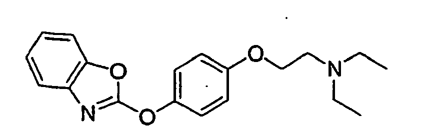

{2-[4-(Benzooxazol-2-yloxy)-phenoxy]-ethyl}-diethyl-amine.

The title compound was prepared according to the procedure for EXAMPLE 11, using (2-chloro-ethyl)-diethyl-amine. MS (ESI): mass calculated for C₁₉H₂₂N₂O₃, 326.16; m/z found, 327.1 [M+H]⁺. ¹H NMR (400 MHz, CDCl₃): 7.51 (d, *J* = 7.6, 1H), 7.42 (d, *J* = 7.2, 1 H), 7.36-7.29 (m, 2H), 7.28-7.19 (m, 2H), 7.02-6.94 (m, 2H), 4.07 (t, *J* = 6.3, 2H), 2.90 (t, *J* = 6.3, 2H), 2.65 (q, *J* = 7.2, 4H), 1.09 (t, *J* = 7.1, 6H).

### Assay Methods

Assay results providd herein are illustrative results of the assays that were performed for compounds of this invention.

### Recombinant Human LTA4 Hydrolase Assay for LTA4 Hydrolase Inhibitor Activity

Compounds of the present invention were tested for LTA4 hydrolase inhibitor activity against recombinant human LTA4 hydrolase (rhLTA4H). Vectors were prepared and used to express rhLTA4H essentially as follows: LTA4 hydrolase encoding DNA was amplified by polymerase chain reaction (PCR) using a human placental cDNA library as a template. Oligonucleotide primers for the PCR reaction were based on the 5'-end, and the complement of the 3'-end, of the published nucleotide sequence for the coding region of the human LTA4 hydrolase gene (C.D. Funk et al., Proc. Natl. Acad. Sci. USA 1987, 84:6677-6681). The amplified 1.9 kb DNA fragment encoding LTA4 hydrolase was isolated and cloned into the pFastBac1 vector (Invitrogen). Recombinant baculovirus was generated as described by the manufacturer, and used to infect Spodoptera frugiperda (Sf-9) cells. Recombinant LTA4 hydrolase enzyme was purified from the infected Sf-9 cells essentially as described by J.K. Gierse et al. (Protein Expression and Purification 1993, 4:358-366). The purified enzyme solution was adjusted to contain 0.29 mg/mL LTA4 hydrolase, 50 mM Tris (pH 8.0), 150 mM NaCl, 5 mM dithiothreitol, 50% glycerol, and EDTA-free Complete protease inhibitor cocktail (Roche). The specific activity of the enzyme was about 3.8 µmol/min/mg.

LTA4 substrate was prepared from the methyl ester of LTA4 (Cayman Chemical) by treatment with 67 equiv of NaOH under nitrogen at room temperature for 40 min. The LTA4 substrate in its free acid form was kept frozen at -80 °C until needed. Each compound was diluted to different concentrations in assay buffer (0.1 M potassium phosphate (pH 7.4), 5 mg/mL fatty acid free BSA) containing 10% DMSO. A 25-uL aliquot of each compound dilution was incubated for 10 min at room temperature with an equal volume of assay buffer containing 36 ng of recombinant human LTA4H. The solution was then adjusted to 200 µL with assay buffer. LTA4 (free acid) was thawed and diluted in assay buffer to a concentration of 357 ng/mL, and 25 µL (9 ng) of LTA4 substrate was added to the reaction mixture (total volume = 225 µL) at time zero. Each reaction was carried out at room temperature for 10 min. The reaction was stopped by diluting 10 µL of the reaction mixture with 200 µL of assay buffer. LTB4 was quantified in the diluted sample by a commercially available enzyme-linked immunoassay (Cayman Chemical Co.), as recommended by the manufacturer. Positive controls, under essentially identical conditions but without addition of an inhibitor compound, and negative controls, containing all assay components except enzyme, were routinely run in each experiment. IC₅₀ values were determined by fitting the activity data at different compound concentrations to a 4-parameter equation using the Grafit program (Erithacus software).

The IC₅₀ values presented in the tables below should be expected to fall within the typical three-fold variability of assays of this type. The values presented here are, in general, an average of one to three determinations.

**Table 1**

| Example | IC₅₀ (nM) | Example | IC₅₀ (nM) | Example | IC₅₀ (nM) |
|---|---|---|---|---|---|
| **11** | 6 | **45** | 15 | **94** | 1 |
| **13** | 2 | **46** | 17 | **135** | 92 |
| **14** | 9 | **59** | 5 | **136** | 25 |
| **27** | 7 | **77** | 10 | **271** | 2 |
| **36** | 14 | **78** | 3 | **481** | 55 |
| **44** | 8 | **92** | 1 | **484** | 19 |

**Table 2**

| Example | IC₅₀ (nM) | Example | IC₅₀ (nM) | Example | IC₅₀ (nM) |
|---|---|---|---|---|---|
| **12** | 100 | **176** | 12 | **298** | 15 |
| **15** | 22 | **180** | 2 | **302** | 60 |
| **18** | 6 | **183** | 18 | **325** | 12 |
| **22** | 7 | **192** | 3 | **328** | 22 |
| **23** | 1 | **195** | 58 | **331** | 13 |
| **25** | 4 | **202** | 4 | **334** | 100 |
| **31** | 17 | **206** | 4 | **336** | 24 |
| **80** | 23 | **207** | 16 | **353** | 7 |
| **81** | 5 | **211** | 9 | **357** | 45 |
| **102** | 3 | **214** | 18 | **358** | 11 |
| **104** | 14 | **224** | 14 | **359** | 48 |
| **109** | 21 | **225** | 25 | **360** | 33 |
| **110** | 40 | **250** | 26 | **361** | 8 |
| **143** | 64 | **251** | 16 | **363** | 4 |
| **146** | 43 | **255** | 1 | **366** | 39 |
| **147** | 33 | **259** | 14 | **380** | 6 |
| **150** | 1 | **261** | 33 | **389** | 2 |
| **153** | 8 | **262** | 4 | **419** | 4 |
| **155** | 9 | **263** | 5 | **437** | 21 |
| **156** | 7 | **265** | 112 | **446** | 8 |
| **157** | 1 | **270** | 16 | **447** | 28 |
| **161** | 1 | **274** | 29 | **450** | 7 |
| **163** | 9 | **285** | 76 | **454** | 4 |
| **167** | 37 | **287** | 28 | **462** | 27 |
| **168** | 27 | **288** | 4 | **471** | 19 |
| **169** | 32 | **296** | 33 | **479** | 7 |
| **172** | 27 | **294** | 6 | **483** | 45 |

**Table 3**

| Example | IC₅₀ (nM) | Example | IC₅₀ (nM) |
|---|---|---|---|
| **37** | 10 | **134** | 110 |
| **123** | 4 | **230** | 36 |
| **127** | 29 | **231** | 18 |
| **132** | 139 | **485** | 39 |
| **133** | 111 | | |

### LTB4 Production by Calcium lonophore-Stimulated Murine Blood for LTA4H Inhibitor Activity

CD-1 mice were sacrificed, and blood was collected in heparin-containing syringes by cardiac puncture. The blood was diluted 1:15 with RPMI-1640 medium, and 200-µL aliquots of the diluted blood were added to wells of a 96-well microtiter plate. LTA4H inhibitor test compounds were prepared at different concentrations in RPMI-1640 medium containing 1% DMSO, and 20 µL of each test solution was added to a well containing diluted whole blood (final DMSO concentration of 0.1%). After the microtiter plate contents were incubated for 15 min at 37°C in a humidified incubator, calcium ionophore A23187 (Sigma Chemical Co., St. Louis, Mo.) was added to each sample well (final concentration = 20 ng/mL). The incubation was continued under the same conditions for an additional 10 min to allow LTB4 formation. The reaction was terminated by centrifugation (833 x g, 10 min at 4°C), and supernatants were analyzed for LTB4 by a commercially available enzyme-linked immunoassay (Cayman Chemical Co.) according to the manufacturer's instructions. Positive controls, under essentially identical conditions but without addition of an inhibitor compound, and negative unstimulated controls, containing all assay components except calcium ionophore, were routinely run in each experiment. IC₅₀ values were determined by fitting the activity data at different compound concentrations to a 4-parameter equation using the Grafit program (Erithacus software).

**Table 4**

| Example | IC₅₀ (nM) | Example | IC₅₀ (nM) | Example | IC₅₀ (nM) |
|---|---|---|---|---|---|
| **11** | 44 | **27** | 143 | **94** | 72 |
| **13** | 41 | **44** | 23 | **481** | 264 |
| **14** | 89 | **46** | 29 | **484** | 11 |

**Table 5**

| Example | IC₅₀ (nM) | Example | IC₅₀ (nM) | Example | IC₅₀ (nM) |
|---|---|---|---|---|---|
| **15** | 251 | **250** | 99 | **328** | 143 |
| **18** | 162 | **198** | 39 | **331** | 85 |
| **22** | 123 | **206** | 43 | **334** | .453 |
| **23** | 93 | **207** | 81 | **336** | 153 |
| **81** | 41 | **251** | 159 | **359** | 446 |
| **102** | 55 | **259** | 49 | **360** | 246 |
| **104** | 520 | **261** | 81 | **380** | 17 |
| **109** | 189 | **263** | 49 | **437** | 105 |
| **150** | 52 | **274** | 331 | **446** | 131 |
| **155** | 127 | **288** | 57 | **447** | 240 |
| **156** | 2000 | **296** | 133 | **462** | 144 |
| **161** | 100 | **298** | 69 | **483** | 161 |
| **180** | 15 | **302** | 212 | | |

**Table 6**

| Example | IC₅₀ (nM) | Example | IC₅₀ (nM) |
|---|---|---|---|
| **127** | 376 | **133** | 290 |
| **132** | 347 | **134** | 436 |

### Murine arachidonic acid-induced inflammation model

LTA4H inhibitor compounds of the present invention were dissolved in 20% cyclodextran/H₂O at a concentration of 3 mg/mL. The solutions were administered by oral gavage to female Balb/c mice weighing approximately 20 grams each (0.2 mL per mouse, 30 mg of LTA4H inhibitor compound per kg). Sixty minutes after being administered an LTA4 inhibitor, each mouse received topical application of 20 µL of arachidonic acid (100 mg/mL in acetone) to the left ear and 20 µL of acetone only to the right ear. After 3 h, the mice were sacrificed, blood was withdrawn in heparinized syringes, and 8 mm ear biopsies were taken. Ear biopsies were weighed to determine edema and then frozen at -80 °C until needed for determination of neutrophil influx.

One hundred-microliter aliquots of heparinized blood were added to wells of a microtiter plate, along with equal volumes of RPMI-1640 medium, and calcium ionophore A23187 was added to each sample well (final concentration = 20 ng/µL). The microtiter plate contents were incubated for 10 min at 37°C in a humidified incubator. The reaction was terminated by centrifugation (833 x g, 10 min at 4°C). Supernatants were analyzed for LTB4 by a commercially available enzyme-linked immunoassay (Cayman Chemical Co.) in accordance with the manufacturer's instructions. The percent inhibition of ex vivo stimulated LTB4 production (% Inh. LTB4) was determined by comparison to animals treated identically except that the solution admininstered by oral gavage was devoid of inhibitor compound.

Neutrophil influx was quantified by measuring the activity of myeloperoxidase (MPO), a neutrophil-specific enzyme. The ear biopsies were homogenized in 0.5 mL extraction buffer (0.3 M sucrose, 0.22% (w/v) hexadecyl trimethyl ammonium bromide (CTAB), and 2.5 mM citrate prepared from 0.5 M citrate stock solution (pH 5.0)). Debris was removed by centrifugation at 14000 x g for 10 min. Aliquots of 10 µL of the resulting supernatant were added to wells of a microtiter plate, along with 90-µL aliquots of dilution buffer (10 mM citrate, 0.22% CTAB), followed by addition of 20 µL TMB liquid substrate system (Sigma Chemical Co.) to each sample well. The microtiter plate contents were held at room temperature for 1 h. The reaction was stopped by addition of 100 µL 1 M H₂SO₄ to each sample well, and the myeloperoxidase activity in each sample was determined from the absorbance at 405 nm. The background value from the right ear, treated only with acetone, was subtracted from that for the left ear, treated with arachidonic acid in acetone, for each animal. The percent inhibition of neutrophil influx (% Inh. MPO) by compounds of the invention was determined by comparison to animals treated identical, except that the solution administered by oral gavage was devoid of inhibitor compound.

**Table 7**

| Example | % Inh. LTB4 | % Inh. MPO |
|---|---|---|
| **11** | 83 | 95 |
| **14** | 81 | 56 |
| **27** | 50 | 72 |

**Table 8**

| Example | % Inh. LTB4 | % Inh. MPO | Example | % Inh. LTB4 | % Inh. MPO |
|---|---|---|---|---|---|
| **15** | 32 | 29 | **251** | 79 | 66 |
| **18** | 78 | 83 | **259** | 95 | 74 |
| **22** | 79 | 79 | **263** | 0 | 19 |
| **23** | 67 | 84 | **274** | 93 | 87 |
| **81** | 78 | 83 | **325** | 76 | 67 |
| **109** | 51 | 44 | **336** | 67 | 0 |
| **150** | 81 | 91 | **360** | 87 | |
| **155** | 96 | 93 | **361** | 86 | 90 |
| **180** | 87 | 87 | **446** | 64 | 43 |
| **250** | 80 | 94 | **447** | 90 | 48 |
| **206** | 67 | 76 | **471** . | 90 | 89 |
| **250** | 80 | 94 | | | |

Having described the invention in specific detail and exemplified the manner in which it may be carried into practice, it will be apparent to those skilled in the art that innumerable variations, applications, modifications, and extensions of the basic principles involved may be made without departing from its spirit or scope. It is to be understood that the foregoing is merely exemplary and the present invention is not to be limited to the specific form or arrangements of parts herein described and shown.

## Claims

1. A compound of formula (II): 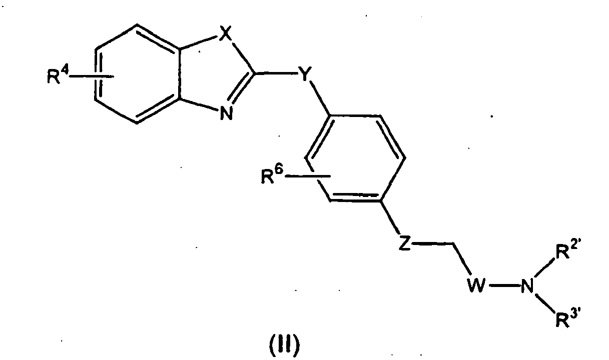 or an enantiomer, diasteromer, racemic, tautomer, hydrate, solvate, or a pharmaceutically acceptable salt, ester, or amide thereof,
wherein
X is selected from the group consisting of NR⁵, O, and S, with R⁵ being one of
H and CH₃;
Y is selected from the group consisting of CH₂, and O;
Z is selected from the group consisting of O and bond;
W is selected from the group consisting of CH₂ and CHR¹-CH₂, with R¹ being one of H and OH, wherein the R¹-attached carbon member in said CHR¹-CH₂ is not directly attached to the nitrogen member to which said W is attached;
R⁴ is selected from the group consisting of H, OCH₃, Cl, F, Br, I, OH, NH₂, CN, CF₃ and CH₃;
R⁶ is H or F; and
R² is defined as R² and R³ is defined as R³, as follows:
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said -C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two -N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=; and
L) one of 2-(4-ethyl-phenoxy)-benzothiazole, 2-(4-ethyl-phenoxy)-benzooxazole, and 2-(4-ethyl-phenoxy)-1*H*-benzoimidazole;
M) SO₂C₁₋₄alkyl;
alternatively R² and R³ are taken together with the nitrogen to which they are attached to form a heterocyclic ring that contains at least one heteroatom member that is said attachment nitrogen, said heterocyclic ring being selected from the group consisting of
i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄alkylOR^{Y}, -C₀₋₄alkylC(O)NR^{Y}R^{Z}, -C₀₋₄alkylNR^{Y}C(O)R^{Z}, - C(O)NR^{Z}OR^{Y}, -C₀₋₄alkylNR^{Y}C(O)CH₂OR^{Y}, - C₀₋₄alkylNR^{Y}C(O)CH₂C(O)R^{Y}, -C₀₋₄alkylNR^{Y}CO₂R^{Y}, - C₀₋₄alkylNR^{Y}C(O)NR^{Y}R^{Z}, -C₀₋₄alkylNR^{Y}C(S)NR^{Y}R^{Z}, -NR^{Y}C(O)CO₂R^{Y},-NR^{Y}R^{Z}, -C₀₋₄alkylNR^{W}SO₂R^{Y},1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H-*tetrazol-5-yl, pyrrolidine-2-thion-1-yl, piperidine-2-thion-1-yl, - C₀₋₄alkylC(O)N(R^{Y})(SO₂R^{Y}), -C₀₋₄alkylN(R^{Y})(SO₂)NR^{Y}R^{Y}, -C₀₋ 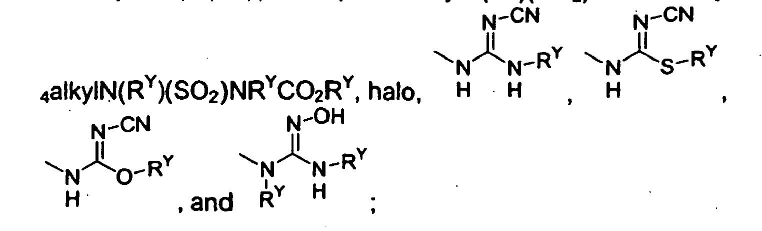
ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y},-CO₂R^{Y} -C₃₋₄alkylCO₂R^{Y} and R^{Z};
iii) one of imidazolidin-1-yl, 2-imidazolin-1-yl, pyrazol-1-yl, imidazol-1-yl, 2*H*-tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1*H*-tetrazol-1-yl is substituted at the carbon member with 0 or 1 of -C₀₋₄alkylR^{Z}, -C₀₋₄alkylSR^{Y}, - C₀₋₄alkylCO₂R^{Y}, and substituent HetR^{a}; and
iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl , ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR^{M} as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N}
R^{L} is selected from the group consisting of -CO₂R^{S} and -C(O)NR^{S}R^{S'};
R^{M} is selected from the group consisting of R^{Z}, indol-7-yl, -SO₂R^{Y}, -C₃₋₄alkylCO₂R^{Y}, -CO₂R^{Y}, -C(O)NR^{Z}OR^{Y}, -C(O)R^{Y}, C(O)C₁₋₄alkylOR^{Y}, - C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H-*tetrazol-5-yl,
R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN,
CF₃, CH₃, OC(O)CH₃, and NO₂;
R^{P} is selected from the group consisting of R^{Y}, -C₂₋₄alkylOR^{Y}, R^{Ar}, -C₁₋₂alkylCO₂R^{Y}, -C₁₋₂alkylCONR^{S}R^{S'}, indol-7-yl, and -SO₂C₁₋₄alkyl;
R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alklR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y};
R^{S} and R^{S'} are independently selected from the group consisting of H, -C₁₋₄alkyl, and -C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L};
R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar};
R^{Y} is selected from the group consisting of H, -C₁₋₄alkyl, C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y}, -C₁₋₂alkylCO₂R^{Y}, -C₁₋₂alkylC(O)NR^{S}R^{S'}, and -C₂₋₄alkylNR^{S}R^{S'};
when R and R^{Z'} are attached to a nitrogen member, R^{Y} and R^{Z} are selected as defined above, or R^{Y} and R^{Z} are taken together with the R^{Y}- and R^{Z}-attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring HetR having 0 or 1 valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1
unsaturated carbon-carbon bonds and having 0 or 1 carbonyl members;
provided that
(a) at least one of said R^{2'} and R^{3'} is not ethyl when one selection in the group consisting of selections (s1), (s2), (s3), and (s4), is satisfied, and each of said selections is specified as
(s1): R⁴ is H, Z is O, W is CH₂, Y is CH₂, and X is S;
(s2): R⁴ is H, Z is O, W is CH₂, Y is CH₂, and X is NH;
(s3): R⁴ is H, Z is O, W is CH₂, Y is O, and X is S;
(s4): R⁴ is 5-chloro, Z is O, W is CH₂, Y is CH₂, and X is S;
(b) further provided that when Z is bond, Y is CH₂, W is CHR¹-CH₂, R¹ is H, and one of R^{2'} and R^{3'} is 1*H*-imidazol-2-yl, then the other of R^{2'} and R^{3'} is selected from A1), B)-L), wherein B)-L) are as defined above for compound of formula (I), and A1) consists of H, C₃₋₇alkenyl, wherein the carbon in said C₃₋₇alkenyl that is attached to the nitrogen member has only single bonds, C₃₋₇alkynyl,
wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl; and
(c) further provided that when X is S, Y is O, Z is bond, and W is CH₂, then one of R^{2'} and R^{3'} Is not XCG when the other is C₁₋₆alkyl, wherein XCG is the group 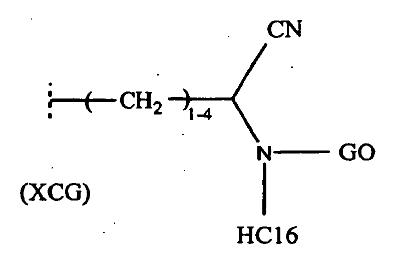
wherein HC16 is one of H, C₁₋₆alkyl, haloC₁₋₆alkyl, allyl, and C₁₋₆alkoxymethyl, and GO is a group attached by a carbon member that has a =O substituent forming an amido group with the nitrogen member to which said GO group is attached.

2. A compound of formula (III): 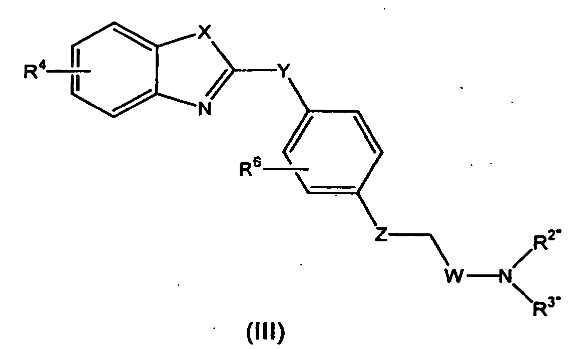 or an enantiomer, diasteromer, racemic, tautomer, hydrate, solvate, or a pharmaceutically acceptable salt, ester, or amide thereof,
wherein
X is selected from the group consisting of NR⁵, O, and S, with R⁵ being one of
H and CH₃;
Y is selected from the group consisting of CH₂, and O;
Z is selected from the group consisting of O and bond;
W is selected from the group consisting of CH₂ and CHR¹-CH₂, with R¹ being one of H and OH, wherein the R¹-attached carbon member in said CHR¹-CH₂ is not directly attached to the nitrogen member to which said W is attached;
R⁴ is selected from the group consisting of H, OCH₃, Cl, F, Br, I, OH, NH₂, CN, CF₃ and CH₃;
R⁶ is H or F; and
R^{2"} is defined as R² and R^{3"} is defined as R³, as follows:
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said -C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two -N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=; and
L) one of 2-(4-ethyl-phenoxy)-benzothiazole, 2-(4-ethyl-phenoxy)-benzooxazole, and 2-(4-ethyl-phenoxy)-1*H*-benzoimidazole;
M) SO₂C₁₋₄alkyl;
alternatively R² and R³ are taken together with the nitrogen to which they are attached to form a heterocyclic ring that contains at least one heteroatom member that is said attachment nitrogen, said heterocyclic ring being selected from the group consisting of
i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄alkylOR^{Y}, -C₀₋₄alkylC(O)NR^{Y}R^{Z}, -C₀₋₄alkylNR^{Y}C(O)R^{Z}, - C(O)NR^{Z}OR^{Y}, -C₀₋₄alkylNR^{Y}C(O)CH₂OR^{Y}, - C₀₋₄alkylNR^{Y}C(O)CH₂C(O)R^{Y}, -C₀₋₄alkylNR^{Y}CO₂R^{Y}, - C₀₋₄alkylNR^{Y}C(O)NR^{Y}R^{Z}, -C₀₋₄alkylNR^{Y}C(S)NR^{Y}R^{Z}, -NR^{Y}C(O)CO₂R^{Y},-NR^{Y}R^{Z}, -C₀₋₄alkylNR^{W}SO₂R^{Y}, 1,3-dihydro-indol-2-one-1-yl, 1,3-dihydro-benzoimidazol-2-one-1-yl, tetrazol-5-yl, 1-R^{Y}-1*H-*tetrazol-5-yl, R^{Y}-triazolyl, 2-R^{Y}-2*H-*tetrazol-5-yl, pyrrolidine-2-thion-1-yl, piperidine-2-thion-1-yl, - C₀₋₄alkylC(O)N(R^{Y})(SO₂R^{Y}), -C₀₋₄alkylN(R^{Y})(SO₂)NR^{Y}R^{Y}, -C₀₋₄alkylN(R^{Y})(SO₂)NR^{Y}CO₂R^{Y}, halo, 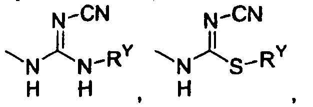 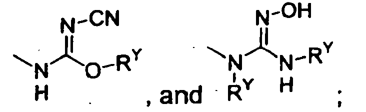
ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y}, -CO₂R^{Y} -C₃₋₄alkylCO₂R^{Y} and R^{Z};
iii) one of imidazolidin-1-yl, 2-imidazolin-1-yl, pyrazol-1-yl, imidazol-1-yl, 2*H*-tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1*H-*tetrazol-1-yl is substituted at the carbon member with 0 or 1 of -C₀₋₄alkylR^{Z}, -C₀₋₄alkylSR^{Y}, -C₀₋₄alkylCO₂R^{Y}, and substituent HetR^{a}; and
iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N}
R^{L} is selected from the group consisting of -CO₂R^{S} and -C(O)NR^{S}R^{S'};
R^{M} is selected from the group consisting of R^{Z}, indol-7-yl, -SO₂R^{Y}, -C₃₋₄alkylCO₂R^{Y}, -CO₂R^{Y}, -C(O)NR^{Z}OR^{Y}, -C(O)R^{Y}, -C(O)C₁₋₄alkylOR^{Y}, - C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H-*tetrazol-5-yl,
R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN, CF₃, CH₃, OC(O)CH₃, and NO₂;
R^{P} is selected from the group consisting of R^{Y}, -C₂₋₄alkylOR^{Y}, R^{Ar}, -C₁₋₂alkylCO₂R^{Y}, -C₁₋₂alkylCONR^{S}R^{S'}, indol-7-yl, and -SO₂C₁₋₄alkyl;
R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}, -C₀₋₄alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y};
R^{S} and R^{S'} are independently selected from the group consisting of H, -C₁₋₄alkyl, and -C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L};
R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar};
R^{Y} is selected from the group consisting of H, -C₁₋₄alkyl, -C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y},
-C₁₋₂alkylCO₂R^{Y}, -C₁₋₂alkylC(O)NR^{S}R^{S'}, and -C₂₋₄alkylNR^{S}R^{S'};
when R and R^{Z'} are attached to a nitrogen member, R^{Y} and R^{Z} are selected as defined above, or R^{Y} and R^{Z} are taken together with the R^{Y}- and R^{Z}-attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring HetR^{d} having 0 or 1 valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1 unsaturated carbon-carbon bonds and having 0 or 1 carbonyl members;
provided that
(a) said R^{2"} and R^{3"} further satisfy one of the following:
(e1): at least one of said R^{2"} and R^{3"} is not C₁₋₅alkyl , when Z is O and X is S;
(e2): none of R^{2"} and R^{3"} is C₁₋₄alkylC(O)R^{X}. with R^{X} being one of C₁₋₄alkyl, OH, -OC₁₋₄alkyl, -OC₀₋₄alkylR^{Ar}, or -NR^{Y}R^{Y}, when Y is O, Z is bond, and R^{2"} is different from R^{3"}; and
(e3): none of R^{2"} and R^{3"} is -C₁₋₆alkylCN, when Y is O, Z is bond, and R^{2"} is different from R^{3"}; and
(b) further provided that when X is S, Y is O, Z is bond, and W is CH₂, then one of R^{2"} and R^{3"} is not XCG when the other is C₁₋₆alkyl, wherein XCG is the group 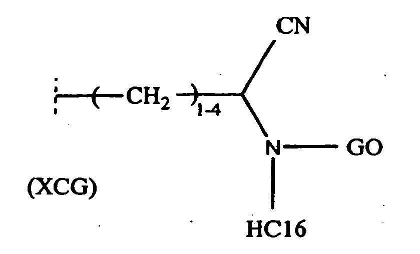
wherein CC16 is one of H, C₁₋₆alkyl, haloC₁₋₆alkyl, allyl, and C₁₋₆alkoxymethyl, and GO is a group attached by a carbon member that has a =O substituent forming an amido group with the nitrogen member to which said GO group is attached.

3. A compound as claimed in claim 1 wherein said R⁴ is H.

4. A compound as claimed in claim 2 wherein said R⁴ is H.

5. A compound as claimed in claim 1, wherein said R² and R³ are each independently selected from
(i) the group consisting of A), B), C), D), E), and I) when R² and R³ are selected from different groups; or
(ii) group A); or
(iii) group B); or
(iv) group C); or
(v) group D); or
(vi) group E); or
(vii) group I),
as defined in claim 1.

6. A compound as claimed in claim 2, wherein said R² and R³ are each independently selected from
(i) the group consisting of A), B), C), D), E), and I) when R² and R³ are selected from different groups; or
(ii) group A); or
(iii) group B); or
(iv) group C); or
(v) group D); or
(vi) group E); or
(vii) group I),
as defined in claim 2.

7. A compound as claimed in claim 1 wherein said R² and R³ are taken together with the nitrogen to which they are attached to form a heterocyclic ring that contains at least one heteroatom member that is said attachment nitrogen, said heterocyclic ring being selected from any one of
(a) the group consisting of i) and ii) when R² and R³ are selected from different groups;
(b) the group i), and
(c) the group ii)
as defined in claim 1.

8. A compound as claimed in claim 2 wherein said R² and R³ are taken together with the nitrogen to which they are attached to form a heterocyclic ring that contains at least one heteroatom member that is said attachment nitrogen, said heterocyclic ring being selected from any one of
(d) the group consisting of i) and ii) when R² and R³ are selected from different groups;
(e) the group i), and
(f) the group ii)
as defined in claim 2.

9. A compound as claimed in claim 1, wherein said compound of formula (II) is one of:
2-[4-(2-Piperidin-1-yl-ethoxy)-phenoxy]-benzooxazole;
(1-{2-[4-(Benzooxazol-2-yloxyrphenoxy]ethyl}-piperidin-4-yl)-methanol;
1-{2-[4-(Benzooxazol-2-yloxy)-phenoxy]-ethyl}-piperidin-4-ol;
{2-[4-(Benzooxazol-2-yloxy)-phenoxy]-ethyl}-dibutyl-amine;
(1-{2-[4-(Benzooxazol-2-yloxy)-phenoxy]-ethyl}-piperidin-2-yl)-methanol;
1-{3-[4-(Benzooxazol-2-yloxy)-phenoxy]-propyl}-4-phenyl-piperidin-4-ol;
1-{3-[4-(Benzooxazol-2-yloxy)-phenoxy]-propyl}-4-benzyl-piperidin-4-ol;
2-[4-(2-Piperidin-1-yl-ethyl)-phenoxy]-benzooxazole;
{3-[4-(Benzooxazol-2-yloxy)-phenyl]-propyl}-cyclohexyl-ethyl-amine;
1-{3-[4-(Benzooxazol-2-yloxy)-phenyl]-propyl}-piperidin-4-ol;
1-{3-[4-(Benzooxazol-2-yloxy)-phenoxy]-2-hydroxy-propyl}-4-phenyl-piperidin-4-ol;
1-[2-(4-Benzooxazol-2-ylmethyl-phenoxy)-ethyl]-piperidine-4-carboxylic acid ethyl ester;
2-[4-(2-Pyrrolidin-1-yl-ethoxy)-phenoxy]-benzooxazole;
{3-[4-(Benzooxazol-2-yloxyl)-phenoxy]-propyl}-dimethyl-amine;
{2-[4-(Benzooxazol-2-yloxy)-phenoxy]-ethyl}-dimethyl-amine; and
2-[4-(2-Azepan-1-yl-ethoxy)-phenoxy]-benzooxazole.

10. A compound as in claim 1, wherein said compound of formula (II) is one of:
1-{2-[4-(Benzooxazol-2-yloxy)-phenoxy]-ethyl}-4-phenyl-piperidin-4-ol;
{2-[4-(Benzooxazol-2-yloxy)-phenoxy]-ethyl}-cyclohexyl-ethyl-amine;
2-{4-[2-(2-Ethyl-piperidin-1-yl)-ethoxy]-phenoxy}-benzooxazole;
1-{2-[4-(Benzooxazol-2-yloxy)-phenoxy]-ethyl}-4-phenyl-piperidine-4-carbonitrile;
1-(1-{2-[4-(Benzooxazol-2-yloxy)-phenoxy]-ethyl}-4-phenyl-piperidin-4-yl)-ethanone;
2-{4-[2-(4-Methyl-piperidin-1-yl)-ethoxy]-phenoxy}-benzooxazole;
1-{2-[4-(Benzooxazol-2-yloxy)-phenoxy]-ethyl}-4-(4-chloro-phenyl)-piperidin-4-ol;
1-{2-[4-(Benzooxazol-2-yloxy)-phenoxy]-ethyl}-4-(4-bromo-phenyl)-piperidin-4-ol;
1-{2-[4-(Benzooxazol-2-yloxy)-phenoxy]-ethyl}-4-(4-chloro-3-trifluoromethyl-phenyl)-piperidin-4-ol;
1-{2-[4-(Benzooxazol-2-yloxy)-phenoxy]-ethyl}-4-benzyl-piperidin-4-ol;
{2-[4-(Benzooxazol-2-yloxy)-phenoxy]-ethyl}-cyclohexyl-methyl-amine; and
{2-[4-(Benzooxazol-2-yloxy]-phenoxy]-ethyl}-cyclopropylmethyl-propyl-amine.

11. A compound as in claim 1, wherein said compound of formula (II) is one of:
{2-[4-(Benzooxazol-2-yloxy)-phenoxy]-ethyl}-butyl-ethyl-amine;
2-({2-[4-(Benzooxazol-2-yloxy)-phenoxy]-ethyl}-benzyl-amino)-ethanol;
2-{4-[2-(4-Benzyl-piperidin-1-yl)-ethoxy]-phenoxy}-benzooxazole;
(1-{2-[4-(Benzooxazol-2-yloxy)-phenoxy]-ethyl}-piperidin-3-yl)-methanol;
2-({2-[4-(Benzooxazol-2-yloxy)-phenoxy]-ethyl}-propyl-amino)-ethanol;
2-[4-(2-Azetidin-1-yl-ethoxy)-phenoxy]-benzooxazole;
*N*-(1-{2-[4-(Benzooxazol-2-yloxy)-phenoxy]-ethyl}-piperidin-4-yl)-2-phenyl-acetamide;
1-{2-[4-(Benzooxazol-2-yloxy)-phenoxy]-ethyl}-piperidine-3-carboxylic acid ethyl ester;
2-{4-[3-(4-Phenyl-piperidin-1-yl)-propoxy]-phenoxy}-benzooxazole;
1-{2-[4-(Benzooxazol-2-yloxy)-phenyl]-ethyl}-4-phenyl-piperidin-4-ol;
{2-[4-(Benzooxazol-2-yloxy)-phenyl]-ethyl}-cyclohexyl-ethyl-amine;
2-[4-(2-Pyrrolidin-1-yl-ethyl)-phenoxy]-benzooxazole; and
2-[4-(2-Azepan-1-yl-ethyl)-phenoxy]-benzooxazole.

12. A compound as in claim 1, wherein said compound of formula (II) is one of:
{2-[4-(Benzooxazol-2-yloxy)-phenyl]-ethyl}-cyclopropylmethyl-propyl-amine;
{2-[4-(Benzooxazol-2-yloxy)-phenyl]-ethyl}-dibutyl-amine;
1-{2-[4-(Benzooxazol-2-yloxy)-phenyl]-ethyl}-piperidin-4-ol;
1-{2-[4-(Benzooxazol-2-yloxy)-phenyl]-ethyl}-piperidine-4-carboxylic acid methyl ester;
1-{3-[4-(Benzooxazol-2-yloxy)-phenyl]-propyl}-4-phenyl-piperidin-4-ol;
2-[4-(3-Piperidin-1-yl-propyl)-phenoxy]-benzooxazole;
{3-[4-(Benzooxazol-2-yloxy)-phenyl]-propyl}-dibutyl-amine;
{3-[4-(Benzooxazol-2-yloxy)-phenyl]-propyl}-cyclopropylmethyl-propyl-amine;
1-[4-(Benzooxazol-2-yloxy)-phenoxy]-3-pyrrolidin-1-yl-propan-2-ol;
1-[2-(4-Benzooxazol-2-ylmethyl-phenoxy)-ethyl]-4-phenyl-piperidin-4-ol;
1-[2-(4-Benzooxazol-2-ylmethyl-phenoxy)-ethyl]-piperidine-4-carboxylic acid amide
2-[4-(2-Morpholin-4-yl-ethoxy)-phenoxy]-benzooxazole; and
{2-[4-(Benzooxazol-2-yloxy)-phenoxy]-ethyl}-diethyl-amine.

13. A compound as in claim 1, wherein said compound of formula (II) is one of:
{2-[4-(6-Chloro-benzothiazol-2-yloxy)-phenoxy]-ethyl}-diethylamine;
1-{2-[4-(Benzothiazol-2-yloxyl)-phenoxy]-ethyl}-piperidin-4-ol;
1-{2-[4-(Benzothiazol-2-yloxy)-phenoxy]-ethyl}-piperidine-4-carboxylic acid ethyl ester;
1-{2-[4-(Benzothiazol-2-yloxy)-phenoxy]-ethyl}-piperidine-4-carboxylic acid;
(1-{2-[4-(Benzothiazol-2-yloxy)-phenoxy]-ethyl}-pipendin-4-yl)-pyrrolidin-1-yl-methanone;
3-[(1-{2-[4-(Benzothiazol-2-yloxy)-phenoxy]-ethyl}-piperidine-4-carbonyl)-amino]-propionic acid ethyl ester;
1-{2-[4-(Benzothiazol-2-yloxy)-phenoxy]-ethyl}-piperidine-4-carboxylic acid amide;
1-(1-{2-[4-(Benzothiazol-2-yloxy)-phenoxy]-ethyl}-piperidin-4-yl)-pyrrolidin-2-one;
1'-{2-[4-(Benzothiazol-2-yloxy)-phenoxy]-ethyl}-[1,4]bipiperidinyl-2-one;
8-{2-[4-(Benzothiazol-2-yloxy)-phenoxy]-ethyl}-2,8-diaza-spiro[4.5]decan-1-one;
2-[4-(3-Pyrrolidin-1-yl-propoxy)-phenoxy]-benzothiazole;
{2-[4-(Benzothiazol-2-yloxy)-phenyl]-ethyl}-cyclohexyl-ethyl-amine;
1-{2-[4-(Benzothiazol-2-yloxy)-phenyl]-ethyl}-piperidine-3-carboxylic acid amide;
1-(1-{2-[4-(Benzothiazol-2-yloxy)-phenyl]-ethyl}-piperidin-4-yl)-3-methyl-1,3-dihydro-benzoimidazol-2-one;
1-{2-[4-(Benzothiazol-2-yloxy)-phenyl]-ethyl}-piperidine-4-carboxylic acid methyl ester;
(1-{2-[4-(Benzothiazol-2-yloxy)-phenyl]-ethyl}-piperidin-4-yl)-(4-methyl-piperazin-1-yl)-methanone;
1-[2-(4-Benzothiazol-2-ylmethyl-phenoxy)-ethyl]-piperidine-4-carboxylic acid methyl ester;
3-({2-[4-(Benzothiazol-2-yloxy)-phenoxy]-ethyl}-cyclopropyl-amino)-propionic acid;
{2-[4-(Benzothiazol-2-yloxy)-phenoxy]-ethyl}-dimethyl-amine;
2-[4-(2-Pyrrolidin-1-yl-ethoxy)-phenoxy]-benzothiazole;
{3-[4-(Benzothiazol-2-yloxy)-phenoxy]-propyl}-dimethyl-amine;
2-[4-(2-Azepan-1-yl-ethoxy)-phenoxy]-benzothiazole;
2-[4-(2-Azepan-1-yl-ethoxy)-phenoxy]-6-methoxy-benzothiazole;
1-{2-[4-(Benzothiazol-2-yloxy)-phenoxy]-ethyl}-4-phenyl-piperidin-4-ol;
{2-[4-(Benzothiazol-2-yloxy)-phenoxy]-ethyl}-cyclohexyl-ethyl-amine;
1-{2-[4-(Benzothiazol-2-yloxy)-phenoxy]-ethyl}-4-(4-chlorophenyl)-piperidin-4-ol;
{2-[4-(Benzothiazol-2-yloxy)-phenoxy]-ethyl}-dibutyl-amine;
1-{2-[4-(Benzothiazol-2-yloxy)-phenoxy]-ethyl}-4-(4-bromophenyl)-piperidin-4-ol;
1-{2-[4-(Benzothiazol-2-yloxy)-phenoxy]-ethyl}-4-(4-chloro-3-trifluoromethyl-phenyl)-piperidin-4-ol;
1-{2-[4-(Benzothiazol-2-yloxy)-phenoxy]-ethyl}-4-benzyl-piperidin-4-ol;
1'-{2-[4-(Benzothiazol-2-yloxy)-phenoxy]-ethyl}-[1,4']bipiperidine;
(1-{2-[4-(Benzothiazol-2-yloxy)-phenoxy]-ethyl}-piperidin-4-yl)-methanol;
*N*-(1-{2-[4-(Benzothiazol-2-yloxy)-phenoxy]-ethyl}-piperidin-4-yl)-2-phenyl-acetamide;
2-(4-{2-[4-(2-Morpholin-4-yl-ethyl)-piperazin-1-yl]-ethoxy}-phenoxy)-benzothiazole;
2-(4-{2-[4-(2-Morpholin-4-yl-ethyl)-piperazin-1-yl]-ethyl}-phenoxy)-benzothiazole;
1-{3-[4-(Benzothiazol-2-yloxy)-phenoxy]-propyl}-4-phenyl-piperidin-4-ol;
1-{2-[4-(Benzothiazol-2-yloxy)-phenyl]-ethyl}-4-phenyl-piperidin-4-ol;
2-[4-(2-Pyrrolidin-1-yl-ethyl)-phenoxy]-benzothiazole;
2-[4-(2-Azepan-1-yl-ethyl)-phenoxy]-benzothiazole;
{2-[4-(Benzothiazol-2-yloxy)-phenyl]-ethyl}-cyclopropylmethyl-propyl-amine;
{2-[4-(Benzothiazol-2-yloxy)-phenyl]-ethyl}-dibutyl-amine;
2-[4-(2-Piperidin-1-yl-ethyl)-phenoxy]-benzothiazole;
1-{2-[4-(Benzothiazol-2-yloxy)-phenyl]-ethyl}-piperidin-4-ol;
1-{2-[4-(Benzothiazol-2-yloxy)-phenyl]-ethyl}-piperidine-4-carboxylic acid amide;
1-{2-[4-(Benzothiazol-2-yloxy)-phenyl]-ethyl}-piperidine-3-carboxylic acid ethyl ester;
1-{2-[4-(Benzothiazol-2-yloxy)-phenyl]-ethyl}-4-phenyl-piperidine-4-carboxylic acid ethyl ester;
(1-{2-[4-(Benzothiazol-2-yloxy)-phenyl]-ethyl}-piperidin-4-yl)-acetic acid ethyl ester;
1-(1-{2-[4-(Benzothiazol-2-yloxy)-phenyl]-ethyl}-piperidin-4-yl)-1,3-dihydro-indol-2-one;
1-(1-{2-[4-(Benzothiazol-2-yloxy)-phenyl]-ethyl}-piperidin-4-yl)-pyrrolidin-2-one;
*N*-(1-{2-[4-(Benzothiazol-2-yloxy)-phenyl]-ethyl}-piperidin-4-yl)-2-phenyl-acetamide;
8-{2-[4-(Benzothiazol-2-yloxy)-phenyl]-ethyl}-2,8-diaza-spiro[4.5]decan-1-one;
1-{2-[4-(Benzothiazol-2-yloxy)-phenyl]-ethyl}-piperidin-3-ol;
1-{2-[4-(Benzothiazol-2-yloxy)-phenyl]-ethyl}-piperidine-4-carboxylic acid ethyl ester;
1'-{2-[4-(Benzothiazol-2-yloxy)-phenyl]-ethyl}-[1,4']bipiperidine;
2-{4-[2-(4-Methyl-piperazin-1-yl)-ethyl]-phenoxy}-benzothiazole;
2-(4-{2-[4-(1-Benzyl-1*H*-tetrazol-5-yl)-piperidin-1-yl]-ethoxy}-phenoxy)-benzothiazole;
4-(1-{2-[4-(Benzothiazol-2-yloxy)-phenyl]-ethyl}-piperidine-4-carbonyl)-piperazine-1-carboxylic acid *tert*-butyl ester;
1-[2-(4-Benzothiazol-2-ylmethyl-phenoxy)-ethyl]-piperidine-4-carboxylic acid amide;
1-{1-[2-(4-Benzothiazol-2-ylmethyl-phenoxy)-ethyl]-piperidin-4-yl}-pyrrolidin-2-one;
1-[4-(1-{2-[4-(Benzothiazol-2-yloxy)-phenyl]-ethyl}-piperidine-4-carbonyl)-piperazin-1-yl]-ethanone;
1-{2-[4-(Benzothiazol-2-yloxy)-phenyl]-ethyl}-piperidine-4-carboxylic acid;
1-(1-{2-[4-(Benzothiazol-2-yloxy)-phenoxy]-ethyl}-piperidin-4-yl)-pyrrolidine-2-thione;
2-(4-{2-[4-(Benzothiazol-2-yloxy)-phenoxy]-ethyl}-piperazin-1-yl)-ethanol;
2-(4-{2-[4-(Benzothiazol-2-yloxy)-phenoxy]-ethyl}-piperazin-1-yl)-1-pyrrolidin-1-yl-ethanone;
2-(4-{2-[4-(Benzothiazol-2-yloxy)-phenoxy]-ethyl}-piperazin-1-yl)-1-morpholin-4-yl-ethanone;
1-{2-[4-(Benzothiazol-2-yloxy)-phenyl]-ethyl}-piperidine-3-carboxylic acid;
1-{2-[4-(Benzothiazol-2-yloxy)-phenyl]-ethyl}-piperidine-2-carboxylic acid;
(1-{2-[4-(Benzothiazol-2-yloxy)-phenyl]-ethyl}-piperidin-3-yl)-acetic acid;
(1-{2-[4-(Benzothiazol-2-yloxy)-phenoxy]-ethyl}-piperidin-4-yl)-acetic acid ethyl ester;
(1-{2-[4-(Benzothiazol-2-yloxy)-phenoxy]-ethyl}-piperidin-4-yl)-carbamic acid tert-butyl ester;
(1-{2-[4-(Benzothiazol-2-yloxy)-phenoxy]-ethyl}-piperidin-4-yl)-acetic acid;
(1-{2-[4-(Benzothiazol-2-yloxy)-phenyl]-ethyl}-piperidin-3-yl)-methanol;
({2-[4-(Benzothiazol-2-yloxy)-phenyl]-ethyl}-cyclohexyl-amino)-acetic acid methyl ester;
(4-{2-[4-(Benzothiazol-2-yloxy)-phenoxy]-ethyl}-piperazin-1-yl)-acetic acid;
1-(1-{2-[4-(Benzothiazol-2-yloxy)-phenoxy]-ethyl}-piperidin-4-yl)-5-oxo-pyrrolidine-2-carboxylic acid ethyl ester;
1-(1-{2-[4-(Benzothiazol-2-yloxy)-phenoxy]-ethyl}-piperidin-4-yl)-5-oxo-pyrrolidine-2-carboxylic acid;
4-(4-{2-[4-(Benzothiazol-2-yloxy)-phenoxy]-ethyl}-piperazin-1-yl)-phenol;
*N*-(1-{2-[4-(Benzothiazol-2-yloxy)-phenoxy]-ethyl}-piperidin-4-yl)-4-chloro-*N*-cyclopropyl-benzene sulfonamide;
3-({2-[4-(Benzothiazol-2-yloxy)-phenoxy]-ethyl}-cyclopropylmethyl-amino)-propionic acid;
3-({2-[4-(Benzothiazol-2-yloxy)-phenoxy]-ethyl}-isopropyl-amino)-propionic acid;
1-{2-[4-(Benzothiazol-2-yloxy)-phenoxy]-ethyl}-piperidin-4-ylamine;
3-[{2-[4-(Benzothiazol-2-yloxy)-phenoxy]-ethyl}-(1-methyl-piperidin-4-yl)-amino]-propionic acid;
3-({2-[4-(Benzothiazol-2-yloxy)-phenoxy]-ethyl}-benzyl-amino)-propionic acid;
3-((1-Acetyl-piperidin-4-yl)-{2-[4-(benzothiazol-2-yloxy)-phenoxy]-ethyl}-amino)-propionic acid;
4-(1-{2-[4-(Benzothiazol-2-yloxy)-phenoxy]-ethyl}-1H-tetrazol-5-yl)-piperidine-1-carboxylic acid tert-butyl ester;
3-({2-[4-(Benzothiazol-2-yloxy)-phenyl]-ethyl}-cyclopropyl-amino)-propionic acid;
3-((1-Acetyl-piperidin-4-yl)-{2-[4-(benzothiazol-2-yloxy)-phenyl]-ethyl}-amino)-propionic acid;
3-[{2-[4-(Benzothiazol-2-yloxy)-phenyl]-ethyl}-(1-methyl-piperidin-4-yl)-amino]-propionic acid;
3-({2-[4-(Benzothiazol-2-yloxy)-phenyl]-ethyl}-cyclopropylmethyl-amino)-propionic acid;
3-({2-[4-(Benzothiazol-2-yloxy)-phenyl]-ethyl}-isopropyl-amino)-propionic acid;
2-(4-{2-[4-(Benzothiazol-2-yloxy)-phenyl]-ethyl}-piperazin-1-yl)-1-pyrrolidin-1-yl-ethanone;
(*R*)-1-{2-[4-(Benzothiazol-2-yloxy)-phenyl]-ethyl}-piperidine-3-carboxylic acid;
1-(1-{2-[4-(Benzothiazol-2-yloxy)-phenyl]-ethyl}-piperidin-4-yl)-1,3-dihydro-benzoimidazol-2-one;
2-(4-{2-[4-(6-Methyl-pyridin-2-yl)-piperazin-1-yl]-ethyl}-phenoxy)-benzothiazole;
2-{4-[2-(4-Ethanesulfonyl-piperazin-1-yl)-ethyl]-phenoxy}-benzothiazole;
2-(4-{2-[4-(Benzothiazol-2-yloxy)-phenyl]-ethyl}-piperazin-1-yl)-1-morpholin-4-yl-ethanone;
3-({2-[4-(Benzothiazol-2-yloxy)-phenyl]-ethyl}-methyl-amino)-propionic acid;
3-({2-[4-(Benzothiazol-2-yloxy)-phenyl]-ethyl}-cyclopentyl-amino)-propionic acid;
3-({2-[4-(Benzothiazol-2-yloxy)-phenyl]-ethyl}-cyclobutyl-amino)-propionic acid;
3-({2-[4-(Benzothiazol-2-yloxy)-phenyl]-ethyl}-benzyl-amino)-propionic acid;
(1-{2-[4-(Benzothiazol-2-yloxy)-phenoxy]-ethyl}-piperidin-4-yl)-(4-hydroxymethyl-piperidin-1-yl)-methanone;
{2-[4-(Benzothiazol-2-yloxy)-phenyl]-ethyl}-cyclopropyl-amine;
(1-{2-[4-(Benzothiazol-2-yloxy)-phenoxy]-ethyl}-piperidin-4-yl)-[4-(2-hydroxy-ethyl)-piperazin-1-yl]-methanone;
(1-{2-[4-(Benzothiazol-2-yloxy)-phenoxy]-ethyl}-piperidin-4-yl)-[4-(2-hydroxy-ethyl)-piperidin-1-yl]-methanone;
2-({2-[4-(Benzothiazol-2-yloxy)-phenyl]-ethyl}-cyclopropyl-amino)-ethanol;
3-({2-[4-(Benzothiazol-2-yloxy)-phenyl]-ethyl}-cyclopropyl-amino)-propan-1-ol;
4-({2-[4-(Benzothiazol-2-yloxy)-phenyl]-ethyl}-cyclopropyl-amino)-butyric acid;
3-[(1-{2-[4-(Benzothiazol-2-yloxy)-phenoxy]-ethyl}-piperidine-4-carbonyl)-amino]-prooionic acid;
4-({2-[4-(Benzothiazol-2-yloxy)-phenyl]-ethyl}-cyclopropyl-amino)-butyronitrile;
3-(1-{2-[4-(Benzothiazol-2-yloxy)-phenyl]-ethyl}-piperidin-4-yl)-propionic acid;
[(1-{2-[4-(Benzothiazol-2-yloxy)-phenoxy]-ethyl}-piperidine-4-carbonyl)-methyl-amino]-acetic acid;
3-(4-{2-[4-(Benzothiazol-2-yloxy)-phenoxy]-ethyl}-piperazin-1-yl)-phenol;
2-(4-{2-[4-(4-Methoxy-phenyl)-piperazin-1-yl]-ethoxy}-phenoxy)-benzothiazole;
2-{4-[2-(5-Piperidin-4-yl-tetrazol-1-yl)-ethoxy]-phenoxy}-benzothiazole;
(*S*)-1-(1-{2-[4-(Benzothiazol-2-yloxy)-phenoxy]-ethyl}-piperidin-4-yl)-4-hydroxy-pyrrolidin-2-one;
{2-[4-(Benzothiazol-2-yloxy)-phenyl]-ethyl}-cyclopropylmethyl-amine; .
2-[({2-[4-(Benzothiazol-2-yloxy)-phenyl]-ethyl}-cyclopropyl-amino)-methyl]-cyclopropanecarboxylic acid ethyl ester;
4-(4-{2-[4-(Benzothiazol-2-yloxy)-phenoxy]-ethyl}-piperazine-1-carbonyl)-benzoic acid ethyl ester;
2-[({2-[4-(Benzothiazol-2-yloxy)-phenyl]-ethyl}-cyclopropyl-amino)-methyl]-cyclopropanecarboxylic acid;
1-({2-[4-(Benzothiazol-2-yloxy)-phenyl]-ethyl}-cyclopropyl-amino)-propan-2-ol;
3-({2-[4-(Benzothiazol-2-yloxy)-phenyl]-ethyl}-cyclopropyl-amino)-1,1,1-trifluoro-propan-2-ol;
3-({2-[4-(Benzothiazol-2-yloxy)-phenyl]-ethyl}-cyclopropyl-amino)-propionamide;
3-({2-[4-(Benzothiazol-2-yloxy)-phenyl]-ethyl}-cyclopropyl-amino)-propane-1,2-diol;
2-{4-[2-(5-Phenyl-tetrazol-2-yl)-ethoxy]-phenoxy}-benzothiazole;
2-{4-[2-(5-Phenyl-tetrazol-1-yl)-ethoxy]-phenoxy}-benzothiazole;
*N*-{2-[4-(Benzothiazol-2-yloxy)-phenyl]-ethyl}-*N*-cyclopropyl-2-(2H-tetrazol-5-yl)-acetamide;
(S)-3-({2-[4-(Benzothiazol-2-yloxy)-phenyl]-ethyl}-cyclopropyl-amino)-2-methyl-propan-1-ol;
(*R*)-3-({2-[4-(Benzothiazol-2-yloxy)-phenyl]-ethyl}-cyclopropyl-amino)-2-methyl-propan-1-ol;
2-{4-[2-(5-Methylsulfanyl-tetrazol-2-yl)-ethoxy]-phenoxy}-benzothiazole;
2-{4-[2-(5-Methylsulfanyl-tetrazol-1-yl)-ethoxy]-phenoxy}-benzothiazole;
2-[4-(2-Tetrazol-2-yl-ethoxy)-phenoxy]-benzothiazole;
2-[4-(2-Tetrazol-1-yl-ethoxy)-phenoxy]-benzothiazole;
(1*R*,2*R*)-2-{2-[4-(Benzothiazol-2-yloxy)-phenyl]-ethylamino}-cyclohexanecarboxylic acid;
(1*S*,2*R*)-2-{2-[4-(Benzothiazol-2-yloxy)-phenyl]-ethylamino}-cyclohexanecarboxylic acid;
(1*R*,2*R*)-2-{2-[4-(Benzothiazol-2-yloxy)-phenyl]-ethylamino}-cyclohexanol;
(1*S*,2*R*)-2-{2-[4-(Benzothiazol-2-yloxy)-phenyl]-ethylamino}-cyclohexanol;
4-(1-{2-[4-(Benzothiazol-2-yloxy)-phenyl]-ethyl}-piperidin-4-yl)-butyric acid;
(R) 1-(1-{2-[4-(Benzothiazol-2-yloxy)-phenoxy]-ethyl}-piperidin-4-yl)-4-hydroxy-pyrrolidin-2-one;
2-(4-{2-[4-(1H-Tetrazol-5-yl)-piperidin-1-yl]-ethyl}-phenoxy)-benzothiazole;
(1-{2-[4-(Benzothiazol-2-yloxy)-phenoxy]-ethyl}-piperidin-2-yl)-methanol;
(1-{2-[4-(Benzothiazol-2-yloxy)-phenoxy]-ethyl}-1H-tetrazol-5-yl)-acetic acid ethyl ester;
(1-{2-[4-(Benzothiazol-2-yloxy)-phenoxy]-ethyl}-1H-tetrazol-5-yl)-acetic acid ethyl ester;
2-{4-[2-(5-Piperidin-4-yl-tetrazol-2-yl)-ethoxy]-phenoxy}-benzothiazole hydrochloride;
7-{2-[4-(Benzothiazol-2-yloxy)-phenoxy]-ethyl}-4-spiro-[3-phthalide]-piperidine;
1-{3-[4-(Benzothiazol-2-yloxy)-phenyl]-propyl}-piperidine-4-carboxylic acid ethyl ester;
2-[4-(Benzothiazol-2-yloxy)-phenyl]-ethylamine hydrochloride;
2-(4-{2-[4-(1H-Tetrazol-5-yl)-piperidin-1-yl]-ethoxy}-phenoxy)-benzothiazole;
Cis-4-{2-[4-(Benzothiazol-2-yloxy)-phenyl]-ethylamino}-cyclohexanecarboxylic acid trifluoromethanesulfonate salt;
(4-{2-[4-(Benzothiazol-2-yloxy)-phenyl]-ethyl}-piperazin-1-yl)-(tetrahydro-furan-2-yl)-methanone;
Propane-2-sulfonic acid (1-{2-[4-(benzothiazol-2-yloxy)-phenoxy]-ethyl}-piperidine-4-carbonyl)-amide;
(4-{2-[4-(Benzothiazol-2-yloxy)-phenyl]-ethyl}-piperazin-1-yl)-oxo-acetic acid methyl ester;
N-(1-{2-[4-(Benzothiazol-2-yloxy)-phenyl]-ethyl}-piperidine-4-carbonyl)-benzenesulfonamide trifluoromethanesulfonate salt;
N-(1-{2-[4-(Benzothiazol-2-yloxy)-phenyl]-ethyl}-piperidine-4-carbonyl)-methanesulfonamide trifluoromethanesulfonate salt;
(4-{2-[4-(Benzothiazol-2-yloxy)-phenyl]-ethyl}-piperazin-1-yl)-oxo-acetic acid trifluoromethanesulfonate salt;
(4-{2-[4-(Benzothiazol-2-yloxy)-phenyl]-ethyl}-piperazin-1-yl)-morpholin-4-yl-methanone;
1-(4-{2-[4-(Benzothiazol-2-yloxy)-phenyl]-ethyl}-piperazin-1-yl)-2-thiophen-2-yl-ethanone;
(4-{2-[4-(Benzothiazol-2-yloxy)-phenyl]-ethyl}-piperazin-1-yl)-pyridin-3-yl-methanone;
(4-{2-[4-(Benzothiazol-2-yloxy)-phenyl]-ethyl}-piperazin-1-yl)-cyclopropyl-methanone;
1-(4-{2-[4-(Benzothiazol-2-yloxy)-phenyl]-ethyl}-piperazin-1-yl)-2-methoxy-ethanone;
1-(4-{2-[4-(Benzothiazol-2-yloxy)-phenyl]-ethyl}-piperazin-1-yl)-2,2,2-trifluoro-ethanone;
4-(4-{2-[4-(Benzothiazol-2-yloxy)-phenyl]-ethyl}-piperazine-1-carbonyl)-benzoic acid;
(4-{2-[4-(Benzothiazol-2-yloxy)-phenyl]-ethyl}-piperazin-1-yl)-pyridin-4-yl-methanone;
(4-{2-[4-(Benzothiazol-2-yloxy)-phenyl]-ethyl}-piperazin-1-yl)-(5-methyl-pyrazin-2-yl)-methanone;
(*R*)-(4-{2-[4-(Benzothiazol-2-yloxy)-phenyl]-ethyl}-piperazin-1-yl)-(tetrahydro-furan-2-yl)-methanone;
(*S*)-(-4-{2-[4-(Benzothiazol-2-yloxy)-phenyl]-ethyl}-piperazin-1-yl)-(tetrahydro-furan-2-yl)-methanone;
(4-{2-[4-(Benzothiazol-2-yloxy)-phenyl]-ethyl}-piperazin-1-yl)-(tetrahydro-furan-3-yl)-methanone;
1-(4-{2-[4-(Benzothiazol-2-yloxy)-phenyl]-ethyl}-piperazin-1-yl)-2-hydroxy-ethanone;
2-[2-(4-{2-[4-(Benzothiazol-2-yloxy)-phenyl]-ethyl}-piperazin-1-yl)-2-oxo-ethyl]-cyclopentanone;
3-(4-{2-[4-(Benzothiazol-2-yloxy)-phenyl]-ethyl}-piperazin-1-yl)-propionic acid trifluoromethanesufonate salt;
3-(1-{2-[4-(Benzothiazol-2-yloxy)-phenyl]-ethyl}-piperidin-4-yl)-oxazolidin-2-one;
4-(1-{2-[4-(Benzothiazol-2-yloxy)-phenyl]-ethyl}-pipendin-4-yl)-morpholin-3-one;
4-(1-{2-[4-(Benzothiazol-2-yloxy)-phenoxy]-ethyl}-piperidin-4-yl)-morpholin-3-one;
3-(1-{2-[4-(Benzothiazol-2-yloxy)-phenoxy]-ethyl}-piperidin-4-yl)-oxazolidin-2-one;
1-{2-[4-(Benzothiazol-2-yloxy)-phenyl]-ethyl}-piperidine-4-carboxylic acid benzyloxy-amide;
(1-{2-[4-(Benzothiazol-2-yloxy)-phenyl]-ethyl}-piperidin-4-yl)-acetic acid;
(*R*)-1-(1-{2-[4-(Benzothiazol-2-yloxy)-phenyl]-ethyl}-piperidin-4-yl)-4-hydroxy-pyrrolidin-2-one;
1-{2-[4-(Benzothiazol-2-yloxy)-phenyl]-ethyl}-piperidine-4-carboxylic acid hydroxyamide;
(*S*)-1-(1-{2-[4-(Benzothiazol-2-yloxy)-phenyl]-ethyl}-piperidin-4-yl)-4-hydroxy-pyrrolidin-2-one;
(1-{2-[4-(Benzothiazol-2-yloxy)-phenyl]-ethyl}-piperidin-4-yl)-carbamic acid tert-butyl ester;
2-{4-[2-(4-Fluoro-piperidin-1-yl)-ethyl]-phenoxy}-benzothiazole;
2-{4-[2-(4,4-Difluoro-piperidin-1-yl)-ethyl]-phenoxy}-benzothiazole;
(*R*)-1-{2-[4-(Benzothiazol-2-yloxy)-phenyl]-ethyl}-pyrrolidin-3-ol;
N-(1-{2-[4-(Benzothiazol-2-yloxy)-phenyl]-ethyl}-piperidin-4-yl)-formamide;
(1-{2-[4-(Benzothiazol-2-yloxy)-phenyl]-ethyl}-piperidin-4-yl)-urea;
1-(1-{2-[4-(Benzothiazol-2-yloxy)-phenyl]-ethyl}-piperidin-4-yl)-3-cyano-2-phenyl-isourea;
1-(1-{2-[4-(Benzothiazol-2-yloxy)-phenyl]-ethyl}-piperidin-4-yl)-3-cyano-2-methyl-isothiourea;
N-(1-{2-[4-(Benzothiazol-2-yloxy)-phenyl]-ethyl}-piperidin-4-yl)-methanesulfonamide;
1-(1-{2-[4-(Benzothiazol-2-yloxy)-phenyl]-ethyl}-piperidin-4-yl)-3-cyano-2-methyl-guanidine;
8-{2-[4-(Benzothiazol-2-yloxy)-phenyl]-ethyl}-1-phenyl-1,3,8-triaza-spiro[4.5]decan-4-one;
8-{2-[4-(Benzothiazol-2-yloxy)-phenyl]-ethyl}-1,3,8-triaza-spiro[4.5]decane-2,4-dione;
(1-{2-[4-(Benzothiazol-2-yloxy)-phenyl]-ethyl}-piperidin-4-yl)-methyl-carbamic acid tert-butyl ester;
N-(1-{2-[4-(Benzothiazol-2-yloxy)-phenyl]-ethyl}-piperidin-4-yl)-N-methyl-acetamide;
N-(1-{2-[4-(Benzothiazol-2-yloxy)-phenyl]-ethyl}-piperidin4-yl)-N-methyl-methanesulfonamide;
Acetic acid [(1-{2-[4-(benzothiazol-2-yloxy)-phenyl]-ethyl}-piperidin-4-yl)-methyl-carbamoyl]-methyl ester;
N-(1-{2-[4-(Benzothiazol-2-yloxy)-phenyl]-ethyl}-piperidin-4-yl)-N-acetamide;
Acetic acid (1-{2-[4-(benzothiazol-2-yloxy)-phenyl]-ethyl}-piperidin-4-ylcarbamoyl)-methyl ester;
2-({2-[4-(Benzothiazol-2-yloxy)-phenyl]-ethyl}-methyl-amino)-3-(1H-imidazol-2-yl)-propionic acid;
2-(4-{2-[4-(3-Nitro-pyridin-2-yl)-[1,4]diazepan-1-yl]-ethyl}-phenoxy)-benzothiazole;
[(1-{2-[4-(Benzothiazol-2-yloxy)-phenoxy]-ethyl}-piperidine-4-carbonyl)-methyl-amino]-acetic acid ethyl ester;
1'-{2-[4-(Benzothiazol-2-yloxy)-phenoxy]-ethyl}-[1,4']bipiperidinyl-4-carboxylic acid ethyl ester;
1'-{2-[4-(Benzothiazol-2-yloxy)-phenoxy]-ethyl}-[1,4']bipiperidinyl-4-carboxylic acid;
{2-[4-(Benzothiazol-2-yloxy)-phenyl]-ethyl}-cyclopropyl-ethyl-amine;
3-({2-[4-(Benzothiazol-2-yloxy)-phenyl]-ethyl}-cyclopropyl-amino)-2-methyl-propionic acid trifluoromethansulfonic acid salt;
2-({2-[4-(Benzothiazol-2-yloxy)-phenyl]-ethyl}-cyclopropylmethyl-amino)-ethanol;
2-[2-({2-[4-(Benzothiazol-2-yloxy)-phenyl]-ethyl}-cyclopropylmethyl-amino)-ethoxy]-ethanol;
3-({2-[4-(Benzothiazol-2-yloxy)-phenyl]-ethyl}-cyclopropylmethyl-amino)-propan-1-ol;
{2-[4-(Benzothiazol-2-yloxy)-phenyl]-ethyl}-cyclopropylmethyl-(3-tetrazol-2-y1-propyl)-amine;
{2-[4-(Benzothiazol-2-yloxy)-phenyl]-ethyl}-cyclopropyl-(3-pyrrol-1-yl-propyl)-amine;
4-({2-[4-(Benzothiazol-2-yloxy)-phenyl]-ethyl}-cyclopropylmethyl-amino)-butyronitrile;
1-{2-[4-(Benzothiazol-2-yloxy)-phenyl]-ethyl}-piperidine-4-carboxylic acid (2-cyano-ethyl)-amide;
{2-[4-(Benzothiazol-2-yloxy)-phenyl]-ethyl}-cyclopropylmethyl-[3-(2H-tetrazol-5-yl)-propyl]-amine;
3-[5-(1-{2-[4-(Benzothiazol-2-yloxy)-phenyl]-ethyl}-piperidin-4-yl)-tetrazol-1-yl]-propionitrile;
{2-[4-(Benzothiazol-2-yloxy)-phenyl]-ethyl}-cyclopropyl-[3-(2H-tetrazol-5-yl)-propyl]-amine;
1-{2-[4-(Benzothiazol-2-yloxy)-phenyl]-ethyl}-piperidine-4-carboxylic acid (2-hydroxy-1,1-dimethyl-ethyl)-amide;
{2-[4-(Benzothiazol-2-yloxy)-phenyl]-ethyl}-cyclopropylmethyl-[3-(1H-[1,2,4]triazol-3-yl)-propyl]-amine;
{2-[4-(Benzothiazol-2-yloxy)-phenyl]-ethyl}-cyclopropylmethyl-[3-(5-methyl-1H-[1,2,4]triazol-3-yl)-propyl]-amine;
{2-[4-(Benzothiazol-2-yloxy)-phenyl]-ethyl}-cyclopropylmethyl-[3-(5-phenyl-1 H-[1,2,4]triazol-3-yl)-propyl]-amine;
2-(4-{2-[4-(1-Methyl-1H-tetrazol-5-yl)-piperidin-1-yl]-ethyl}-phenoxy)-benzothiazole;
2-(4-{2-[4-(2-Methyl-2H-tetrazol-5-yl)-piperidin-1-yl]-ethyl}-phenoxy)-benzothiazole;
1-{2-[4-(Benzothiazol-2-yloxy)-phenyl]-ethyl}-piperidine-4-carbonitrile;
2-(4-{2-[4-(1H-[1,2,3]Triazol-4-yl)-piperidin-1-yl]-ethyl}-phenoxy)-benzothiazole;
4-{2-[4-(Benzothiazol-2-yloxy)-phenyl]-ethylamino}-butyric acid ethyl ester;
4-({2-[4-(Benzothiazol-2-yloxy)-phenyl]-ethyl}-cyclopropylmethyl-amino)-butyric acid ethyl ester;
2-[3-({2-[4-(Benzothiazol-2-yloxy)-phenyl]-ethyl}-cyclopropylmethyl-amino)-propyl]-isoindole-1,3-dione;
4-({2-[4-(Benzothiazol-2-yloxy)-phenyl]-ethyl}-cyclopropylmethyl-amino)-butyric acid;
1-(3-{2-[4-(Benzothiazol-2-yloxy)-phenyl]-ethylamino}-propyl)-pyrrolidin-2-one;
N-1-{2-[4-(Benzothiazol-2-yloxy)-phenyl]-ethyl}-N1-cyclopropylmethyl-propane-1,3-diamine;
5.({2-[4-(Benzothiazol-2-yloxy)-phenyl]-ethyl}-cyclopropyl-amino)-pentanoic acid methyl ester;
N-[3-({2-[4-(Benzothiazol-2-yloxy)-phenyl]-ethyl}-cyclopropylmethyl-amino)-propyl]-acetamide;
Morpholine-4-carboxylic acid [3-({2-[4-(benzothiazol-2-yloxy)-phenyl]-ethyl}-cyclopropylmethyl-amino)-propyl]-amide;
N-[3-({2-[4-(Benzothiazol-2-yloxy)-phenyl]-ethyl}-cyclopropylmethyl-amino)-propyl]-methanesulfonamide
5-({2-[4-(Benzothiazol-2-yloxy)-phenyl]-ethyl}-cyclopropyl-amino)-pentanoic acid;
1-[3-({2-[4-(Benzothiazol-2-yloxy)-phenyl]-ethyl}-isopropyl-amino)-propyl]-pyrrolidin-2-one;
1-[3-({2-[4-(Benzothiazol-2-yloxy)-phenyl]-ethyl}-cyclopropylmethyl-amino)-propyl]-pyrrolidin-2-one;
1-[3-({2-[4-(Benzothiazol-2-yloxy)-phenyl]-ethyl}-cyclopropyl-amino)-propyl]-pyrrolidin-2-one;
1-[3-({2-[4-(Benzothiazol-2-yloxy)-phenyl]-ethyl}-propyl-amino)-propyl]-pyrrolidin-2-one;
4-((1-Acetyl-piperidin-4-yl)-{2-[4-(benzothiazol-2-yloxy)-phenyl]-ethyl}-amino)-butyric acid ethyl ester,
4-((1-Acetyl-piperidin-4-yl)-{2-[4-(benzothiazol-2-yloxy)-phenyl]-ethyl}-amino)-butyric acid ethyl ester;
4-({2-[4-(Benzothiazol-2-yloxy)-phenyl]-ethyl}-methanesulfonyl-amino)-butyric acid;
({2-[4-(Benzothiazol-2-yloxy)-phenyl]-ethyl}-cyclopropyl-amino)-acetic acid;
6-({2-[4-(Benzothiazol-2-yloxy)-phenyl]-ethyl}-cyclopropyl-amino)-hexanoic acid ethyl ester;
7-({2-[4-(Benzothiazol-2-yloxy)-phenyl]-ethyl}-cyclopropyl-amino)-heptanoic acid ethyl ester;
6-({2-[4-(Benzothiazol-2-yloxy)-phenyl]-ethyl}-cyclopropyl-amino)-hexanoic acid;
7-({2-[4-(Benzothiazol-2-yloxy)-phenyl]-ethyl}-cyclopropyl-amino)-heptanoic acid;
N-1-{2-[4-(Benzothiazol-2-yloxy)-phenyl]-ethyl}-N1-cyclopropyl-propane-1,3-diamine;
N-[3-({2-[4-(Benzothiazol-2-yloxy)-phenyl]-ethyl}-cyclopropyl-amino)-propyl]-acetamide;
N-[3-({2-[4-(Benzothiazol-2-yloxy)-phenyl]-ethyl}-cyclopropyl-amino)-propyl]-isobutyramide;
N-[3-({2-[4-(Benzothiazol-2-yloxy)-phenyl]-ethyl}-cyclopropyl-amino)-propyl]-benzamide;
N-[3-({2-[4-(Benzothiazol-2-yloxy)-phenyl]-ethyl}-cyclopropyl-amino)-propyl]-4-chloro-benzamide;
N-[3-({2-[4-(Benzothiazol-2-yloxy)-phenyl]-ethyl}-cyclopropyl-amino)-propyl]-methanesulfonamide;
Propane-2-sulfonic acid [3-({2-[4-(benzothiazol-2-yloxy)-phenyl]-ethyl}-cyclopropyl-amino)-propyl]-amide trifluoromethansulfonic acid salt;
8-({2-[4-(Benzothiazol-2-yloxy)-phenyl]-ethyl}-cyclopropyl-amino)-octanoic acid ethyl ester;
1-[3-({2-[4-(Benzothiazol-2-yloxy)-phenyl]-ethyl}-cyclopropyl-amino)-propyl]-3-phenyl-urea;
8-({2-[4-(Benzothiazol-2-yloxy)-phenyl]-ethyl}-cyclopropyl-amino)-octanoic acid;
Tetrahydro-furan-2-carboxylic acid [3-({2-[4-(benzothiazol-2-yloxy)-phenyl]-ethyl}-cyclopropyl-amino)-propyl]-amide;
N-[3-({2-[4-(Benzothiazol-2-yloxy)-phenyl]-ethyl}-cyclopropyl-amino)-propyl]-2-hydroxy-acetamide;
4-({2-[4-(Benzothiazol-2-yloxy)-phenoxy]-ethyl}-cyclopropyl-amino)-butyric acid;
2-[4-(2-Morpholin-4-yl-ethoxy)-phenoxy]-benzothiazole;
2-[4-(2-Piperidin-1-yl-ethoxy)-phenoxy]-benzothiazole.

14. A compound as in claim 1, wherein said compound of formula (II) is one of:
1-{2-[4-(1*H*-Benzoimidazol-2-yloxy)-phenoxy]-ethyl}-4-phenyl-piperidin-4-ol;
{2-[4-(1*H*-Benzoimidazol-2-yloxy)-phenyl]-ethyl}-cyclopropylmethyl-propyl-amine;
Cyclohexyl-ethyl-{2-[4-(1-methyl-1*H*-benzoimidazol-2-yloxy)-phenyl]-ethyl}-amine;
1-{2-[4-(1*H*-Benzoimidazol-2-yloxy)-phenoxy]-ethyl}-4-(4-bromophenyl)-piperidin-4-ol;
1-{2-[4-(1*H*-Benzoimidazol-2-yloxy)-phenoxy]-ethyl}-4-(4-chlorophenyl)-piperidin-4-ol;
1-{2-[4-(1*H*-Benzoimidazol-2-yloxy)-phenoxy]-ethyl}-4-benzyl-piperidin-4-ol;
{2-[4-(1*H*-Benzoimidazol-2-yloxy)-phenyl]-ethyl}-cyclohexyl-ethyl-amine;
2-[4-(2-Pyrrolidin-1-yl-ethyl)-phenoxy]-1*H*-benzoimidazole;
2-[4-(2-Azepan-1-yl-ethyl)-phenoxy]-1*H*-benzoimidazole;
{2-[4-(1*H*-Benzoimidazol-2-yloxy)-phenyl]-ethyl}-dibutyl-amine;
1-{2-[4-(1*H*-Benzoimidazol-2-yloxy)-phenyl]-ethyl}-piperidin-4-ol;
1-{2-[4-(1*H*-Benzoimidazol-2-yloxy)-phenyl]-ethyl}-piperidine-4-carboxylic acid methyl ester;
{2-[4-(1*H*-Benzoimidazol-2-yloxy)-phenoxy]-ethyl}-cyclohexyl-ethyl-amine;
2-{4-[2-(4-Methyl-piperidin-1-yl)-ethoxy]-phenoxy}-1*H-*benzoimidazole;
2-{4-[2-(2-Ethyl-piperidin-1-yl)-ethoxy]-phenoxy}-1*H-*benzoimidazole;
2-[4-(2-Piperidin-1-yl-ethoxy)-phenoxy]-1*H*-benzoimidazole;
1-{2-[4-(1*H*-Benzoimidazol-2-yloxy)-phenoxy]-ethyl}-piperidin-4-ol-;
2-[4-(2-Azepan-1-yl-ethoxy)-phenoxy]-1*H*-benzoimidazole amide;
{3-[4-(1*H*-Benzoimidazol-2-yloxy)-phenoxy]-propyl}-dimethylamine;
2-[4-(2-Pyrrolidin-1-yl-ethoxy)-phenoxy]-1*H*-benzoimidazole;
{2-[4-(1*H*-Benzoimidazol-2-yloxy)-phenoxy]-ethyl}-diethyl-amine;
2-[4-(2-Morpholin-4-yl-ethoxy)-phenoxy]-1*H*-benzoimidazole;
2-[4-(2-Piperidin-1-yl-ethyl)-phenoxy]-1*H*-benzoimidazole;
1-(1-{2-[4-(1*H*-Benzoimidazol-2-yloxy)-phenoxy]-ethyl}-piperidin-4-yl)-pyrrolidin-2-one;
(1-{2-[4-(1H-Benzoimidazol-2-yloxy)-phenoxy]-ethyl}-piperidin-4-yl)-methanol; and
1-{2-[4-(1*H*-Benzoimidazol-2-yloxy)-phenoxy]-ethyl)-piperidine-4-carboxylic acid ethyl ester.

15. A pharmaceutical composition comprising a therapeutically effective amount of at least one compound of formula (I) 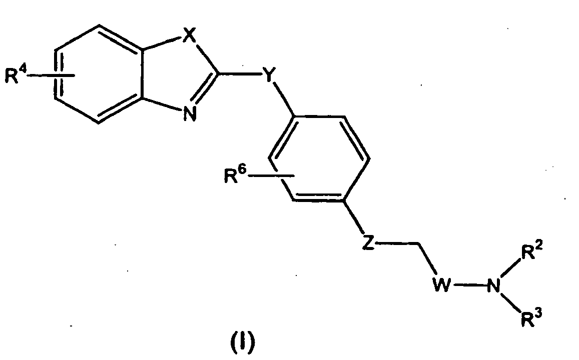 wherein
X is selected from the group consisting of NR⁵, O, and S, with R⁵ being one of
H and CH₃;
Y is selected from the group consisting of CH₂, and O;
Z is selected from the group consisting of O and bond;
W is selected from the group consisting of CH₂ and CHR¹-CH₂, with R¹ being one of H and OH, wherein the R¹-attached carbon member in said CHR¹-CH₂ is not directly attached to the nitrogen member to which said W is attached;
R⁴ is selected from the group consisting of H, OCH₃, Cl, F, Br, I, OH, NH₂, CN, CF₃ and CH₃;
R⁶ is H or F; and
R² and R³ are each independently selected from the group consisting of
A) H, C₁₋₇alkyl, C₃₋₇alkenyl, wherein the carbon in said alkenyl that is attached to the nitrogen member has only single bonds, C₃₋₇alkynyl, wherein the carbon in said alkynyl that is attached to the nitrogen member has only single bonds, C₃₋₇cycloalkyl optionally benzofused, C₅₋₇cycloalkenyl, -C₃₋₇cycloalkylC₁₋₇alkyl, -C₁₋₇alkylC₃₋₇cycloalkyl and phenyl, wherein each of the substituents A) is independently substituted with 0, 1, or 2 R^{Q}, and each of said R^{Q} is a substituent at a carbon member that is at least one carbon member removed from the nitrogen member;
B) a substituent HetR^{a};
C) -C₁₋₇alkylC(O)R^{x}, optionally substituted with CH₂R^{Ar} or CH₂R^{Ar'};
D) -C₂₋₅alkylC(O)R^{x}, wherein two valence allowed carbon members in the C₂₋₅alkyl of said -C₂₋₅alkylC(O)R^{x} are part of a saturated C₃₋₆carbocycle;
E) -C₂₋₅alkylOH wherein two valence allowed carbon members in the C₂₋₅alkyl of said -C₂₋₅alkylOH are part of a saturated C₃₋₆carbocycle;
F) -C₀₋₄alkylphenyl, wherein the phenyl in said -C₀₋₄alkylphenyl is fused at two adjacent carbon members in said phenyl to R^{f}, or is benzofused;
G) -C₀₋₄alkylAr⁶, where Ar⁶ is a 6-membered heteroaryl having a carbon member point of attachment and having one or two -N= heteroatom members, and benzofused;
H) -C₀₋₄alkylAr⁵, where Ar⁵ is a 5-membered heteroaryl, having one heteroatom member selected from the group consisting of O, S, and >NR^{Y}, and having 0 or 1 -N= additional heteroatom member, optionally containing two carbonyl groups, and optionally benzofused;
I) -C₁₋₄alkylAr^{5'}, where Ar^{5'} is a 5-membered heteroaryl containing 3 or 4 nitrogen members, optionally substituted with R^{Y}, and having a valence allowed site as a point of attachment;
J) -C₀₋₄alkylAr⁶⁻⁶, where Ar⁶⁻⁶ is a C₀₋₄alkyl-attached phenyl fused at valence allowed sites to a 6-membered heteroaryl, wherein said 6-membered heteroaryl has one or two -N= heteroatom members;
K) -C₀₋₄alkylAr⁶⁻⁵, where Ar⁶⁻⁵ is a C₀₋₄alkyl-attached phenyl fused at valence allowed sites to a 5-membered heteroaryl, said 5-membered heteroaryl having one heteroatom member selected from the group consisting of O, S, and >NR^{Y}, and said 5-membered heteroaryl having 0 or 1 additional heteroatom member which is -N=; and
L) one of 2-(4-ethyl-phenoxy)-benzothiazole, 2-(4-ethyl-phenoxy)-benzooxazole, and 2-(4-ethyl-phenoxy)-1*H*-benzoimidazole;
M) SO₂C₁₋₄alkyl;
alternatively R² and R³ are taken together with the nitrogen to which they are attached to form a heterocyclic ring that contains at least one heteroatom member that is said attachment nitrogen, said heterocyclic ring being selected from the group consisting of
i) a 4-7 membered heterocyclic ring HetR^{b}, said 4-7 membered heterocyclic ring HetR^{b} having one heteroatom member that is said attachment nitrogen, and being substituted with 0, 1, or 2 substituents at the same or at different substitution members, said substituents being selected from the group consisting of -R^{Y}, -CN, -C(O)R^{Y}, -C₀₋₄alkylCO₂R^{Y}, -C₀₋₄alkylC(O)CO₂R^{Y}, -C₀₋₄alkylOR^{Y}, -C₀₋₄alkylC(O)NR^{Y}R^{Z}, -C₀₋₄alkylNR^{Y}C(O)R^{Z}, - C(O)NR^{Z}OR^{Y}, -C₀₋₄alkylNR^{Y}C(O)CH₂OR^{Y},-C₀₋₄alkylNR^{Y}C(O)CH₂C(O)R^{Y}, -C₀₋₄alkylNR^{Y}CO₂R^{Y}, - C₀₋₄alkylNR^{Y}C(O)NR^{Y}R^{Z}, -C₀₋₄alkylNR^{Y}C(S)NR^{Y}R^{Z}, -NR^{Y}C(O)CO₂R^{Y},-NR^{Y}R^{Z}, -C₀₋₄alkylNR^{W}SO₂R^{Y},1,3-dihydro-indol-2-one-1-yl, 1,3-dihydro-benzoimidazol-2-one-1-yl, tetrazol-5-yl, 1-R^{Y}-1*H*-tetrazol-5-yl, R^{Y}-triazolyl, 2-R^{Y}-2*H-*tetrazol-5-yl, pyrrolidine-2-thion-1-yl, piperidine-2-thion-1-yl, - C₀₋₄alkylC(O)N(R^{Y})(SO₂R^{Y}), -C₀₋₄alkylN(R^{Y})(SO₂)NR^{Y}R^{Y}, -C_{0- 4}alkylN(R^{Y})(SO₂)NR^{Y}CO₂R^{Y}, halo, 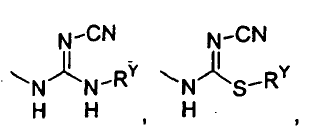 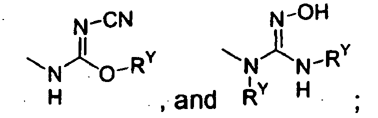
ii) a 5-7 membered heterocyclic ring HetR^{c}, said 5-7 heterocyclic ring HetR^{c} having one additional heteroatom member separated from said attachment nitrogen by at least one carbon members, said additional heteroatom member being selected from the group consisting of O, S(=O)₀₋₂, and >NR^{M}, said 5-7 membered heterocyclic ring HetR^{c} having 0 or 1 carbonyl members, and being substituted with 0, 1, or 2 substituents at the same or at different carbon substitution members, said substituents being selected from the group consisting of -C(O)R^{Y}, -CO₂R^{Y} -C₃₋₄alkylCO₂R^{Y} and R^{Z};
iii) one of imidazolidin-1-yl, 2-imidazolin-1-yl, pyrazol-1-yl, imidazol-1-yl, 2*H*-tetrazol-2-yl, 1*H*-tetrazol-1-yl, pyrrol-1-yl, 2-pyrrolin-1-yl, and 3-pyrrolin-1-yl, wherein each of said 2*H*-tetrazol-2-yl and 1*H*-tetrazol-1-yl is substituted at the carbon member with 0 or 1 of -C₀₋₄alkylR^{Z}, -C₀₋₄alkylSR^{Y}, -C₀₋₄alkylCO₂R^{Y}, and substituent HetR^{a}; and
iv) one of 1,2,3,4-tetrahydro-quinolin-1-yl, 1,2,3,4-tetrahydro-isoquinolin-2-yl, indol-1-yl, isoindol-2-yl, indolin-1-yl, benzimidazol-1-yl, 2,8-diaza-spiro[4.5]decan-1-one-8-yl, 4-{[(2-tert-butoxycarbonylamino-cyclobutanecarbonyl)-amino]-methyl}-piperidin-1-yl, 4-{[(2-amino-cyclobutanecarbonyl)-amino]-methyl}-piperidin-1-yl, 3,9-diaza-spiro[5.5]undecane-3-carboxylic acid-9-yl tert-butyl ester, 4-oxo-1-phenyl-1,3,8-triaza-spiro[4.5]dec-8-yl, and 4-oxo-1,3,8-triaza-spiro[4.5]dec-8-yl;
wherein
substituent HetR^{a} is a 4-7 membered heterocyclic ring having a carbon member point of attachment and containing a member >NR^{M} as a heteroatom member, and said heteroatom member being separated from said carbon member point of attachment by at least 1 additional carbon member;
R^{K} is selected from the group consisting of H, -C₁₋₄alkyl, -C₀₋₄alkylR^{Ar} each optionally substituted with 1, 2, or 3 substituents R^{N}
R^{L} is selected from the group consisting of -CO₂R^{S} and -C(O)NR^{S}R^{S'};
R^{M} is selected from the group consisting of R^{Z}, indol-7-yl, -SO₂R^{Y}, -C₃₋₄alkylCO₂R^{Y}, -CO₂R^{Y}, -C(O)NR^{Z}OR^{Y}, -C(O)R^{Y}, -C(O)C₁₋₄alkylOR^{Y}, - C₀₋₄alkylC(O)NR^{S}R^{S'}, C₀₋₄alkylC(O)CO₂R^{Y}, 1,3-dihydro-indol-2-one-1-yl, 1,3-dihydro-benzoimidazol-2-one-1-yl, tetrazol-5-yl, 1-R^{Y}-1*H*-tetrazol-5-yl,
R^{Y}-triazolyl, 2-R^{Y}-2*H*-tetrazol-5-yl and -C₀₋₄alkylC(O)N(R^{Y})(SO₂R^{Y}), each optionally substituted with 1, 2 or 3 substituents R^{N};
R^{N} is selected from the group consisting of OCH₃, Cl, F, Br, I, OH, NH₂, CN, CF₃, CH₃, OC(O)CH₃, and N0₂;
R^{P} is selected from the group consisting of R^{Y}, -C₂₋₄alkylOR^{Y}, R^{Ar}, -C₁₋₂alkylCO₂R^{Y}, -C₁₋₂alkylCONR^{S}R^{S'}, indol-7-yl, and -SO₂C₁₋₄alkyl;
R^{Q} is selected from the group consisting of fluoro, chloro, bromo, iodo, trifluoromethyl, trichloromethyl, -CN, -C₁₋₄alkyl, -C₀₋₄alkylR^{Ar}, -C₀₋₄alkylR^{Ar'}, -C₀₋₄alkylOR^{Y}, -C₀₋₄alkylCO₂R^{Y}, -C₀₋₄alkylNR^{Y}R^{Z}_{,} -C₀₋₄4alkylNR^{Y}COR^{Y}, -C₀₋₄alkylNR^{Y}CONR^{Y}R^{Z}, -C₀₋₄alkylNR^{Y}SO₂R^{Y}, and -C₀₋₄alkylSR^{Y};
R^{S} and R^{S'} are independently selected from the group consisting of H, -C₁₄alkyl, and -C₀₋₄alkylphenyl; alternatively, R^{S} and R^{S'} are taken together with the nitrogen member to which said R^{S} and R^{S'} are attached to form a 4-7 membered heterocyclic ring having 0 or 1 additional heteroatom member selected from the group consisting of O, S, and >NR^{Y}, provided that said additional heteroatom member is separated by at least two carbon members from said nitrogen member to which said R^{S} and R^{S'} are attached, and provided that where R^{Y} is C₀₋₄alkylR^{Ar}, then R^{Ar} is not substituted with R^{L};
R^{W} is selected from the group consisting of R^{Y}, and -C₃₋₇cycloalkyl;
R^{X} is selected from the group consisting of -OR^{Y}, -NR^{Y}R^{Z}, -C₁₋₄alkyl, and -C₀₋₄alkylR^{Ar};
R^{Y} is selected from the group consisting of H, -C₁₋₄alkyl, -C₀₋₄alkylR^{Ar} and -C₀₋₄alkylR^{Ar'}, each optionally substituted with 1, 2, or 3 substituents R^{N};
R^{Z} is selected from the group consisting of R^{Y}, -C₂₋₄alkylOR^{Y}, -C₁₋₂alkylCO₂R^{Y}, -C₁₋₂alkylC(O)NR^{S}R^{S'}, and -C₂₋₄alkylNR^{S}R^{S'};
when R^{Y} and R^{Z'} are attached to a nitrogen member, R^{Y} and R^{Z} are selected as defined above, or R^{Y} and R^{Z} are taken together with the R^{Y}- and R^{Z}-attached nitrogen member to form a 4-7 membered heterocyclic ring HetR^{d} having 0 or 1 additional heteroatom members selected from the group consisting of O, S, and >NR^{M}, said 4-7 membered heterocyclic ring HetR^{d} having 0 or 1 carbonyl members, and said 4-7 membered heterocyclic ring
HetR^{d} having 0 or 1 valence allowed carbon members substituted with at least one of R^{M}, -CO₂H, and -C₀₋₁alkylOR^{Y};
R^{Ar} is a moiety with a carbon member attachment point and said moiety is selected from the group consisting of phenyl, pyridyl, pyrimidyl, and pyrazinyl, wherein each valence allowed carbon member in each of said moieties is independently substituted with at least one of 0, 1, 2 or 3 R^{N}, and 0 or 1 R^{L};
R^{Ar'} is a 3-8 membered ring, having 0, 1 or 2 heteroatom members selected from the group consisting of O, S, N, and >NR^{Y}, having 0, 1, or 2 unsaturated bonds, having 0 or 1 carbonyl members, wherein each valence allowed member in each of said rings is independently substituted with 0, 1, or 2 R^{K}; and
R^{f} is a linear 3- to 5-membered hydrocarbon moiety having 0 or 1
unsaturated carbon-carbon bonds and having 0 or 1 carbonyl members;
or an enantiomer, diasteromer, racemic, tautomer, hydrate, solvate, or a pharmaceutically acceptable salt, ester, or amide thereof.

16. A pharmaceutical composition comprising at least one compound as claimed in any one of claims 1 - 14.

17. Use of a therapeutically effective amount of at least one LTA4H modulator selected from compounds of any one of claim 1, claim 2 or compounds of formula (I) as claimed in claim 12, in the manufacture of a medicament for the treatment or prevention of an LTA4H-mediated condition in a subject:

18. Use of at least one LTA4H modulator selected from compounds as claimed in any one of claim 1, claim 2 or compounds of formula (I) as claimed in claim 12 in the manufacture of a medicament for the treatment, prevention or inhibition of inflammation in a subject.

19. Use of an inhibitory amount of at least one LTA4H modulator selected from compounds as claimed in any one of claim 1, claim 2 or compounds of formula (I) as claimed in claim 12 in the manufacture of a medicament for the inhibition of LTA4H enzyme activity.

20. A use as claimed in claim 17 wherein said R⁴ is H.

21. A use as claimed in claim 18 wherein said R⁴ is H.

22. A use as claimed in laim 19 wherein said R⁴ is H.

23. A use as claimed in claim 17, wherein said R² and R³ are each independently selected from
(i) the group consisting of A), B), C), D), E), and I) when R² and R³ are selected from different groups; or
(ii) group A); or
(iii) group B); or
(iv) group C); or
(v) group D); or
(vi) group E); or
(vii) group I),
as defined in claim 1, claim 2 or claim 12.

24. A use as claimed in claim 18, wherein said R² and R³ are each independently selected from
(i) the group consisting of A), B), C), D), E), and I) when R² and R³ are selected from different groups; or
(ii) group A); or
(iii) group B); or
(iv) group C); or
(v) group D); or
(vi) group E); or
(vii) group I),
as defined in claim 1, claim 2 or claim 12.

25. A use as claimed in claim 19, wherein said R² and R³ are each independently selected from
(i) the group consisting of A), B), C), D), E), and I) when R² and R³ are selected from different groups; or
(ii) group A); or
(iii) group B); or
(iv) group C); or
(v) group D); or
(vi) group E); or
(vii) group I),
as defined in claim 1, claim 2 or claim 12.

26. A use as claimed in claim 17, wherein said R² and R³ are taken together with the nitrogen to which they are attached to form a heterocyclic ring that contains at least one heteroatom member that is said attachment nitrogen, said heterocyclic ring being selected from any one of
(g) the group consisting of i) and ii) when R² and R³ are selected from different groups;
(h) the group i), and
(i) the group ii)
as defined in claim 1, claim 2 or claim 12.

27. A use as claimed in claim 18, wherein said R² and R³ are taken together with the nitrogen to which they are attached to form a heterocyclic ring that contains at least one heteroatom member that is said attachment nitrogen, said heterocyclic ring being selected from any one of
(j) the group consisting of i) and ii) when R² and R³ are selected from different groups;
(k) the group i), and
(I) the group ii)
as defined in claim 1, claim 2 or claim 12.

28. A use as claimed in claim 19, wherein said R² and R³ are taken together with the nitrogen to which they are attached to form a heterocyclic ring that contains at least one heteroatom member that is said attachment nitrogen, said heterocyclic ring being selected from any one of
(m)the group consisting of i) and ii) when R² and R³ are selected from different groups;
(n) the group i), and
(o) the group ii)
as defined in claim 1, claim 2 or claim 12.

29. A use as claimed in any one of claims 17 - 28, wherein said LTA4H-mediated condition is inflammation due to at least one of asthma, chronic obstructed pulmonary disease, atherosclerosis, rheumatoid arthritis, multiple sclerosis, inflammatory bowel disease, and psoriasis.

30. A use as claimed in any one of claims 17 - 19, wherein said at least one LTA4H modulator is selected from the compounds as claimed in any one of claims 9 - 14.

## Patentansprüche

1. Verbindung nach Formel (II): 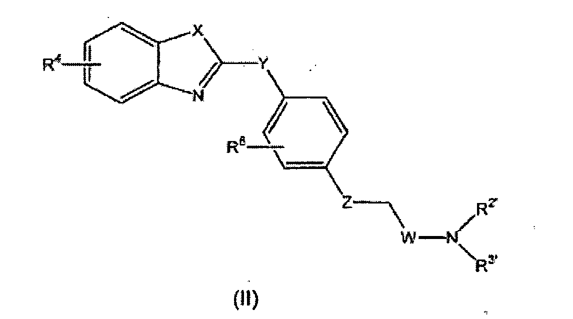 oder ein Enantiomer, Diastereomer, Racemat, Tautomer, Hydrat, Solvat oder ein pharmazeutisch annehmbares Salz, ein Ester oder Amid davon, wobei
X ausgewählt ist aus der Gruppe, bestehend aus NR⁵, O und S, wobei R⁵ einer von H und CH₃ ist;
Y ausgewählt ist aus der Gruppe, bestehend aus CH₂ und O;
Z ausgewählt ist aus der Gruppe, bestehend aus O und einer Bindung;
W ausgewählt ist aus der Gruppe, bestehend aus CH₂ und CHR¹-CH₂, wobei R¹ einer von H und OH ist, wobei das an R¹ angefügte Kohlenstoffglied in dem CHR¹-CH₂ nicht direkt an das Stickstoffglied angefügt ist, an das W angefügt ist;
R⁴ ausgewählt ist aus der Gruppe, bestehend aus H, OCH₃, Cl, F, Br, J, OH, NH₂, CN, CF₃ und CH₃;
R⁶ H oder F ist; und
R^{2'} als R² definiert ist und R^{3'} als R³ definiert ist, wie folgt:
A) H, C₁₋₇Alkyl, C₃₋₇Alkenyl, wobei der Kohlenstoff in dem Alkenyl, das an das Stickstoffglied angefügt ist, nur Einfachbindungen aufweist, C₃₋₇Alkynyl, wobei der Kohlenstoff in dem Alkynyl, das an das Stickstoffglied angefügt ist, nur Einzelbindungen aufweist, C₃₋₇Cycloalkyl, wahlweise fusioniert mit Benzol, C₅₋₇Cycloalkenyl, -C₃₋₇CycloalkylC₁₋₇Alkyl, -C₁₋₇AlkylC₃₋₇Cycloalkyl und Phenyl, wobei jeder der Substituenten A) unabhängig substituiert ist mit 0, 1 oder 2 R^{Q} und jeder von dem R^{Q} ein Substituent an einem Kohlenstoffglied ist, das mindestens ein Kohlenstoffglied ist, das von dem Stickstoffglied entfernt ist,
B) ein Substituent HetR^{a}
C) -C₁₋₇AlkylC(O)R^{X}, wahlweise substituiert mit CH₂R^{Ar} oder CH₂R^{Ar'};
D) -C₂₋₅AlkylC(O)R^{X}, wobei zwei valenzerlaubte Kohlenstoffglieder (*two valence allowed carbon members*) in dem C₂₋₅Alkyl des -C₂₋₅AlkylC(O)R^{X} Teil eines gesättigten C₃₋₆Carbozyklus sind;
E) -C₂₋₅AlkylOH, wobei zwei valenzerlaubte Kohlenstoffglieder in dem C₂₋₅Alkyl des -C₂₋₅AlkylOH Teil eines gesättigten C₃₋₆Carbozyklus sind;
F) -C₀₋₄Alkylphenyl, wobei das Phenyl in dem -C₀₋₄Alkylphenyl an zwei angrenzenden Kohlenstoffgliedern in dem Phenyl mit R^{f} fusioniert ist oder mit Benzol fusioniert ist;
G) -C₀₋₄AlkylAr⁶, wobei Ar⁶ ein 6-gliedriges Heteroaryl ist, das einen Kohlenstoffgliedanfügungspunkt aufweist und ein oder zwei -N=-Heteroatomglieder aufweist und mit Benzol fusioniert ist;
H) -C₀₋₄AlkylAr⁵, wobei Ar⁵ ein 5-gliedriges Heteroaryl ist, das ein Heteroatomglied, ausgewählt aus der Gruppe, bestehend aus O, S und >NR^{Y}, aufweist, und 0 oder 1 zusätzliches -N=-Heteroatomglied aufweist, das wahlweise zwei Carbonylgruppen enthält, und wahlweise mit Benzol fusioniert ist;
I) -C₁₋₄AlkylAr^{5'}, wobei Ar^{5'} ein 5-gliedriges Heteroaryl ist, das 3 oder 4 Stickstoffglieder, wahlweise substituiert mit R^{Y}, enthält und eine valenzerlaubte Stelle als Anfügungspunkt aufweist;
J) -C₀₋₄AlkylAr⁶⁻⁶, wobei Ar⁶⁻⁶ ein an C₀₋₄Alkyl angefügte Phenyl ist, das an valenzerlaubten Stellen mit einem 6-gliedrigen Heteroaryl fusioniert ist, wobei das 6-gliedrige Heteroaryl ein oder zwei -N=-Heteroatomglieder aufweist;
K) -C₀₋₄AlkylAr⁶⁻⁵, wobei Ar⁶⁻⁵ ein an C₀₋₄Alkyl angefügtes Phenyl ist, das an valenzerlaubten Stellen mit einem 5-gliedrigen Heteroaryl fusioniert ist, wobei das 5-gliedrige Heteroaryl ein Heteroatomglied aufweist, ausgewählt aus der Gruppe, bestehend aus O, S und >NR^{Y}, und das 5-gliedrige Heteroaryl 0 oder 1 zusätzliches Heteroatomglied aufweist, das -N= ist; und
L) einer von 2-(4-Ethyl-phenoxy)-benzothiazol, 2-(4-Ethyl-phenoxy)-benzooxazol und 2-(4-Ethyl-phenoxy)-1H-benzoimidazol;
M) SO₂C₁-4Alkyl;
R² und R³ alternativ mit dem Stickstoff, an den sie angefügt sind, zusammengenommen sind, um einen heterozyklischen Ring zu bilden, der mindestens ein Heteroatomglied enthält, welches der Anfügungsstickstoff ist, wobei der heterozyklische Ring ausgewählt ist aus der Gruppe, bestehend aus
i) einem 4- bis 7-gliedrigen heterozyklischen Ring HetR^{b}, wobei der 4- bis 7-gliedrige heterozyklische Ring HetR^{b} ein Heteroatomglied aufweist, das der Anfügungsstickstoff ist, und mit 0, 1 oder 2 Substituenten an denselben oder an verschiedenen Substitutionsgliedern substituiert wird, wobei die Substituenten ausgewählt sind aus der Gruppe, bestehend aus -R^{Y}, -CN, -C(O)R^{Y}, -C₀₋₄AlkylCO₂R^{Y}, -C₀₋₄AlkylC(O)CO₂R^{Y}, -C₀₋₄AlkylOR^{Y}, -C₀₋₄AlkylC(O)-NR^{Y}R^{Z}, -C₀₋₄AlkylNR^{Y}C(O)R^{Z}, -C(O)NR^{Z}OR^{Y}, -C₀₋₄AlkylNR^{Y}C(O)CH₂OR^{Y}, -C₀₋₄AlkylNR^{Y}C(O)CH₂C(O)R^{Y}, -C₀₋₄AlkylNR^{Y}CO₂R^{Y}, - C₀₋₄AlkylNR^{Y}C(O)NR^{Y}R^{Z}, -C₀₋₄AlkylNR^{Y}C(S)NR^{Y}R^{Z}, -NR^{Y}C(O)CO₂R^{Y}, -NR^{Y}R^{Z}, - C₀₋₄AlkylNR^{W}SO₂R^{Y}, 1,3-Dihydroindol-2-on-1-yl, 1,3-Dihydro-benzoimidazol-2-on-1-yl, Tetrazol-5-yl, 1-R^{Y}-1H-Tetrazol-5-yl, R^{Y}-Triazolyl, 2-R^{Y}-2H-tetrazol-5-yl, Pyrrolidin-2-thion-1-yl, Piperidin-2-thion-1-yl, -C₀₋₄AlkylC(O)N(R^{Y})(SO₂R^{Y}), -C₀₋₄AlkylN(R^{Y})(SO₂)NR^{Y}R^{Y}, -C₀₋₄AlkylN(R^{Y})(SO₂)NR^{Y}CO₂R^{Y}, Halo, 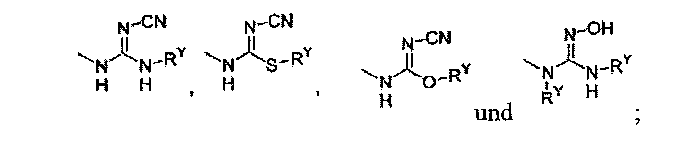
ii) einem 5- bis 7-gliedrigen heterozyklischen Ring HetR^{c}, wobei der 5- bis 7-heterozyklische Ring HetR^{c} ein zusätzliches Heteroatomglied aufweist, das von dem Anfügungsstickstoff durch mindestens ein Kohlenstoffglied getrennt ist, wobei das zusätzliche Heteroatomglied ausgewählt ist aus der Gruppe, bestehend aus O, S(=O)₀₋₂ und >NR^{M}, wobei der 5- bis 7-gliedrige heterozyklische Ring HetR^{c} 0 oder 1 Carbonylglied aufweist, und mit 0, 1 oder 2 Substituenten an denselben oder an verschiedenen Kohlenstoffsubstitutionsgliedern substituiert ist, wobei die Substituenten ausgewählt sind aus der Gruppe, bestehend aus -C(O)R^{Y}, -CO₂R^{Y}, -C₃₋₄Alkyl CO₂R^{Y} und R^{Z};
iii) einem von Imidazolidin-1-yl, 2-Imidazolin-1-yl, Pyrazol-1-yl, Imidazol-1-yl, 2H-tetrazol-2-yl, 1H-tetrazol-1-yl, Pyrrol-1-yl, 2-pyrrolin-1-yl und 3-pyrrolin-1-yl, wobei jeder von dem 2H-tetrazol-2-yl und 1H-tetrazol-1-yl substituiert ist an dem Kohlenstoffglied mit 0 oder 1 von -C₀₋₄-AlkylR^{Z}, -C₀₋₄AlkylSR^{Y}, -C₀₋₄AlkylCO₂R^{Y} und Substituent HetR^{a}; und
iv) einem von 1,2,3,4-Tetrahydro-chinolin-1-yl, 1,2,3,4-Tetrahydro-isochinolin-2-yl, Indol-1-yl, Isoindol-2-yl, Indolin-1-yl, Benzimidazol-1-yl, 2,8-Diaza-spiro[4.5]dekan-1-on-8-yl, 4-{[(2-tert-Butoxycarbonylamino-cyclobutancarbonyl)-amino]methyl}-piperidin-1-yl, 4-{[(2-amino-cyclobutancarbonyl)-amino]methyl}-piperidin-1-yl, 3,9-diaza-spiro[5,5]undecan-3-carboxysäure-9-yl-tert-butylester, 4-Oxo-1-phenyl-1,3,8-triaza-spiro[4.5]dec-8-yl und 4-Oxo-1,3,8-triaza-spiro[4.5]dec-8-yl;
wobei
der Substituent HetR^{a} ein 4- bis 7gliedriger heterozyklischer Ring ist, der einen Kohlenstoffanfügungspunkt aufweist und ein Glied >NR^{M} als ein Heteroatomglied enthält, und das Heteroatomglied vom Kohlenstoffgliedanfügungspunkt durch mindestens ein zusätzliches Kohlenstoffglied getrennt ist;
R^{K} ausgewählt ist aus der Gruppe, bestehend aus H, -C₁₋₄Alkyl, -C₀₋₄AlkylR^{Ar}, jeweils wahlweise substituiert mit 1, 2 oder 3 Substituenten R^{N};
R^{L} ausgewählt aus der Gruppe, bestehend aus -CO₂R^{S} und -C(O)NR^{S}R^{S'};
R^{M} ausgewählt ist aus der Gruppe, bestehend aus R^{Z}, Indol-7-yl, -SO₂R^{Y}, -C₃-₄AlkylCO₂R^{Y}, -CO₂R^{Y}, -C(O)NR^{Z}OR^{Y}, -C(O)R^{Y}, -C(O)C₁₋₄AlkylOR^{Y}, -C₀₋₄AlkylC(O)NR^{S}R^{S'}, C₀₋₄AlkylC(O)CO₂R^{Y}, 1,3-Dihydro-indol-2-on-1-yl, 1,3-Dihydro-benzoimidazol-2-on-1-yl, Tetrazol-5-yl, 1-R^{Y}-1H-tetrazol-5-yl, R^{Y}-Triazolyl, 2-R^{Y}-2H-Tetrazol-5-yl und -C₀₋₄AlkylC(O)N(R^{Y})(SO₂R^{Y}), jeweils wahlweise substituiert mit 1, 2 oder 3 Substituenten R^{N};
R^{N} ausgewählt ist aus der Gruppe, bestehend aus OCH₃, Cl, F, Br, I, OH, NH₂, CN, CF₃, CH₃, OC(O)CH₃ und NO₂;
R^{P} ausgewählt ist aus der Gruppe, bestehend aus R^{Y}, -C₂₋₄AlkylOR^{Y}, R^{Ar}, -C₁₋₂AlkylCO₂R^{Y}, -C₁₋₂AlkylCONR^{S}R^{S'}, Indol-7-yl und -SO₂C₁₋₄Alkyl;
R^{Q} ausgewählt ist aus der Gruppe, bestehend aus Fluor, Chlor, Brom, Jod, Trifluormethyl, Trichlormethyl, -CN, -C₁₋₄Alkyl, C₀₋₄AlkylR^{Ar}, -C₀₋₄AlkylR^{Ar}, -C₀₋₄AlkylOR^{Y}, -C₀₋₄AlkylCO₂R^{Y}, -C₀₋₄AlkylNR^{Y}R^{Z}, -C₀₋₄AlkylNR^{Y}COR^{Y}, -C₀₋₄AlkylNR^{Y}CONR^{Y}R^{Z}, -C₀₋₄AlkylNR^{Y}SO₂R^{Y} und -C₀₋₄AlkylSR^{Y};
R^{S} und R^{S'} unabhängig ausgewählt sind aus der Gruppe, bestehend aus H, -C₁₋₄Alkyl und -C₀₋₄Alkylphenyl; alternativ sind R^{S} und R^{S'} mit dem Stickstoffglied, an das R^{S} und R^{S'} angefügt sind, zusammengenommen, um einen 4- bis 7-gliedrigen heterozyklischen Ring zu bilden, der 0 oder 1 zusätzliches Heteroatomglied, ausgewählt aus der Gruppe, bestehend aus O, S und >NR^{Y}, aufweist, vorausgesetzt, dass das zusätzliche Heteroatomglied durch mindestens zwei Kohlenstoffglieder von dem Stickstoffglied, an das R^{S} und R^{S'} angefügt sind, getrennt ist, und vorausgesetzt, dass, wenn R^{Y} C₀₋₄Alkyl R^{Ar} ist, R^{Ar} nicht mit R^{L} substituiert ist;
R^{W} ausgewählt ist aus der Gruppe, bestehend aus R^{Y} und -C₃₋₇Cycloalkyl;
R^{X} ausgewählt ist aus der Gruppe, bestehend aus OR^{Y}, -NR^{Y}R^{Z}, -C₁₋₄Alkyl und -C₀₋₄AlkylR^{Ar};
R^{Y} ausgewählt ist aus der Gruppe, bestehend aus H, -C₁₋₄Alkyl, -C₀₋₄AlkylR^{Ar} und -C₀₋₄AlkylR^{Ar'}, jeweils wahlweise substituiert mit 1, 2 oder 3 Substituenten R^{N};
R^{Z} ausgewählt ist aus der Gruppe, bestehend aus R^{Y}, -C₂₋₄AlkylOR^{Y}, -C₁₋₂AlkylCO₂R^{Y}, -C₁₋₂AlkylC(O)NR^{S}R^{S'} und -C₂₋₄AlkylNR^{S}R^{S'};
wobei wenn R^{Y} und R^{Z} an ein Stickstoffglied angefügt sind, R^{Y} und R^{Z} ausgewählt sind, wie oben definiert, oder R^{Y} und R^{Z} mit dem an R^{Y} und R^{Z} angefügten Stickstoffglied zusammengenommen sind, um einen 4- bis 7-gliedrigen heterozyklischen Ring HetR^{D} zu bilden, der 0 oder 1 zusätzliches Heteroatomglied, ausgewählt aus der Gruppe, bestehend aus O, S und >NR^{M}, aufweist, wobei der 4- bis 7-gliedrige heterozyklische Ring HetR^{d} 0 oder 1 Carbonylglied aufweist, und der 4- bis 7-gliedrige heterozyklische Ring HetR^{d} 0 oder 1 valenzerlaubtes Kohlenstoffglied, substituiert mit mindestens einem von R^{M}, -CO₂H und -C₀₋₁AlkylOR^{Y}, aufweist;
R^{Ar} ein Rest mit einem Kohlenstoffgliedanfügungspunkt ist und der Rest ausgewählt ist aus der Gruppe, bestehend aus Phenyl, Pyridyl, Pyrimidyl und Pyrazinyl, wobei jedes valenzerlaubte Kohlenstoffglied in jedem der Reste unabhängig mit mindestens einem von 0, 1, 2 oder 3 R^{N} substituiert ist und 0 oder 1 R^{L} ist;
R^{Ar'} ein 3- bis 8-gliedriger Ring ist, der 0, 1 oder 2 Heteroatomglieder aufweist, ausgewählt aus der Gruppe, bestehend aus O, S, N und >NR^{Y}, 0, 1 oder 2 ungesättigte Bindungen aufweist, 0 oder 1 Carbonylglieder aufweist, wobei jedes valenzerlaubte Glied in jedem der Ringe unabhängig substituiert ist mit 0, 1 oder 2 R^{k}; und
R^{f} eine lineare 3- bis 5-gliedrige Kohlenwasserstoffgruppe ist, die 0 oder 1 ungesättigte Kohlenstoff-Kohlenstoff-Bindung aufweist und die 0 oder 1 Carbonylglied aufweist.
vorausgesetzt, dass
(a) mindestens einer von dem R^{2'} und R^{3'} nicht Ethyl ist, wenn eine Auswahl in der Gruppe, bestehend aus den Auswahlen (s1), (s2), (s3) und (s4), erfüllt ist und jede der Auswahlen bestimmt ist als
(s1): R⁴ ist H, Z ist O, W ist CH₂, Y ist CH₂ und X ist S;
(s2): R⁴ ist H, Z ist O, W ist CH₂, Y ist CH₂ und X ist NH;
(s3): R⁴ ist H, Z ist O, W ist CH₂, Y ist O und X ist S;
(s4): R⁴ ist 5-Chloro, Z ist O, W ist CH₂, Y ist CH₂ und X ist S;
(b) weiter vorausgesetzt, dass, wenn Z eine Bindung ist, Y CH₂ ist, W CHR¹-CH₂ ist, R¹ H ist und einer von R^{2'} und R^{3'} 1H-imidazol-2-yl ist, der andere von R^{2'} und R^{3'} dann ausgewählt ist aus A1), B)-L), wobei B)-L) wie oben für die Verbindung nach Formel (I) definiert sind und A1) besteht aus H, C₃₋₇Alkenyl, wobei der Kohlenstoff in dem C₃₋₇Alkenyl, das an das Stickstoffglied angefügt ist, nur Einzelbindungen aufweist, C₃₋₇Alkynyl, wobei der Kohlenstoff in dem Alkynyl, das an das Stickstoffglied angefügt ist, nur Einzelbindungen aufweist, C₃₋₇Cycloalkyl, wahlweise Benzol fusioniert, C₅₋₇Cycloalkenyl, -C₃₋₇CycloalkylC₁₋₇Alkyl, -C₁₋₇AlkylC₃₋₇Cycloalkyl; und
(c) weiter vorausgesetzt, dass, wenn X S ist, Y O ist, Z eine Bindung ist und W CH₂ ist, einer von R^{2'} und R^{3'} dann nicht XCG ist, wenn der andere C₁₋₆Alkyl ist, wobei XCG die folgende Gruppe ist 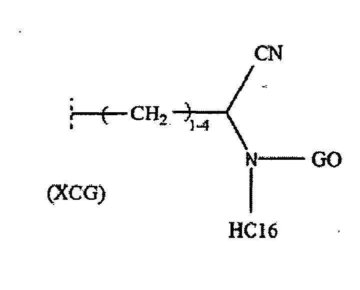
wobei HC16 einer von H, C₁₋₆Alkyl, HaloC₁₋₆Alkyl, Allyl und C₁₋₆Alkoxymethyl ist, und GO eine Gruppe ist, die durch ein Kohlenstoffglied angefügt ist, das einen =O-Substituenten aufweist, der eine Amidogruppe mit dem Stickstoffglied bildet, an das die GO-Gruppe angefügt ist.

2. Verbindung nach Formel (III): 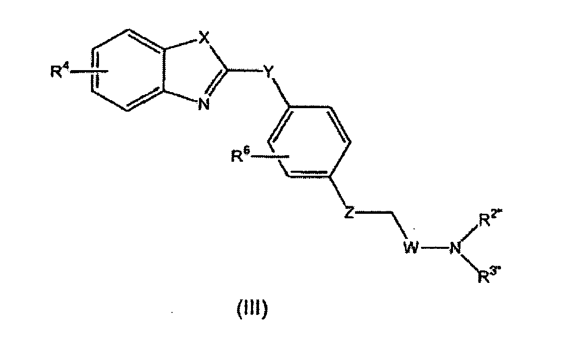 oder ein Enantiomer, Diastereomer, Racemat, Tautomer, Hydrat, Solvat oder ein pharmazeutisch annehmbares Salz, ein Ester oder Amid davon, wobei
X ausgewählt ist aus der Gruppe, bestehend aus NR⁵, O und S, wobei R⁵ einer von H und CH₃ ist;
Y ausgewählt ist aus der Gruppe, bestehend aus CH₂ und O;
Z ausgewählt ist aus der Gruppe, bestehend aus O und einer Bindung;
W ausgewählt ist aus der Gruppe, bestehend aus CH₂ und CHR¹-CH₂, wobei R¹ einer von H und OH ist, wobei das an R¹ angefügte Kohlenstoffglied in dem CHR¹-CH₂ nicht direkt an das Stickstoffglied angefügt ist, an das W angefügt ist;
R⁴ ausgewählt ist aus der Gruppe, bestehend aus H, OCH₃, Cl, F, Br, J, OH, NH₂, CN, CF₃ und CH₃;
R⁶ H oder F ist; und
R^{2"} als R² definiert ist und R^{3"} als R³ definiert ist, wie folgt:
R² und R³ sind jeweils unabhängig ausgewählt sind aus der Gruppe, bestehend aus
A) H, C₁₋₇Alkyl, C₃₋₇Alkenyl, wobei der Kohlenstoff in dem Alkenyl, das an das Stickstoffglied angefügt ist, nur Einfachbindungen aufweist, C₃₋₇Alkynyl, wobei der Kohlenstoff in dem Alkynyl, das an das Stickstoffglied angefügt ist, nur Einzelbindungen aufweist, C₃₋₇Cycloalkyl, fusioniert wahlweise mit Benzol, C₅₋₇Cycloalkenyl, - C₃₋₇CycloalkylC₁₋₇Alkyl, -C₁₋₇AlkylC₃₋₇Cycloalkyl und Phenyl, wobei jeder der Substituenten A) unabhängig substituiert ist mit 0, 1 oder 2 R^{Q} und jeder von dem R^{Q} ein Substituent an einem Kohlenstoffglied ist, das mindestens ein Kohlenstoffglied ist, das von dem Stickstoffglied entfernt ist,
B) ein Substituent HetR^{a}
C) -C₁₋₇AlkylC(O)R^{X}, wahlweise substituiert mit CH₂R^{Ar} oder CH₂R^{Ar'};
D) -C₂₋₅AlkylC(O)R^{X}, wobei zwei valenzerlaubte Kohlenstoffglieder in dem C₂₋₅Alkyl des -C₂₋₅AlkylC(O)R^{X} Teil eines gesättigten C₃₋₆Carbozyklus sind;
E) -C₂₋₅AlkylOH, wobei zwei valenzerlaubte Kohlenstoffglieder in dem C₂₋₅Alkyl des -C₂₋₅AlkylOH Teil eines gesättigten C₃₋₆Carbozyklus sind;
F) -C₀₋₄Alkylphenyl, wobei das Phenyl in dem -C₀₋₄Alkylphenyl an zwei angrenzenden Kohlenstoffgliedern in dem Phenyl mit R^{f} fusioniert ist oder mit Benzol fusioniert ist;
G) -C₀₋₄AlkylAr⁶, wobei Ar⁶ ein 6-gliedriges Heteroaryl ist, das einen Kohlenstoffgliedanfügungspunkt aufweist und ein oder zwei -N=-Heteroatomglieder aufweist und mit Benzol fusioniert ist;
H) -C₀₋₄AlkylAr⁵, wobei Ar⁵ ein 5-gliedriges Heteroaryl ist, das ein Heteroatomglied, ausgewählt aus der Gruppe, bestehend aus O, S und >NR^{Y}, aufweist, und 0 oder 1 zusätzliches -N=-Heteroatomglied aufweist, das wahlweise zwei Carbonylgruppen enthält, und wahlweise mit Benzol fusioniert ist;
I) -C₁₋₄AlkylAr^{5'}-, wobei Ar^{5'} ein 5-gliedriges Heteroaryl ist, das 3 oder 4 Stickstoffglieder, wahlweise substituiert mit R^{Y}, enthält und eine valenzerlaubte Stelle als Anfügungspunkt aufweist;
J) -C₀₋₁AlkylAr⁶⁻⁶, wobei Ar⁶⁻⁶ ein an C₀₋₄Alkyl angefügtes Phenyl ist, das an valenzerlaubten Stellen mit einem 6-gliedrigen Heteroaryl fusioniert ist, wobei das 6-gliedrige Heteroaryl ein oder zwei -N=-Heteroatomglieder aufweist;
K) -C₀₋₄AlkylAr⁶⁻⁵, wobei Ar⁶⁻⁵ ein an C₀₋₄Alkyl angefügtes Phenyl ist, das an valenzerlaubten Stellen mit einem 5-gliedrigen Heteroaryl fusioniert ist, wobei das 5-gliedrige Heteroaryl ein Heteroatomglied aufweist, ausgewählt aus der Gruppe, bestehend aus O, S und >NR^{Y}, und das 5-gliedrige Heteroaryl 0 oder 1 zusätzliches Heteroatomglied aufweist, das -N= ist; und
L) einer von 2-(4-Ethyl-phenoxy)-benzothiazol, 2-(4-Ethyl-phenoxy)-benzooxazol und 2-(4-Ethyl-phenoxy)-1*H*-benzoimidazol;
M) SO₂C₁-4Alkyl;
R² und R³ alternativ mit dem Stickstoff, an den sie angefügt sind, zusammengenommen sind, um einen heterozyklischen Ring zu bilden, der mindestens ein Heteroatomglied enthält, welches der Anfügungsstickstoff ist, wobei der heterozyklische Ring ausgewählt ist aus der Gruppe, bestehend aus
i) einem 4- bis 7-gliedrigen heterozyklischen Ring HetR^{b}, wobei der 4- bis 7-gliedrige heterozyklische Ring HetR^{b} ein Heteroatomglied aufweist, das der Anfügungsstickstoff ist, und mit 0, 1 oder 2 Substituenten an denselben oder an verschiedenen Substitutionsgliedern substituiert ist, wobei die Substituenten ausgewählt sind aus der Gruppe, bestehend aus -R^{Y}, -CN, -C(O)R^{Y}, -C₀₋₄AlkylCO₂R^{Y}, -C₀₋₄AlkylC(O)CO₂R^{Y}, -C₀₋₄AlkylOR^{Y}, -C₀₋₄AlkylC(O)NR^{Y}R^{Z}, -C₀₋₄AlkylNR^{Y}C(O)R^{Z}, -C(O)NR^{Z}OR^{Y}, -C₀₋₄AlkylNR^{Y}C(O)CH₂OR^{Y}, -C₀₋₄AlkylNR^{Y}C(O)CH₂C(O)R^{Y}, -C₀₋₄AlkylNR^{Y}CO₂R^{Y}, - C₀₋₄AlkylNR^{Y}C(O)NR^{Y}R^{Z}, -C₀₋₄AlkylNR^{Y}C(S)NR^{Y}R^{Z}, -NR^{Y}C(O)CO₂R^{Y}, -NR^{Y}R^{Z}, - C₀₋₄AlkylNR^{W}SO₂R^{Y}, 1,3-Dihydroindol-2-on-1-yl, 1,3-Dihydro-benzoimidazol-2-on-1-yl, Tetrazol-5-yl, 1-R^{Y}-1H-Tetrazol-5-yl, R^{Y}-Triazolyl, 2-R^{Y}-2H-tetrazol-5-yl, Pyrrolidin-2-thion-1-yl, Piperidin-2-thion-1-yl, -C₀₋₄AlkylC(O)N(R^{Y})(SO₂R^{Y}), -C₀₋₄AlkylN(R^{Y})(SO₂)NR^{Y}R^{Y}, -C₀₋₄AlkylN(R^{Y})(SO₂)NR^{Y}CO₂R^{Y}, Halo, 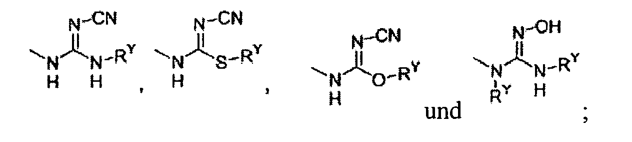
ii) einem 5- bis 7-gliedrigen heterozyklischen Ring HetR^{c}, wobei der 5- bis 7-heterozyklische Ring HetR^{c} ein zusätzliches Heteroatomglied aufweist, das von dem Anfügungsstickstoff durch mindestens ein Kohlenstoffglied getrennt ist, wobei das zusätzliche Heteroatomglied ausgewählt ist aus der Gruppe, bestehend aus O, S(=O)₀₋₂ und >NR^{M}, wobei der 5- bis 7-gliedrige heterozyklische Ring HetR^{c} 0 oder 1 Carbonylglied aufweist, und mit 0, 1 oder 2 Substituenten an denselben oder an verschiedenen Kohlenstoffsubstitutionsgliedern substituiert ist, wobei die Substituenten ausgewählt sind aus der Gruppe, bestehend aus -C(O)R^{Y}, -CO₂R^{Y}, -C₃₋₄Alkyl CO₂R^{Y} und R^{Z};
iii) einem von Imidazolidin-1-yl, 2-Imidazolin-1-yl, Pyrazol-1-yl, Imidazol-1-yl, 2H-tetrazol-2-yl, 1H-tetrazol-1-yl, Pyrrol-1-yl, 2-pyrrolin-1-yl und 3-pyrrolin-1-yl, wobei jeder Rest von dem 2H-tetrazol-2-yl und 1H-tetrazol-1-yl substituiert ist an dem Kohlenstoffglied mit 0 oder 1 von -C₀₋₄-AlkylR^{Z}, -C₀₋₄AlkylSR^{Y}, -C₀₋₄AlkylCO₂R^{Y} und Substituent HetR^{a}; und
iv) einem von 1,2,3,4-Tetrahydro-chinolin-1-yl, 1,2,3,4-Tetrahydro-isochinolin-2-yl, Indol-1-yl, Isoindol-2-yl, Indolin-1-yl, Benzimidazol-1-yl, 2,8-Diaza-spiro[4.5]dekan-1-on-8-yl, 4-{[(2-tert-Butoxycarbonylamino-cyclobutancarbonyl)-amino]methyl}-piperidin-1-yl, 4-{[(2-amino-cyclobutancarbonyl)-amino]methyl}-piperidin-1-yl, 3,9-diaza-spiro[5.5]undecan-3-carboxysäure-9-yl-tert-butylester, 4-Oxo-1-phenyl-1,3,8-triaza-spiro[4.5]dec-8-yl und 4-Oxo-1,3,8-triaza-spiro[4.5]dec-8-yl;
wobei der Substituent HetR^{a} ein 4- bis 7-gliedriger heterozyklischer Ring ist, der einen Kohlenstoffgliedanfügungspunkt aufweist und ein Glied >NR^{M} als ein Heteroatomglied enthält, und das Heteroatomglied vom Kohlenstoffgliedanfügungspunkt durch mindestens ein zusätzliches Kohlenstoffglied getrennt ist;
R^{K} ausgewählt ist aus der Gruppe, bestehend aus H, -C₁₋₄Alkyl, -C₀₋₄AlkylR^{Ar}, jeweils wahlweise substituiert mit 1, 2 oder 3 Substituenten R^{N};
R^{L} ausgewählt aus der Gruppe, bestehend aus -CO₂R^{S} und -C(O)NR^{S}R^{S'};
R^{M} ausgewählt ist aus der Gruppe, bestehend aus R^{Z}, Indol-7-yl, -SO₂R^{Y}, -C₃-₄AlkylCO₂R^{Y}, -CO₂R^{Y}, -C(O)NR^{Z}OR^{Y}, -C(O)R^{Y}, -C(O)C₁₋₄AlkylOR^{Y}, -C₀₋₄AlkylC(O)NR^{S}R^{S'}, -C₀₋₄AlkylC(O)CO₂R^{Y}, 1,3-Dihydroindol-2-on-1-yl, 1,3-Dihydrobezoimidazol-2-on-1-yl, Tetrazol-5-yl, 1-R^{Y}-1*H*-Tetrazol-5-yl, R^{Y}-Triazolyl, 2-R^{Y}-2*H*-Tetrazol-5-yl und -C₀₋₄AlkylC(O)N(R^{Y})(SO₂R^{Y}), jeweils wahlweise substituiert mit 1, 2 oder 3 Substituenten R^{N};
R^{N} ausgewählt ist aus der Gruppe, bestehend aus OCH₃, Cl, F, Br, I, OH, NH₂, CN, CF₃, CH₃, OC(O)CH₃ und NO₂;
R^{P} ausgewählt ist aus der Gruppe, bestehend aus R^{Y}, -C₂₋₄AlkylOR^{Y}, R^{Ar}, -C₁₋₂AlkylCO₂R^{Y}, -C₁₋₂AlkylCONR^{S}R^{S'}, Indol-7-yl und -SO₂C₁₋₄Alkyl;
R^{Q} ausgewählt ist aus der Gruppe, bestehend aus Fluor, Chlor, Brom, Jod, Trifluormethyl, Trichlormethyl, -CN, -C₁₋₄Alkyl, C₀₋₄AlkylR^{Ar}, -C₀₋₄AlkylR^{Ar'}, -C₀₋₄AlkylOR^{Y}, -C₀₋₄AlkylCO₂R^{Y}, -C₀₋₄AlkylNR^{Y}R^{Z}, -C₀₋₄AlkylNR^{Y}COR^{Y}, -C₀₋₄AlkylNR^{Y}CONR^{Y}R^{Z}, -C₀₋₄AlkylNR^{Y}SO₂R^{Y} und -C₀₋₄AlkylSR^{Y};
R^{S} und R^{S'} unabhängig ausgewählt sind aus der Gruppe, bestehend aus H, -C₁₋₄Alkyl und -C₀₋₄Alkylphenyl; alternativ sind R^{S} und R^{S'} mit dem Stickstoffglied, an das R^{S} und R^{S'} angefügt sind, zusammengenommen, um einen 4- bis 7-gliedrigen heterozyklischen Ring zu bilden, der 0 oder 1 zusätzliches Heteroatomglied, ausgewählt aus der Gruppe, bestehend aus O, S und >NR^{Y}, aufweist, vorausgesetzt, dass das zusätzliche Heteroatomglied durch mindestens zwei Kohlenstoffglieder von dem Stickstoffglied, an das R^{S} und R^{S'} angefügt sind, getrennt ist, und vorausgesetzt, dass, wenn R^{Y} C₀₋₄Alkyl R^{Ar} ist, R^{Ar} nicht mit R^{L} substituiert ist;
R^{W} ausgewählt ist aus der Gruppe, bestehend aus R^{Y} und -C₃₋₇Cycloalkyl;
R^{X} ausgewählt ist aus der Gruppe, bestehend aus OR^{Y}, -NR^{Y}R^{Z}, -C₁₋₄Alkyl und -C₀₋₄AlkylR^{Ar};
R^{Y} ausgewählt ist aus der Gruppe, bestehend aus H, -C₁₋₄Alkyl, -C₀₋₄AlkylR^{Ar} und -C₀₋₄AlkylR^{Ar}, jeweils wahlweise substituiert mit 1, 2 oder 3 Substituenten R^{N};
R^{Z} ausgewählt ist aus der Gruppe, bestehend aus R^{Y}, -C₂₋₄AlkylOR^{Y}, -C₁₋₂AlkylCO₂R^{Y}, -C₁₋₂AlkylC(O)NR^{S}R^{S'} und -C₂₋₄AlkylNR^{S}R^{S'};
wobei wenn R^{Y} und R^{Z'} an ein Stickstoffglied angefügt sind, R^{Y} und R^{Z} ausgewählt sind, wie oben definiert, oder R^{Y} und R^{Z} mit dem an R^{Y} und R^{Z} angefügten Stickstoffglied zusammengenommen sind, um einen 4- bis 7-gliedrigen heterozyklischen Ring HetR^{D} zu bilden, der 0 oder 1 zusätzliches Heteroatomglied, ausgewählt aus der Gruppe, bestehend aus O, S und >NR^{M}, aufweist, wobei der 4- bis 7-gliedrige heterozyklische Ring HetR^{d} 0 oder 1 Carbonylglied aufweist, und der 4- bis 7-gliedrige heterozyklische Ring HetR^{d} 0 oder 1 valenzerlaubtes Kohlenstoffglied, substituiert mit mindestens einem von R^{M}, -CO₂H und -C₀₋₁AlkylOR^{Y}, aufweist;
R^{Ar} ein Rest mit einem Kohlenstoffgliedanfügungspunkt ist und der Rest ausgewählt ist aus der Gruppe, bestehend aus Phenyl, Pyridyl, Pyrimidyl und Pyrazinyl, wobei jedes valenzerlaubte Kohlenstoffglied in jedem der Reste unabhängig mit mindestens einem von 0, 1, 2 oder 3 R^{N} substituiert ist, und 0 oder 1 R^{L};
R^{Ar'} ein 3- bis 8-gliedriger Ring ist, der 0, 1 oder 2 Heteroatomglieder aufweist, ausgewählt aus der Gruppe, bestehend aus O, S, N und >NR^{Y}, der 0, 1 oder 2 ungesättigte Bindungen aufweist, der 0 oder 1 Carbonylglieder aufweist, wobei jedes valenzerlaubte Glied in jedem der Ringe unabhängig substituiert ist mit 0, 1 oder 2 R^{K}; und
R^{f} eine lineare 3- bis 5-gliedrige Kohlenwasserstoffgruppe ist, die 0 oder 1 ungesättigte Kohlenstoff-Kohlenstoff-Bindung aufweist und die 0 oder 1 Carbonylglied aufweist.
vorausgesetzt, dass
(a) R^{2"} und R^{3"} weiterhin eine der folgenden Voraussetzungen erfüllen:
(e1): mindestens einer von R²" und R^{3"} ist nicht C₁₋₅Alkyl, wenn Z O ist und X S ist;
(e2): keiner von R^{2"} und R^{3"} ist C₁₋₄AlkylC(O)R^{X}, wobei R^{X} einer von -C₁₋₄Alkyl, OH, -OC₁₋₄Alkyl, -OC₀₋₄AlkylR^{Ar} oder -NR^{Y}R^{Y} ist, wenn Y O ist, Z eine Bindung ist und R^{2"} von R^{3"} verschieden ist; und
(e3): keiner von R^{2"} und R^{3"} -C₁₋₆AlkylCN ist, wenn Y O ist, Z eine Bindung ist und R^{2"} von R^{3"} verschieden ist; und
(b) weiter vorausgesetzt, dass, wenn X S ist, Y O ist, Z eine Bindung ist und W CH₂ ist, dann einer von R² und R^{3"} nicht XCG ist, wenn der andere C₁₋₆Alkyl ist, wobei XCG folgende Gruppe ist. 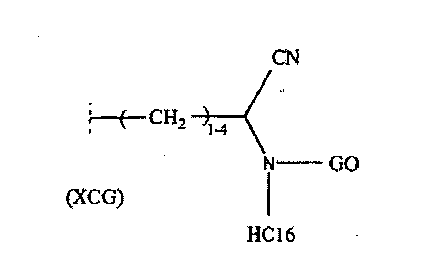
wobei HC16 einer von H, C₁₋₆Alkyl, HaloC₁₋₆Alkyl, Allyl und C₁₋₆Alkoxymethyl ist, und GO eine Gruppe ist, die durch ein Kohlenstoffglied angefügt ist, das einen =0-Substituenten aufweist, der eine Amidogruppe mit dem Stickstoffglied bildet, an das die GO-Gruppe angefügt ist.

3. Verbindung wie in Anspruch 1 beansprucht, wobei R⁴ H ist.

4. Verbindung wie in Anspruch 2 beansprucht, wobei R⁴ H ist.

5. Verbindung wie in Anspruch 1 beansprucht, wobei R² und R³ jeweils unabhängig ausgewählt sind aus
(i) der Gruppe, bestehend aus A), B), C), D), E) und I), wenn R² und R³ aus verschiedenen Gruppen ausgewählt sind; oder
(ii) Gruppe A); oder
(iii) Gruppe B); oder
(iv) Gruppe C); oder
(v) Gruppe D); oder
(vi) Gruppe E); oder
(vii) Gruppe I),
wie in Anspruch 1 definiert.

6. Verbindung wie in Anspruch 2 beansprucht, wobei R² und R³ jeweils unabhängig ausgewählt sind aus
(i) der Gruppe, bestehend aus A), B), C), D), E) und I), wenn R² und R³ aus verschiedenen Gruppen ausgewählt sind; oder
(ii) Gruppe A); oder
(iii) Gruppe B); oder
(iv) Gruppe C); oder
(v) Gruppe D); oder
(vi) Gruppe E); oder
(vii) Gruppe I),
wie in Anspruch 2 definiert.

7. Verbindung wie in Anspruch 1 definiert, wobei R² und R³ mit dem Stickstoff, an den sie angefügt sind, zusammengenommen sind, um einen heterozyklischen Ring zu bilden, der mindestens ein Heteroatomglied enthält, das der angefügte Stickstoff ist, wobei der heterozyklische Ring ausgewählt ist aus einer der folgenden Gruppen:
(a) der Gruppe, bestehend aus (i) und (ii), wenn R² und R³ aus verschiedenen Gruppen ausgewählt sind;
(b) der Gruppe (i), und
(c) der Gruppe (ii),
wie in Anspruch 1 definiert.

8. Verbindung wie in Anspruch 2 beansprucht, wobei R² und R³ mit dem Stickstoff, an den sie angefügt sind, zusammengenommen sind, um einen heterozyklischen Ring zu bilden, der mindestens ein Heteroatomglied enthält, das der Anfügungsstickstoff ist, wobei der heterozyklische Ring ausgewählt ist aus einer der folgenden Gruppen:
(d) der Gruppe, bestehend aus (i) und (ii), wenn R² und R³ aus verschiedenen Gruppen ausgewählt sind;
(e) der Gruppe (i), und
(f) der Gruppe (ii),
wie in Anspruch 2 definiert.

9. Verbindung wie in Anspruch 1 beansprucht, wobei die Verbindung nach Formel (II) eine der folgenden ist:
2-[4-(2-Piperidin-1-yl-ethoxy)-phenoxy]-benzooxazol;
(1-{2-[4-(Benzooxazol-2-yloxy)-phenoxy]-ethyl}-piperidin-4-yl)-methanol;
1-{2-[4-(Benzooxazol-2-yloxy)-phenoxy]-ethyl}-piperidin-4-ol;
{2-[4-(Benzooxazol-2-yloxy)-phenoxy]-ethyl}-dibutyl-amin;
(1-{2-[4-(Benzooxazol-2-yloxy)-phenoxy]-ethyl}-piperidin-2-yl)-methanol;
1-{3-[4-(Benzooxazol-2-yloxy)-phenoxy]-propyl}-4-phenyl-piperidin-4-ol;
1-{3-[4-(Benzooxazol-2-yloxy)-phenoxy]-propyl}-4-benzyl-piperidin-4-ol;
2-[4-(2-Piperidin-1-yl-ethyl)-phenoxy]-benzooxazol;
{3-[4-(Benzooxazol-2-yloxy)-phenyl]-propyl}-cyclohexyl-ethyl-amin;
1-{3-[4-(Benzooxazol-2-yloxy)-phenyl]-propyl}-piperidin-4-ol;
1-{3-[4-(Benzooxazol-2-yloxy)-phenoxy]-2-hydroxy-propyl}-4-phenyl-piperidin-4-ol;
1-[2-(4-Benzooxazol-2-ylmethyl-phenoxy)-ethyl]-piperidin-4-carbonsäureethylester;
2-[4-(2-Pyrrolidin-1-yl-ethoxy)-phenoxy]-benzooxazol;
{3-[4-(Benzooxazol-2-yloxy)-phenoxy]-propyl}-dimethyl-amin;
{2-[4-(Benzooxazol-2-yloxy)-phenoxy]-ethyl}-dimethyl-amin; und
2-[4-(2-Azepan-1-yl-ethoxy)-phenoxy]-benzooxazol.

10. Verbindung nach Anspruch 1, wobei die Verbindung nach Formel (II) eine der folgenden ist:
1-{2-[4-(Benzooxazol-2-yloxy)-phenoxy]-ethyl}-4-phenyl-piperidin-4-ol;
{2-[4-(Benzooxazol-2-yloxy)-phenoxy]-ethyl}-cyclohexyl-ethyl-amin;
2-{4-[2-(2-Ethyl-piperidin-1-yl)-ethoxy]-phenoxy}-benzooxazol;
1-{2-[4-Benzooxazol-2-yloxy-phenoxy]-ethyl}-4-phenyl-piperidin-4-carbonitril;
1-(1-{2-[4-(Benzooxazol-2-yloxy)-phenoxy]-ethyl}-4-phenyl-piperidin-4-yl)-ethanon;
2-{4-[2-(4-Methyl-piperidin-1-yl)-ethoxy]-phenoxy}-benzooxazol;
1-{2-[4-(Benzooxazol-2-yloxy)-phenoxy]-ethyl}-4-(4-chlor-phenyl)-piperidin-4-ol;
1-{2-[4-(Benzooxazol-2-yloxy)-phenoxy]-ethyl}-4-(4-brom-phenyl)-piperidin-4-ol;
1-{2-[4-(Benzooxazol-2-yloxy)-phenoxy]-ethyl}-4-(4-chlor-3-trifluormethyl-phenyl)-piperidin-4-ol;
1-{2-[4-Benzooxazol-2-yloxy)-phenoxy]-ethyl}-4-benzyl-piperidin-4-ol;
{2-[4-Benzooxazol-2-yloxy)-phenoxy]-ethyl}-cyclohexyl-methylamin; und
{2-[4-(Benzooxazol-2-yloxy)-phenoxy]-ethyl}-cyclopropylmethyl-propyl-amin.

11. Verbindung nach Anspruch 1, wobei die Verbindung nach Formel (II) eine der folgenden ist:
{2-[4-(Benzooxazol-2-yloxy)-phenoxy]-ethyl}-butyl-ethyl-amin;
2-({2-[4-(Benzooxazol-2-yloxy)-phenoxy]-ethyl}-benzyl-amino)-ethanol;
2-{4-[2-(4-Benzyl-piperidin-1-yl)-ethoxy]-phenoxy}-benzooxazol;
(1-{2-[4-Benzooxazol-2-yloxy)-phenoxy]-ethyl}-piperidin-3-yl)-methanol;
2-({2-[4-Benzooxazol-2-yloxy)-phenoxy]-ethyl}-propyl-amino)-ethanol;
2-[4-(2-Azetidin-1-yl-ethoxy)-phenoxy]-benzooxazol;
*N*-(1-{2-[4-Benzooxazol-2-yloxy)-phenoxy]-ethyl}-piperidin-4-yl)-2-phenyl-acetamid;
1-{2-[4-Benzooxazol-2-yloxy)-phenoxy]-ethyl}-piperidin-3-carbonsäureethylester;
2-{4-[3-(4-Phenyl-piperidin-1-yl)-propoxy]-phenoxy}-benzooxazol;
1-{2-[4-Benzooxazol-2-yloxy)-phenyl]-ethyl}-4-phenyl-piperidin-4-ol;
{2-[4-(Benzooxazol-2-yloxy)-phenyl]-ethyl}-cyclohexyl-ethyl-amin;
2-[4-(2-Pyrrolidin-1-yl-ethyl)-phenoxy]-benzooxazol; und
2-[4-(2-Azepan-1-yl-ethyl)-phenoxy]-benzooxazol.

12. Verbindung nach Anspruch 1, wobei die Verbindung nach Formel (II) eine der folgenden ist:
{2-[4-(Benzooxazol-2-yloxy)-phenyl]-ethyl}-cyclopropylmethyl-propyl-amin;
2-[4-(Benzooxazol-2-yloxy)-phenyl]-ethyl}-dibutyl-amin;
1-{2-[4-(Benzooxazol-2-yloxy)-phenyl]-ethyl}-piperidin-4-ol;
1-{2-[4-(Benzooxazol-2-yloxy)-phenyl]-ethyl}-piperidin-4-carbonsäuremethylester;
1-{3-[4-(Benzooxazol-2-yloxy)-phenyl]-propyl}-4-phenyl-piperidin-4-ol;
2-[4-(3-Piperidin-1-yl-propyl)-phenoxy]-benzooxazol;
{3-[4-(Benzooxazol-2-yloxy)-phenyl]-propyl}-dibutyl-amin;
{3-[4-(Benzooxazol-2-yloxy)-phenyl]-propyl}-cyclopropylmethyl-propyl-amin;
1-[4-(Benzooxazol-2-yloxy)-phenoxy]-3-pyrrolidin-1-yl-propan-2-ol;
1-[2-(4-Benzooxazol-2-ylmethyl-phenoxy)-ethyl]-4-phenyl-piperidin-4-ol;
1-[2-(4-Benzooxazol-2-ylmethyl-phenoxy)-ethyl]-piperidin-4-carbonsäureamid;
2-[4-(2-Morpholin-4-yl-ethoxy)-phenoxy]-benzooxazole; und
{2-[4-(Benzooxazol-2-yloxy)-phenoxy]-ethyl}-diethyl-amin.

13. Verbindung nach Anspruch 1, wobei die Verbindung nach Formel (II) eine der folgenden ist:
{2-[4-(6-Chlor-benzothiazol-2-yloxy)-phenoxy]-ethyl}-diethyl-amin;
1-{2-[4-(Benzothiazol-2-yloxy)-phenoxy]-ethyl}-piperidin-4-ol;
1-{2-[4-(Benzothiazol-2-yloxy)-phenoxy]-ethyl}-piperidin-4-carbonsäureethylester;
1-{2-[4-(Benzothiazol-2-yloxy)-phenoxy]-ethyl}-piperidin-4-carbonsäure;
(1-{2-[4-(Benzothiazol-2-yloxy)-phenoxy]-ethyl}-piperidin-4-yl)-pyrrolidin-1-yl-methanon;
3-[(1-{2-[4-(Benzothiazol-2-yloxy)-phenoxy]-ethyl}-piperidin-4-carbonyl)-amino]-propionsäureethylester;
1-{2-[4-(Benzothiazol-2-yloxy)-phenoxy]-ethyl}-piperidin-4-carbonsäureamid;
1-(1-{2-[4-(Benzothiazol-2-yloxy)-phenoxy]-ethyl}-piperidin-4-yl)-pyrrolidin-2-on;
1'-{2-[4-(Benzothiazol-2-yloxy)-phenoxyl-ethyl}-[1,4']bipiperidinyl-2-on;
8-{2-[4-(Benzothiazol-2-yloxy)-phenoxy]-ethyl}-2,8-diaza-spiro[4.5]decan-1-on;
2-[4-(3-Pyrrolidin-1-yl-propoxy)-phenoxy]-benzothiazol;
{2-[4-(Benzothiazol-2-yloxy)-phenyl]-ethyl}-cyclohexyl-ethyl-amin;
1-{2-[4-(Benzothiazol-2-yloxy)-phenyl]-ethyl}-piperidin-3-carbonsäureamid;
1-(1-{2-[4-(Benzothiazol-2-yloxy)-phenyl]-ethyl}-piperidin-4-yl)-3-methyl-1,3-dihydrobenzoimidazol-2-on;
1-{2-[4-(Benzothiazol-2-yloxy)-phenyl]-ethyl}-piperdin-4-carbonsäuremethylester;
1-{2-[4-(Benzothiazol-2-yloxy)-phenyl]-ethyl}-piperidin-4-yl)-(4-methyl-piperazin-1-yl)-methanon;
1-[2-(4-(Benzothiazol-2-ylmethyl-phenoxy)-ethyl]-piperidin-4-carbonsäuremethylester;
3-({2-[4-(Benzothiazol-2-yloxy)-phenoxy]-ethyl}-cyclopropyl-amino)-propionsäure;
{2-[4-(Benzothiazol-2-yloxy)-phenoxy]-ethyl}-dimethyl-amin;
2-[4-(2-Pyrrolidin-1-yl-ethoxy)-phenoxy]-benzothiazol;
{3-[4-(Benzothiazol-2-yloxy)-phenoxy]-propyl}-dimethyl-amin;
2-[4-(2-Azepan-1-yl-ethoxy)-phenoxy]-benzothiazol;
2-[4-(2-Azepan-1-yl-ethoxy)-phenoxy]-6-methoxy-benzothiazol;
1-{2-[4-(Benzothiazol-2-yloxy)-phenoxy]-ethyl}-4-phenyl-piperidin-4-ol;
{2-[4-(Benzothiazol-2-yloxy)-phenoxy]-ethyl}-cyclohexyl-ethyl-amin;
1-{2-[4-(Benzothiazol-2-yloxy)-phenoxy]-ethyl}-4-(4-chlorphenyl)-piperidin-4-ol;
2-[4-(Benzothiazol-2-yloxy)-phenoxy]-ethyl}-dibutyl-amin;
1-{2-[4-(Benzothiazol-2-yloxy)-phenoxy]-ethyl}-4-(4-brom-phenyl)-piperidin-4-ol;
1-{2-[4-(Benzothiazol-2-yloxy)-phenoxy]-ethyl}-4-(4-chlor-3-trifluormethyl-phenyl)-piperidin-4-ol;
1-{2-[4-(Benzothiazol-2-yloxy)-phenoxy]-ethyl}-4-benzyl-piperidin-4-ol;
1'-{2-[4-(Benzothiazol-2-yloxy)-phenoxy]-ethyl}-[1,4']bipiperidin;
(1-{2-[4-(Benzothiazol-2-yloxy)-phenoxy]-ethyl}-piperidin-4-yl)-methanol;
*N*-(1-{2-[4-(Benzothiazol-2-yloxy)-phenoxy]-ethyl}-piperidin-4-yl)-2-phenyl-acetamid;
2-(4-{2-[4-(2-Morpholin-4-yl-ethyl)-piperazin-1-yl]-ethoxy}-phenoxy)-benzothiazol;
2-(4-{2-[4-(2-Morpholin-4-yl-ethyl)-piperazin-1-yl]-ethyl}-phenoxy)-benzothiazol;
1-{3-[4-(Benzothiazol-2-yloxy)-phenoxy]-propyl}-4-phenyl-piperidin-4-ol;
(1-{2-[4-(Benzothiazol-2-yloxy)-phenyl]-ethyl}-4-phenyl-piperidin-4-ol);
2-[4-(2-Pyrrolidin-1-yl-ethyl)-phenoxy]-benzothiazol;
2-[4-(2-Azepan-1-yl-ethyl)-phenoxy]-benzothiazol;
{2-[4-(Benzothiazol-2-yloxy)-phenyl]-ethyl}-cyclopropylmethyl-propyl-amin;
{2-[4-(Benzothiazol-2-yloxy)-phenyl]-ethyl}-dibutyl-amin;
2-[4-(2-Piperidin-1-yl-ethyl)-phenoxy]-benzothiazol;
1-{2-[4-(Benzothiazol-2-yloxy)-phenyl]-ethyl}-piperidin-4-ol;
1-{2-[4-(Benzothiazol-2-yloxy)-phenyl]-ethyl}-piperidin-4-carbonsäureamid;
1-{2-[4-(Benzothiazol-2-yloxy)-phenyl]-ethyl}-piperdin-3-carbonsäureethylester;
1-{2-[4-(Benzothiazol-2-yloxy)-phenyl]-ethyl}-4-phenyl-piperidin-4-carbonsäureethylester;
(1-{2-[4-(Benzothiazol-2-yloxy)-phenyl)-ethyl]-piperidin-4-yl)-essigsäureethylester;
1-(1-{2-[4-(Benzothiazol-2-yloxy)-phenyl]-ethyl}-piperidin-4-yl)-1,3-dihydro-indol-2-on;
1-(1-{2-[4-(Benzothiazol-2-yloxy)-phenyl]-ethyl}-piperidin-4-yl)-pyrrolidin-2-on;
*N*-(1-{2-[4-(Benzothiazol-2-yloxy)-phenyl]-ethyl}-piperidin-4-yl)-2-phenyl-acetamid;
8-{2-[4-Benzothiazol-2-yloxy)-phenyl]-ethyl}-2,8-diaza-spiro[4.5]decan-1-on;
1-{2-[4-Benzothiazol-2-yloxy)-phenyl]-ethyl}-piperidin-3-ol;
1-{2-[4-(Benzothiazol-2-yloxy)-phenyl]-ethyl}-piperidin-4-carbonsäureethylester;
1'-{2-[4-(Benzothiazol-2-yloxy)-phenyl]-ethyl}-[1,4']bipiperidin;
2-{4-[2-(4-Methyl-piperazin-1-yl)-ethyl-phenoxy-benzothiazol;
2-(4-{2-[4-(1-Benzyl-1*H*-tetrazol-5-yl)-piperidin-1-yl]-ethoxy}-phenoxy)-benzothiazol;
4-(1-{2-[4-(Benzothiazol-2-yloxy)-phenyl]-ethyl}-piperidin-4-carbonyl)-piperazin-1-carbonsäure-*tert*-butylester;
1-[2-(4-Benzothiazol-2-ylmethyl-phenoxy)-ethyl]-piperidin-4-carbonsäureamid;
1-{1-[2-(4-Benzothiazol-2-ylmethyl-phenoxy)-ethyl]-piperidin-4-yl}-pyrrolidin-2-on;
1-[4-(1-{2-[4-Benzothiazol-2-yloxy)-phenyl]-ethyl}-piperidin-4-carbonyl)-piperazin-1-yl-ethanon;
1-{2-[4-Benzothiazol-2-yloxy)-phenyl]-ethyl}-piperidin-4-carbonsäure;
1-(1-{2-[4-(Benzothiazol-2-yloxy)-phenoxy]-ethyl}-piperidin-4-yl)-pyrrolidin-2-thion;
2-(4-{2-[4-(Benzothiazol-2-yloxy)-phenoxy]-ethyl}-piperazin-1 -yl)-ethanol;
2-(4-{2-[4-(Benzothiazol-2-yloxy)-phenoxy]-ethyl}-piperazin-1-yl)-1-pyrrolidin-1-yl-ethanon;
2-(4-{2-[4-(Benzothiazol-2-yloxy)-phenoxy]-ethyl}-piperazin-1-yl)-1-morpholin-4-yl-ethanon;
1-{2-[4-(Benzothiazol-2-yloxy)-phenyl]-ethyl}-piperidin-3-carbonsäure;
1-{2-[4-(Benzothiazol-2-yloxy)-phenyl]-ethyl}-piperidin-2-carbonsäure;
(1-{2-[4-Benzothiazol-2-yloxy)-phenyl]-ethyl}-piperidin-3-yl)-essigsäure;
(1-{2-[4-(Benzothiazol-2-yloxy)-phenoxy)-ethyl}-piperidin-4-yl)-essigsäureethylester;
(1-{2-[4-(Benzothiazol-2-yloxy)-phenoxy]-ethyl}-piperidin-4-yl)-carbamidsäure-*tert-*butylester;
(1-{2-[4-(Benzothiazol-2-yloxy)-phenoxy]-ethyl}-piperidin-4-yl)-essigsäure;
(1-{2-[4-(Benzothiazol-2-yloxy)-phenyl]-ethyl}-piperidin-3-yl)-methanol;
({2-[4-Benzothiazol-2-yloxy)-phenyl]-ethyl}-cyclohexyl-amino)-essigsäuremethylester;
(4-{2-[4-Benzothiazol-2-yloxy)-phenoxy]-ethyl}-piperazin-1-yl)-essigsäure;
1-(1-{2-[4-(Benzothiazol-2-yloxy)-phenoxy]-ethyl}-piperidin-4-yl)-5-oxo-pyrrolidin-2-carbonsäureethylester;
1-(1-{2-[4-(Benzothiazol-2-yloxy)-phenoxy]-ethyl}-piperidin-4-yl)-5-oxo-pyrrolidin-2-carbonsäure;
4-(4-{2-[4-(Benzothiazol-2-yloxy)-phenoxy-ethyl}-piperazin-1-yl)-phenol;
*N*-(1-{2-[4-(Benzothiazol-2-yloxy)-phenoxy]-ethyl}-piperidin-4-yl)-4-chlor-N-cyclopropyl-benzolsulfonamid;
3-({2-[4-(Benzothiazol-2-yloxy)-phenoxy]-ethyl}-cyclopropylmethylamino)-propionsäure;
3-({2-[4-Benzothiazol-2-yloxy)-phenoxy]-ethyl}-isopropyl-amino)-propionsäure;
1-{2-[4-(Benzothiazol-2-yloxy)-phenoxy]-ethyl}-piperidin-4-ylamin;
3-[{2-[4-(Benzothiazol-2-yloxy)-phenoxy]-ethyl}-(1-methyl-piperidin-4-yl)-amino-propionsäure;
3-({2-[4-(Benzothiazol-2-yloxy)-phenoxy]-ethyl}-benzyl-amino)-propionsäure;
3-((1-Acetyl-piperidin-4-yl)-{2-[4-(benzothiazol-2-yloxy)-phenoxy]-ethyl -amino)-propionsäure;
4-(1-{2-[4-(Benzothiazol-2-yloxy)-phenoxy]-ethyl}-1H-tetrazol-5-yl)-piperidin-1-carbonsäure-*tert*-butylester;
3-({2-[4-(Benzothiazol-2-yloxy)-phenyl]-ethyl}-cyclopropyl-amino)-propionsäure;
3-((1-Acetyl-piperidin-4-yl)-{2-[4-(benzothiazol-2-yloxy)-phenyl]-ethyl}-amino)-propionsäure;
3-[{2-[4-Benzothiazol-2-yloxy)-phenyl]-ethyl}-(1-methyl-piperidin-4-yl)-amino]-propionsäure;
3-[{2-[4-Benzothiazol-2-yloxy)-phenyl]-ethyl}-cyclopropylmethyl-amino)-propionsäure;
3-({2-[4-Benzothiazol-2-yloxy)-phenyl]-ethyl}-isopropyl-amino)-propionsäure;
2-(4-{2-[4-Benzothiazol-2-yloxy)-phenyl]-ethyl}-piperazin-1-yl)-1-pyrrolidin-1-yl-ethanon;
(*R*)-1-{2-[4-(Benzothiazol)-2-yloxy)-phenyl]-ethyl}-piperidin-3-carbonsäure;
1-(1-{2-[4-(Benzothiazol-2-yloxy)-phenyl]-ethyl}-piperidin-4-yl)-1,3-dihydro-benzoimidazol-2-on;
2-(4-{2-[4-(6-Methyl-pyridin-2-yl)-piperiazin-1-yl]-ethyl}-phenoxy)-benzothiazol;
2-{4-[2-(4-Ethansulfonyl-piperazin-1-yl)-ethyl]-phenoxy}-benzothiazol;
2-(4-{2-[4-(Benzothiazol-2-yloxy)-phenyl]-ethyl}-piperazin-1-yl)-1-morpholin-4-yl-ethanon;
3-({2-[4-(Benzothiazol-2-yloxy)-phenyl]-ethyl}-methyl-amino)-propionsäure;
3-({2-[4-(Benzothiazol-2-yloxy)-phenyl]-ethyl}-cyclopentyl-amino)-propionsäure;
3-({2-[4-(Benzothiazol-2-yloxy)-phenyl]-ethyl}-cyclobutyl-amino)-propionsäure;
3-({2-[4-(Benzothiazol-2-yloxy)-phenyl]-ethyl}-benzyl-amino)-propionsäure;
(1-{2-[4-(Benzothiazol-2-yloxy)-phenoxy]-ethyl}-piperidin-4-yl)-(4-hydroxymethyl-piperidin-1-yl)-methanon;
{2-[4-Benzothianol-2-yloxy)-phenyl]-ethyl}-cylclopropyl-amin;
(1-{2-[4-(Benzothiazol-2-yloxy)-phenoxy]-ethyl}-piperidin-4-yl)-[4-(2-hydroxyethyl)-piperazin-1-yl]-methanon;
(1-{2-[4-(Benzothiazol-2-yloxy)-phenoxy]-ethyl}-piperidin-4-yl)-[4-(2-hydroxyethyl)-piperidin-1-yl]-methanon;
2-({2-[4-(Benzothiazol-2-yloxy)-phenyl]-ethyl}-cyclopropyl-amino)-ethanol;
3-({2-[4-(Benzothiazol-2-yloxy)-phenyl]-ethyl}-cyclopropyl-amino)-propan-1-ol;
4-({2-[4-Benzothiazol-2-yloxy)-phenyl]-ethyl}-cyclopropyl-amino)-buttersäure;
3-[(1-{2-[4-(Benzothiazol-2-yloxy-phenoxy]-ethyl-piperidin-4-carbonyl)-amino-propionsäure;
4-({2-[4-(Benzothiazol-2-yloxy)-phenyl]-ethyl}-cyclopropyl-amino)-butyronitril;
3-(1-{2-[4-(Benzothiazol-2-yloxy)-phenyl]-ethyl}-piperidin-4-yl)-propionsäure;
[(1-{2-[4-(Benzothiazol-2-yloxy)-phenoxy]-ethyl}-piperidin-4-carbonyl)-methylamino]-essigsäure;
3-(4-{2-[4-(Benzothiazol-2-yloxy)-phenoxy]-ethyl}-piperazin-1-yl)-phenol;
2-(4-{2-[4-(4-Methoxy-phenyl)-piperazin-1-yl]-ethoxy}-phenoxy)-benzothiazol;
2-{4-[2-(5-Piperidin-4-yl-tetrazol-1-yl)-ethoxy]-phenoxy}-benzothiazol;
(*S*)-1-(1-{2-[4-(Benzothiazol-2-yloxy)-phenoxy-ethyl-piperidin-4-yl)-4-hydroxy-pyrrolidin-2-on;
2-[4-(Benzothiazol-2-yloxy)-phenyl]-ethyl}-cyclopropylmethyl-amin;
2-[({2-[4-(Benzothiazol-2-yloxy)-phenyl]-ethyl}-cyclopropyl-amino-methyl-cyclopropan-carbonsäureethylester;
4-(4-{2-[4-(Benzothiazol-2-yloxy)-phenoxy]-ethyl}-piperazin-1-carbonyl)-benzoesäureethylester;
2-[({2-[4-(Benzothiazol-2-yloxy)-phenyl]-ethyl}-cyclopropyl-amino)-methyl-cyclopropancarbonsäure;
1-({2-[4-(Benzothiazol-2-yloxy)-phenyl]-ethyl}-cyclopropyl-amino)-propan-2-ol;
3-({2-[4-(Benzothiazol-2-yloxy)-phenyl]-ethyl}-cyclopropyl-amino)-1,1,1-trifluor-propan-2-ol;
3-({2-[4-(Benzothiazol-2-yloxy)-phenyl]-ethyl}-cyclopropyl-amino)-propionamid;
3-({2-[4-(Benzothiazol-2-yloxy)-phenyl]-ethyl}-cyclopropyl-amino)-propan-1,2-diol;
2-{4-[2-(5-Phenyl-tetrazol-2-yl)-ethoxy]-phenoxy}-benzothiazol;
2-{4-[2-(5-Phenyl-tetrazol-1-yl)-ethoxy]-phenoxy}-benzothiazol;
*N*-{2-[4-(Benzothiazol-2-yloxy)-phenyl]-ethyl)-*N*-cyclopropyl-2-(2H-tetrazol-5-yl)-acetamid;
(*S*)-3-({2-[4-(Benzothiazol-2-yloxy)-phenyl-ethyl}-cyclopropyl-amino)-2-methyl-propan-1-ol;
(*R*)-3-({2-[4-(Benzothiazol-2-yloxy)-phenyl]-ethyl}-cyclopropyl-amino)-2-methyl-propan-1-ol;
2-{4-[2-(5-Methylsulfanyl-tetrazol-2-yl)-ethoxy]-phenoxy}-benzothiazol;
2-{4-[2-(5-Methylsulfanyl-tetrazol-1-yl)-ethoxy]-phenoxy}-benzothiazol;
2-[4-(2-Tetrazol-2-yl-ethoxy)-phenoxy]-benzothiazol;
2-[4-(2-Tetrazol-1-yl-ethoxy)-phenoxy]-benzothiazol;
(1*R*,2*R*)-2-{2-[4-(Benzothiazol-2-yloxy)-phenyl]-ethylamino-cyclohexancarbonsäure;
(1*S*,2*R*)-2-{2-[4-(Benzothiazol-2-yloxy)-phenyl]-ethylamino}-cyclohexancarbonsäure;
(1*R*,2*R*)-2-{2-[4-(Benzothiazol-2-yloxy)-phenyl]-ethylamino}-cyclohexanol;
(1*S*,2*R*)-2-{2-[4-(Benzothiazol-2-yloxy)-phenyl]-ethylamino}-cyclohexanol;
4-(1-{2-[4-(Benzothiazol-2-yloxy)-phenyl]-ethyl}-piperidin-4-yl)-buttersäure;
(*R*)-1-(1-{2-[4-(Benzothiazol-2-yloxy)-phenoxy]-ethyl}-piperidin-4-yl)-4-hydroxy-pyrrolidin-2-on;
2-(4-{2-[4-(1H-Tetrazol-5-yl)-piperidin-1-yl]-ethyl}-phenoxy)-benzothiazol;
(1-{2-[4-(Benzothiazol-2-yloxy)-phenoxy]-ethyl}-piperidin-2-yl)-methanol;
(1-{2-[4-(Benzothiazol-2-yloxy)-phenoxy]-ethyl}-1*H*-tetrazol-5-yl)-essigsäureethylester;
(1-{2-[4-(Benzothiazol-2-yloxy)-phenoxy]-ethyl}-1*H*-tetrazol-5-yl)-essigsäureethylester;
2-{4-[2-(5-Piperidin-4-yl-tetrazol-2-yl)-ethoxy]-phenoxy}-benzothiazol-hydrochlorid;
7-{2-[4-(Benzothiazol-2-yloxy)-phenoxy]-ethyl}-4-spiro-[3-phthalid]-piperidin;
1-{3-[4-(Benzothiazol-2-yloxy)-phenyl]-propyl}-piperidin-4-carbonsäureethylester;
2-[4-(Benzothiazol-2-yloxy)-phenyl]-ethylamin-hydrochlorid;
2-(4-{2-[4-(1H-Tetrazol-5-yl)-piperidin-1-yl]-ethoxy}-phenoxy)-benzothiazol;
Cis-4-{2-[4-(Benzothiazol-2-yloxy)-phenyl]-ethylamino}-cyclohexancarbonsäure-trifluormethansulfonatsalz;
(4-{2-[4-(Benzothiazol-2-yloxy)-phenyl]-ethyl}-piperazin-1-yl)-(tetrahydro-furan-2-yl)-methanon;
Propan-2-sulfonsäure (1-{2-[4-(Benzothiazol-2-yloxy)-phenoxy]-ethyl}-piperidin-4-carbonyl)-amid;
(4-{2-[4-(Benzothiazol-2-yloxy)-phenyl]-ethyl}-piperazin-1-yl)-oxo-essigsäure-methylester;
N-(1-{2-[4-(Benzothiazol-2-yloxy)-phenyl]-ethyl}-piperidin-4-carbonyl)-benzolsulfonamid-trifluormethansulfonatsalz;
N-(1-{2-[4-(Benzothiazol-2-yloxy)-phenyl]-ethyl}-piperidin-4-carbonyl)-methansulfonamid-trifluormethansulfonatsalz;
(4-{2-[4-(Benzothiazol-2-yloxy)-phenyl-ethyl-piperazin-1-yl)-oxo-essigsäure-trifluormethansulfonatsalz;
(4-{2-[4-(Benzothiazol-2-yloxy)-phenyl]-ethyl}-piperazin-1-yl)-morpholin-4-yl-methanon;
1-(4-{2-[4-(Benzothiazol-2-yloxy)-phenyl]-ethyl}-piperazin-1-yl)-2-thiophen-2-yl-ethanon;
(4-{2-[4-(Benzothiazol-2-yloxy)-phenyl]-ethyl}-piperazin-1-yl)-pyridin-3-yl-methanon;
4-{2-[4-(Benzothiazol-2-yloxy)-phenyl]-ethyl}-piperazin-1-yl)-cyclopropyl-methanon;
1-(4-{2-[4-(Benzothiazol-2-yloxy)-phenyl]-ethyl}-piperazin-1-yl)-2-methoxy-ethanon;
1-(4-{2-[4-(Benzothiazol-2-yloxy)-phenyl]-ethyl}-piperazin-1-yl)-2,2,2-trifluor-ethanon;
4-(4-{2-[4-(Benzothiazol-2-yloxy)-phenyl]-ethyl}-piperazin-1-carbonyl)-benzoesäure;
(4-{2-[4-(Benzothiazol-2-yloxy)-phenyl]-ethyl}-piperazin-1-yl)-pyridin-4-yl-methanon;
(4-{2-[4-(Benzothiazol-2-yloxy)-phenyl]-ethyl}-piperazin-1-yl)-(5-methyl-pyrazin-2-yl)-methanon;
(*R*)-(4-{2-[4-(Benzothiazol-2-yloxy)-phenyl]-ethyl}-piperazin-1-yl)-(tetrahydro-furan-2-yl)-methanon;
(*S*)-(4-{2-[4-(Benzothiazol-2-yloxy)-phenyl]-ethyl}-piperazin-1-yl)-(tetrahydro-furan-2-yl)-methanon;
(4-{2-[4-(Benzothiazol-2-yloxy)-phenyl]-ethyl}-piperazin-1-yl)-(tetrahydro-furan-3-yl)-methanon;
1-(4-{2-[4-(Benzothiazol-2-yloxy)-phenyl]-ethyl}-piperazin-1-yl)-2-hydroxy-ethanon;
2-[2-(4-{2-[4-(Benzothiazol-2-yloxy)-phenyl]-ethyl}-piperazin-1-yl)-2-oxo-ethyl]-cyclopentanon;
3-(4-{2-[4-(Benzothiazol-2-yloxy)-phenyl]-ethyl}-piperazin-1-yl)-propionsäure-trifluormethansulfonatsalz;
3-(1-{2-[4-(Benzothiazol-2-yloxy)-phenyl]-ethyl}-piperidin-4-yl)-oxazolidin-2-on;
4-(1-{2-[4-(Benzothiazol-2-yloxy)-phenyl]-ethyl}-piperidin-4-yl)-morpholin-3-on;
4-(1-{2-[4-(Benzothiazol-2-yloxy)-phenoxy]-ethyl}-piperidin-4-yl)-morpholin-3-on;
3-(1-{2-[4-(Benzothiazol-2-yloxy)-phenoxy]-ethyl}-piperidin-4-yl)-oxazolidin-2-on;
1-{2-[4-(Benzothiazol-2-yloxy)-phenyl]-ethyl}-piperidin-4-carbonsäurebenzyloxy-amid;
(1-{2-[4-(Benzothiazol-2-yloxy)-phenyl]-ethyl}-piperidin-4-yl)-essigsäure;
(*R*)-1-(1-{2-[4-(Benzothiazol-2-yloxy)-phenyl]-ethyl}-piperidin-4-yl)-4-hydroxy-pyrrolidin-2-on;
1-{2-[4-(Benzothiazol-2-yloxy)-phenyl]-ethyl}-piperidin-4-carbonsäure-hydroxyamid;
(*S*)-1-(1-{2-[4-(Benzothiazol-2-yloxy)-phenyl]-ethyl}-piperidin-4-yl)-4-hydroxy-pyrrolidin-2-on;
(1-{2-[4-(Benzothiazol-2-yloxy)-phenyl]-ethyl}-piperidin-4-yl)-carbamidsäure-tert-butylester;
2-{4-[2-(4-Fluor-piperidin-1-yl)-ethyl]-phenoxy}-benzothiazol;
2-{4-[2-(4,4-Difluor-piperidin-1-yl)-ethyl]-phenoxy}-benzothiazol;
(*R*)-1-{2-[4-(Benzothiazol-2-yloxy)-phenyl]-ethyl}-pyrrolidin-3-ol;
N-(1-{2-[4-(Benzothiazol-2-yloxy)-phenyl]-ethyl}-piperidin-4-yl)-formamid;
(1-{2-[4-(Benzothiazol-2-yloxy)-phenyl]-ethyl}-piperidin-4-yl)-urea;
1-(1-{2-[4-(Benzothiazol-2-yloxy)-phenyl]-ethyl}-piperidin-4-yl)-3-cyano-2-phenyl-isourea;
1-(1-{2-[4-(Benzothiazol-2-yloxy)-phenyl]-ethyl}-piperidin-4-yl)-3-cyano-2-methyl-isothiourea;
N-(1-{2-[4-(Benzothiazol-2-yloxy)-phenyl]-ethyl}-piperidin-4-yl)-methansulfonamid;
1-(1-{2-[4-(Benzothiazol-2-yloxy)-phenyl]-ethyl}-piperidin-4-yl)-3-cyano-2-methyl-guanidin;
8-{2-[4-(Benzothiazol-2-yloxy)-phenyl]-ethyl}-1-phenyl-1,3,8-triaza-spiro[4.5]decan-4-on;
8-{2-[4-(Benzothiazol-2-yloxy)-phenyl]-ethyl}-1,3,8-triaza-spiro[4.5]decan-2,4-dion;
(1-{2-[4-(Benzothiazol-2-yloxy)-phenyl]-ethyl}-piperidin-4-yl)-methyl-carbamidsäure-tert-butylester;
N-(1-{2-[4-(Benzothiazol-2-yloxy)-phenyl]-ethyl}-piperidin-4-yl-N-methyl-acetamid;
N-(1-{2-[4-(Benzothiazol-2-yloxy)-phenyl]-ethyl}-piperidin-4-yl)-N-methyl-methansulfonamid;
Essigsäure [(1-{2-[4-(Benzothiazol-2-yloxy)-phenyl]-ethyl}-piperidin-4-yl-methylcarbamoyl]-methylester;
N-(1-{2-[4-(Benzothiazol-2-yloxy)-phenyl]-ethyl}-piperidin-4-yl-N-acetamid;
Essigsäure-(1-{2-[4-(Benzothiazol-2-yloxy)-phenyl]-ethyl}-piperidin-4-ylcarbamoyl)-methylester;
2-({2-[4-(Benzothiazol-2-yloxy)-phenyl]-ethyl}-methyl-amino)-3-(1H-imidazol-2-yl)-propionsäure);
2-(4-{2-[4-(3-Nitro-pyridin-2-yl)-[1,4]diazepan-1-yl]-ethyl-phenoxy)-benzothiazol;
[(1-{2-[4-(Benzothiazol-2-yloxy)-phenoxy]-ethyl}-piperidin-4-carbonyl)-methylamino]-essigsäureethylester;
1'-{2-[4-(Benzothiazol-2-yloxy)-phenoxy]-ethyl}-[1,4']bipiperidinyl-4-carbonsäure-ethylester;
1'-{2-[4-(Benzothiazol-2-yloxy)-phenoxy]-ethyl}-[1,4']bipiperidinyl-4-carbonsäure;
{2-[4-(Benzothiazol-2-yloxy)-phenyl]-ethyl}-cyclopropyl-ethyl-amin;
3-({2-[4-(Benzothiazol-2-yloxy)-phenyl]-ethyl}-cyclopropyl-amino)-2-methyl-propionsäure-trifluormethansulfonsäuresalz;
2-({2-[4-(Benzothiazol-2-yloxy)-phenyl]-ethyl}-cyclopropylmethylamino)-ethanol;
2-[2-({2-[4-(Benzothiazol-2-yloxy)-phenyl]-ethyl}-cyclopropylmethyl-amino)-ethoxyethanol;
3-({2-[4-(Benzothiazol-2-yloxy)-phenyl]-ethyl}-cyclopropylmethyl-amino)-propan-1-ol;
{2-[4-(Benzothiazol-2-yloxy)-phenyl]-ethyl}-cyclopropylmethyl-(3-tetrazol-2-yl-propyl)-amin;
{2-[4-(Benzothiazol-2-yloxy)-phenyl]-ethyl}-cyclopropyl-(3-pyrrol-1-yl-propyl)-amin;
4-({2-[4-(Benzothiazol-2-yloxy)-phenyl]-ethyl}-cyclopropylmethylamino)-butyronitril;
1-{2-[4-(Benzothiazol-2-yloxy)-phenyl]-ethyl}-piperidin-4-carbonsäure-(2-cyanoethyl)-amid;
{2-[4-(Benzothiazol-2-yloxy)-phenyl]-ethyl}-cyclopropylmethyl-[3-(2H-tetrazol-5-yl)-propylamin;
3-[5-(1-{2-[4-(Benzothiazol-2-yloxy)-phenyl]-ethyl}-piperidin-4-yl)-tetrazol-1-yl-propionitril;
{2-[4-(Benzothiazol-2-yloxy)-phenyl]-ethyl}-cyclopropyl-[3-(2H-tetrazol-5-yl)-propyl]-amin;
1-{2-[4-(Benzothiazol-2-yloxy)-phenyl]-ethyl}-piperidin-4-carbonsäure-(2-hydroxy-1,1-dimethyl-ethyl)-amid;
{2-[4-(Benzothiazol-2-yloxy)-phenyl]-ethyl}-cyclopropylmethyl-[3-(1H-[1,2,4]-triazol-3-yl)-proyl]-amin;
{2-[4-(Benzothiazol-2-yloxy)-phenyl]-ethyl}-cyclopropylmethyl-[3-(5-methyl-1H-[1,2,4]triazol-3-yl-propyl]amin;
{2-[4-(Benzothiazol-2-yloxy)-phenyl]-ethyl]-cyclopropylmethyl-3-(5-phenyl-1H-[1,2,4]triazol-3-yl)-propylamin;
2-(4-{2-[4-(1-Methyl-1H-tetrazol-5-yl)-piperidin-1-yl]-ethyl-phenoxy)-benzothiazol;
2-(4-{2-[4-Methyl-2H-tetrazol-5-yl)-piperidin-1-yl]-ethyl}-phenoxy)-benzothiazol;
1-{2-[4-(Benzothiazol-2-yloxy)-phenyl]-ethyl}-piperidin-4-carbnonitril;
2-(4-{2-[4-(1H-[1,2,3]Triazol-4-yl)-piperidin-1-yl]-ethyl}-phenoxy)-benzothiazol;
4-{2-[4-(Benzothiazol-2-yloxy)-phenyl]-ethylamino}-buttersäure-ethylester;
4-({2-[4-(Benzothiazol-2-yloxy)-phenyl]-ethyl}-cyclopropylmethyl-amino)-buttersäure-ethylester;
2-[3-({2-[4-(Benzothiazol-2-yloxy)-phenyl]-ethyl}-cyclopropylmethyl-amino)-propyl]-isoindol-1,3-dion;
4-({2-[4-(Benzothiazol-2-yloxy)-phenyl]-ethyl}-cyclopropyl-methylamino)-buttersäure;
1-(3-{2-[4-(Benzothiazol-2-yloxy)-phenyl-ethylamino}-propyl)-pyrrolidin-2-on;
N-1-{2-[4-(Benzothiazol-2-yloxy)-phenyl]-ethyl}-N1-cyclopropylmethyl-propan-1,3-diamin;
5-({2-[4-(Benzothiazol-2-yloxy)-phenyl]-ethyl}-cyclopropyl-amino)-pentansäuremethyl ester;
N-[3-({2-[4-(Benzothiazol-2-yloxy)-phenyl]-ethyl}-cyclopropylmethyl-amino)-propyl-acetamid;
Morpholin-4-carbonsäure-[3-({2-[4-(benzothiazol-2-yloxy)phenyl]-ethyl}-cyclopropylmethylamino)-propyl]-amid;
N-[3-({2-[4-(Benzothiazol-2-yloxy)-phenyl]-ethyl}-cyclopropylmethyl-amino)-propyl]-methansulfonamid;
5-({2-[4-(Benzothiazol-2-yloxy)-phenyl]-ethyl}-cyclopropyl-amino)-pentansäure;
1-[3-({2-[4-(Benzothiazol-2-yloxy)-phenyl]-ethyl}-isopropyl-amino)-propyl]-pyrrolidin-2-on;
1-[3-({2-[4-(Benzothiazol-2-yloxy)-phenyl]-ethyl}-cyclopropylmethyl-amino)-propyl]-pyrrolidin-2-on;
1-[3-({2-[4-(Benzothiazol-2-yloxy)-phenyl]-ethyl}-cyclopropyl-amino)-propyl]-pyrrolidin-2-on;
1-[3-({2-[4-(Benzothiazol-2-yloxy)-phenyl]-ethyl}-propyl-amino)-propyl]-pyrrolidin-2-on;
4-((1-Acetyl-piperidin-4-yl)-{2-[4-(benzothiazol-2-yloxy)-phenyl]-ethyl}-amino)-buttersäureethylester;
4-((1-Acetyl-piperidin-4-yl)-{2-[4-(benzothiazol-2-yloxy)-phenyl]-ethyl}-amino)-buttersäureethylester;
4-({2-[4-(Benzothiazol-2-yloxy)-phenyl]-ethyl}-methansulfonylamino)-buttersäure;
({2-[4-(Benzothiazol-2-yloxy)-phenyl]-ethyl}-cyclopropyl-amino)-essigsäure;
6-({2-[4-(Benzothiazol-2-yloxy)-phenyl]-ethyl}-cyclopropyl-amino)-hexansäureethylester;
7-({2-[4-(Benzothiazol-2-yloxy)-phenyl]-ethyl}-cyclopropyl-amino)-heptansäureethylester;
6-({2-[4-(Benzothiazol-2-yloxy)-phenyl]-ethyl}-cyclopropyl-amino)-hexansäure;
7-({2-[4-(Benzothiazol-2-yloxy)-phenyl]-ethyl}-cyclopropyl-amino)-heptansäure;
N-1-{2-[4-(Benzothiazol-2-yloxy)-phenyl]-ethyl}-N1-cyclopropyl-propan-1,3-diamin;
N-[3-({2-[4-(Benzothiazol-2-yloxy)-phenyl]-ethyl}-cyclopropyl-amino)-propyl]-acetamid;
N-[3-({2-[4-(Benzothiazol-2-yloxy)-phenyl]-ethyl}-cyclopropyl-amino)-propyl]-isobutyramid;
N-[3-({2-[4-(Benzothiazol-2-yloxy)-phenyl]-ethyl}-cyclopropyl-amino)-propyl]-benzamid;
N-[3-({2-[4-(Benzothiazol-2-yloxy)-phenyl]-ethyl}-cyclopropyl-amino)-propyl]-4-chlor-benzamid;
N-[3-({2-[4-(Benzothiazol-2-yloxy)-phenyl]-ethyl}-cyclopropyl-amino)-propyl]-methansulfonamid;
Propan-2-sulfonsäure-[3-({2-[4-(benzothiazol-2-yloxy)-phenyl]-ethyl}-cyclopropyl-amino)-propyl]-amid-trifluormethansulfonsäuresalz;
8-({2-[4-(Benzothiazol-2-yloxy)-phenyl]-ethyl}-cyclopropyl-amino)-octansäureethylester;
1-[3-({2-[4-(Benzothiazol-2-yloxy)-phenyl]-ethyl}-cyclopropyl-amino)-propyl]-3-phenyl-urea;
8-({2-[4-(Benzothiazol-2-yloxy)-phenyl]-ethyl}-cyclopropyl-amino)-octansäure;
Tetrahydro-furan-2-carbonsäure-[3-({2-[4-(benzothiazol-2-yloxy)-phenyl]-ethyl}-cyclopropyl-amino)-propyl]-amid;
N-[3-({2-[4-(Benzothiazol-2-yloxy)-phenyl]-ethyl}-cyclopropyl-amino)-propyl]-2-hydroxy-acetamid;
4-({2-[4-(Benzothiazol-2-yloxy)-phenoxy]-ethyl}-cyclopropyl-amino)-buttersäure;
2-[4-(2-Morpholin-4-yl-ethoxy)-phenoxy]-benzothiazol;
2-[4-(2-Piperidin-1-yl-ethoxy)-phenoxy]-benzothiazol.

14. Verbindung nach Anspruch 1, wobei die Verbindung nach Formel (II) eine der folgenden ist:
1-{2-[4-(1*H*-Benzoimidazol-2-yloxy)-phenoxy]-ethyl}-4-phenyl-piperidin-4-ol;
{2-[4-(1*H*-Benzoimidazol-2-yloxy)-phenyl]-ethyl}-cyclopropylmethyl-propyl-amin;
Cyclohexyl-ethyl-{2-[4-(1-methyl-1*H*-benzoimidazol-2-yloxy)-phenyl]-ethyl}-amin;
1-{2-[4-(1*H*-Benzoimidazol-2-yloxy)-phenoxy]-ethyl}-4-(4-brom-phenyl)-piperidin-4-ol;
1-{2-[4-(1*H*-Benzoimidazol-2-yloxy)-phenoxy]-ethyl}-4-(4-chlor-phenyl)-piperidin-4-ol;
1-{2-[4-(1*H*-Benzoimidazol-2-yloxy)-phenoxy]-ethyl}-4-benzyl-piperidin-4-ol;
{2-[4-(1*H*-Benzoimidazol-2-yloxy)-phenyl]-ethyl}-cyclohexyl-ethyl-amin;
2-[4-(2-Pyrrolidin-1-yl-ethyl)-phenoxy]-1*H*-benzoimidazol;
2-[4-(2-Azepan-1-yl-ethyl)-phenoxy]-1*H*-benzoimidazol;
{2-[4-(1*H*-Benzoimidazol-2-yloxy)-phenyl]-ethyl}-dibutyl-amin;
1-{2-[4-(1*H*-Benzoimidazol-2-yloxy)-phenyl]-ethyl}-piperidin-4-ol;
1-(2-[4-(1*H*-Benzoimidazol-2-yloxy)-phenyl]-ethyl}-piperidin-4-carbonsäuremethylester;
{2-[4-(1*H*-Benzoimidazol-2-yloxy)-phenoxy]-ethyl-cyclohexyl-ethyl-amin;
2-{4-{2-(4-Methyl-piperidin-1-yl)-ethoxy]-phenoxy}-1*H*-benzoimidazol;
2-{4-[2-(2-Ethyl-piperidin-1-yl)-ethoxy]-phenoxy}-1*H*-benzoimidazol;
2-[4-(2-Piperidin-1-yl-ethoxy)-phenoxy]-1*H*-benzoimidazol;
1-{2-[4-(1*H*-Benzoimidazol-2-yloxy)-phenoxy]-ethyl}-piperidin-4-ol;
2-[4-(2-Azepan-1-yl-ethoxy)-phenoxy]-1*H*-benzoimidozolamid;
{3-[4-(1*H*-Benzoimidazol-2-yloxy)-phenoxy]-propyl}-dimethylamin;
2-[4-(2-Pyrrolidin-1-yl-ethoxy)-phenoxy]-1*H*-benzoimidazol;
{2-[4-(1*H*-Benzoimidazol-2-yloxy)-phenoxy]-ethyl}-diethyl-amin;
2-[4-(2-Morpholin-4-yl-ethoxy)-phenoxy]-1*H*benzoimidazol;
2-[4-(2-Piperidin-1-yl-ethyl)-phenoxy]-1*H*-benzoimidazol;
1-(1-{2-[4-(1*H*-Benzoimidazol-2-yloxy)-phenoxy]-ethyl}-piperidin-4-yl)-pyrrolidin-2-on;
(1-{2-[4-(1H-Benzoimidazol-2-yloxy)-phenoxy]-ethyl}-piperidin-4-yl)-methanol; und
1-{2-[4-(1*H*-Benzoimidazol-2-yloxy)-phenoxy]-ethyl)-piperidin-4-carbonsäureethylester.

15. Pharmazeutische Verbindung, die eine therapeutisch wirksame Menge von mindestens einer Verbindung der Formel (I) umfasst: 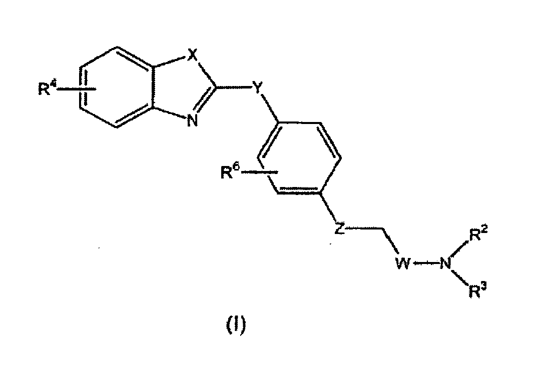 wobei
X ausgewählt ist aus der Gruppe, bestehend aus NR⁵, O und S, wobei R⁵ einer von H und CH₃ ist;
Y ausgewählt ist aus der Gruppe, bestehend aus CH₂ und O;
Z ausgewählt ist aus der Gruppe, bestehend aus O und einer Bindung;
W ausgewählt ist aus der Gruppe, bestehend aus CH₂ und CHR¹-CH₂, wobei R¹ einer von H und OH ist, wobei das an R¹ angefügte Kohlenstoffglied in dem CHR¹-CH₂ nicht direkt an das Stickstoffglied angefügt ist, an das W angefügt ist;
R⁴ ausgewählt ist aus der Gruppe, bestehend aus H, OCH₃, Cl, F, Br, J, OH, NH₂, CN, CF₃ und CH₃;
R⁶ H oder F ist; und
R² und R³ jeweils unabhängig ausgewählt sind aus der Gruppe, bestehend aus
A) H, C₁₋₇Alkyl, C₃₋₇Alkenyl, wobei der Kohlenstoff in dem Alkenyl, das an das Stickstoffglied angefügt ist, nur Einfachbindungen aufweist, C₃₋₇Alkynyl, wobei der Kohlenstoff in dem Alkynyl, das an das Stickstoffglied angefügt ist, nur Einzelbindungen aufweist, C₃₋₇Cycloalkyl wahlweise mit Benzol fusioniert ist, C₅₋₇Cycloalkenyl, -C₃₋₇CycloalkylC₁₋₇Alkyl, -C₁₋₇AlkylC₃₋₇Cycloalkyl und Phenyl, wobei jeder der Substituenten A) unabhängig substituiert ist mit 0, 1 oder 2 R^{Q} und jeder von R^{Q} ein Substituent an einem Kohlenstoffglied ist, das mindestens ein Kohlenstoffglied ist, das von dem Stickstoffglied entfernt wurde,
B) ein Substituent HetR^{a};
C) -C₁₋₇AlkylC(O)R^{X}, wahlweise substituiert mit CH₂R^{Ar} oder CH₂R^{Ar'};
D) -C₂₋₅AlkylC(O)R^{X}, wobei zwei valenzerlaubte Kohlenstoffglieder in dem C₂₋₅Alkyl des -C₂₋₅AlkylC(O)R^{X} Teil eines gesättigten C₃₋₆Carbozyklus sind;
E) -C₂₋₅AlkylOH, wobei zwei valenzerlaubte Kohlenstoffglieder in dem C₂₋₅Alkyl des -C₂₋₅AlkylOH Teil eines gesättigten C₃₋₆Carbozyklus sind;
F) -C₀₋₄Alkylphenyl, wobei das Phenyl in dem -C₀₋₄Alkylphenyl an zwei angrenzenden Kohlenstoffglieder in dem Phenyl mit R^{f} fusioniert ist oder mit Benzol fusioniert ist;
G) -C₀₋₄AlkylAr⁶, wobei Ar⁶ ein 6-gliedriges Heteroaryl ist, das einen Kohlenstoffgliedanfügungspunkt aufweist und ein oder zwei -N=-Heteroatomglieder aufweist und mit Benzol fusioniert ist;
H) -C₀₋₄AlkylAr⁵, wobei Ar⁵ ein 5-gliedriges Heteroaryl ist, das ein Heteroatomglied aufweist, ausgewählt aus der Gruppe, bestehend aus O, S und >NR^{Y}, und 0 oder 1 zusätzliches -N=-Heteroatomglied aufweist, das wahlweise zwei Carbonylgruppen enthält und wahlweise mit Benzol fusioniert ist;
I) -C₁₋₄AlkylAr^{5'}-, wobei Ar^{5'}- ein 5-gliedriges Heteroaryl ist, das 3 oder 4 Stickstoffglieder aufweist, wahlweise substituiert mit R^{Y}, und das eine valenzerlaubte Stelle als Anfügungspunkt aufweist;
J) -C₀₋₁AlkylAr⁶⁻⁶, wobei Ar⁶⁻⁶ ein an C₀₋₄Alkyl angefügtes Phenyl ist, das an valenzerlaubten Stellen mit einem 6-gliedrigen Heteroaryl fusioniert ist, wobei das 6-gliedrige Heteroaryl ein oder zwei -N=-Heteroatomglieder aufweist;
K) -C₀₋₄AlkylAr⁶⁻⁵, wobei Ar⁶⁻⁵ ein an C₀₋₄Alkyl angefügtes Phenyl ist, das an valenzerlaubten Stellen mit einem 5-gliedrigen Heteroaryl fusioniert ist, wobei das 5-gliedrige Heteroaryl ein Heteroatomglied aufweist, ausgewählt aus der Gruppe, bestehend aus O, S und >NR^{Y}, und das 5-gliedrige Heteroaryl 0 oder 1 zusätzliches Heteroatomglied aufweist, das -N= ist; und
L) einer von 2-(4-Ethyl-phenoxy)-benzothiazol, 2-(4-Ethyl-phenoxy)-benzooxazol und 2-(4-Ethyl-phenoxy)-1H-benzoimidazol;
M) SO₂C₁₋₄Alkyl;
R² und R³ alternativ mit dem Stickstoff, an den sie angefügt sind, zusammengenommen sind, um einen heterozyklischen Ring zu bilden, der mindestens ein Heteroatomglied enthält, welches der angefügt Stickstoff ist, wobei der heterozyklische Ring ausgewählt ist aus der Gruppe, bestehend aus
i) einem 4- bis 7-gliedrigen heterozyklischen Ring HetR^{b}, wobei der 4- bis 7-gliedrige heterozyklische Ring HetR^{b} ein Heteroatomglied aufweist, das der Anfügungsstickstoff ist und mit 0, 1 oder 2 Substituenten an denselben oder an verschiedenen Substitutionsgliedern substituiert wird, wobei die Substituenten ausgewählt sind aus der Gruppe, bestehend aus -R^{Y}, -CN, -C(O)R^{Y}, -C₀₋₄AlkylCO₂R^{Y}, -C₀₋₄Alkylc(O)CO₂R^{Y}, -C₀₋₄AlkylOR^{Y}, -C₀₋₄AlkylC(O)NR^{Y}R^{Z}, -C₀₋₄AlkylNR^{Y}C(O)R^{Z}, -C(O)NR^{Z}OR^{Y}, -C₀₋₄AlkylNR^{Y}C(O)CH₂OR^{Y}, -C₀₋₄AlkylNR^{Y}C(O)CH₂C(O)R^{Y}, -C₀₋₄AlkylNR^{Y}CO₂R^{Y},-C₀₋₄AlkylNR^{Y}C(O)NR^{Y}R^{Z}, -C₀₋₄AlkylNR^{Y}C(S)NR^{Y}R^{Z}, -NR^{Y}C(O)CO₂R^{Y}, -NR^{Y}R^{Z}, - C₀₋₄AlkylNR^{W}SO₂R^{Y}, 1,3-Dihydroindol-2-on-1-yl, 1,3-Dihydro-benzoimidazol-2-on-1-yl, Tetrazol-5-yl, 1-R^{Y}-1H-Tetrazol-5-yl, R^{Y}-Triazolyl, 2-R^{Y}-2H-Tetrazol-5-yl, Pyrrolidin-2-thion-1-yl, Piperidin-2-thion-1-yl, -C₀₋₄AlkylC(O)N(R^{Y})(SO₂R^{Y}), -C₀₋₄AlkylN(R^{Y})(SO₂)NR^{Y}R^{Y}, -C₀₋₄AlkylN(R^{Y})(SO₂)NR^{Y}CO₂R^{Y}, Halo, 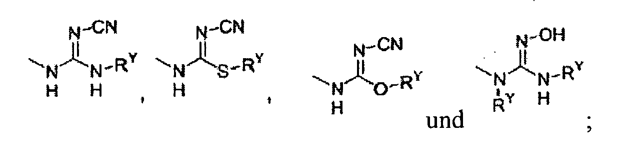
ii) einem 5- bis 7-gliedrigen heterozyklischen Ring HetR^{c}, wobei der 5- bis 7-heterozyklische Ring HetR^{c} ein zusätzliches Heteroatomglied aufweist, das von dem Anfügungsstickstoff durch mindestens ein Kohlenstoffglied getrennt ist, wobei das zusätzliche Heteroatomglied ausgewählt ist aus der Gruppe, bestehend aus O, S(=O)₀₋₂ und >NR^{M}, wobei der 5- bis 7-gliedrige heterozyklische Ring HetR^{c} 0 oder 1 Carbonylglieder aufweist, und mit 0, 1 oder 2 Substituenten an denselben oder an verschiedenen Kohlenstoffsubstitutionsgliedern substituiert ist, wobei die Substituenten ausgewählt sind aus der Gruppe, bestehend aus -C(O)R^{Y}, -CO₂R^{Y}, -C₃₋₄AlkylCO₂R^{Y} und R^{Z};
iii) einem von Imidazolidin-1-yl, 2-Imidazolin-1-yl, Pyrazol-1-yl, Imidazol-1-yl, 2H-tetrazol-2-yl, 1H-tetrazol-1-yl, Pyrrol-1-yl, 2-pyrrolin-1-yl und 3-pyrrolin-1-yl, wobei jeder von dem 2H-tetrazol-2-yl und 1H-tetrazol-1-yl an dem Kohlenstoffglied substituiert ist mit 0 oder 1 von -C₀₋₄-AlkylR^{Z}, -C₀₋₄AlkylSR^{Y}, -C₀₋₄AlkylCO₂R^{Y} und Substituent HetR^{a}; und
iv) einem von 1,2,3,4-Tetrahydro-chinolin-1-yl, 1,2,3,4-Tetrahydro-isochinolin-2-yl, Indol-1-yl, Isoindol-2-yl, Indolin-1-yl, Benzimidazol-1-yl, 2,8-Diazaspiro[4.5]dekan-1-on-8-yl, 4-{[(2-tert-Butoxycarbonylamino-cyclobutancarbonyl)-amino]-methyl}-piperidin-1-yl, 4-{[(2-amino-cyclobutancarbonyl)-amino]methyl}-piperidin-1-yl, 3,9-Diaza-spiro[5.5]undecan-3-carbonsäure-9-yl-tert-butylester, 4-Oxo-1-phenyl-1,3,8-triaza-spiro[4.5]dec-8-yl und 4-Oxo-1,3,8-triaza-spiro[4.5]dec-8-yl;
wobei
der Substituent HetR^{a} ein 4- bis 7-gliedriger heterozyklischer Ring ist, der einen Kohlenstoffgliedanfügungspunkt aufweist und ein Glied >NR^{M} als ein Heteroatomglied enthält, und wobei das Heteroatomglied vom Kohlenstoffanfügungspunkt durch mindestens ein zusätzliches Kohlenstoffglied getrennt ist;
R^{K} ausgewählt ist aus der Gruppe, bestehend aus H, -C₁₋₄Alkyl, -C₀₋₄AlkylR^{Ar}, jeweils wahlweise substituiert mit 1, 2 oder 3 Substituenten R^{N};
R^{L} ausgewählt aus der Gruppe von -CO₂R^{S} und -C(O)NR^{S}R^{S'};
R^{M} ausgewählt ist aus der Gruppe, bestehend aus R^{Z}, Indol-7-yl, -SO₂R^{Y}, -C₃-₄AlkylCO₂R^{Y}, -CO₂R^{Y}, -C(O)NR^{Z}OR^{Y}, -C(O)R^{Y}, -C(O)C₁₋₄AlkylOR^{Y}, -C₀₋₄AlkylC(O)NR^{S}R^{S'}, C₀₋₄AlkylC(O)CO₂R^{Y}, 1,3-Dihydro-indol-2-on-1-yl, 1,3-Dihydro-benzoimidazol-2-on-1-yl, Tetrazol-5-yl, 1-R^{Y}-1H-Tetrazol-5-yl, R^{Y}-Triazolyl, 2-R^{Y}-2H-Tetrazol-5-yl und -C₀₋₄AlkylC(O)N(R^{Y})(SO₂R^{Y}), jeweils wahlweise substituiert mit 1, 2 oder 3 Substituenten R^{N};
R^{N} ausgewählt ist aus der Gruppe, bestehend aus OCH₃, Cl, F, Br, I, OH, NH₂, CN, CF₃, CH₃, OC(O)CH₃ und NO₂;
R^{P} ausgewählt ist aus der Gruppe, bestehend aus R^{Y}, -C₂₋₄AlkylOR^{Y}, R^{Ar}, -C₁₋₂AlkylCO₂R^{Y}, -C₁₋₂AlkylCONR^{S}R^{S}, Indol-7-yl und -SO₂C₁₋₄Alkyl;
R^{Q} ausgewählt ist aus der Gruppe, bestehend aus Fluor, Chlor, Brom, Jod, Trifluormethyl, Trichlormethyl, -CN, -C₁₋₄Alkyl, C₀₋₄AlkylR^{Ar}, -C₀₋₄AlkylR^{Ar}, -C₀₋₄AlkylOR^{Y}, -C₀₋₄AlkylCO₂R^{Y}, -C₀₋₄AlkylNR^{Y}R^{Z}, -C₀₋₄AlkylNR^{Y}COR^{Y}, -C₀₋₄AlkylNR^{Y}CONR^{Y}R^{Z}, -C₀₋₄AlkylNR^{Y}SO₂R^{Y} und -C₀₋₄AlkylSR^{Y};
R^{S} und R^{S'} unabhängig ausgewählt sind aus der Gruppe, bestehend aus H, -C₁₋₄Alkyl und -C₀₋₄Alkylphenyl; alternativ sind R^{S} und R^{S'} mit dem Stickstoffglied, an das R^{S} und R^{S'} angefügt sind, zusammengenommen, um einen 4- bis 7-gliedrigen heterozyklischen Ring zu bilden, der 0 oder 1 zusätzliches Heteroatomglied, ausgewählt aus der Gruppe, bestehend aus O, S und >NR^{Y}, aufweist, vorausgesetzt, dass das zusätzliche Heteroatomglied durch mindestens zwei Kohlenstoffglieder von dem Stickstoffglied, an das R^{S} und R^{S'} angefügt sind, getrennt ist, und vorausgesetzt, dass, wenn R^{Y} C₀₋₄AlkylR^{Ar} ist, R^{Ar} dann nicht mit R^{L} substituiert ist;
R^{W} ausgewählt ist aus der Gruppe, bestehend aus R^{Y} und -C₃₋₇Cycloalkyl;
R^{X} ausgewählt ist aus der Gruppe, bestehend aus OR^{Y}, -NR^{Y}R^{Z}, -C₁₋₄Alkyl und -C₀₋₄AlkylR^{Ar};
R^{Y} ausgewählt ist aus der Gruppe, bestehend aus H, -C₁₋₄Alkyl, -C₀₋₄AlkylR^{Ar} und -C₀₋₄AlkylR^{Ar}, jeweils wahlweise substituiert mit 1, 2 oder 3 Substituenten R^{N};
R^{Z} ausgewählt ist aus der Gruppe, bestehend aus R^{Y}, -C₂₋₄AlkylOR^{Y}, -C₁₋₂AlkylCO₂R^{Y}, -C₁₋₂AlkylC(O)NR^{S}R^{S'} und -C₂₋₄AlkylNR^{S}R^{S'};
wobei wenn R^{Y} und R^{Z} an ein Stickstoffglied angefügt sind, R^{Y} und R^{Z} ausgewählt sind, wie oben definiert, oder R^{Y} und R^{Z} mit dem an R^{Y} und R^{Z} angefügten Stickstoffglied zusammengenommen sind, um einen 4- bis 7-gliedrigen heterozyklischen Ring HetR^{d} zu bilden, der 0 oder 1 zusätzliches Heteroatomglied, ausgewählt aus der Gruppe, bestehend aus O, S und >NR^{M}, aufweist, wobei der 4- bis 7-gliedrige heterozyklische Ring HetR^{d} 0 oder 1 Carbonylglied aufweist, und der 4- bis 7-gliedrige heterozyklische Ring HetR^{d} 0 oder 1 valenzerlaubte Kohlenstoffglieder, substituiert mit mindestens einem von R^{M}, -CO₂H und -C₀₋₁AlkylOR^{Y}, aufweist;
R^{Ar} ein Rest ist mit einem Kohlenstoffgliedanfügungspunkt und der Rest ausgewählt ist aus der Gruppe, bestehend aus Phenyl, Pyridyl, Pyrimidyl und Pyrazinyl, wobei jedes valenzerlaubte Kohlenstöffglied in jedem der Reste unabhängig mit mindestens einem von 0, 1, 2 oder 3 R^{N} substituiert ist, und 0 oder 1 R^{L};
R^{Ar'} ein 3- bis 8-gliedriger Ring ist, der 0, 1 oder 2 Heteroatomglieder aufweist, ausgewählt aus der Gruppe, bestehend aus O, S, N und >NR^{Y}, der 0, 1 oder 2 ungesättigte Bindungen aufweist, der 0 oder 1 Carbonylglieder aufweist, wobei jedes valenzerlaubte Mitglied in jedem der Ringe unabhängig substituiert ist mit 0, 1 oder 2 R^{k}; und
R^{f} eine lineare 3- bis 5-gliedrige Kohlenwasserstoffgruppe ist, die 0 oder 1 ungesättigte Kohlenstoff-Kohlenstoff-Bindungen aufweist und 0 oder 1 Carbonylmitglied aufweist;
oder ein Enantiomer, Diastereomer, Racemat, Tautomer, Hydrat, Solvat oder ein pharmazeutisch annehmbares Salz, ein Ester oder Amid davon.

16. Pharmazeutische Zusammensetzung, die mindestens eine Verbindung, wie in einem der Ansprüche 1-14 beansprucht, umfasst.

17. Verwendung einer therapeutisch wirksamen Menge von mindestens einem LTA4H-Modulator, ausgewählt aus Verbindungen nach Anspruch 1 oder Anspruch 2, oder Verbindungen nach Formel (I), wie in Anspruch 12 beansprucht, zur Herstellung eines Medikaments zur Behandlung oder Verhütung eines durch LTA4H vermittelten Zustands in einem Subjekt.

18. Verwendung von mindestens einem LTA4H-Modulator, ausgewählt aus Verbindungen, wie in Anspruch 1 oder Anspruch 2 beansprucht, oder Verbindungen nach Formel (I), wie in Anspruch 12 beansprucht, zur Herstellung eines Medikaments zur Behandlung, Verhütung oder Hemmung einer Entzündung in einem Subjekt.

19. Verwendung einer hemmenden Menge von mindestens einem LTA4H-Modulator, ausgewählt aus Verbindungen, wie in Anspruch 1 oder Anspruch 2 beansprucht, oder Verbindungen nach Formel (I), wie in Anspruch 12 beansprucht, zur Herstellung eines Medikaments zur Hemmung der Enzymaktivität von LTA4H.

20. Verwendung wie in Anspruch 17 beansprucht, wobei R⁴ H ist.

21. Verwendung wie in Anspruch 18 beansprucht, wobei R⁴ H ist.

22. Verwendung wie in Anspruch 19 beansprucht, wobei R⁴ H ist.

23. Verwendung wie in Anspruch 17 beansprucht, wobei R² und R³ jeweils unabhängig ausgewählt sind aus einer der folgenden Gruppen:
(i) der Gruppe, bestehend aus A), B), C), D), E) und I), wenn R² und R³ aus verschiedenen Gruppen ausgewählt sind; oder
(ii) Gruppe A); oder
(iii) Gruppe B); oder
(iv) Gruppe C); oder
(v) Gruppe D); oder
(vi) Gruppe E); oder
(vii) Gruppe I),
wie in Anspruch 1, Anspruch 2 oder Anspruch 12 definiert.

24. Verwendung wie in Anspruch 18 beansprucht, wobei R² und R³ jeweils unabhängig ausgewählt sind aus einer der folgenden Gruppen:
(i) der Gruppe, bestehend aus A), B), C), D), E) und I), wenn R² und R³ aus verschiedenen Gruppen ausgewählt sind; oder
(ii) Gruppe A); oder
(iii) Gruppe B); oder
(iv) Gruppe C); oder
(v) Gruppe D); oder
(vi) Gruppe E); oder
(vii) Gruppe I),
wie in Anspruch 1, Anspruch 2 oder Anspruch 12 definiert.

25. Verwendung wie in Anspruch 19 definiert, wobei R² und R³ jeweils unabhängig ausgewählt sind aus einer der folgenden Gruppen:
(i) der Gruppe, bestehend aus A), B), C), D), E) und I), wenn R² und R³ aus verschiedenen Gruppen ausgewählt sind; oder
(ii) Gruppe A); oder
(iii) Gruppe B); oder
(iv) Gruppe C); oder
(v) Gruppe D); oder
(vi) Gruppe E); oder
(vii) Gruppe I),
wie in Anspruch 1, Anspruch 2 oder Anspruch 12 definiert.

26. Verwendung wie in Anspruch 17 beansprucht, wobei R² und R³ mit dem Stickstoff, an den sie angefügt sind, zusammengenommen sind, um einen heterozyklischen Ring zu bilden, der mindestens ein Heteroatomglied enthält, das der Anfügungsstickstoff ist,
wobei der heterozyklische Ring ausgewählt ist aus einer der folgenden Gruppen:
(g) der Gruppe, bestehend aus (i) und (ii), wenn R² und R³ aus verschiedenen Gruppen ausgewählt sind;
(h) der Gruppe (i), und
(i) der Gruppe (ii),
wie in Anspruch 1, Anspruch 2 oder Anspruch 12 definiert.

27. Verwendung wie in Anspruch 18 definiert, wobei R² und R³ mit dem Stickstoff, an den sie angefügt sind, zusammengenommen sind, um einen heterozyklischen Ring zu bilden, der mindestens ein Heteroatomglied enthält, das der angefügte Stickstoff ist,
wobei der heterozyklische Ring ausgewählt ist aus einer der folgenden Gruppen:
(j) der Gruppe, bestehend aus (i) und (ii), wenn R² und R³ aus verschiedenen Gruppen ausgewählt sind;
(k) der Gruppe (i), und
(l) der Gruppe (ii),
wie in Anspruch 1, Anspruch 2 oder Anspruch 12 definiert.

28. Verbindung wie in Anspruch 19 beansprucht, wobei R² und R³ mit dem Stickstoff, an den sie angefügt sind, zusammengenommen sind, um einen heterozyklischen Ring zu bilden, der mindestens ein Heteroatomglied ist, das der Anfügungsstickstoff ist, wobei der heterozyklische Ring ausgewählt ist aus einer der folgenden Gruppen:
(m) der Gruppe, bestehend aus (i) und (ii), wenn R² und R³ aus verschiedenen Gruppen ausgewählt sind;
(n) der Gruppe (i), und
(o) der Gruppe (ii),
wie in Anspruch 1, Anspruch 2 oder Anspruch 12 definiert.

29. Verwendung wie in einem der Ansprüche 17 bis 28 definiert, wobei der durch LTA4H vermittelte Zustand eine Entzündung aufgrund von mindestens einer Erkrankung von Asthma, chronisch obstruktiver Lungenkrankheit, Arteriosklerose, rheumatoider Arthritis, multipler Sklerose, entzündlicher Darmerkrankung und Psoriasis ist.

30. Verwendung wie in einem der Ansprüche 17 bis 19 beansprucht, wobei der mindestens eine LTA4H-Modulator ausgewählt ist aus den Verbindungen, wie in einem der Ansprüche 9 bis 14 beansprucht.

## Revendications

1. Composé de formule (II): 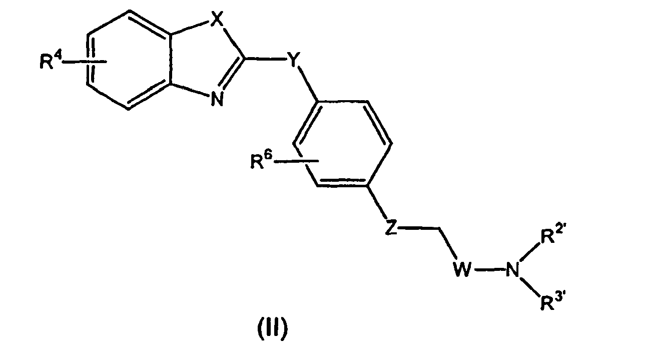 ou un énantiomère, diastéréomère, racémique, tautomère, hydrate, solvate, ou un sel, ester, ou amide pharmaceutiquement acceptable de celui-ci,
où
X est sélectionné dans le groupe consistant en NR⁵, O, et S, avec R⁵ qui est l'un de H et CH₃;
Y est sélectionné dans le groupe consistant en CH₂, et O;
Z est sélectionné dans le groupe consistant en O et une liaison;
W est sélectionné dans le groupe consistant en CH₂ et CHR¹-CH₂, avec R¹ étant l'un de H et OH, où l'élément de carbone attaché à R¹ dans ledit CHR¹-CH₂ n'est pas directement attaché à l'élément d'azote auquel est attaché ledit W;
R⁴ est sélectionné dans le groupe consistant en H, OCH₃, Cl, F, Br, I, OH, NH₂, CN, CF₃ et CH₃;
R⁶ est H ou F; et
R^{2'} est défini comme R² et R^{3'}, est défini comme R³ , comme suit:
chacun de R² et R³ est indépendamment sélectionné dans le groupe consistant en:
A) H, alkyle C₁₋₇, alcényle C₃₋₇, où le carbone dans ledit alcényle qui est attaché à l'élément d'azote n'a que de simples liaisons, alkynyle C₃₋₇, où le carbone dans ledit alkynyle qui est attaché à l'élément d'azote n'a que de simples liaisons, cycloalkyle C₃₋₇ facultativement benzofusionné, cycloalcényleC₅₋₇, -cycloalkylC₃₋₇alkyleC₁₋₇, -alkylC₁₋₇cycloalkyleC₃₋₇ et phényle, où chacun des substituants A) est indépendamment substitué par 0, 1 ou 2 R^{Q}, et chacun desdits R^{Q} est un substituant à un élément de carbone qui est au moins un élément de carbone retiré de l'élément d'azote;
B) un substituant HetR^{a};
C) -alkylC₁₋₇C(O)R^{x}, facultativement substitué par CH₂R^{Ar} ou CH₂R^{Ar'};
D) -alkylC₂₋₅C(O)R^{x}, où deux éléments de carbone de valence admise dans l'alkyleC₂₋₅ dudit alkylC₂₋₅C(O)R^{x} font partie d'un carbocycleC₃₋₆ saturé;
E) -alkyleC₂₋₅OH où deux éléments de carbone à valence admise dans l'alkyleC₂₋₅ dudit alkylC₂₋₅OH font partie d'un carbocycleC₃₋₆ saturé;
F) -alkylC₀₋₄phényle, où le phényle dans ledit -alkylC₀₋₄phényle est fusionné en deux éléments de carbone adjacents dans ledit phényle à R^{f}, ou est benzofusionné;
G) -alkylC₀₋₄Ar⁸, où Ar⁶ est un hétéroaryle à 6 membres ayant un point d'attachement d'élément de carbone ayant un ou deux éléments hétéroatomes -N=, et benzofusionné;
H) -alkyl-C₀₋₄Ar⁵, où Ar⁵ est un hétéroaryle à 5 membres, ayant un élément d'hétéroatome sélectionné dans le groupe consistant en O, S, et >NR^{Y} et ayant 0 ou 1 élément hétéroatome additionnel -N=, contenant facultativement deux groupes carbonyles, et facultativement benzofusionnés;
I) -alkyleC₁₋₄Ar^{5'}, où Ar^{5'} est un hétéroaryle à 5 membres contenant 3 ou 4 éléments d'azote, facultativement substitués par R^{Y}, et ayant un site de valence admise comme point d'attachement;
J) -alkyl-C₀₋₄Ar⁶⁻⁶, où Ar⁶⁻⁶ est un phényle attaché à alkyle C₀₋₄ fusionné aux sites de valence admise à un hétéroaryle à 6 membres, où ledit hétéroaryle à 6 membres a un ou deux éléments hétéroatomes -N=;
K) -alkyle-C₀₋₄Ar⁶⁻⁵, où Ar⁶⁻⁵ est un phényle attaché à alkyleC₀₋₄ fusionné aux sites à valence admise à un hétéroaryle à 5 membres, ledit hétéroaryle à 5 membres ayant un élément hétéroatome sélectionné dans le groupe consistant en O, S, et >NR^{Y}, et ledit hétéroaryle à 5 membres ayant 0 ou 1 élément hétéroatome additionnel qui est -N=; et
L) l'un de 2-(4-éthyl-phénoxy)-benzothiazole, 2-(4-éthyl-phénoxy)-benzooxazole, et 2-(4-éthyl-phénoxy)-1*H*-benzoimidazole;
M) SO₂alkyleC₁₋₄;
Alternativement, R² et R³ sont pris ensemble avec l'azote auquel ils sont attachés pour former un cycle hétérocyclique qui contient au moins un élément hétéroatome qui est ledit azote d'attachement, ledit cycle hétérocyclique étant sélectionné dans le groupe consistant en:
un cycle hétérocyclique à 4-7 membres HetR^{b}, ledit cycle hétérocyclique à 4-7 membres Het^{Rb} ayant un élément hétéroatome qui est ledit azote d'attachement, et étant substitué par 0, 1 ou 2 substituants sur le même ou des éléments différents de substitution, lesdits substituants étant sélectionnés dans le groupe consistant en -R^{Y}, -CN, -C(O)R^{Y}, -alkylC₀₋₄CO₂R^{Y}, -alkylC₀₋₄C(O)CO₂R^{Y}, -alkylC₀₋₄OR^{Y}, -alkylC₀₋₄C(O)NR^{Y}R^{Z}, alkylC₀₋₄NR^{Y}C(O)R^{Z}, -C(O)NR^{Z}OR^{Y}, -alkylC₀₋₄NR^{Y}C(O)CH₂OR^{Y}, -alkylC₀₋₄NR^{Y}C(O)CH₂C(O)R^{Y}, -alkylC₀₋₄NR^{Y}CO₂R^{Y}, -alkyle C₀₋₄NR^{Y}C(O)NE^{Y}R^{Z}, -alkylC₀₋₄NR^{Y}C(S)NR^{Y}R^{Z}, -NR^{Y} C(O)CO₂R^{Y}, -NR^{Y}R^{Z}, -alkylC₀₋₄NR^{W}SO₂R^{Y}, 1,3-dihydro-indol-2-one-1-yle, 1,3-dihydro-benzo-imidazol-2-one-1-yle, tétrazol-5-yle, 1-R^{Y}-1*H*-tétrazol-5-yle, R^{Y}-triazolyle, 2-R^{Y}-2*H-*tétrazol-5-yle, pyrrolidine-2-thion-1-yle, pipéridine-2-thion-1-yle, -alkyleC₀₋₄C(O)N(R^{Y}) (SO₂R^{Y}), -alkylC₀₋₄N(R^{Y})(SO₂)NR^{Y}R^{Y}, -alkyl C₀₋₄N(R^{Y})(SO₂)NR^{Y}R^{Y}, halo, 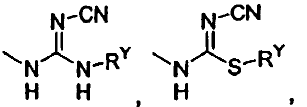 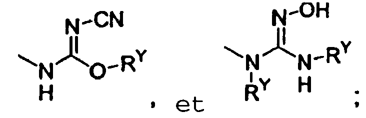
ii) un cycle hétérocyclique à 5-7 membres HetR^{c}, ledit cycle hétérocyclique à 5-7 HetR^{c} ayant un élément hétéroatome additionnel séparé dudit azote d'attachement par au moins un élément de carbone, ledit élément d'hétéroatome additionnel étant sélectionné dans le groupe consistant en O, S(=O)₀₋₂, et >NR^{M}, ledit cycle hétérocyclique à 5-7 membres HetR^{c} ayant 0 ou 1 éléments de carbonyle, et étant substitué par 0, 1, ou 2 substituants aux mêmes éléments ou en des éléments différents de substitution de carbone, lesdits substituants étant sélectionnés dans le groupe consistant en -C (O)R^{Y}, -CO₂R^{Y}, alkylC₃₋₄CO₂R^{Y} et R^{Z};
iii) l'un de imidazolidin-1-yle, 2-imidazolin-1-yle, pyrazol-1-yle, imidazol-1-yle, 2^{H}-tétrazol-2-yle, 1*H*-tétrazol-1-yle, pyrrol-1-yle, 2-pyrrolin-1-yle, et 3-pyrrolin-1-yle, où chacun dudit 2*H*-tétrazol-2-yle et 1H-tétrazol-1-yle est substitué à un élément de carbone par 0 ou 1 de-alkyl-C₀₋₄R^{Z}, -alkyleC₀₋₄SR^{Y}, -alkylC₀₋₄CO₂R^{Y}, et le substituant HetR^{a}; et
iv) l'un de 1,2,3,4-tétrahydro-quinoléin-1-yle, 1,2,3,4-tétrahydro-isoquinoléin-2-yle, indol-1-yle, isoindol-2-yle, indolin-1-yle, benzi-midazol-1-yle, 2,8-diaza-spiro[4.5]décan-1-one-8-yle, 4-{[(2-tert-butoxycarbonylamino-cyclobutanecarbonyl)-amino]-méthyl}-pipéridin-1-yle, 4-{[2-amino-cyclobutane-carbonyl)-amino]-méthyl}-pipéridin-1-yle, ester tert-butylique de l'acide 3,9-diaza-spiro[5.5]undécane-3-carboxylique -9-yle, 4-oxo-1-phényl-1,3,8-triaza-spiro[4.5]déc-8-yle, et 4-oxo-1,3,8-triaza-spiro[4.5]déc-8-yle;
où
le substituant HetR^{a} est un cycle hétérocyclique à 4-7 membres ayant un point d'attachement d'élément de carbone et contenant un élément >NR^{M} en tant qu'élément d'hétéroatome, et ledit élément d'hétéroatome étant séparé dudit point d'attachement d'élément de carbone par au moins un élément de carbone additionnel;
R^{K} est sélectionné dans le groupe consistant en H, alkyle C₁₋₄, -alkylC₀₋₄R^{Ar}, chacun facultativement substitué par 1, 2, ou 3 substituants R^{N}
R^{L} est sélectionné dans le groupe consistant en -CO₂R^{S} et -C(O)NR^{S}R^{S'};
R^{M} est sélectionné dans le groupe consistant en R^{Z}, indol-7-yle, -SO₂R^{Y}, -alkylC₃₋₄CO₂R^{Y}, -CO₂R^{Y}, -C(O)NR^{Z}OR^{Y}, -C(O)R^{Y}, -C(O)alkylC₁₋₄OR^{Y}, -alkylC₀₋₄C(O)NR^{S}R^{S'}, -alkylC₀₋₄C(O)CO₂R^{Y}, 1,3-dihydro-indol-2-one-1-yle, 1,3-dihydro-benzoimidazol-2-one-1-yle, tétrazol-5-yle, 1-R^{Y}-1*H-*tétrazol-5-yle, 1-R^{Y}-triazolyle, 2-R^{Y}-2*H*-tétrazol-5-yle et -alkylC₀₋₄C(O)N(R^{Y}) (SO₂R^{Y}), chacun facultativement substitué par 1,2 ou 3 substituants R^{N};
R^{N} est sélectionné dans le groupe consistant en OCH₃, Cl, F, Br, I, OH, NH₂, CN, CF₃, CH₃, OC(O)CH₃, et NO₂;
R^{P} est sélectionné dans le groupe consistant en R^{Y}, -alkyl-C₂₋₄OR^{Y}, R^{Ar}, -alkylC₁₋₂CO₂R^{Y}, -alkylC₁₋₂CONR^{S}R^{S'}, indol-7-yle, et -SO₂alkyleC₁₋₄;
R^{Q} est sélectionné dans le groupe consistant en fluoro, chloro, bromo, iodo, trifluorométhyle, trichloro-méthyle, -CN, -alkyleC₁₋₄, -alkyleC₀₋₄R^{Ar}, -alkyleC₀₋₄R^{Ar'}, -alkyleC₀₋₄OR^{Y}, -alkyleC₀₋₄CO₂R^{Y}, -alkyleC₀₋₄NR^{Y}R^{Z}, alkyleC₀₋₄NR^{Y}COR^{Y}, alkyle-C₀₋₄NR^{Y}CONR^{Y}R^{Z}, -alkyleC₀₋₄NR^{Y}SO₂R^{Y}, et alkyleC₀₋₄SR^{Y};
R^{S} et R^{S'} sont indépendamment sélectionnés dans le groupe consistant en H, alkyleC₁₋₄, et alkyle C₀₋₄ phényle; alternativement, R^{S} et R^{S'} sont pris ensemble avec l'élément d'azote auquel ledit R^{S} et ledit R^{S'} sont attachés pour former un cycle hétérocyclique à 4-7 membres ayant 0 ou 1 élément hétéroatome additionnel sélectionné dans le groupe consistant en O, S, et >NR^{Y}, à condition que ledit élément hétéroatome additionnel soit séparé par au moins deux éléments de carbone dudit élément d'azote auquel ledit R^{S} et ledit R^{S'} sont attachés et à condition que quand R^{Y} est alkylC₀₋₄^{Ar}, alors R^{Ar} ne soit pas substitué par R^{L};
R^{W} est sélectionné dans le groupe consistant en R^{Y}, et -cycloalkyle-C₃₋₇;
R^{X} est sélectionné dans le groupe consistant en -OR^{Y}, -NR^{Y}R^{Z}, -alkyleC₁₋₄, et -alkylC₀₋₄R^{Ar};
R^{Y} est sélectionné dans le groupe consistant en H, -alkyleC₁₋₄, -alkylC₀₋₄R^{Ar} et -alkylC₀₋₄R^{Ar'}, chacun facultativement substitué par 1, 2 ou 3 substituants R^{N};
R^{Z} est sélectionné dans le groupe consistant en R^{Y}, -alkylC₂₋₄OR^{Y}, -alkyleC₁₋₂CO₂R^{Y}, -alkyleC₁₋₂C(O)NR^{S}R^{S'}, et -alkylC₂₋₄NR^{S}R^{S'};
quand R^{Y} et R^{Z'} sont attachés à un élément d'azote, R^{Y} et R^{Z} sont sélectionnés comme défini ci-dessus, ou bien R^{Y} et R^{Z} sont pris ensemble avec l'élément d'azote attaché à R^{Y} et R^{Z} pour former un cycle hétérocyclique à 4-7 membres HetR^{d} ayant 0 ou 1 élément d'hétéroatome additionnel sélectionné dans le groupe consistant en O, S, et >NR^{M}, ledit cycle hétérocyclique à 4-7 membres HetR^{d} ayant 0 ou 1 élément carbonyle et ledit cycle hétérocyclique à 4-7 membres HetR^{d} ayant 0 ou 1 élément de carbone à valence admise substitué par au moins l'un de R^{M}, -CO₂H, et alkylC₀₋₁OR^{Y};
R^{Ar} est une fraction avec un point d'attachement d'élément carbone et ladite fraction est sélectionnée dans le groupe consistant en phényle, pyridyle, pyrimidyle, et pyrazinyle, où chaque élément de carbone à valence admise dans chacune desdites fractions est indépendamment substitué par au moins l'un de 0, 1, 2 ou 3 R^{N}, et 0 ou 1 R^{L};
R^{Ar'} est un cycle à 3-8 membres ayant 0, 1 ou 2 éléments d'hétéroatomes sélectionnés dans le groupe consistant en O, S, N, et >NR^{Y}, ayant, 0, 1, ou 2 liaisons insaturées, ayant 0 ou 1 éléments carbonyle, où chaque élément à valence admise dans chacun desdits cycles est indépendamment substitué par 0, 1, ou 2 R^{K}; et
R^{f} est une fraction d'hydrocarbure linéaire à 3 à 5 membres ayant 0 ou 1 liaison carbone-carbone insaturée ayant 0 ou 1 éléments carbonyle;
à condition que
(a) au moins l'un dudit R^{2'} et R^{3'} ne soit pas éthyle quand une sélection dans le groupe consistant en sélections (s1), (s2), (s3), et (s4), est satisfaite, et chacune desdites sélections est spécifiée par
(s1): R⁴ est H, Z est O, W est CH₂, Y est CH₂, et X est S;
(s2): R⁴ est H, Z est O, W est CH₂, Y est CH₂, et X est NH;
(s3): R⁴ est H, Z est O, W est CH₂, Y est O, et X est S;
(s4): R⁴ est 5-chloro, Z est O, W est CH₂, Y est CH₂, et X est S;
(b) à condition de plus que quand Z est une liaison, Y est CH₂, W est CHR¹-CH², R¹ est H, et l'un de R^{2'} et R^{3'} est 1*H-*imidazol-2-yle, alors l'autre de R^{2'} et R^{3'} soit sélectionné parmi A1), B)-L), où B)-L) sont tels que définis ci-dessus pour le composé de formule (I), et A1) consiste en H, alcényle C₃₋₇, où le carbone dans ledit alkényle C₃₋₇ qui est attaché à l'élément d'azote n'a que de simples liaisons, alkynyle C₃₋₇ où le carbone dans ledit alkynyle qui est attaché à l'élément d'azote n'a que de simples liaisons, cycloalkyleC₃₋₇ facultativement benzofusionné, cycloalcényle C₅₋₇, -cyclalkyl -C₃₋₇ alkyle C₁₋₇ -alkyl C₁₋₇ cycloalkyle C₃₋₇; et
(c) à condition de plus que quand X est S, Y est O, Z est une liaison, et W est CH₂, alors l'un de R^{2'} et R^{3'} ne soit pas XCG quand l'autre est alkyleC₁₋₆, où XCG est le groupe 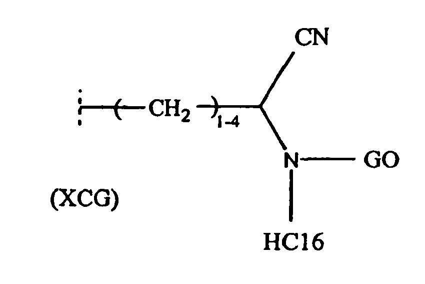
où HC16 est l'un de H, alkyle C₁₋₆, haloalkyleC₁₋₆, allyle et alcoxyC₁₋₆méthyle, et GO est un groupe attaché par un élément de carbone qui a un substituant =O formant un groupe amido avec l'élément d'azote auquel le groupe GO est attaché.

2. Composé de la formule (III): 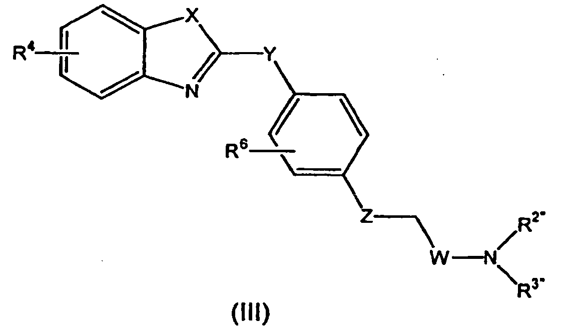 ou un énantiomère, diastéréromère, racémique, tautomère, hydrate, solvate, ou un sel, ester, ou amide pharmaceutiquement acceptable de celui-ci,
où
X est sélectionné dans le groupe consistant en NR⁵, O, et S, avec R⁵ étant l'un de H et CH₃;
Y est sélectionné dans le groupe consistant en CH₂, et O;
Z est sélectionné dans le groupe consistant en O et une liaison;
W est sélectionné dans le groupe consistant en CH₂ et CHR¹ CH₂, avec R¹ étant l'un de H et OH, où l'élément de carbone attaché à R¹- dans ledit CHR¹-CH₂ n'est pas directement attaché à l'élément d'azote auquel est attaché ladite W;
R⁴ est sélectionné dans le groupe consistant en H, OCH₃, Cl, F, Br, I, OH, NH₂, CN, CF₃ et CH₃;
R⁶ est H ou F; et
R^{2"} est défini comme R² et R^{3"} est défini comme R³, comme suit:
R² et R³ sont chacun indépendamment sélectionnés dans le groupe consistant en
A) H, alkyle C₁₋₇, alcényle C₃₋₇, où le carbone dans ledit alcényle qui est attaché à l'élément d'azote n'a que de simples liaisons, alkynyleC₃₋₇, où le carbone dans ledit alkynyle qui est attaché à l'élément d'azote n'a que de simples liaisons, cycloalkyleC₃₋₇ facultativement benzofusionné, cycloalcényleC₅₋₇, -cycloalkylC₃₋₇, -alkyleC₁₋₇, alkyle C₁₋₇ cycloalkyleC₃₋₇ et phényle, où chacun des substituants A) est indépendamment substitué par 0, 1, ou 2 R^{Q}, et chacun dudit R^{Q} est un substituant à un élément de carbone qui est au moins un élément de carbone retiré de l'élément d'azote.
B) un substituant HetR^{a};
C) -alkylC₁₋₇C(O)R^{x}, facultativement substitué par CH₂R^{Ar} ou CH₂R^{Ar'} ;
D) -alkylC₂₋₅C(O)R^{x}, où deux éléments de carbone à valence admise dans l'alkyleC₂₋₅ dudit-alkylC₂₋₅C(O)R^{x} font partie d'un carbocycleC₃₋₆ saturé;
E) alkyle-C₂₋₅OH où deux éléments de carbone à valence admise dans l'alkyleC₂₋₅ dudit alkyleC₂₋₅OH font partie d'un carbocycle C₃₋₆ saturé;
F) -alkylphényle-C₀₋₄, où ledit phényle dans ledit -alkylphényle C₀₋₄ est fusionné à deux éléments adjacents de carbone dans ledit phényle à R^{f}, ou est benzofusionné;
G) -alkyl-C₀₋₄Ar⁶, où Ar⁶ est un hétéroaryle à 6 membres ayant un point d'attachement d'élément de carbone et ayant un ou deux éléments d'hétéroatome -N=, et benzofusionné;
H) -alkylC₀₋₄Ar⁵, où Ar⁵ est un hétéroaryle à 5 membres ayant un élément d'hétéroatome sélectionné dans le groupe consistant en O, S, et >NR^{Y}, et ayant 0 ou 1 élément d'hétéroatome additionnel -N=, contenant facultativement deux groupes carbonyles, et facultativement benzofusionné;
I) -alkylc₁₋₄Ar^{5'}, où Ar^{5'} est un hétéroaryle à 5 membres contenant 3 ou 4 éléments d'azote, facultativement substitués par R^{Y}, et ayant un site à valence admise en un point d'attachement;
J) -alkylC₀₋₄Ar⁶⁻⁶, où Ar⁶⁻⁶ est un phényle attaché à l'alkyle C₀₋₄ fusionné au site à valence admise à un hétéroaryle à 6 membres où ledit hétéroaryle à 6 membres a un ou deux éléments d'hétéroatomes -N=;
K) -alkylC₀₋₄Ar⁶⁻⁵, où Ar⁶⁻⁵ est un phényle attaché à C₀₋₄ fusionné au site de valence admise à un hétéroaryle à 5 membres, ledit hétéroaryle à 5 membres ayant un élément d'hétéroatome sélectionné dans le groupe consistant en O, S, et >NR^{Y}, et ledit hétéroaryle à 5 membres ayant 0 ou 1 élément d'hétéroatome additionnel qui est -N=; et
L) l'un de 2-(4-éthyl-phénoxy)-benzothiazole, 2-(4-éthyl-phénoxy)-benzooxazole, et 2-(4-éthyl-phénoxy)-1*H*-benzoimidazole;
M) SO₂alkyleC₁₋₄;
alternativement R² et R³ sont pris ensemble avec l'azote auquel ils sont attachés pour former un cycle hétérocyclique qui contient au moins un élément d'hétéroatome qui est ledit azote d'attachement, ledit cycle hétérocyclique étant sélectionné dans le groupe consistant en
i) un cycle hétérocyclique à 4 à 7 membres HetR^{b}, ledit cycle hétérocyclique à 4 à 7 membres HetR^{b} ayant un élément hétéroatome qui est l'azote d'attachement et étant substitué par 0, 1, ou 2 substituants aux mêmes éléments de substitution ou en des éléments différents, lesdits substituants étant sélectionnés dans le groupe consistant en -R^{Y}, -CN, -C(O)R^{Y}, alkylC₀₋₄CO₂R^{Y}, -alkylC₀₋₄C(O)CO₂R^{Y}, -alkylC₀₋₄OR^{Y}, -alkylC₀₋₄C (O)NR^{Y}R^{Z}, -alkylC₀₋₄NR^{Y}C(O)R^{Z}, -C(O)NR^{Z}OR^{Y}, -alkylC₀₋₄NR^{Y}C(O)CH₂OR^{Y}, -alkylC₀₋₄NR^{Y}C(O)CH₂C(O), -alkyl-C₀₋₄NR^{Y}CO₂R^{Y}, -alkyl-C₀₋₄NR^{Y}C(O)NR^{Y}R^{Z}, -alkyl C₀₋₄NR^{Y}C(S)NR^{Y}R^{Z}, -NR^{Y}C(O)CO₂R^{Y}, -NR^{Y}R^{Z}, alkyle-C₀₋₄N R^{W}SO_{2R}^{Y}, 1,3-dihydro-indol-2-one-1-yle, 1,3-dihydro-benzoimidazol-2-one-1-yle, tétrazol-5-yle, 1-R^{Y}-1*H*-tétrazol-5-yle, R^{Y}-triazolyle, 2-R^{Y}-2H-tétrazol-5-yle, pyrrolidine-2-thion-1-yle, pipéridine-2-thion-1-yle, -alkylC₀₋₄C(O)N(R^{Y})(SO₂ R^{Y}), -alkylC₀₋₄N(R^{Y})(SO₂)NR^{Y}R^{Y}, -C₀₋₄alkylN(R^{Y})(SO₂) NR^{Y}CO₂R^{Y}, halo, 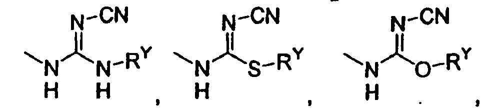 et 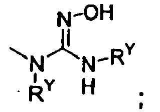
ii) un cycle hétérocyclique à 5-7 membres HetR^{c}, ledit cycle hétérocyclique à 5-7 HetR^{c} ayant un élément hétéroatome additionnel séparé dudit azote d'attachement par au moins un élément de carbone, ledit élément d'hétéroatome additionnel étant sélectionné dans le groupe consistant en O, S(=O)₀₋₂, et >NR^{M}, ledit cycle hétérocyclique à 5-7 membres HetR^{c} ayant 0 ou 1 élément de carbonyle, et étant substitué par 0, 1, ou 2 substituants aux mêmes éléments ou en des éléments différents de substitution de carbone, lesdits substituants étant sélectionnés dans le groupe consistant en -C (O)R^{Y}, -CO₂R^{Y}, -alkylC₃₋₄CO₂R^{Y} et R^{Z};
iii) l'un de imidazolidin-1-yle, 2-imidazolin-1-yle, pyrazol-1-yle, imidazol-1-yle, 2H-tétrazol-2-yle, 1*H*-tétrazol-1-yle, pyrrol-1-yle, 2-pyrrolin-1-yle, et 3-pyrrolin-1-yle, où chacun dudit 2*H*-tétrazol-2-yle et 1H-tétrazol-1-yle est substitué à un élément de carbone par 0 ou 1 de -alkylC₀₋₄R^{Z}, -alkylC₀₋₄SR^{Y}, -alkylC₀₋₄CO₂R^{Y}, et le substituant HetR^{a}; et
iv) l'un de 1,2,3,4-tétrahydro-quinoléin-1-yle, 1,2,3,4-tétrahydro-isoquinoléin-2-yle, indol-1-yle, isoindol-2-yle, indolin-1-yle, benzi-midazol-1-yle, 2,8-diaza-spiro[4.5]décan-1-one-8-yle, 4-{[(2-tert-butoxycarbonylamino-cyclobutanecarbonyl)-amino]-méthyl}-pipéridin-1-yle, 4-{[(2-amino-cyclobutanecar-bonyl)-amino]-méthyl}-pipéridin-1-yle, ester tert-butylique de l'acide 3,9-diaza-spiro[5.5]undécane-3-carboxylique -9-yle, 4-oxo-1-phényl-1,3,8-triaza-spiro[4.5]déc-8-yle, et 4-oxo-1,3,8-triaza-spiro[4.5]déc-8-yle;
où
le substituant HetR^{a} est un cycle hétérocyclique à 4-7 membres ayant un poids d'attachement d'élément de carbone et contenant un élément >NR^{M} en tant qu'élément d'hétéroatome, et ledit élément d'hétéroatome étant séparé dudit point d'attachement d'élément de carbone par au moins un élément de carbone additionnel;
R^{K} est sélectionné dans le groupe consistant en H, -alkyl C₁₋₄, -alkylC₀₋₄R^{Ar} chacun facultativement substitué par 1, 2, ou 3 substituants R^{N}
R^{L} est sélectionné dans le groupe consistant en -CO₂R^{S} et -C(O)NR^{S}R^{S'};
R^{M} est sélectionné dans le groupe consistant en R^{Z}, indol-7-yle, -SO₂R^{Y}, -alkylC₃₋₄CO₂R^{Y}, -CO₂R^{Y}, -C(O)NR^{Z}OR^{Y}, -C(O)R^{Y}, -C(O)alkylC₁₋₄OR^{Y}, -alkylC₀₋₄C(O)NR^{S}R^{S}', alkylC₀₋₄C(O)CO₂R^{Y}, 1,3-dihydro-indol-2-one-1-yle, 1,3-dihydro-benzoimidazol-2-one-1-yle, tétrazol-5-yle, R^{Y}-1*H-*tétrazol-5-yle, 1-R^{Y}-triazolyle, 2-R^{Y}-2*H*-tétrazol-5-yle et -alkylC₀₋₄C(O)N(R^{Y})(SO₂R^{Y}), chacun facultativement substitué par 1,2 ou 3 substituants R^{N};
R^{N} est sélectionné dans le groupe consistant en OCH₃, Cl, F, Br, I, OH, NH₂, CN, CF₃, CH₃, OC(O)CH₃, et NO₂;
R^{P} est sélectionné dans le groupe consistant en R^{Y}, -alkylC₂₋₄OR^{Y}, R^{Ar}, -alkylC₁₋₂CO₂R^{Y}, -alkylC₁₋₂CONR^{S}R^{S'}, indol-7-yle, et -SO₂alkyleC₁₋₄;
R^{Q} est sélectionné dans le groupe consistant en fluoro, chloro, bromo, iodo, trifluorométhyle, trichloro-méthyle, -CN, -alkyleC₁₋₄, -alkylC₀₋₄R^{Ar}, -alkylC₀₋₄R^{Ar'}, -alkylC₀₋₄OR^{Y}, -alkylC₀₋₄CO₂R^{Y}, -alkylC₀₋₄NR^{Y}R^{Z}, alkylC₀₋₄NR^{Y}COR^{Y}, -alkylC₀₋₄NR^{Y}CONR^{Y}R^{Z}, -alkylC₀₋₄NR^{Y}SO₂R^{Y}, et -alkylC₀₋₄SR^{Y};
R^{S} et R^{S'} sont indépendamment sélectionnés dans le groupe consistant en H, alkyleC₁₋₄, et alkyl C₀₋₄ phényle; alternativement, R^{S} et R^{S'} sont pris ensemble avec l'élément d'azote auquel ledit RS et ledit RS' sont attachés pour former un cycle hétérocyclique à 4-7 membres ayant 0 ou 1 élément hétéroatome additionnel sélectionné dans le groupe consistant en O, S, et >NR^{Y}, à condition que ledit élément hétéroatome additionnel soit séparé par au moins deux éléments de carbone dudit élément d'azote auquel ledit R^{S} et ledit R^{S'} sont attachés et à condition que quand R^{Y} est alkyleC₀₋₄ R^{Ar}, alors R^{Ar} ne sont pas substitués par R^{L};
R^{W'} est sélectionné dans le groupe consistant en R^{Y}, et -cycloalkyleC₃₋₇;
R^{X} est sélectionné dans le groupe consistant en -OR^{Y}, -NR^{Y}R^{Z}, -alkyleC₁₋₄, et -alkylC₀₋₄R^{Ar};
R^{Y} est sélectionné dans le groupe consistant en H, alkyle-C₁₋₄, -alkylC₀₋₄R^{Ar} et -alkylC₀₋₄R^{Ar'}, chacun facultativement substitué par 1, 2 ou 3 substituants R^{N};
R^{Z} est sélectionné dans le groupe consistant en R^{Y}, -alkylC₂₋₄OR^{Y}, -alkylC₁₋₂CO₂R^{Y}, alkyl-C₁₋₂C(O)NR^{S}R^{S'}, et -alkylC₂₋₄NR^{S}R^{S'};
quand R^{Y} et R^{Z'} sont attachés à un élément d'azote, R^{Y} et R^{Z} sont sélectionnés comme définis ci-dessus, ou bien R^{Y} et R^{Z} sont pris ensemble avec l'élément d'azote attaché à R^{Y} et R^{Z} pour former un cycle hétérocyclique à 4-7 membres HetR^{d} ayant 0 ou 1 élément d'hétéroatome additionnel sélectionné dans le groupe consistant en O, S, et >NR^{M}, ledit cycle hétérocyclique à 4-7 membres HetR^{d} ayant 0 ou 1 éléments carbonyles et ledit cycle hétérocyclique à 4-7 membres HetR^{d} ayant 0 ou 1 élément de carbone à valence admise substitués par au moins l'un de R^{M}, -CO₂H, et alkyle-C₀₋₁OR^{Y};
R^{Ar} est une fraction avec un point d'attachement d'élément carbone et ladite fraction est sélectionnée dans le groupe consistant en phényle, pyridyle, pyrimidyle, et pyrazinyle, où chaque élément de carbone à valence admise dans chacune desdites fractions est indépendamment substitué par au moins l'un de 0, 1, 2 ou 3 R^{N}, et 0 ou 1 R^{L};
R^{Ar'} est un cycle à 3-8 membres ayant 0, 1 ou 2 éléments d'hétéroatome sélectionnés dans le groupe consistant en O, S, N, et >NR^{Y}, ayant, 0, 1, ou 2 liaisons insaturées, ayant 0 ou 1 éléments carbonyle, où chaque élément à valence admise dans chacun desdits cycles est indépendamment substitué par 0, 1, ou 2 R^{K}; et
R^{f} est une fraction d'hydrocarbure linéaire à 3 à 5 membres ayant 0 ou 1 liaison carbone-carbone insaturée ayant 0 ou 1 éléments carbonyle;
à condition que
(a) lesdits R^{2"} et R^{3"} satisfasse de plus l'un de ce qui suit:
(e1): au moins l'un de R^{2"} et R^{3"} n'est pas alkyle C₁₋₅, quand Z est O et X est S;
(e2): aucun de R^{2"} et R^{3"} n'est alkyleC₁₋₄C(O)R^{x}, avec R^{x} étant l'un de alkyleC₁₋₄, OH, -OalkyleC₁₋₄, -alkyl C₀₋₄Ar, ou -NR^{Y}R^{Y}, quand Y est O, Z est une liaison, et R^{2"} est différent de R^{3"}; et
(e3): aucun de R^{2"} et R^{3"} n'est alkylC₁₋₆CN, quand Y est O, Z est une liaison, et R^{2"} est différent de R^{3"}; et
(b) à condition de plus que quand X est S, Y est O, Z est une liaison et W est CH₂, alors l'un de R^{2"} et R^{3"} ne soit pas XCG quand l'autre est alkyleC₁₋₆, où XCG est le groupe 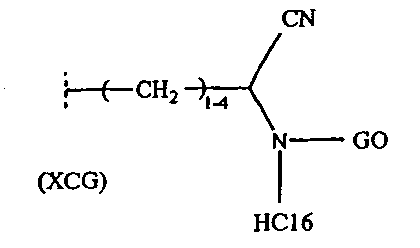
où HC16 est l'un de H, alkyleC₁₋₆, haloalkyleC₁₋₆, allyle, et alcoxyC₁₋₆méthyle, et GO est un groupe attaché par un élément de carbone qui a un substituant =O formant un groupe amido avec l'élément d'azote auquel le groupe GO est attaché.

3. Composé selon la revendication 1 où ledit R⁴ est H.

4. Composé selon la revendication 2, où ledit R⁴ est H.

5. Composé selon la revendication 1, où ledit R² et R³ sont chacun indépendamment sélectionnés parmi
(i) le groupe consistant en A), B), C), D), E), et I) quand R² et R³ sont sélectionnés parmi différents groupes; ou
(ii) le groupe A); ou
(iii)le groupe B); ou
(iv) le groupe C); ou
(v) le groupe D); ou
(vi) le groupe E); ou
(vii)le groupe I),
comme défini dans la revendication 1.

6. Composé selon la revendication 2, où lesdits R² et R³ sont chacun indépendamment sélectionnés parmi
(i) le groupe consistant en A), B), C), D), E), et I) quand R² et R³ sont sélectionnés parmi différents groupes; ou
(ii) le groupe A); ou
(iii)le groupe B); ou
(iv) le groupe C); ou
(v) le groupe D); ou
(vi) le groupe E); ou
(vii)le groupe I),
comme défini à la revendication 2.

7. Composé selon la revendication 1 où ledit R² et R³ sont pris avec l'azote auquel ils sont attachés pour former un cycle hétérocyclique qui contient au moins un élément d'hétéroatome qui est ledit azote d'attachement, ledit cycle hétérocyclique étant sélectionné parmi l'un de
(a) le groupe consistant en i) et ii) quand R² et R³ sont sélectionnés dans différents groupes;
(b) le groupe i), et
(c) le groupe ii)
comme défini à la revendication 1.

8. Composé selon la revendication 2 où lesdits R² et R³ sont pris ensemble avec l'azote auquel ils sont attachés pour former un cycle hétérocyclique qui contient au moins un élément d'hétéroatome qui est ledit azote d'attachement, ledit cycle hétérocyclique étant sélectionné parmi l'un de
(d) le groupe consistant en i) et ii) quand R² et R³ sont sélectionnés dans différents groupes;
(e) le groupe i), et
(f) le groupe ii)
tel que défini à la revendication 2.

9. Composé selon la revendication 1, où ledit composé de formule (II) est l'un de:
2-[4-(2-Pipéridin-1-yl-éthoxy)-phénoxy]-benzooxazole;
(1-{2-[4-(Benzooxazol-2-yloxy)-phénoxy]éthyl}-pipéridin-4-yl)-méthanol;
1-{2-[4-(Benzooxazol-2-yloxy)-phénoxy]-éthyl}-pipéridin-4-ol;
{2-[4-(Benzooxazol-2-yloxy)-phénoxy]-éthyl}-dibutyl-amine;
(1-{2-[4-(Benzooxazol-2-yloxy)-phénoxy]-éthyl)-pipéridin-2-yl)-méthanol;
1-(3-[4-(Benzooxazol-2-yloxy)-phénoxy]-propyl}-4-phényl-pipéridin-4-ol;
1-{3-[4-(Benzooxazol-2-yloxy)-phénoxy]-propyl}-4-benzyl-pipéridin-4-ol;
2-[4-(2-Pipéridin-1-yl-éthyl)-phénoxy]-benzooxazole;
{3-[4-(Benzooxazol-2-yloxy)-phényl]-propyl}-cyclohexyl-éthyl-amine;
1-{3-[4-(Benzooxazol-2-yloxy)-phényl]-propyl}-pipéridin-4-ol;
1-{3-[4-(Benzooxazol-2-yloxy)-phénoxy]-2-hydroxy-propyl}-4-phényl-pipéridin-4-ol;
Ester de l'acide 1-[2-(4-benzooxazol-2-ylméthyl-phénoxy)-éthyl]-pipéridine-4-carboxylique;
2-[4-(2-Pyrrodin-1-yl-éthoxy)-phénoxy]-benzooxazole;
{3-[4-(Benzooxazol-2-yloxy)-phénoxy]-propyl}-diméthyl-amine;
{2-[4-(Benzooxazol-2-yloxy)-phénoxy]-éthyl}-diméthyl-amine; et
2-[4-(2-Azépan-1-yl-éthoxy)-phénoxy]-benzooxazole.

10. Composé selon la revendication 1, où ledit composé de formule (II) est l'un de:
1-(2-[4-(Benzooxazol-2-yloxy)-phénoxy]-éthyl}-4-phényl-pipéridin-4-ol;
{2-[4-(Benzooxazol-2-yloxy)-phénoxy]-éthyl}-cyclohexyl-éthyl-amine;
2-{4-[2-(2-Ethyl-pipéridin-1-yl)-éthoxy]-phénoxy}-benzooxazole;
1-{2-[4-(Benzooxazol-2-yloxy)-phénoxy]-éthyl}-4-phényl-pipéridine-4-carbonitrile;
1-(1-{2-[4-(Benzooxazol-2-yloxy)-phénoxy]-éthyl}-4-phényl-pipéridin-4-yl)-éthanone.
2-{4-[2-(4-Méthyl-pipéridin-1-yl)-éthoxy]-phénoxy}-benzooxazole;
1-(2-[4-(Benzooxazol-2-yloxy)-phénoxy]-éthyl}-4-(4-chloro-phényl)-pipéridin-4-ol;
1-{2-[4-(Benzooxazol-2-yloxy)-phénoxy]-éthyl}-4-(4-bromo-phényl)-pipéridin-4-ol;
1-(2-[4-(Benzooxazol-2-yloxy)-phénoxy]-éthyl}-4-(4-chloro-3-trifluorométhyl-phényl)-pipéridin-4-ol;
1-(2-[4-(Benzooxazol-2-yloxy)-phénoxy]-éthyl}-4-benzyl-pipéridin-4-ol;
{2-[4-(Benzooxazol-2-yloxy)-phénoxy]-éthyl}-cyclohexyl-méthyl-amine; et
{2-[4-(Benzooxazol-2-yloxy)-phénoxy]-éthyl}-cyclopropylméthyl-propyl-amine.

11. Composé selon la revendication 1, où ledit composé de formule (II) est l'un de:
{2-[4-(Benzooxazol-2-yloxy)-phénoxy]-éthyl}-butyl-éthyl-amine;
2-({2-[4-(Benzooxazol-2-yloxy)-phénoxy]-éthyl}-benzylamino)-éthanol;
2-{4-[2-(4-Benzyl-pipéridin-1-yl)-éthoxy]-phénoxy}-benzooxazole;
(1-{2-[4-(Benzooxazol-2-yloxy)-phénoxy]-éthyl}-pipéridin-3-yl)-méthanol;
2-({2-[4-(Benzooxazol-2-yloxy)-phénoxy]-éthyl}-propylamino)-éthanol;
2-[4-(2-Azetidin-1-yl-éthoxy)-phénoxy]-benzooxazole;
*N*-(1-{2-[4-(Benzooxazol-2-yloxy)-phénoxy]-éthyl}-pipéridin-4-yl)-2-phényl-acétamide;
Ester éthylique de l'acide 1-{2-[4-(benzooxazol-2-yloxy)-phénoxy]-éthyl}-pipéridine-3- carboxylique;
2-{4-[3-(4-Phényl-pipéridin-1-yl)-propoxy]-phénoxy}-benzooxazole;
1-{2-[4-(Benzooxazol-2-yloxy)-phényl]-éthyl}-4-phényl-pipéridin-4-ol;
{2-[4-(Benzooxazol-2-yloxy)-phényl]-éthyl}-cyclohexyl-éthyl-amine;
2-[4-(2-Pyrrolidin-1-yl-éthyl)-phénoxy]-benzooxazole; et
2-[4-(2-Azépan-1-yl-éthyl)-phénoxy]-benzooxazole.

12. Composé selon la revendication 1, où ledit composé de formule (II) est l'un de:
{2-[4-(Benzooxazol-2-yloxy)-phényl]-éthyl}-cyclopropyméthyl-propyl-amine;
{2-[4-(Benzooxazol-2-yloxy)-phényl]-éthyl}-dibutyl-amine;
1-{2-[4-(Benzooxazol-2-yloxy)-phényl]-éthyl}-pipéridin-4-ol;
Ester méthylique de l'acide 1-{2-[4-(benzooxazol-2-yloxy)-phényl]-éthyl}-pipéridine-4- carboxylique;
1-{3-[4-(Benzooxazol-2-yloxy)-phényl]-propyl}-4-phényl-pipéridin-4-ol;
2-[4-(3-Piperidin-1-yl-propyl)-phénoxy]-benzooxazole;
{3-[4-(Benzooxazol-2-yloxy)-phényl]-propyl}-dibutyl-amine;
{3-[4-(Benzooxazol-2-yloxy)-phényl]-propyl}-cyclopropylméthyl-propyl-amine;
1-[4-(Benzooxazol-2-yloxy)-phénoxy]-3-pyrrolidin-1-yl-propan-2-ol;
1-[2-(4-Benzooxazol-2-ylméthyl-phénoxy)-éthyl]-4-phényl-pipéridin-4-ol;
Amide de l'acide 1-[2-(4-benzooxazol-2-ylméthyl-phénoxy)-éthyl]-pipéridine-4-carboxylique
2-[4-(2-Morpholin-4-yl-éthoxy)-phénoxy]-benzooxazole; et
{2-[4-(Benzooxazol-2-yloxy)-phénoxy]-éthyl}-diéthyl-amine.

13. Composé selon la revendication 1, où ledit composé de formule (II) est l'un de:
{2-[4-(6-Chloro-benzothiazol-2-yloxy)-phénoxy]-éthyl}-diéthyl-amine;
1-{2-[4-(Benzothiazol-2-yloxy)-phénoxy]-éthyl}-pipéridin-4-ol;
Ester éthylique de l'acide 1-{2-[4-(benzothiazol-2-yloxy)-phénoxy]-éthyl}-pipéridine-4-carboxylique;
Acide 1-{2-[4-(benzothiazol-2-yloxy)-phénoxy]-éthyl)-pipé-ridine-4-carboxylique;
(1-{2-[4-(Benzothiazol-2-yloxy)-phénoxy]-éthyl}-pipéridin-4-yl)-pyrrolidin-1-yl-méthanone;
Ester éthylique de l'acide 3-[(1-{2-[4-(benzothiazol-2-yloxy)-phénoxy]-éthyl)-pipéridine-4-carbonyl)-amino]-propionique;
amide de l'acide 1-{2-[4-(benzothiazol-2-yloxy)-phénoxy]-éthyl}-pipéridine-4-carboxylique;
1-(1-{2-[4-(Benzothiazol-2-yloxy)-phénoxy]-éthyl}-pipéridin-4-yl)-pyrrolidin-2-one;
1'-{2-[4-(Benzothiazol-2-yloxy)-phénoxy]-éthyl}-[1,4']bipipéridinyl-2-one;
8-{2-[4-(Benzothiazol-2-yloxy)-phénoxy]-éthyl}-2,8-diaza-spiro[4,5]décan-1-one;
2-[4-(3-Pyrrolidin-1-yl-propoxy)-phénoxy]-benzothiazole;
{2-[4-(Benzothiazol-2-yloxy)-phényl]-éthyl}-cyclohexyl-éthyl-amine;
Amide de l'acide 1-{2-[4-(benzothiazol-2-yloxy)-phényl]-éthyl}-pipéridine-3-carboxylique;
1-(1-{2-[4-(Benzothiazol-2-yloxy)-phényl]-éthyl}-pipéridin-4-yl)-3-méthyl-1,3-dihydro-benzoimidazol-2-one;
Ester méthylique de l'acide 1-{2-[4-(benzothiazol-2-yloxy)-phényl]-éthyl}-pipéridine-4-carboxylique;
(1-{2-[4-(Benzothiazol-2-yloxy)-phényl]-éthyl}-pipéridin-4-yl)-(4-méthyl-pipérazin-1-yl)-méthanone;
Ester méthylique de l'acide 1-[2-(4-benzothiazol-2-ylméthyl-phénoxy)-éthyl]-pipéridine-4-carboxylique;
Acide 3-({2-[4-(benzothiazol-2-yloxy)-phénoxy]-éthyl}-cyclopropyl-amino)-propionique;
{2-[4-(Benzothiazol-2-yloxy)-phénoxy]-éthyl}-diméthyl-amine;
2-[4-(2-Pyrrolidin-1-yl-éthoxy)-phénoxy]-benzothiazole;
{3-[4-(Benzothiazol-2-yloxy)-phénoxy]-propyl}-diméthyl-amine;
2-[4-(2-Azépan-1-yl-éthoxy)-phénoxy]-benzothiazole;
2-[4-(2-Azépan-1-yl-éthoxy)-phénoxy]-6-méthoxy-benzothiazole;
1-(2-[4-(Benzothiazol-2-yloxy)-phénoxy]-éthyl}-4-phényl-pipéridin-4-ol;
{2-[4-(Benzothiazol-2-yloxy)-phénoxy]-éthyl}-cyclohexyl-éthyl-amine;
1-{2-[4-(Benzothiazol-2-yloxy)-phénoxy]-éthyl}-4-(4-chloro-phényl)-pipéridin-4-ol;
{2-[4-(Benzothiazol-2-yloxy)-phénoxy]-éthyl}-dibutyl-amine;
1-{2-[4-(Benzothiazol-2-yloxy)-phénoxy]-éthyl}-4-(4-bromo-phényl)-pipéridin-4-ol;
1-{2-[4-(Benzothiazol-2-yloxy)-phénoxy]-éthyl}-4-(4-chloro-3-trifluorométhyl-phényl)-pipéridin-4-ol;
1-{2-[4-(Benzothiazol-2-yloxy)-phénoxy]-éthyl}-4-benzyl-pipéridin-4-ol;
1'-{2-[4-(Benzothiazol-2-yloxy)-phénoxy]-éthyl-}-[1,4']bipipéridine;
(1-{2-[4-(Benzothiazol-2-yloxy)-phénoxy]-éthyl}-pipéridin-4-yl)-méthanol;
*N*-(1-{2-[4-(Benzothiazol-2-yloxy)-phénoxy]-éthyl}-pipéridin-4-yl)-2-phényl-acétamide;
2-(4-{2-[4-(2-Morpholin-4-yl-éthyl)-pipérazin-1-yl]-éthoxy}-phénoxy)-benzothiazole;
2-(4-{2-[4-(2-Morpholin-4-yl-éthyl)-pipérazin-1-yl]-éthyl}-phénoxy)-benzothiazole;
1-{3-[4-(Benzothiazol-2-yloxy)-phénoxy]-propyl}-4-phényl-pipéridin-4-ol;
1-{2-[4-(Benzothiazol-2-yloxy)-phényl]-éthyl}-4-phényl-pipéridin-4-ol;
2-[4-(2-Pyrrolidin-1-yl-éthyl)-phénoxy]-benzothiazole;
2-[4-(2-Azépan-1-yl-éthyl)-phénoxy]-benzothiazole;
{2-[4-(Benzothiazol-2-yloxy)-phényl]-éthyl}-cyclopropyl-méthyl-propyl-amine;
{2-[4-(Benzothiazol-2-yloxy)-phényl]-éthyl}-dibutyl-amine;
2-[4-(2-Pipéridin-1-yl-éthyl)-phénoxy]-benzothiazole;
1-(2-[4-(Benzothiazol-2-yloxy)-phényl]-éthyl}-pipéridin-4-ol;
Amide de l'acide 1-{2-[4-(benzothiazol-2-yloxy)-phényl]-éthyl}-pipéridine-4-carboxylique;
Ester éthylique de l'acide 1-(2-[4-(benzothiazol-2-yloxy)-phényl]-éthyl}-pipéridine-3-carboxylique;
Ester éthylique de l'acide 1-(2-[4-(benzothiazol-2-yloxy)-phényl]-éthyl}-4-phényl-pipéridine-4- carboxylique;
Ester éthylique de l'acide (1-{2-[4-(benzothiazol-2-yloxy)-phényl]-éthyl]-pipéridin-4-yl)acétique;
1-(1-{2-[4-(Benzothiazol-2-yloxy)-phényl]-éthyl}-pipéridin-4-yl)-1,3-dihydro-indol-2-one;
1-(1-[2-[4-(Benzothiazol-2-yloxy)-phényl]-éthyl}-pipéridin-4-yl)-pyrrolidin-2-one;
*N*-(1-{2-[4-(Benzothiazol-2-yloxy)-phényl]-éthyl}-pipéridin-4-yl)-phényl-acétamide;
8-{2-[4-(Benzothiazol-2-yloxy)-phényl]-éthyl}-2,8-diaza-spiro[4.5]décan-1-one;
1-{2-[4-(Benzothiazol-2-yloxy)-phényl]-éthyl}-pipéridin-3-ol;
Ester éthylique de l'acide 1-{2-[4-(benzothiazol-2-yloxy)-phényl]-éthyl}-pipéridine-4 carboxylique;
1'-{2-[4-(Benzothiazol-2-yloxy)-phényl]-éthyl}-[1,4']bipipéridine;
2-(4-[2-(4-Méthyl-pipérazin-1-yl)-éthyl]-phénoxy}-benzothiazole;
2-(4-{2-[4-(1-Benzyl-1*H*-tétrazol-5-yl)-pipéridin-1-yl]-éthoxy}-phénoxy)-benzothiazole;
Ester *tert*-butylique de l'acide 4-(1-{2-[4-(benzothiazol-2-yloxy)-phényl]-éthyl}-pipéridine-4-carbonyl)-pipérazine-1-carboxylique;
Amide de l'acide 1-[2-(4-benzothiazol-2-ylméthyl-phénoxy)-éthyl]-pipéridine-4-carboxylique;
1-{1-[2-(4-Benzothiazol-2-ylméthyl-phénoxy)-éthyl]-pipéridin-4-yl}-pyrrolidin-2-one;
1-[4-(1-{2-[4-(Benzothiazol-2-yloxy)-phényl]-éthyl}-pipéridine-4-carbonyl)-pipérazin-1-yl]-éthanone;
Acide 1-{2-[4-(benzothiazol-2-yloxy)-phényl]-éthyl}-pipéridine-4-carboxylique;
1-(1-{2-[4-(Benzothiazol-2-yloxy)-phénoxy]-éthyl}-pipéridin-4-yl)-pyrrolidine-2-thione;
2-(4-{2-[4-(Benzothiazol-2-yloxy)-phénoxy]-éthyl}-pipérazin-1-yl)-éthanol;
2-(4-{2-[4-(Benzothiazol-2-yloxy)-phénoxy]-éthyl}-pipérazin-1-yl)-1-pyrrolidin-1-yl-éthanone;
2-(4-{2-[4-(Benzothiazol-2-yloxy)-phénoxy]-éthyl}-pipérazin-1-yl)-1-morpholin-4-yl-éthanone;
Acide 1-{2-[4-(benzothiazol-2-yloxy)-phényl]-éthyl}-pipéridine-3-carboxylique;
Acide 1-{2-[4-(benzothiazol-2-yloxy)-phényl]-éthyl}-pipéridine-2-carboxylique;
Acide (1-{2-[4-(benzothiazol-2-yloxy)-phényl]-éthyl}-pipéridin-3-yl)acétique;
Ester éthylique de l'acide (1-{2-[4-(benzothiazol-2-yloxy)-phénoxy]-éthyl}-pipéridin-4-yl) acétique;
Ester tert-butylique de l'acide (1-{2-[4-(benzothiazol-2-yloxy)-phénoxy]-éthyl}-pipéridin-4-yl)-carbamique;
Acide (1-{2-[4-(benzothiazol-2-yloxy)-phénoxy]-éthyl}-pipéridin-4-yl)-acétique;
(1-{2-[4-(Benzothiazol-2-yloxy)-phényl]-éthyl}-pipéridin-3-yl)-méthanol;
Ester méthylique de l'acide ({2-[4-(benzothiazol-2-yloxy)-phényl]-éthyl}-cyclohexyl-amino)-acétique;
Acide (4-{2-[4-(Benzothiazol-2-yloxy)-phénoxy]-éthyl}-pipérazin-1-yl)-acétique;
ester éthylique de l'acide 1-(1-{2-[4-(benzothiazol-2-yloxy)-phénoxy]-éthyl}-pipéridin-4-yl)-5-oxo-pyrrolidine-2-carboxylique;
acide 1-(1-[2-[4-(benzothiazol-2-yloxy)-phénoxy]-éthyl}-pipéridin-4-yl)-5-oxo-pyrrolidine-2-carboxylique;
4-(4-{2-[4-(Benzothiazol-2-yloxy)-phénoxy]-éthyl}-pipérazin-1-yl)-phénol;
*N*-(1-{2-[4-(Benzothiazol-2-yloxy)-phénoxy]-éthyl}-pipéridin-4-yl)-4-chloro-N-cyclopropyl-benzène sulfonamide;
Acide 3-({2-[4-(benzothiazol-2-yloxy)-phénoxy]-éthyl)-cyclopropylméthyl-amino)-propionique;
Acide 3-({2-[4-(benzothiazol-2-yloxy)-phénoxy]-éthyl}-isopropyl-amino)-propionique;
1-{2-[4-(Benzothiazol-2-yloxy)-phénoxy]-éthyl}-pipéridin-4-ylamine;
Acide 3-[{2-[4-(benzothiazol-2-yloxy)-phénoxy]-éthyl}-(1-méthyl-pipéridin-4-y)-amino]-propionique;
Acide 3-({2-[4-(benzothiazol-2-yloxy)-phénoxy]-éthyl}-benzyl-amino)-propionique;
Acide 3-((1-acétyl-pipéridin-4-yl)-{2-[4-(benzothiazol-2-yloxy)-phénoxy]-éthyl}-amino)-propionique;
Ester tert-butylique de l'acide 4-(1-{2-[4-(benzothiazol-2-yloxy)-phénoxy]-éthyl}-1H-tétrazol-5-yl)-pipéridine-1-carboxylique;
Acide 3-({2-[4-(benzothiazol-2-yloxy)-phényl]-éthyl}-cyclopropyl-amino)-propionique;
Acide 3-((1-acétyl-pipéridin-4-yl)-{2-[4-(benzothiazol-2-yloxy)-phényl]-éthyl}-amino)-propionique;
Acide 3-[{2-[4-(benzothiazol-2-yloxy)-phényl]-éthyl}-(1-méthyl-pipéridin-4-yl)-amino]-propionique;
Acide 3-({2-[4-(benzothiazol-2-yloxy)-phényl]-éthyl}-cyclopropylméthyl-amino)-propionique;
Acide 3-({2-[4-(benzothiazol-2-yloxy)-phényl]-éthyl}-isopropyl-amino)-propionique;
2-(4-{2-[4-(Benzothiazol-2-yloxy)-phényl]-éthyl}-pipérazin-1-yl)-1-pyrrolidin-1-yl-éthanone;
Acide (*R*)-1-{2-[4-(benzothiazol-2-yloxy)-phényl]-éthyl}-pipéridine-3-carboxylique;
1-(1-{2-[4-(Benzothiazol-2-yloxy)-phényl]-éthyl}-pipéridin-4-yl)-1,3-dihydro-benzoimidazol-2-one;
2-(4-{2-[4-(6-Méthyl-pyridin-2-yl)-pipérazin-1-yl]-éthyl}-phénoxy)-benzothiazole;
2-(4-{2-[4-Ethanesulfonyl-pipérazin-1-yl)-éthyl]-phénoxy}-benzothiazole;
2-(4-{2-[4-(Benzothiazol-2-yloxy)-phényl]-éthyl}-pipérazin-1-yl)-1-morpholin-4-yl-éthanone;
Acide 3-({2-[4-(benzothiazol-2-yloxy)-phényl]-éthyl}-méthyl-amino)-propionique;
Acide 3-({2-[4-(benzothiazol-2-yloxy)-phényl]-éthyl}-cyclopentyl-amino)-propionique;
Acide 3-({2-[4-(benzothiazol-2-yloxy)-phényl]-éthyl}-cyclobutyl-amino)-propionique;
Acide 3-({2-[4-(benzothiazol-2-yloxy)-phényl]-éthyl}-benzyl-amino)-propionique;
(1-{2-[4-(Benzothiazol-2-yloxy)-phénoxy]-éthyl}-pipéridin-4-yl)-(4-hydroxyméthyl-pipéridin-1-yl)-méthanone;
{2-[4-(Benzothiazol-2-yloxy)-phényl]-éthyl}-cyclopropylamine;
(1-{2-[4-(Benzothiazol-2-yloxy)-phénoxy]-éthyl}-pipéridin-4-yl)-[4-(2-hydroxy-éthyl)-pipéridin-1-yl]-méthanone;
(1-{2-[4-(Benzothiazol-2-yloxy)-phénoxy]-éthyl}-pipéridin-4-yl)-[4-(2-hydroxy-éthyl)-pipéridin-1-yl]-méthanone;
2-({2-[4-(Benzothiazol-2-yloxy)-phényl]-éthyl}-cyclopropyl-amino)-éthanol;
3-({2-[4-(Benzothiazol-2-yloxy)-phényl]-éthyl}-cyclopropyl-amino)-propan-1-ol;
Acide 4-({2-[4-(benzothiazol-2-yloxy)-phényl]-éthyl}-cyclopropyl-amino)-butyrique;
Acide 3-[(1-{2-[4-(benzothiazol-2-yloxy)-phénoxy]-éthyl}-pipéridine-4-carbonyl)-amino]-propionique;
4-({2-[4-(Benzothiazol-2-yloxy)-phényl]-éthyl}-cyclopropyl-amino)-butyronitrile;
Acide 3-(1-{2-[4-(benzothiazol-2-yloxy)-phényl]-éthyl}-pipéridin-4-yl)-propionique;
acide [(1-{2-[4-(benzothiazol-2-yloxy)-phénoxy]-éthyl}-pipéridine-4-carbonyl)-méthyl-amino]-acétique;
3-(4-{2-[4-(Benzothiazol-2-yloxy)-phénoxy]-éthyl}-pipérazin-1-yl)-phénol;
2-(4-{2-[4-(4-Méthoxy-phényl)-pipérazin-1-yl]-éthoxy}-phénoxy)-benzothiazole;
2-{4-[2-(5-Pipéridin-4-yl-tétrazol-1-yl)-éthoxy]-phénol}-benzothiazole;
(*S*)-1-(1-{2-[4-(Benzothiazol-2-yloxy)-phénoxy]-éthyl}-pipéridin-4-yl)-4-hydroxy-pyrrolidin-2-one;
{2-[4-(Benzothiazol-2-yloxy)-phényl]-éthyl}-cyclopropylméthyl-amine;
Ester éthylique de l'acide 2-[({2-[4-(benzothiazol-2-yloxy)-phényl]-éthyl}-cyclopropyl-amino)-méthyl]-cyclopropanecarboxylique;
Ester éthylique de l'acide 4-(4-{2-[4-(benzothiazol-2-yloxy)-phénoxy]-éthyl}-pipérazine-1-carbonyl)-benzoïque;
Acide 2-[({2-[4-(benzothiazol-2-yloxy)-phényl]-éthyl}-cyclopropyl-amino)-méthyl]-cyclopropanecarboxylique;
1-({2-[4-(Benzothiazol-2-yloxy)-phényl]-éthyl}-cyclopropyl-amino)-propan-2-ol;
3-({2-[4-(Benzothiazol-2-yloxy)-phényl]-éthyl}-cyclopropyl-amino)-1,1,1-trifluoro-propan-2-ol;
3-({2-[4-(Benzothiazol-2-yloxy)-phényl]-éthyl}-cyclopropyl-amino)-propionamide;
3-({2-[4-(Benzothiazol-2-yloxy)-phényl]-éthyl}-cyclopropyl-amino)-propane-1,2-diol;
2-{4-[2-(5-Phényl-tétrazol-2-yl)-éthoxy]-phénoxy)-benzothiazole;
2-{4-[2-(5-Phényl-tétrazol-2-yl)-éthoxy]-phénoxy)-benzothiazole;
*N*-{2-[4-(Benzothiazol-2-yloxy)-phényl]-éthyl}-*N-*cyclopropyl-2-(2H-tétrazol-5-yl)-acétamide;
(*S*)-3-({2-[4-(Benzothiazol-2-yloxy)-phényl]-éthyl}-cyclopropyl-amino)-2-méthyl-propan-1-ol;
(*R*)-3-({2-[4-(Benzothiazol-2-yloxy)-phényl]-éthyl}-cyclopropyl-amino)-2-méthyl-propan-1-ol;
2-{4-[2-(5-Méthylsulfanyl-tétrazol-2-yl)-éthoxy]-phénoxy}-benzothiazole;
2-{4-[2-(5-Méthylsulfanyl-tétrazol-1-yl)-éthoxy]-phénoxy}-benzothiazole;
2-[4-(2-Tétrazol-2-yl-éthoxy)-phénoxy]-benzotriazole;
2-[4-(2-Tétrazol-1-yl-éthoxy)-phénoxy]-benzotriazole;
Acide (1*R*,2*R*)-2-(2-[4-(benzothiazol-2-yloxy)-phényl]-éthylamino}-cyclohexanecarboxylique;
Acide (1S,2R)-2-{2-[4-(benzothiazol-2-yloxy)-phényl]-éthylaminol-cyclohexanecarboxylique;
(1R,2R)-2-{2-[4-(Benzothiazol-2-yloxy)-phényl]-éthylamino}-cyclohexanol;
(1*S*,2*R*)-2-{2-[4-(Benzothiazol-2-yloxy)-phényl]-éthylamino}-cyclohexanol;
Acide 4-(1-{2-[4-(Benzothiazol-2-yloxy)-phényl]-éthyl}-pipéridin-4-yl)-butyrique;
(*R*) 1-(1-{2-[4-(Benzothiazol-2-yloxy)-phénoxy]-éthyl}-pipéridin-4-yl)-4-hydroxy-pyrrolidin-2-one;
2-(4-{2-[4-(1*H*-Tétrazol-5-yl)-pipéridin-1-yl]-éthyl}-phénoxy}-benzothiazole;
(1-{2-[4-(Benzothiazol-2-yloxy)-phénoxy]-éthyl}-pipéridin-2-yl)-méthanol;
Ester éthylique de l'acide (1-{2-[4-(benzothiazol-2-yloxy)-phénoxy]-éthyl}-1H-tétrazol-5-yl)-acétique.
Ester éthylique de l'acide (1-{2-[4-(benzothiazol-2-yloxy)-phénoxy]-éthyl}-1H-tétrazol-5-yl)-acétique;
Chlorhydrate 2-{4-[2-(5-pipéridin-4-yl-tétrazol-2-yl)-éthoxy]-phénoxy}-benzothiazole;
7-{2-[4-(Benzothiazol-2-yloxy)-phénoxy]-éthyl}-4-spiro-[3-phtalide]-pipéridine;
Ester éthylique de l'acide 1-{3-[4-(benzothiazol-2-yloxy)-phényl]-propyl}-pipéridine-4-carboxylique;
Chlorhydrate de 2-[4-(benzothiazol-2-yloxy)-phényl]-éthylamine;
2-(4-{2-[4-(1H-Tétrazol-5-yl)-pipéridin-1-yl]-éthoxy}-phénoxy)-benzothiazole;
Acide cis-4-{2-[4-(benzothiazol-2-yloxy)-phényl]-éthylamino}-cyclohexanecarboxylique sel de trifluorométhanesulfonate;
(4-{2-[4-(Benzothiazol-2-yloxy)-phényl]-éthyl}-pipérazin-1-yl)-(tétrahydro-furan-2-yl)-méthanone;
Propane-2-sulfonique (1-{2-[4-(benzothiazol-2-yloxy)-phénoxy]-éthyl}-pipéridine-4-carbonyl)-amide;
Ester méthylique de l'acide (4-{2-[4-(Benzothiazol-2-yloxy)-phényl]-éthyl}-pipérazin-1-yl)-oxo-acétique;
N-(1-{2-[4-(Benzothiazol-2-yloxy)-phényl]-éthyl}-pipéridine-4-carbonyl)-benzènesulfonamide sel de trifluorométhanesulfonate;
N-(1-{2-[4-(Benzothiazol-2-yloxy)-phényl]-éthyl}-pipéridine-4-carbonyl)-méthanesulfonamide sel de trifluorométhanesulfonate;
Acide (4-{2-[4-(benzothiazol-2-yloxy)-phényl]-éthyl}-pipérazine-1-yl)-oxo- acétique sel de trifluorométhanesulfonate;
(4-{2-[4-(Benzothiazol-2-yloxy)-phényl]-éthyl}-pipérazine-1-yl)-morpholin-4-yl-méthanone;
1-(4-{2-[4-(Benzothiazol-2-yloxy)-phényl]-éthyl}-pipérazin-1-yl)-2-thiophen-2-yl-éthanone;
(4-{2-[4-(Benzothiazol-2-yloxy)-phényl]-éthyl-}-pipérazin-1-yl)-pyridin-3-yl-méthanone;
(4-{2-[4-(Benzothiazol-2-yloxy)-phényl]-éthyl}-pipérazin-1-yl)-cyclopropyl-méthanone;
1-(4-{2-[4-(Benzothiazol-2-yloxy)-phényl]-éthyl}-pipérazin-1-yl)-2-méthoxy-éthanone;
1-(4-{2-[4-(Benzothiazol-2-yloxy)-phényl]-éthyl}-pipérazin-1-yl)-2,2,2-trifluoro-éthanone;
Acide 4-(4-{2-[4-(benzothiazol-2-yloxy)-phényl]-éthyl}-pipérazine-1-carbonyl)-benzoïque;
(4-{2-[4-(Benzothiazol-2-yloxy)-phényl]-éthyl}-pipérazine-1-yl)-pyridin-4-yl-méthanone;
(4-{2-[4-(Benzothiazol-2-yloxy)-phényl]-éthyl}-pipérazin-1-yl)-(5-méthyl-pyrazin-2-yl)-méthanone;
(*R*)-(4-{2-[4-(Benzothiazol-2-yloxy)-phényl]-éthyl}-pipérazin-1-yl)-(tétrahydro-furan-2-yl)-méthanone;
(S)-(4-[2-[4-(Benzothiazol-2-yloxy)-phényl]-éthyl)-pipérazin-1-yl]-(tétrahydro-furan-2-yl)-méthanone;
(4-{2-[4-(Benzothiazol-2-yloxy)-phényl]-éthyl}-pipérazin-1-yl)-(tétrahydro-furan-3-yl)-méthanone;
1-(4-{2-[4-(Benzothiazol-2-yloxy)-phényl]-éthyl}-pipérazine-1-yl)-2-hydroxy-éthanone;
2-[2-(4-{2-[4-(Benzothiazol-2-yloxy)-phényl]-éthyl}-pipérazin-1-yl)-2-oxo-éthyl]-cyclopentanone;
Acide 3-(4-{2-[4-(benzothiazol-2-yloxy)-phényl]-éthyl}-pipérazin-1-yl)-propionique sel de trifluorométhanesulfonate;
3-(1-{2-[4-(Benzothiazol-2-yloxy)-phényl]-éthyl}-pipéridin-4-yl)-oxazolidin-2-one;
4-(1-{2-[4-(Benzothiazol-2-yloxy)-phényl]-éthyl}-pipéridin-4-yl)-morpholin-3-one;
4-(1-{2-[4-(Benzothiazol-2-yloxy)-phénoxy]-éthyl}-pipéridin-4-yl)-morpholin-3-one;
3-(1-{2-[4-(Benzothiazol-2-yloxy)-phénoxy]-éthyl}-pipéridin-4-yl)-oxazolidin-2-one;
Benzyloxy-amide de l'acide 1-{2-[4-(benzothiazol-2-yloxy)-phényl]-éthyl}-pipéridine-4-carboxylique;
Acide (1-{2-[4-(benzothiazol-2-yloxy)-phényl]-éthyl}-pipéridin-4-yl)acétique;
(R)-1-(1-{2-[4-(Benzothiazol-2-yloxy)-phényl]-éthyl}-pipéridin-4-yl)-4-hydroxy-pyrrolidin-2-one;
Hydroxyamide de l'acide 1-{2-[4-(benzothiazol-2-yloxy)-phényl]-éthyl}-pipéridine-4-carboxylique;
(S)-1-(1-{2-[4-(Benzothiazol-2-yloxy)-phényl]-éthyl}-pipéridin-4-yl)-4-hydroxy-pyrrolidin-2-one;
Ester tert-butylique de l'acide (1-{2-[4-(benzothiazol-2-yloxy)-phényl]-éthyl}-pipéridin-4-yl)-carbamique;
2-{4-[2-(4-Fluoro-pipéridin-1-yl)-éthyl]-phénoxy}-benzothiazole;
2-{4-[2-(4,4-Difluoro-pipéridin-1-yl)-éthyl]-phénoxy}-benzothiazole;
(R)-1-{2-[4-(Benzothiazol-2-yloxy)-phényl]-éthyl}-pyrrolidin-3-ol;
N-(1-{2-[4-(Benzothiazol-2-yloxy)-phényl]-éthyl}-pipéridin-4-yl)-formamide;
(1-{2-[4-(Benzothiazol-2-yloxy)-phényl]-éthyl}-pipéridin-4-yl)-urée;
1-(1-{2-[4-(Benzothiazol-2-yloxy)-phényl]-éthyl}-pipéridin-4-yl)-3-cyano-2-phényl-isourée;
1-(1-{2-[4-(Benzothiazol-2-yloxy)-phényl]-éthyl}-pipéridin-4-yl)-3-cyano-2-méthyl-isothiourée;
N-(1-{2-[4-(Benzothiazol-2-yloxy)-phényl]-éthyl}-pipéridin-4-yl)-méthanesulfonamide;
1-(1-{2-[4-(Benzothiazol-2-yloxy)-phényl]-éthyl}-pipéridin-4-yl)-3-cyano-2-méthyl-guanidine;
8-{2-[4-(Benzothiazol-2-yloxy)-phényl]-éthyl}-1-phényl-1,3,8-triaza-spiro[4.5]décan-4-one;
8-{2-[4-(Benzothiazol-2-yloxy)-phényl]-éthyl}-1,3,8-triaza-spiro[4.5]décane-2,4-dione;
Ester tert-butylique de l'acide (1-{2-[4-(benzothiazol-2-yloxy)-phényl]-éthyl}-pipéridin-4-yl)-méthyl-carbamique;
N-(1-{2-[4-(Benzothiazol-2-yloxy)-phényl]-éthyl}-pipéridin-4-yl)-N-méthyl-acétamide;
N-(1-{2-[4-(Benzothiazol-2-yloxy)-phényl]-éthyl}-pipéridin-4-yl)-N-méthyl-méthanesulfonamide;
Ester méthylique de l'acide acétique [(1-{2-[4-(benzothiazol-2-yloxy)-phényl]-éthyl}-pipéridin-4-yl)-méthyl-carbamoyl];
N-(1-{2-[4-(Benzothiazol-2-yloxy)-phényl]-éthyl}-pipéridin-4-yl)-N-acétamide;
Ester méthylique de l'acide (1-{2-[4-(benzothiazol-2-yloxy)-phényl]-éthyl}-pipéridin-4-ylcarbamoyl)-;
Acide 2-({2-[4-(benzothiazol-2-yloxy)-phényl]-éthyl}-méthyl-amino)-3-(1H-imidazol-2-yl)-propionique;
2-(4-{2-[4-(3-Nitro-pyridin-2-yl)-[1.4]diazépan-1-yl]-éthyl]-phénoxy)-benzothiazole;
Ester éthylique de l'acide [(1-{2-[4-(benzothiazol-2-yloxy)-phénoxy]-éthyl}-pipéridine-4-carbonyl)-méthyl-amino]-acétique;
Ester éthylique de l'acide 1'-{2-[4-(benzothiazol-2-yloxy)-phénoxy]-éthyl}-[1,4']bipipéridinyl-4-carboxylique;
Acide 1'-{2-[4-(benzothiazol-2-yloxy)-phénoxy]-éthyl}-[1,4']bipipéridinyl-4-carboxylique;
{2-[4-(Benzothiazol-2-yloxy)-phényl]-éthyl}-cyclopropyl-éthyl-amine;
Sel de l'acide trifluorométhanesulfonique d'acide 3-({2-[4-(benzothiazol-2-yloxy)-phényl]-éthyl}-cyclopropyl-amino)-2-méthyl-propionique;
2-({2-[4-(Benzothiazol-2-yloxy)-phényl]-éthyl}-cyclopropylméthyl-amino)-éthanol;
2-[2-({2-[4-(Benzothiazol-2-yloxy)-phényl]-éthyl}-cyclopropylméthyl-amino)-éthoxy]-éthanol;
3-({2-[4-(Benzothiazol-2-yloxy)-phényl]-éthyl}-cyclopropylméthyl-amino)-propan-1-ol;
{2-[4-(Benzothiazol-2-yloxy)-phényl]-éthyl}-cyclopropylméthyl-(3-tétrazol-2-y1-propyl)-amine;
{2-[4-(Benzothiazol-2-yloxy)-phényl]-éthyl}-cyclopropyl-(3-pyrrol-1-yl-propyl)-amine;
4-({2-[4-(Benzothiazol-2-yloxy)-phényl]-éthyl}-cyclopropylméthyl-amino)-butyronitrile;
Acide 1-{2-[4-(benzothiazol-2-yloxy)-phényl]-éthyl}-pipéridine-4-carboxylique (2-cyano-éthyl)-amide;
{2-[4-(Benzothiazol-2-yloxy)-phényl]-éthyl}-cyclopropylméthyl-[3-(2H-tétrazol-5-yl)-propyl]-amine;
3-[5-(1-{2-[4-(Benzothiazol-2-yloxy)-phényl]-éthyl}-pipéridin-4-yl)-tétrazol-1-yl]-propionitrile;
{2-[4-(Benzothiazol-2-yloxy)-phényl]-éthyl}-cyclopropyl-[3-(2H-tétrazol-5-yl)-propyl]-amine;
Acide carboxylique 1-{2-[4-(Benzothiazol-2-yloxy)-phényl]-éthyl}-pipéridine-4-(2-hydroxy-1,1-diméthyl-éthyl)-amide;
{2-[4-(Benzothiazol-2-yloxy)-phényl]-éthyl}-cyclopropylméthyl-[3-(1H-[1,2,4]triazol-3-yl)-propyl]-amine;
{2-[4-(Benzothiazol-2-yloxy)-phényl]-éthyl}-cyclopropylméthyl-[3-(5-méthyl-1H-[1,2,4]triazol-3-yl)-propyl]-amine;
{2-[4-(Benzothiazol-2-yloxy)-phényl]-éthyl}-cyclopropylméthyl-[3-(5-phényl-1H-[1,2,4]triazol-3-yl)-propyl]-amine;
2-(4-{2-[4-(1-Méthyl-1H-tétrazol-5-yl)-pipéridin-1-yl]-éthyl}-phénoxy)-benzothiazole;
2-(4-{2-[4-(2-Méthyl-2H-tétrazol-5-yl)-pipéridin-1-yl]-éthyl}-phénoxy)-benzothiazole;
1-{2-[4-(Benzothiazol-2-yloxy)-phényl]-éthyl}-pipéridine-4-carbonitrile;
2-(4-{2-[4-(1H-[1,2,3]Triazol-4-yl)-pipéridin-1-yl]-éthyl}-phénoxy)-benzothiazole;
Ester éthylique de l'acide 4-{2-[4-(benzothiazol-2-yloxy)-phényl]-éthylamino}-butyrique;
Ester éthylique de l'acide 4-({2-[4-(benzothiazol-2-yloxy)-phényl]-éthyl}-cyclopropylméthyl-amino)-butyrique;
2-[3-({2-[4-(Benzothiazol-2-yloxy)-phényl]-éthyl)-cyclopropylméthyl-amino)-propyl]-isoindole-1,3-dione;
Acide 4-({2-[4-(benzothiazol-2-yloxy)-phényl]-éthyl}-cyclopropylméthyl-amino)-butyrique;
1-(3-{2-[4-(Benzothiazol-2-yloxy)-phényl]-éthylamino}-propyl)-pyrrolidin-2-one;
N-1-{2-[4-(Benzothiazol-2-yloxy)-phényl]-éthyl}-N1-cyclopropylméthyl-propane-1,3-diamine;
Ester méthylique de l'acide 5-({2-[4-(benzothiazol-2-yloxy)-phényl]-éthyl}-cyclopropyl-amino)-pentanoïque;
N-[3-({2-[4-(Benzothiazol-2-yloxy)-phényl]-éthyl}-cyclopropylméthyl-amino)-propyl]-acétamide;
Acide Morpholine-4-carboxylique [3-({2-[4-(benzothiazol-2-yloxy)-phényl]-éthyl}-cyclopropylméthyl-amino)-propyl]-amide;
N-[3-({2-[4-(Benzothiazol-2-yloxy)-phényl]-éthyl}-cyclopropylméthyl-amino)-propyl]-méthanesulfonamide acide 5-({2-[4-(benzothiazol-2-yloxy)-phényl]-éthyl}-cyclopropyl-amino)-pentanoïque;
1-[3-({2-[4-(Benzothiazol-2-yloxy)-phényl]-éthyl}-isopropyl-amino)-propyl]-pyrrolidin-2-one;
1-[3-({2-[4-(Benzothiazol-2-yloxy)-phényl]-éthyl}-cyclopropylméthyl-amino)-propyl]-pyrrolidin-2-one;
1-[3-({2-[4-(Benzothiazol-2-yloxy)-phényl]-éthyl}-cyclopropylamino)-propyl]-pyrrolidin-2-one;
1-[3-({2-[4-(Benzothiazol-2-yloxy)-phényl]-éthyl}-propylamino)-propyl]-pyrrolidin-2-one;
Ester éthylique de l'acide 4-((1-acétyl-pipéridin-4-yl)-{2-[4-(benzothiazol-2-yloxy)-phényl]-éthyl}-amino)-butyrique;
Ester éthylique de l'acide 4-({1-acétyl-pipéridin-4-yl)-{2-[4-(benzothiazol-2-yloxy)-phényl]-éthyl}-amino)-butyrique;
Acide 4-({2-[4-(benzothiazol-2-yloxy)-phényl]-éthyl}-méthanesulfonyl-amino)-butyrique;
Acide acétique ({2-[4-(benzothiazol-2-yloxy)-phényl]-éthyl}-cyclopropyl-amino)-acétique;
Ester éthylique de l'acide 6-({2-[4-(benzothiazol-2-yloxy)-phényl]-éthyl}-cyclopropyl-amino)-hexanoïque;
Ester éthylique de l'acide 7-({2-[4-(benzothiazol-2-yloxy)-phényl]-éthyl}-cyclopropyl-amino)-heptanoïque;
Acide 6-({2-[4-(benzothiazol-2-yloxy)-phényl]-éthyl}-cyclopropyl-amino)-hexanoïque;
Acide 7-({2-[4-(Benzothiazol-2-yloxy)-phényl]-éthyl}-cyclopropyl-amino)-heptanoïque;
N-1-{2-[4-(Benzothiazol-2-yloxy)-phényl]-éthyl}-N1-cyclopropyl-propane-1,3-diamine;
N-[3-({2-[4-(Benzothiazol-2-yloxy)-phényl]-éthyl}-cyclopropyl-amino)-propyl]-acétamide;
N-[3-({2-[4-(Benzothiazol-2-yloxy)-phényl]-éthyl}-cyclopropyl-amino)-propyl]-isobutyramide;
N-[3-({2-[4-(Benzothiazol-2-yloxy)-phényl]-éthyl}-cyclopropyl-amino)-propyl]-benzamide;
N-[3-({2-[4-(Benzothiazol-2-yloxy)-phényl]-éthyl}-cyclopropyl-amino)-propyl]-benzamide;
N-[3-({2-[4-(Benzothiazol-2-yloxy)-phényl]-éthyl}-cyclopropyl-amino)-propyl]-méthanesulfonamide;
Sel de l'acide propane-2-sulfonique [3-({2-[4-(benzothiazol-2-yloxy)-phényl]-éthyl)-cyclopropyl-amino)-propyl]-amide trifluorométhanesulfonique;
Ester éthylique de l'acide 8-({2-[4-(benzothiazol-2-yloxy)-phényl]-éthyl}-cyclopropyl-amino)-octanoïque;
1-[3-({2-[4-(Benzothiazol-2-yloxy)-phényl]-éthyl}-cyclopropyl-amino)-propyl]-3-phényl-urée;
Acide 8-({2-[4-(benzothiazol-2-yloxy)-phényl]-éthyl}-cyclopropyl-amino)-octanoïque;
Acide tétrahydro-furan-2-carboxylique [3-({2-[4-(benzothiazol-2-yloxy)-phényl]-éthyl}-cyclopropyl-amino)-propyl]-amide;
N-[3-({2-[4-(Benzothiazol-2-yloxy)-phényl]-éthyl}-cyclopropyl-amino)-propyl]-2-hydroxy-acétamide;
Acide 4-({2-[4-(benzothiazol-2-yloxy)-phénoxy]-éthyl}-cyclopropyl-amino)-butyrique;
2-[4-(2-Morpholin-4-yl-éthoxy)-phénoxy]-benzothiazole;
2-[4-(2-Pipéridin-1-yl-éthoxy)-phénoxy]-benzothiazole.

14. Composé selon la revendication 1, où ledit composé de formule (II) est l'un de:
1-{2-[4-(1*H*-Benzoimidazol-2-yloxy)-phénoxy]-éthyl}-4-phényl-pipéridin-4-ol;
{2-[4-(1*H*-Benzoimidazol-2-yloxy)-phényl]-éthyl}-cyclopropylméthyl-propyl-amine;
Cyclohexyl-éthyl-{2-[4-(1-méthyl-1*H*-benzoimidazol-2-yloxy)-Phényl]-éthyl}-amine;
1-{2-[4-(1*H*-Benzoimidazol-2-yloxy)-phénoxy]-éthyl}-4-(4-bromo-phényl)-pipéridin-4-ol;
1-{2-[4-(1*H*-Benzoimidazol-2-yloxy)-phénoxy]-éthyl}-4-(4-chloro-phényl)-pipéridin-4-ol;
1-{2-[4-(1*H*-Benzoimidazol-2-yloxy)-phénoxy]-éthyl}-4-benzyl-pipéridin-4-ol;
{2-[4-(1*H*-Benzoimidazol-2-yloxy)-phényl]-éthyl}-cyclohexyl-éthyl-amine;
2-[4-(2-Pyrrolidin-1-yl-éthyl)-phénoxy]-1*H*-benzoimidazole; 2-[4-(2-Azepan-1-yl-éthyl)-phénoxy]-1H-benzoimidazole;
{2-[4-(1*H*-Benzoimidazol-2-yloxy)-phényl]-éthyl}-dibutyl-amine;
1-{2-[4-(1*H*-Benzoimidazol-2-yloxy)-phényl]-éthyl}-pipéridin-4-ol;
Ester méthylique de l'acide 1-{2-[4-(1*H*-benzoimidazol-2-yloxy)-phényl]-éthyl}-pipéridine-4-carboxylique;
{2-[4-(1*H*-Benzoimidazol-2-yloxy)-phénoxy]-éthyl}-cyclohexyl-éthyl-amine;
2-{4-[2-(4-Méthyl-pipéridin-1-yl)-éthoxy]-phénoxy}-1*H-*benzoimidazole;
2-(4-[2-(2-Ethyl-pipéridin-1-yl)-éthoxy]-phénoxy}-1*H-*benzoimidazole;
2-[4-(2-Pipéridin-1-yl-éthoxy)-phénoxy]-1*H*-benzoimidazole;
1-{2-[4-(1*H*-Benzoimidazol-2-yloxy)-phénoxy]-éthyl}-pipéridin-4-ol;
2-[4-(2-Azepan-1-yl-éthoxy)-phénoxy]-1H-benzoimidazole amide;
{3-[4-(1*H*-Benzoimidazol-2-yloxy)-phénoxy]-propyl}-diméthyl-amine;
2-[4-(2-Pyrrolidin-1-yl-éthoxy)-phénoxy]-1*H*-benzoimidazole;
{2-[4-(1*H*-Benzoimidazol-2-yloxy)-phénoxy]-éthyl}-diéthyl-amine;
2-[4-(2-Morpholin-4-yl-éthoxy)-phénoxy]-1*H*-benzoimidazole;
2-[4-(2-Pipéridin-1-yl-éthyl)-phénoxyl-1*H*-benzoimidazole;
1-(1-{2-[4-(1*H*-Benzoimidazol-2-yloxy)-phénoxy]-éthyl}-pipéridin-4-yl)-pyrrolidin-2-one;
(1-{2-[4-(1H-Benzoimidazol-2-yloxy)-phénoxy]-éthyl}-pipéridin-4-yl)-méthanol; et
Ester éthylique de l'acide 1-{2-[4-(1*H*-benzoimidazol-2-yloxy)-phénoxy]-éthyl)-pipéridine-4-carboxylique;

15. Composition pharmaceutique comprenant une quantité thérapeutiquement efficace d'au moins un composé de formule (I) 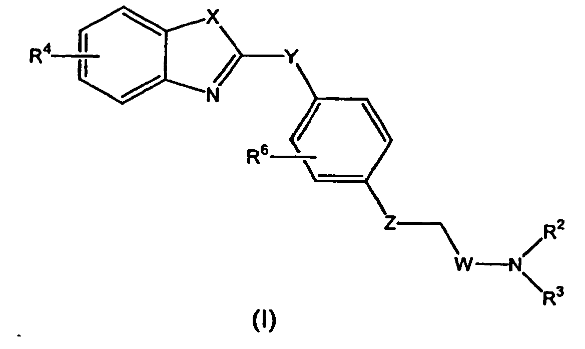 où
X est sélectionné dans le groupe consistant en NR⁵, O, et S, avec R⁵ qui est l'un de H et CH₃;
Y est sélectionné dans le groupe consistant en CH₂, et O;
Z est sélectionné dans le groupe consistant en O et une liaison;
W est sélectionné dans le groupe consistant en CH₂ et CHR¹-CH₂, avec R¹ étant l'un de H et OH, où l'élément de carbone attaché à R¹ dans ledit CHR¹-CH₂ n'est pas directement attaché à l'élément d'azote auquel est attaché ledit W;
R⁴ est sélectionné dans le groupe consistant en H, OCH₃, Cl, F, Br, I, OH, NH₂, CN, CF₃ et CH₃;
R⁶ est H ou F; et
chacun de R² et R³ est indépendamment sélectionné dans le groupe consistant en:
A) H, alkyle C₁₋₇, alcényle C₃₋₇, où le carbone dans ledit alcényle qui est attaché à l'élément d'azote n'a que de simples liaisons, alkynyle C₃₋₇, où le carbone dans ledit alkynyle qui est attaché à l'élément d'azote n'a que de simples liaisons, cycloalkyle C₃₋₇ facultativement benzofusionné, cycloalcényleC₅₋₇, cycloalkylC₃₋₇alkyleC₁₋₇, alkyleC₁₋₇cycloalkyleC₃₋₇ et phényle, où chacun des substituants A) est indépendamment substitué par 0, 1 ou 2 R^{Q}, et chacun desdits R^{Q} est un substituant à un élément de carbone qui est au moins un élément de carbone retiré de l'élément d'azote;
B) un substituant HetR^{a};
C) alkyle-C₁₋₇C(O)R^{x}, facultativement substitute par CH₂R^{Ar} ou CH₂R^{Ar'};
D) alkyle-C₂₋₅C(O)R^{x}, où deux éléments de carbone de valence admise dans l'alkylC₂₋₅ dudit alkyl-C₂₋₅C(O)R^{x} font partie d'un carbocycleC₃₋₆ saturé;
E) alkyle-C₂₋₅OH où deux éléments de carbone à valence admise dans l'alkylC₂₋₅ dudit alkylC₂₋₅OH font partie du carbocycleC₃₋₆ saturé;
F) -alkyl-C₀₋₄phényle, où le phényle dans ledit -alkylC₀₋₄phényle est fusionné en deux éléments de carbone adjacents dans ledit phényle à R^{f}, ou est benzofusionné;
G) -alkylC₀₋₄Ar⁸, où Ar⁶ est un hétéroaryle à 6-membres ayant un point d'attachement d'élément de carbone ayant un ou deux éléments hétéroatomes -N=, et benzofusionné;
H) -alkyl-C₀₋₄Ar⁴, où Ar⁵ est un hétéroaryle à 5-membres, ayant un élément d'hétéroatome sélectionné dans le groupe consistant en O, S, et >NR^{Y} et ayant 0 ou 1 élément hétéroatome additionnel -N=, contenant facultativement deux groupes carbonyle, et facultativement benzofusionnés;
I) alkyleC₁₋₄Ar^{5'}, où Ar^{5'} est un hétéroaryle à 5-membres contenant 3 ou 4 éléments d'azote, facultativement substitués par R^{Y}, et ayant un site de valence admise comme point d'attachement;
J) -alkyl-C₀₋₄Ar⁶⁻⁶, où Ar⁶⁻⁶ est un phényle attaché à alkyle C₀₋₄ fusionné au site de valence admise à un hétéroaryle à 6-membres, où ledit hétéroaryle à 6-membres a un ou deux éléments hétéroatome -N=;
K) alkyle-C₀₋₄Ar⁶⁻⁵, où Ar⁶⁻⁵ est un phényle attaché à alkyleC₀₋₄ fusionné au site à valence admise à un hétéroaryle à 5-membres, ledit hétéroaryle à 5-membres ayant un élément hétéroatome sélectionné dans le groupe consistant en O, S, et >NR^{Y}, et ledit hétéroaryle à 5-membres ayant 0 ou 1 élément hétéroatome additionnel qui est -N=; et
L) l'un de 2-(4-éthyl-phénoxy)-benzothiazole, 2-(4-éthyl-phénoxy)-benzooxazole, et 2-(4-éthyl-phénoxy)-1*H*-benzoimidazole;
M) SO₂alkyleC₁₋₄;
alternativement R² et R³ sont pris ensemble avec l'azote auquel ils sont attachés pour former un cycle hétérocyclique qui contient au moins un élément hétéroatome qui est ledit azote d'attachement, ledit cycle hétérocyclique étant sélectionné dans le groupe consistant en:
un cycle hétérocyclique à 4-7 membres HetR^{b}, ledit cycle hétérocyclique à 4-7 membres Het^{Rb} ayant un élément hétéroatome qui est ledit azote d'attachement, et étant substitué par 0, 1 ou 2 substituants sur le même ou des éléments différents de substitutants, lesdits substituants étant sélectionnés dans le groupe consistant en -R^{Y}, -CN, -C(O)R^{Y}, alkyleC₀₋₄CO₂R^{Y}, alkyle-C₀₋₄C(O)CO₂R^{Y}, alkyle-C₀₋₄OR^{Y}, alkyle-C₀₋₄C(O)NR^{Y}R², alkyleC₀₋₄NR^{Y}C(O)R^{Z}, -C(O)NR^{Z}OR^{Y}, alkyle-C₀₋₄NR^{Y}C(O)CH₂OR^{Y}, alkyleC₀₋₄NR^{Y}C(O)CH₂C(O)R^{Y}, alkyle-C₀₋₄NR^{Y}CO₂R^{Y}, -alkyle C₀₋₄NR^{Y}C(O)NR^{Y}R^{Z}, alkyleC₀₋₄NR^{Y}C(S)NR^{Y}R^{Z}, -NR^{Y} C(O)CO₂R^{Y}, -NR^{Y}R^{Z}, -alkylC₀₋₄NR^{W}SO₂R^{Y}, 1, 3-dihydro-indol-2-one-1-yle, 1,3-dihydro-benzo-imidazol-2-one-1-yle, tétrazol-5-yle, 1-R^{Y}-1H-tétrazol-5-yle, R^{Y}-triazolyle, 2-R^{Y}-2*H-*tétrazol-5-yle, pyrrolidine-2-thion-1-yle, pipéridine-2-thion-1-yle, pipéridine-2-thion-1-yle, -alkyleC₀₋₄C(O)N(R^{Y})(SO₂R^{Y}), alkyle-C₀₋₄N(R^{Y}) (SO₂)NR^{Y}R^{Y}, -alkyleC₀₋₄N(R^{Y})(SO₂)NR^{Y}R^{Y}, 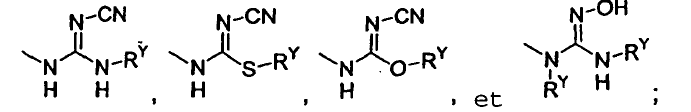
ii) un cycle hétérocyclique à 5-7 membres HetR^{C}, ledit cycle hétérocyclique à 5-7 HetR^{C} ayant un élément hétéroatome additionnel séparé dudit azote d'attachement par au moins un élément de carbone, ledit élément d'hétéroatome additionnel étant sélectionné dans le groupe consistant en O, S(=O)₀₋₂, et >NR^{M}, ledit cycle hétérocyclique à 5-7 membres HetR^{C} ayant 0 ou 1 éléments de carbonyle, et étant substitué par 0, 1, ou 2 substituants aux mêmes éléments ou en des éléments différents de substitution de carbone, lesdits substituants étant sélectionnés dans le groupe consistant en -C (O)R^{Y}, -CO₂R^{Y}, alkyle-C₃₋₄CO₂R^{Y} et R^{Z};
iii) l'un de imidazolidin-1-yle, 2-imidazolin-1-yle, pyrazol-1-yle, imidazol-1-yle, 2^{H}-tétrazol-2-yle, 1*H*-tétrazol-1-yle, pyrrol-1-yle, 2-pyrrolin-1-yle, et 3-pyrrolin-1-yle, où chacun dudit 2H-tétrazol-2-yle et 1H-tétrazol-1-yle est substitué à un élément de carbone par 0 ou 1 de alkyle-C₀₋₄R^{Z}, alkyle-C₀₋₄SRY, -alkyleC₀₋₄CO₂R^{Y}, et le substituant HetR^{a}; et
iv) l'un de 1,2,3,4-tétrahydro-quinolin-1-yle, 1,2,3,4-tétrahydro-isoquinolin-2-yle, indol-1-yle, isoindol-2-yle, indolin-1-yle, benzi-midazol-1-yle, 2,8-diaza-spiro[4.5]décan-1-one-8-yle, 4-{[(2-tert-butoxycarbonylamino-cyclobutanecarbonyl)-amino]-méthyl}-pipéridin-1-yle, 4-{[2-amino-cyclobutanecarbonyl)-amino]-méthyl}-pipéridin-1-yle, ester tert-butylique de l'acide 3,9-diaza-spiro[5.5]undécane-3-carboxylique -9-yle, 4-oxo-1-phényl-1,3,8-triaza-spiro[4.5]dec-8-yle, et 4-oxo-1,3,8-triaza-spiro[4.5]dec-8-yle;
où
le substituant HetR^{a} est un cycle hétérocyclique à 4-7 membres ayant un poids d'attachement d'éléments de carbone et contenant un élément >NR^{M} en tant qu'élément d'hétéroatome, et ledit élément d'hétéroatome étant séparé dudit point d'attachement d'élément de carbone par au moins un élément de carbones additionnels;
R^{K} est sélectionné dans le groupe consistant en H, alkyle C₁₋₄, alkyle-C₀₋₄R^{Ar} chacun facultativement substitué par 1, 2, ou 3 substituants R^{N}
R^{L} est sélectionné dans le groupe consistant en -CO₂R^{S} et -C(O)NR^{S}R^{S'};
R^{M} est sélectionné dans le groupe consistant en R^{Z}, indol-7-yle, -SO₂R^{Y}, alkyleC₃₋₄CO₂RY, -CO₂R^{Y}, -C(O)NR^{Z}OR^{Y}, -C(O)R^{Y}, -C(O)alkyleC₁₋₄OR^{Y}, alkyleC₀₋₄C(O)NR^{S}R^{S'}, alkyleC₀₋₄C(O)CO₂R^{Y}, 1,3-dihydro-indol-2-one-1-yle, 1,3-dihydro-benzoimidazol-2-one-1-yle, tétrazol-5-yle, 1-R^{Y}-1*H*-tétrazol-5-yle, 1-R^{Y}-triazolyle, 2-R^{Y}-2*H-*tétrazol-5-yle et alkyleC₀₋₄C(O)N(R^{Y})(SO₂R^{Y}), chacun facultativement substitué par 1,2 ou 3 substituants R^{N};
R^{N} est sélectionné dans le groupe consistant en OCH₃, Cl, F, Br, I, OH, NH₂, CN, CF₃, CH₃, OC(O)CH₃, et NO₂;
R^{P} est sélectionné dans le groupe consistant en R^{Y}, alkyle-C₂₋₄OR^{Y}, R^{Ar}, alkyleC₁₋₂CO₂R^{Y}, -alkyleC₁₋₂CONR^{S}R^{S'}, indol-7-yle, et -SO₂alkyleC₁₋₄;
R^{Q} est sélectionné dans le groupe consistant en fluoro, chloro, bromo, iodo, trifluorométhyle, trichloro-méthyle, -CN, -alkyleC₁₋₄, -alkyleC₀₋₄R^{Ar}, -alkyleC₀₋₄R^{Ar'}, -alkyleC₀₋₄OR^{Y}, -alkyleC₀₋₄CO₂R^{Y}, -alkyleC₀₋₄NR^{Y}R^{Z}, alkyleC₀₋₄NR^{Y}COR^{Y}, alkyle-C₀₋₄NR^{Y}CONR^{Y}R^{Z}, -alkyleC₀₋₄NR^{Y}SO₂R^{Y}, et alkyleC₀₋₄SR^{Y};
R^{S} et R^{S'} sont indépendamment sélectionnés dans le groupe consistant en H, alkyleC₁₋₄, et alkyle C₀₋₄ phényle; alternativement, R^{S} et R^{S'} sont pris ensemble avec l'élément d'azote auquel ledit RS et ledit RS' sont attachés pour former un cycle hétérocyclique à 4-7 membres ayant 0 ou 1 éléments hétéroatomes additionnels sélectionnés dans le groupe consistant en O, S, et >NR^{Y}, à condition que ledit élément hétéroatome additionnel soit séparé par au moins deux éléments de carbone dudit élément d'azote auquel ledit R^{S} et ledit R^{S}' sont attachés et à condition que quand R^{Y} est alkyleC0-4^{Ar}, alors R^{Ar} ne sont pas substitués par R^{L};
R^{W'} est sélectionné dans le groupe consistant en R^{Y}, et cycloalkyle-C₃₋₇;
R^{X} est sélectionné dans le groupe consistant en -OR^{Y}, -NR^{Y}R^{Z}, -alkyleC₁₋₄, et -alkyleC₀₋₄R^{Ar};
R^{Y} est sélectionné dans le groupe consistant en H, alkyle-C₁₋₄, -alkyleC₀₋₄R^{Ar} et -alkyleC₀₋₄R^{Ar'}, chacun facultativement substitué par 1, 2 ou 3 substituants R^{N};
R^{Z} est sélectionné dans le groupe consistant en R^{Y}, -alkyleC₂₋₄OR^{Y}, -alkyleC₁₋₂CO₂R^{Y}, alkyle-C₁₋₂C(O)NR^{S}R^{S'}, et -alkyleC2-4NR^{S}R^{S'};
quand R^{Y} et R^{Z'} sont attachés à un élément d'azote, R^{Y} et R^{Z} sont sélectionnés comme définis ci-dessus, ou bien R^{Y} et R^{Z} sont pris ensemble avec l'élément d'azote attaché R^{Y} et R^{Z} pour former un cycle hétérocyclique à 4-7 membres HetR^{d} ayant 0 ou 1 élément d'hétéroatome additionnel sélectionné dans le groupe consistant en O, S, et >NR^{M}, ledit cycle hétérocyclique à 4-7 membres HetR^{d} ayant 0 ou 1 élément carbonyle et ledit cycle hétérocyclique à 4-7 membres HetR^{d} ayant 0 ou 1 élément de carbone à valence admise substitués par au moins l'un de R^{M}, -CO₂H, et alkyle-C₀₋₁OR^{Y};
R^{Ar} est une fraction avec un point d'attachement d'élément carbone et ladite fraction est sélectionnée dans le groupe consistant en phényle, pyridyle, pyridinyle, et pyrazinyle, où chaque élément de carbone à valence admise dans chacune desdites fractions est indépendamment substituée par au moins l'un de 0, 1, 2 ou 3 R^{N}, et 0 ou 1 R^{L};
R^{Ar'} est un cycle à 3-8 membres ayant 0, 1 ou 2 éléments d'hétéroatomes sélectionnés dans le groupe consistant en O, S, N, et >NR^{Y}, ayant, 0, 1, ou 2 liaisons insaturées ayant 0 ou 1 éléments carbonyle, où chaque élément à valence admise dans chacun desdits cycles est indépendamment substitué par 0, 1, ou 2 R^{K}; et
R^{f} est une fraction d'hydrocarbure linéaire à 3 à 5 membres ayant 0 ou 1 liaison carbone-carbone insaturée ayant 0 ou 1 éléments carbonyle;
ou un énantiomère, diastéréomère, racémique, tautomère, hydrate, solvate, ou un sel, ester ou amide pharmaceutiquement acceptables de celui-ci.

16. Composition pharmaceutique comprenant au moins un composé selon l'une quelconque des revendications 1 - 14.

17. Utilisation d'une quantité thérapeutiquement efficace d'au moins un modulateur de LTA4H sélectionné parmi des composés d'une quelconque des revendications 1, revendication 2 ou des composés de formule (I) selon la revendication 12, dans la fabrication d'un médicament pour le traitement ou la prévention d'une condition provoquée par LTA4H chez un sujet.

18. Utilisation d'au moins un modulateur de LTA4H sélectionné parmi les composés selon l'une quelconque de la revendication 1, la revendication 2 ou les composés de formule (I), selon la revendication 12 dans la fabrication d'un médicament pour le traitement, la prévention ou l'inhibition d'une inflammation chez un sujet.

19. Utilisation d'une quantité d'inhibition d'au moins un modulateur de LTA4H sélectionné parmi les composés selon l'une quelconque de la revendication 1, de la revendication 2 ou des composés de formule (I) selon la revendication 12 dans la fabrication d'un médicament pour l'inhibition de l'activité de l'enzyme LTA4H.

20. Utilisation selon la revendication 17 où ledit R⁴ est H.

21. Utilisation selon la revendication 18 où ledit R⁴ est H.

22. Utilisation selon la revendication 19 où ledit R⁴ est H.

23. Utilisation selon la revendication 17, où ledit R² et ledit R³ sont chacun indépendamment sélectionnés parmi
(i) le groupe consistant en A), B), C), D), E), et I) quand R² et R³ sont sélectionnés parmi différents groupes; ou
(ii) groupe A); ou
(iii)groupe B); ou
(iv) groupe C); ou
(v) groupe D); ou
(vi) groupe E); ou
(vii)groupe I),
comme défini à la revendication 1, la revendication 2 ou la revendication 12.

24. Utilisation selon la revendication 18, où ledit R² et ledit R³ sont chacun indépendamment sélectionnés parmi
(i) le groupe consistant en A), B), C), D), E), et I) quand R² et R³ sont sélectionnés parmi différents groupes; ou
(ii) groupe A); ou
(iii)groupe B); ou
(iv) groupe C); ou
(v) groupe D); ou
(vi) groupe E); ou
(vii)groupe I),
comme défini à la revendication 1, la revendication 2 ou la revendication 12.

25. Utilisation selon la revendication 19, où ledit R² et R³ sont chacun indépendamment sélectionnés parmi
(i) le groupe consistant en A), B), C), D), E), et I) quand R² et R³ sont sélectionnés parmi différents groupes, ou
(ii) groupe A); ou
(iii)groupe B); ou
(iv) groupe C); ou
(v) groupe D); ou
(vi) groupe E); ou
(vii)groupe I),
comme défini à la revendication 1, la revendication 2 ou la revendication 12.

26. Utilisation selon la revendication 17, où ledit R² et ledit R³ sont pris ensemble avec l'azote auquel ils sont attachés pour former un cycle hétérocyclique qui contient au moins un élément d'hétéroatome qui est ledit azote d'attachement, ledit cycle hétérocyclique étant sélectionné parmi l'un de
(g) le groupe consistant en i) et ii) quand R² et R³ sont sélectionnés dans différents groupes;
(h) le groupe i), et
(i) le groupe ii)
comme défini à la revendication 1, à la revendication 2 ou la revendication 12.

27. Utilisation selon la revendication 18, où ledit R² et R³ sont pris avec l'azote auquel ils sont attachés pour former un cycle hétérocyclique qui contient au moins un élément d'hétéroatome qui est ledit azote d'attachement, ledit cycle hétérocyclique étant sélectionné parmi l'un de
(j) le groupe consistant en i) et ii) quand R² et R³ sont sélectionnés dans différents groupes;
(k) le groupe i), et
(l) le groupe ii)
comme défini à la revendication 1, la revendication 2 ou la revendication 12.

28. Utilisation selon la revendication 19, où ledit R² et R³ sont pris avec l'azote auquel ils sont attachés pour forme un cycle hétérocyclique qui contient au moins un élément d'hétéroatome qui est ledit azote d'attachement, ledit cycle hétérocyclique étant sélectionné parmi l'un de
(m) le groupe consistant en i) et ii) quand R² et R³ sont sélectionnés parmi différents groupes;
(n) le groupe i), et
(o) le groupe ii)
comme défini à la revendication 1, la revendication 2 ou la revendication 12.

29. Utilisation selon l'une quelconque des revendications 17-28, où ladite condition provoquée par LTA4H est une inflammation due à au moins l'un de l'asthme, une maladie d'obstruction pulmonaire chronique, l'athérosclérose, l'arthrite rhumatoïde, la sclérose en plaques, une maladie inflammatoire des intestins, et le psoriasis.

30. Utilisation selon l'une quelconque des revendications 17-19, où ledit au moins un modulateur de LTA4H est sélectionné parmi des composés selon l'une quelconque des revendications 9-14.
